(19) Europäisches Patentamt
European Patent Office
Office européen des brevets

(11) EP 4 238 577 A2

(12) EUROPEAN PATENT APPLICATION

(43) Date of publication:
**06.09.2023 Bulletin 2023/36**

(21) Application number: **22203154.4**

(22) Date of filing: **21.10.2022**

(51) International Patent Classification (IPC):
***A61K 39/12*** $^{(2006.01)}$ ***A61P 31/14*** $^{(2006.01)}$

(52) Cooperative Patent Classification (CPC):
**A61K 39/12; A61K 9/0019; A61K 47/18; A61K 47/26; A61P 31/14;** A61K 2039/53; A61K 2039/545; A61K 2039/55; A61K 2039/55555; A61K 2039/58; A61K 2039/70; C12N 2770/20034

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**22.10.2021 US 202163271108 P**
**29.10.2021 US 202163273878 P**
**17.12.2021 US 202163291339 P**
**25.01.2022 US 202263303011 P**
**16.05.2022 US 202263342620 P**

(71) Applicant: **BioNTech SE**
**55131 Mainz (DE)**

(72) Inventors:
- **BADKAR, Advait Vijay**
  **Andover, 01810 (US)**
- **PANZNER, Steffen**
  **55131 Mainz (DE)**
- **WEISER, Sarah Elizabeth**
  **Andover, 01810 (US)**

(74) Representative: **Thomann, William John**
**ZSP**
**Patentanwälte PartG mbB**
**Hansastraße 32**
**80686 München (DE)**

Remarks:
- The complete document including Reference Table(s) and the Sequence Listing(s) can be downloaded from the EPO website
- Claims 17, 18, 20 - 22, 24 - 31, 35, 36, 38 - 72, 74 - 76, 87, 88, 90 - 93, 96 - 103, 107, 109 and 111 are deemed to be abandoned due to non-payment of the claims fees (Rule 45(3) EPC).

# (54) COMPOSITIONS FOR ADMINISTRATION OF DIFFERENT DOSES OF RNA

(57) This disclosure relates to technologies for preventing or treating coronavirus infection. In particular, the present disclosure relates to methods and agents for vaccination against coronavirus infection and inducing effective coronavirus antigen-specific immune responses such as antibody and/or T cell responses.

EP 4 238 577 A2

## Description

### Technical Field

The invention provides technologies relating to pharmaceutical RNA preparations. Among other things, the invention provides technologies for providing a pharmaceutical RNA preparation which is suitable for the administration of different doses of RNA, pharmaceutical RNA preparations and systems which are suitable for the administration of different doses of RNA, and methods of using a pharmaceutical RNA preparation for the administration of different doses of RNA. The invention further provides particular pharmaceutical RNA preparations, including certain administration forms (e.g., unit dose forms, ready to use forms, shipping forms, etc).

### Background

Apart from their well-known ability to encode biologically active proteins, nucleic acids such as DNA and RNA have other remarkable properties that make them attractive therapeutic agents. Nucleic acid-based therapeutics are easy to manufacture and relatively inexpensive. Generally, DNA is more stable than RNA, but has some potential safety risks such as the induction of anti-DNA antibodies and the integration of the transgene into the host genome. The use of RNA to deliver foreign genetic information into target cells offers an attractive alternative to DNA. The advantages of RNA include transient expression and non-transforming character. RNA does not require nucleus infiltration for expression and moreover cannot integrate into the host genome, thereby eliminating the risk of oncogenesis.

mRNAs encoding antigens can be used as vaccines to evoke protective immunity against infectious diseases. The COVID-19 pandemic has showcased the utility and advantages of RNA technology for vaccination, as out of all COVID-19 vaccines under development, the first two to have received emergency use authorization by the FDA were RNA-based. The biotechnology response to the COVID-19 pandemic has highlighted the speed and flexibility of mRNA vaccines, and reveals mRNA therapeutics to be a powerful tool to address epidemic outbreaks caused by newly emerging viruses. The relative simplicity of the development process and flexibility of the manufacturing platform can markedly accelerate clinical development. As such, mRNA-based vaccine technology has attracted a lot of attention during the COVID-19 pandemic.

The first authorized vaccine was developed by BioNTech in collaboration with Pfizer. The RNA of this vaccine, BNT162b2, encodes full length spike protein modified by two proline mutations to stabilize the prefusion conformation. The RNA incorporates 1-methyl-pseudouridine, which dampens innate immune sensing and increases mRNA translation in *vivo* and is formulated in lipid nanoparticles (LNP). BNT162b2 is administered to adults intramuscularly (IM) in two 30 μg doses given 21 days apart.

Results from a Phase 2/3 trial showed a favorable safety profile and robust neutralizing antibody responses in children 5 to 11 years of age using a two-dose regimen of 10 μg administered 21 days apart, a smaller dose than the 30 μg dose used for people 12 and older. The antibody responses in the participants given 10 μg doses were comparable to those recorded in a previous Pfizer-BioNTech study in people 16 to 25 years of age immunized with 30 μg doses. The 10 μg dose was carefully selected as the preferred dose for safety, tolerability and immunogenicity in children 5 to 11 years of age.

The situation thus may arise that RNA therapeutics such as RNA vaccines are to be administered in different doses, e.g., to different groups of patients such as patients of different ages. Administration of smaller doses could be sufficient for administration to younger patients such as children compared to adults.

It is in principle possible to produce different RNA formulations which are each adapted for administration of different dose amounts of the RNA. However, this complicates the manufacturing process in particular because different mixtures have to be prepared. Furthermore, providing an RNA formulation with a high concentration of RNA may require too much of a dilution for lower doses, while providing an RNA formulation with a low concentration of RNA may require administration of high volumes for higher doses and may not provide for the required stability of the RNA.

It is therefore desirable to provide pharmaceutical RNA preparations which are suitable for administration of RNA in different dose amounts without having to prepare different mixtures. Such pharmaceutical RNA preparations, their manufacture and use by clinicians should also meet existing requirements of pharmaceuticals with regard to the production, filling, stability, handling and administration.

### Summary

It has been observed according to the invention that it is possible to provide a single pharmaceutical RNA preparation with an RNA concentration (e.g., within a certain RNA concentration range as described herein) which is suitable for the administration of different doses (dose amounts) of the RNA and which meets existing requirements of pharmaceuticals with regard to production, filling, stability, handling and administration. In particular, it has been observed

according to the invention that it is possible to provide such a single pharmaceutical RNA preparation which allows the administration of the different doses of the RNA while administration volumes are in a range suitable for administration of RNA pharmaceuticals and, which provides the required storage stability of the RNA. In some embodiments, such single pharmaceutical RNA preparations may have one or more desirable stability characteristics, for example, in some embodiments showing stability through freeze/thaw cycles. Furthermore, such single pharmaceutical RNA preparations may fulfil requirements with regard to pharmaceutical filling processes and can be provided in volumes that allow administration of a suitable number of doses from a single container such as a vial. The invention provides a method for providing a pharmaceutical RNA preparation for the administration of different doses of the RNA comprising the steps:

(i) determining different doses in which the RNA is to be administered,
(ii) determining a concentration of the RNA in the pharmaceutical RNA preparation which allows the administration of the different doses of the RNA with suitable administration volumes and, optionally, suitable dilution of the pharmaceutical RNA preparation, and
(iii) determining a suitable formulation for the pharmaceutical RNA preparation to ensure a desired storage stability of the RNA in the pharmaceutical RNA preparation at the determined concentration.

In some embodiments, the lowest dose of the RNA and the largest dose of the RNA differ by a factor of 2 or more and the smallest administration volume for one dose and the largest administration volume for another dose differ by a factor of 2 or less.
In some embodiments, the lowest dose of the RNA and the largest dose of the RNA differ by a factor of 2 or more and suitable administration volumes are 100 to 400 µl.
In some embodiments, the different doses comprise at least two doses.
In some embodiments, the different doses comprise at least three doses.
In some embodiments, the different doses of the RNA are to be administered to different age groups.
In some embodiments, the lowest dose of the RNA and the largest dose of the RNA differ by a factor of 2 or more.
In some embodiments, the lowest dose of the RNA and the largest dose of the RNA differ by a factor of 3 or more.
In some embodiments, the lowest dose of the RNA and the largest dose of the RNA differ by a factor of 5 or more.
In some embodiments, the lowest dose of the RNA and the largest dose of the RNA differ by a factor of 10 or more.
In some embodiments, the smallest administration volume for one dose and the largest administration volume for another dose differ by a factor of 2 or less.
In some embodiments, the smallest administration volume for one dose and the largest administration volume for another dose differ by a factor of 1.5 or less.
In some embodiments, suitable administration volumes are 100 to 400 µl.
In some embodiments, suitable administration volumes are 150 to 350 µl.
In some embodiments, suitable administration volumes are 200 to 300 µl.
In some embodiments, optionally a dilution of the pharmaceutical RNA preparation by a factor of 1:10 or less takes place for at least one dose.
In some embodiments, optionally a dilution of the pharmaceutical RNA preparation by a factor of 1:8 or less takes place for at least one dose.
In some embodiments, optionally a dilution of the pharmaceutical RNA preparation by a factor of 1:6 or less takes place for at least one dose.
In some embodiments, at least one dose of the RNA is to be administered without diluting the RNA.
In some embodiments, the at least one dose of the RNA which is to be administered without diluting the RNA comprises the largest dose of the RNA.
In some embodiments, at least one dose of the RNA is to be administered with diluting the RNA.
In some embodiments, the at least one dose of the RNA which is to be administered with diluting the RNA comprises the lowest dose of the RNA.
In some embodiments, the pharmaceutical RNA preparation is provided as multi-dose preparations, wherein each multi-dose preparation allows multiple administrations of a dose of the RNA.
In some embodiments, the multi-dose preparations have filling volumes that are suitable for pharmaceutical manufacture.
In some embodiments, a multi-dose preparation allows the administration of a desired number of doses of the RNA.
In some embodiments, the desired number of doses of the RNA is 5 or more, e.g., 5-20, 5-15, or 5-10.
In some embodiments, a multi-dose preparation allows an optional dilution of the pharmaceutical RNA preparation.
In some embodiments, the pharmaceutical RNA preparation is provided in vials.
In some embodiments, vials which are used to administer different doses of the RNA are labelled differently.

In some embodiments, the different labels comprise a different color of the lid.

In some embodiments, the pharmaceutical RNA preparation for administration of different doses of the RNA has a uniform RNA concentration.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof.

In some embodiments, the pharmaceutical RNA preparation is for inducing an immune response against SARS-CoV-2.

In some embodiments, the pharmaceutical RNA preparation is for vaccination against SARS-CoV-2.

The invention also provides a use of a pharmaceutical RNA preparation for the administration of different doses of the RNA (or: a method for the administration of different doses of RNA from a pharmaceutical RNA preparation), comprising the steps:

(i) providing the pharmaceutical RNA preparation,
(ii) administering different doses of the RNA, wherein said different doses of the RNA are administered by administering the same and/or different volumes of the optionally diluted pharmaceutical RNA preparation, wherein

the concentration of the RNA in the pharmaceutical RNA preparation is selected such that it allows the administration of the different doses of the RNA with suitable administration volumes and, optionally, suitable dilution of the pharmaceutical RNA preparation, and
the pharmaceutical RNA preparation is formulated to ensure a desired stability of the RNA in the pharmaceutical RNA preparation.

In some embodiments, the lowest dose of the RNA and the largest dose of the RNA differ by a factor of 2 or more and the smallest administration volume for one dose and the largest administration volume for another dose differ by a factor of 2 or less.

In some embodiments, the lowest dose of the RNA and the largest dose of the RNA differ by a factor of 2 or more and suitable administration volumes are 100 to 400 μl.

In some embodiments, the different doses comprise at least two doses.

In some embodiments, the different doses comprise at least three doses.

In some embodiments, the different doses of the RNA are administered to different age groups.

In some embodiments, the lowest dose of the RNA and the largest dose of the RNA differ by a factor of 2 or more.

In some embodiments, the lowest dose of the RNA and the largest dose of the RNA differ by a factor of 3 or more.

In some embodiments, the lowest dose of the RNA and the largest dose of the RNA differ by a factor of 5 or more.

In some embodiments, the lowest dose of the RNA and the largest dose of the RNA differ by a factor of 10 or more.

In some embodiments, the smallest administration volume for one dose and the largest administration volume for another dose differ by a factor of 2 or less.

In some embodiments, the smallest administration volume for one dose and the largest administration volume for another dose differ by a factor of 1.5 or less.

In some embodiments, suitable administration volumes are 100 to 400 μl.

In some embodiments, suitable administration volumes are 150 to 350 μl.

In some embodiments, suitable administration volumes are 200 to 300 μl.

In some embodiments, optionally a dilution of the pharmaceutical RNA preparation by a factor of 1:10 or less takes place for at least one dose.

In some embodiments, optionally a dilution of the pharmaceutical RNA preparation by a factor of 1:8 or less takes place for at least one dose.

In some embodiments, optionally a dilution of the pharmaceutical RNA preparation by a factor of 1:6 or less takes place for at least one dose.

In some embodiments, at least one dose of the RNA is administered without diluting the RNA. In some embodiments, the at least one dose of the RNA which is administered without diluting the RNA comprises the largest dose of the RNA.

In some embodiments, at least one dose of the RNA is administered with diluting the RNA.

In some embodiments, the at least one dose of the RNA which is administered with diluting the RNA comprises the lowest dose of the RNA.

In some embodiments, the pharmaceutical RNA preparation is provided as multi-dose preparations, wherein

each multi-dose preparation allows multiple administrations of a dose of the RNA.

In some embodiments, the multi-dose preparations have filling volumes that are suitable for pharmaceutical manufacture.

In some embodiments, a multi-dose preparation allows the administration of a desired number of doses of the RNA.

In some embodiments, the desired number of doses of the RNA is 5 or more, e.g., 5-20, 5-15, or 5-10.

In some embodiments, a multi-dose preparation allows an optional dilution of the pharmaceutical RNA preparation.

In some embodiments, the pharmaceutical RNA preparation is provided in vials.

In some embodiments, vials which are used to administer different doses of the RNA are labelled differently.

In some embodiments, the different labels comprises a different color of the lid.

In some embodiments, the pharmaceutical RNA preparation for administration of different doses of the RNA has a uniform RNA concentration.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof.

In some embodiments, the pharmaceutical RNA preparation is for inducing an immune response against SARS-CoV-2.

In some embodiments, the pharmaceutical RNA preparation is for vaccination against SARS-CoV-2.

In some embodiments, the different doses comprise doses of about 10 μg and about 30 μg. In some embodiments, the different doses comprise doses of about 3μg, about 10 μg and about 30 μg. In some embodiments, the different doses comprise doses of about 3 μg, about 10 μg, about 15 μg, about 25 μg, about 30 μg, about 50 μg and about 60 μg.

In some embodiments, the concentration of the RNA in the pharmaceutical RNA preparation is about 0.1 mg/ml. In some embodiments, the concentration of the RNA in the pharmaceutical RNA preparation is about 30 μg/ml to about 100 μg/ml. In some embodiments, the concentration of the RNA in the pharmaceutical RNA preparation is about 50 μg/ml to about 100 μg/ml.

In some embodiments, the administration volumes are between about 200 μl and about 600 μl.

In some embodiments, the administration volumes are between about 200 μl and about 300 μl.

In some embodiments, the RNA in pharmaceutical RNA preparation is formulated in about 10 mM Tris buffer, about 10% sucrose.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 10 μg is administered by diluting the pharmaceutical RNA preparation about 1:1 and administering about 200 μl of diluted pharmaceutical RNA preparation.

In some embodiments, a third dose of the RNA of about 3 μg is administered by diluting the pharmaceutical RNA preparation about 1:5.75 and administering about 200 μl of diluted pharmaceutical RNA preparation.

The invention further provides a pharmaceutical RNA preparation for the administration of different doses of the RNA, wherein the concentration of the RNA in the pharmaceutical RNA preparation is selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation.

Embodiments of the pharmaceutical RNA preparation are as described above.

The invention further provides a pharmaceutical RNA preparation for the administration of different doses of the RNA, wherein the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose. Embodiments of the pharmaceutical RNA preparation are as described above.

The invention further provides a system for the administration of different doses of RNA, wherein the system comprises a plurality of vials for administering a plurality of different doses of the RNA, wherein each of the plurality of vials comprises optionally different volumes of a pharmaceutical RNA preparation, wherein the concentration of the RNA in the pharmaceutical RNA preparation and the volumes of the pharmaceutical RNA preparation in the vials are selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation.
Embodiments of the system are as described above.
The invention further provides a system for the administration of different doses of RNA, wherein the system comprises a plurality of vials for administering a plurality of different doses of the RNA, wherein each of the plurality of vials comprises a pharmaceutical RNA preparation, wherein

the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose, and wherein
a first of the plurality of vials is for administering a first dose of the RNA of about 30 μg by administering about 300 μl of undiluted pharmaceutical RNA preparation, and a second of the plurality of vials is for administering a second dose of the RNA of about 10 μg by diluting the pharmaceutical RNA preparation about 1:1 and administering about 200 μl of diluted pharmaceutical RNA preparation.

In some embodiments:

the volume of the pharmaceutical RNA preparation in the first of the plurality of vials is about 2.25 ml for administering a total of at least about 6 doses, and
the volume of the pharmaceutical RNA preparation in the second of the plurality of vials is about 1.3 ml for administering a total of at least about 10 doses.

In some embodiments, a third of the plurality of vials is for administering a third dose of the RNA of about 3 μg by diluting the pharmaceutical RNA preparation about 1:5.75 and administering about 200 μl of diluted pharmaceutical RNA preparation.
In some embodiments, the volume of the pharmaceutical RNA preparation in the third of the plurality of vials is about 0.4 ml for administering a total of at least about 10 doses.
In some embodiments, vials for administering different doses of the RNA are labelled differently.
In some embodiments, the different labels comprise a different color of the lid.
Additional embodiments of the system are as described above.
In some embodiments, the invention provides certain dosage forms (e.g., forms for administration), which may, for example, contain a particular buffer and/or a particular dose of an RNA; in some embodiments, the invention provides sets of such dosage forms, e.g., having buffer and RNA concentrations related to one another e.g., by a factor such as can be achieved, for example, by dilution.
In some embodiments, provided pharmaceutical RNA preparations include a Tris buffer (e.g., without sodium chloride and/or potassium chloride ions). In some embodiments, provided pharmaceutical RNA preparations include sucrose. In some embodiments, provided pharmaceutical RNA preparations do not include a preservative.
In some embodiments, an appropriate buffer *(e.g.,* a Tris buffer as described herein) is introduced into provided pharmaceutical RNA preparations during a filtration step, e.g., a Tangential Flow Filtration (TFF) step; in some embodiments, such introduction is prior to one or more steps of sterile filtration, aseptic filling, capping/crimping, labelling, freezing, etc.
In some embodiments, provided pharmaceutical RNA preparations, or sets thereof, are disposed in containers (*e.g.,* vials); in some such embodiments, such containers are amenable to entry with a syringe (*e.g.,* with a needle attached to a syringe), for example through a membrane. In some embodiments, a container is a glass vial (e.g., a Type I borosiligate glass or aluminosilicate glass, for example sealed with a rubber stopper such as a bromobutyl rubber stopper (*e.g.,* a 13 mm bromobutyl stopper) and/or an aluminium seal with a flip-off plastic cap. In some embodiments, containers may be present in packs, for example containing about 5 to about 500 vials, for example about 5 to about 200 vials, for example about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 10, 175, 180, 185, 190, 195, 200, or more vials, for example about 10 vials or for example about 195 vials. In some embodiments, a container is a syringe (*e.g.,* a chamber and/or a needle attached thereto).
In some embodiments, provided pharmaceutical RNA preparations have been stored in a container in which

they are disposed, *e.g.,* for a particular period of time and/or under particular conditions, in some embodiments while maintaining certain stability parameters (*e.g.,* one or more of colloidal stability, polydispersity, zeta potential, RNA integrity, etc). In some embodiments, such storage conditions may include temperatures within a range of about -20 °C and about 2-8 °C. In some embodiments, provided pharmaceutical RNA preparations show greater stability after storage for a given time (*e.g.,* after storage for a 1, 2, 3, or 4 weeks, or 1, 2, 3, 4, 5, 6, 7, 8, 9, 10, 11, 12 months, or 1 year or more) and/or under a given set of conditions that is observed for otherwise comparable (*e.g.,* at the same concentration and/or temperature, and/or in the same state - *e.g.,* frozen or liquid) preparations of the same RNA/LNP composition in a different buffer (*e.g.,* a PBS buffer).

In some embodiments, provided pharmaceutical RNA preparations are manufactured in commercial batch size within a range of about 700 - about 1600 L of finished product solution, which may in some embodiments correspond to approximately 300,000 - 700,000 vials (*e.g.,* at 2.25 mL fill volume).

In some embodiments, vials containing provided pharmaceutical RNA preparations (e.g., single-dose vials or multi-dose vials) may be transported in a frozen state, e.g., in some embodiments at ultra-cold conditions in thermal containers with dry ice, or in some embodiments at -25°C to -15°C (-13°F to 5°F). In some embodiments, vials containing provided pharmaceutical RNA preparations (e.g., single-dose vials or multi-dose vials) may be transported at -90°C to -60°C (-130°F to -76°F) or at a refrigerated temperature, e.g., 2°C to 8°C (35°F to 46°F).

In some embodiments, frozen pharmaceutical RNA preparations described herein may be stored at a refrigerated temperature (e.g., 2ºC to 8ºC), for example, to thaw, and stored for an extended period of time, e.g., at least 4 weeks, at least 5 weeks, at least 6 weeks, at least 7 weeks, at least 8 weeks, at least 9 weeks, at least 10 weeks or longer. In some embodiments, thawed pharmaceutical RNA preparations described herein can be stored for up to 10 weeks. Alternatively, in some embodiments, frozen pharmaceutical RNA preparations described herein may be stored in an ultra-low temperature freezer at -90ºC to -60ºC (-130ºF to -76ºF). In some embodiments, it may be desirable to refrain from storing frozen pharmaceutical RNA preparations described herein at -25°C to -15°C (-13°F to 5°F). Once frozen pharmaceutical RNA preparations described herein are thawed, they should not be refrozen. In some embodiments, frozen pharmaceutical RNA preparations described herein may be thawed at room temperature (e.g., up to 25ºC (77ºF)], for example in some embodiments for 30 minutes.

In some embodiments, vials containing provided pharmaceutical RNA preparations (e.g., single-dose vials or multi-dose vials) may be transported at a refrigerated temperature (e.g., at 2°C to 8°C). In some such embodiments, it may be desirable to store such vials at a refrigerated temperature (e.g., at 2°C to 8°C) upon their arrival.

In some embodiments, provided pharmaceutical RNA preparations described herein may be stored at room temperature (e.g., 8°C to 25°C (46°F to 77°F)) for a total of 12 hours prior to dilution or use. In some embodiments, after dilution or first use, such pharmaceutical RNA preparations described herein can be maintained between a refrigerated temperature and room temperature (e.g., between 2ºC to 25°C (35°F to 77°F)). In some embodiments, provided pharmaceutical RNA preparations after dilution or first use (e.g., in the case of multi-dose vials) can be maintained at such temperatures for about 12 hours before they are discarded. In some embodiments, diluted pharmaceutical RNA preparations as described herein can be maintained after first use at such temperatures for about 6 hours before they are discarded. In some embodiments, one or more doses of an RNA composition described herein is administered after storage and/or transportation under conditions as described herein.

In some embodiments, the RNA described herein is single-stranded RNA that may be translated into the respective protein upon entering cells, e.g., cells of a recipient. In addition to wildtype or codon-optimized sequences encoding an amino acid sequence comprising the amino acid sequence of a peptide or polypeptide having biological activity, e.g., a pharmaceutically active peptide or polypeptide such as antigen sequence (peptide or polypeptide comprising an epitope), the RNA may contain one or more structural elements optimized for maximal efficacy of the RNA with respect to stability and translational efficiency (5' cap, 5' UTR, 3' UTR, poly(A)-tail). In some embodiments, the RNA contains all of these elements. In some embodiments, beta-S-ARCA(D1) ($m_2^{7,2'-O}$GppSpG) or $m_2^{7,3'-O}$Gppp($m_1^{2'-O}$)ApG may be utilized as specific capping structure at the 5'-end of the RNA drug substances. As 5'-UTR sequence, in some embodiments, the 5'-UTR sequence of the human alpha-globin mRNA, optionally with an optimized 'Kozak sequence' to increase translational efficiency may be used. As 3'-UTR sequence, in some embodiments, a combination of two sequence elements (FI element) derived from the "amino terminal enhancer of split" (AES) mRNA (called F) and the mitochondrial encoded 12S ribosomal RNA (called I) placed between the coding sequence and the poly(A)-tail to assure higher maximum protein levels and prolonged persistence of the mRNA may be used. These were identified by an *ex vivo* selection process for sequences that confer RNA stability and augment total protein expression (see WO 2017/060314, herein incorporated by reference). Alternatively, the 3'-UTR may be two re-iterated 3'-UTRs of the human beta-globin mRNA. Additionally or alternatively, in some embodiments, a poly(A)-tail may comprise a length of at least 100 adenosine residues (including, e.g., at least 110 adenosine residues, at least 120 adenosine residues, 130 adenosine residues, or longer). In some embodiments, a poly(A)-tail may comprise a length of about 100 to about 150 adenosine residues. In some embodiments a poly(A)-tail may comprise an interrupted poly(A)-tail. For example, in some such embodiments, a poly(A)-tail measuring 110 nucleotides in length, consisting of a stretch of 30 adenosine residues,

followed by a 10 nucleotide linker sequence (of random nucleotides) and another 70 adenosine residues may be used. This poly(A)-tail sequence was designed to enhance RNA stability and translational efficiency.

The RNA described herein may encode an amino acid sequence comprising the amino acid sequence of a peptide or polypeptide having biological activity, e.g., a pharmaceutically active peptide or polypeptide such as antigen sequence. The encoded amino acid sequence may comprise amino acid sequences other than the amino acid sequence of a peptide or polypeptide having biological activity. Such other amino acid sequences may support the function or activity of the peptide or polypeptide having biological activity. In some embodiments, such other amino acid sequences comprise an amino acid sequence enhancing antigen processing and/or presentation. Alternatively, or additionally, such other amino acid sequences comprise an amino acid sequence which breaks immunological tolerance.

## Brief description of the Figures

**Figure 1A** is a table showing vials containing exemplary compositions as described herein for use in administration of different doses of RNA, e.g., to different target populations.

**Figure 1B** is a table showing compositions of exemplary stock buffers before and after dilutions. In some embodiments, such compositions can be useful for administration of different doses of RNA.

**Figure 2** is a table showing an exemplary stock composition, which can be filled into vials at different volumes (e.g., as described herein) for administration of RNA at different doses (e.g., as described herein)

**Figure 3** is a schematic showing various exemplary vaccination regimens.

**Figure 4** shows 50% neutralization titers of sera collected 7 days after a fourth dose of BNT162b2, an Omicron-specific booster (specifically, a BA.1-specific booster), or a bivalent vaccine. Subjects who were previously administered two doses of BNT162b2 (30 ug), and a third (booster) dose of BNT162b2 (30 ug) received (i) a 30 ug dose of BNT162b2 (encoding a SARS-CoV-2 S protein from a Wuhan strain), (ii) a 60 ug dose of BNT162b2, (iii) a 30 ug dose of RNA encoding a SARS-CoV-2 S protein having mutations characteristic of an Omicron variant (e.g., as described herein (referred to herein as "Omicron-specific RNA vaccine")), (iii) a 60 ug dose of RNA encoding a SARS-CoV-2 S protein having mutations characteristic of an Omicron variant, (iv) a 30 ug dose of a bivalent vaccine, comprising 15 ug of BNT162b2 and 15 ug of RNA encoding a SARS-CoV-2 S protein comprising mutations characteristic of an Omicron variant, or (v) a 60 ug dose of a bivalent vaccine, comprising 30 ug of BNT162b2 and 30 ug of RNA encoding a SARS-CoV-2 S protein comprising mutations characteristic of an Omicron variant. Geometric mean ratio (GMR) of titers in serum from subjects were collected 7 days after administration of a 4th dose. "b2" refers to sera from subjects administered a Wuhan-specific RNA vaccine as a 4th dose of BNT162b2. "OMI" refers to sera from subjects administered an Omicron-specific 4th dose. "Bivalent" refers to sera from subjects administered a composition comprising BNT162b2 and an RNA encoding a SARS-CoV-2 S protein comprising mutations that are characteristic of an Omicron variant (specifically, a BA.1-specific variant) as a 4th dose. Also shown is the fold-rise in titer from before administration of a 4th dose to 7 days after administration of a 4th dose (*Fold-Rise). "FFRNT" refers to fluorescent focus reduction neutralization test. Neutralization data was obtained using an FFRNT assay, with a viral particle containing a SARS-CoV-2 S protein having mutations characteristic of the variant indicated in the figures. LLOQ refers to Lower Limit of Quantification and ULOQ refers to Upper Limit of Quantification. (A) Comparison of titers of neutralizing antibodies against a SARS-CoV-2 pseudovirus comprising a SARS-CoV-2 S protein having mutations characteristics of an Omicron variant (specifically, a BA.1 variant). Sera from subjects previously or currently infected with SARS-CoV-2 excluded. (B) Comparison of titers of neutralizing antibodies against a SARS-CoV-2 pseudovirus comprising a SARS-CoV-2 S protein having mutations characteristics of an Omicron variant (specifically, a BA.1 variant) in sera from a population that includes subjects previously or currently infected with SARS-CoV-2 (e.g., as determined by an antibody test or a PCR assay respectively). (C) Comparison of titers of neutralizing antibodies against a SARS-CoV-2 pseudovirus comprising a SARS-CoV-2 S protein of a Wuhan strain. Sera from subjects previously or currently infected with SARS-CoV-2 excluded. (D) Comparison of titers of neutralizing antibodies against a SARS-CoV-2 pseudovirus comprising a SARS-CoV-2 S protein of a Wuhan strain, in sera from a population that includes individuals previously or currently infected with SARS-CoV-2. (E) Comparison of titers of neutralizing antibodies against a SARS-CoV-2 pseudovirus comprising a SARS-CoV-2 S protein having mutations characteristics of a Delta variant. Sera from subjects previously or currently infected with SARS-CoV-2 excluded. (F) Comparison of titers of neutralization antibodies against a SARS-CoV-2 pseudovirus comprising a SARS-CoV-2 S protein having mutations characteristic of a Delta variant, in sera from a population including subjects previously or currently infected with SARS-CoV-2. (G) Geometric mean rise (GMR) of neutralization antibodies observed in subjects administered 60 ug of BNT162b2, 30 ug of RNA encoding a SARS-CoV-2 S protein having mutations characteristic of an Omicron variant, (specifically, a BA.1 variant; OMI 30 ug), 60 ug of RNA encoding a SARS-CoV-2 S protein having mutations characteristic of an Omicron variant (specifically, a BA.1

variant; OMI 60 ug), 30 ug of a bivalent vaccine comprising 15 ug of BNT162b2 and 15 ug of RNA encoding a SARS-CoV-2 S protein having mutations characteristic of an Omicron variant (specifically, a BA.1 variant; Bivalent 30 ug), or 60 ug of a bivalent vaccine comprising 30 ug of BNT162b2 and 30 ug of RNA encoding a SARS-CoV-2 S protein having mutations characteristic of an Omicron variant (specifically, a BA.1 variant; Bivalent 60 ug), as compared to subjects administered 30 ug of BNT162b2 as a 4th dose. Results are shown both for a population pool that excludes subjects previously or currently infected with SARS-CoV-2 and a population pool that includes these subjects.

**Figure 5** shows reactogenicity of certain exemplary RNA (formulated in LNP) at a given dose: subjects administered a 60 ug dose of RNA encoding a SARS-CoV-2 S protein are more likely to exhibit a higher injection site pain and exhibit similar systemic reactions as subjects administered a 30 ug dose of RNA. Subjects were administered 30 ug or 60 ug of RNA encoding a SARS-CoV-2 S protein from a Wuhan strain (BNT162b2, corresponding to groups G1 and G2, respectively), 30 ug or 60 ug of RNA encoding a SARS-CoV-2 S protein having mutations characteristic of an Omicron variant (specifically, a BA.1 variant; BNT162b2 OMI, corresponding to groups G3 and G4, respectively), 30 ug of a bivalent vaccine comprising 15 ug of RNA encoding a SARS-CoV-2 S protein from a Wuhan strain and 15 ug of RNA encoding a SARS-CoV-2 S protein having mutations characteristic of an Omicron variant (specifically, a BA.1 variant; BNT162B2 (15 ug) + BNT162b2 OMI (15 ug), corresponding to group G5), or 60 ug of a bivalent vaccine comprising 30 ug of RNA encoding a SARS-CoV-2 S protein from a Wuhan strain and 30 ug of RNA encoding a SARS-CoV-2 S protein having mutations characteristic of an Omicron variant (specifically, a BA.1 variant; BNT162b2 (30 ug) + BNT162b2 OMI (30 ug), corresponding to group G6). (A) Local reactions, including redness, swelling, and pain at the injection site, observed within 7 days of injection. Injection site pain was found to be increased in subjects administered 60 ug of RNA encoding a SARS-CoV-2 S protein comprising mutations characteristic an Omicron variant (specifically, a BA.1 variant) or a bivalent vaccine, as compared to other doses tested. (B) Systemic reactions, including fever, fatigue, headache, chills, vomiting, diarrhea, muscle pain, joint pain, and use of medication, observed within 7 days of injection. Systemic reactions through 7 days were observed to be broadly similar across different groups. Fatigue was found to trend higher after administration of 60 ug doses, as compared to 30 ug doses.

## Description of Certain Sequences

The following table provides a listing of certain sequences referenced herein.

| TABLE 1: DESCRIPTION OF CERTAIN SEQUENCES | | |
|---|---|---|
| SEQ ID NO: | Description | SEQUENCE |
| Sec/MITD | | |
| 1 | Sec (amino acid) | MRVMAPRTLILLLSGALALTETWAGS |
| 2 | MITD (amino acid) | IVGIVAGLAVLAVVVIGAVVATVMCRRKSSGGKGGSYSQAASSDSAQGSDVSLTA |
| P2P16 | | |
| 3 | P2P16 (amino acid) | KKQYIKANSKFIGITELKKLGGGKRGGGKKMTNSVDDALINSTKIYSYFPSVISKVNQGAQGKKL |
| GS Linker | | |
| 4 | GS Linker 1 | GGSGGGGSGG |
| 5 | GS Linker 2 | GSSGGGGSPGGGSS |
| 5'-UTR (hAg-Kozak) | | |
| 6 | 5'-UTR | AACUAGUAUUCUUCUGGUCCCCACAGACUCAGAGAGAACCCGCCACC |
| 3'-UTR (FI element) | | |
| 7 | 3'-UTR | CUGGUACUGCAUGCACGCAAUGCUAGCUGCCCCUUUCCCGUCCUGGGUACCCCGAGUCUCCCCCGACCUCGGGU<br>CCCAGGUAUGCUCCCACCUCCACCUGCCCCACUCACCACCUCUGCUAGUUCCAGACACCUCCCAAGCACGCAGCAA<br>UGCAGCUCAAAACGCUUAGCCUAGCCACACCCCCACGGGAAACAGCAGUGAUUAACCUUUAGCAAUAAACGAA<br>AGUUUAACUAAGCUAUACUAACCCCAGGGUUGGUCAAUUUCGUGCCAGCCACACC |
| A30L70 | | |
| 8 | A30L70 | AAAAAAAAAAAAAAAAAAAAAAAAAAAAAAGCAUAUGACUAAAAAAAAAAAAAAAAAAAAAAAAAAAAAAAAA<br>AAAAAAAAAAAAAAAAAAAAAAAAAAAAAAAAAAAAA |
| Helper epitopes | | |
| 9 | P2 | QYIKANSKFIGITEL |
| 10 | P16 | MTNSVDDALINSTKIYSYFPSVISKVNQGAQG |

(continued)

| SARS-CoV-2 S protein sequences | | |
|---|---|---|
| 11 | S protein | MFVFLVLLPLVSSQCVNLTTRTQLPPAYTNSFTRGVYYPDKVFRSSVLHSTQDLFLPFFSNVTWFHAIHVSGTNGTKRFDN<br>PVLPFNDGVYFASTEKSNIIRGWIFGTTLDSKTQSLLIVNNATNVVIKVCEFQFCNDPFLGVYYHKNNKSWMESEFRVYSS<br>ANNCTFEYVSQPFLMDLEGKQGNFKNLREFVFKNIDGYFKIYSKHTPINLVRDLPQGFSALEPLVDLPIGINITRFQTLLALH<br>RSYLTPGDSSSGWTAGAAAYYVGYLQPRTFLLKYNENGTITDAVDCALDPLSETKCTLKSFTVEKGIYQTSNFRVQPTESIV<br>RFPNITNLCPFGEVFNATRFASVYAWNRKRISNCVADYSVLYNSASFSTFKCYGVSPTKLNDLCFTNVYADSFVIRGDEVR<br>QIAPGQTGKIADYNYKLPDDFTGCVIAWNSNNLDSKVGGNYNYLYRLFRKSNLKPFERDISTEIYQAGSTPCNGVEGFNC<br>YFPLQSYGFQPTNGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQDVNCTEVPVAIHADQLTPTWRVYSTGSNVFQTRAG<br>CLIGAEHVNNSYECDIPIGAGICASYQTQTNSPRRARSVASQSIIAYTMSLGAENSVAYSNNSIAIPTNFTISVTTEILPVSMT<br>KTSVDCTMYICGDSTECSNLLLQYGSFCTQLNRALTGIAVEQDKNTQEVFAQVKQIYKTPPIKDFGGFNFSQILPDPSKPSK<br>RSFIEDLLFNKVTLADAGFIKQYGDCLGDIAARDLICAQKFNGLTVLPPLLTDEMIAQYTSALLAGTITSGWTFGAGAALQI<br>PFAMQMAYRFNGIGVTQNVLYENQKLIANQFNSAIGKIQDSLSSTASALGKLQDVVNQNAQALNTLVKQLSSNFGAISS<br>VLNDILSRLDKVEAEVQIDRLITGRLQSLQTYVTQQLIRAAEIRASANLAATKMSECVLGQSKRVDFCGKGYHLMSFPQSA<br>PHGVVFLHVTYVPAQEKNFTTAPAICHDGKAHFPREGVFVSNGTHWFVTQRNFYEPQIITTDNTFVSGNCDVVIGIVNN<br>TVYDPLQPELDSFKEELDKYFKNHTSPDVDLGDISGINASVVNIQKEIDRLNEVAKNLNESLIDLQELGKYEQYIKWPWYI<br>WLGFIAGLIAIVMVTIMLCCMTSCCSCLKGCCSCGSCCKFDEDDSEPVLKGVKLHYT |
| 12 | S protein PP | MFVFLVLLPLVSSQCVNLTTRTQLPPAYTNSFTRGVYYPDKVFRSSVLHSTQDLFLPFFSNVTWFHAIHVSGTNGTKRFDN<br>PVLPFNDGVYFASTEKSNIIRGWIFGTTLDSKTQSLLIVNNATNVVIKVCEFQFCNDPFLGVYYHKNNKSWMESEFRVYSS |

(continued)

| | | |
|---|---|---|
| **SARS-CoV-2 S protein sequences** | | |
| | | ANNCTFEYVSQPFLMDLEGKQGNFKNLREFVFKNIDGYFKIYSKHTPINLVRDLPQGFSALEPLVDLPIGINITRFQTLLALHRSYLTPGDSSSGWTAGAAAYYVGYLQPRTFLLKYNENGTITDAVDCALDPLSETKCTLKSFTVEKGIYQTSNFRVQPTESIVRFPNITNLCPFGEVFNATRFASVYAWNRKRISNCVADYSVLYNSASFSTFKCYGVSPTKLNDLCFTNVYADSFVIRGDEVRQIAPGQTGKIADYNYKLPDDFTGCVIAWNSNNLDSKVGGNYNYLYRLFRKSNLKPFERDISTEIYQAGSTPCNGVEGFNCYFPLQSYGFQPTNGVGYQPYRVVVLSFELLHAPATVCGPKKSTNLVKNKCVNFNFNGLTGTGVLTESNKKFLPFQQFGRDIADTTDAVRDPQTLEILDITPCSFGGVSVITPGTNTSNQVAVLYQDVNCTEVPVAIHADQLTPTWRVYSTGSNVFQTRAGCLIGAEHVNNSYECDIPIGAGICASYQTQTNSPRRARSVASQSIIAYTMSLGAENSVAYSNNSIAIPTNFTISVTTEILPVSMTKTSVDCTMYICGDSTECSNLLLQYGSFCTQLNRALTGIAVEQDKNTQEVFAQVKQIYKTPPIKDFGGFNFSQILPDPSKPSKRSFIEDLLFNKVTLADAGFIKQYGDCLGDIAARDLICAQKFNGLTVLPPLLTDEMIAQYTSALLAGTITSGWTFGAGAALQIPFAMQMAYRFNGIGVTQNVLYENQKLIANQFNSAIGKIQDSLSSTASALGKLQDVVNQNAQALNTLVKQLSSNFGAISSVLNDILSRLDPPEAEVQIDRLITGRLQSLQTYVTQQLIRAAEIRASANLAATKMSECVLGQSKRVDFCGKGYHLMSFPQSAPHGVVFLHVTYVPAQEKNFTTAPAICHDGKAHFPREGVFVSNGTHWFVTQRNFYEPQIITTDNTFVSGNCDVVIGIVNNTVYDPLQPELDSFKEELDKYFKNHTSPDVDLGDISGINASVVNIQKEIDRLNEVAKNLNESLIDLQELGKYEQYIKWPWYIWLGFIAGLIAIVMVTIMLCCMTSCCSCLKGCCSCGSCCKFDEDDSEPVLKGVKLHYT |
| **Trimerization domain** | | |
| 13 | Foldon | GSGYIPEAPRDGQAYVRKDGEWVLLSTFLGRSLEVLFQGPG |
| **SARS-CoV-2 Vaccine constructs** | | |
| 14 | Construct 1 | MFVFLVLLPLVSSQCVVRFPNITNLCPFGEVFNATRFASVYAWNRKRISNCVADYSVLYNSASFSTFKCYGVSPTKLNDLCFTNVYADSFVIRGDEVRQIAPGQTGKIADYNYKLPDDFTGCVIAWNSNNLDSKVGGNYNYLYRLFRKSNLKPFERDISTEIYQAGSTPCNGVEGFNCYFPLQSYGFQPTNGVGYQPYRVVVLSFELLHAPATVCGPKGSPGSGSGSGYIPEAPRDGQAYVRKDGEWVLLSTFLGRSLEVLFQGPG |

(continued)

| SARS-CoV-2 Vaccine constructs | | |
|---|---|---|
| 15 | **Construct 3c** | **MFVFLVLLPLVSSQCVNLTVRFPNITNLCPFGEVFNATRFASVYAWNRKRISNCVADYSVLYNSASFSTFKCYGVSPTKLN**<br>**DLCFTNVYADSFVIRGDEVRQIAPGQTGKIADYNYKLPDDFTGCVIAWNSNNLDSKVGGNYNYLYRLFRKSNLKPFERDIS**<br>**TEIYQAGSTPCNGVEGFNCYFPLQSYGFQPTNGVGYQPYRVVVLSFELLHAPATVCGPKGSPGSGSGSGYIPEAPRDGQA**<br>**YVRKDGEWVLLSTFLGSGSGSEQYIKWPWYIWLGFIAGLIAIVMVTIMLCCMTSCCSCLKGCCSCGSCC** |
| 16 | **Construct 3d** | **MDWIWRILFLVGAATGAHSQMQVRFPNITNLCPFGEVFNATRFASVYAWNRKRISNCVADYSVLYNSASFSTFKCYGVS**<br>**PTKLNDLCFTNVYADSFVIRGDEVRQIAPGQTGKIADYNYKLPDDFTGCVIAWNSNNLDSKVGGNYNYLYRLFRKSNLKP**<br>**FERDISTEIYQAGSTPCNGVEGFNCYFPLQSYGFQPTNGVGYQPYRVVVLSFELLHAPATVCGPKGSPGSGSGSGYIPEAP**<br>**RDGQAYVRKDGEWVLLSTFLGSGSGSEQYIKWPWYIWLGFIAGLIAIVMVTIMLCCMTSCCSCLKGCCSCGSCC** |
| **SARS-CoV-2 Vaccine RNA constructs** | | |
| 17 | **Construct 2** | agaauaaacu aguauucuuc ugguccccac agacucagag agaacccgcc accauguucg 60<br>uguuccuggu gcugcugccu cuggugucca gccagugugu gaaccugacc accagaacac 120<br>agcugccucc agccuacacc aacagcuuua ccagaggcgu guacuacccc gacaaggugu 180<br>ucagauccag cgugcugcac ucuacccagg accuguuccu gccuuucuuc agcaacguga 240<br>ccugguucca cgccauccac guguccggca ccaauggcac caagagauuc gacaaccccg 300<br>ugcugcccuu caacgacggg guguacuuug ccagcaccga gaaguccaac aucaucagag 360 |

(continued)

| SARS-CoV-2 Vaccine RNA constructs | | |
|---|---|---|
| | | gcuggaucuu cggcaccaca cuggacagca agacccagag ccugcugauc gugaacaacg 420 |
| | | ccaccaacgu ggucaucaaa gugugcgagu uccaguucug caacgacccc uuccugggcg 480 |
| | | ucuacuacca caagaacaac aagagcugga uggaaagcga guuccgggug uacagcagcg 540 |
| | | ccaacaacug caccuucgag uacgugucc agccuuuccu gauggaccug gaaggcaagc 600 |
| | | agggcaacuu caagaaccug cgcgaguucg uguuuaagaa caucgacggc uacuucaaga 660 |
| | | ucuacagcaa gcacaccccu aucaaccucg ugcgggaucu gccucagggc uucucugcuc 720 |
| | | uggaaccccu gguggaucug cccaucggca ucaacaucac ccgguuucag acacugcugg 780 |
| | | cccugcacag aagcuaccug acaccuggcg auagcagcag cggauggaca gcuggugccg 840 |
| | | ccgcuuacua ugugggcuac cugcagccua gaaccuuccu gcugaaguac aacgagaacg 900 |
| | | gcaccaucac cgacgccgug gauugugcuc uggauccucu gagcgagaca aagugcaccc 960 |
| | | ugaaguccuu caccguggaa aagggcaucu accagaccag caacuuccgg gugcagccca 1020 |
| | | ccgaauccau cgugcgguuc cccaauauca ccaaucugug ccccuucggc gagguguuca 1080 |
| | | augccaccag auucgccucu guguacgccu ggaaccggaa gcggaucagc aauugcgugg 1140 |
| | | ccgacuacuc cgugcuguac aacuccgcca gcuucagcac cuucaagugc uacggcgugu 1200 |
| | | ccccuaccaa gcugaacgac cugugcuuca caaacgugua cgccgacagc uucgugaucc 1260 |
| | | ggggagauga agugcggcag auugccccug gacagacagg caagaucgcc gacuacaacu 1320 |
| | | acaagcugcc cgacgacuuc accggcugug ugauugccug gaacagcaac aaccuggacu 1380 |
| | | ccaaagucgg cggcaacuac aauuaccugu accggcuguu ccggaagucc aaucugaagc 1440 |
| | | ccuucgagcg ggacaucucc accgagaucu aucaggccgg cagcaccccu uguaacggcg 1500 |
| | | uggaaggcuu caacugcuac uucccacugc aguccuacgg cuuucagccc acaaauggcg 1560 |

(continued)

| SARS-CoV-2 Vaccine RNA constructs | | |
|---|---|---|
| | | ugggcuauca gcccuacaga gugguggugc ugagcuucga acugcugcau gccccugcca 1620<br>cagugugcgg cccuaagaaa agcaccaauc ucgugaagaa caaaugcgug aacuucaacu 1680<br>ucaacggccu gaccggcacc ggcgugcuga cagagagcaa caagaaguuc cugccauucc 1740<br>agcaguuugg ccgggauauc gccgauacca cagacgccgu uagagauccc cagacacugg 1800<br>aaauccugga caucaccccu ugcagcuucg gcggagugc ugugaucacc ccuggcacca 1860<br>acaccagcaa ucagguggca gugcuguacc aggacgugaa cuguaccgaa gugcccgugg 1920<br>ccauucacgc cgaucagcug acaccuacau ggcgggugua cuccaccggc agcaaugugu 1980<br>uucagaccag agccggcugu cugaucggag ccgagcacgu gaacaauagc uacgagugcg 2040<br>acaucccau cggcgcugga aucugcgcca gcuaccagac acagacaaac agcccucgga 2100<br>gagccagaag cguggccagc cagagcauca uugccuacac aaugucucug ggcgccgaga 2160<br>acagcguggc cuacuccaac aacucuaucg cuauccccac caacuucacc aucagcguga 2220<br>ccacagagau ccugccugug uccaugacca agaccagcgu ggacugcacc auguacaucu 2280<br>gcggcgauuc caccgagugc uccaaccugc ugcugcagua cggcagcuuc ugcacccagc 2340<br>ugaauagagc ccugacaggg aucgccgugg aacaggacaa gaacacccaa gagguguucg 2400<br>cccaagugaa gcagaucuac aagaccccuc cuaucaagga cuucggcggc uucaauuuca 2460<br>gccagauucu gcccgauccu agcaagccca gcaagcggag cuucaucgag gaccugcugu 2520<br>ucaacaaagu gacacuggcc gacgccggcu ucaucaagca guauggcgau ugucugggcg 2580<br>acauugccgc cagggaucug auuugcgccc agaaguuuaa cggacugaca gugcugccuc 2640<br>cucugcugac cgaugagaug aucgcccagu acacaucugc ccugcuggcc ggcacaauca 2700<br>caagcggcug gacauuugga gcaggcgccg cucugcagau ccccuuugcu augcagaugg 2760 |

(continued)

| SARS-CoV-2 Vaccine RNA constructs | | |
|---|---|---|
| | | ccuaccgguu caacggcauc ggagugaccc agaaugugcu guacgagaac cagaagcuga 2820<br>ucgccaacca guucaacagc gccaucggca agauccagga cagccugagc agcacagcaa 2880<br>gcgcccuggg aaagcugcag gacgugguca accagaaugc ccaggcacug aacacccugg 2940<br>ucaagcagcu guccuccaac uucggcgcca ucagcucugu gcugaacgau auccugagca 3000<br>gacuggaccc uccugaggcc gaggugcaga ucgacagacu gaucacaggc agacugcaga 3060<br>gccuccagac auacgugacc cagcagcuga ucagagccgc cgagauuaga gccucugcca 3120<br>aucuggccgc caccaagaug ucugagugug ugcugggcca gagcaagaga guggacuuuu 3180<br>gcggcaaggg cuaccaccug augagcuucc cucagucugc cccucacggc gugguguuuc 3240<br>ugcacgugac auaugugccc gcucaagaga agaauuucac caccgcucca gccaucugcc 3300<br>acgacggcaa agcccacuuu ccuagagaag gcguguucgu guccaacggc acccauuggu 3360<br>ucgugacaca gcggaacuuc uacgagcccc agaucaucac caccgacaac accuucgugu 3420<br>cuggcaacug cgacgucgug aucggcauug ugaacaauac cguguacgac ccucugcagc 3480<br>ccgagcugga cagcuucaaa gaggaacugg acaaguacuu uaagaaccac acaagccccg 3540<br>acguggaccu gggcgauauc agcggaauca augccagcgu cgugaacauc cagaaagaga 3600<br>ucgaccggcu gaacgaggug gccaagaauc ugaacgagag ccugaucgac cugcaagaac 3660<br>uggggaagua cgagcaguac aucaaguggc ccugguacau cuggcugggc uuuaucgccg 3720<br>gacugauugc caucgugaug gucacaauca ugcuguguug caugaccagc ugcuguagcu 3780<br>gccugaaggg cuguuguagc uguggcagcu gcugcaaguu cgacgaggac gauucugagc 3840<br>ccgugcugaa gggcgugaaa cugcacuaca caugaugacu cgagcuggua cugcaugcac 3900<br>gcaaugcuag cugccccuuu cccguccugg guaccccgag ucucccccga ccucgggucc 3960 |

(continued)

| SARS-CoV-2 Vaccine RNA constructs | | |
|---|---|---|
| | | cagguaugcu cccaccucca ccugccccac ucaccaccuc ugcuaguucc agacaccucc 4020<br>caagcacgca gcaaugcagc ucaaaacgcu uagccuagcc acacccccac gggaaacagc 4080<br>agugauuaac cuuuagcaau aaacgaaagu uuaacuaagc uauacuaacc ccaggguugg 4140<br>ucaauuucgu gccagccaca cccuggagcu agcaaaaaaa aaaaaaaaaa aaaaaaaaaa 4200<br>aaagcauaug acuaaaaaaa aaaaaaaaaa aaaaaaaaaa aaaaaaaaaa aaaaaaaaaa 4260<br>aaaaaaaaaa aaaaaaaaaa aaa 4283 |
| 18 | Construct 1 | agaauaaacu aguauucuuc ugguccccac agacucagag agaacccgcc accauguuug 60<br>uguuucuugu gcugcugccu cuugugucuu cucagugugu ggugagauuu ccaaauauua 120<br>caaaucugug uccauuugga gaaguguuua augcaacaag auuugcaucu guguaugcau 180<br>ggaauagaaa aagaauuucu aauugugugg cugauuauuc ugugcuguau aauagugcuu 240<br>cuuuuuccac auuuaaaugu uauggagugu cuccaacaaa auuaaaugau uuauguuuua 300<br>caaaugugua ugcugauucu uuugugauca gaggugauga agugagacag auugcccccg 360<br>gacagacagg aaaaauugcu gauuacaauu acaaacugcc ugaugauuuu acaggaugug 420<br>ugauugcuug gaauucuaau aauuuagauu cuaaaguggg aggaaauuac aauuaucugu 480<br>acagacuguu uagaaaauca aaucugaaac cuuuugaaag agauauuuca acagaaauuu 540<br>aucaggcugg aucaacaccu uguaauggag uggaaggauu uaauuguuau uuuccauuac 600<br>agagcuaugg auuucagcca accaauggug ugggauauca gccauauaga gugguggugc 660<br>ugucuuuuga acugcugcau gcaccugcaa cagugugugg accuaaaggc ucccccggcu 720<br>ccggcuccgg aucugguuau auuccugaag cuccaagaga ugggcaagcu uacguucgua 780<br>aagauggcga augggauuaa cuuucuaccu uuuuaggccg gucccuggag gugcuguucc 840 |

(continued)

| SARS-CoV-2 Vaccine RNA constructs | | |
|---|---|---|
| | | agggccccgg cugaugacuc gagcugguac ugcaugcacg caaugcuagc ugccccuuuc 900<br>ccguccuggg uaccccgagu cucccccgac cucggguccc agguaugcuc ccaccuccac 960<br>cugccccacu caccaccucu gcuaguucca gacaccuccc aagcacgcag caaugcagcu 1020<br>caaaacgcuu agccuagcca cacccccacg ggaaacagca gugauuaacc uuuagcaaua 1080<br>aacgaaaguu uaacuaagcu auacuaaccc cagguuuggu caauuucgug ccagccacac 1140<br>ccuggagcua gcaaaaaaaa aaaaaaaaaa aaaaaaaaaa aagcauauga cuaaaaaaaa 1200<br>aaaaaaaaaa aaaaaaaaaa aaaaaaaaaa aaaaaaaaaa aaaaaaaaaa aaaaaaaaaa 1260<br>aa 1262 |
| 19 | Construct 3c | agaauaaacu aguauucuuc ugguccccac agacucagag agaacccgcc accauguuug 60<br>uguuucuugu gcugcugccu cuugugucuu cucagugugu gaauuugaca gugagauuuc 120<br>caaauauuac aaaucugugu ccauuuggag aaguguuuaa ugcaacaaga uuugcaucug 180<br>uguaugcaug gaauagaaaa agaauuucua auuguguggc ugauuauucu gugcuguaua 240<br>auagugcuuc uuuuuccaca uuuaaauguu auggaguguc uccaacaaaa uuaaaugauu 300<br>uauguuuuac aaauguguau gcugauucuu uugugaucag aggugaugaa gugagacaga 360<br>uugcccccgg acagacagga aaaauugcug auuacaauua caaacugccu gaugauuuua 420<br>caggaugugu gauugcuugg aauucuaaua auuuagauuc uaaaguggga ggaaauuaca 480<br>auuaucugua cagacuguuu agaaaaucaa aucugaaacc uuuugaaaga gauauuucaa 540<br>cagaaauuua ucaggcugga ucaacaccuu guaauggagu ggaaggauuu aauuguuauu 600<br>uuccauuaca gagcuaugga uuucagccaa ccaauggugu gggauaucag ccauauagag 660<br>ugguggugcu gucuuuugaa cugcugcaug caccugcaac agugugugga ccuaaaggcu 720 |

(continued)

| SARS-CoV-2 Vaccine RNA constructs | | | |
|---|---|---|---|
| | | ccccggcuc cggcuccgga ucugguuaua uuccugaagc uccaagagau gggcaagcuu | 780 |
| | | acguucguaa agauggcgaa uggguauuac uuucuaccuu uuuaggaagc ggcagcggau | 840 |
| | | cugaacagua cauuaaaugg ccuugguaca uuuggcuugg auuuauugca ggauuaauug | 900 |
| | | caauugugau ggugacaauu auguuauguu guaugacauc auguuguucu uguuuaaaag | 960 |
| | | gauguuguuc uuguggaagc uguuguugau gacucgagcu gguacugcau gcacgcaaug | 1020 |
| | | cuagcugccc cuuucccguc cuggguaccc cgagucuccc ccgaccucgg gucccaggua | 1080 |
| | | ugcucccacc uccaccugcc ccacucacca ccucugcuag uuccagacac cucccaagca | 1140 |
| | | cgcagcaaug cagcucaaaa cgcuuagccu agccacaccc ccacgggaaa cagcagugau | 1200 |
| | | uaaccuuuag caauaaacga aaguuuaacu aagcuauacu aaccccaggg uuggucaauu | 1260 |
| | | ucgugccagc cacacccugg agcuagcaaa aaaaaaaaaa aaaaaaaaaa aaaaaaagca | 1320 |
| | | uaugacuaaa aaaaaaaaaa aaaaaaaaaa aaaaaaaaaa aaaaaaaaaa aaaaaaaaaa | 1380 |
| | | aaaaaaaaaa aaaaaaa | 1397 |
| 20 | **Construct 3d** | agaauaaacu aguauucuuc ugguccccac agacucagag agaacccgcc accauggauu | 60 |
| | | ggauuuggag aauccuguuc cucgugggag ccgcuacagg agcccacucc cagaugcagg | 120 |
| | | ugagauuucc aaauauuaca aaucuguguc cauuuggaga aguguuuaau gcaacaagau | 180 |
| | | uugcaucugu guaugcaugg aauagaaaaa gaauuucuaa uuguguggcu gauuauucug | 240 |
| | | ugcuguauaa uagugcuucu uuuuccacau uuaaauguua uggagugucu ccaacaaaau | 300 |
| | | uaaaugauuu auguuuuaca aauguguaug cugauucuuu ugugaucaga ggugaugaag | 360 |
| | | ugagacagau ugcccccgga cagacaggaa aaauugcuga uuacaauuac aaacugccug | 420 |
| | | augauuuuac aggaugugug auugcuugga auucuaauaa uuuagauucu aaagugggag | 480 |

(continued)

| SARS-CoV-2 Vaccine RNA constructs | | | |
|---|---|---|---|
| | | gaaauuacaa uuaucuguac agacuguuua gaaaaucaaa ucugaaaccu uuugaaagag | 540 |
| | | auauuucaac agaaauuuau caggcuggau caacaccuug uaauggagug gaaggauuua | 600 |
| | | auuguuauuu uccauuacag agcuauggau uucagccaac caauggugug ggauaucagc | 660 |
| | | cauauagagu gguggugcug ucuuuugaac ugcugcaugc accugcaaca gugugugac | 720 |
| | | cuaaaggcuc ccccggcucc ggcuccggau cugguuauau uccugaagcu ccaagagaug | 780 |
| | | ggcaagcuua cguucguaaa gauggcgaau ggguauuacu uucuaccuuu uuaggaagcg | 840 |
| | | gcagcggauc ugaacaguac auuaaauggc cuugguacau uuggcuugga uuuauugcag | 900 |
| | | gauuaauugc aauugugaug gugacaauua uguuauguug uaugacauca uguuguucuu | 960 |
| | | guuuaaaagg auguuguucu uguggaagcu guuguugaug acucgagcug guacugcaug | 1020 |
| | | cacgcaaugc uagcugcccc uuucccgucc uggguacccc gagucucccc cgaccucggg | 1080 |
| | | ucccagguau gcucccaccu ccaccugccc cacucaccac cucugcuagu uccagacacc | 1140 |
| | | ucccaagcac gcagcaaugc agcucaaaac gcuuagccua gccacacccc cacgggaaac | 1200 |
| | | agcagugauu aaccuuuagc aauaaacgaa aguuuaacua agcuauacua accccagggu | 1260 |
| | | uggucaauuu cgugccagcc acacccugga gcuagcaaaa aaaaaaaaaa aaaaaaaaaa | 1320 |
| | | aaaaaagcau augacuaaaa aaaaaaaaaa aaaaaaaaaa aaaaaaaaaa aaaaaaaaaa | 1380 |
| | | aaaaaaaaaa aaaaaaaaaa aaaaaa | 1406 |
| 21 | Construct 4 | auguucgugu uccuggugcu gcugccucug guguccagcc agugugugaa ccugaccacc | 60 |
| | | agaacacagc ugccuccagc cuacaccaac agcuuuacca gaggcgugua cuaccccgac | 120 |
| | | aagguguuca gauccagcgu gcugcacucu acccaggacc uguuccugcc uuucuucagc | 180 |
| | | aacgugaccu gguuccacgc cauccacgug uccggcacca auggcaccaa gagauucgac | 240 |

(continued)

| SARS-CoV-2 Vaccine RNA constructs | | |
|---|---|---|
| | | aaccccgugc ugcccuucaa cgacggggug uacuuugcca gcaccgagaa guccaacauc 300<br>aucagaggcu ggaucuucgg caccacacug gacagcaaga cccagagccu gcugaucgug 360<br>aacaacgcca ccaacguggu caucaaagug ugcgaguucc aguucugcaa cgaccccuuc 420<br>cugggcgucu acuaccacaa gaacaacaag agcuggaugg aaagcgaguu ccggguguac 480<br>agcagcgcca acaacugcac cuucgaguac gugucccagc cuuuccugau ggaccuggaa 540<br>ggcaagcagg gcaacuucaa gaaccugcgc gaguucgugu uuaagaacau cgacggcuac 600<br>uucaagaucu acagcaagca caccccuauc aaccucgugc gggaucugcc ucagggcuuc 660<br>ucugcucugg aaccccuggu ggaucugccc aucggcauca acaucacccg guuucagaca 720<br>cugcuggccc ugcacagaag cuaccugaca ccuggcgaua gcagcagcgg auggacagcu 780<br>ggugccgccg cuuacuaugu gggcuaccug cagccuagaa ccuuccugcu gaaguacaac 840<br>gagaacggca ccaucaccga cgccguggau ugugcucugg auccucugag cgagacaaag 900<br>ugcacccuga aguccuucac cguggaaaag ggcaucuacc agaccagcaa cuuccgggug 960<br>cagcccaccg aauccaucgu gcgguucccc aauaucacca aucugugccc cuucggcgag 1020<br>guguucaaug ccaccagauu cgccucugug uacgccugga accggaagcg gaucagcaau 1080<br>ugcguggccg acuacuccgu gcuguacaac uccgccagcu ucagcaccuu caagugcuac 1140<br>ggcguguccc cuaccaagcu gaacgaccug ugcuucacaa acguguacgc cgacagcuuc 1200<br>gugauccggg gagaugaagu gcggcagauu gccccuggac agacaggcaa gaucgccgac 1260<br>uacaacuaca agcugcccga cgacuucacc ggcuguguga uugccuggaa cagcaacaac 1320<br>cuggacucca aagucggcgg caacuacaau uaccuguacc ggcuguuccg gaaguccaau 1380 |

(continued)

| SARS-CoV-2 Vaccine RNA constructs | | |
|---|---|---|
| | | cugaagcccu ucgagcggga caucuccacc gagaucuauc aggccggcag caccccuugu 1440 |
| | | aacggcgugg aaggcuucaa cugcuacuuc ccacugcagu ccuacggcuu ucagcccaca 1500 |
| | | aauggcgugg gcuaucagcc cuacagagug guggugcuga gcuucgaacu gcugcaugcc 1560 |
| | | ccugccacag ugugcggccc uaagaaaagc accaaucucg ugaagaacaa augcgugaac 1620 |
| | | uucaacuuca acggccugac cggcaccggc gugcugacag agagcaacaa gaaguuccug 1680 |
| | | ccauuccagc aguuuggccg ggauaucgcc gauaccacag acgccguuag agaucccccag 1740 |
| | | acacuggaaa uccuggacau caccccuugc agcuucggcg gagugucugu gaucaccccu 1800 |
| | | ggcaccaaca ccagcaauca gguggcagug cuguaccagg acgugaacug uaccgaagug 1860 |
| | | cccguggcca uucacgccga ucagcugaca ccuacauggc ggguguacuc caccggcagc 1920 |
| | | aauguguuuc agaccagagc cggcugucug aucggagccg agcacgugaa caauagcuac 1980 |
| | | gagugcgaca uccccaucgg cgcuggaauc ugcgccagcu accagacaca gacaaacagc 2040 |
| | | ccucggagag ccagaagcgu ggccagccag agcaucauug ccuacacaau gucucugggc 2100 |
| | | gccgagaaca gcguggccua cuccaacaac ucuaucgcua uccccaccaa cuucaccauc 2160 |
| | | agcgugacca cagagauccu gccuguguce augaccaaga ccagcgugga cugcaccaug 2220 |
| | | uacaucugcg gcgauuccac cgagugcucc aaccugcugc ugcaguacgg cagcuucugc 2280 |
| | | acccagcuga auagagcccu gacagggauc gccguggaac aggacaagaa cacccaagag 2340 |
| | | guguucgccc aagugaagca gaucuacaag accccuccua ucaaggacuu cggcggcuuc 2400 |
| | | aauuucagcc agauucugcc cgauccuagc aagcccagca agcggagcuu caucgaggac 2460 |
| | | cugcuguuca acaaagugac acuggccgac gccggcuuca ucaagcagua uggcgauugu 2520 |
| | | cugggcgaca uugccgccag ggaucugauu ugcgcccaga aguuuaacgg acugacagug 2580 |

(continued)

| SARS-CoV-2 Vaccine RNA constructs | | |
|---|---|---|
| | | cugccuccuc ugcugaccga ugagaugauc gcccaguaca caucugcccu gcuggccggc 2640<br>acaaucacaa gcggcuggac auuuggagca ggcgccgcuc ugcagauccc cuuugcuaug 2700<br>cagauggccu accgguucaa cggcaucgga gugacccaga augugcugua cgagaaccag 2760<br>aagcugaucg ccaaccaguu caacagcgcc aucggcaaga uccaggacag ccugagcagc 2820<br>acagcaagcg cccugggaaa gcugcaggac guggucaacc agaaugccca ggcacugaac 2880<br>acccugguca agcagcuguc cuccaacuuc ggcgccauca gcucugugcu gaacgauauc 2940<br>cugagcagac uggacccucc ugaggccgag gugcagaucg acagacugau cacaggcaga 3000<br>cugcagagcc uccagacaua cgugacccag cagcugauca gagccgccga gauuagagcc 3060<br>ucugccaauc uggccgccac caagaugucu gagugugugc ugggccagag caagagagug 3120<br>gacuuuugcg gcaagggcua ccaccugaug agcuucccuc agucugcccc ucacggcgug 3180<br>guguuucugc acgugacaua ugugcccgcu caagagaaga auuucaccac cgcuccagcc 3240<br>aucugccacg acggcaaagc ccacuuuccu agagaaggcg uguucguguc caacggcacc 3300<br>cauugguucg ugacacagcg gaacuucuac gagccccaga ucaucaccac cgacaacacc 3360<br>uucgugucug gcaacugcga cgucgugauc ggcauuguga acaauaccgu guacgacccu 3420<br>cugcagcccg agcuggacag cuucaaagag gaacuggaca aguacuuuaa gaaccacaca 3480<br>agccccgacg uggaccuggg cgauaucagc ggaaucaaug ccagcgucgu gaacauccag 3540<br>aaagagaucg accggcugaa cgagguggcc aagaaucuga acgagagccu gaucgaccug 3600<br>caagaacugg ggaaguacga gcaguacauc aaguggcccu gguacaucug gcugggcuuu 3660<br>aucgccggac ugauugccau cgugaugguc acaaucaugc uguguugcau gaccagcugc 3720<br>uguagcugcc ugaagggcug uuguagcugu ggcagcugcu gcaaguucga cgaggacgau 3780 |
| | | ucugagcccg ugcugaaggg cgugaaacug cacuacaca 3819 |

**Variant Specific Vaccines**

In some embodiments, RNA disclosed herein encodes an S protein comprising one or more mutations that are characteristic of a SARS-CoV-2 variant. In some embodiments, RNA disclosed herein encodes an S protein comprising one or more mutations that are characteristic of a SARS-CoV-2 variant of concern (e.g., a SARS-CoV-2 that is prevalent in a relevant jurisdiction and/or is predicted to become prevalent). In some embodiments, the present disclosure refers to a SARS-CoV-2 variant that is prevalent and/or rapidly spreading in a relevant jurisdiction. In some embodiments, such variants may be identified based on publicly available data (e.g., data provided in the GISAID Initiative database: https://www.gisaid.org, and/or data provided by the World Health Organization WHO (e.g., as provided at https://www.who.int/activities/tracking-SARS-CoV-2-variants). In some embodiments, such a variant refers to a variant disclosed herein.

In some embodiments, RNA encodes a SARS-CoV-2 S protein comprising one or more mutations characteristic of an Alpha variant. The Alpha variant was one of the earliest SARS-CoV-2 variants that emerged and rapidly became globally dominant. The alpha variant (also known as B.1.1.7, VOC202012/01, 501Y.V1 or GRY) was initially detected in the United Kingdom. The alpha variant has a large number of mutations, including several mutations in the S gene. It has been shown to be inherently more transmissible, with a growth rate that has been estimated to be 40-70% higher than other SARS-CoV-2 lineages in multiple countries (Volz et al., 2021, Nature, https://doi.org/10.1038/s41586-021-03470-x; Washington et al., 2021, Cell https://doi.org/10.1016/j.cell.2021.03.052).

In some embodiments, RNA encodes a SARS-CoV-2 S protein of a beta variant (also known as B.1.351 or GH/501Y.V2). The Beta variant was first detected in South Africa. The beta variant carries several mutations in the S gene. Three of these mutations are at sites in the RBD that are associated with immune evasion: N501Y (shared with alpha) and E484K and K417N.

In some embodiments, RNA encodes a SARS-CoV-2 S protein comprising one or more mutations characteristic of a Delta variant. The delta variant (also known as B.1.617.2 or G/478K.V1) was first documented in India. The delta variant has several point mutations that affect the spike protein, including P681R (a mutation position shared with alpha and adjacent to the furin cleavage site), and L452R, which is in the RBD and has been linked with increased binding to ACE2 and neutralizing antibody resistance. There is also a deletion in the spike protein at position 156/157.

In some embodiments, RNA disclosed herein comprises a nucleotide sequence encoding an amino acid sequence of a SARS-CoV-2 S protein comprising one or more mutations of an Omicron variant (also known as B.1.529). B.1.529 was first detected in South Africa in November 2021. Omicron multiplies around 70 times faster than Delta variants, and quickly became the dominant strain of SARS-CoV-2 worldwide. Since its initial detection, a number of Omicron sublineages have arisen. Listed below are the current Omicron variants of concern, along with certain characteristic mutations associated with the S protein of each. The S protein of BA.4 and BA.5 have the same set of characteristic mutations, which is why the below table has a single row for "BA.4 or BA.5", and why the present disclosure refers to a "BA.4/5" S protein in some embodiments.

**Table 2: Omicron Variants of Concern and Characteristic mutations**

| Subvariant | Common mutations |
|---|---|
| BA.1 | A67V, Δ69-70, T95I, G142D, Δ143-145, Δ211, L212I, ins214EPE, G339D, S371L, S373P, S375F, K417N, N440K, G446S, S477N, T478K, E484A, Q493R, G496S, Q498R, N501Y, Y505H, T547K, D614G, H655Y, N679K, P681H, N764K, D796Y, N856K, Q954H, N969K, L981F |
| BA.2 | T19I, Δ24-26, A27S, G142D, V213G, G339D, S371F, S373P, S375F, T376A, D405N, R408S, K417N, N440K, S477N, T478K, E484A, Q493R, Q498R, N501Y, Y505H, D614G, H655Y, N679K, P681H, N764K, D796Y, Q954H, N969K |
| BA.2.12.1 | T19I, Δ24-26, A27S, G142D, V213G, G339D, S371F, S373P, S375F, T376A, D405N, R408S, K417N, N440K, L452Q, S477N, T478K, E484A, Q493R, Q498R, N501Y, Y505H, D614G, H655Y, N679K, P681H, S704L, N764K, D796Y, Q954H, N969K |
| BA.4 or BA.5 | T19I, Δ24-26, A27S, Δ69/70, G142D, V213G, G339D, S371F, S373P, S375F, T376A, D405N, R408S, K417N, N440K, L452R, S477N, T478K, E484A, F486V, Q498R, N501Y, Y505H, D614G, H655Y, N679K, P681H, N764K, D796Y, Q954H, N969K |
| BA.2.75 | T19I, Δ24-26, A27S, G142D, K147E, W152R, F157L, I210V, V213G, G257S, G339H, N354D, S371F, S373P, S375F, T376A, D405N, R408S, K417N, N440K, G446S, N460K, S477N, T478K, E484A, Q498R, N501Y, Y505H D614G, H655Y, N679K, P681H, N764K, D796Y, Q954H, and N969K |

(continued)

| Subvariant | Common mutations |
|---|---|
| BA.2.75.2 | T19I, Δ24-26, A27S, G142D, K147E, W152R, F157L, I210V, V213G, G257S, G339H, R346T, N354D, S371F, S373P, S375F, T376A, D405N, R408S, K417N, N440K, G446S, N460K, S477N, T478K, E484A, F486S, Q498R, N501Y, Y505H, D614G, H655Y, N679K, P681H, N764K, D796Y, Q954H, N969K, and D1199N |
| BJ.1 | T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, S477N, T478K, V483A, E484A, F490V, Q493R, Q498R, N501Y, Y505H, D614G, H655Y, N679K, P681H, N764K, D796Y, G798D, Q954H, N969K, and S1003I |
| BA.4.6 | T19I, Δ24-26, A27S, Δ69/70, G142D, V213G, G339D, R346T, S371F, S373P, S375F, T376A, D405N, R408S, K417N, N440K, L452R, S477N, T478K, E484A, F486V, Q498R, N501Y, Y505H, D614G, H655Y, N658S, N679K, P681H, N764K, D796Y, Q954H, and N969K |
| XBB | T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, F486S, F490S, Q493R, Q498R, N501Y, Y505H, D614G, H655Y, N679K, P681H, N764K, D796Y, Q954H, and N969K |
| BA.2.3.20 | T19I, Δ24-26, A27S, G142D, M153T, N164K, V213G, H245N, G257D, G339D, S371F, S373P, S375F, T376A, D405N, R408S, K417N, N440K, K444R, E484R N450D, L452M, N460K, S477N, T478K, E484A, Q493R, Q498R, N501Y, Y505H, D614G, H655Y, N679K, P681H, N764K, D796Y, Q954H, and N969K |

In some embodiments, RNA described herein comprises a nucleotide sequence encoding a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron variant. In some embodiments, an RNA comprises a nucleotide sequence encoding a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) listed in Table 2. In some such embodiments, one or more mutations may come from two or more variants as listed in Table 2. In some embodiments, an RNA comprises a nucleotide sequence encoding a SARS-CoV-2 S protein comprising each of the mutations identified in Table 2 as being characteristic of a certain Omicron variant (e.g., in some embodiments, an RNA comprises a nucleotide sequence encoding a SARS-CoV-2 S protein comprising each of the mutations listed in Table 2 as being characteristic of an Omicron BA.1, BA.2, BA.2.12.1, BA.4/5, BA.2.75, BA.2.75.1, BA.4.6 or XBB variant).

In some embodiments, an RNA encodes a SARS-CoV-2 S protein comprising a subset of the mutations listed in Table 2. In some embodiments, an RNA encodes a SARS-CoV-2 S protein comprising the mutations listed in Table 2 that are most prevalent in a certain variant (e.g., mutations that have been detected in at least 30%, at least 40%, at least 50%, at least 60%, at least 70%, at least 80%, at least 90%, at least 95%, at least 96%, at least 97%, at least 98%, at least 99%, or 100% of sequences collected to date for a given variant sequenced). Mutation prevalence can be determined, e.g., based on published sequences (e.g., sequences that are collected and made available to the public by GISAID).

In some embodiments, RNA described herein encodes a SARS-CoV-2 S protein comprising one or more mutations that are characteristic of a BA.4/5 variant. In some embodiments, the one or more mutations characteristic of a BA.4/5 variant include T19I, Δ24-26, A27S, Δ69/70, G142D, V213G, G339D, S371F, S373P, S375F, T376A, D405N, K417N, N440K, L452R, S477N, T478K, E484A, F486V, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations that are characteristic of a BA.4/5 variant and excludes R408S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4/5 variant and excludes R408S.

In some embodiments, RNA described herein encodes a SARS-CoV-2 S protein comprising one or more (including, e.g., 2, 3, 4, 5, 6, 7, 8, 9, 10, 11, 12, 13, 14, 15, 16, 17, 18, 19, 20, or more) mutations characteristic of a BA.2.75 variant. In some embodiments, the one or more mutations characteristic of a BA.2.75 variant include T19I, Δ24-26, A27S, G142D, K147E, W152R, F157L, I210V, V213G, G257S, G339H, S371F, S373P, S375F, T376A, D405N, R408S, K417N, N440K, G446S, N460K, S477N, T478K, E484A, Q498R, N501Y, Y505H D614G, H655Y, N679K, P681H, N764K, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are

characteristic of a BA.2.75 variant, and which excludes N354D. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75 variant, and which excludes D796Y. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75 variant, and which excludes D796Y and N354D.

In some embodiments, RNA described herein encodes a SARS-CoV-2 S protein comprising one or more mutations characteristic of a BA.2.75.2 variant. In some embodiments, the one or more mutations characteristic of a BA.2.75.2 variant include T19I, Δ24-26, A27S, G142D, K147E, W152R, F157L, I210V, V213G, G257S, G339H, R346T, N354D, S371F, S373P, S375F, T376A, D405N, R408S, K417N, N440K, G446S, N460K, S477N, T478K, E484A, F486S, Q498R, N501Y, Y505H, D614G, H655Y, N679K, P681H, N764K, D796Y, Q954H, N969K, and D1199N. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.2.75.2 variant, and which excludes R346T.

In some embodiments, RNA described herein encodes a SARS-CoV-2 S protein comprising one or more mutations characteristic of a BA.4.6 variant. In some embodiments, the one or more mutations characteristic of a BA.4.6 variant include T19I, Δ24-26, A27S, Δ69/70, G142D, V213G, G339D, R346T, S371F, S373P, S375F, T376A, D405N, K417N, N440K, L452R, S477N, T478K, E484A, F486V, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 variant, and which exclude R408S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 variant, and which exclude N658S. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of a BA.4.6 variant, and which exclude N658S and R408S.

In some embodiments, RNA described herein encodes a SARS-CoV-2 S protein comprising one or more mutations characteristic of an Omicron XBB variant. In some embodiments, the one or more mutations characteristic of an Omicron-XBB variant include T19I, Δ24-26, A27S, V83A, G142D, Δ144, H146Q, Q183E, V213E, G339H, R346T, L368I, S371F, S373P, S375F, T376A, D405N, R408S, K417N, N440K, V445P, G446S, N460K, S477N, T478K, E484A, F486S, F490S, Q498R, N501Y, Y505H, D614G, H655Y, N679K, P681H, N764K, D796Y, Q954H, and N969K. In some embodiments, the one or more mutations characteristic of an Omicron-XBB variant include T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, F486S, F490S, Q498R, N501Y, Y505H, D614G, H655Y, N679K, P681H, N764K, D796Y, Q954H, and N969K. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron XBB variant and which exclude G252V. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron XBB variant and which exclude Q493R. In some embodiments, RNA described herein encodes a SARS-CoV-2 S protein comprising one or more mutations (including, e.g., 2, 3, 4, 5, 6, 7, 8, 9, 10, 11, 12, 13, 14, 15, 16, 17, 18, 19, 20, or more) that are characteristic of an Omicron XBB variant and which exclude Q493R and G252V.In one embodiment, the vaccine antigen described herein comprises, consists essentially of or consists of a spike protein (S) of SARS-CoV-2, a variant thereof, or a fragment thereof.

**Nucleotide Sequence of RBP020.11 (Beta-specific vaccine)**

Nucleotide sequence is shown with individual sequence elements as indicated in bold letters. In addition, the sequence of the translated protein is shown in italic letters below the coding nucleotide sequence (* = stop codon). Red text indicates point mutations in the nucleotide and amino acid sequences.

```
         10         20         30         40         50   53
AGAATAAACT AGTATTCTTC TGGTCCCCAC AGACTCAGAG AGAACCCGCC ACC
```

hAg-Kozak

```
        63         73         83         93        103        113
ATGTTCGTGT TCCTGGTGCT GCTGCCTCTG GTGTCCAGCC AGTGTGTGAA CTTCACCACC
 M  F  V   F  L  V   L  L  P  L   V  S  S   Q  C  V   N  F  T  T
```

S Protein mut

```
       123        133        143        153        163        173
AGAACACAGC TGCCTCCAGC CTACACCAAC AGCTTTACCA GAGGCGTGTA CTACCCCGAC
 R  T  Q   L  P  P   A  Y  T  N   S  F  T   R  G  V   Y  Y  P  D
```

S Protein mut

```
       183        193        203        213        223        233
AAGGTGTTCA GATCCAGCGT GCTGCACTCT ACCCAGGACC TGTTCCTGCC TTTCTTCAGC
 K  V  F   R  S  S   V  L  H  S   T  Q  D   L  F  L   P  F  F  S
```

S Protein mut

```
       243        253        263        273        283        293
AACGTGACCT GGTTCCACGC CATCCACGTG TCCGGCACCA ATGGCACCAA GAGATTCGCC
 N  V  T   W  F  H   A  I  H  V   S  G  T   N  G  T   K  R  F  A
```

S Protein mut

```
       303        313        323        333        343        353
AACCCCGTGC TGCCCTTCAA CGACGGGGTG TACTTTGCCA GCACCGAGAA GTCCAACATC
 N  P  V   L  P  F   N  D  G  V   Y  F  A   S  T  E   K  S  N  I
```

S Protein mut

```
       363        373        383        393        403        413
ATCAGAGGCT GGATCTTCGG CACCACACTG GACAGCAAGA CCCAGAGCCT GCTGATCGTG
 I  R  G   W  I  F   G  T  T  L   D  S  K   T  Q  S   L  L  I  V
```

S Protein mut

```
       423        433        443        453        463        473
AACAACGCCA CCAACGTGGT CATCAAAGTG TGCGAGTTCC AGTTCTGCAA CGACCCCTTC
 N  N  A   T  N  V   V  I  K  V   C  E  F   Q  F  C   N  D  P  F
```

S Protein mut

```
       483        493        503        513        523        533
CTGGGCGTCT ACTACCACAA GAACAACAAG AGCTGGATGG AAAGCGAGTT CCGGGTGTAC
 L  G  V   Y  Y  H   K  N  N  K   S  W  M   E  S  E   F  R  V  Y
```

S Protein mut

```
       543        553        563        573        583        593
AGCAGCGCCA ACAACTGCAC CTTCGAGTAC GTGTCCCAGC CTTTCCTGAT GGACCTGGAA
 S  S  A   N  N  C   T  F  E  Y   V  S  Q   P  F  L   M  D  L  E
```

S Protein mut

```
       603        613        623        633        643        653
GGCAAGCAGG GCAACTTCAA GAACCTGCGC GAGTTCGTGT TTAAGAACAT CGACGGCTAC
 G  K  Q   G  N  F   K  N  L  R   E  F  V   F  K  N   I  D  G  Y
```

S Protein mut

```
       663        673        683        693        703        713
TTCAAGATCT ACAGCAAGCA CACCCCTATC AACCTCGTGC GGGGCCTGCC TCAGGGCTTC
 F  K  I   Y  S  K   H  T  P  I   N  L  V   R  G  L   P  Q  G  F
```

S Protein mut

```
        723        733        743        753        763        773
TCTGCTCTGG AACCCCTGGT GGATCTGCCC ATCGGCATCA ACATCACCCG GTTTCAGACA
 S  A  L   E  P  L   V  D  L  P   I  G  I   N  I  T   R  F  Q  T
                          S Protein mut

        783        793        803        813        823        833
CTGCACATCA GCTACCTGAC ACCTGGCGAT AGCAGCAGCG GATGGACAGC TGGTGCCGCC
 L  H  I   S  Y  L   T  P  G  D   S  S  S   G  W  T   A  G  A  A
                          S Protein mut

        843        853        863        873        883        893
GCTTACTATG TGGGCTACCT GCAGCCTAGA ACCTTCCTGC TGAAGTACAA CGAGAACGGC
 A  Y  Y   V  G  Y   L  Q  P  R   T  F  L   L  K  Y   N  E  N  G
                          S Protein mut

        903        913        923        933        943        953
ACCATCACCG ACGCCGTGGA TTGTGCTCTG GATCCTCTGA GCGAGACAAA GTGCACCCTG
 T  I  T   D  A  V   D  C  A  L   D  P  L   S  E  T   K  C  T  L
                          S Protein mut

        963        973        983        993       1003       1013
AAGTCCTTCA CCGTGGAAAA GGGCATCTAC CAGACCAGCA ACTTCCGGGT GCAGCCCACC
 K  S  F   T  V  E   K  G  I  Y   Q  T  S   N  F  R   V  Q  P  T
                          S Protein mut

       1023       1033       1043       1053       1063       1073
GAATCCATCG TGCGGTTCCC CAATATCACC AATCTGTGCC CCTTCGGCGA GGTGTTCAAT
 E  S  I   V  R  F   P  N  I  T   N  L  C   P  F  G   E  V  F  N
                          S Protein mut

       1083       1093       1103       1113       1123       1133
GCCACCAGAT TCGCCTCTGT GTACGCCTGG AACCGGAAGC GGATCAGCAA TTGCGTGGCC
 A  T  R   F  A  S   V  Y  A  W   N  R  K   R  I  S   N  C  V  A
                          S Protein mut

       1143       1153       1163       1173       1183       1193
GACTACTCCG TGCTGTACAA CTCCGCCAGC TTCAGCACCT TCAAGTGCTA CGGCGTGTCC
 D  Y  S   V  L  Y   N  S  A  S   F  S  T   F  K  C   Y  G  V  S
                          S Protein mut

       1203       1213       1223       1233       1243       1253
CCTACCAAGC TGAACGACCT GTGCTTCACA AACGTGTACG CCGACAGCTT CGTGATCCGG
 P  T  K   L  N  D   L  C  F  T   N  V  Y   A  D  S   F  V  I  R
                          S Protein mut

       1263       1273       1283       1293       1303       1313
GGAGATGAAG TGCGGCAGAT TGCCCCTGGA CAGACAGGCA ACATCGCCGA CTACAACTAC
 G  D  E   V  R  Q   I  A  P  G   Q  T  G   N  I  A   D  Y  N  Y
                          S Protein mut

       1323       1333       1343       1353       1363       1373
AAGCTGCCCG ACGACTTCAC CGGCTGTGTG ATTGCCTGGA ACAGCAACAA CCTGGACTCC
 K  L  P   D  D  F   T  G  C  V   I  A  W   N  S  N   N  L  D  S
                          S Protein mut
```

1383 1393 1403 1413 1423 1433
AAAGTCGGCG GCAACTACAA TTACCTGTAC CGGCTGTTCC GGAAGTCCAA TCTGAAGCCC
*K V G G N Y N Y L Y R L F R K S N L K P*
S Protein mut

1443 1453 1463 1473 1483 1493
TTCGAGCGGG ACATCTCCAC CGAGATCTAT CAGGCCGGCA GCACCCCTTG TAACGGCGTG
*F E R D I S T E I Y Q A G S T P C N G V*
S Protein mut

1503 1513 1523 1533 1543 1553
AAGGGCTTCA ACTGCTACTT CCCACTGCAG TCCTACGGCT TTCAGCCCAC ATACGGCGTG
*K G F N C Y F P L Q S Y G F Q P T Y G V*
S Protein mut

1563 1573 1583 1593 1603 1613
GGCTATCAGC CCTACAGAGT GGTGGTGCTG AGCTTCGAAC TGCTGCATGC CCCTGCCACA
*G Y Q P Y R V V V L S F E L L H A P A T*
S Protein mut

1623 1633 1643 1653 1663 1673
GTGTGCGGCC CTAAGAAAAG CACCAATCTC GTGAAGAACA AATGCGTGAA CTTCAACTTC
*V C G P K K S T N L V K N K C V N F N F*
S Protein mut

1683 1693 1703 1713 1723 1733
AACGGCCTGA CCGGCACCGG CGTGCTGACA GAGAGCAACA AGAAGTTCCT GCCATTCCAG
*N G L T G T G V L T E S N K K F L P F Q*
S Protein mut

1743 1753 1763 1773 1783 1793
CAGTTTGGCC GGGATATCGC CGATACCACA GACGCCGTTA GAGATCCCCA GACACTGGAA
*Q F G R D I A D T T D A V R D P Q T L E*
S Protein mut

1803 1813 1823 1833 1843 1853
ATCCTGGACA TCACCCCTTG CAGCTTCGGC GGAGTGTCTG TGATCACCCC TGGCACCAAC
*I L D I T P C S F G G V S V I T P G T N*
S Protein mut

1863 1873 1883 1893 1903 1913
ACCAGCAATC AGGTGGCAGT GCTGTACCAG GGCGTGAACT GTACCGAAGT GCCCGTGGCC
*T S N Q V A V L Y Q G V N C T E V P V A*
S Protein mut

1923 1933 1943 1953 1963 1973
ATTCACGCCG ATCAGCTGAC ACCTACATGG CGGGTGTACT CCACCGGCAG CAATGTGTTT
*I H A D Q L T P T W R V Y S T G S N V F*
S Protein mut

1983 1993 2003 2013 2023 2033
CAGACCAGAG CCGGCTGTCT GATCGGAGCC GAGCACGTGA ACAATAGCTA CGAGTGCGAC
*Q T R A G C L I G A E H V N N S Y E C D*
S Protein mut

```
     2043      2053      2063      2073      2083      2093
ATCCCCATCG GCGCTGGAAT CTGCGCCAGC TACCAGACAC AGACAAACAG CCCTCGGAGA
 I  P  I   G  A  G   I  C  A  S   Y  Q  T   Q  T  N   S  P  R  R
                         S Protein mut

     2103      2113      2123      2133      2143      2153
GCCAGAAGCG TGGCCAGCCA GAGCATCATT GCCTACACAA TGTCTCTGGG CGTCGAGAAC
 A  R  S   V  A  S   Q  S  I  I   A  Y  T   M  S  L   G  V  E  N
                         S Protein mut

     2163      2173      2183      2193      2203      2213
AGCGTGGCCT ACTCCAACAA CTCTATCGCT ATCCCCACCA ACTTCACCAT CAGCGTGACC
 S  V  A   Y  S  N   N  S  I  A   I  P  T   N  F  T   I  S  V  T
                         S Protein mut

     2223      2233      2243      2253      2263      2273
ACAGAGATCC TGCCTGTGTC CATGACCAAG ACCAGCGTGG ACTGCACCAT GTACATCTGC
 T  E  I   L  P  V   S  M  T  K   T  S  V   D  C  T   M  Y  I  C
                         S Protein mut

     2283      2293      2303      2313      2323      2333
GGCGATTCCA CCGAGTGCTC CAACCTGCTG CTGCAGTACG GCAGCTTCTG CACCCAGCTG
 G  D  S   T  E  C   S  N  L  L   L  Q  Y   G  S  F   C  T  Q  L
                         S Protein mut

     2343      2353      2363      2373      2383      2393
AATAGAGCCC TGACAGGGAT CGCCGTGGAA CAGGACAAGA ACACCCAAGA GGTGTTCGCC
 N  R  A   L  T  G   I  A  V  E   Q  D  K   N  T  Q   E  V  F  A
                         S Protein mut

     2403      2413      2423      2433      2443      2453
CAAGTGAAGC AGATCTACAA GACCCCTCCT ATCAAGGACT TCGGCGGCTT CAATTTCAGC
 Q  V  K   Q  I  Y   K  T  P  P   I  K  D   F  G  G   F  N  F  S
                         S Protein mut

     2463      2473      2483      2493      2503      2513
CAGATTCTGC CCGATCCTAG CAAGCCCAGC AAGCGGAGCT TCATCGAGGA CCTGCTGTTC
 Q  I  L   P  D  P   S  K  P  S   K  R  S   F  I  E   D  L  L  F
                         S Protein mut

     2523      2533      2543      2553      2563      2573
AACAAAGTGA CACTGGCCGA CGCCGGCTTC ATCAAGCAGT ATGGCGATTG TCTGGGCGAC
 N  K  V   T  L  A   D  A  G  F   I  K  Q   Y  G  D   C  L  G  D
                         S Protein mut

     2583      2593      2603      2613      2623      2633
ATTGCCGCCA GGGATCTGAT TTGCGCCCAG AAGTTTAACG GACTGACAGT GCTGCCTCCT
 I  A  A   R  D  L   I  C  A  Q   K  F  N   G  L  T   V  L  P  P
                         S Protein mut

     2643      2653      2663      2673      2683      2693
CTGCTGACCG ATGAGATGAT CGCCCAGTAC ACATCTGCCC TGCTGGCCGG CACAATCACA
 L  L  T   D  E  M   I  A  Q  Y   T  S  A   L  L  A   G  T  I  T
                         S Protein mut
```

```
       2703      2713      2723      2733      2743      2753
AGCGGCTGGA CATTTGGAGC AGGCGCCGCT CTGCAGATCC CCTTTGCTAT GCAGATGGCC
 S  G  W   T  F  G   A  G  A  A   L  Q  I   P  F  A   M  Q  M  A
                       S Protein mut

       2763      2773      2783      2793      2803      2813
TACCGGTTCA ACGGCATCGG AGTGACCCAG AATGTGCTGT ACGAGAACCA GAAGCTGATC
 Y  R  F   N  G  I   G  V  T  Q   N  V  L   Y  E  N   Q  K  L  I
                       S Protein mut

       2823      2833      2843      2853      2863      2873
GCCAACCAGT TCAACAGCGC CATCGGCAAG ATCCAGGACA GCCTGAGCAG CACAGCAAGC
 A  N  Q   F  N  S   A  I  G  K   I  Q  D   S  L  S   S  T  A  S
                       S Protein mut

       2883      2893      2903      2913      2923      2933
GCCCTGGGAA AGCTGCAGGA CGTGGTCAAC CAGAATGCCC AGGCACTGAA CACCCTGGTC
 A  L  G   K  L  Q   D  V  V  N   Q  N  A   Q  A  L   N  T  L  V
                       S Protein mut

       2943      2953      2963      2973      2983      2993
AAGCAGCTGT CCTCCAACTT CGGCGCCATC AGCTCTGTGC TGAACGATAT CCTGAGCAGA
 K  Q  L   S  S  N   F  G  A  I   S  S  V   L  N  D   I  L  S  R
                       S Protein mut

       3003      3013      3023      3033      3043      3053
CTGGACCCTC CTGAGGCCGA GGTGCAGATC GACAGACTGA TCACAGGCAG ACTGCAGAGC
 L  D  P   P  E  A   E  V  Q  I   D  R  L   I  T  G   R  L  Q  S
                       S Protein mut

       3063      3073      3083      3093      3103      3113
CTCCAGACAT ACGTGACCCA GCAGCTGATC AGAGCCGCCG AGATTAGAGC CTCTGCCAAT
 L  Q  T   Y  V  T   Q  Q  L  I   R  A  A   E  I  R   A  S  A  N
                       S Protein mut

       3123      3133      3143      3153      3163      3173
CTGGCCGCCA CCAAGATGTC TGAGTGTGTG CTGGGCCAGA GCAAGAGAGT GGACTTTTGC
 L  A  A   T  K  M   S  E  C  V   L  G  Q   S  K  R   V  D  F  C
                       S Protein mut

       3183      3193      3203      3213      3223      3233
GGCAAGGGCT ACCACCTGAT GAGCTTCCCT CAGTCTGCCC CTCACGGCGT GGTGTTTCTG
 G  K  G   Y  H  L   M  S  F  P   Q  S  A   P  H  G   V  V  F  L
                       S Protein mut

       3243      3253      3263      3273      3283      3293
CACGTGACAT ATGTGCCCGC TCAAGAGAAG AATTTCACCA CCGCTCCAGC CATCTGCCAC
 H  V  T   Y  V  P   A  Q  E  K   N  F  T   T  A  P   A  I  C  H
                       S Protein mut

       3303      3313      3323      3333      3343      3353
GACGGCAAAG CCCACTTTCC TAGAGAAGGC GTGTTCGTGT CCAACGGCAC CCATTGGTTC
 D  G  K   A  H  F   P  R  E  G   V  F  V   S  N  G   T  H  W  F
                       S Protein mut
```

```
       3363      3373      3383      3393      3403      3413
GTGACACAGC GGAACTTCTA CGAGCCCCAG ATCATCACCA CCGACAACAC CTTCGTGTCT
  V  T  Q   R  N  F   Y  E  P  Q   I  I  T   T  D  N   T  F  V  S
                          S Protein mut

       3423      3433      3443      3453      3463      3473
GGCAACTGCG ACGTCGTGAT CGGCATTGTG AACAATACCG TGTACGACCC TCTGCAGCCC
  G  N  C   D  V  V   I  G  I  V   N  N  T   V  Y  D   P  L  Q  P
                          S Protein mut

       3483      3493      3503      3513      3523      3533
GAGCTGGACA GCTTCAAAGA GGAACTGGAC AAGTACTTTA AGAACCACAC AAGCCCCGAC
  E  L  D   S  F  K   E  E  L  D   K  Y  F   K  N  H   T  S  P  D
                          S Protein mut

       3543      3553      3563      3573      3583      3593
GTGGACCTGG GCGATATCAG CGGAATCAAT GCCAGCGTCG TGAACATCCA GAAAGAGATC
  V  D  L   G  D  I   S  G  I  N   A  S  V   V  N  I   Q  K  E  I
                          S Protein mut

       3603      3613      3623      3633      3643      3653
GACCGGCTGA ACGAGGTGGC CAAGAATCTG AACGAGAGCC TGATCGACCT GCAAGAACTG
  D  R  L   N  E  V   A  K  N  L   N  E  S   L  I  D   L  Q  E  L
                          S Protein mut

       3663      3673      3683      3693      3703      3713
GGGAAGTACG AGCAGTACAT CAAGTGGCCC TGGTACATCT GGCTGGGCTT TATCGCCGGA
  G  K  Y   E  Q  Y   I  K  W  P   W  Y  I   W  L  G   F  I  A  G
                          S Protein mut

       3723      3733      3743      3753      3763      3773
CTGATTGCCA TCGTGATGGT CACAATCATG CTGTGTTGCA TGACCAGCTG CTGTAGCTGC
  L  I  A   I  V  M   V  T  I  M   L  C  C   M  T  S   C  C  S  C
                          S Protein mut

       3783      3793      3803      3813      3823      3833
CTGAAGGGCT GTTGTAGCTG TGGCAGCTGC TGCAAGTTCG ACGAGGACGA TTCTGAGCCC
  L  K  G   C  C  S   C  G  S  C   C  K  F   D  E  D   D  S  E  P
                          S Protein mut

       3843      3853      3863   3870
GTGCTGAAGG GCGTGAAACT GCACTACACA TGATGAC
  V  L  K   G  V  K   L  H  Y  T   *  *
                          S Protein mut

       3880      3890      3900      3910      3920      3930
TCGAGCTGGT ACTGCATGCA CGCAATGCTA GCTGCCCCTT TCCCGTCCTG GGTACCCCGA
                          FI Element

       3940      3950      3960      3970      3980      3990
GTCTCCCCCG ACCTCGGGTC CCAGGTATGC TCCCACCTCC ACCTGCCCCA CTCACCACCT
                          FI Element

       4000      4010      4020      4030      4040      4050
CTGCTAGTTC CAGACACCTC CCAAGCACGC AGCAATGCAG CTCAAAACGC TTAGCCTAGC
                          FI Element
```

```
      4060       4070       4080       4090       4100       4110
CACACCCCCA CGGGAAACAG CAGTGATTAA CCTTTAGCAA TAAACGAAAG TTTAACTAAG
                        FI Element

      4120       4130       4140       4150       4160 4164
CTATACTAAC CCCAGGGTTG GTCAATTTCG TGCCAGCCAC ACCCTGGAGC TAGC
                        FI Element

      4174       4184       4194       4204       4214       4224
AAAAAAAAAA AAAAAAAAAA AAAAAAAAAA GCATATGACT AAAAAAAAAA AAAAAAAAAA
                        A30L70

      4234       4244       4254       4264       4274
AAAAAAAAAA AAAAAAAAAA AAAAAAAAAA AAAAAAAAAA AAAAAAAAAA
                        A30L70
```

Sequences of RBP020.11 are also shown in Table 3.

**Table 3:** Sequences of RBP020.11 a Beta-specific RNA vaccine

| SEQ ID NO. | Brief Description | Sequence |
|---|---|---|
| 22 | Amino acid sequence of RNA-encoded SARS-CoV-2 S protein from a Beta variant | MFVFLVLLPLVSSQCVNFTTRTQLPPAYTNSFTRGVYYPDKVFRSSVLHSTQDLFLPFFSNVTWFH<br>AIHVSGTNGTKRFANPVLPFNDGVYFASTEKSNIIRGWIFGTTLDSKTQSLLIVNNATNVVIKVCE<br>FQFCNDPFLGVYYHKNNKSWMESEFRVYSSANNCTFEYVSQPFLMDLEGKQGNFKNLREFVFKNID<br>GYFKIYSKHTPINLVRGLPQGFSALEPLVDLPIGINITRFQTLHISYLTPGDSSSGWTAGAAAYYV<br>GYLQPRTFLLKYNENGTITDAVDCALDPLSETKCTLKSFTVEKGIYQTSNFRVQPTESIVRFPNIT<br>NLCPFGEVFNATRFASVYAWNRKRISNCVADYSVLYNSASFSTFKCYGVSPTKLNDLCFTNVYADS<br>FVIRGDEVRQIAPGQTGNIADYNYKLPDDFTGCVIAWNSNNLDSKVGGNYNYLYRLFRKSNLKPFE<br>RDISTEIYQAGSTPCNGVKGFNCYFPLQSYGFQPTYGVGYQPYRVVVLSFELLHAPATVCGPKKST<br>NLVKNKCVNFNFNGLTGTGVLTESNKKFLPFQQFGRDIADTTDAVRDPQTLEILDITPCSFGGVSV<br>ITPGTNTSNQVAVLYQGVNCTEVPVAIHADQLTPTWRVYSTGSNVFQTRAGCLIGAEHVNNSYECD<br>IPIGAGICASYQTQTNSPRRARSVASQSIIAYTMSLGVENSVAYSNNSIAIPTNFTISVTTEILPV<br>SMTKTSVDCTMYICGDSTECSNLLLQYGSFCTQLNRALTGIAVEQDKNTQEVFAQVKQIYKTPPIK<br>DFGGFNFSQILPDPSKPSKRSFIEDLLFNKVTLADAGFIKQYGDCLGDIAARDLICAQKFNGLTVL<br>PPLLTDEMIAQYTSALLAGTITSGWTFGAGAALQIPFAMQMAYRFNGIGVTQNVLYENQKLIANQF<br>NSAIGKIQDSLSSTASALGKLQDVVNQNAQALNTLVKQLSSNFGAISSVLNDILSRLDPPEAEVQI<br>DRLITGRLQSLQTYVTQQLIRAAEIRASANLAATKMSECVLGQSKRVDFCGKGYHLMSFPQSAPHG<br>VVFLHVTYVPAQEKNFTTAPAICHDGKAHFPREGVFVSNGTHWFVTQRNFYEPQIITTDNTFVSGN<br>CDVVIGIVNNTVYDPLQPELDSFKEELDKYFKNHTSPDVDLGDISGINASVVNIQKEIDRLNEVAK<br>NLNESLIDLQELGKYEQYIKWPWYIWLGFIAGLIAIVMVTIMLCCMTSCCSCLKGCCSCGSCCKFD<br>EDDSEPVLKGVKLHYT** |
| 23 | RNA sequence encoding a SARS-CoV-2 S protein from a Beta variant | auguucgugu uccuggugcu gcugccucug guguccagcc agugugugaa cuucaccacc<br>agaacacagc ugccuccagc cuacaccaac agcuuuacca gaggcgugua cuaccccgac<br>aagguguuca gauccagcgu gcugcacucu acccaggacc uguuccugcc uuucuucagc<br>aacgugaccu gguuccacgc cauccacgug uccggcacca auggcaccaa gagauucgcc<br>aaccccgugc ugcccuucaa cgacggggug uacuuugcca gcaccgagaa guccaacauc<br>aucagaggcu ggaucuucgg caccacacug gacagcaaga cccagagccu gcugaucgug<br>aacaacgcca ccaacguggu caucaaagug ugcgaguucc aguucugcaa cgaccccuuc<br>cugggcgucu acuaccacaa gaacaacaag agcuggaugg aaagcgaguu ccggguguac<br>agcagcgcca acaacugcac cuucgaguac gugucccagc cuuuccugau ggaccuggaa<br>ggcaagcagg gcaacuucaa gaaccugcgc gaguucgugu uuaagaacau cgacggcuac<br>uucaagaucu acagcaagca caccccuauc aaccucgugc ggggccugcc ucagggcuuc<br>ucugcucugg aaccccuggu ggaucugccc aucggcauca acaucacccg guuucagaca<br>cugcacauca gcuaccugac accuggcgau agcagcagcg gauggacagc uggugccgcc |

(continued)

| SEQ ID NO. | Brief Description | Sequence |
|---|---|---|
| | | gcuuacuaug ugggcuaccu gcagccuaga accuuccugc ugaaguacaa cgagaacggc accaucaccg acgccgugga uugugcucug gauccucuga gcgagacaaa gugcacccug aaguccuuca ccguggaaaa gggcaucuac cagaccagca acuuccgggu gcagcccacc gaauccaucg ugcgguuccc caauaucacc aaucugugcc ccuucggcga gguguucaau gccaccagau ucgccucugu guacgccugg aaccggaagc ggaucagcaa uugcguggcc gacuacuccg ugcuguacaa cuccgccagc uucagcaccu ucaagugcua cggcgugucc ccuaccaagc ugaacgaccu gugcuucaca aacguguacg ccgacagcuu cgugauccgg ggagaugaag ugcggcagau ugccccugga cagacaggca acaucgccga cuacaacuac aagcugcccg acgacuucac cggcugugug auugccugga acagcaacaa ccuggacucc aaagucggcg gcaacuacaa uuaccuguac cggcuguucc ggaaguccaa ucugaagccc uucgagcggg acaucuccac cgagaucuau caggccggca gcaccccuug uaacggcgug aagggcuuca acugcuacuu cccacugcag uccuacggcu uucagcccac auacggcgug ggcuaucagc ccuacagagu gguggugcug agcuucgaac ugcugcaugc cccugccaca gugugcggcc cuaagaaaag caccaaucuc gugaagaaca aaugcgugaa cuucaacuuc aacggccuga ccggcaccgg cgugcugaca gagagcaaca agaaguuccu gccauuccag caguuuggcc gggauaucgc cgauaccaca gacgccguua gagaucccca gacacuggaa auccuggaca ucaccccuug cagcuucggc ggagugucug ugaucacccc uggcaccaac accagcaauc agguggcagu gcuguaccag ggcgugaacu guaccgaagu gcccguggcc auucacgccg aucagcugac accuacaugg cggguguacu ccaccggcag caauguguuu cagaccagag ccggcugucu gaucggagcc gagcacguga acaauagcua cgagugcgac auccccaucg gcgcuggaau cugcgccagc uaccagacac agacaaacag cccucggaga gccagaagcg uggccagcca gagcaucauu gccuacacaa ugucucuggg cgucgagaac agcguggccu acuccaacaa cucuaucgcu auccccacca acuucaccau cagcgugacc acagagaucc ugccuguguc caugaccaag accagcgugg acugcaccau guacaucugc ggcgauucca ccgagugcuc caaccugcug cugcaguacg gcagcuucug cacccagcug aauagagccc ugacagggau cgccguggaa caggacaaga acacccaaga gguguucgcc caagugaagc agaucuacaa gaccccuccu aucaaggacu ucggcggcuu caauuucagc cagauucugc ccgauccuag caagcccagc aagcggagcu ucaucgagga ccugcuguuc aacaaaguga cacuggccga cgccggcuuc aucaagcagu auggcgauug ucugggcgac auugccgcca gggaucugau uugcgcccag aaguuuaacg gacugacagu gcugccuccu cugcugaccg augagaugau cgcccaguac acaucugccc ugcuggccgg cacaaucaca agcggcugga cauuuggagc aggcgccgcu cugcagaucc ccuuugcuau gcagauggcc uaccgguuca acggcaucgg agugacccag aaugugcugu acgagaacca gaagcugauc gccaaccagu ucaacagcgc caucggcaag auccaggaca gccugagcag cacagcaagc gcccugggaa agcugcagga cguggucaac cagaaugccc aggcacugaa cacccugguc aagcagcugu ccuccaacuu cggcgccauc agcucugugc ugaacgauau ccugagcaga |
| | | cuggacccuc cugaggccga ggugcagauc gacagacuga ucacaggcag acugcagagc cuccagacau acgugaccca gcagcugauc agagccgccg agauuagagc cucugccaau cuggccgcca ccaagauguc ugagugugug cugggccaga gcaagagagu ggacuuuugc ggcaagggcu accaccugau gagcuucccu cagucugccc cucacggcgu ggugguuucug cacgugacau augugcccgc ucaagagaag aauuucacca ccgcuccagc caucugccac gacggcaaag cccacuuucc uagagaaggc guguucgugu ccaacggcac ccauugguuc gugacacagc ggaacuucua cgagccccag aucaucacca ccgacaacac cuucgugucu ggcaacugcg acgucgugau cggcauugug aacaauaccg uguacgaccc ucugcagccc gagcuggaca gcuucaaaga ggaacuggac aaguacuuua agaaccacac aagccccgac guggaccugg gcgauaucag cggaaucaau gccagcgucg ugaacaucca gaaagagauc gaccggcuga acgagguggc caagaaucug aacgagagcc ugaucgaccu gcaagaacug ggaaguacg agcaguacau caaguggccc ugguacaucu ggcugggcuu uaucgccgga cugauugcca ucgugauggu cacaaucaug cuguguugca ugaccagcug cuguagcugc cugaagggcu guuguagcug uggcagcugc ugcaaguucg acgaggacga uucugagccc gugcugaagg gcgugaaacu gcacuacaca ugaugac |

(continued)

| SEQ ID NO. | Brief Description | Sequence |
|---|---|---|
| 24 | Full length RNA sequence of RBP020.11 | agaauaaacu aguauucuuc ugguccccac agacucagag agaacccgcc acc<br>auguucgugu uccuggugcu gcugccucug guguccagcc agugugugaa cuucaccacc<br>agaacacagc ugccuccagc cuacaccaac agcuuuacca gaggcgugua cuaccccgac<br>aagguguuca gauccagcgu gcugcacucu acccaggacc uguuccugcc uuucuucagc<br>aacgugaccu gguuccacgc cauccacgug uccggcacca auggcaccaa gagauucgcc<br>aaccccgugc ugcccuucaa cgacggggug uacuuugcca gcaccgagaa guccaacauc<br>aucagaggcu ggaucuucgg caccacacug gacagcaaga cccagagccu gcugaucgug<br>aacaacgcca ccaacguggu caucaaagug ugcgaguucc aguucugcaa cgaccccuuc<br>cugggcgucu acuaccacaa gaacaacaag agcuggaugg aaagcgaguu ccggguguac<br>agcagcgcca acaacugcac cuucgaguac gugucccagc cuuuccugau ggaccuggaa<br>ggcaagcagg gcaacuucaa gaaccugcgc gaguucgugu uuaagaacau cgacggcuac<br>uucaagaucu acagcaagca caccccuauc aaccucgugc ggggccugcc ucagggcuuc<br>ucugcucugg aaccccuggu ggaucugccc aucggcauca acaucacccg guuucagaca<br>cugcacauca gcuaccugac accuggcgau agcagcagcg gauggacagc uggugccgcc<br>gcuuacuaug ugggcuaccu gcagccuaga accuuccugc ugaaguacaa cgagaacggc<br>accaucaccg acgccgugga uugugcucug gauccucuga gcgagacaaa gugcacccug<br>aaguccuuca ccguggaaaa gggcaucuac cagaccagca acuuccgggu gcagcccacc<br>gaauccaucg ugcgguuccc caauaucacc aaucugugcc ccuucggcga gguguucaau<br>gccaccagau ucgccucugu guacgccugg aaccggaagc ggaucagcaa uugcguggcc<br>gacuacuccg ugcuguacaa cuccgccagc uucagcaccu ucaagugcua cggcgugucc<br>ccuaccaagc ugaacgaccu gugcuucaca aacguguacg ccgacagcuu cgugauccgg |
| | | ggagaugaag ugcggcagau ugccccugga cagacaggca acaucgccga cuacaacuac<br>aagcugcccg acgacuucac cggcugugug auugccugga acagcaacaa ccuggacucc<br>aaagucggcg gcaacuacaa uuaccuguac cggcuguucc ggaaguccaa ucugaagccc<br>uucgagcggg acaucuccac cgagaucuau caggccggca gcaccccuug uaacggcgug<br>aagggcuuca acugcuacuu cccacugcag uccuacggcu uucagcccac auacggcgug<br>ggcuaucagc ccuacagagu gguggugcug agcuucgaac ugcugcaugc cccugccaca<br>gugugcggcc cuaagaaaag caccaaucuc gugaagaaca aaugcgugaa cuucaacuuc<br>aacggccuga ccggcaccgg cgugcugaca gagagcaaca agaaguuccu gccauuccag<br>caguuuggcc gggauaucgc cgauaccaca gacgccguua gagaucccca gacacuggaa<br>auccuggaca ucaccccuug cagcuucggc ggagugucug ugaucacccc uggcaccaac<br>accagcaauc agguggcagu gcuguaccag ggcgugaacu guaccgaagu gcccguggcc<br>auucacgccg aucagcugac accuacaugg cggguguacu ccaccggcag caauguguuu<br>cagaccagag ccggcugucu gaucggagcc gagcacguga acaauagcua cgagugcgac<br>auccccaucg gcgcuggaau cugcgccagc uaccagacac agacaaacag cccucggaga<br>gccagaagcg uggccagcca gagcaucauu gccuacacaa ugucucuggg cgucgagaac<br>agcguggccu acuccaacaa cucuaucgcu auccccacca acuucaccau cagcgugacc<br>acagagaucc ugccuguguc caugaccaag accagcgugg acugcaccau guacaucugc<br>ggcgauucca ccgagugcuc caaccugcug cugcaguacg gcagcuucug cacccagcug<br>aauagagccc ugacagggau cgccguggaa caggacaaga acacccaaga gguguucgcc<br>caagugaagc agaucuacaa gaccccuccu aucaaggacu ucggcggcuu caauuucagc<br>cagauucugc ccgauccuag caagcccagc aagcggagcu ucaucgagga ccugcuguuc<br>aacaaaguga cacuggccga cgccggcuuc aucaagcagu auggcgauug ucugggcgac<br>auugccgcca gggaucugau uugcgcccag aaguuuaacg gacugacagu gcugccuccu<br>cugcugaccg augagaugau cgcccaguac acaucugccc ugcuggccgg cacaaucaca<br>agcggcugga cauuuggagc aggcgccgcu cugcagaucc ccuuugcuau gcagauggcc<br>uaccgguuca acggcaucgg agugacccag aaugugcugu acgagaacca gaagcugauc<br>gccaaccagu ucaacagcgc caucggcaag auccaggaca gccugagcag cacagcaagc<br>gcccugggaa agcugcagga cguggucaac cagaaugccc aggcacugaa cacccugguc<br>aagcagcugu ccuccaacuu cggcgccauc agcucugugc ugaacgauau ccugagcaga<br>cuggacccuc cugaggccga ggugcagauc gacagacuga ucacaggcag acugcagagc<br>cuccagacau acgugaccca gcagcugauc agagccgccg agauuagagc cucugccaau<br>cuggccgcca ccaagauguc ugagugugug cugggccaga gcaagagagu ggacuuuugc<br>ggcaagggcu accaccugau gagcuucccu cagucugccc cucacggcgu gguguuucug<br>cacgugacau augugcccgc ucaagagaag aauuucacca ccgcuccagc caucugccac<br>gacggcaaag cccacuuucc uagagaaggc guguucgugu ccaacggcac ccauugguuc<br>gugacacagc ggaacuucua cgagccccag aucaucacca ccgacaacac cuucgugucu |

(continued)

| SEQ ID NO. | Brief Description | Sequence |
|---|---|---|
| | | ggcaacugcg acgucgugau cggcauugug aacaauaccg uguacgaccc ucugcagccc gagcuggaca gcuucaaaga ggaacuggac aaguacuuua agaaccacac aagccccgac guggaccugg gcgauaucag cggaaucaau gccagcgucg ugaacaucca gaaagagauc gaccggcuga acgagguggc caagaaucug aacgagagcc ugaucgaccu gcaagaacug gggaaguacg agcaguacau caaguggccc ugguacaucu ggcugggcuu uaucgccgga cugauugcca ucgugauggu cacaaucaug cuguguugca ugaccagcug cuguagcugc cugaagggcu guuguagcug uggcagcugc ugcaaguucg acgaggacga uucugagccc gugcugaagg gcgugaaacu gcacuacaca ugaugac ucgagcuggu acugcaugca cgcaaugcua gcugccccuu ucccguccug gguaccccga gucucccccg accucggguc ccagguaugc ucccaccucc accugcccca cucaccaccu cugcuaguuc cagacaccuc ccaagcacgc agcaaugcag cucaaaacgc uuagccuagc cacaccccca cgggaaacag cagugauuaa ccuuuagcaa uaaacgaaag uuuaacuaag cuauacuaac cccaggguug gucaauuucg ugccagccac acccuggagc uagc aaaaaaaaaa aaaaaaaaaa aaaaaaaaaa gcauaugacu aaaaaaaaaa aaaaaaaaaa aaaaaaaaaa aaaaaaaaaa aaaaaaaaaa aaaaaaaaaa aaaaaaaaaa |

**Nucleotide Sequence of RBP020.14 (Alpha-specific vaccine)**

Nucleotide sequence is shown with individual sequence elements as indicated in bold letters. In addition, the sequence of the translated protein is shown in italic letters below the coding nucleotide sequence (* = stop codon). Red text indicates point mutations in both the nucleotide and amino acid sequences.

```
         10         20         30         40          50  53
AGAAUAAACU AGUAUUCUUC UGGUCCCCAC AGACUCAGAG AGAACCCGCC ACC
```

**hAg-Kozak**

```
        63         73         83         93        103        113
AUGUUCGUGU UCCUGGUGCU GCUGCCUCUG GUGUCCAGCC AGUGUGUGAA CCUGACCACC
 M  F  V   F  L  V   L  L  P  L   V  S  S   Q  C  V   N  L  T  T
```

**S protein mut4**

```
       123        133        143        153        163        173
AGAACACAGC UGCCUCCAGC CUACACCAAC AGCUUUACCA GAGGCGUGUA CUACCCCGAC
 R  T  Q   L  P  P   A  Y  T  N   S  F  T   R  G  V   Y  Y  P  D
```

**S protein mut3**

```
       183        193        203        213        223        233
AAGGUGUUCA GAUCCAGCGU GCUGCACUCU ACCCAGGACC UGUUCCUGCC UUUCUUCAGC
 K  V  F   R  S  S   V  L  H  S   T  Q  D   L  F  L   P  F  F  S
```

**S protein mut3**

```
       243        253        263        273        283        293
AACGUGACCU GGUUCCACGC CAUCUCCGGC ACCAAUGGCA CCAAGAGAUU CGACAACCCC
 N  V  T   W  F  H   A  I  S  G   T  N  G   T  K  R   F  D  N  P
```

**S protein mut3**

```
       303        313        323        333        343        353
GUGCUGCCCU UCAACGACGG GGUGUACUUU GCCAGCACCG AGAAGUCCAA CAUCAUCAGA
 V  L  P   F  N  D   G  V  Y  F   A  S  T   E  K  S   N  I  I  R
```

**S protein mut3**

```
       363        373        383        393        403        413
GGCUGGAUCU UCGGCACCAC ACUGGACAGC AAGACCCAGA GCCUGCUGAU CGUGAACAAC
 G  W  I   F  G  T   T  L  D  S   K  T  Q   S  L  L   I  V  N  N
```

**S protein mut3**

```
       423        433        443        453        463        473
GCCACCAACG UGGUCAUCAA AGUGUGCGAG UUCCAGUUCU GCAACGACCC CUUCCUGGGC
 A  T  N   V  V  I   K  V  C  E   F  Q  F   C  N  D   P  F  L  G
```

**S protein mut3**

```
       483        493        503        513        523        533
GUCUACCACA AGAACAACAA GAGCUGGAUG GAAAGCGAGU UCCGGGUGUA CAGCAGCGCC
 V  Y  H   K  N  N   K  S  W  M   E  S  E   F  R  V   Y  S  S  A
```

**S protein mut3**

```
       543        553        563        573        583        593
AACAACUGCA CCUUCGAGUA CGUGUCCCAG CCUUUCCUGA UGGACCUGGA AGGCAAGCAG
 N  N  C   T  F  E   Y  V  S  Q   P  F  L   M  D  L   E  G  K  Q
```
**S protein mut3**

```
       603        613        623        633        643        653
GGCAACUUCA AGAACCUGCG CGAGUUCGUG UUUAAGAACA UCGACGGCUA CUUCAAGAUC
 G  N  F   K  N  L   R  E  F  V   F  K  N   I  D  G   Y  F  K  I
```
**S protein mut3**

```
       663        673        683        693        703        713
UACAGCAAGC ACACCCCUAU CAACCUCGUG CGGGAUCUGC CUCAGGGCUU CUCUGCUCUG
 Y  S  K   H  T  P   I  N  L  V   R  D  L   P  Q  G   F  S  A  L
```
**S protein mut3**

```
       723        733        743        753        763        773
GAACCCCUGG UGGAUCUGCC CAUCGGCAUC AACAUCACCC GGUUUCAGAC ACUGCUGGCC
 E  P  L   V  D  L   P  I  G  I   N  I  T   R  F  Q   T  L  L  A
```
**S protein mut3**

```
       783        793        803        813        823        833
CUGCACAGAA GCUACCUGAC ACCUGGCGAU AGCAGCAGCG GAUGGACAGC UGGUGCCGCC
 L  H  R   S  Y  L   T  P  G  D   S  S  S   G  W  T   A  G  A  A
```
**S protein mut3**

```
       843        853        863        873        883        893
GCUUACUAUG UGGGCUACCU GCAGCCUAGA ACCUUCCUGC UGAAGUACAA CGAGAACGGC
 A  Y  Y   V  G  Y   L  Q  P  R   T  F  L   L  K  Y   N  E  N  G
```
**S protein mut3**

```
       903        913        923        933        943        953
ACCAUCACCG ACGCCGUGGA UUGUGCUCUG GAUCCUCUGA GCGAGACAAA GUGCACCCUG
 T  I  T   D  A  V   D  C  A  L   D  P  L   S  E  T   K  C  T  L
```
**S protein mut3**

```
       963        973        983        993       1003       1013
AAGUCCUUCA CCGUGGAAAA GGGCAUCUAC CAGACCAGCA ACUUCCGGGU GCAGCCCACC
 K  S  F   T  V  E   K  G  I  Y   Q  T  S   N  F  R   V  Q  P  T
```
**S protein mut3**

```
      1023       1033       1043       1053       1063       1073
GAAUCCAUCG UGCGGUUCCC CAAUAUCACC AAUCUGUGCC CCUUCGGCGA GGUGUUCAAU
 E  S  I   V  R  F   P  N  I  T   N  L  C   P  F  G   E  V  F  N
```
**S protein mut3**

```
      1083       1093       1103       1113       1123       1133
GCCACCAGAU UCGCCUCUGU GUACGCCUGG AACCGGAAGC GGAUCAGCAA UUGCGUGGCC
 A  T  R   F  A  S   V  Y  A  W   N  R  K   R  I  S   N  C  V  A
```
**S protein mut3**

```
      1143       1153       1163       1173       1183       1193
GACUACUCCG UGCUGUACAA CUCCGCCAGC UUCAGCACCU UCAAGUGCUA CGGCGUGUCC
 D  Y  S   V  L  Y   N  S  A  S   F  S  T   F  K  C   Y  G  V  S
```
**S protein mut3**

```
      1203       1213       1223       1233       1243       1253
CCUACCAAGC UGAACGACCU GUGCUUCACA AACGUGUACG CCGACAGCUU CGUGAUCCGG
 P  T  K   L  N  D   L  C  F  T   N  V  Y   A  D  S   F  V  I  R
```
**S protein mut3**

```
      1263      1273      1283      1293      1303      1313
GGAGAUGAAG UGCGGCAGAU UGCCCCUGGA CAGACAGGCA AGAUCGCCGA CUACAACUAC
  G  D  E   V  R  Q   I  A  P  G   Q  T  G   K  I  A   D  Y  N  Y
```
**S protein mut3**

```
      1323      1333      1343      1353      1363      1373
AAGCUGCCCG ACGACUUCAC CGGCUGUGUG AUUGCCUGGA ACAGCAACAA CCUGGACUCC
  K  L  P   D  D  F   T  G  C  V   I  A  W   N  S  N   N  L  D  S
```
**S protein mut3**

```
      1383      1393      1403      1413      1423      1433
AAAGUCGGCG GCAACUACAA UUACCUGUAC CGGCUGUUCC GGAAGUCCAA UCUGAAGCCC
  K  V  G   G  N  Y   N  Y  L  Y   R  L  F   R  K  S   N  L  K  P
```
**S protein mut3**

```
      1443      1453      1463      1473      1483      1493
UUCGAGCGGG ACAUCUCCAC CGAGAUCUAU CAGGCCGGCA GCACCCCUUG UAACGGCGUG
  F  E  R   D  I  S   T  E  I  Y   Q  A  G   S  T  P   C  N  G  V
```
**S protein mut3**

```
      1503      1513      1523      1533      1543      1553
GAAGGCUUCA ACUGCUACUU CCCACUGCAG UCCUACGGCU UUCAGCCCAC AUACGGCGUG
  E  G  F   N  C  Y   F  P  L  Q   S  Y  G   F  Q  P   T  Y  G  V
```
**S protein mut3**

```
      1563      1573      1583      1593      1603      1613
GGCUAUCAGC CCUACAGAGU GGUGGUGCUG AGCUUCGAAC UGCUGCAUGC CCCUGCCACA
  G  Y  Q   P  Y  R   V  V  V  L   S  F  E   L  L  H   A  P  A  T
```
**S protein mut3**

```
      1623      1633      1643      1653      1663      1673
GUGUGCGGCC CUAAGAAAAG CACCAAUCUC GUGAAGAACA AAUGCGUGAA CUUCAACUUC
  V  C  G   P  K  K   S  T  N  L   V  K  N   K  C  V   N  F  N  F
```
**S protein mut3**

```
      1683      1693      1703      1713      1723      1733
AACGGCCUGA CCGGCACCGG CGUGCUGACA GAGAGCAACA AGAAGUUCCU GCCAUUCCAG
  N  G  L   T  G  T   G  V  L  T   E  S  N   K  K  F   L  P  F  Q
```
**S protein mut3**

```
      1743      1753      1763      1773      1783      1793
CAGUUUGGCC GGGAUAUCGA CGAUACCACA GACGCCGUUA GAGAUCCCCA GACACUGGAA
  Q  F  G   R  D  I   D  D  T  T   D  A  V   R  D  P   Q  T  L  E
```
**S protein mut3**

```
      1803      1813      1823      1833      1843      1853
AUCCUGGACA UCACCCCUUG CAGCUUCGGC GGAGUGUCUG UGAUCACCCC UGGCACCAAC
  I  L  D   I  T  P   C  S  F  G   G  V  S   V  I  T   P  G  T  N
```
**S protein mut3**

```
      1863      1873      1883      1893      1903      1913
ACCAGCAAUC AGGUGGCAGU GCUGUACCAG GGCGUGAACU GUACCGAAGU GCCCGUGGCC
  T  S  N   Q  V  A   V  L  Y  Q   G  V  N   C  T  E   V  P  V  A
```
**S protein mut3**

```
      1923      1933      1943      1953      1963      1973
AUUCACGCCG AUCAGCUGAC ACCUACAUGG CGGGUGUACU CCACCGGCAG CAAUGUGUUU
  I  H  A   D  Q  L   T  P  T  W   R  V  Y   S  T  G   S  N  V  F
```
**S protein mut3**

```
      1983       1993       2003       2013       2023       2033
CAGACCAGAG CCGGCUGUCU GAUCGGAGCC GAGCACGUGA ACAAUAGCUA CGAGUGCGAC
 Q  T  R   A  G  C   L  I  G  A   E  H  V   N  N  S   Y  E  C  D
```
**S protein mut3**

```
      2043       2053       2063       2073       2083       2093
AUCCCCAUCG GCGCUGGAAU CUGCGCCAGC UACCAGACAC AGACAAACAG CCACCGGAGA
 I  P  I   G  A  G   I  C  A  S   Y  Q  T   Q  T  N   S  H  R  R
```
**S protein mut3**

```
      2103       2113       2123       2133       2143       2153
GCCAGAAGCG UGGCCAGCCA GAGCAUCAUU GCCUACACAA UGUCUCUGGG CGCCGAGAAC
 A  R  S   V  A  S   Q  S  I  I   A  Y  T   M  S  L   G  A  E  N
```
**S protein mut3**

```
      2163       2173       2183       2193       2203       2213
AGCGUGGCCU ACUCCAACAA CUCUAUCGCU AUCCCCAUCA ACUUCACCAU CAGCGUGACC
 S  V  A   Y  S  N   N  S  I  A   I  P  I   N  F  T   I  S  V  T
```
**S protein mut3**

```
      2223       2233       2243       2253       2263       2273
ACAGAGAUCC UGCCUGUGUC CAUGACCAAG ACCAGCGUGG ACUGCACCAU GUACAUCUGC
 T  E  I   L  P  V   S  M  T  K   T  S  V   D  C  T   M  Y  I  C
```
**S protein mut3**

```
      2283       2293       2303       2313       2323       2333
GGCGAUUCCA CCGAGUGCUC CAACCUGCUG CUGCAGUACG GCAGCUUCUG CACCCAGCUG
 G  D  S   T  E  C   S  N  L  L   L  Q  Y   G  S  F   C  T  Q  L
```
**S protein mut3**

```
      2343       2353       2363       2373       2383       2393
AAUAGAGCCC UGACAGGGAU CGCCGUGGAA CAGGACAAGA ACACCCAAGA GGUGUUCGCC
 N  R  A   L  T  G   I  A  V  E   Q  D  K   N  T  Q   E  V  F  A
```
**S protein mut3**

```
      2403       2413       2423       2433       2443       2453
CAAGUGAAGC AGAUCUACAA GACCCCUCCU AUCAAGGACU UCGGCGGCUU CAAUUUCAGC
 Q  V  K   Q  I  Y   K  T  P  P   I  K  D   F  G  G   F  N  F  S
```
**S protein mut3**

```
      2463       2473       2483       2493       2503       2513
CAGAUUCUGC CCGAUCCUAG CAAGCCCAGC AAGCGGAGCU UCAUCGAGGA CCUGCUGUUC
 Q  I  L   P  D  P   S  K  P  S   K  R  S   F  I  E   D  L  L  F
```
**S protein mut3**

```
      2523       2533       2543       2553       2563       2573
AACAAAGUGA CACUGGCCGA CGCCGGCUUC AUCAAGCAGU AUGGCGAUUG UCUGGGCGAC
 N  K  V   T  L  A   D  A  G  F   I  K  Q   Y  G  D   C  L  G  D
```
**S protein mut3**

```
      2583       2593       2603       2613       2623       2633
AUUGCCGCCA GGGAUCUGAU UUGCGCCCAG AAGUUUAACG GACUGACAGU GCUGCCUCCU
 I  A  A   R  D  L   I  C  A  Q   K  F  N   G  L  T   V  L  P  P
```
**S protein mut3**

```
      2643       2653       2663       2673       2683       2693
CUGCUGACCG AUGAGAUGAU CGCCCAGUAC ACAUCUGCCC UGCUGGCCGG CACAAUCACA
 L  L  T   D  E  M   I  A  Q  Y   T  S  A   L  L  A   G  T  I  T
```
**S protein mut3**

```
        2703       2713       2723       2733       2743       2753
AGCGGCUGGA CAUUUGGAGC AGGCGCCGCU CUGCAGAUCC CCUUUGCUAU GCAGAUGGCC
 S  G  W   T  F  G   A  G  A  A   L  Q  I   P  F  A   M  Q  M  A
```
**S protein mut3**

```
        2763       2773       2783       2793       2803       2813
UACCGGUUCA ACGGCAUCGG AGUGACCCAG AAUGUGCUGU ACGAGAACCA GAAGCUGAUC
 Y  R  F   N  G  I   G  V  T  Q   N  V  L   Y  E  N   Q  K  L  I
```
**S protein mut3**

```
        2823       2833       2843       2853       2863       2873
GCCAACCAGU UCAACAGCGC CAUCGGCAAG AUCCAGGACA GCCUGAGCAG CACAGCAAGC
 A  N  Q   F  N  S   A  I  G  K   I  Q  D   S  L  S   S  T  A  S
```
**S protein mut3**

```
        2883       2893       2903       2913       2923       2933
GCCCUGGGAA AGCUGCAGGA CGUGGUCAAC CAGAAUGCCC AGGCACUGAA CACCCUGGUC
 A  L  G   K  L  Q   D  V  V  N   Q  N  A   Q  A  L   N  T  L  V
```
**S protein mut3**

```
        2943       2953       2963       2973       2983       2993
AAGCAGCUGU CCUCCAACUU CGGCGCCAUC AGCUCUGUGC UGAACGAUAU CCUGGCCAGA
 K  Q  L   S  S  N   F  G  A  I   S  S  V   L  N  D   I  L  A  R
```
**S protein mut3**

```
        3003       3013       3023       3033       3043       3053
CUGGACCCUC CUGAGGCCGA GGUGCAGAUC GACAGACUGA UCACAGGCAG ACUGCAGAGC
 L  D  P   P  E  A   E  V  Q  I   D  R  L   I  T  G   R  L  Q  S
```
**S protein mut3**

```
        3063       3073       3083       3093       3103       3113
CUCCAGACAU ACGUGACCCA GCAGCUGAUC AGAGCCGCCG AGAUUAGAGC CUCUGCCAAU
 L  Q  T   Y  V  T   Q  Q  L  I   R  A  A   E  I  R   A  S  A  N
```
**S protein mut3**

```
        3123       3133       3143       3153       3163       3173
CUGGCCGCCA CCAAGAUGUC UGAGUGUGUG CUGGGCCAGA GCAAGAGAGU GGACUUUUGC
 L  A  A   T  K  M   S  E  C  V   L  G  Q   S  K  R   V  D  F  C
```
**S protein mut3**

```
        3183       3193       3203       3213       3223       3233
GGCAAGGGCU ACCACCUGAU GAGCUUCCCU CAGUCUGCCC CUCACGGCGU GGUGUUUCUG
 G  K  G   Y  H  L   M  S  F  P   Q  S  A   P  H  G   V  V  F  L
```
**S protein mut3**

```
        3243       3253       3263       3273       3283       3293
CACGUGACAU AUGUGCCCGC UCAAGAGAAG AAUUUCACCA CCGCUCCAGC CAUCUGCCAC
 H  V  T   Y  V  P   A  Q  E  K   N  F  T   T  A  P   A  I  C  H
```
**S protein mut3**

```
        3303       3313       3323       3333       3343       3353
GACGGCAAAG CCCACUUUCC UAGAGAAGGC GUGUUCGUGU CCAACGGCAC CCAUUGGUUC
 D  G  K   A  H  F   P  R  E  G   V  F  V   S  N  G   T  H  W  F
```
**S protein mut3**

```
        3363       3373       3383       3393       3403       3413
GUGACACAGC GGAACUUCUA CGAGCCCCAG AUCAUCACCA CCCACAACAC CUUCGUGUCU
 V  T  Q   R  N  F   Y  E  P  Q   I  I  T   T  H  N   T  F  V  S
```
**S protein mut3**

```
      3423       3433       3443       3453       3463       3473
GGCAACUGCG ACGUCGUGAU CGGCAUUGUG AACAAUACCG UGUACGACCC UCUGCAGCCC
  G  N  C   D  V  V   I  G  I  V  N  N  T   V  Y  D   P  L  Q  P
```

**S protein mut3**

```
      3483       3493       3503       3513       3523       3533
GAGCUGGACA GCUUCAAAGA GGAACUGGAC AAGUACUUUA AGAACCACAC AAGCCCCGAC
  E  L  D   S  F  K   E  E  L  D  K  Y  F   K  N  H   T  S  P  D
```

**S protein mut3**

```
      3543       3553       3563       3573       3583       3593
GUGGACCUGG GCGAUAUCAG CGGAAUCAAU GCCAGCGUCG UGAACAUCCA GAAAGAGAUC
  V  D  L   G  D  I   S  G  I  N  A  S  V   V  N  I   Q  K  E  I
```

**S protein mut3**

```
      3603       3613       3623       3633       3643       3653
GACCGGCUGA ACGAGGUGGC CAAGAAUCUG AACGAGAGCC UGAUCGACCU GCAAGAACUG
  D  R  L   N  E  V   A  K  N  L  N  E  S   L  I  D   L  Q  E  L
```

**S protein mut3**

```
      3663       3673       3683       3693       3703       3713
GGGAAGUACG AGCAGUACAU CAAGUGGCCC UGGUACAUCU GGCUGGGCUU UAUCGCCGGA
  G  K  Y   E  Q  Y   I  K  W  P  W  Y  I   W  L  G   F  I  A  G
```

**S protein mut3**

```
      3723       3733       3743       3753       3763       3773
CUGAUUGCCA UCGUGAUGGU CACAAUCAUG CUGUGUUGCA UGACCAGCUG CUGUAGCUGC
  L  I  A   I  V  M   V  T  I  M  L  C  C   M  T  S   C  C  S  C
```

**S protein mut3**

```
      3783       3793       3803       3813       3823       3833
CUGAAGGGCU GUUGUAGCUG UGGCAGCUGC UGCAAGUUCG ACGAGGACGA UUCUGAGCCC
  L  K  G   C  C  S   C  G  S  C  C  K  F   D  E  D   D  S  E  P
```

**S protein mut3**

```
      3843       3853       3863    3869
GUGCUGAAGG GCGUGAAACU GCACUACACA UGAUGA
  V  L  K   G  V  K   L  H  Y  T  *  *
```

**S protein mut3**

```
      3879       3889       3899       3909       3919       3929
GAUCUGCUGG UACUGCAUGC ACGCAAUGCU AGCUGCCCCU UUCCCGUCCU GGGUACCCCG
```

**FI element**

```
      3939       3949       3959       3969       3979       3989
AGUCUCCCCC GACCUCGGGU CCCAGGUAUG CUCCCACCUC CACCUGCCCC ACUCACCACC
```

**FI element**

```
      3999       4009       4019       4029       4039       4049
UCUGCUAGUU CCAGACACCU CCCAAGCACG CAGCAAUGCA GCUCAAAACG CUUAGCCUAG
```

**FI element**

```
      4059       4069       4079       4089       4099       4109
CCACACCCCC ACGGGAAACA GCAGUGAUUA ACCUUUAGCA AUAAACGAAA GUUUAACUAA
```

**FI element**

```
        4119       4129       4139       4149       4159  4164
GCUAUACUAA CCCCAGGGUU GGUCAAUUUC GUGCCAGCCA CACCCUGGAG CUAGC
```

**FI element**

```
        4174       4184       4194       4204       4214       4224
AAAAAAAAAA AAAAAAAAAA AAAAAAAAAA GCAUAUGACU AAAAAAAAAA AAAAAAAAAA
```

**Poly(A)**

```
        4234       4244       4254       4264       4274
AAAAAAAAAA AAAAAAAAAA AAAAAAAAAA AAAAAAAAAA AAAAAAAAAA
```

**Poly(A)**

Sequences of RBP020.14 are also shown in Table 4.

**Table 4:** Sequences of RBP020.14 (Alpha-specific RNA vaccine)

| SEQ ID NO. | Brief Description | Sequence |
|---|---|---|
| 25 | Amino acid sequence of RNA-encoded SARS-CoV-2 S protein from an Alpha variant | MFVFLVLLPLVSSQCVNLTTRTQLPPAYTNSFTRGVYYPDKVFRSSVLHSTQDLFLPFFSNVTWF<br>HAISGTNGTKRFDNPVLPFNDGVYFASTEKSNIIRGWIFGTTLDSKTQSLLIVNNATNVVIKVCE<br>FQFCNDPFLGVYHKNNKSWMESEFRVYSSANNCTFEYVSQPFLMDLEGKQGNFKNLREFVFKNID<br>GYFKIYSKHTPINLVRDLPQGFSALEPLVDLPIGINITRFQTLLALHRSYLTPGDSSSGWTAGAA<br>AYYVGYLQPRTFLLKYNENGTITDAVDCALDPLSETKCTLKSFTVEKGIYQTSNFRVQPTESIVR<br>FPNITNLCPFGEVFNATRFASVYAWNRKRISNCVADYSVLYNSASFSTFKCYGVSPTKLNDLCFT<br>NVYADSFVIRGDEVRQIAPGQTGKIADYNYKLPDDFTGCVIAWNSNNLDSKVGGNYNYLYRLFRK<br>SNLKPFERDISTEIYQAGSTPCNGVEGFNCYFPLQSYGFQPTYGVGYQPYRVVVLSFELLHAPAT<br>VCGPKKSTNLVKNKCVNFNFNGLTGTGVLTESNKKFLPFQQFGRDIDDTTDAVRDPQTLEILDIT<br>PCSFGGVSVITPGTNTSNQVAVLYQGVNCTEVPVAIHADQLTPTWRVYSTGSNVFQTRAGCLIGA<br>EHVNNSYECDIPIGAGICASYQTQTNSHRRARSVASQSIIAYTMSLGAENSVAYSNNSIAIPINF<br>TISVTTEILPVSMTKTSVDCTMYICGDSTECSNLLLQYGSFCTQLNRALTGIAVEQDKNTQEVFA<br>QVKQIYKTPPIKDFGGFNFSQILPDPSKPSKRSFIEDLLFNKVTLADAGFIKQYGDCLGDIAARD<br>LICAQKFNGLTVLPPLLTDEMIAQYTSALLAGTITSGWTFGAGAALQIPFAMQMAYRFNGIGVTQ<br>NVLYENQKLIANQFNSAIGKIQDSLSSTASALGKLQDVVNQNAQALNTLVKQLSSNFGAISSVLN<br>DILARLDPPEAEVQIDRLITGRLQSLQTYVTQQLIRAAEIRASANLAATKMSECVLGQSKRVDFC<br>GKGYHLMSFPQSAPHGVVFLHVTYVPAQEKNFTTAPAICHDGKAHFPREGVFVSNGTHWFVTQRN<br>FYEPQIITTHNTFVSGNCDVVIGIVNNTVYDPLQPELDSFKEELDKYFKNHTSPDVDLGDISGIN<br>ASVVNIQKEIDRLNEVAKNLNESLIDLQELGKYEQYIKWPWYIWLGFIAGLIAIVMVTIMLCCMT<br>SCCSCLKGCCSCGSCCKFDEDDSEPVLKGVKLHYT** |
| 26 | RNA sequence encoding a SARS-CoV-2 S protein from a Alpha variant | AUGUUCGUGU UCCUGGUGCU GCUGCCUCUG GUGUCCAGCC AGUGUGUGAA CCUGACCACC<br>AGAACACAGC UGCCUCCAGC CUACACCAAC AGCUUUACCA GAGGCGUGUA CUACCCCGAC<br>AAGGUGUUCA GAUCCAGCGU GCUGCACUCU ACCCAGGACC UGUUCCUGCC UUUCUUCAGC<br>AACGUGACCU GGUUCCACGC CAUCUCCGGC ACCAAUGGCA CCAAGAGAUU CGACAACCCC<br>GUGCUGCCCU UCAACGACGG GGUGUACUUU GCCAGCACCG AGAAGUCCAA CAUCAUCAGA<br>GGCUGGAUCU UCGGCACCAC ACUGGACAGC AAGACCCAGA GCCUGCUGAU CGUGAACAAC<br>GCCACCAACG UGGUCAUCAA AGUGUGCGAG UUCCAGUUCU GCAACGACCC CUUCCUGGGC<br>GUCUACCACA AGAACAACAA GAGCUGGAUG GAAAGCGAGU UCCGGGUGUA CAGCAGCGCC<br>AACAACUGCA CCUUCGAGUA CGUGUCCCAG CCUUUCCUGA UGGACCUGGA AGGCAAGCAG<br>GGCAACUUCA AGAACCUGCG CGAGUUCGUG UUUAAGAACA UCGACGGCUA CUUCAAGAUC<br>UACAGCAAGC ACACCCCUAU CAACCUCGUG CGGGAUCUGC CUCAGGGCUU CUCUGCUCUG<br>GAACCCCUGG UGGAUCUGCC CAUCGGCAUC AACAUCACCC GGUUUCAGAC ACUGCUGGCC<br>CUGCACAGAA GCUACCUGAC ACCUGGCGAU AGCAGCAGCG GAUGGACAGC UGGUGCCGCC<br>GCUUACUAUG UGGGCUACCU GCAGCCUAGA ACCUUCCUGC UGAAGUACAA CGAGAACGGC<br>ACCAUCACCG ACGCCGUGGA UUGUGCUCUG GAUCCUCUGA GCGAGACAAA GUGCACCCUG |

(continued)

| SEQ ID NO. | Brief Description | Sequence |
|---|---|---|
| | | AAGUCCUUCA CCGUGGAAAA GGGCAUCUAC CAGACCAGCA ACUUCCGGGU GCAGCCCACC GAAUCCAUCG UGCGGUUCCC CAAUAUCACC AAUCUGUGCC CCUUCGGCGA GGUGUUCAAU GCCACCAGAU UCGCCUCUGU GUACGCCUGG AACCGGAAGC GGAUCAGCAA UUGCGUGGCC GACUACUCCG UGCUGUACAA CUCCGCCAGC UUCAGCACCU UCAAGUGCUA CGGCGUGUCC CCUACCAAGC UGAACGACCU GUGCUUCACA AACGUGUACG CCGACAGCUU CGUGAUCCGG GGAGAUGAAG UGCGGCAGAU UGCCCCUGGA CAGACAGGCA AGAUCGCCGA CUACAACUAC AAGCUGCCCG ACGACUUCAC CGGCUGUGUG AUUGCCUGGA ACAGCAACAA CCUGGACUCC AAAGUCGGCG GCAACUACAA UUACCUGUAC CGGCUGUUCC GGAAGUCCAA UCUGAAGCCC UUCGAGCGGG ACAUCUCCAC CGAGAUCUAU CAGGCCGGCA GCACCCCUUG UAACGGCGUG GAAGGCUUCA ACUGCUACUU CCCACUGCAG UCCUACGGCU UUCAGCCCAC AUACGGCGUG GGCUAUCAGC CCUACAGAGU GGUGGUGCUG AGCUUCGAAC UGCUGCAUGC CCCUGCCACA GUGUGCGGCC CUAAGAAAAG CACCAAUCUC GUGAAGAACA AAUGCGUGAA CUUCAACUUC AACGGCCUGA CCGGCACCGG CGUGCUGACA GAGAGCAACA AGAAGUUCCU GCCAUUCCAG CAGUUUGGCC GGGAUAUCGA CGAUACCACA GACGCCGUUA GAGAUCCCCA GACACUGGAA AUCCUGGACA UCACCCCUUG CAGCUUCGGC GGAGUGUCUG UGAUCACCCC UGGCACCAAC ACCAGCAAUC AGGUGGCAGU GCUGUACCAG GGCGUGAACU GUACCGAAGU GCCCGUGGCC AUUCACGCCG AUCAGCUGAC ACCUACAUGG CGGGUGUACU CCACCGGCAG CAAUGUGUUU CAGACCAGAG CCGGCUGUCU GAUCGGAGCC GAGCACGUGA ACAAUAGCUA CGAGUGCGAC AUCCCCAUCG GCGCUGGAAU CUGCGCCAGC UACCAGACAC AGACAAACAG CCACCGGAGA GCCAGAAGCG UGGCCAGCCA GAGCAUCAUU GCCUACACAA UGUCUCUGGG CGCCGAGAAC AGCGUGGCCU ACUCCAACAA CUCUAUCGCU AUCCCCAUCA ACUUCACCAU CAGCGUGACC ACAGAGAUCC UGCCUGUGUC CAUGACCAAG ACCAGCGUGG ACUGCACCAU GUACAUCUGC GGCGAUUCCA CCGAGUGCUC CAACCUGCUG CUGCAGUACG GCAGCUUCUG CACCCAGCUG AAUAGAGCCC UGACAGGGAU CGCCGUGGAA CAGGACAAGA ACACCCAAGA GGUGUUCGCC CAAGUGAAGC AGAUCUACAA GACCCCUCCU AUCAAGGACU UCGGCGGCUU CAAUUUCAGC CAGAUUCUGC CCGAUCCUAG CAAGCCCAGC AAGCGGAGCU UCAUCGAGGA CCUGCUGUUC AACAAAGUGA CACUGGCCGA CGCCGGCUUC AUCAAGCAGU AUGGCGAUUG UCUGGGCGAC AUUGCCGCCA GGGAUCUGAU UUGCGCCCAG AAGUUUAACG GACUGACAGU GCUGCCUCCU CUGCUGACCG AUGAGAUGAU CGCCCAGUAC ACAUCUGCCC UGCUGGCCGG CACAAUCACA AGCGGCUGGA CAUUUGGAGC AGGCGCCGCU CUGCAGAUCC CCUUUGCUAU GCAGAUGGCC UACCGGUUCA ACGGCAUCGG AGUGACCCAG AAUGUGCUGU ACGAGAACCA GAAGCUGAUC GCCAACCAGU UCAACAGCGC CAUCGGCAAG AUCCAGGACA GCCUGAGCAG CACAGCAAGC GCCCUGGGAA AGCUGCAGGA CGUGGUCAAC CAGAAUGCCC AGGCACUGAA CACCCUGGUC AAGCAGCUGU CCUCCAACUU CGGCGCCAUC AGCUCUGUGC UGAACGAUAU CCUGGCCAGA CUGGACCCUC CUGAGGCCGA GGUGCAGAUC GACAGACUGA UCACAGGCAG ACUGCAGAGC CUCCAGACAU ACGUGACCCA GCAGCUGAUC AGAGCCGCCG AGAUUAGAGC CUCUGCCAAU CUGGCCGCCA CCAAGAUGUC UGAGUGUGUG CUGGGCCAGA GCAAGAGAGU GGACUUUUGC GGCAAGGGCU ACCACCUGAU GAGCUUCCCU CAGUCUGCCC CUCACGGCGU GGUGUUUCUG |
| | | CACGUGACAU AUGUGCCCGC UCAAGAGAAG AAUUUCACCA CCGCUCCAGC CAUCUGCCAC GACGGCAAAG CCCACUUUCC UAGAGAAGGC GUGUUCGUGU CCAACGGCAC CCAUUGGUUC GUGACACAGC GGAACUUCUA CGAGCCCCAG AUCAUCACCA CCCACAACAC CUUCGUGUCU GGCAACUGCG ACGUCGUGAU CGGCAUUGUG AACAAUACCG UGUACGACCC UCUGCAGCCC GAGCUGGACA GCUUCAAAGA GGAACUGGAC AAGUACUUUA AGAACCACAC AAGCCCCGAC GUGGACCUGG GCGAUAUCAG CGGAAUCAAU GCCAGCGUCG UGAACAUCCA GAAAGAGAUC GACCGGCUGA ACGAGGUGGC CAAGAAUCUG AACGAGAGCC UGAUCGACCU GCAAGAACUG GGGAAGUACG AGCAGUACAU CAAGUGGCCC UGGUACAUCU GGCUGGGCUU UAUCGCCGGA CUGAUUGCCA UCGUGAUGGU CACAAUCAUG CUGUGUUGCA UGACCAGCUG CUGUAGCUGC CUGAAGGGCU GUUGUAGCUG UGGCAGCUGC UGCAAGUUCG ACGAGGACGA UUCUGAGCCC GUGCUGAAGG GCGUGAAACU GCACUACACA UGAUGA |

(continued)

| SEQ ID NO. | Brief Description | Sequence |
|---|---|---|
| 27 | Full length sequence of RBP020.14 | AGAAUAAACU AGUAUUCUUC UGGUCCCCAC AGACUCAGAG AGAACCCGCC ACC<br>AUGUUCGUGU UCCUGGUGCU GCUGCCUCUG GUGUCCAGCC AGUGUGUGAA CCUGACCACC<br>AGAACACAGC UGCCUCCAGC CUACACCAAC AGCUUUACCA GAGGCGUGUA CUACCCCGAC<br>AAGGUGUUCA GAUCCAGCGU GCUGCACUCU ACCCAGGACC UGUUCCUGCC UUUCUUCAGC<br>AACGUGACCU GGUUCCACGC CAUCUCCGGC ACCAAUGGCA CCAAGAGAUU CGACAACCCC<br>GUGCUGCCCU UCAACGACGG GGUGUACUUU GCCAGCACCG AGAAGUCCAA CAUCAUCAGA<br>GGCUGGAUCU UCGGCACCAC ACUGGACAGC AAGACCCAGA GCCUGCUGAU CGUGAACAAC<br>GCCACCAACG UGGUCAUCAA AGUGUGCGAG UUCCAGUUCU GCAACGACCC CUUCCUGGGC<br>GUCUACCACA AGAACAACAA GAGCUGGAUG GAAAGCGAGU UCCGGGUGUA CAGCAGCGCC<br>AACAACUGCA CCUUCGAGUA CGUGUCCCAG CCUUUCCUGA UGGACCUGGA AGGCAAGCAG<br>GGCAACUUCA AGAACCUGCG CGAGUUCGUG UUUAAGAACA UCGACGGCUA CUUCAAGAUC<br>UACAGCAAGC ACACCCCUAU CAACCUCGUG CGGGAUCUGC CUCAGGGCUU CUCUGCUCUG<br>GAACCCCUGG UGGAUCUGCC CAUCGGCAUC AACAUCACCC GGUUUCAGAC ACUGCUGGCC<br>CUGCACAGAA GCUACCUGAC ACCUGGCGAU AGCAGCAGCG GAUGGACAGC UGGUGCCGCC<br>GCUUACUAUG UGGGCUACCU GCAGCCUAGA ACCUUCCUGC UGAAGUACAA CGAGAACGGC<br>ACCAUCACCG ACGCCGUGGA UUGUGCUCUG GAUCCUCUGA GCGAGACAAA GUGCACCCUG<br>AAGUCCUUCA CCGUGGAAAA GGGCAUCUAC CAGACCAGCA ACUUCCGGGU GCAGCCCACC<br>GAAUCCAUCG UGCGGUUCCC CAAUAUCACC AAUCUGUGCC CCUUCGGCGA GGUGUUCAAU<br>GCCACCAGAU UCGCCUCUGU GUACGCCUGG AACCGGAAGC GGAUCAGCAA UUGCGUGGCC<br>GACUACUCCG UGCUGUACAA CUCCGCCAGC UUCAGCACCU UCAAGUGCUA CGGCGUGUCC<br>CCUACCAAGC UGAACGACCU GUGCUUCACA AACGUGUACG CCGACAGCUU CGUGAUCCGG<br>GGAGAUGAAG UGCGGCAGAU UGCCCCUGGA CAGACAGGCA AGAUCGCCGA CUACAACUAC<br>AAGCUGCCCG ACGACUUCAC CGGCUGUGUG AUUGCCUGGA ACAGCAACAA CCUGGACUCC<br>AAAGUCGGCG GCAACUACAA UUACCUGUAC CGGCUGUUCC GGAAGUCCAA UCUGAAGCCC<br>UUCGAGCGGG ACAUCUCCAC CGAGAUCUAU CAGGCCGGCA GCACCCCUUG UAACGGCGUG<br>GAAGGCUUCA ACUGCUACUU CCCACUGCAG UCCUACGGCU UUCAGCCCAC AUACGGCGUG |
| | | GGCUAUCAGC CCUACAGAGU GGUGGUGCUG AGCUUCGAAC UGCUGCAUGC CCCUGCCACA<br>GUGUGCGGCC CUAAGAAAAG CACCAAUCUC GUGAAGAACA AAUGCGUGAA CUUCAACUUC<br>AACGGCCUGA CCGGCACCGG CGUGCUGACA GAGAGCAACA AGAAGUUCCU GCCAUUCCAG<br>CAGUUUGGCC GGGAUAUCGA CGAUACCACA GACGCCGUUA GAGAUCCCCA GACACUGGAA<br>AUCCUGGACA UCACCCCUUG CAGCUUCGGC GGAGUGUCUG UGAUCACCCC UGGCACCAAC<br>ACCAGCAAUC AGGUGGCAGU GCUGUACCAG GGCGUGAACU GUACCGAAGU GCCCGUGGCC<br>AUUCACGCCG AUCAGCUGAC ACCUACAUGG CGGGUGUACU CCACCGGCAG CAAUGUGUUU<br>CAGACCAGAG CCGGCUGUCU GAUCGGAGCC GAGCACGUGA ACAAUAGCUA CGAGUGCGAC<br>AUCCCCAUCG GCGCUGGAAU CUGCGCCAGC UACCAGACAC AGACAAACAG CCACCGGAGA<br>GCCAGAAGCG UGGCCAGCCA GAGCAUCAUU GCCUACACAA UGUCUCUGGG CGCCGAGAAC<br>AGCGUGGCCU ACUCCAACAA CUCUAUCGCU AUCCCCAUCA ACUUCACCAU CAGCGUGACC<br>ACAGAGAUCC UGCCUGUGUC CAUGACCAAG ACCAGCGUGG ACUGCACCAU GUACAUCUGC<br>GGCGAUUCCA CCGAGUGCUC CAACCUGCUG CUGCAGUACG GCAGCUUCUG CACCCAGCUG<br>AAUAGAGCCC UGACAGGGAU CGCCGUGGAA CAGGACAAGA ACACCCAAGA GGUGUUCGCC<br>CAAGUGAAGC AGAUCUACAA GACCCCUCCU AUCAAGGACU UCGGCGGCUU CAAUUUCAGC<br>CAGAUUCUGC CCGAUCCUAG CAAGCCCAGC AAGCGGAGCU UCAUCGAGGA CCUGCUGUUC<br>AACAAAGUGA CACUGGCCGA CGCCGGCUUC AUCAAGCAGU AUGGCGAUUG UCUGGGCGAC<br>AUUGCCGCCA GGGAUCUGAU UUGCGCCCAG AAGUUUAACG GACUGACAGU GCUGCCUCCU<br>CUGCUGACCG AUGAGAUGAU CGCCCAGUAC ACAUCUGCCC UGCUGGCCGG CACAAUCACA<br>AGCGGCUGGA CAUUUGGAGC AGGCGCCGCU CUGCAGAUCC CCUUUGCUAU GCAGAUGGCC<br>UACCGGUUCA ACGGCAUCGG AGUGACCCAG AAUGUGCUGU ACGAGAACCA GAAGCUGAUC<br>GCCAACCAGU UCAACAGCGC CAUCGGCAAG AUCCAGGACA GCCUGAGCAG CACAGCAAGC<br>GCCCUGGGAA AGCUGCAGGA CGUGGUCAAC CAGAAUGCCC AGGCACUGAA CACCCUGGUC<br>AAGCAGCUGU CCUCCAACUU CGGCGCCAUC AGCUCUGUGC UGAACGAUAU CCUGGCCAGA<br>CUGGACCCUC CUGAGGCCGA GGUGCAGAUC GACAGACUGA UCACAGGCAG ACUGCAGAGC<br>CUCCAGACAU ACGUGACCCA GCAGCUGAUC AGAGCCGCCG AGAUUAGAGC CUCUGCCAAU<br>CUGGCCGCCA CCAAGAUGUC UGAGUGUGUG CUGGGCCAGA GCAAGAGAGU GGACUUUUGC<br>GGCAAGGGCU ACCACCUGAU GAGCUUCCCU CAGUCUGCCC CUCACGGCGU GGUGUUUCUG<br>CACGUGACAU AUGUGCCCGC UCAAGAGAAG AAUUUCACCA CCGCUCCAGC CAUCUGCCAC<br>GACGGCAAAG CCCACUUUCC UAGAGAAGGC GUGUUCGUGU CCAACGGCAC CCAUUGGUUC<br>GUGACACAGC GGAACUUCUA CGAGCCCCAG AUCAUCACCA CCCACAACAC CUUCGUGUCU<br>GGCAACUGCG ACGUCGUGAU CGGCAUUGUG AACAAUACCG UGUACGACCC UCUGCAGCCC<br>GAGCUGGACA GCUUCAAAGA GGAACUGGAC AAGUACUUUA AGAACCACAC AAGCCCCGAC<br>GUGGACCUGG GCGAUAUCAG CGGAAUCAAU GCCAGCGUCG UGAACAUCCA GAAAGAGAUC<br>GACCGGCUGA ACGAGGUGGC CAAGAAUCUG AACGAGAGCC UGAUCGACCU GCAAGAACUG<br>GGGAAGUACG AGCAGUACAU CAAGUGGCCC UGGUACAUCU GGCUGGGCUU UAUCGCCGGA<br>CUGAUUGCCA UCGUGAUGGU CACAAUCAUG CUGUGUUGCA UGACCAGCUG CUGUAGCUGC<br>CUGAAGGGCU GUUGUAGCUG UGGCAGCUGC UGCAAGUUCG ACGAGGACGA UUCUGAGCCC |

(continued)

| SEQ ID NO. | Brief Description | Sequence |
|---|---|---|
| | | GUGCUGAAGG GCGUGAAACU GCACUACACA UGAUGA<br>GAUCUGCUGG UACUGCAUGC ACGCAAUGCU AGCUGCCCCU UUCCCGUCCU GGGUACCCCG<br>AGUCUCCCCC GACCUCGGGU CCCAGGUAUG CUCCCACCUC CACCUGCCCC ACUCACCACC<br>UCUGCUAGUU CCAGACACCU CCCAAGCACG CAGCAAUGCA GCUCAAAACG CUUAGCCUAG<br>CCACACCCCC ACGGGAAACA GCAGUGAUUA ACCUUUAGCA AUAAACGAAA GUUUAACUAA<br>GCUAUACUAA CCCCAGGGUU GGUCAAUUUC GUGCCAGCCA CACCCUGGAG CUAGC<br>AAAAAAAAAA AAAAAAAAAA AAAAAAAAAA GCAUAUGACU AAAAAAAAAA AAAAAAAAAA<br>AAAAAAAAAA AAAAAAAAAA AAAAAAAAAA AAAAAAAAAA AAAAAAAAAA |

**Nucleotide Sequence of RBP020.16 (Delta-specific vaccine)**

Nucleotide sequence is shown with individual sequence elements as indicated in bold letters. In addition, the sequence of the translated protein is shown in italic letters below the coding nucleotide sequence (* = stop codon). Point mutations in the amino acid and nucleotide sequence shown in red text.

```
           10         20         30         40          50 53
AGAATAAACT AGTATTCTTC TGGTCCCCAC AGACTCAGAG AGAACCCGCC ACC
```

**hAg-Kozak**

```
        63         73         83         93        103        113
ATGTTCGTGT TCCTGGTGCT GCTGCCTCTG GTGTCCAGCC AGTGTGTGAA CCTGAGAACC
  M  F  V   F  L  V   L  L  P  L   V  S  S   Q  C  V   N  L  R  T
```

**S protein mut4**

```
       123        133        143        153        163        173
AGAACACAGC TGCCTCCAGC CTACACCAAC AGCTTTACCA GAGGCGTGTA CTACCCCGAC
  R  T  Q   L  P  P   A  Y  T  N   S  F  T   R  G  V   Y  Y  P  D
```

**S protein mut4**

```
       183        193        203        213        223        233
AAGGTGTTCA GATCCAGCGT GCTGCACTCT ACCCAGGACC TGTTCCTGCC TTTCTTCAGC
  K  V  F   R  S  S   V  L  H  S   T  Q  D   L  F  L   P  F  F  S
```

**S protein mut4**

```
       243        253        263        273        283        293
AACGTGACCT GGTTCCACGC CATCCACGTG TCCGGCACCA ATGGCACCAA GAGATTCGAC
  N  V  T   W  F  H   A  I  H  V   S  G  T   N  G  T   K  R  F  D
```

**S protein mut4**

```
       303        313        323        333        343        353
AACCCCGTGC TGCCCTTCAA CGACGGGGTG TACTTTGCCA GCACCGAGAA GTCCAACATC
  N  P  V   L  P  F   N  D  G  V   Y  F  A   S  T  E   K  S  N  I
```

**S protein mut4**

```
       363        373        383        393        403        413
ATCAGAGGCT GGATCTTCGG CACCACACTG GACAGCAAGA CCCAGAGCCT GCTGATCGTG
  I  R  G   W  I  F   G  T  T  L   D  S  K   T  Q  S   L  L  I  V
```

**S protein mut4**

```
       423        433        443        453        463        473
AACAACGCCA CCAACGTGGT CATCAAAGTG TGCGAGTTCC AGTTCTGCAA CGACCCCTTC
  N  N  A   T  N  V   V  I  K  V   C  E  F   Q  F  C   N  D  P  F
```

**S protein mut4**

```
       483        493        503        513        523        533
CTGGACGTCT ACTACCACAA GAACAACAAG AGCTGGATGG AAAGCGGCGT GTACAGCAGC
  L  D  V   Y  Y  H   K  N  N  K   S  W  M   E  S  G   V  Y  S  S
```

**S protein mut4**

```
       543        553        563        573        583        593
GCCAACAACT GCACCTTCGA GTACGTGTCC CAGCCTTTCC TGATGGACCT GGAAGGCAAG
  A  N  N   C  T  F   E  Y  V  S   Q  P  F   L  M  D   L  E  G  K
                          S protein mut4

       603        613        623        633        643        653
CAGGGCAACT TCAAGAACCT GCGCGAGTTC GTGTTTAAGA ACATCGACGG CTACTTCAAG
  Q  G  N   F  K  N   L  R  E  F   V  F  K   N  I  D   G  Y  F  K
                          S protein mut4

       663        673        683        693        703        713
ATCTACAGCA AGCACACCCC TATCAACCTC GTGCGGGATC TGCCTCAGGG CTTCTCTGCT
  I  Y  S   K  H  T   P  I  N  L   V  R  D   L  P  Q   G  F  S  A
                          S protein mut4

       723        733        743        753        763        773
CTGGAACCCC TGGTGGATCT GCCCATCGGC ATCAACATCA CCCGGTTTCA GACACTGCTG
  L  E  P   L  V  D   L  P  I  G   I  N  I   T  R  F   Q  T  L  L
                          S protein mut4

       783        793        803        813        823        833
GCCCTGCACA GAAGCTACCT GACACCTGGC GATAGCAGCA GCGGATGGAC AGCTGGTGCC
  A  L  H   R  S  Y   L  T  P  G   D  S  S   S  G  W   T  A  G  A
                          S protein mut4

       843        853        863        873        883        893
GCCGCTTACT ATGTGGGCTA CCTGCAGCCT AGAACCTTCC TGCTGAAGTA CAACGAGAAC
  A  A  Y   Y  V  G   Y  L  Q  P   R  T  F   L  L  K   Y  N  E  N
                          S protein mut4

       903        913        923        933        943        953
GGCACCATCA CCGACGCCGT GGATTGTGCT CTGGATCCTC TGAGCGAGAC AAAGTGCACC
  G  T  I   T  D  A   V  D  C  A   L  D  P   L  S  E   T  K  C  T
                          S protein mut4

       963        973        983        993       1003       1013
CTGAAGTCCT TCACCGTGGA AAAGGGCATC TACCAGACCA GCAACTTCCG GGTGCAGCCC
  L  K  S   F  T  V   E  K  G  I   Y  Q  T   S  N  F   R  V  Q  P
                          S protein mut4

      1023       1033       1043       1053       1063       1073
ACCGAATCCA TCGTGCGGTT CCCCAATATC ACCAATCTGT GCCCCTTCGG CGAGGTGTTC
  T  E  S   I  V  R   F  P  N  I   T  N  L   C  P  F   G  E  V  F
                          S protein mut4

      1083       1093       1103       1113       1123       1133
AATGCCACCA GATTCGCCTC TGTGTACGCC TGGAACCGGA AGCGGATCAG CAATTGCGTG
  N  A  T   R  F  A   S  V  Y  A   W  N  R   K  R  I   S  N  C  V
                          S protein mut4

      1143       1153       1163       1173       1183       1193
GCCGACTACT CCGTGCTGTA CAACTCCGCC AGCTTCAGCA CCTTCAAGTG CTACGGCGTG
  A  D  Y   S  V  L   Y  N  S  A   S  F  S   T  F  K   C  Y  G  V
                          S protein mut4

      1203       1213       1223       1233       1243       1253
TCCCCTACCA AGCTGAACGA CCTGTGCTTC ACAAACGTGT ACGCCGACAG CTTCGTGATC
  S  P  T   K  L  N   D  L  C  F   T  N  V   Y  A  D   S  F  V  I
                          S protein mut4
```

```
       1263       1273       1283       1293       1303       1313
CGGGGAGATG AAGTGCGGCA GATTGCCCCT GGACAGACAG GCAAGATCGC CGACTACAAC
  R  G  D   E  V  R   Q  I  A  P   G  Q  T   G  K  I   A  D  Y  N
```

**S protein mut4**

```
       1323       1333       1343       1353       1363       1373
TACAAGCTGC CCGACGACTT CACCGGCTGT GTGATTGCCT GGAACAGCAA CAACCTGGAC
  Y  K  L   P  D  D   F  T  G  C   V  I  A   W  N  S   N  N  L  D
```

**S protein mut4**

```
       1383       1393       1403       1413       1423       1433
TCCAAAGTCG GCGGCAACTA CAATTACAGG TACCGGCTGT TCCGGAAGTC CAATCTGAAG
  S  K  V   G  G  N   Y  N  Y  R   Y  R  L   F  R  K   S  N  L  K
```

**S protein mut4**

```
       1443       1453       1463       1473       1483       1493
CCCTTCGAGC GGGACATCTC CACCGAGATC TATCAGGCCG GCAGCAAGCC TTGTAACGGC
  P  F  E   R  D  I   S  T  E  I   Y  Q  A   G  S  K   P  C  N  G
```

**S protein mut4**

```
       1503       1513       1523       1533       1543       1553
GTGGAAGGCT TCAACTGCTA CTTCCCACTG CAGTCCTACG GCTTTCAGCC CACAAATGGC
  V  E  G   F  N  C   Y  F  P  L   Q  S  Y   G  F  Q   P  T  N  G
```

**S protein mut4**

```
       1563       1573       1583       1593       1603       1613
GTGGGCTATC AGCCCTACAG AGTGGTGGTG CTGAGCTTCG AACTGCTGCA TGCCCCTGCC
  V  G  Y   Q  P  Y   R  V  V  V   L  S  F   E  L  L   H  A  P  A
```

**S protein mut4**

```
       1623       1633       1643       1653       1663       1673
ACAGTGTGCG GCCCTAAGAA AAGCACCAAT CTCGTGAAGA ACAAATGCGT GAACTTCAAC
  T  V  C   G  P  K   K  S  T  N   L  V  K   N  K  C   V  N  F  N
```

**S protein mut4**

```
       1683       1693       1703       1713       1723       1733
TTCAACGGCC TGACCGGCAC CGGCGTGCTG ACAGAGAGCA ACAAGAAGTT CCTGCCATTC
  F  N  G   L  T  G   T  G  V  L   T  E  S   N  K  K   F  L  P  F
```

**S protein mut4**

```
       1743       1753       1763       1773       1783       1793
CAGCAGTTTG GCCGGGATAT CGCCGATACC ACAGACGCCG TTAGAGATCC CCAGACACTG
  Q  Q  F   G  R  D   I  A  D  T   T  D  A   V  R  D   P  Q  T  L
```

**S protein mut4**

```
       1803       1813       1823       1833       1843       1853
GAAATCCTGG ACATCACCCC TTGCAGCTTC GGCGGAGTGT CTGTGATCAC CCCTGGCACC
  E  I  L   D  I  T   P  C  S  F   G  G  V   S  V  I   T  P  G  T
```

**S protein mut4**

```
       1863       1873       1883       1893       1903       1913
AACACCAGCA ATCAGGTGGC AGTGCTGTAC CAGGGCGTGA ACTGTACCGA AGTGCCCGTG
  N  T  S   N  Q  V   A  V  L  Y   Q  G  V   N  C  T   E  V  P  V
```

**S protein mut4**

```
       1923       1933       1943       1953       1963       1973
GCCATTCACG CCGATCAGCT GACACCTACA TGGCGGGTGT ACTCCACCGG CAGCAATGTG
  A  I  H   A  D  Q   L  T  P  T   W  R  V   Y  S  T   G  S  N  V
```

**S protein mut4**

```
      1983       1993       2003       2013       2023       2033
TTTCAGACCA GAGCCGGCTG TCTGATCGGA GCCGAGCACG TGAACAATAG CTACGAGTGC
  F  Q  T   R  A  G   C  L  I   G  A  E   H  V  N   N  S  Y  E  C
```
**S protein mut4**

```
      2043       2053       2063       2073       2083       2093
GACATCCCCA TCGGCGCTGG AATCTGCGCC AGCTACCAGA CACAGACAAA CAGCAGGCGG
  D  I  P   I  G  A   G  I  C   A  S  Y   Q  T  Q   T  N  S  R  R
```
**S protein mut4**

```
      2103       2113       2123       2133       2143       2153
AGAGCCAGAA GCGTGGCCAG CCAGAGCATC ATTGCCTACA CAATGTCTCT GGGCGCCGAG
  R  A  R   S  V  A   S  Q  S   I  I  A   Y  T  M   S  L  G  A  E
```
**S protein mut4**

```
      2163       2173       2183       2193       2203       2213
AACAGCGTGG CCTACTCCAA CAACTCTATC GCTATCCCCA CCAACTTCAC CATCAGCGTG
  N  S  V   A  Y  S   N  N  S   I  A  I   P  T  N   F  T  I  S  V
```
**S protein mut4**

```
      2223       2233       2243       2253       2263       2273
ACCACAGAGA TCCTGCCTGT GTCCATGACC AAGACCAGCG TGGACTGCAC CATGTACATC
  T  T  E   I  L  P   V  S  M   T  K  T   S  V  D   C  T  M  Y  I
```
**S protein mut4**

```
      2283       2293       2303       2313       2323       2333
TGCGGCGATT CCACCGAGTG CTCCAACCTG CTGCTGCAGT ACGGCAGCTT CTGCACCCAG
  C  G  D   S  T  E   C  S  N   L  L  L   Q  Y  G   S  F  C  T  Q
```
**S protein mut4**

```
      2343       2353       2363       2373       2383       2393
CTGAATAGAG CCCTGACAGG GATCGCCGTG GAACAGGACA AGAACACCCA AGAGGTGTTC
  L  N  R   A  L  T   G  I  A   V  E  Q   D  K  N   T  Q  E  V  F
```
**S protein mut4**

```
      2403       2413       2423       2433       2443       2453
GCCCAAGTGA AGCAGATCTA CAAGACCCCT CCTATCAAGG ACTTCGGCGG CTTCAATTTC
  A  Q  V   K  Q  I   Y  K  T   P  P  I   K  D  F   G  G  F  N  F
```
**S protein mut4**

```
      2463       2473       2483       2493       2503       2513
AGCCAGATTC TGCCCGATCC TAGCAAGCCC AGCAAGCGGA GCTTCATCGA GGACCTGCTG
  S  Q  I   L  P  D   P  S  K   P  S  K   R  S  F   I  E  D  L  L
```
**S protein mut4**

```
      2523       2533       2543       2553       2563       2573
TTCAACAAAG TGACACTGGC CGACGCCGGC TTCATCAAGC AGTATGGCGA TTGTCTGGGC
  F  N  K   V  T  L   A  D  A   G  F  I   K  Q  Y   G  D  C  L  G
```
**S protein mut4**

```
      2583       2593       2603       2613       2623       2633
GACATTGCCG CCAGGGATCT GATTTGCGCC CAGAAGTTTA ACGGACTGAC AGTGCTGCCT
  D  I  A   A  R  D   L  I  C   A  Q  K   F  N  G   L  T  V  L  P
```
**S protein mut4**

```
      2643       2653       2663       2673       2683       2693
CCTCTGCTGA CCGATGAGAT GATCGCCCAG TACACATCTG CCCTGCTGGC CGGCACAATC
  P  L  L   T  D  E   M  I  A   Q  Y  T   S  A  L   L  A  G  T  I
```
**S protein mut4**

```
      2703       2713       2723       2733       2743       2753
ACAAGCGGCT GGACATTTGG AGCAGGCGCC GCTCTGCAGA TCCCCTTTGC TATGCAGATG
  T  S  G   W  T  F   G  A  G  A   A  L  Q   I  P  F   A  M  Q  M
```
**S protein mut4**

```
      2763       2773       2783       2793       2803       2813
GCCTACCGGT TCAACGGCAT CGGAGTGACC CAGAATGTGC TGTACGAGAA CCAGAAGCTG
  A  Y  R   F  N  G   I  G  V  T   Q  N  V   L  Y  E   N  Q  K  L
```
**S protein mut4**

```
      2823       2833       2843       2853       2863       2873
ATCGCCAACC AGTTCAACAG CGCCATCGGC AAGATCCAGG ACAGCCTGAG CAGCACAGCA
  I  A  N   Q  F  N   S  A  I  G   K  I  Q   D  S  L   S  S  T  A
```
**S protein mut4**

```
      2883       2893       2903       2913       2923       2933
AGCGCCCTGG GAAAGCTGCA GAACGTGGTC AACCAGAATG CCCAGGCACT GAACACCCTG
  S  A  L   G  K  L   Q  N  V  V   N  Q  N   A  Q  A   L  N  T  L
```
**S protein mut4**

```
      2943       2953       2963       2973       2983       2993
GTCAAGCAGC TGTCCTCCAA CTTCGGCGCC ATCAGCTCTG TGCTGAACGA TATCCTGAGC
  V  K  Q   L  S  S   N  F  G  A   I  S  S   V  L  N   D  I  L  S
```
**S protein mut4**

```
      3003       3013       3023       3033       3043       3053
AGACTGGACC CTCCTGAGGC CGAGGTGCAG ATCGACAGAC TGATCACAGG CAGACTGCAG
  R  L  D   P  P  E   A  E  V  Q   I  D  R   L  I  T   G  R  L  Q
```
**S protein mut4**

```
      3063       3073       3083       3093       3103       3113
AGCCTCCAGA CATACGTGAC CCAGCAGCTG ATCAGAGCCG CCGAGATTAG AGCCTCTGCC
  S  L  Q   T  Y  V   T  Q  Q  L   I  R  A   A  E  I   R  A  S  A
```
**S protein mut4**

```
      3123       3133       3143       3153       3163       3173
AATCTGGCCG CCACCAAGAT GTCTGAGTGT GTGCTGGGCC AGAGCAAGAG AGTGGACTTT
  N  L  A   A  T  K   M  S  E  C   V  L  G   Q  S  K   R  V  D  F
```
**S protein mut4**

```
      3183       3193       3203       3213       3223       3233
TGCGGCAAGG GCTACCACCT GATGAGCTTC CCTCAGTCTG CCCCTCACGG CGTGGTGTTT
  C  G  K   G  Y  H   L  M  S  F   P  Q  S   A  P  H   G  V  V  F
```
**S protein mut4**

```
      3243       3253       3263       3273       3283       3293
CTGCACGTGA CATATGTGCC CGCTCAAGAG AAGAATTTCA CCACCGCTCC AGCCATCTGC
  L  H  V   T  Y  V   P  A  Q  E   K  N  F   T  T  A   P  A  I  C
```
**S protein mut4**

```
      3303       3313       3323       3333       3343       3353
CACGACGGCA AAGCCCACTT TCCTAGAGAA GGCGTGTTCG TGTCCAACGG CACCCATTGG
  H  D  G   K  A  H   F  P  R  E   G  V  F   V  S  N   G  T  H  W
```
**S protein mut4**

```
      3363       3373       3383       3393       3403       3413
TTCGTGACAC AGCGGAACTT CTACGAGCCC CAGATCATCA CCACCGACAA CACCTTCGTG
  F  V  T   Q  R  N   F  Y  E  P   Q  I  I   T  T  D   N  T  F  V
```
**S protein mut4**

```
       3423        3433        3443        3453        3463        3473
TCTGGCAACT GCGACGTCGT GATCGGCATT GTGAACAATA CCGTGTACGA CCCTCTGCAG
   S  G  N   C  D  V   V  I  G  I   V  N  N   T  V  Y   D  P  L  Q
```
**S protein mut4**

```
       3483        3493        3503        3513        3523        3533
CCCGAGCTGG ACAGCTTCAA AGAGGAACTG GACAAGTACT TTAAGAACCA CACAAGCCCC
   P  E  L   D  S  F   K  E  E  L   D  K  Y   F  K  N   H  T  S  P
```
**S protein mut4**

```
       3543        3553        3563        3573        3583        3593
GACGTGGACC TGGGCGATAT CAGCGGAATC AATGCCAGCG TCGTGAACAT CCAGAAAGAG
   D  V  D   L  G  D   I  S  G  I   N  A  S   V  V  N   I  Q  K  E
```
**S protein mut4**

```
       3603        3613        3623        3633        3643        3653
ATCGACCGGC TGAACGAGGT GGCCAAGAAT CTGAACGAGA GCCTGATCGA CCTGCAAGAA
   I  D  R   L  N  E   V  A  K  N   L  N  E   S  L  I   D  L  Q  E
```
**S protein mut4**

```
       3663        3673        3683        3693        3703        3713
CTGGGGAAGT ACGAGCAGTA CATCAAGTGG CCCTGGTACA TCTGGCTGGG CTTTATCGCC
   L  G  K   Y  E  Q   Y  I  K  W   P  W  Y   I  W  L   G  F  I  A
```
**S protein mut4**

```
       3723        3733        3743        3753        3763        3773
GGACTGATTG CCATCGTGAT GGTCACAATC ATGCTGTGTT GCATGACCAG CTGCTGTAGC
   G  L  I   A  I  V   M  V  T  I   M  L  C   C  M  T   S  C  C  S
```
**S protein mut4**

```
       3783        3793        3803        3813        3823        3833
TGCCTGAAGG GCTGTTGTAG CTGTGGCAGC TGCTGCAAGT TCGACGAGGA CGATTCTGAG
   C  L  K   G  C  C   S  C  G  S   C  C  K   F  D  E   D  D  S  E
```
**S protein mut4**

```
       3843        3853        3863        3872
CCCGTGCTGA AGGGCGTGAA ACTGCACTAC ACATGATGA
   P  V  L   K  G  V   K  L  H  Y   T  *  *
```
**S protein mut4**

```
       3882        3892        3902        3912        3922        3932
TTTCACCTGG TACTGCATGC ACGCAATGCT AGCTGCCCCT TTCCCGTCCT GGGTACCCCG
```
**FI element**

```
       3942        3952        3962        3972        3982        3992
AGTCTCCCCC GACCTCGGGT CCCAGGTATG CTCCCACCTC CACCTGCCCC ACTCACCACC
```
**FI element**

```
       4002        4012        4022        4032        4042        4052
TCTGCTAGTT CCAGACACCT CCCAAGCACG CAGCAATGCA GCTCAAAACG CTTAGCCTAG
```
**FI element**

```
       4062        4072        4082        4092        4102        4112
CCACACCCCC ACGGGAAACA GCAGTGATTA ACCTTTAGCA ATAAACGAAA GTTTAACTAA
```
**FI element**

```
      4122      4132      4142      4152      4162 4167
GCTATACTAA CCCCAGGGTT GGTCAATTTC GTGCCAGCCA CACCCTGGAG CTAGC
```

**FI element**

```
      4177      4187      4197      4207      4217      4227
AAAAAAAAAA AAAAAAAAAA AAAAAAAAAA GCATATGACT AAAAAAAAAA AAAAAAAAAA
```

**Poly(A)**

```
      4237      4247      4257      4267      4277
AAAAAAAAAA AAAAAAAAAA AAAAAAAAAA AAAAAAAAAA AAAAAAAAAA
```

**Poly(A)**

Sequences of RBP020.16 are also shown in Table 6.

**Table 6:** Sequences of RBP020.16 (Delta-specific RNA vaccine)

| SEQ ID NO. | Brief Description | Sequence |
|---|---|---|
| 28 | Amino acid sequence of RNA-encoded SARS-CoV-2 S protein from a Delta variant | MFVFLVLLPLVSSQCVNLRTRTQLPPAYTNSFTRGVYYPDKVFRSSVLHSTQDLFLPFFSNVTWF<br>HAIHVSGTNGTKRFDNPVLPFNDGVYFASTEKSNIIRGWIFGTTLDSKTQSLLIVNNATNVVIKV<br>CEFQFCNDPFLDVYYHKNNKSWMESGVYSSANNCTFEYVSQPFLMDLEGKQGNFKNLREFVFKNI<br>DGYFKIYSKHTPINLVRDLPQGFSALEPLVDLPIGINITRFQTLLALHRSYLTPGDSSSGWTAGA<br>AAYYVGYLQPRTFLLKYNENGTITDAVDCALDPLSETKCTLKSFTVEKGIYQTSNFRVQPTESIV<br>RFPNITNLCPFGEVFNATRFASVYAWNRKRISNCVADYSVLYNSASFSTFKCYGVSPTKLNDLCF<br>TNVYADSFVIRGDEVRQIAPGQTGKIADYNYKLPDDFTGCVIAWNSNNLDSKVGGNYNYRYRLFR<br>KSNLKPFERDISTEIYQAGSKPCNGVEGFNCYFPLQSYGFQPTNGVGYQPYRVVVLSFELLHAPA<br>TVCGPKKSTNLVKNKCVNFNFNGLTGTGVLTESNKKFLPFQQFGRDIADTTDAVRDPQTLEILDI<br>TPCSFGGVSVITPGTNTSNQVAVLYQGVNCTEVPVAIHADQLTPTWRVYSTGSNVFQTRAGCLIG<br>AEHVNNSYECDIPIGAGICASYQTQTNSRRRARSVASQSIIAYTMSLGAENSVAYSNNSIAIPTN<br>FTISVTTEILPVSMTKTSVDCTMYICGDSTECSNLLLQYGSFCTQLNRALTGIAVEQDKNTQEVF<br>AQVKQIYKTPPIKDFGGFNFSQILPDPSKPSKRSFIEDLLFNKVTLADAGFIKQYGDCLGDIAAR<br>DLICAQKFNGLTVLPPLLTDEMIAQYTSALLAGTITSGWTFGAGAALQIPFAMQMAYRFNGIGVT<br>QNVLYENQKLIANQFNSAIGKIQDSLSSTASALGKLQNVVNQNAQALNTLVKQLSSNFGAISSVL<br>NDILSRLDPPEAEVQIDRLITGRLQSLQTYVTQQLIRAAEIRASANLAATKMSECVLGQSKRVDF<br>CGKGYHLMSFPQSAPHGVVFLHVTYVPAQEKNFTTAPAICHDGKAHFPREGVFVSNGTHWFVTQR<br>NFYEPQIITTDNTFVSGNCDVVIGIVNNTVYDPLQPELDSFKEELDKYFKNHTSPDVDLGDISGI<br>NASVVNIQKEIDRLNEVAKNLNESLIDLQELGKYEQYIKWPWYIWLGFIAGLIAIVMVTIMLCCM<br>TSCCSCLKGCCSCGSCCKFDEDDSEPVLKGVKLHYT-- |
| 29 | RNA sequence encoding a SARS-CoV-2 S protein from a Delta variant | ATGTTCGTGT TCCTGGTGCT GCTGCCTCTG GTGTCCAGCC AGTGTGTGAA CCTGAGAACC<br>AGAACACAGC TGCCTCCAGC CTACACCAAC AGCTTTACCA GAGGCGTGTA CTACCCCGAC<br>AAGGTGTTCA GATCCAGCGT GCTGCACTCT ACCCAGGACC TGTTCCTGCC TTTCTTCAGC<br>AACGTGACCT GGTTCCACGC CATCCACGTG TCCGGCACCA ATGGCACCAA GAGATTCGAC<br>AACCCCGTGC TGCCCTTCAA CGACGGGGTG TACTTTGCCA GCACCGAGAA GTCCAACATC<br>ATCAGAGGCT GGATCTTCGG CACCACACTG GACAGCAAGA CCCAGAGCCT GCTGATCGTG<br>AACAACGCCA CCAACGTGGT CATCAAAGTG TGCGAGTTCC AGTTCTGCAA CGACCCCTTC<br>CTGGACGTCT ACTACCACAA GAACAACAAG AGCTGGATGG AAAGCGGCGT GTACAGCAGC<br>GCCAACAACT GCACCTTCGA GTACGTGTCC CAGCCTTTCC TGATGGACCT GGAAGGCAAG<br>CAGGGCAACT TCAAGAACCT GCGCGAGTTC GTGTTTAAGA ACATCGACGG CTACTTCAAG<br>ATCTACAGCA AGCACACCCC TATCAACCTC GTGCGGGATC TGCCTCAGGG CTTCTCTGCT<br>CTGGAACCCC TGGTGGATCT GCCCATCGGC ATCAACATCA CCCGGTTTCA GACACTGCTG<br>GCCCTGCACA GAAGCTACCT GACACCTGGC GATAGCAGCA GCGGATGGAC AGCTGGTGCC<br>GCCGCTTACT ATGTGGGCTA CCTGCAGCCT AGAACCTTCC TGCTGAAGTA CAACGAGAAC<br>GGCACCATCA CCGACGCCGT GGATTGTGCT CTGGATCCTC TGAGCGAGAC AAAGTGCACC<br>CTGAAGTCCT TCACCGTGGA AAAGGGCATC TACCAGACCA GCAACTTCCG GGTGCAGCCC<br>ACCGAATCCA TCGTGCGGTT CCCCAATATC ACCAATCTGT GCCCCTTCGG CGAGGTGTTC<br>AATGCCACCA GATTCGCCTC TGTGTACGCC TGGAACCGGA AGCGGATCAG CAATTGCGTG |

(continued)

| SEQ ID NO. | Brief Description | Sequence |
|---|---|---|
| | | GCCGACTACT CCGTGCTGTA CAACTCCGCC AGCTTCAGCA CCTTCAAGTG CTACGGCGTG TCCCCTACCA AGCTGAACGA CCTGTGCTTC ACAAACGTGT ACGCCGACAG CTTCGTGATC CGGGGAGATG AAGTGCGGCA GATTGCCCCT GGACAGACAG GCAAGATCGC CGACTACAAC TACAAGCTGC CCGACGACTT CACCGGCTGT GTGATTGCCT GGAACAGCAA CAACCTGGAC TCCAAAGTCG GCGGCAACTA CAATTACAGG TACCGGCTGT TCCGGAAGTC CAATCTGAAG CCCTTCGAGC GGGACATCTC CACCGAGATC TATCAGGCCG GCAGCAAGCC TTGTAACGGC GTGGAAGGCT TCAACTGCTA CTTCCCACTG CAGTCCTACG GCTTTCAGCC CACAAATGGC GTGGGCTATC AGCCCTACAG AGTGGTGGTG CTGAGCTTCG AACTGCTGCA TGCCCCTGCC ACAGTGTGCG GCCCTAAGAA AAGCACCAAT CTCGTGAAGA ACAAATGCGT GAACTTCAAC TTCAACGGCC TGACCGGCAC CGGCGTGCTG ACAGAGAGCA ACAAGAAGTT CCTGCCATTC CAGCAGTTTG GCCGGGATAT CGCCGATACC ACAGACGCCG TTAGAGATCC CCAGACACTG GAAATCCTGG ACATCACCCC TTGCAGCTTC GGCGGAGTGT CTGTGATCAC CCCTGGCACC AACACCAGCA ATCAGGTGGC AGTGCTGTAC CAGGGCGTGA ACTGTACCGA AGTGCCCGTG GCCATTCACG CCGATCAGCT GACACCTACA TGGCGGGTGT ACTCCACCGG CAGCAATGTG TTTCAGACCA GAGCCGGCTG TCTGATCGGA GCCGAGCACG TGAACAATAG CTACGAGTGC GACATCCCCA TCGGCGCTGG AATCTGCGCC AGCTACCAGA CACAGACAAA CAGCAGGCGG AGAGCCAGAA GCGTGGCCAG CCAGAGCATC ATTGCCTACA CAATGTCTCT GGGCGCCGAG AACAGCGTGG CCTACTCCAA CAACTCTATC GCTATCCCCA CCAACTTCAC CATCAGCGTG ACCACAGAGA TCCTGCCTGT GTCCATGACC AAGACCAGCG TGGACTGCAC CATGTACATC TGCGGCGATT CCACCGAGTG CTCCAACCTG CTGCTGCAGT ACGGCAGCTT CTGCACCCAG CTGAATAGAG CCCTGACAGG GATCGCCGTG GAACAGGACA AGAACACCCA AGAGGTGTTC GCCCAAGTGA AGCAGATCTA CAAGACCCCT CCTATCAAGG ACTTCGGCGG CTTCAATTTC AGCCAGATTC TGCCCGATCC TAGCAAGCCC AGCAAGCGGA GCTTCATCGA GGACCTGCTG TTCAACAAAG TGACACTGGC CGACGCCGGC TTCATCAAGC AGTATGGCGA TTGTCTGGGC GACATTGCCG CCAGGGATCT GATTTGCGCC CAGAAGTTTA ACGGACTGAC AGTGCTGCCT CCTCTGCTGA CCGATGAGAT GATCGCCCAG TACACATCTG CCCTGCTGGC CGGCACAATC ACAAGCGGCT GGACATTTGG AGCAGGCGCC GCTCTGCAGA TCCCCTTTGC TATGCAGATG GCCTACCGGT TCAACGGCAT CGGAGTGACC CAGAATGTGC TGTACGAGAA CCAGAAGCTG ATCGCCAACC AGTTCAACAG CGCCATCGGC AAGATCCAGG ACAGCCTGAG CAGCACAGCA AGCGCCCTGG GAAAGCTGCA GAACGTGGTC AACCAGAATG CCCAGGCACT GAACACCCTG GTCAAGCAGC TGTCCTCCAA CTTCGGCGCC ATCAGCTCTG TGCTGAACGA TATCCTGAGC AGACTGGACC CTCCTGAGGC CGAGGTGCAG ATCGACAGAC TGATCACAGG CAGACTGCAG AGCCTCCAGA CATACGTGAC CCAGCAGCTG ATCAGAGCCG CCGAGATTAG AGCCTCTGCC AATCTGGCCG CCACCAAGAT GTCTGAGTGT GTGCTGGGCC AGAGCAAGAG AGTGGACTTT TGCGGCAAGG GCTACCACCT GATGAGCTTC CCTCAGTCTG CCCCTCACGG CGTGGTGTTT CTGCACGTGA CATATGTGCC CGCTCAAGAG AAGAATTTCA CCACCGCTCC AGCCATCTGC CACGACGGCA AAGCCCACTT TCCTAGAGAA GGCGTGTTCG TGTCCAACGG CACCCATTGG TTCGTGACAC AGCGGAACTT CTACGAGCCC CAGATCATCA CCACCGACAA CACCTTCGTG TCTGGCAACT GCGACGTCGT GATCGGCATT GTGAACAATA CCGTGTACGA CCCTCTGCAG |
| | | CCCGAGCTGG ACAGCTTCAA AGAGGAACTG GACAAGTACT TTAAGAACCA CACAAGCCCC GACGTGGACC TGGGCGATAT CAGCGGAATC AATGCCAGCG TCGTGAACAT CCAGAAAGAG ATCGACCGGC TGAACGAGGT GGCCAAGAAT CTGAACGAGA GCCTGATCGA CCTGCAAGAA CTGGGGAAGT ACGAGCAGTA CATCAAGTGG CCCTGGTACA TCTGGCTGGG CTTTATCGCC GGACTGATTG CCATCGTGAT GGTCACAATC ATGCTGTGTT GCATGACCAG CTGCTGTAGC TGCCTGAAGG GCTGTTGTAG CTGTGGCAGC TGCTGCAAGT TCGACGAGGA CGATTCTGAG CCCGTGCTGA AGGGCGTGAA ACTGCACTAC ACATGATGA |

(continued)

| SEQ ID NO. | Brief Description | Sequence |
|---|---|---|
| 30 | Full length sequence of RBP020.16 | AGAATAAACT AGTATTCTTC TGGTCCCCAC AGACTCAGAG AGAACCCGCC ACC<br>ATGTTCGTGT TCCTGGTGCT GCTGCCTCTG GTGTCCAGCC AGTGTGTGAA CCTGAGAACC<br>AGAACACAGC TGCCTCCAGC CTACACCAAC AGCTTTACCA GAGGCGTGTA CTACCCCGAC<br>AAGGTGTTCA GATCCAGCGT GCTGCACTCT ACCCAGGACC TGTTCCTGCC TTTCTTCAGC<br>AACGTGACCT GGTTCCACGC CATCCACGTG TCCGGCACCA ATGGCACCAA GAGATTCGAC<br>AACCCCGTGC TGCCCTTCAA CGACGGGGTG TACTTTGCCA GCACCGAGAA GTCCAACATC<br>ATCAGAGGCT GGATCTTCGG CACCACACTG GACAGCAAGA CCCAGAGCCT GCTGATCGTG<br>AACAACGCCA CCAACGTGGT CATCAAAGTG TGCGAGTTCC AGTTCTGCAA CGACCCCTTC<br>CTGGACGTCT ACTACCACAA GAACAACAAG AGCTGGATGG AAAGCGGCGT GTACAGCAGC<br>GCCAACAACT GCACCTTCGA GTACGTGTCC CAGCCTTTCC TGATGGACCT GGAAGGCAAG<br>CAGGGCAACT TCAAGAACCT GCGCGAGTTC GTGTTTAAGA ACATCGACGG CTACTTCAAG<br>ATCTACAGCA AGCACACCCC TATCAACCTC GTGCGGGATC TGCCTCAGGG CTTCTCTGCT<br>CTGGAACCCC TGGTGGATCT GCCCATCGGC ATCAACATCA CCCGGTTTCA GACACTGCTG<br>GCCCTGCACA GAAGCTACCT GACACCTGGC GATAGCAGCA GCGGATGGAC AGCTGGTGCC<br>GCCGCTTACT ATGTGGGCTA CCTGCAGCCT AGAACCTTCC TGCTGAAGTA CAACGAGAAC<br>GGCACCATCA CCGACGCCGT GGATTGTGCT CTGGATCCTC TGAGCGAGAC AAAGTGCACC<br>CTGAAGTCCT TCACCGTGGA AAAGGGCATC TACCAGACCA GCAACTTCCG GGTGCAGCCC<br>ACCGAATCCA TCGTGCGGTT CCCCAATATC ACCAATCTGT GCCCCTTCGG CGAGGTGTTC<br>AATGCCACCA GATTCGCCTC TGTGTACGCC TGGAACCGGA AGCGGATCAG CAATTGCGTG<br>GCCGACTACT CCGTGCTGTA CAACTCCGCC AGCTTCAGCA CCTTCAAGTG CTACGGCGTG<br>TCCCCTACCA AGCTGAACGA CCTGTGCTTC ACAAACGTGT ACGCCGACAG CTTCGTGATC<br>CGGGGAGATG AAGTGCGGCA GATTGCCCCT GGACAGACAG GCAAGATCGC CGACTACAAC<br>TACAAGCTGC CCGACGACTT CACCGGCTGT GTGATTGCCT GGAACAGCAA CAACCTGGAC<br>TCCAAAGTCG GCGGCAACTA CAATTACAGG TACCGGCTGT TCCGGAAGTC CAATCTGAAG<br>CCCTTCGAGC GGGACATCTC CACCGAGATC TATCAGGCCG GCAGCAAGCC TTGTAACGGC<br>GTGGAAGGCT TCAACTGCTA CTTCCCACTG CAGTCCTACG GCTTTCAGCC CACAAATGGC<br>GTGGGCTATC AGCCCTACAG AGTGGTGGTG CTGAGCTTCG AACTGCTGCA TGCCCCTGCC<br>ACAGTGTGCG GCCCTAAGAA AAGCACCAAT CTCGTGAAGA ACAAATGCGT GAACTTCAAC<br>TTCAACGGCC TGACCGGCAC CGGCGTGCTG ACAGAGAGCA ACAAGAAGTT CCTGCCATTC<br>CAGCAGTTTG GCCGGGATAT CGCCGATACC ACAGACGCCG TTAGAGATCC CCAGACACTG<br>GAAATCCTGG ACATCACCCC TTGCAGCTTC GGCGGAGTGT CTGTGATCAC CCCTGGCACC<br>AACACCAGCA ATCAGGTGGC AGTGCTGTAC CAGGGCGTGA ACTGTACCGA AGTGCCCGTG |

(continued)

| SEQ ID NO. | Brief Description | Sequence |
|---|---|---|
| | | GCCATTCACG CCGATCAGCT GACACCTACA TGGCGGGTGT ACTCCACCGG CAGCAATGTG<br>TTTCAGACCA GAGCCGGCTG TCTGATCGGA GCCGAGCACG TGAACAATAG CTACGAGTGC<br>GACATCCCCA TCGGCGCTGG AATCTGCGCC AGCTACCAGA CACAGACAAA CAGCAGGCGG<br>AGAGCCAGAA GCGTGGCCAG CCAGAGCATC ATTGCCTACA CAATGTCTCT GGGCGCCGAG<br>AACAGCGTGG CCTACTCCAA CAACTCTATC GCTATCCCCA CCAACTTCAC CATCAGCGTG<br>ACCACAGAGA TCCTGCCTGT GTCCATGACC AAGACCAGCG TGGACTGCAC CATGTACATC<br>TGCGGCGATT CCACCGAGTG CTCCAACCTG CTGCTGCAGT ACGGCAGCTT CTGCACCCAG<br>CTGAATAGAG CCCTGACAGG GATCGCCGTG GAACAGGACA AGAACACCCA AGAGGTGTTC<br>GCCCAAGTGA AGCAGATCTA CAAGACCCCT CCTATCAAGG ACTTCGGCGG CTTCAATTTC<br>AGCCAGATTC TGCCCGATCC TAGCAAGCCC AGCAAGCGGA GCTTCATCGA GGACCTGCTG<br>TTCAACAAAG TGACACTGGC CGACGCCGGC TTCATCAAGC AGTATGGCGA TTGTCTGGGC<br>GACATTGCCG CCAGGGATCT GATTTGCGCC CAGAAGTTTA ACGGACTGAC AGTGCTGCCT<br>CCTCTGCTGA CCGATGAGAT GATCGCCCAG TACACATCTG CCCTGCTGGC CGGCACAATC<br>ACAAGCGGCT GGACATTTGG AGCAGGCGCC GCTCTGCAGA TCCCCTTTGC TATGCAGATG<br>GCCTACCGGT TCAACGGCAT CGGAGTGACC CAGAATGTGC TGTACGAGAA CCAGAAGCTG<br>ATCGCCAACC AGTTCAACAG CGCCATCGGC AAGATCCAGG ACAGCCTGAG CAGCACAGCA<br>AGCGCCCTGG GAAAGCTGCA GAACGTGGTC AACCAGAATG CCCAGGCACT GAACACCCTG<br>GTCAAGCAGC TGTCCTCCAA CTTCGGCGCC ATCAGCTCTG TGCTGAACGA TATCCTGAGC<br>AGACTGGACC CTCCTGAGGC CGAGGTGCAG ATCGACAGAC TGATCACAGG CAGACTGCAG<br>AGCCTCCAGA CATACGTGAC CCAGCAGCTG ATCAGAGCCG CCGAGATTAG AGCCTCTGCC<br>AATCTGGCCG CCACCAAGAT GTCTGAGTGT GTGCTGGGCC AGAGCAAGAG AGTGGACTTT<br>TGCGGCAAGG GCTACCACCT GATGAGCTTC CCTCAGTCTG CCCCTCACGG CGTGGTGTTT<br>CTGCACGTGA CATATGTGCC CGCTCAAGAG AAGAATTTCA CCACCGCTCC AGCCATCTGC<br>CACGACGGCA AAGCCCACTT TCCTAGAGAA GGCGTGTTCG TGTCCAACGG CACCCATTGG<br>TTCGTGACAC AGCGGAACTT CTACGAGCCC CAGATCATCA CCACCGACAA CACCTTCGTG<br>TCTGGCAACT GCGACGTCGT GATCGGCATT GTGAACAATA CCGTGTACGA CCCTCTGCAG<br>CCCGAGCTGG ACAGCTTCAA AGAGGAACTG GACAAGTACT TTAAGAACCA CACAAGCCCC<br>GACGTGGACC TGGGCGATAT CAGCGGAATC AATGCCAGCG TCGTGAACAT CCAGAAAGAG<br>ATCGACCGGC TGAACGAGGT GGCCAAGAAT CTGAACGAGA GCCTGATCGA CCTGCAAGAA<br>CTGGGGAAGT ACGAGCAGTA CATCAAGTGG CCCTGGTACA TCTGGCTGGG CTTTATCGCC<br>GGACTGATTG CCATCGTGAT GGTCACAATC ATGCTGTGTT GCATGACCAG CTGCTGTAGC<br>TGCCTGAAGG GCTGTTGTAG CTGTGGCAGC TGCTGCAAGT TCGACGAGGA CGATTCTGAG<br>CCCGTGCTGA AGGGCGTGAA ACTGCACTAC ACATGATGA<br>TTTCACCTGG TACTGCATGC ACGCAATGCT AGCTGCCCCT TTCCCGTCCT GGGTACCCCG<br>AGTCTCCCCC GACCTCGGGT CCCAGGTATG CTCCCACCTC CACCTGCCCC ACTCACCACC<br>TCTGCTAGTT CCAGACACCT CCCAAGCACG CAGCAATGCA GCTCAAAACG CTTAGCCTAG<br>CCACACCCCC ACGGGAAACA GCAGTGATTA ACCTTTAGCA ATAAACGAAA GTTTAACTAA<br>GCTATACTAA CCCCAGGGTT GGTCAATTTC GTGCCAGCCA CACCCTGGAG CTAGC<br>AAAAAAAAAA AAAAAAAAAA AAAAAAAAAA GCATATGACT AAAAAAAAAA AAAAAAAAAA |
| | | AAAAAAAAAA AAAAAAAAAA AAAAAAAAAA AAAAAAAAAA AAAAAAAAAA |

**Nucleotide Sequence of RBP020.17 (Omicron BA.1-specific vaccine)**

Nucleotide sequence is shown with individual sequence elements as indicated in bold letters. In addition, the sequence of the translated protein is shown in italic letters below the coding nucleotide sequence (* = stop codon). Red text indicates point mutations in both the nucleotide and amino acid sequences.

```
        10         20         30         40         50  53
AGAAUAAACU AGUAUUCUUC UGGUCCCCAC AGACUCAGAG AGAACCCGCC ACC
```

hAg-Kozak

```
        63         73         83         93        103        113
AUGUUCGUGU UCCUGGUGCU GCUGCCUCUG GUGUCCAGCC AGUGUGUGAA CCUGACCACC
 M  F  V   F  L  V   L  L  P  L   V  S  S   Q  C  V   N  L  T  T
```

S protein omicron

```
       123        133        143        153        163        173
AGAACACAGC UGCCUCCAGC CUACACCAAC AGCUUUACCA GAGGCGUGUA CUACCCCGAC
 R  T  Q   L  P  P   A  Y  T  N   S  F  T   R  G  V   Y  Y  P  D
```

S protein omicron

```
       183        193        203        213        223        233
AAGGUGUUCA GAUCCAGCGU GCUGCACUCU ACCCAGGACC UGUUCCUGCC UUUCUUCAGC
 K  V  F   R  S  S   V  L  H  S   T  Q  D   L  F  L   P  F  F  S
```

S protein omicron

```
       243        253        263        273        283        293
AACGUGACCU GGUUCCACGU GAUCUCCGGC ACCAAUGGCA CCAAGAGAUU CGACAACCCC
 N  V  T   W  F  H   V  I  S  G   T  N  G   T  K  R   F  D  N  P
```

S protein omicron

```
       303        313        323        333        343        353
GUGCUGCCCU UCAACGACGG GGUGUACUUU GCCAGCAUCG AGAAGUCCAA CAUCAUCAGA
 V  L  P   F  N  D   G  V  Y  F   A  S  I   E  K  S   N  I  I  R
```

S protein omicron

```
       363        373        383        393        403        413
GGCUGGAUCU UCGGCACCAC ACUGGACAGC AAGACCCAGA GCCUGCUGAU CGUGAACAAC
 G  W  I   F  G  T   T  L  D  S   K  T  Q   S  L  L   I  V  N  N
```

S protein omicron

```
       423        433        443        453        463        473
GCCACCAACG UGGUCAUCAA AGUGUGCGAG UUCCAGUUCU GCAACGACCC CUUCCUGGAC
 A  T  N   V  V  I   K  V  C  E   F  Q  F   C  N  D   P  F  L  D
```

S protein omicron

```
       483        493        503        513        523        533
CACAAGAACA ACAAGAGCUG GAUGGAAAGC GAGUUCCGGG UGUACAGCAG CGCCAACAAC
 H  K  N   N  K  S   W  M  E  S   E  F  R   V  Y  S   S  A  N  N
```

S protein omicron

```
        543        553        563        573        583        593
UGCACCUUCG AGUACGUGUC CCAGCCUUUC CUGAUGGACC UGGAAGGCAA GCAGGGCAAC
 C  T  F   E  Y  V   S  Q  P  F   L  M  D   L  E  G   K  Q  G  N
```
S protein omicron

```
        603        613        623        633        643        653
UUCAAGAACC UGCGCGAGUU CGUGUUUAAG AACAUCGACG GCUACUUCAA GAUCUACAGC
 F  K  N   L  R  E   F  V  F  K   N  I  D   G  Y  F   K  I  Y  S
```
S protein omicron

```
        663        673        683        693        703        713
AAGCACACCC CUAUCAUCGU GAGAGAGCCC GAGGAUCUGC CUCAGGGCUU CUCUGCUCUG
 K  H  T   P  I  I   V  R  E  P   E  D  L   P  Q  G   F  S  A  L
```
S protein omicron

```
        723        733        743        753        763        773
GAACCCCUGG UGGAUCUGCC CAUCGGCAUC AACAUCACCC GGUUUCAGAC ACUGCUGGCC
 E  P  L   V  D  L   P  I  G  I   N  I  T   R  F  Q   T  L  L  A
```
S protein omicron

```
        783        793        803        813        823        833
CUGCACAGAA GCUACCUGAC ACCUGGCGAU AGCAGCAGCG GAUGGACAGC UGGUGCCGCC
 L  H  R   S  Y  L   T  P  G  D   S  S  S   G  W  T   A  G  A  A
```
S protein omicron

```
        843        853        863        873        883        893
GCUUACUAUG UGGGCUACCU GCAGCCUAGA ACCUUCCUGC UGAAGUACAA CGAGAACGGC
 A  Y  Y   V  G  Y   L  Q  P  R   T  F  L   L  K  Y   N  E  N  G
```
S protein omicron

```
        903        913        923        933        943        953
ACCAUCACCG ACGCCGUGGA UUGUGCUCUG GAUCCUCUGA GCGAGACAAA GUGCACCCUG
 T  I  T   D  A  V   D  C  A  L   D  P  L   S  E  T   K  C  T  L
```
S protein omicron

```
        963        973        983        993       1003       1013
AAGUCCUUCA CCGUGGAAAA GGGCAUCUAC CAGACCAGCA ACUUCCGGGU GCAGCCCACC
 K  S  F   T  V  E   K  G  I  Y   Q  T  S   N  F  R   V  Q  P  T
```
S protein omicron

```
       1023       1033       1043       1053       1063       1073
GAAUCCAUCG UGCGGUUCCC CAAUAUCACC AAUCUGUGCC CCUUCGACGA GGUGUUCAAU
 E  S  I   V  R  F   P  N  I  T   N  L  C   P  F  D   E  V  F  N
```
S protein omicron

```
       1083       1093       1103       1113       1123       1133
GCCACCAGAU UCGCCUCUGU GUACGCCUGG AACCGGAAGC GGAUCAGCAA UUGCGUGGCC
 A  T  R   F  A  S   V  Y  A  W   N  R  K   R  I  S   N  C  V  A
```
S protein omicron

```
       1143       1153       1163       1173       1183       1193
GACUACUCCG UGCUGUACAA CCUGGCCCCC UUCUUCACCU UCAAGUGCUA CGGCGUGUCC
 D  Y  S   V  L  Y   N  L  A  P   F  F  T   F  K  C   Y  G  V  S
```
S protein omicron

```
      1203       1213       1223       1233       1243       1253
CCUACCAAGC UGAACGACCU GUGCUUCACA AACGUGUACG CCGACAGCUU CGUGAUCCGG
  P  T  K   L  N  D   L  C  F  T  N  V  Y   A  D  S   F  V  I  R
                      S protein omicron

      1263       1273       1283       1293       1303       1313
GGAGAUGAAG UGCGGCAGAU UGCCCCUGGA CAGACAGGCA ACAUCGCCGA CUACAACUAC
  G  D  E   V  R  Q   I  A  P  G  Q  T  G   N  I  A   D  Y  N  Y
                      S protein omicron

      1323       1333       1343       1353       1363       1373
AAGCUGCCCG ACGACUUCAC CGGCUGUGUG AUUGCCUGGA ACAGCAACAA GCUGGACUCC
  K  L  P   D  D  F   T  G  C  V  I  A  W   N  S  N   K  L  D  S
                      S protein omicron

      1383       1393       1403       1413       1423       1433
AAAGUCAGCG GCAACUACAA UUACCUGUAC CGGCUGUUCC GGAAGUCCAA UCUGAAGCCC
  K  V  S   G  N  Y   N  Y  L  Y  R  L  F   R  K  S   N  L  K  P
                      S protein omicron

      1443       1453       1463       1473       1483       1493
UUCGAGCGGG ACAUCUCCAC CGAGAUCUAU CAGGCCGGCA ACAAGCCUUG UAACGGCGUG
  F  E  R   D  I  S   T  E  I  Y  Q  A  G   N  K  P   C  N  G  V
                      S protein omicron

      1503       1513       1523       1533       1543       1553
GCCGGCUUCA ACUGCUACUU CCCACUGCGG UCCUACAGCU UUAGGCCCAC AUACGGCGUG
  A  G  F   N  C  Y   F  P  L  R  S  Y  S   F  R  P   T  Y  G  V
                      S protein omicron

      1563       1573       1583       1593       1603       1613
GGCCACCAGC CCUACAGAGU GGUGGUGCUG AGCUUCGAAC UGCUGCAUGC CCCUGCCACA
  G  H  Q   P  Y  R   V  V  V  L  S  F  E   L  L  H   A  P  A  T
                      S protein omicron

      1623       1633       1643       1653       1663       1673
GUGUGCGGCC CUAAGAAAAG CACCAAUCUC GUGAAGAACA AAUGCGUGAA CUUCAACUUC
  V  C  G   P  K  K   S  T  N  L  V  K  N   K  C  V   N  F  N  F
                      S protein omicron

      1683       1693       1703       1713       1723       1733
AACGGCCUGA AGGGCACCGG CGUGCUGACA GAGAGCAACA AGAAGUUCCU GCCAUUCCAG
  N  G  L   K  G  T   G  V  L  T  E  S  N   K  K  F   L  P  F  Q
                      S protein omicron

      1743       1753       1763       1773       1783       1793
CAGUUUGGCC GGGAUAUCGC CGAUACCACA GACGCCGUUA GAGAUCCCCA GACACUGGAA
  Q  F  G   R  D  I   A  D  T  T  D  A  V   R  D  P   Q  T  L  E
                      S protein omicron

      1803       1813       1823       1833       1843       1853
AUCCUGGACA UCACCCCUUG CAGCUUCGGC GGAGUGUCUG UGAUCACCCC UGGCACCAAC
  I  L  D   I  T  P   C  S  F  G  G  V  S   V  I  T   P  G  T  N
                      S protein omicron

      1863       1873       1883       1893       1903       1913
ACCAGCAAUC AGGUGGCAGU GCUGUACCAG GGCGUGAACU GUACCGAAGU GCCCGUGGCC
  T  S  N   Q  V  A   V  L  Y  Q  G  V  N   C  T  E   V  P  V  A
                      S protein omicron

      1923       1933       1943       1953       1963       1973
AUUCACGCCG AUCAGCUGAC ACCUACAUGG CGGGUGUACU CCACCGGCAG CAAUGUGUUU
  I  H  A   D  Q  L   T  P  T  W  R  V  Y   S  T  G   S  N  V  F
                      S protein omicron
```

```
         1983       1993       2003       2013       2023       2033
CAGACCAGAG CCGGCUGUCU GAUCGGAGCC GAGUACGUGA ACAAUAGCUA CGAGUGCGAC
 Q  T  R   A  G  C   L  I  G  A   E  Y  V   N  N  S   Y  E  C  D
```

S protein omicron

```
         2043       2053       2063       2073       2083       2093
AUCCCCAUCG GCGCUGGAAU CUGCGCCAGC UACCAGACAC AGACAAAGAG CCACCGGAGA
 I  P  I   G  A  G   I  C  A  S   Y  Q  T   Q  T  K   S  H  R  R
```

S protein omicron

```
         2103       2113       2123       2133       2143       2153
GCCAGAAGCG UGGCCAGCCA GAGCAUCAUU GCCUACACAA UGUCUCUGGG CGCCGAGAAC
 A  R  S   V  A  S   Q  S  I  I   A  Y  T   M  S  L   G  A  E  N
```

S protein omicron

```
         2163       2173       2183       2193       2203       2213
AGCGUGGCCU ACUCCAACAA CUCUAUCGCU AUCCCCACCA ACUUCACCAU CAGCGUGACC
 S  V  A   Y  S  N   N  S  I  A   I  P  T   N  F  T   I  S  V  T
```

S protein omicron

```
         2223       2233       2243       2253       2263       2273
ACAGAGAUCC UGCCUGUGUC CAUGACCAAG ACCAGCGUGG ACUGCACCAU GUACAUCUGC
 T  E  I   L  P  V   S  M  T  K   T  S  V   D  C  T   M  Y  I  C
```

S protein omicron

```
         2283       2293       2303       2313       2323       2333
GGCGAUUCCA CCGAGUGCUC CAACCUGCUG CUGCAGUACG GCAGCUUCUG CACCCAGCUG
 G  D  S   T  E  C   S  N  L  L   L  Q  Y   G  S  F   C  T  Q  L
```

S protein omicron

```
         2343       2353       2363       2373       2383       2393
AAAAGAGCCC UGACAGGGAU CGCCGUGGAA CAGGACAAGA ACACCCAAGA GGUGUUCGCC
 K  R  A   L  T  G   I  A  V  E   Q  D  K   N  T  Q   E  V  F  A
```

S protein omicron

```
         2403       2413       2423       2433       2443       2453
CAAGUGAAGC AGAUCUACAA GACCCCUCCU AUCAAGUACU UCGGCGGCUU CAAUUUCAGC
 Q  V  K   Q  I  Y   K  T  P  P   I  K  Y   F  G  G   F  N  F  S
```

S protein omicron

```
         2463       2473       2483       2493       2503       2513
CAGAUUCUGC CCGAUCCUAG CAAGCCCAGC AAGCGGAGCU UCAUCGAGGA CCUGCUGUUC
 Q  I  L   P  D  P   S  K  P  S   K  R  S   F  I  E   D  L  L  F
```

S protein omicron

```
         2523       2533       2543       2553       2563       2573
AACAAAGUGA CACUGGCCGA CGCCGGCUUC AUCAAGCAGU AUGGCGAUUG UCUGGGCGAC
 N  K  V   T  L  A   D  A  G  F   I  K  Q   Y  G  D   C  L  G  D
```

S protein omicron

```
         2583       2593       2603       2613       2623       2633
AUUGCCGCCA GGGAUCUGAU UUGCGCCCAG AAGUUUAAGG GACUGACAGU GCUGCCUCCU
 I  A  A   R  D  L   I  C  A  Q   K  F  K   G  L  T   V  L  P  P
```

S protein omicron

```
         2643       2653       2663       2673       2683       2693
CUGCUGACCG AUGAGAUGAU CGCCCAGUAC ACAUCUGCCC UGCUGGCCGG CACAAUCACA
 L  L  T   D  E  M   I  A  Q  Y   T  S  A   L  L  A   G  T  I  T
```

S protein omicron

```
       2703       2713       2723       2733       2743       2753
AGCGGCUGGA CAUUUGGAGC AGGCGCCGCU CUGCAGAUCC CCUUUGCUAU GCAGAUGGCC
  S  G  W   T  F  G   A  G  A  A   L  Q  I   P  F  A   M  Q  M  A
                         S protein omicron

       2763       2773       2783       2793       2803       2813
UACCGGUUCA ACGGCAUCGG AGUGACCCAG AAUGUGCUGU ACGAGAACCA GAAGCUGAUC
  Y  R  F   N  G  I   G  V  T  Q   N  V  L   Y  E  N   Q  K  L  I
                         S protein omicron

       2823       2833       2843       2853       2863       2873
GCCAACCAGU UCAACAGCGC CAUCGGCAAG AUCCAGGACA GCCUGAGCAG CACAGCAAGC
  A  N  Q   F  N  S   A  I  G  K   I  Q  D   S  L  S   S  T  A  S
                         S protein omicron

       2883       2893       2903       2913       2923       2933
GCCCUGGGAA AGCUGCAGGA CGUGGUCAAC CACAAUGCCC AGGCACUGAA CACCCUGGUC
  A  L  G   K  L  Q   D  V  V  N   H  N  A   Q  A  L   N  T  L  V
                         S protein omicron

       2943       2953       2963       2973       2983       2993
AAGCAGCUGU CCUCCAAGUU CGGCGCCAUC AGCUCUGUGC UGAACGAUAU CUUCAGCAGA
  K  Q  L   S  S  K   F  G  A  I   S  S  V   L  N  D   I  F  S  R
                         S protein omicron

       3003       3013       3023       3033       3043       3053
CUGGACCCUC CUGAGGCCGA GGUGCAGAUC GACAGACUGA UCACAGGCAG ACUGCAGAGC
  L  D  P   P  E  A   E  V  Q  I   D  R  L   I  T  G   R  L  Q  S
                         S protein omicron

       3063       3073       3083       3093       3103       3113
CUCCAGACAU ACGUGACCCA GCAGCUGAUC AGAGCCGCCG AGAUUAGAGC CUCUGCCAAU
  L  Q  T   Y  V  T   Q  Q  L  I   R  A  A   E  I  R   A  S  A  N
                         S protein omicron

       3123       3133       3143       3153       3163       3173
CUGGCCGCCA CCAAGAUGUC UGAGUGUGUG CUGGGCCAGA GCAAGAGAGU GGACUUUUGC
  L  A  A   T  K  M   S  E  C  V   L  G  Q   S  K  R   V  D  F  C
                         S protein omicron

       3183       3193       3203       3213       3223       3233
GGCAAGGGCU ACCACCUGAU GAGCUUCCCU CAGUCUGCCC CUCACGGCGU GGUGUUUCUG
  G  K  G   Y  H  L   M  S  F  P   Q  S  A   P  H  G   V  V  F  L
                         S protein omicron

       3243       3253       3263       3273       3283       3293
CACGUGACAU AUGUGCCCGC UCAAGAGAAG AAUUUCACCA CCGCUCCAGC CAUCUGCCAC
  H  V  T   Y  V  P   A  Q  E  K   N  F  T   T  A  P   A  I  C  H
                         S protein omicron

       3303       3313       3323       3333       3343       3353
GACGGCAAAG CCCACUUUCC UAGAGAAGGC GUGUUCGUGU CCAACGGCAC CCAUUGGUUC
  D  G  K   A  H  F   P  R  E  G   V  F  V   S  N  G   T  H  W  F
                         S protein omicron

       3363       3373       3383       3393       3403       3413
GUGACACAGC GGAACUUCUA CGAGCCCCAG AUCAUCACCA CCGACAACAC CUUCGUGUCU
  V  T  Q   R  N  F   Y  E  P  Q   I  I  T   T  D  N   T  F  V  S
                         S protein omicron
```

```
       3423       3433       3443       3453       3463       3473
GGCAACUGCG ACGUCGUGAU CGGCAUUGUG AACAAUACCG UGUACGACCC UCUGCAGCCC
 G  N  C   D  V  V   I  G  I  V   N  N  T   V  Y  D   P  L  Q  P
```
**S protein omicron**

```
       3483       3493       3503       3513       3523       3533
GAGCUGGACA GCUUCAAAGA GGAACUGGAC AAGUACUUUA AGAACCACAC AAGCCCCGAC
 E  L  D   S  F  K   E  E  L  D   K  Y  F   K  N  H   T  S  P  D
```
**S protein omicron**

```
       3543       3553       3563       3573       3583       3593
GUGGACCUGG GCGAUAUCAG CGGAAUCAAU GCCAGCGUCG UGAACAUCCA GAAAGAGAUC
 V  D  L   G  D  I   S  G  I  N   A  S  V   V  N  I   Q  K  E  I
```
**S protein omicron**

```
       3603       3613       3623       3633       3643       3653
GACCGGCUGA ACGAGGUGGC CAAGAAUCUG AACGAGAGCC UGAUCGACCU GCAAGAACUG
 D  R  L   N  E  V   A  K  N  L   N  E  S   L  I  D   L  Q  E  L
```
**S protein omicron**

```
       3663       3673       3683       3693       3703       3713
GGGAAGUACG AGCAGUACAU CAAGUGGCCC UGGUACAUCU GGCUGGGCUU UAUCGCCGGA
 G  K  Y   E  Q  Y   I  K  W  P   W  Y  I   W  L  G   F  I  A  G
```
**S protein omicron**

```
       3723       3733       3743       3753       3763       3773
CUGAUUGCCA UCGUGAUGGU CACAAUCAUG CUGUGUUGCA UGACCAGCUG CUGUAGCUGC
 L  I  A   I  V  M   V  T  I  M   L  C  C   M  T  S   C  C  S  C
```
**S protein omicron**

```
       3783       3793       3803       3813       3823       3833
CUGAAGGGCU GUUGUAGCUG UGGCAGCUGC UGCAAGUUCG ACGAGGACGA UUCUGAGCCC
 L  K  G   C  C  S   C  G  S  C   C  K  F   D  E  D   D  S  E  P
```
**S protein omicron**

```
       3843       3853       3863  3869
GUGCUGAAGG GCGUGAAACU GCACUACACA UGAUGA
 V  L  K   G  V  K   L  H  Y  T   *  *
```
**S protein omicron**

```
       3879       3889       3999       3909       3919       3929
CUCGAGCUGG UACUGCAUGC ACGCAAUGCU AGCUGCCCCU UUCCCGUCCU GGGUACCCCG
```
**FI element**

```
       3939       3949       3959       3969       3979       3989
AGUCUCCCCC GACCUCGGGU CCCAGGUAUG CUCCCACCUC CACCUGCCCC ACUCACCACC
```
**FI element**

```
       3999       4009       4019       4029       4039       4049
UCUGCUAGUU CCAGACACCU CCCAAGCACG CAGCAAUGCA GCUCAAAACG CUUAGCCUAG
```
**FI element**

```
       4059       4069       4079       4089       4099       4109
CCACACCCCC ACGGGAAACA GCAGUGAUUA ACCUUUAGCA AUAAACGAAA GUUUAACUAA
```
**FI element**

4119 4129 4139 4149 4159 4164
GCUAUACUAA CCCCAGGGUU GGUCAAUUUC GUGCCAGCCA CACCCUGGAG CUAGC
**FI element**

4174 4184 4194 4204 4214 4224
AAAAAAAAAA AAAAAAAAAA AAAAAAAAAA GCAUAUGACU AAAAAAAAAA AAAAAAAAAA
**Poly(A)**

4234 4244 4254 4264 4274
AAAAAAAAAA AAAAAAAAAA AAAAAAAAAA AAAAAAAAAA AAAAAAAAAA
**Poly(A)**

Sequences of RBP020.17 are also shown in Table 5.

**Table 5:** Sequences of RBP020.17 Omicron BA.1-specific RNA vaccine)

| SEQ ID NO. | Brief Description | Sequence |
|---|---|---|
| 31 | Amino acid sequence of RNA-encoded SARS-CoV-2 S protein from an Omicron BA.1 variant | MFVFLVLLPLVSSQCVNLTTRTQLPPAYTNSFTRGVYYPDKVFRSSVLHSTQDLFLPFFSNVTWF HVISGTNGTKRFDNPVLPFNDGVYFASIEKSNIIRGWIFGTTLDSKTQSLLIVNNATNVVIKVCE FQFCNDPFLDHKNNKSWMESEFRVYSSANNCTFEYVSQPFLMDLEGKQGNFKNLREFVFKNIDGY FKIYSKHTPIIVREPEDLPQGFSALEPLVDLPIGINITRFQTLLALHRSYLTPGDSSSGWTAGAA AYYVGYLQPRTFLLKYNENGTITDAVDCALDPLSETKCTLKSFTVEKGIYQTSNFRVQPTESIVR FPNITNLCPFDEVFNATRFASVYAWNRKRISNCVADYSVLYNLAPFFTFKCYGVSPTKLNDLCFT NVYADSFVIRGDEVRQIAPGQTGNIADYNYKLPDDFTGCVIAWNSNKLDSKVSGNYNYLYRLFRK SNLKPFERDISTEIYQAGNKPCNGVAGFNCYFPLRSYSFRPTYGVGHQPYRVVVLSFELLHAPAT VCGPKKSTNLVKNKCVNFNFNGLKGTGVLTESNKKFLPFQQFGRDIADTTDAVRDPQTLEILDIT PCSFGGVSVITPGTNTSNQVAVLYQGVNCTEVPVAIHADQLTPTWRVYSTGSNVFQTRAGCLIGA EYVNNSYECDIPIGAGICASYQTQTKSHRRARSVASQSIIAYTMSLGAENSVAYSNNSIAIPTNF TISVTTEILPVSMTKTSVDCTMYICGDSTECSNLLLQYGSFCTQLKRALTGIAVEQDKNTQEVFA QVKQIYKTPPIKYFGGFNFSQILPDPSKPSKRSFIEDLLFNKVTLADAGFIKQYGDCLGDIAARD LICAQKFKGLTVLPPLLTDEMIAQYTSALLAGTITSGWTFGAGAALQIPFAMQMAYRFNGIGVTQ NVLYENQKLIANQFNSAIGKIQDSLSSTASALGKLQDVVNHNAQALNTLVKQLSSKFGAISSVLN DIFSRLDPPEAEVQIDRLITGRLQSLQTYVTQQLIRAAEIRASANLAATKMSECVLGQSKRVDFC GKGYHLMSFPQSAPHGVVFLHVTYVPAQEKNFTTAPAICHDGKAHFPREGVFVSNGTHWFVTQRN FYEPQIITTDNTFVSGNCDVVIGIVNNTVYDPLQPELDSFKEELDKYFKNHTSPDVDLGDISGIN ASVVNIQKEIDRLNEVAKNLNESLIDLQELGKYEQYIKWPWYIWLGFIAGLIAIVMVTIMLCCMT SCCSCLKGCCSCGSCCKFDEDDSEPVLKGVKLHYT** |
| 32 | RNA sequence encoding a SARS-CoV-2 S protein from an Omicron BA.1 variant | auguucgugu uccuggugcu gcugccucug guguccagcc agugugugaa ccugaccacc agaacacagc ugccuccagc cuacaccaac agcuuuacca gaggcgugua cuaccccgac aagguguuca gauccagcgu gcugcacucu acccaggacc uguuccugcc uuucuucagc aacgugaccu gguuccacgu gaucuccggc accaauggca ccaagagauu cgacaacccc gugcugcccu ucaacgacgg gguguacuuu gccagcaucg agaaguccaa caucaucaga ggcuggaucu ucggcaccac acuggacagc aagacccaga gccugcugau cgugaacaac gccaccaacg uggucaucaa agugugcgag uuccaguucu gcaacgaccc cuuccuggac cacaagaaca acaagagcug gauggaaagc gaguuccggg uguacagcag cgccaacaac ugcaccuucg aguacguguc ccagccuuuc cugauggacc uggaaggcaa gcagggcaac uucaagaacc ugcgcgaguu cguguuuaag aacaucgacg gcuacuucaa gaucuacagc aagcacaccc cuaucaucgu gagagagccc gaggaucugc cucagggcuu cucugcucug gaaccccugg uggaucugcc caucggcauc aacaucaccc gguuucagac acugcuggcc cugcacagaa gcuaccugac accuggcgau agcagcagcg gauggacagc uggugccgcc |

(continued)

| SEQ ID NO. | Brief Description | Sequence |
|---|---|---|
| | | gcuuacuaug ugggcuaccu gcagccuaga accuuccugc ugaaguacaa cgagaacggc accaucaccg acgccgugga uugugcucug gauccucuga gcgagacaaa gugcacccug aaguccuuca ccguggaaaa gggcaucuac cagaccagca acuuccgggu gcagcccacc gaauccaucg ugcgguuccc caauaucacc aaucugugcc ccuucgacga gguguucaau gccaccagau ucgccucugu guacgccugg aaccggaagc ggaucagcaa uugcguggcc gacuacuccg ugcuguacaa ccuggccccc uucuucaccu ucaagugcua cggcgugucc ccuaccaagc ugaacgaccu gugcuucaca aacguguacg ccgacagcuu cgugauccgg ggagaugaag ugcggcagau ugccccugga cagacaggca acaucgccga cuacaacuac aagcugcccg acgacuucac cggcugugug auugccugga acagcaacaa gcuggacucc aaagucagcg gcaacuacaa uuaccuguac cggcuguucc ggaaguccaa ucugaagccc uucgagcggg acaucuccac cgagaucuau caggccggca acaagccuug uaacggcgug gccggcuuca acugcuacuu cccacugcgg uccuacagcu uuaggcccac auacggcgug ggccaccagc ccuacagagu gguggugcug agcuucgaac ugcugcaugc cccugccaca gugugcggcc cuaagaaaag caccaaucuc gugaagaaca aaugcgugaa cuucaacuuc aacggccuga agggcaccgg cgugcugaca gagagcaaca agaaguuccu gccauuccag caguuuggcc gggauaucgc cgauaccaca gacgccguua gagaucccca gacacuggaa auccuggaca ucaccccuug cagcuucggc ggagugucug ugaucacccc uggcaccaac accagcaauc agguggcagu gcuguaccag ggcgugaacu guaccgaagu gcccguggcc auucacgccg aucagcugac accuacaugg cggguguacu ccaccggcag caauguguuu cagaccagag ccggcugucu gaucggagcc gaguacguga acaauagcua cgagugcgac auccccaucg gcgcuggaau cugcgccagc uaccagacac agacaaagag ccaccggaga gccagaagcg uggccagcca gagcaucauu gccuacacaa ugucucuggg cgccgagaac agcguggccu acuccaacaa cucuaucgcu auccccacca acuucaccau cagcgugacc acagagaucc ugccugugucc caugaccaag accagcgugg acugcaccau guacaucugc ggcgauucca ccgagugcuc caaccugcug cugcaguacg gcagcuucug cacccagcug aaaagagccc ugacagggau cgccguggaa caggacaaga acacccaaga gguguucgcc caagugaagc agaucuacaa gaccccuccu aucaaguacu ucggcggcuu caauuucagc cagauucugc ccgauccuag caagcccagc aagcggagcu ucaucgagga ccugcuguuc aacaaaguga cacuggccga cgccggcuuc aucaagcagu auggcgauug ucugggcgac auugccgcca gggaucugau uugcgcccag aaguuuaagg gacugacagu gcugccuccu cugcugaccg augagaugau cgcccaguac acaucugccc ugcuggccgg cacaaucaca agcggcugga cauuuggagc aggcgccgcu cugcagaucc ccuuugcuau gcagauggcc uaccgguuca acggcaucgg agugacccag aaugugcugu acgagaacca gaagcugauc gccaaccagu ucaacagcgc caucggcaag auccaggaca gccugagcag cacagcaagc gcccugggaa agcugcagga cguggucaac cacaaugccc aggcacugaa cacccugguc aagcagcugu ccuccaaguu cggcgccauc agcucugugc ugaacgauau cuucagcaga |
| | | cuggacccuc cugaggccga ggugcagauc gacagacuga ucacaggcag acugcagagc cuccagacau acgugaccca gcagcugauc agagccgccg agauuagagc cucugccaau cuggccgcca ccaagauguc ugagugugug cugggccaga gcaagagagu ggacuuuugc ggcaagggcu accaccugau gagcuucccu cagucugccc cucacggcgu gguguuucug cacgugacau augugcccgc ucaagagaag aauuucacca ccgcuccagc caucugccac gacggcaaag cccacuuucc uagagaaggc guguucgugu ccaacggcac ccauugguuc gugacacagc ggaacuucua cgagccccag aucaucacca ccgacaacac cuucgugucu ggcaacugcg acgucgugau cggcauugug aacaauaccg uguacgaccc ucugcagccc gagcuggaca gcuucaaaga ggaacuggac aaguacuuua agaaccacac aagccccgac guggaccugg gcgauaucag cggaaucaau gccagcgucg ugaacaucca gaaagagauc gaccggcuga acgagguggc caagaaucug aacgagagcc ugaucgaccu gcaagaacug ggaaguacg agcaguacau caaguggccc ugguacaucu ggcugggcuu uaucgccgga cugauugcca ucgugauggu cacaaucaug cuguguugca ugaccagcug cuguagcugc cugaagggcu guuguagcug uggcagcugc ugcaaguucg acgaggacga uucugagccc gugcugaagg gcgugaaacu gcacuacaca ugauga |

(continued)

| SEQ ID NO. | Brief Description | Sequence |
|---|---|---|
| 33 | Full length sequence of RBP020.17 | agaauaaacu aguauucuuc ugguccccac agacucagag agaacccgcc accauguucg<br>uguuccuggu gcugcugccu cuggugucca gccagugugu gaaccugacc accagaacac<br>agcugccucc agccuacacc aacagcuuua ccagaggcgu guacuacccc gacaaggugu<br>ucagauccag cgugcugcac ucuacccagg accuguuccu gccuuucuuc agcaacguga<br>ccugguucca cgugaucucc ggcaccaaug gcaccaagag auucgacaac cccgugcugc<br>ccuucaacga cggggugauc uuugccagca ucgagaaguc caacaucauc agaggcugga<br>ucuucggcac cacacuggac agcaagaccc agagccugcu gaucgugaac aacgccacca<br>acguggucau caaagugugc gaguuccagu ucugcaacga ccccuuccug gaccacaaga<br>acaacaagag cuggauggaa agcgaguucc ggguguacag cagcgccaac aacugcaccu<br>ucgaguacgu gucccagccu uuccugaugg accuggaagg caagcagggc aacuucaaga<br>accugcgcga guucguguuu aagaacaucg acggcuacuu caagaucuac agcaagcaca<br>ccccuaucau cgugagagag cccgaggauc ugccucaggg cuucucugcu cuggaacccc<br>ugguggaucu gcccaucggc aucaacauca cccgguuuca gacacugcug gcccugcaca<br>gaagcuaccu gacaccuggc gauagcagca gcggauggac agcuggugcc gccgcuuacu<br>auguggcua ccugcagccu agaaccuucc ugcugaagua caacgagaac ggcaccauca<br>ccgacgccgu ggauugugcu cuggauccuc ugagcgagac aaagugcacc cugaaguccu<br>ucaccgugga aaagggcauc uaccagacca gcaacuuccg ggugcagccc accgaaucca<br>ucgugcgguu ccccaauauc accaaucugu gccccuucga cgagguguuc aaugccacca<br>gauucgccuc uguguacgcc uggaaccgga agcggaucag caauugcgug gccgacuacu<br>ccgugcugua caaccuggcc cccuucuuca ccuucaagug cuacggcgug uccccuacca<br>agcugaacga ccugugcuuc acaaacgugu acgccgacag cuucgugauc cggggagaug |
|  |  | aagugcggca gauugccccu ggacagacag gcaacaucgc cgacuacaac uacaagcugc<br>ccgacgacuu caccggcugu gugauugccu ggaacagcaa caagcuggac uccaaaguca<br>gcggcaacua caauuaccug uaccggcugu uccggaaguc caaucugaag cccuucgagc<br>gggacaucuc caccgagauc uaucaggccg gcaacaagcc uuguaacggc guggccggcu<br>ucaacugcua cuucccacug cgguccuaca gcuuuaggcc cacauacggc gugggccacc<br>agcccuacag agugguggug cugagcuucg aacugcugca ugccccugcc acagugugcg<br>gcccuaagaa aagcaccaau cucgugaaga acaaaugcgu gaacuucaac uucaacggcc<br>ugaagggcac cggcgugcug acagagagca acaagaaguu ccugccauuc cagcaguuug<br>gccgggauau cgccgauacc acagacgccg uuagagaucc ccagacacug gaaauccugg<br>acaucacccc uugcagcuuc ggcggagugu cugugaucac cccuggcacc aacaccagca<br>aucagguggc agugcuguac cagggcguga acuguaccga agugcccgug gccauucacg<br>ccgaucagcu gacaccuaca uggcgggugu acuccaccgg cagcaaugug uuucagacca<br>gagccggcug ucugaucgga gccgaguacg ugaacaauag cuacgagugc gacaucccca<br>ucggcgcugg aaucugcgcc agcuaccaga cacagacaaa gagccaccgg agagccagaa<br>gcguggccag ccagagcauc auugccuaca caaugucucu gggcgccgag aacagcgugg<br>ccuacuccaa caacucuauc gcuaucccca ccaacuucac caucagcgug accacagaga<br>uccugccugu guccaugacc aagaccagcg uggacugcac cauguacauc ugcggcgauu<br>ccaccgagug cuccaaccug cugcugcagu acggcagcuu cugcacccag cugaaaagag<br>cccugacagg gaucgccgug gaacaggaca agaacaccca agagguguuc gcccaaguga<br>agcagaucua caagaccccu ccuaucaagu acuucggcgg cuucaauuuc agccagauuc<br>ugcccgaucc uagcaagccc agcaagcgga gcuucaucga ggaccugcug uucaacaaag<br>ugacacuggc cgacgccggc uucaucaagc aguauggcga uugucugggc gacauugccg<br>ccagggaucu gauuugcgcc cagaaguuua agggacugac agugcugccu ccucugcuga<br>ccgaugagau gaucgcccag uacacaucug cccugcuggc cggcacaauc acaagcggcu<br>ggacauuugg agcaggcgcc gcucugcaga uccccuuugc uaugcagaug gccuaccggu<br>ucaacggcau cggagugacc cagaaugugc uguacgagaa ccagaagcug aucgccaacc<br>aguucaacag cgccaucggc aagauccagg acagccugag cagcacagca agcgcccugg<br>gaaagcugca ggacgugguc aaccacaaug cccaggcacu gaacacccug gucaagcagc<br>uguccuccaa guucggcgcc aucagcucug ugcugaacga uaucuucagc agacuggacc<br>cuccugaggc cgaggugcag aucgacagac ugaucacagg cagacugcag agccuccaga<br>cauacgugac ccagcagcug aucagagccg ccgagauuag agccucugcc aaucuggccg<br>ccaccaagau gucugagugu gugcugggcc agagcaagag aguggacuuu ugcggcaagg<br>gcuaccaccu gaugagcuuc ccucagucug ccccucacgg cgugguguuu cugcacguga<br>cauaugugcc cgcucaagag aagaauuuca ccaccgcucc agccaucugc cacgacggca<br>aagcccacuu uccuagagaa ggcguguucg uguccaacgg cacccauugg uucgugacac<br>agcggaacuu cuacgagccc cagaucauca ccaccgacaa caccuucgug ucuggcaacu |

(continued)

| SEQ ID NO. | Brief Description | Sequence |
|---|---|---|
| | | gcgacgucgu gaucggcauu gugaacaaua ccguguacga cccucugcag cccgagcugg acagcuucaa agaggaacug gacaaguacu uuaagaacca cacaagcccc gacguggacc ugggcgauau cagcggaauc aaugccagcg ucgugaacau ccagaaagag aucgaccggc ugaacgaggu ggccaagaau cugaacgaga gccugaucga ccugcaagaa cuggggaagu acgagcagua caucaagugg cccugguaca ucuggcuggg cuuuaucgcc ggacugauug ccaucgugau ggucacaauc augcuguguu gcaugaccag cugcuguagc ugccugaagg gcuguuguag cuguggcagc ugcugcaagu ucgacgagga cgauucugag cccgugcuga agggcgugaa acugcacuac acaugaugac ucgagcuggu acugcaugca cgcaaugcua gcugccccuu ucccguccug gguaccccga gucucccccg accucggguc ccagguaugc ucccaccucc accugcccca cucaccaccu cugcuaguuc cagacaccuc ccaagcacgc agcaaugcag cucaaaacgc uuagccuagc cacaccccca cgggaaacag cagugauuaa ccuuuagcaa uaaacgaaag uuuaacuaag cuauacuaac cccaggguug gucaauuucg ugccagccac acccuggagc uagcaaaaaa aaaaaaaaaa aaaaaaaaaa aaaagcauau gacuaaaaaa aaaaaaaaaa aaaaaaaaaa aaaaaaaaaa aaaaaaaaaa aaaaaaaaaa aaaaaaaaaa aaaa |

## Detailed Description of Certain Embodiments

Although the present disclosure is further described in more detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

In the following, the elements of the present disclosure will be described in more detail. These elements are listed with specific embodiments, however, it should be understood that they may be combined in any manner and in any number to create additional embodiments. The variously described examples and preferred embodiments should not be construed to limit the present disclosure to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

The practice of the present disclosure will employ, unless otherwise indicated, conventional chemistry, biochemistry, cell biology, immunology, and recombinant DNA techniques which are explained in the literature in the field.

Throughout this specification and the claims which follow, unless the context requires otherwise, the word "comprise", and variations such as "comprises" and "comprising", will be understood to imply the inclusion of a stated feature, element, member, integer or step or group of features, elements, members, integers or steps but not the exclusion of any other feature, element, member, integer or step or group of features, elements, members, integers or steps. The term "consisting essentially of" limits the scope of a claim or disclosure to the specified features, elements, members, integers, or steps and those that do not materially affect the basic and novel characteristic(s) of the claim or disclosure. The term "consisting of" limits the scope of a claim or disclosure to the specified features, elements, members, integers, or steps. The term "comprising" encompasses the term "consisting essentially of" which, in turn, encompasses the term "consisting of". Thus, at each occurrence in the present application, the term "comprising" may be replaced with the term "consisting essentially of" or "consisting of". Likewise, at each occurrence in the present application, the term "consisting essentially of" may be replaced with the term "consisting of".

The terms "a", "an" and "the" and similar references used in the context of describing the present disclosure (especially in the context of the claims) are to be construed to cover both the singular and the plural, unless otherwise indicated herein or clearly contradicted by the context.

All methods described herein can be performed in any suitable order unless otherwise indicated herein or otherwise clearly contradicted by the context.

The use of any and all examples, or exemplary language (*e.g.*, "such as"), provided herein is intended merely to better illustrate the present disclosure and does not pose a limitation on the scope of the present disclosure otherwise claimed. No language in the specification should be construed as indicating any non-claimed element essential to the practice of the present disclosure.

The term "optional" or "optionally" as used herein means that the subsequently described event, circumstance

or condition may or may not occur, and that the description includes instances where said event, circumstance, or condition occurs and instances in which it does not occur.

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present disclosure, the term "about" denotes an interval of accuracy that the person of ordinary skill will understand to still ensure the technical effect of the feature in question. The term typically indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical value by ±0.3%. In some embodiments, "about" indicates deviation from the indicated numerical value by ±0.2%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Recitation of ranges of values herein is merely intended to serve as a shorthand method of referring individually to each separate value falling within the range. Unless otherwise indicated herein, each individual value is incorporated into the specification as if it were individually recited herein.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

**Methods for providing a pharmaceutical RNA preparation**

In some embodiments, the invention provides a method for providing a pharmaceutical RNA preparation for the administration of different doses of the RNA comprising the steps:

(i) determining different doses in which the RNA is to be administered,
(ii) determining a concentration of the RNA in the pharmaceutical RNA preparation which allows the administration of the different doses of the RNA with suitable administration volumes and, optionally, suitable dilution of the pharmaceutical RNA preparation, and
(iii) determining a suitable formulation for the pharmaceutical RNA preparation to ensure a desired storage stability of the RNA in the pharmaceutical RNA preparation at the determined concentration.

The method of the invention aims at providing a single pharmaceutical preparation comprising RNA that optionally is formulated, e.g., in particles such as LNPs, wherein such single pharmaceutical preparation may be used for administering different doses of the RNA.

In some embodiments, at least some of the different doses in which the RNA is to be administered should differ significantly, e.g., by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the lowest dose of the RNA and the largest dose of the RNA differ by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the administration volumes for the different doses in which the RNA is to be administered

should not differ significantly, e.g., by a factor of 2 or less, 1.9 or less, 1.8 or less, 1.7 or less, 1.6 or less, 1.5 or less, 1.4 or less, 1.3 or less, 1.2 or less, or 1.1 or less. In some embodiments, the administration volumes for the different doses in which the RNA is to be administered do not differ, i.e., the same administration volumes are to be used for the different doses.

In some embodiments, the smallest administration volume for one dose and the largest administration volume for another dose differ by a factor of 2 or less, 1.9 or less, 1.8 or less, 1.7 or less, 1.6 or less, 1.5 or less, 1.4 or less, 1.3 or less, 1.2 or less, or 1.1 or less.

In some embodiments, suitable administration volumes for each dose are 100 to 400 μl. In some embodiments, suitable administration volumes for each dose are 150 to 350 μl. In some embodiments, suitable administration volumes for each dose are 200 to 300 μl. In some embodiments, suitable administration volumes for each dose are about 200 μl, about 250 μl or about 300 μl.

In some embodiments, the lowest dose of the RNA and the largest dose of the RNA differ by a factor of 2 or more (e.g., by a factor of 2 to 20, 2 to 15, 2 to 10, 2 to 9, 2 to 8, 2 to 7, 2 to 6, 2 to 5, 2 to 4, or 2 to 3) and the smallest administration volume for one dose and the largest administration volume for another dose differ by a factor of 2 or less. In some embodiments, the lowest dose of the RNA and the largest dose of the RNA differ by a factor of 5 or more (e.g., by a factor of 5 to 20, 5 to 15, 5 to 10, 5 to 9, 5 to 8, 5 to 7, or 5 to 6) and the smallest administration volume for one dose and the largest administration volume for another dose differ by a factor of 2 or less. In some embodiments, the lowest dose of the RNA and the largest dose of the RNA differ by a factor of 10 or more (e.g., by a factor of 10 to 20, 10 to 19, 10 to 18, 10 to 17,10 to 16, 10 to 15, 10 to 14,10 to 13, 10 to 12, or 10 to 11) and the smallest administration volume for one dose and the largest administration volume for another dose differ by a factor of 2 or less (e.g., by a factor of 1 to 2).

In some embodiments, the lowest dose of the RNA and the largest dose of the RNA differ by a factor of 2 or more (e.g., by 2 to 10) and the smallest administration volume for one dose and the largest administration volume for another dose differ by a factor of 1.5 or less (e.g., by a factor of 1.5 to 1). In some embodiments, the lowest dose of the RNA and the largest dose of the RNA differ by a factor of 5 or more (e.g., by 5 to 10) and the smallest administration volume for one dose and the largest administration volume for another dose differ by a factor of 1.5 or less (e.g., by a factor of 1 to 1.5). In some embodiments, the lowest dose of the RNA and the largest dose of the RNA differ by a factor of 10 or more (e.g., by a factor of 10 to 20) and the smallest administration volume for one dose and the largest administration volume for another dose differ by a factor of 1.5 or less (e.g., by a factor of 1 to 1.5).

In some embodiments, the lowest dose of the RNA and the largest dose of the RNA differ by a factor of 2 or more (e.g., by a factor of 2 to 10) and suitable administration volumes are 100 to 400 μ!. In some embodiments, the lowest dose of the RNA and the largest dose of the RNA differ by a factor of 2 or more (e.g., by a factor of 2 to 10) and suitable administration volumes are 200 to 300 μl. In some embodiments, the lowest dose of the RNA and the largest dose of the RNA differ by a factor of 5 or more (e.g., by a factor of 5 to 10) and suitable administration volumes are 100 to 400 μ!. In some embodiments, the lowest dose of the RNA and the largest dose of the RNA differ by a factor of 5 or more (e.g., by a factor of 5 to 10) and suitable administration volumes are 200 to 300 μ!. In some embodiments, the lowest dose of the RNA and the largest dose of the RNA differ by a factor of 10 or more (e.g., by a factor of 10 to 20) and suitable administration volumes are 100 to 400 μ!. In some embodiments, the lowest dose of the RNA and the largest dose of the RNA differ by a factor of 10 or more (e.g., by a factor of 10 to 20) and suitable administration volumes are 200 to 300 μl.

In some embodiments, the different doses comprise at least two doses. In some embodiments, the different doses comprise at least three doses. In some embodiments, the different doses comprise at least four doses. In some embodiments, the different doses comprise at least five doses.

In some embodiments, the different doses of the RNA are to be administered to different patient populations in need thereof, e.g., depending on their physical condition, medical condition, etc. In some embodiments, the different doses of the RNA are to be administered to different age groups. In some embodiments, the different doses of the RNA are to be administered to different patient groups having a different health status. In some embodiments, the different doses of the RNA are to be administered to different patient groups having a different immune status, e.g., immunocompetent and immunocompromised due to, e.g., cancer or infections. In some embodiments, higher doses are administered to adults, e.g., 12 years or older, compared to children, e.g. 5 to 11 years old, 2 to less than 5 years old, or 6 months to less than 2 years old. In some embodiments, higher doses are administered to adults, e.g., 12 years or older, compared to younger children or infants, e.g. 2 to less than 5 years old, 6 months to less than 2 years old, or less than 6 months old. In some embodiments, higher doses are administered to older children, e.g. 5 to 11 years old, compared to younger children, e.g., 2 to less than 5 years old or 6 months to less than 5 years old. In some embodiments, higher doses are administered to children who are 2 to less than 5 years old, as compared to toddlers and/or infants, e.g., who are 6 months to less than 2 years old, or less than 6 months old. In some embodiments, higher doses are administered to immunocompromised patients compared to immunocompetent patients.

In some embodiments, low doses of the RNA are to be administered to patient populations that may benefit

from or desire such low doses. For example, in some embodiments, low doses of RNA are to be administered to underweight people. In some embodiments, low doses of RNA are to be administered to poor people or poorly nourished people. In some embodiments, low doses of RNA are to be administered to people who may not tolerate well higher doses of the RNA (e.g., people who have previously had adverse reactions after administration of the RNA at higher or full doses). In some embodiments, low doses of RNA are to be administered to immunocompromised patients. In some embodiments, low doses of RNA are to be administered to populations where expense and/or distribution may be challenge for higher or full doses to all.

In some embodiments, a dilution of the pharmaceutical RNA preparation takes place for at least one dose. In some embodiments, said at least one dose comprises the lowest dose of the RNA. In some embodiments, a dilution of the pharmaceutical RNA preparation by a factor of 1:10 or less takes place for at least one dose. In some embodiments, a dilution of the pharmaceutical RNA preparation by a factor of 1:9 or less takes place for at least one dose. In some embodiments, a dilution of the pharmaceutical RNA preparation by a factor of 1:8 or less takes place for at least one dose. In some embodiments, a dilution of the pharmaceutical RNA preparation by a factor of 1:7 or less takes place for at least one dose. In some embodiments, a dilution of the pharmaceutical RNA preparation by a factor of 1:6 or less takes place for at least one dose. In some embodiments, a dilution of the pharmaceutical RNA preparation by a factor of 1:5 or less takes place for at least one dose. In some embodiments, a dilution of the pharmaceutical RNA preparation by a factor of 1:4 or less takes place for at least one dose. In some embodiments, a dilution of the pharmaceutical RNA preparation by a factor of 1:3 or less takes place for at least one dose. In some embodiments, a dilution of the pharmaceutical RNA preparation by a factor of 1:2 or less takes place for at least one dose. In some embodiments, at least one dose of the RNA is to be administered without diluting the RNA. In some embodiments, said at least one dose comprises the largest dose of the RNA.

In some embodiments, the pharmaceutical RNA preparation is provided as multi-dose preparations, wherein each multi-dose preparation allows multiple administrations of a dose of the RNA. In some embodiments, the multi-dose preparations comprise at least two different preparations for administering at least two different doses. In some embodiments, the multi-dose preparations comprise at least three different preparations for administering at least three different doses. In some embodiments, the multi-dose preparations comprise at least four different preparations for administering at least four different doses. In some embodiments, the multi-dose preparations comprise at least five different preparations for administering at least five different doses.

In some embodiments, a multi-dose preparation comprises several units such as containers, e.g., vials, for said multi-dose preparation. In some embodiments, each of several multi-dose preparations comprises several units such as containers, e.g., vials, for said multi-dose preparation. In some embodiments, each multi-dose preparation comprises several units such as containers, e.g., vials, for said multi-dose preparation. In some embodiments, said several units such as containers, e.g., vials, comprise 2 or more, 3 or more, 4 or more, 5 or more, 6 or more, 7 or more, 8 or more, 9 or more, or 10 or more of said units. In some embodiments, said several units such as containers, e.g., vials, are provided as a kit. In some embodiments, different multi-dose preparations are provided as a kit. In some embodiments, different multi-dose preparations are provided as a kit and several units such as containers, e.g., vials, for each multi-dose preparation are provided in the kit. In some embodiments, said several units such as containers, e.g., vials, each contain the same filling volume of the pharmaceutical RNA preparation.

In some embodiments, the multi-dose preparations have filling volumes that are suitable for pharmaceutical RNA manufacture. In some embodiments, the multi-dose preparations have filling volumes between 0.2 and 2.5 ml. In some embodiments, the lower limit of the filling volumes is 0.3 ml. In some embodiments, the lower limit of the filling volumes is 0.4 ml. In some embodiments, the lower limit of the filling volumes is 0.5 ml. In some embodiments, the upper limit of the filling volumes is 2.4 ml. In some embodiments, the upper limit of the filling volumes is 2.3 ml.

In some embodiments, a multi-dose preparation allows the administration of a desired number of doses of the RNA, e.g., 5 or more, such as 5-20, 5-15, or 5-10 doses (optionally following suitable dilution of the pharmaceutical RNA preparation.

In some embodiments, a multi-dose preparation allows an optional dilution of the pharmaceutical RNA preparation., e.g., a dilution by a factor as described herein.

In some embodiments, the pharmaceutical RNA preparation, e.g., provided as multi-dose preparation, is provided in vials.

In some embodiments, vials which are used to administer different doses of the RNA are labelled differently.

In some embodiments, the different labels comprise a different color of the lid.

In some embodiments, vial stoppers do not comprise natural rubber latex.

In some embodiments, the pharmaceutical RNA preparation for administration of different doses of the RNA has a uniform RNA concentration. In some embodiments, the RNA concentration is between 0.03 mg/ml to 0.5 mg/ml. In some embodiments, the RNA concentration is between 0.05 mg/ml to 0.5 mg/ml. In some embodiments, the RNA concentration is between 0.06 mg/ml to 0.4 mg/ml. In some embodiments, the RNA concentration is between 0.07 mg/ml to 0.3 mg/ml. In some embodiments, the RNA concentration is between 0.08 mg/ml to 0.2 mg/ml. In some embodiments,

the RNA concentration is between 0.09 mg/ml to 0.15 mg/ml. In some embodiments, the RNA concentration is about 0.03 mg/ml to about 0.1 mg/ml. In some embodiments, the RNA concentration is about 0.05 mg/ml to about 0.1 mg/ml. In some embodiments, the RNA concentration is about 0.1 mg/ml.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA encodes an amino acid sequence comprising a SARS-CoV-2 S protein, an immunogenic variant thereof (e.g., a SARS-CoV-2 S protein comprising one or more mutations of a variant of concern disclosed herein), or an immunogenic fragment of the SARS-CoV-2 S protein or the immunogenic variant thereof.

In some embodiments, the pharmaceutical RNA preparation comprises two or more (e.g., three or more, four or more, or five or more) RNA molecules, each comprising a nucleotide sequence encoding a SARS-CoV-2 S protein of a different variant. In some such embodiments, the pharmaceutical RNA preparation comprises a first RNA and a second RNA, wherein the first RNA comprises a nucleotide sequence that encodes a SARS-CoV-2 S protein of a Wuhan strain and the second RNA comprises a nucleotide sequence that encodes a SARS-CoV-2 S protein of a SARS-CoV-2 variant of concern (e.g., a variant of concern in a relevant jurisdiction, at the time of administration). In some embodiments, the pharmaceutical RNA preparation comprises a first RNA and a second RNA, wherein the first RNA comprises a nucleotide sequence that encodes a SARS-CoV-2 S protein of a Wuhan strain and the second RNA comprises a nucleotide sequence that encodes a SARS-CoV-2 S protein of an Omicron variant (e.g., an Omicron variant disclosed herein, e.g., a BA.1 and/or BA.4/5 variant). In some embodiments, two or more RNAs are formulated in a particle (e.g., an LNP). In some embodiments, two or more RNAs are co-formulated in the same particle (e.g., the same LNP, e.g., by mixing the two or more RNAs prior to formulation). In some embodiments, the two or more RNAs are formulated in separate particles (e.g., separate LNPs, e.g., by formulating each RNA separately and then mixing). In some embodiments, the pharmaceutical RNA preparation is for inducing an immune response against SARS-CoV-2.

In some embodiments, the pharmaceutical RNA preparation is for vaccination against SARS-CoV-2.

**Methods of using a pharmaceutical RNA preparation**

In some embodiments, the invention provides a method for administering different doses of RNA from a pharmaceutical RNA preparation, comprising the steps:

(i) providing the pharmaceutical RNA preparation,
(ii) administering different doses of the RNA, wherein said different doses of the RNA are administered by administering the same and/or different volumes of the optionally diluted pharmaceutical RNA preparation, wherein

the concentration of the RNA in the pharmaceutical RNA preparation is selected such that it allows the administration of the different doses of the RNA with suitable administration volumes and, optionally, suitable dilution of the pharmaceutical RNA preparation, and
the pharmaceutical RNA preparation is formulated to ensure a desired stability of the RNA in the pharmaceutical RNA preparation.

The method of the invention aims at administering different doses of RNA from a single pharmaceutical preparation comprising RNA that is optionally formulated, e.g., in particles such as LNPs.

In some embodiments, at least some of the different doses in which the RNA is administered should differ significantly, e.g., by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more. In some embodiments, at least some of the different doses in which the RNA is administered should differ by a factor of about 2 to about 50, about 2 to about 40, about 2 to about 35, about 2 to about 30, about 2 to about 25, about 2 to about 20, about 2 to about 19, about 2 to about 18, about 2 to about 17, about 2 to about 16, about 2 to about 15, about 2 to about 14, about 2 to about 13, about 2 to about 12, about 2 to about 11, about 2 to about 10, about 2 to about 9, about 2 to about 8, about 2 to about 7, about 2 to about 6, about 2 to about 5, about 2 to about 4, about 2 to about 3. In some embodiments, at least some of the different doses in which the RNA is administered should differ by a factor of about 2. In some embodiments, at least some of the different doses in which the RNA is administered should differ by a factor of about 6.75.

In some embodiments, the lowest dose of the RNA and the largest dose of the RNA differ by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the administration volumes for the different doses in which the RNA is administered

should not differ significantly, e.g., by a factor of 2 or less, 1.9 or less, 1.8 or less, 1.7 or less, 1.6 or less, 1.5 or less, 1.4 or less, 1.3 or less, 1.2 or less, or 1.1 or less. In some embodiments, the administration volumes for the different doses in which the RNA is administered do not differ, i.e., the same administration volumes are to be used for the different doses.

In some embodiments, the smallest administration volume for one dose and the largest administration volume for another dose differ by a factor of 2 or less, 1.9 or less, 1.8 or less, 1.7 or less, 1.6 or less, 1.5 or less, 1.4 or less, 1.3 or less, 1.2 or less, or 1.1 or less. In some embodiments, the smallest administration volume for one dose and the largest administration volume for another dose differ by a factor of 1 to 2, 1 to 1.9 or less, 1 to 1.8, 1 to 1.7, 1 to 1.6, 1 to 1.5, 1 to 1.4, 1 to 1.3, 1 to 1.2, or 1 to 1.1.

In some embodiments, suitable administration volumes for each dose are 100 to 400 µl. In some embodiments, suitable administration volumes for each dose are 150 to 350 µl. In some embodiments, suitable administration volumes for each dose are 200 to 300 µl. In some embodiments, suitable administration volumes for each dose are about 200 µl, about 250 µl or about 300 µl.

In some embodiments, the lowest dose of the RNA and the largest dose of the RNA differ by a factor of 2 or more and the smallest administration volume for one dose and the largest administration volume for another dose differ by a factor of 2 or less. In some embodiments, the lowest dose of the RNA and the largest dose of the RNA differ by a factor of 5 or more and the smallest administration volume for one dose and the largest administration volume for another dose differ by a factor of 2 or less. In some embodiments, the lowest dose of the RNA and the largest dose of the RNA differ by a factor of 10 or more and the smallest administration volume for one dose and the largest administration volume for another dose differ by a factor of 2 or less.

In some embodiments, the lowest dose of the RNA and the largest dose of the RNA differ by a factor of 2 or more and the smallest administration volume for one dose and the largest administration volume for another dose differ by a factor of 1.5 or less. In some embodiments, the lowest dose of the RNA and the largest dose of the RNA differ by a factor of 5 or more and the smallest administration volume for one dose and the largest administration volume for another dose differ by a factor of 1.5 or less. In some embodiments, the lowest dose of the RNA and the largest dose of the RNA differ by a factor of 10 or more and the smallest administration volume for one dose and the largest administration volume for another dose differ by a factor of 1.5 or less.

In some embodiments, the lowest dose of the RNA and the largest dose of the RNA differ by a factor of 2 or more and suitable administration volumes are 100 to 400 µl. In some embodiments, the lowest dose of the RNA and the largest dose of the RNA differ by a factor of 2 or more and suitable administration volumes are 200 to 300 µl. In some embodiments, the lowest dose of the RNA and the largest dose of the RNA differ by a factor of 5 or more and suitable administration volumes are 100 to 400 µ!. In some embodiments, the lowest dose of the RNA and the largest dose of the RNA differ by a factor of 5 or more and suitable administration volumes are 200 to 300 µ!. In some embodiments, the lowest dose of the RNA and the largest dose of the RNA differ by a factor of 10 or more and suitable administration volumes are 100 to 400 µ!. In some embodiments, the lowest dose of the RNA and the largest dose of the RNA differ by a factor of 10 or more and suitable administration volumes are 200 to 300 µ!.

In some embodiments, the different doses comprise at least two doses. In some embodiments, the different doses comprise at least three doses. In some embodiments, the different doses comprise at least four doses. In some embodiments, the different doses comprise at least five doses.

In some embodiments, the different doses of the RNA are to be administered to different age groups. In some embodiments, the different doses of the RNA are to be administered to different patient groups having a different health status. In some embodiments, the different doses of the RNA are to be administered to different patient groups having a different immune status, e.g., immunocompetent and immunocompromised due to, e.g., cancer or infections. In some embodiments, higher doses are administered to adults, e.g., 12 years or older, compared to children, e.g. 5 to 11 years old, 6 months to less than 2 years old, or 2 to less than 5 years old. In some embodiments, higher doses are administered to adults, e.g., 12 years or older, compared to younger children or infants, e.g. 2 to less than 5 years old, 6 months to less than 2 years old, or less than 6 months old. In some embodiments, higher doses are administered to older children, e.g. 5 to 11 years old, compared to younger children, e.g., 2 to less than 5 years old. In some embodiments, higher doses are administered to children who are 2 to less than 5 years old, as compared to toddlers and/or infants, e.g., who are 6 months to less than 2 years old, or less than 6 months old. In some embodiments, higher doses are administered to immunocompromised patients compared to immunocompetent patients.

In some embodiments, a dilution of the pharmaceutical RNA preparation takes place for at least one dose. In some embodiments, said at least one dose comprises the lowest dose of the RNA. In some embodiments, a dilution of the pharmaceutical RNA preparation by a factor of 1:10 or less takes place for at least one dose. In some embodiments, a dilution of the pharmaceutical RNA preparation by a factor of 1:9 or less takes place for at least one dose. In some embodiments, a dilution of the pharmaceutical RNA preparation by a factor of 1:8 or less takes place for at least one dose. In some embodiments, a dilution of the pharmaceutical RNA preparation by a factor of 1:7 or less takes place for at least one dose. In some embodiments, a dilution of the pharmaceutical RNA preparation by a factor of 1:6 or less

takes place for at least one dose. In some embodiments, a dilution of the pharmaceutical RNA preparation by a factor of 1:5 or less takes place for at least one dose. In some embodiments, a dilution of the pharmaceutical RNA preparation by a factor within a range of 1:2 to 1:20 takes place for at least one dose. In some embodiments, a dilution of the pharmaceutical RNA preparation by a factor within a range of 1:2 to 1:10 takes place for at least one dose. In some embodiments, a dilution of the pharmaceutical RNA preparation by a factor within a range of 1:2 to 1:5 takes place for at least one dose. In some embodiments, a dilution of the pharmaceutical RNA preparation by a factor within a range of 1:5 to 1:7 takes place for at least one dose.

In some embodiments, at least one dose of the RNA is administered without diluting the RNA. In some embodiments, said at least one dose comprises the largest dose of the RNA.

In some embodiments, the pharmaceutical RNA preparation is provided as multi-dose preparations, wherein each multi-dose preparation allows multiple administrations of a dose of the RNA. In some embodiments, the multi-dose preparations comprise at least two different preparations for administering at least two different doses. In some embodiments, the multi-dose preparations comprise at least three different preparations for administering at least three different doses. In some embodiments, the multi-dose preparations comprise at least four different preparations for administering at least four different doses. In some embodiments, the multi-dose preparations comprise at least five different preparations for administering at least five different doses.

In some embodiments, a multi-dose preparation comprises several units such as containers, e.g., vials, for said multi-dose preparation. In some embodiments, each of several multi-dose preparations comprises several units such as containers, e.g., vials, for said multi-dose preparation. In some embodiments, each multi-dose preparation comprises several units such as containers, e.g., vials, for said multi-dose preparation. In some embodiments, said several units such as containers, e.g., vials, comprise 2 or more, 3 or more, 4 or more, 5 or more, 6 or more, 7 or more, 8 or more, 9 or more, or 10 or more of said units. In some embodiments, said several units such as containers, e.g., vials, are provided as a kit. In some embodiments, different multi-dose preparations are provided as a kit. In some embodiments, different multi-dose preparations are provided as a kit and several units such as containers, e.g., vials, for each multi-dose preparation are provided in the kit. In some embodiments, said several units such as containers, e.g., vials, each contain the same filling volume of the pharmaceutical RNA preparation.

In some embodiments, the multi-dose preparations have filling volumes that are suitable for pharmaceutical RNA manufacture. In some embodiments, the multi-dose preparations have filling volumes between 0.2 and 2.5 ml. In some embodiments, the lower limit of the filling volumes is 0.3 ml. In some embodiments, the lower limit of the filling volumes is 0.4 ml. In some embodiments, the lower limit of the filling volumes is 0.5 ml. In some embodiments, the upper limit of the filling volumes is 2.4 ml. In some embodiments, the upper limit of the filling volumes is 2.3 ml.

In some embodiments, a multi-dose preparation allows the administration of a desired number of doses of the RNA, e.g., 5 or more, such as 5-20, 5-15, or 5-10 doses (optionally following suitable dilution of the pharmaceutical RNA preparation.

In some embodiments, a multi-dose preparation allows an optional dilution of the pharmaceutical RNA preparation, e.g., a dilution by a factor as described herein.

In some embodiments, the pharmaceutical RNA preparation, e.g., provided as multi-dose preparation, is provided in vials.

In some embodiments, vials which are used to administer different doses of the RNA are labelled differently.

In some embodiments, the different labels comprise a different color of the lid.

In some embodiments, the pharmaceutical RNA preparation for administration of different doses of the RNA has a uniform RNA concentration. In some embodiments, the RNA concentration is between 0.03 mg/ml to 0.5 mg/ml. In some embodiments, the RNA concentration is between 0.05 mg/ml to 0.5 mg/ml. In some embodiments, the RNA concentration is between 0.06 mg/ml to 0.4 mg/ml. In some embodiments, the RNA concentration is between 0.07 mg/ml to 0.3 mg/ml. In some embodiments, the RNA concentration is between 0.08 mg/ml to 0.2 mg/ml. In some embodiments, the RNA concentration is between 0.09 mg/ml to 0.15 mg/ml. In some embodiments, the RNA concentration is about 0.03 mg/ml to about 0.1 mg/ml. In some embodiments, the RNA concentration is about 0.05 mg/ml to about 0.1 mg/ml. In some embodiments, the RNA concentration is about 0.1 mg/ml.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof.

In some embodiments, the pharmaceutical RNA preparation is for inducing an immune response against SARS-CoV-2.

In some embodiments, the pharmaceutical RNA preparation is for vaccination against SARS-CoV-2.

In some embodiments, the different doses comprise doses of about 10 μg and about 30 μg. In some embod-

iments, the different doses comprise doses of about 3 μg and about 10 μg. In some embodiments, the different doses comprise doses of about 3 μg and about 30 μg. In some embodiments, the different doses comprise doses of about 3 μg, about 10 μg and about 30 μg.

In some embodiments, the different doses comprise doses of about 10 μg and about 30 μg and the pharmaceutical RNA preparation comprises RNA encoding an amino acid sequence comprising a SARS-CoV-2 S protein, an immunogenic variant thereof (e.g., a SARS-CoV-2 S protein comprising one or more mutations characteristic of a variant of concern described herein), or an immunogenic fragment of the SARS-CoV-2 S protein or the immunogenic variant thereof. In some embodiments, the different doses comprise doses of about 3 μg and about 10 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3μg, about 10 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is an RNA as described herein.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments,
In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the different doses comprise doses of about 10 μg and about 30 μg and the pharmaceutical RNA preparation comprises BNT162b2. In some embodiments, the different doses comprise doses of about 3 μg and about 10 μg and the pharmaceutical RNA preparation comprises BNT162b2. In some embodiments, the different doses comprise doses of about 3 μg and about 30 μg and the pharmaceutical RNA preparation comprises BNT162b2. In some embodiments, the different doses comprise doses of about 3μg, about 10 μg and about 30 μg and the pharmaceutical RNA preparation comprises BNT162b2.

In some embodiments, BNT162b2 comprises RNA comprising the sequence of SEQ ID NO: 17. In some embodiments, BNT162b2 is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, BNT162b2 is formulated as LNPs. In some embodiments, lipid nanoparticles comprise one or more cationically ionizable lipids; one or more neutral lipids (e.g., in some embodiments sterol such as, e.g., cholesterol; and/or phospholipids) and, and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the concentration of the RNA in the pharmaceutical RNA preparation is about 0.1 mg/ml. In some embodiments, the concentration of the RNA in the pharmaceutical RNA preparation is about 30 μg/ml to about 100 μg/ml. In some embodiments, the concentration of the RNA in the pharmaceutical RNA preparation is about 50 μg/ml to about 100 μg/ml.

In some embodiments, the administration volumes are between about 200 μl and about 300 μl.

In some embodiments, the RNA in pharmaceutical RNA preparation is formulated in about 10 mM Tris buffer, and about 10% sucrose.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 µg is administered by administering about 300 µl of undiluted pharmaceutical RNA preparation, and

(ii) a second dose of the RNA of about 10 µg is administered by diluting the pharmaceutical RNA preparation about 1:1 and administering about 200 µl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 10 µg is administered by diluting the pharmaceutical RNA preparation about 1:1 and administering about 200 µl of diluted pharmaceutical RNA preparation, and

(ii) a second dose of the RNA of about 3 µg is administered by diluting the pharmaceutical RNA preparation about 1:5.75 and administering about 200 µl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 µg is administered by administering about 300 µl of undiluted pharmaceutical RNA preparation, and

(ii) a second dose of the RNA of about 3 µg is administered by diluting the pharmaceutical RNA preparation about 1:5.75 and administering about 200 µl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 µg is administered by administering about 300 µl of undiluted pharmaceutical RNA preparation,

(ii) a second dose of the RNA of about 10 µg is administered by diluting the pharmaceutical RNA preparation about 1:1 and administering about 200 µl of diluted pharmaceutical RNA preparation, and

(iii) a third dose of the RNA of about 3 µg is administered by diluting the pharmaceutical RNA preparation about 1:5.75 and administering about 200 µl of diluted pharmaceutical RNA preparation.

**Pharmaceutical RNA preparation**

In some embodiments, the invention provides a pharmaceutical RNA preparation for the administration of different doses of the RNA, wherein the concentration of the RNA in the pharmaceutical RNA preparation is selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation. The pharmaceutical RNA preparation described herein is suitable for administering different doses of RNA from a single pharmaceutical preparation comprising RNA that is optionally formulated, e.g., in particles such as LNPs.

In some embodiments, at least some of the different doses in which the RNA is administered should differ significantly, e.g., by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the lowest dose of the RNA and the largest dose of the RNA differ by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the administration volumes for the different doses in which the RNA is administered should not differ significantly, e.g., by a factor of 2 or less, 1.9 or less, 1.8 or less, 1.7 or less, 1.6 or less, 1.5 or less, 1.4 or less, 1.3 or less, 1.2 or less, or 1.1 or less. In some embodiments, the administration volumes for the different doses in which the RNA is administered do not differ, i.e., the same administration volumes are to be used for the different

doses.

In some embodiments, the smallest administration volume for one dose and the largest administration volume for another dose differ by a factor of 2 or less, 1.9 or less, 1.8 or less, 1.7 or less, 1.6 or less, 1.5 or less, 1.4 or less, 1.3 or less, 1.2 or less, or 1.1 or less.

In some embodiments, suitable administration volumes for each dose are 100 to 400 μl. In some embodiments, suitable administration volumes for each dose are 150 to 350 μl. In some embodiments, suitable administration volumes for each dose are 200 to 300 μl. In some embodiments, suitable administration volumes for each dose are about 200 μl, about 250 μl or about 300 μl.

In some embodiments, the lowest dose of the RNA and the largest dose of the RNA differ by a factor of 2 or more and the smallest administration volume for one dose and the largest administration volume for another dose differ by a factor of 2 or less. In some embodiments, the lowest dose of the RNA and the largest dose of the RNA differ by a factor of 5 or more and the smallest administration volume for one dose and the largest administration volume for another dose differ by a factor of 2 or less. In some embodiments, the lowest dose of the RNA and the largest dose of the RNA differ by a factor of 10 or more and the smallest administration volume for one dose and the largest administration volume for another dose differ by a factor of 2 or less.

In some embodiments, the lowest dose of the RNA and the largest dose of the RNA differ by a factor of 2 or more and the smallest administration volume for one dose and the largest administration volume for another dose differ by a factor of 1.5 or less. In some embodiments, the lowest dose of the RNA and the largest dose of the RNA differ by a factor of 5 or more and the smallest administration volume for one dose and the largest administration volume for another dose differ by a factor of 1.5 or less. In some embodiments, the lowest dose of the RNA and the largest dose of the RNA differ by a factor of 10 or more and the smallest administration volume for one dose and the largest administration volume for another dose differ by a factor of 1.5 or less.

In some embodiments, the lowest dose of the RNA and the largest dose of the RNA differ by a factor of 2 or more and suitable administration volumes are 100 to 400 μl. In some embodiments, the lowest dose of the RNA and the largest dose of the RNA differ by a factor of 2 or more and suitable administration volumes are 200 to 300 μl. In some embodiments, the lowest dose of the RNA and the largest dose of the RNA differ by a factor of 5 or more and suitable administration volumes are 100 to 400 μl. In some embodiments, the lowest dose of the RNA and the largest dose of the RNA differ by a factor of 5 or more and suitable administration volumes are 200 to 300 μl. In some embodiments, the lowest dose of the RNA and the largest dose of the RNA differ by a factor of 10 or more and suitable administration volumes are 100 to 400 μl. In some embodiments, the lowest dose of the RNA and the largest dose of the RNA differ by a factor of 10 or more and suitable administration volumes are 200 to 300 μl.

In some embodiments, the different doses comprise at least two doses. In some embodiments, the different doses comprise at least three doses. In some embodiments, the different doses comprise at least four doses. In some embodiments, the different doses comprise at least five doses.

In some embodiments, the different doses of the RNA are to be administered to different age groups. In some embodiments, the different doses of the RNA are to be administered to different patient groups having a different health status. In some embodiments, the different doses of the RNA are to be administered to different patient groups having a different immune status, e.g., immunocompetent and immunocompromised due to, e.g., cancer or infections. In some embodiments, higher doses are administered to adults, e.g., 12 years or older, compared to children, e.g. 5 to 11 years old or 2 to less than 5 years old. In some embodiments, higher doses are administered to adults, e.g., 12 years or older, compared to younger children or infants, e.g. 2 to less than 5 years old, 6 months to less than 2 years old, or less than 6 months old. In some embodiments, higher doses are administered to older children, e.g. 5 to 11 years old, compared to younger children, e.g., 2 to less than 5 years old. In some embodiments, higher doses are administered to children who are 2 to less than 5 years old, as compared to toddlers and/or infants, e.g., who are 6 months to less than 2 years old, or less than 6 months old. In some embodiments, higher doses are administered to immunocompromised patients compared to immunocompetent patients.

In some embodiments, a dilution of the pharmaceutical RNA preparation takes place for at least one dose. In some embodiments, said at least one dose comprises the lowest dose of the RNA. In some embodiments, a dilution of the pharmaceutical RNA preparation by a factor of 1:10 or less takes place for at least one dose. In some embodiments, a dilution of the pharmaceutical RNA preparation by a factor of 1:9 or less takes place for at least one dose. In some embodiments, a dilution of the pharmaceutical RNA preparation by a factor of 1:8 or less takes place for at least one dose. In some embodiments, a dilution of the pharmaceutical RNA preparation by a factor of 1:7 or less takes place for at least one dose. In some embodiments, a dilution of the pharmaceutical RNA preparation by a factor of 1:6 or less takes place for at least one dose. In some embodiments, a dilution of the pharmaceutical RNA preparation by a factor of 1:5 or less takes place for at least one dose.

In some embodiments, at least one dose of the RNA is administered without diluting the RNA. In some embodiments, said at least one dose comprises the largest dose of the RNA.

In some embodiments, the pharmaceutical RNA preparation is provided as multi-dose preparations, wherein

each multi-dose preparation allows multiple administrations of a dose of the RNA. In some embodiments, the multi-dose preparations comprise at least two different preparations for administering at least two different doses. In some embodiments, the multi-dose preparations comprise at least three different preparations for administering at least three different doses. In some embodiments, the multi-dose preparations comprise at least four different preparations for administering at least four different doses. In some embodiments, the multi-dose preparations comprise at least five different preparations for administering at least five different doses.

In some embodiments, a multi-dose preparation comprises several units such as containers, e.g., vials, for said multi-dose preparation. In some embodiments, each of several multi-dose preparations comprises several units such as containers, e.g., vials, for said multi-dose preparation. In some embodiments, each multi-dose preparation comprises several units such as containers, e.g., vials, for said multi-dose preparation. In some embodiments, said several units such as containers, e.g., vials, comprise 2 or more, 3 or more, 4 or more, 5 or more, 6 or more, 7 or more, 8 or more, 9 or more, or 10 or more of said units. In some embodiments, said several units such as containers, e.g., vials, are provided as a kit. In some embodiments, different multi-dose preparations are provided as a kit. In some embodiments, different multi-dose preparations are provided as a kit and several units such as containers, e.g., vials, for each multi-dose preparation are provided in the kit. In some embodiments, said several units such as containers, e.g., vials, each contain the same filling volume of the pharmaceutical RNA preparation.

In some embodiments, the multi-dose preparations have filling volumes that are suitable for pharmaceutical RNA manufacture. In some embodiments, the multi-dose preparations have filling volumes between 0.2 and 2.5 ml. In some embodiments, the lower limit of the filling volumes is 0.3 ml. In some embodiments, the lower limit of the filling volumes is 0.4 ml. In some embodiments, the lower limit of the filling volumes is 0.5 ml. In some embodiments, the upper limit of the filling volumes is 2.4 ml. In some embodiments, the upper limit of the filling volumes is 2.3 ml.

In some embodiments, a multi-dose preparation allows the administration of a desired number of doses of the RNA, e.g., 5 or more, such as 5-20, 5-15, or 5-10 doses (optionally following suitable dilution of the pharmaceutical RNA preparation.

In some embodiments, a multi-dose preparation allows an optional dilution of the pharmaceutical RNA preparation, e.g., a dilution by a factor as described herein.

In some embodiments, the pharmaceutical RNA preparation, e.g., provided as multi-dose preparation, is provided in vials.

In some embodiments, vials which are used to administer different doses of the RNA are labelled differently.

In some embodiments, the different labels comprise a different color of the lid.

In some embodiments, the pharmaceutical RNA preparation for administration of different doses of the RNA has a uniform RNA concentration. In some embodiments, the RNA concentration is between 0.03 mg/ml to 0.5 mg/ml. In some embodiments, the RNA concentration is between 0.05 mg/ml to 0.5 mg/ml. In some embodiments, the RNA concentration is between 0.06 mg/ml to 0.4 mg/ml. In some embodiments, the RNA concentration is between 0.07 mg/ml to 0.3 mg/ml. In some embodiments, the RNA concentration is between 0.08 mg/ml to 0.2 mg/ml. In some embodiments, the RNA concentration is between 0.09 mg/ml to 0.15 mg/ml. In some embodiments, the RNA concentration is about 0.03 mg/ml to about 0.1 mg/ml. In some embodiments, the RNA concentration is about 0.05 mg/ml to about 0.1 mg/ml. In some embodiments, the RNA concentration is about 0.1 mg/ml.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA present in the pharmaceutical RNA preparation is formulated in nanoparticles. In some embodiments, exemplary nanoparticles include lipid nanoparticles, polyplexes (PLX), lipidated polyplexes (LPLX), liposomes, or polysaccharide nanoparticles. In some embodiments, the RNA present in the pharmaceutical RNA preparation is formulated in lipid nanoparticles. In some embodiments, lipid nanoparticles comprise one or more cationically ionizable lipids; one or more neutral lipids (e.g., in some embodiments sterol such as, e.g., cholesterol; and/or phospholipids) and, and one or more polymer-conjugated lipids. In some embodiments, the RNA encodes an amino acid sequence comprising a SARS-CoV-2 S protein, an immunogenic variant thereof, or an immunogenic fragment of the SARS-CoV-2 S protein or the immunogenic variant thereof.

In some embodiments, the pharmaceutical RNA preparation is for inducing an immune response against SARS-CoV-2.

In some embodiments, the pharmaceutical RNA preparation is for vaccination against SARS-CoV-2.

In some embodiments, the different doses comprise doses of about 10 μg and about 30 μg. In some embodiments, the different doses comprise doses of about 3 μg and about 10 μg. In some embodiments, the different doses comprise doses of about 3 μg and about 30 μg. In some embodiments, the different doses comprise doses of about 3 μg, about 10 μg and about 30 μg.

In some embodiments, the different doses comprise doses of about 10 μg and about 30 μg and the pharmaceutical RNA preparation comprises RNA encoding an amino acid sequence comprising a SARS-CoV-2 S protein, an

immunogenic variant thereof, or an immunogenic fragment of the SARS-CoV-2 S protein or the immunogenic variant thereof. In some embodiments, the different doses comprise doses of about 3 μg and about 10 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3μg, about 10 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is an RNA as described herein. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the different doses comprise doses of about 10 μg and about 30 μg and the pharmaceutical RNA preparation comprises BNT162b2 or a variant thereof, for example, in some embodiments a variant that encodes a SARS-CoV-2 S protein from a variant of an ancestral strain. In some embodiments, the different doses comprise doses of about 3 μg and about 10 μg and the pharmaceutical RNA preparation comprises BNT162b2 or a variant thereof, for example, in some embodiments a variant that encodes a SARS-CoV-2 S protein from a variant of an ancestral strain. In some embodiments, the different doses comprise doses of about 3 μg and about 30 μg and the pharmaceutical RNA preparation comprises BNT162b2 or a variant thereof, for example, in some embodiments a variant that encodes a SARS-CoV-2 S protein from a variant of an ancestral strain. In some embodiments, the different doses comprise doses of about 3μg, about 10 μg and about 30 μg and the pharmaceutical RNA preparation comprises BNT162b2 or a variant thereof, for example, in some embodiments a variant that encodes a SARS-CoV-2 S protein from a variant of an ancestral strain. In some embodiments, BNT162b2 comprises RNA comprising the sequence of SEQ ID NO: 17. In some embodiments, a variant of BNT162b2 that encodes a SARS-CoV-2 S protein from a variant of an ancestral strain comprises RNA comprising a sequence that is at least 85% (including, e.g., at least 86%, at least 87%, at least 88%, at least 89%, at least 90%, at least 91%, at least 92%, at least 93%, at least 94%, at least 95%, at least 96%, at least 97%, at least 98%, or higher) identical to the sequence of SEQ ID NO: 17. In some embodiments, BNT162b2 is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, BNT162b2 is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the concentration of the RNA in the pharmaceutical RNA preparation is about 0.1 mg/ml.

In some embodiments, the concentration of the RNA in the pharmaceutical RNA preparation is about 30 μg/ml to about 100 μg/ml. In some embodiments, the concentration of the RNA in the pharmaceutical RNA preparation is about 50 μg/ml to about 100 μg/ml. In some embodiments, the administration volumes are between about 200 μl and about 300 μl.

In some embodiments, the RNA in pharmaceutical RNA preparation is formulated in about 10 mM Tris buffer, about 10% sucrose.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 10 μg is administered by diluting the pharmaceutical RNA preparation about 1:1 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 10 μg is administered by diluting the pharmaceutical RNA preparation about 1:1 and administering about 200 μl of diluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation,
(ii) a second dose of the RNA of about 10 μg is administered by diluting the pharmaceutical RNA preparation about 1:1 and administering about 200 μl of diluted pharmaceutical RNA preparation, and
(iii) a third dose of the RNA of about 3 μg is administered by diluting the pharmaceutical RNA preparation about 1:5.75 and administering about 200 μl of diluted pharmaceutical RNA preparation.

In some embodiments, the invention provides a pharmaceutical RNA preparation for the administration of different doses of the RNA, wherein the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof comprises BNT162b2. In some embodiments, BNT162b2 comprises RNA comprising the sequence of SEQ ID NO: 17.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the different doses comprise doses of about 10 μg and about 30 μg. In some embod-

iments, the different doses comprise doses of about 3 μg and about 10 μg. In some embodiments, the different doses comprise doses of about 3 μg and about 30 μg. In some embodiments, the different doses comprise doses of about 3μg, about 10 μg and about 30 μg.

In some embodiments, the administration volumes are between about 200 μl and about 300 μl.

In some embodiments,

(i) a first dose of the RNA of about 30 μg is administered by administering about 300 μl of undiluted pharmaceutical RNA preparation, and
(ii) a second dose of the RNA of about 10 μg is administered by diluting the pharmaceutical RNA preparation about 1:1 and administering about 200 μl of diluted pharmaceutical RNA preparation.

In some embodiments,

(i) a first dose of the RNA of about 10 μg is administered by diluting the pharmaceutical RNA preparation about 1:1 and administering about 200 μl of diluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments,

(i) a first dose of the RNA of about 30 μg is administered by administering about 300 μl of undiluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments,

(i) a first dose of the RNA of about 30 μg is administered by administering about 300 μl of undiluted pharmaceutical RNA preparation,
(ii) a second dose of the RNA of about 10 μg is administered by diluting the pharmaceutical RNA preparation about 1:1 and administering about 200 μl of diluted pharmaceutical RNA preparation, and
(iii) a third dose of the RNA of about 3 μg is administered by diluting the pharmaceutical RNA preparation about 1:5.75 and administering about 200 μl of diluted pharmaceutical RNA preparation.

In some embodiments, the invention provides a composition comprising:

(a) about 0.1 mg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 10 mM Tris buffer; and
(c) about 300 mM sucrose.

In some embodiments, the invention provides a composition comprising:

(a) about 0.1 mg/mL RNA comprising an open reading frame encoding a polypeptide that comprises a SARS-CoV-2 protein or an immunogenic fragment or variant thereof,
(b) about 1.43 mg/ml ALC-0315,
(c) about 0.18 mg/ml ALC-0159,
(d) about 0.31 mg/ml DSPC,
(e) about 0.62 mg/ml cholesterol,
(f) about 103 mg/ml sucrose,
(g) about 0.20 mg/ml tromethamine (Tris base),
(h) about 1.32 mg/ml Tris (hydroxymethyl) aminomethane hydrochloride (Tris HCl), and
(i) q.s. water.

In some embodiments, the pH of the composition is pH 7.4.

In some embodiments, the invention provides a sealed multi-dose vial comprising a stock composition comprising:

(a) about 0.1 mg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 10 mM Tris buffer; and
(c) about 300 mM sucrose.

In some embodiments, the pH of the stock composition is pH 7.4.
In some embodiments, the RNA and the lipid nanoparticles remain stable at a refrigerated temperature for at least 10 weeks.
In some embodiments, the vial comprises about 2.25 mL of the stock composition.
In some embodiments, the vial comprising about 2.25 mL of the stock composition comprises 6 doses.
In some embodiments, the invention provides a tray comprising 10 sealed multi-dose vials of the vials comprising about 2.25 mL of the stock composition.
In some embodiments, the invention provides a package comprising 20 of said trays.
In some embodiments, the vial comprises about 1.3 mL of the stock composition.
In some embodiments, the vial comprising about 1.3 mL of the stock composition comprises 10 doses.
In some embodiments, the invention provides a tray comprising 10 sealed multi-dose vials of the vials comprising about 1.3 mL of the stock composition.
In some embodiments, the invention provides a package comprising 20 of said trays.
In some embodiments, the vial comprises about 0.4 mL of the stock composition.
In some embodiments, the vial comprising about 0.4 mL of the stock composition comprises 10 doses.
In some embodiments, the invention provides a tray comprising 10 sealed multi-dose vials of the vials comprising about 0.4 mL of the stock composition.
In some embodiments, the invention provides a package comprising 20 of said trays.
In some embodiments, the invention provides a syringe comprising an injection volume of about 300 μL of a drug product,
wherein the drug product comprises:

(a) about 0.1 mg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 10 mM Tris buffer; and
(c) about 300 mM sucrose.

In some embodiments, the syringe is a low dead-volume syringe.
In some embodiments, the invention provides a multi-dose vial comprising about 2.6 mL of diluted drug product, wherein the diluted drug product comprises:

(a) about 50 μg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 5 mM Tris buffer;
(c) about 150 mM sucrose; and
(d) about 0.45% sodium chloride.

In some embodiments, the invention provides a syringe comprising an injection volume of about 200 μL of the diluted drug product,
wherein the diluted drug product comprises:

(a) about 50 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) about 5 mM Tris buffer;
(c) about 150 mM sucrose; and
(d) about 0.45% sodium chloride.

In some embodiments, the syringe is a low dead-volume syringe.
In some embodiments, the invention provides a multi-dose vial comprising about 2.7 mL of diluted drug product,

wherein the diluted drug product comprises:

(a) about 14.8 μg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 1.5 mM Tris buffer;
(c) about 44.4 mM sucrose; and
(d) about 0.77% sodium chloride.

In some embodiments, the invention provides a syringe comprising an injection volume of about 200 uL of the diluted drug product,
wherein the diluted drug product comprises:

(a) about 14.8 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid,
(b) about 1.5 mM Tris buffer;
(c) about 44.4 mM sucrose; and
(d) about 0.77% sodium chloride.

In some embodiments, the syringe is a low dead-volume syringe.

**System**

In some embodiments, the invention provides a system for the administration of different doses of RNA, wherein the system comprises a plurality of vials for administering a plurality of different doses of the RNA, wherein each of the plurality of vials comprises optionally different volumes of a pharmaceutical RNA preparation, wherein the concentration of the RNA in the pharmaceutical RNA preparation and the volumes of the pharmaceutical RNA preparation in the vials are selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation.

The system described herein is suitable for administering different doses of RNA from a single pharmaceutical preparation, wherein the system comprises a plurality of vials (including any kind of suitable container), wherein each of the plurality of vials comprises the pharmaceutical RNA preparation, and wherein the RNA is optionally formulated, e.g., in particles such as LNPs. In some embodiments, one or more of the plurality of vials comprises different volumes of the pharmaceutical RNA preparation, wherein the volumes of the pharmaceutical RNA preparation in the vials may be selected such that it allows for the administration of the different doses of the RNA with suitable administration volumes and, optionally, suitable dilution of the pharmaceutical RNA preparation, and the administration of a desired number of doses.

In some embodiments, at least some of the different doses in which the RNA is administered should differ significantly, e.g., by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the lowest dose of the RNA and the largest dose of the RNA differ by a factor of 2 or more, 2.25 or more, 2.5 or more, 2.75 or more, 3 or more, 3.25 or more, 3.5 or more, 3.75 or more, 4 or more, 4.5 or more, 5 or more, 5.5 or more, 6 or more, 6.5 or more, 7 or more, 7.5 or more, 8 or more, 8.5 or more, 9 or more, 9.5 or more, or 10 or more.

In some embodiments, the administration volumes for the different doses in which the RNA is administered should not differ significantly, e.g., by a factor of 2 or less, 1.9 or less, 1.8 or less, 1.7 or less, 1.6 or less, 1.5 or less, 1.4 or less, 1.3 or less, 1.2 or less, or 1.1 or less. In some embodiments, the administration volumes for the different doses in which the RNA is administered do not differ, i.e., the same administration volumes are to be used for the different doses.

In some embodiments, the smallest administration volume for one dose and the largest administration volume for another dose differ by a factor of 2 or less, 1.9 or less, 1.8 or less, 1.7 or less, 1.6 or less, 1.5 or less, 1.4 or less, 1.3 or less, 1.2 or less, or 1.1 or less.

In some embodiments, suitable administration volumes for each dose are 100 to 400 μl. In some embodiments, suitable administration volumes for each dose are 150 to 350 μl. In some embodiments, suitable administration volumes for each dose are 200 to 300 μl. In some embodiments, suitable administration volumes for each dose are about 200

µl, about 250 µl or about 300 µl.

In some embodiments, the lowest dose of the RNA and the largest dose of the RNA differ by a factor of 2 or more and the smallest administration volume for one dose and the largest administration volume for another dose differ by a factor of 2 or less. In some embodiments, the lowest dose of the RNA and the largest dose of the RNA differ by a factor of 5 or more and the smallest administration volume for one dose and the largest administration volume for another dose differ by a factor of 2 or less. In some embodiments, the lowest dose of the RNA and the largest dose of the RNA differ by a factor of 10 or more and the smallest administration volume for one dose and the largest administration volume for another dose differ by a factor of 2 or less.

In some embodiments, the lowest dose of the RNA and the largest dose of the RNA differ by a factor of 2 or more and the smallest administration volume for one dose and the largest administration volume for another dose differ by a factor of 1.5 or less. In some embodiments, the lowest dose of the RNA and the largest dose of the RNA differ by a factor of 5 or more and the smallest administration volume for one dose and the largest administration volume for another dose differ by a factor of 1.5 or less. In some embodiments, the lowest dose of the RNA and the largest dose of the RNA differ by a factor of 10 or more and the smallest administration volume for one dose and the largest administration volume for another dose differ by a factor of 1.5 or less.

In some embodiments, the lowest dose of the RNA and the largest dose of the RNA differ by a factor of 2 or more and suitable administration volumes are 100 to 400 µl In some embodiments, the lowest dose of the RNA and the largest dose of the RNA differ by a factor of 2 or more and suitable administration volumes are 200 to 300 µl. In some embodiments, the lowest dose of the RNA and the largest dose of the RNA differ by a factor of 5 or more and suitable administration volumes are 100 to 400 µl. In some embodiments, the lowest dose of the RNA and the largest dose of the RNA differ by a factor of 5 or more and suitable administration volumes are 200 to 300 µl. In some embodiments, the lowest dose of the RNA and the largest dose of the RNA differ by a factor of 10 or more and suitable administration volumes are 100 to 400 µl. In some embodiments, the lowest dose of the RNA and the largest dose of the RNA differ by a factor of 10 or more and suitable administration volumes are 200 to 300 µl.

In some embodiments, the different doses comprise at least two doses. In some embodiments, the different doses comprise at least three doses. In some embodiments, the different doses comprise at least four doses. In some embodiments, the different doses comprise at least five doses.

In some embodiments, the different doses of the RNA are to be administered to different age groups. In some embodiments, the different doses of the RNA are to be administered to different patient groups having a different health status. In some embodiments, the different doses of the RNA are to be administered to different patient groups having a different immune status, e.g., immunocompetent and immunocompromised due to, e.g., cancer or infections. In some embodiments, higher doses are administered to adults, e.g., 12 years or older, compared to children, e.g. 5 to 11 years old or 2 to less than 5 years old. In some embodiments, higher doses are administered to adults, e.g., 12 years or older, compared to younger children or infants, e.g. 2 to less than 5 years old, 6 months to less than 2 years old, or less than 6 months old. In some embodiments, higher doses are administered to older children, e.g. 5 to 11 years old, compared to younger children, e.g., 2 to less than 5 years old. In some embodiments, higher doses are administered to children who are 2 to less than 5 years old, as compared to toddlers and/or infants, e.g., who are 6 months to less than 2 years old, or less than 6 months old. In some embodiments, higher doses are administered to immunocompromised patients compared to immunocompetent patients.

In some embodiments, a dilution of the pharmaceutical RNA preparation takes place for at least one dose. In some embodiments, said at least one dose comprises the lowest dose of the RNA. In some embodiments, a dilution of the pharmaceutical RNA preparation by a factor of 1:10 or less takes place for at least one dose. In some embodiments, a dilution of the pharmaceutical RNA preparation by a factor of 1:9 or less takes place for at least one dose. In some embodiments, a dilution of the pharmaceutical RNA preparation by a factor of 1:8 or less takes place for at least one dose. In some embodiments, a dilution of the pharmaceutical RNA preparation by a factor of 1:7 or less takes place for at least one dose. In some embodiments, a dilution of the pharmaceutical RNA preparation by a factor of 1:6 or less takes place for at least one dose. In some embodiments, a dilution of the pharmaceutical RNA preparation by a factor of 1:5 or less takes place for at least one dose.

In some embodiments, at least one dose of the RNA is administered without diluting the RNA. In some embodiments, said at least one dose comprises the largest dose of the RNA.

In some embodiments, the pharmaceutical RNA preparation is provided as multi-dose preparations, wherein each multi-dose preparation allows multiple administrations of a dose of the RNA. In some embodiments, the multi-dose preparations comprise at least two different preparations for administering at least two different doses. In some embodiments, the multi-dose preparations comprise at least three different preparations for administering at least three different doses. In some embodiments, the multi-dose preparations comprise at least four different preparations for administering at least four different doses. In some embodiments, the multi-dose preparations comprise at least five different preparations for administering at least five different doses.

In some embodiments, a multi-dose preparation comprises several units such as containers, e.g., vials, for

said multi-dose preparation. In some embodiments, each of several multi-dose preparations comprises several units such as containers, e.g., vials, for said multi-dose preparation. In some embodiments, each multi-dose preparation comprises several units such as containers, e.g., vials, for said multi-dose preparation. In some embodiments, said several units such as containers, e.g., vials, comprise 2 or more, 3 or more, 4 or more, 5 or more, 6 or more, 7 or more, 8 or more, 9 or more, or 10 or more of said units. In some embodiments, said several units such as containers, e.g., vials, are provided as a kit. In some embodiments, different multi-dose preparations are provided as a kit. In some embodiments, different multi-dose preparations are provided as a kit and several units such as containers, e.g., vials, for each multi-dose preparation are provided in the kit. In some embodiments, said several units such as containers, e.g., vials, each contain the same filling volume of the pharmaceutical RNA preparation.

In some embodiments, the plurality of vials or multi-dose preparations have filling volumes that are suitable for pharmaceutical RNA manufacture. In some embodiments, the plurality of vials or multi-dose preparations have filling volumes between 0.2 and 2.5 ml. In some embodiments, the lower limit of the filling volumes is 0.3 ml. In some embodiments, the lower limit of the filling volumes is 0.4 ml. In some embodiments, the lower limit of the filling volumes is 0.5 ml. In some embodiments, the upper limit of the filling volumes is 2.4 ml. In some embodiments, the upper limit of the filling volumes is 2.3 ml.

In some embodiments, a vial or multi-dose preparation allows the administration of a desired number of doses of the RNA, e.g., 5 or more, such as 5-20, 5-15, or 5-10 doses (optionally following suitable dilution of the pharmaceutical RNA preparation.

In some embodiments, a vial or multi-dose preparation allows an optional dilution of the pharmaceutical RNA preparation, e.g., a dilution by a factor as described herein.

In some embodiments, vials which are used to administer different doses of the RNA are labelled differently.

In some embodiments, the different labels comprise a different color of the lid.

In some embodiments, the pharmaceutical RNA preparation for administration of different doses of the RNA has a uniform RNA concentration. In some embodiments, the RNA concentration is between 0.05 mg/ml to 0.5 mg/ml. In some embodiments, the RNA concentration is between 0.06 mg/ml to 0.4 mg/ml. In some embodiments, the RNA concentration is between 0.07 mg/ml to 0.3 mg/ml. In some embodiments, the RNA concentration is between 0.08 mg/ml to 0.2 mg/ml. In some embodiments, the RNA concentration is between 0.09 mg/ml to 0.15 mg/ml. In some embodiments, the RNA concentration is about 0.1 mg/ml.

In some embodiments, the pharmaceutical RNA preparation is a vaccine.

In some embodiments, the RNA encodes an amino acid sequence comprising an antigen, an immunogenic variant thereof, or an immunogenic fragment of the antigen or the immunogenic variant thereof.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof.

In some embodiments, the pharmaceutical RNA preparation is for inducing an immune response against SARS-CoV-2.

In some embodiments, the pharmaceutical RNA preparation is for vaccination against SARS-CoV-2.

In some embodiments, the different doses comprise doses of about 10 μg and about 30 μg. In some embodiments, the different doses comprise doses of about 3 μg and about 10 μg. In some embodiments, the different doses comprise doses of about 3 μg and about 30 μg. In some embodiments, the different doses comprise doses of about 3 μg, about 10 μg and about 30 μg.

In some embodiments, the different doses comprise doses of about 10 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3 μg and about 10 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the different doses comprise doses of about 3μg, about 10 μg and about 30 μg and the pharmaceutical RNA preparation comprises RNA encoding an amino acid sequence comprising a SARS-CoV-2 S protein, an immunogenic variant thereof, or an immunogenic fragment of the SARS-CoV-2 S protein or the immunogenic variant thereof. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is an RNA as described herein. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as nanoparticles. In some embodiments, exemplary nanoparticles include lipid

nanoparticles, polyplexes (PLX), lipidated polyplexes (LPLX), liposomes, or polysaccharide nanoparticles. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the different doses comprise doses of about 10 μg and about 30 μg and the pharmaceutical RNA preparation comprises BNT162b2. In some embodiments, the different doses comprise doses of about 3 μg and about 10 μg and the pharmaceutical RNA preparation comprises BNT162b2. In some embodiments, the different doses comprise doses of about 3 μg and about 30 μg and the pharmaceutical RNA preparation comprises BNT162b2. In some embodiments, the different doses comprise doses of about 3μg, about 10 μg and about 30 μg and the pharmaceutical RNA preparation comprises BNT162b2. In some embodiments, BNT162b2 comprises RNA comprising the sequence of SEQ ID NO: 17. In some embodiments, BNT162b2 is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, BNT162b2 is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the concentration of the RNA in the pharmaceutical RNA preparation is about 0.1 mg/ml. In some embodiments, the concentration of the RNA in the pharmaceutical RNA preparation is about 30 μg/ml to about 100 μg/ml. In some embodiments, the concentration of the RNA in the pharmaceutical RNA preparation is about 50 μg/ml to about 100 μg/ml.

In some embodiments, the administration volumes are between about 200 μl and about 300 μl.

In some embodiments, the RNA in pharmaceutical RNA preparation is formulated in about 10 mM Tris buffer, about 10% sucrose.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 10 μg is administered by diluting the pharmaceutical RNA preparation about 1:1 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 10 μg is administered by diluting the pharmaceutical RNA preparation about 1:1 and administering about 200 μl of diluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 3 μg is administered by diluting the pharmaceutical RNA preparation about 1:5.75 and administering about 200 μl of diluted pharmaceutical RNA preparation.

In some embodiments, the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation,
(ii) a second dose of the RNA of about 10 μg is administered by diluting the pharmaceutical RNA preparation about 1:1 and administering about 200 μl of diluted pharmaceutical RNA preparation, and
(iii) a third dose of the RNA of about 3 μg is administered by diluting the pharmaceutical RNA preparation about 1:5.75 and administering about 200 μl of diluted pharmaceutical RNA preparation.

In some embodiments, the invention provides a system for the administration of different doses of RNA, wherein the system comprises a plurality of vials for administering a plurality of different doses of the RNA, wherein each of the plurality of vials comprises a pharmaceutical RNA preparation, wherein
the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof comprises BNT162b2. In some embodiments, BNT162b2 comprises RNA comprising the sequence of SEQ ID NO: 17.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as nanoparticles. In some embodiments, exemplary nanoparticles include lipid nanoparticles, polyplexes (PLX), lipidated polyplexes (LPLX), liposomes, or polysaccharide nanoparticles. In some embodiments, the RNA encoding an amino acid sequence comprising a SARS-CoV-2 S protein, an immunogenic variant thereof, or an immunogenic fragment of the SARS-CoV-2 S protein or the immunogenic variant thereof is formulated as LNPs. In some embodiments, the LNPs comprise one or more cationically ionizable lipids; one or more neutral lipids (e.g., in some embodiments sterol such as, e.g., cholesterol; and/or phospholipids), and one or more polymer-conjugated lipids. In some embodiments, the formulation comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), DSPC (1,2-distearoyl-sn-glycero-3-phosphocholine), cholesterol, sucrose, trometamol (Tris), trometamol hydrochloride and water.

In some embodiments, the different doses comprise doses of about 10 μg and about 30 μg. In some embodiments, the different doses comprise doses of about 3 μg and about 10 μg. In some embodiments, the different doses comprise doses of about 3 μg and about 30 μg. In some embodiments, the different doses comprise doses of about 3μg, about 10 μg and about 30 μg.

In some embodiments, the administration volumes are between about 200 μl and about 300 μl.

In some embodiments, a first of the plurality of vials is for administering a first dose of the RNA of about 30 μg by administering about 300 μl of undiluted pharmaceutical RNA preparation, and
a second of the plurality of vials is for administering a second dose of the RNA of about 10 μg by diluting the pharmaceutical RNA preparation about 1:1 and administering about 200 μl of diluted pharmaceutical RNA preparation.

In some embodiments, a first of the plurality of vials is for administering a first dose of the RNA of about 10 μg by diluting the pharmaceutical RNA preparation about 1:1 and administering about 200 μl of diluted pharmaceutical RNA preparation, and
a second of the plurality of vials is for administering a second dose of the RNA of about 3 μg by diluting the pharmaceutical RNA preparation about 1:5.75 and administering about 200 μl of diluted pharmaceutical RNA preparation.

In some embodiments, a first of the plurality of vials is for administering a first dose of the RNA of about 30 μg by administering about 300 μl of undiluted pharmaceutical RNA preparation, and
a second of the plurality of vials is for administering a second dose of the RNA of about 3 μg by diluting the pharmaceutical RNA preparation about 1:5.75 and administering about 200 μl of diluted pharmaceutical RNA preparation.

In some embodiments, a first of the plurality of vials is for administering a first dose of the RNA of about 30 μg by administering about 300 μl of undiluted pharmaceutical RNA preparation,

a second of the plurality of vials is for administering a second dose of the RNA of about 10 μg by diluting the pharmaceutical RNA preparation about 1:1 and administering about 200 μl of diluted pharmaceutical RNA preparation, and

a third of the plurality of vials is for administering a third dose of the RNA of about 3 μg by diluting the pharmaceutical RNA preparation about 1:5.75 and administering about 200 μl of diluted pharmaceutical RNA preparation

In some embodiments, the volume of the pharmaceutical RNA preparation in the plurality of vials for administering a dose of the RNA of about 30 μg is about 2.25 ml for administering a total of at least about 6 doses.
In some embodiments, the volume of the pharmaceutical RNA preparation in the plurality of vials for administering a dose of the RNA of about 10 μg is about 1.3 ml for administering a total of at least about 10 doses.
In some embodiments, the volume of the pharmaceutical RNA preparation in the plurality of vials for administering a dose of the RNA of about 3 μg is about 0.4 ml for administering a total of at least about 10 doses.
In some embodiments, vials for administering different doses of the RNA are labelled differently.
In some embodiments, the different labels comprise a different color of the lid.
In some embodiments, a vial described herein is a glass vial (e.g., Type 1 borosilicate glass or aluminosilicate glass), e.g., sealed with a bromobutyl rubber stopper and an aluminum seal with flip-off plastic cap, optionally in pack sizes of 195 vials or 10 vials.

**Definitions**

In the following, definitions will be provided which apply to all aspects of the present disclosure. The following terms have the following meanings unless otherwise indicated. Any undefined terms have their art recognized meanings.
Terms such as "reduce" or "inhibit" as used herein means the ability to cause an overall decrease, for example, of about 5% or greater, about 10% or greater, about 15% or greater, about 20% or greater, about 25% or greater, about 30% or greater, about 40% or greater, about 50% or greater, or about 75% or greater, in the level. The term "inhibit" or similar phrases includes a complete or essentially complete inhibition, i.e. a reduction to zero or essentially to zero.
Terms such as "enhance" as used herein means the ability to cause an overall increase, or enhancement, for example, by at least about 5% or greater, about 10% or greater, about 15% or greater, about 20% or greater, about 25% or greater, about 30% or greater, about 40% or greater, about 50% or greater, about 75% or greater, or about 100% or greater in the level.
"Physiological pH" as used herein refers to a pH of about 7.4. In some embodiments, physiological pH is from 7.3 to 7.5. In some embodiments, physiological pH is from 7.35 to 7.45. In some embodiments, physiological pH is 7.3, 7.35, 7.4, 7.45, or 7.5.
As used in the present disclosure, "% w/v" refers to weight by volume percent, which is a unit of concentration measuring the amount of solute in grams (g) expressed as a percent of the total volume of solution in milliliters (mL).
As used in the present disclosure, "% by weight" refers to weight percent, which is a unit of concentration measuring the amount of a substance in grams (g) expressed as a percent of the total weight of the total composition in grams (g).
As used in the present disclosure, "mol %" is defined as the ratio of the number of moles of one component to the total number of moles of all components, multiplied by 100.
As used in the present disclosure, "mol % of the total lipid" is defined as the ratio of the number of moles of one lipid component to the total number of moles of all lipids, multiplied by 100. In this context, in some embodiments, the term "total lipid" includes lipids and lipid-like material.
The term "ionic strength" refers to the mathematical relationship between the number of different kinds of ionic species in a particular solution and their respective charges. Thus, ionic strength I is represented mathematically by the formula:

$$I = \frac{1}{2} \cdot \sum_i z_i^2 \cdot c_i$$

in which c is the molar concentration of a particular ionic species and z the absolute value of its charge. The sum Σ is taken over all the different kinds of ions (i) in solution.
According to the disclosure, the term "ionic strength" in some embodiments relates to the presence of monovalent ions. Regarding the presence of divalent ions, in particular divalent cations, their concentration or effective concentration (presence of free ions) due to the presence of chelating agents is, in some embodiments, sufficiently low so as to prevent degradation of the nucleic acid. In some embodiments, the concentration or effective concentration of divalent ions is below the catalytic level for hydrolysis of the phosphodiester bonds between nucleotides such as RNA nucleotides. In some embodiments, the concentration of free divalent ions is 20 μM or less. In some embodiments, there are no or essentially no free divalent ions.

"Osmolality" refers to the concentration of a particular solute expressed as the number of osmoles of solute per kilogram of solvent.

The term "lyophilizing" or "lyophilization" refers to the freeze-drying of a substance by freezing it and then reducing the surrounding pressure (e.g., below 15 Pa, such as below 10 Pa, below 5 Pa, or 1 Pa or less) to allow the frozen medium in the substance to sublimate directly from the solid phase to the gas phase. Thus, the terms "lyophilizing" and "freeze-drying" are used herein interchangeably.

The term "spray-drying" refers to spray-drying a substance by mixing (heated) gas with a fluid that is atomized (sprayed) within a vessel (spray dryer), where the solvent from the formed droplets evaporates, leading to a dry powder.

The term "reconstitute" relates to adding a solvent such as water to a dried product to return it to a liquid state such as its original liquid state.

The term "recombinant" in the context of the present disclosure means "made through genetic engineering". In some embodiments, a "recombinant object" in the context of the present disclosure is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

As used herein, the terms "room temperature" and "ambient temperature" are used interchangeably herein and refer to temperatures from at least about 15°C, e.g., from about 15°C to about 35°C, from about 15°C to about 30°C, from about 15°C to about 25°C, or from about 17°C to about 22°C. Such temperatures will include 15°C, 16°C, 17°C, 18°C, 19°C, 20°C, 21°C and 22°C.

The term "EDTA" refers to ethylenediaminetetraacetic acid disodium salt. All concentrations are given with respect to the EDTA disodium salt.

The term "cryoprotectant" relates to a substance that is added to a formulation in order to protect the active ingredients during the freezing stages.

The term "lyoprotectant" relates to a substance that is added to a formulation in order to protect the active ingredients during the drying stages.

According to the present disclosure, the term "peptide" refers to substances which comprise about two or more, about 3 or more, about 4 or more, about 6 or more, about 8 or more, about 10 or more, about 13 or more, about 16 or more, about 20 or more, and up to about 50, about 100 or about 150, consecutive amino acids linked to one another via peptide bonds. The term "polypeptide" refers to large peptides, in particular peptides having at least about 151 amino acids. "Peptides" and "polypeptides" are both protein molecules, although the terms "protein" and "polypeptide" are used herein usually as synonyms.

The term "biological activity" means the response of a biological system to a molecule. Such biological systems may be, for example, a cell or an organism. In some embodiments, such response is therapeutically or pharmaceutically useful.

The term "portion" refers to a fraction. With respect to a particular structure such as an amino acid sequence or protein the term "portion" thereof may designate a continuous or a discontinuous fraction of said structure.

The terms "part" and "fragment" are used interchangeably herein and refer to a continuous element. For example, a part of a structure such as an amino acid sequence or protein refers to a continuous element of said structure. When used in context of a composition, the term "part" means a portion of the composition. For example, a part of a composition may be any portion from 0.1% to 99.9% (such as 0.1%, 0.5%, 1%, 5%, 10%, 50%, 90%, or 99%) of said composition.

"Fragment", with reference to an amino acid sequence (peptide or polypeptide), relates to a part of an amino acid sequence, i.e. a sequence which represents the amino acid sequence shortened at the N-terminus and/or C-terminus. A fragment shortened at the C-terminus (N-terminal fragment) is obtainable, e.g., by translation of a truncated open reading frame that lacks the 3'-end of the open reading frame. A fragment shortened at the N-terminus (C-terminal fragment) is obtainable, e.g., by translation of a truncated open reading frame that lacks the 5'-end of the open reading frame, as long as the truncated open reading frame comprises a start codon that serves to initiate translation. A fragment of an amino acid sequence comprises, e.g., at least 50 %, at least 60 %, at least 70 %, at least 80%, at least 90% of the amino acid residues from an amino acid sequence. A fragment of an amino acid sequence comprises, e.g., at least 6, in particular at least 8, , at least 10, at least 12, at least 15, at least 20, at least 30, at least 50, or at least 100 consecutive amino acids from an amino acid sequence. A fragment of an amino acid sequence comprises, e.g., a sequence of up to 8, in particular up to 10, up to 12, up to 15, up to 20, up to 30 or up to 55, consecutive amino acids of the amino acid sequence.

"Variant," as used herein and with reference to an amino acid sequence (peptide or polypeptide), is meant an amino acid sequence that differs from a parent amino acid sequence by virtue of at least one amino acid (e.g., a different amino acid, or a modification of the same amino acid). The parent amino acid sequence may be a naturally occurring or wild type (WT) amino acid sequence, or may be a modified version of a wild type amino acid sequence. In some

embodiments, the variant amino acid sequence has at least one amino acid difference as compared to the parent amino acid sequence, e.g., from 1 to about 20 amino acid differences, such as from 1 to about 10 or from 1 to about 5 amino acid differences compared to the parent.

By "wild type" or "WT" or "native" herein is meant an amino acid sequence that is found in nature, including allelic variations. A wild type amino acid sequence, peptide or polypeptide has an amino acid sequence that has not been intentionally modified.

For the purposes of the present disclosure, "variants" of an amino acid sequence (peptide or polypeptide) may comprise amino acid insertion variants, amino acid addition variants, amino acid deletion variants and/or amino acid substitution variants. The term "variant" includes all mutants, splice variants, post-translationally modified variants, conformations, isoforms, allelic variants, species variants, and species homologs, in particular those which are naturally occurring. The term "variant" includes, in particular, fragments of an amino acid sequence. Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous peptides or polypeptides and/or to replacing amino acids with other ones having similar properties. In some embodiments, amino acid changes in peptide and polypeptide variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. In some embodiments, conservative amino acid substitutions include substitutions within the following groups:

- glycine, alanine;
- valine, isoleucine, leucine;
- aspartic acid, glutamic acid;
- asparagine, glutamine;
- serine, threonine;
- lysine, arginine; and
- phenylalanine, tyrosine.

In some embodiments the degree of similarity, such as identity between a given amino acid sequence and an amino acid sequence which is a variant of said given amino acid sequence, will be at least about 60%, 70%, 80%, 81%, 82%, 83%, 84%, 85%, 86%, 87%, 88%, 89%, 90%, 91%, 92%, 93%, 94%, 95%, 96%, 97%, 98%, or 99%. In some embodiments, the degree of similarity or identity is given for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given, e.g., for at least about 20, at least about 40, at least about 60, at least about 80, at least about 100, at least about 120, at least about 140, at least about 160, at least about 180, or about 200 amino acids, in some embodiments continuous amino acids. In some embodiments, the degree of similarity or identity is given for the entire length of the reference amino acid sequence. The alignment for determining sequence similarity, such as sequence identity, can be done with art known tools, such as using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences. "Sequence identity" between two nucleic acid sequences indicates the percentage of nucleotides that are identical between the sequences.

The terms "% identical" and "% identity" or similar terms are intended to refer, in particular, to the percentage of nucleotides or amino acids which are identical in an optimal alignment between the sequences to be compared. Said

percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK_LOC =align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence. Homologous amino acid sequences exhibit according to the disclosure at least 40%, in particular at least 50%, at least 60%, at least 70%, at least 80%, at least 90% and, e.g., at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or polypeptides having substitutions, additions, insertions or deletions, is described in detail in Molecular Cloning: A Laboratory Manual, 4th Edition, M.R. Green and J. Sambrook eds., Cold Spring Harbor Laboratory Press, Cold Spring Harbor 2012, for example. Furthermore, the peptides, polypeptides and amino acid variants described herein may be readily prepared with the aid of known peptide synthesis techniques such as, for example, by solid phase synthesis and similar methods.

In some embodiments, a fragment or variant of an amino acid sequence (peptide or polypeptide) is a "functional fragment" or "functional variant". The term "functional fragment" or "functional variant" of an amino acid sequence relates to any fragment or variant exhibiting one or more functional properties identical or similar to those of the amino acid sequence from which it is derived, *i.e.*, it is functionally equivalent. With respect to antigens or antigenic sequences, one particular function is one or more immunogenic activities displayed by the amino acid sequence from which the fragment or variant is derived. The term "functional fragment" or "functional variant", as used herein, in particular refers to a variant molecule or sequence that comprises an amino acid sequence that is altered by one or more amino acids compared to the amino acid sequence of the parent molecule or sequence and that is still capable of fulfilling one or more of the functions of the parent molecule or sequence, *e.g.,* inducing an immune response. In some embodiments, the modifications in the amino acid sequence of the parent molecule or sequence do not significantly affect or alter the characteristics of the molecule or sequence. In different embodiments, the function of the functional fragment or functional variant may be reduced but still significantly present, *e.g.,* function of the functional fragment or functional variant may be at least 50%, at least 60%, at least 70%, at least 80%, or at least 90% of the parent molecule or sequence. However, in other embodiments, function of the functional fragment or functional variant may be enhanced compared to the parent molecule or sequence.

An amino acid sequence (peptide or polypeptide) "derived from" a designated amino acid sequence (peptide or polypeptide) refers to the origin of the first amino acid sequence. In some embodiments, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof. For example, it will be understood by one of ordinary skill in the art that the antigens suitable for use herein may be altered such that they vary in sequence from the naturally occurring or native sequences from which they were derived, while retaining the desirable activity of the native sequences.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "transfection" relates to the introduction of nucleic acids, in particular RNA, into a cell. For purposes of the present disclosure, the term "transfection" also includes the introduction of a nucleic acid into a cell or the uptake of a nucleic acid by such cell, wherein the cell may be present in a subject, *e.g.,* a patient, or the cell may be *in vitro, e.g.,* outside of a patient. Thus, according to the present disclosure, a cell for transfection of a nucleic acid described herein can be present *in vitro* or *in vivo, e.g.* the cell can form part of an organ, a tissue and/or the body of a patient. According to the disclosure, transfection can be transient or stable. For some applications of transfection, it is sufficient if the transfected genetic material is only transiently expressed. RNA can be transfected into cells to transiently express its coded protein. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. Such stable transfection can be achieved by using virus-based systems or transposon-based systems for transfection, for example. Generally, nucleic acid encoding antigen is transiently transfected into cells. RNA can be transfected into cells to transiently express its coded protein.

The disclosure includes analogs of a peptide or polypeptide. According to the present disclosure, an analog of a peptide or polypeptide is a modified form of said peptide or polypeptide from which it has been derived and has at least one functional property of said peptide or polypeptide. E.g., a pharmacological active analog of a peptide or polypeptide has at least one of the pharmacological activities of the peptide or polypeptide from which the analog has been derived. Such modifications include any chemical modification and comprise single or multiple substitutions, deletions and/or additions of any molecules associated with the peptide or polypeptide, such as carbohydrates, lipids and/or peptides or polypeptides. In some embodiments, "analogs" of peptides or polypeptides include those modified forms resulting from glycosylation, acetylation, phosphorylation, amidation, palmitoylation, myristoylation, isoprenylation, lipidation, alkylation, derivatization, introduction of protective/blocking groups, proteolytic cleavage or binding to an antibody or to another cellular ligand. The term "analog" also extends to all functional chemical equivalents of said peptides and polypeptides.

As used herein, the terms "linked", "fused", or "fusion" are used interchangeably. These terms refer to the joining together of two or more elements or components or domains.

As used herein "endogenous" refers to any material from or produced inside an organism, cell, tissue or system.

As used herein, the term "exogenous" refers to any material introduced from or produced outside an organism, cell, tissue or system.

According to various embodiments of the present disclosure, a nucleic acid such as RNA encoding a peptide or polypeptide is taken up by or introduced, i.e. transfected or transduced, into a cell which cell may be present *in vitro* or in a subject, resulting in expression of said peptide or polypeptide. The cell may, e.g., express the encoded peptide or polypeptide intracellularly (e.g. in the cytoplasm and/or in the nucleus), may secrete the encoded peptide or polypeptide, and/or may express it on the surface.

According to the present disclosure, terms such as "nucleic acid expressing" and "nucleic acid encoding" or similar terms are used interchangeably herein and with respect to a particular peptide or polypeptide mean that the nucleic acid, if present in the appropriate environment, e.g. within a cell, can be expressed to produce said peptide or polypeptide.

The term "expression" as used herein includes the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

A medical preparation, in particular kit, described herein may comprise instructional material or instructions. As used herein, "instructional material" or "instructions" includes a publication, a recording, a diagram, or any other medium of expression which can be used to communicate the usefulness of the compositions and methods of the invention. The instructional material of the kit of the invention may, for example, be affixed to a container which contains the compositions of the invention or be shipped together with a container which contains the compositions. Alternatively, the instructional material may be shipped separately from the container with the intention that the instructional material and the compositions be used cooperatively by the recipient.

Prodrugs of a particular compound described herein are those compounds that upon administration to an

individual undergo chemical conversion under physiological conditions to provide the particular compound. Additionally, prodrugs can be converted to the particular compound by chemical or biochemical methods in an *ex vivo* environment. For example, prodrugs can be slowly converted to the particular compound when, for example, placed in a transdermal patch reservoir with a suitable enzyme or chemical reagent. Exemplary prodrugs are esters (using an alcohol or a carboxy group contained in the particular compound) or amides (using an amino or a carboxy group contained in the particular compound) which are hydrolyzable *in vivo.* Specifically, any amino group which is contained in the particular compound and which bears at least one hydrogen atom can be converted into a prodrug form. Typical N-prodrug forms include carbamates, Mannich bases, enamines, and enaminones.

In the present specification, a structural formula of a compound may represent a certain isomer of said compound. It is to be understood, however, that the present invention includes all isomers such as geometrical isomers, optical isomers based on an asymmetrical carbon, stereoisomers, tautomers and the like which occur structurally and isomer mixtures and is not limited to the description of the formula.

"Isomers" are compounds having the same molecular formula but differ in structure ("structural isomers") or in the geometrical (spatial) positioning of the functional groups and/or atoms ("stereoisomers"). "Enantiomers" are a pair of stereoisomers which are non-superimposable mirror-images of each other. A "racemic mixture" or "racemate" contains a pair of enantiomers in equal amounts and is denoted by the prefix (±). "Diastereomers" are stereoisomers which are non-superimposable and which are not mirror-images of each other. "Tautomers" are structural isomers of the same chemical substance that spontaneously and reversibly interconvert into each other, even when pure, due to the migration of individual atoms or groups of atoms; *i.e.,* the tautomers are in a dynamic chemical equilibrium with each other. An example of tautomers are the isomers of the keto-enol-tautomerism. "Conformers" are stereoisomers that can be interconverted just by rotations about formally single bonds, and include - in particular - those leading to different 3-dimentional forms of (hetero)cyclic rings, such as chair, half-chair, boat, and twist-boat forms of cyclohexane.

The term "average diameter" refers to the mean hydrodynamic diameter of particles as measured by dynamic light scattering (DLS) with data analysis using the so-called cumulant algorithm, which provides as results the so-called $Z_{average}$ with the dimension of a length, and the polydispersity index (PDI), which is dimensionless (Koppel, D., J. Chem. Phys. 57, 1972, pp 4814-4820, ISO 13321). Here "average diameter", "diameter" or "size" for particles is used synonymously with this value of the $Z_{average}$.

In some embodiments, the "polydispersity index" is may be calculated based on dynamic light scattering measurements by the so-called cumulant analysis as mentioned in the definition of the "average diameter". Under certain prerequisites, it can be taken as a measure of the size distribution of an ensemble of nanoparticles.

The "radius of gyration" (abbreviated herein as $R_g$) of a particle about an axis of rotation is the radial distance of a point from the axis of rotation at which, if the whole mass of the particle is assumed to be concentrated, its moment of inertia about the given axis would be the same as with its actual distribution of mass. Mathematically, $R_g$ is the root mean square distance of the particle's components from either its center of mass or a given axis. For example, for a macromolecule composed of n mass elements, of masses $m_i$ ($i$ = 1, 2, 3, ..., $n$), located at fixed distances $s_i$ from the center of mass, $R_g$ is the square-root of the mass average of $s_i^2$ over all mass elements and can be calculated as follows:

$$R_g = \left( \sum_{i=1}^{n} m_i \cdot s_i^2 \Big/ \sum_{i=1}^{n} m_i \right)^{1/2}$$

The radius of gyration can be determined or calculated experimentally, e.g., by using light scattering. In particular, for small scattering vectors $\vec{q}$ the structure function S is defined as follows:

$$S(\vec{q}) \approx N \cdot \left(1 - \frac{q^2 \cdot R_g^2}{3}\right)$$

wherein N is the number of components (Guinier's law).

The "hydrodynamic radius" (which is sometimes called "Stokes radius" or "Stokes-Einstein radius") of a particle is the radius of a hypothetical hard sphere that diffuses at the same rate as said particle. The hydrodynamic radius is related to the mobility of the particle, taking into account not only size but also solvent effects. For example, a smaller charged particle with stronger hydration may have a greater hydrodynamic radius than a larger charged particle with weaker hydration. This is because the smaller particle drags a greater number of water molecules with it as it moves through the solution. Since the actual dimensions of the particle in a solvent are not directly measurable, the hydrodynamic

radius may be defined by the Stokes-Einstein equation:

$$R_h = \frac{k_B \cdot T}{6 \cdot \pi \cdot \eta \cdot D}$$

wherein $k_B$ is the Boltzmann constant; T is the temperature; $\eta$ is the viscosity of the solvent; and D is the diffusion coefficient. The diffusion coefficient can be determined experimentally, *e.g.,* by using dynamic light scattering (DLS). Thus, one procedure to determine the hydrodynamic radius of a particle or a population of particles (such as the hydrodynamic radius of particles contained in a sample or control composition as disclosed herein or the hydrodynamic radius of a particle peak obtained from subjecting such a sample or control composition to field-flow fractionation) is to measure the DLS signal of said particle or population of particles (such as DLS signal of particles contained in a sample or control composition as disclosed herein or the DLS signal of a particle peak obtained from subjecting such a sample or control composition to field-flow fractionation).

The expression "light scattering" as used herein refers to the physical process where light is forced to deviate from a straight trajectory by one or more paths due to localized nonuniformities in the medium through which the light passes.

The term "UV" means ultraviolet and designates a band of the electromagnetic spectrum with a wavelength from 10 nm to 400 nm, *i.e.,* shorter than that of visible light but longer than X-rays.

The expression "multi-angle light scattering" or "MALS" as used herein relates to a technique for measuring the light scattered by a sample into a plurality of angles. "Multi-angle" means in this respect that scattered light can be detected at different discrete angles as measured, for example, by a single detector moved over a range including the specific angles selected or an array of detectors fixed at specific angular locations. In certain embodiments, the light source used in MALS is a laser source (MALLS: multi-angle laser light scattering). Based on the MALS signal of a composition comprising particles and by using an appropriate formalism (*e.g.,* Zimm plot, Berry plot, or Debye plot), it is possible to determine the radius of gyration ($R_g$) and, thus, the size of said particles. Preferably, the Zimm plot is a graphical presentation using the following equation:

$$\frac{R_\theta}{K^* c} = M_w P(\theta) - 2A_2 c M_w^2 P^2(\theta)$$

wherein c is the mass concentration of the particles in the solvent (g/mL); $A_2$ is the second virial coefficient (mol·mL/g$^2$); $P(\vartheta)$ is a form factor relating to the dependence of scattered light intensity on angle; $R_\vartheta$ is the excess Rayleigh ratio (cm$^{-1}$); and K* is an optical constant that is equal to $4\pi^2\eta_o (dn/dc)^2\lambda_0^{-4}N_A^{-1}$, where $\eta_o$ is the refractive index of the solvent at the incident radiation (vacuum) wavelength, $\lambda_0$ is the incident radiation (vacuum) wavelength (nm), $N_A$ is Avogadro's number (mol$^{-1}$), and dn/dc is the differential refractive index increment (mL/g) (cf., e.g., Buchholz et al. (Electrophoresis 22 (2001), 4118-4128); B.H. Zimm (J. Chem. Phys. 13 (1945), 141; P. Debye (J. Appl. Phys. 15 (1944): 338; and W. Burchard (Anal. Chem. 75 (2003), 4279-4291). Preferably, the Berry plot is calculated the following term:

$$\sqrt{\frac{R_\theta}{K^* c}}$$

wherein c, $R_\vartheta$ and $K^*$ are as defined above. Preferably, the Debye plot is calculated the following term:

$$\frac{K^* c}{R_\theta}$$

wherein *c*, $R_\vartheta$ and $K^*$ are as defined above.

The expression "dynamic light scattering" or "DLS" as used herein refers to a technique to determine the size and size distribution profile of particles, in particular with respect to the hydrodynamic radius of the particles. A monochromatic light source, usually a laser, is shot through a polarizer and into a sample. The scattered light then goes through a second polarizer where it is detected and the resulting image is projected onto a screen. The particles in the solution are being hit with the light and diffract the light in all directions. The diffracted light from the particles can either interfere constructively (light regions) or destructively (dark regions). This process is repeated at short time intervals and

the resulting set of speckle patterns are analyzed by an autocorrelator that compares the intensity of light at each spot over time.

The expression "static light scattering" or "SLS" as used herein refers to a technique to determine the size and size distribution profile of particles, in particular with respect to the radius of gyration of the particles, and/or the molar mass of particles. A high-intensity monochromatic light, usually a laser, is launched in a solution containing the particles. One or many detectors are used to measure the scattering intensity at one or many angles. The angular dependence is needed to obtain accurate measurements of both molar mass and size for all macromolecules of radius. Hence simultaneous measurements at several angles relative to the direction of incident light, known as multi-angle light scattering (MALS) or multi-angle laser light scattering (MALLS), is generally regarded as the standard implementation of static light scattering.

**Nucleic Acids**

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. In some embodiments, a nucleic acid is DNA. In some embodiments, a nucleic acid is RNA. In some embodiments, a nucleic acid is a mixture of DNA and RNA. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified *in vitro,* for example via polymerase chain reaction (PCR) for DNA or *in vitro* transcription (using, *e.g.,* an RNA polymerase) for RNA, (ii) was produced recombinantly by cloning, (iii) was purified, for example, by cleavage and separation by gel electrophoresis, or (iv) was synthesized, for example, by chemical synthesis. The term "nucleoside" (abbreviated herein as "N") relates to compounds which can be thought of as nucleotides without a phosphate group. While a nucleoside is a nucleobase linked to a sugar (*e.g.,* ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is, in some embodiments, modified by one or more alkyl groups, e.g., one or more $C_{1-4}$ alkyl groups, e.g., one or more methyl groups. Particular examples of modified purine or pyrimidine base moieties include $N^7$-alkyl-guanine, $N^6$-alkyl-adenine, 5-alkyl-cytosine, 5-alkyl-uracil, and N(1)-alkyl-uracil, such as $N^7$-$C_{1-4}$ alkyl-guanine, $N^6$-$C_{1-a}$ alkyl-adenine, 5-$C_{1-4}$ alkyl-cytosine, 5-$C_{1-4}$ alkyl-uracil, and N(1)-$C_{1-4}$ alkyl-uracil, preferably $N^7$-methyl-guanine, $N^6$-methyl-adenine, 5-methyl-cytosine, 5-methyl-uracil, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double

stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

The term "*in vitro* transcription" or "IVT" as used herein means that the transcription (*i.e.,* the generation of RNA) is conducted in a cell-free manner. *I.e.,* IVT does not use living/cultured cells but rather the transcription machinery extracted from cells (*e.g.,* cell lysates or the isolated components thereof, including an RNA polymerase (preferably T7, T3 or SP6 polymerase)).

**mRNA**

According to the present disclosure, the term "mRNA" means "messenger-RNA" and includes a "transcript" which may be generated by using a DNA template. Generally, mRNA encodes a peptide or polypeptide.

mRNA is single-stranded but may contain self-complementary sequences that allow parts of the mRNA to fold and pair with itself to form double helices.

According to the present disclosure, "dsRNA" means double-stranded RNA and is RNA with two partially or completely complementary strands.

In preferred embodiments of the present disclosure, the mRNA relates to an RNA transcript which encodes a peptide or polypeptide.

In some embodiments, the mRNA which preferably encodes a peptide or polypeptide has a length of at least 45 nucleotides (such as at least 60, at least 90, at least 100, at least 200, at least 300, at least 400, at least 500, at least 600, at least 700, at least 800, at least 900, at least 1,000, at least 1,500, at least 2,000, at least 2,500, at least 3,000, at least 3,500, at least 4,000, at least 4,500, at least 5,000, at least 6,000, at least 7,000, at least 8,000, at least 9,000 nucleotides), preferably up to 15,000, such as up to 14,000, up to 13,000, up to 12,000 nucleotides, up to 11,000 nucleotides or up to 10,000 nucleotides.

As established in the art, mRNA generally contains a 5' untranslated region (5'-UTR), a peptide/polypeptide coding region and a 3' untranslated region (3'-UTR). In some embodiments, the mRNA is produced by *in vitro* transcription or chemical synthesis. In some embodiments, the mRNA is produced by *in vitro* transcription using a DNA template. The *in vitro* transcription methodology is known to the skilled person; cf., *e.g.,* Molecular Cloning: A Laboratory Manual, 4th Edition, M.R. Green and J. Sambrook eds., Cold Spring Harbor Laboratory Press, Cold Spring Harbor 2012. Furthermore, a variety of *in vitro* transcription kits is commercially available, *e.g.,* from Thermo Fisher Scientific (such as TranscriptAid™ T7 kit, MEGAscript® T7 kit, MAXIscript®), New England BioLabs Inc. (such as HiScribe™ T7 kit, HiScribe™ T7 ARCA mRNA kit), Promega (such as RiboMAX™, HeLaScribe®, Riboprobe® systems), Jena Bioscience (such as SP6 or T7 transcription kits), and Epicentre (such as AmpliScribe™). For providing modified mRNA, correspondingly modified nucleotides, such as modified naturally occurring nucleotides, non-naturally occurring nucleotides and/or modified non-naturally occurring nucleotides, can be incorporated during synthesis (preferably *in vitro* transcription), or modifications can be effected in and/or added to the mRNA after transcription.

In some embodiments, mRNA is *in vitro* transcribed mRNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In some embodiments of the present disclosure, the mRNA is "replicon mRNA" or simply a "replicon", in particular "self-replicating mRNA" or "self-amplifying mRNA". In certain embodiments, the replicon or self-replicating mRNA is

derived from or comprises elements derived from an ssRNA virus, in particular a positive-stranded ssRNA virus such as an alphavirus. Alphaviruses are typical representatives of positive-stranded RNA viruses. Alphaviruses replicate in the cytoplasm of infected cells (for review of the alphaviral life cycle see José et al., Future Microbiol., 2009, vol. 4, pp. 837-856). The total genome length of many alphaviruses typically ranges between 11,000 and 12,000 nucleotides, and the genomic RNA typically has a 5'-cap, and a 3' poly(A) tail. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5'terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA molecule that resembles eukaryotic messenger RNA (mRNA; Gould et al., 2010, Antiviral Res., vol. 87 pp. 111-124). Following infection, *i.e.* at early stages of the viral life cycle, the (+) stranded genomic RNA directly acts like a messenger RNA for the translation of the open reading frame encoding the non-structural poly-protein (nsP1234). Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase, and the other nucleic acid molecule is capable of being replicated by said replicase in trans (hence the designation trans-replication system). Trans-replication requires the presence of both these nucleic acid molecules in a given host cell. The nucleic acid molecule capable of being replicated by the replicase in trans must comprise certain alphaviral sequence elements to allow recognition and RNA synthesis by the alphaviral replicase.

In some embodiments of the present disclosure, the mRNA contains one or more modifications, e.g., in order to increase its stability and/or increase translation efficiency and/or decrease immunogenicity and/or decrease cytotoxicity. For example, in order to increase expression of the mRNA, it may be modified within the coding region, *i.e.,* the sequence encoding the expressed peptide or polypeptide, preferably without altering the sequence of the expressed peptide or polypeptide. Such modifications are described, for example, in WO 2007/036366 and PCT/EP2019/056502, and include the following: a 5'-cap structure; an extension or truncation of the naturally occurring poly(A) tail; an alteration of the 5'- and/or 3'-untranslated regions (UTR) such as introduction of a UTR which is not related to the coding region of said RNA; the replacement of one or more naturally occurring nucleotides with synthetic nucleotides; and codon optimization (*e.g.,* to alter, preferably increase, the GC content of the RNA).

In some embodiments, the mRNA comprises a 5'-cap structure. In some embodiments, the mRNA does not have uncapped 5'-triphosphates. In some embodiments, the mRNA may comprise a conventional 5'-cap and/or a 5'-cap analog. The term "conventional 5'-cap" refers to a cap structure found on the 5'-end of an mRNA molecule and generally consists of a guanosine 5'-triphosphate (Gppp) which is connected via its triphosphate moiety to the 5'-end of the next nucleotide of the mRNA (*i.e.,* the guanosine is connected via a 5' to 5' triphosphate linkage to the rest of the mRNA). The guanosine may be methylated at position $N^7$ (resulting in the cap structure $m^7$Gppp). The term "5'-cap analog" includes a 5'-cap which is based on a conventional 5'-cap but which has been modified at either the 2'- or 3'-position of the $m^7$guanosine structure in order to avoid an integration of the 5'-cap analog in the reverse orientation (such 5'-cap analogs are also called anti-reverse cap analogs (ARCAs)). Particularly preferred 5'-cap analogs are those having one or more substitutions at the bridging and non-bridging oxygen in the phosphate bridge, such as phosphorothioate modified 5'-cap analogs at the β-phosphate (such as $m_2^{7,2'O}$G(5')ppSp(5')G (referred to as beta-S-ARCA or β-S-ARCA)), as described in PCT/EP2019/056502. Providing an mRNA with a 5'-cap structure as described herein may be achieved by *in vitro* transcription of a DNA template in presence of a corresponding 5'-cap compound, wherein said 5'-cap structure is co-transcriptionally incorporated into the generated mRNA strand, or the mRNA may be generated, for example, by *in vitro* transcription, and the 5'-cap structure may be attached to the mRNA post-transcriptionally using capping enzymes, for example, capping enzymes of vaccinia virus.

In some embodiments, the mRNA comprises a 5'-cap structure selected from the group consisting of $m_2^{7,2'O}$G(5')ppSp(5')G (in particular its D1 diastereomer), $m_2^{7,3'O}$G(5')ppp(5')G, and $m_2^{7,3'O}$Gppp($m_1^{2'-O}$)ApG. In some embodiments, RNA encoding a peptide or polypeptide comprising an antigen or epitope comprises $m_2^{7,2'O}$G(5')ppSp(5')G (in particular its D1 diastereomer) as 5'-cap structure.

In some embodiments, the mRNA comprises a capO, cap1, or cap2, preferably capl or cap2. According to the present disclosure, the term "capO" means the structure "$m^7$GpppN", wherein N is any nucleoside bearing an OH moiety at position 2'. According to the present disclosure, the term "cap1" means the structure "$m^7$GpppNm", wherein Nm is any nucleoside bearing an $OCH_3$ moiety at position 2'. According to the present disclosure, the term "cap2" means the structure "$m^7$GpppNmNm", wherein each Nm is independently any nucleoside bearing an $OCH_3$ moiety at position 2'.

The 5'-cap analog beta-S-ARCA (β-S-ARCA) has the following structure:

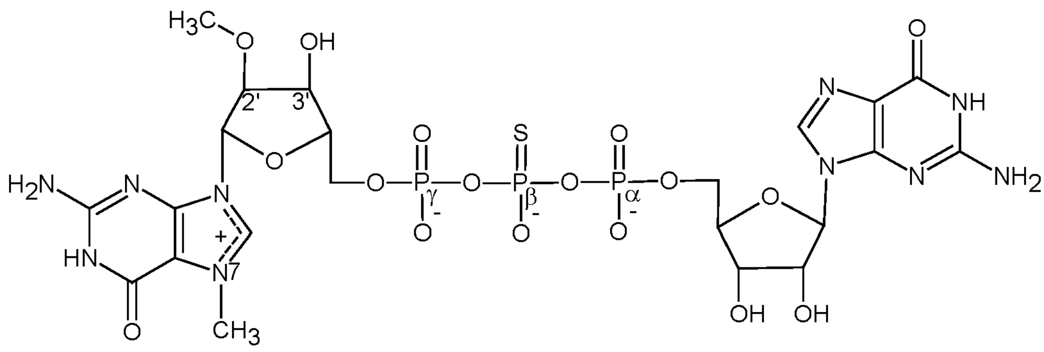

The "D1 diastereomer of beta-S-ARCA" or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time. The HPLC preferably is an analytical HPLC. In some embodiments, a Supelcosil LC-18-T RP column, preferably of the format: 5 μm, 4.6 x 250 mm is used for separation, whereby a flow rate of 1.3 ml/min can be applied. In some embodiments, a gradient of methanol in ammonium acetate, for example, a 0-25% linear gradient of methanol in 0.05 M ammonium acetate, pH = 5.9, within 15 min is used. UV-detection (VWD) can be performed at 260 nm and fluorescence detection (FLD) can be performed with excitation at 280 nm and detection at 337 nm.

The 5'-cap analog $m_2^{7,3'-O}Gppp(m_1^{2'-O})ApG$ (also referred to as $m_2^{7,3'O}G(5')ppp(5')m^{2'-O}ApG$) which is a building block of a capl has the following structure:

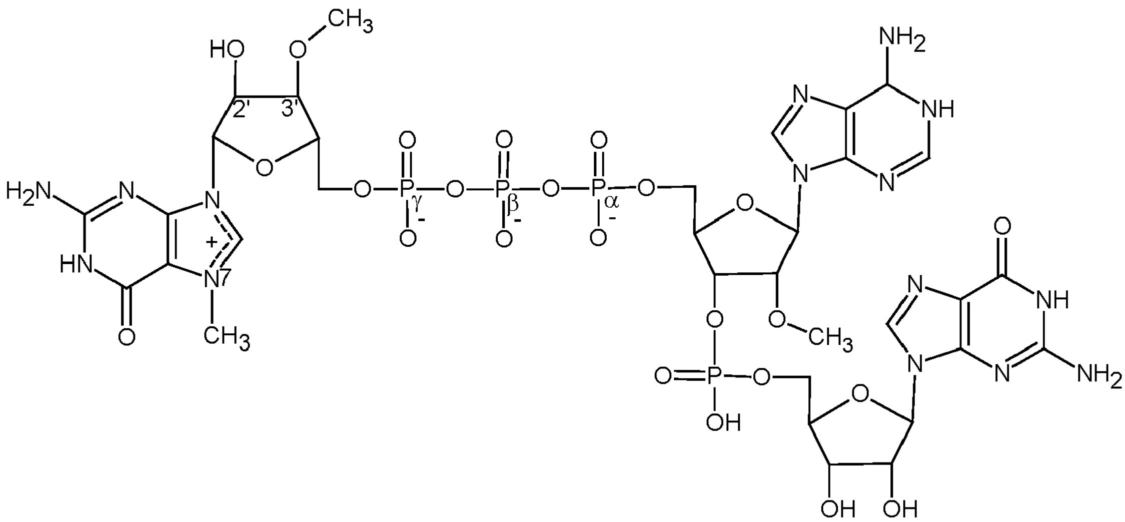

An exemplary capO mRNA comprising β-S-ARCA and mRNA has the following structure:

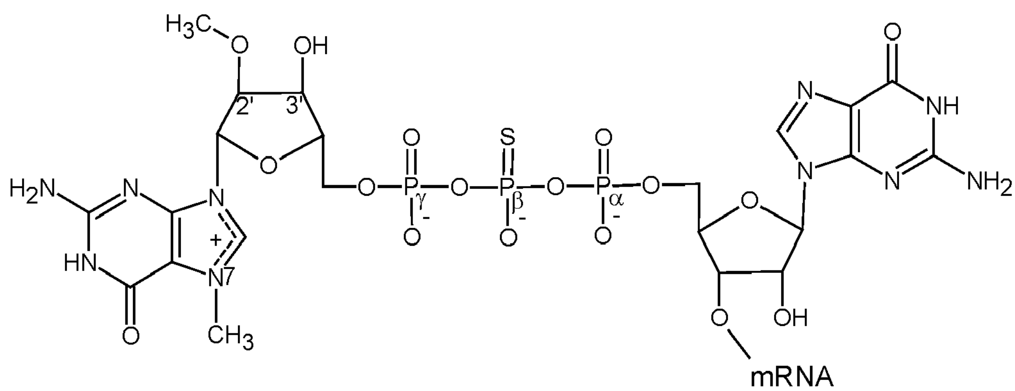

An exemplary capO mRNA comprising $m_2^{7,3'O}G(5')ppp(5')G$ and mRNA has the following structure:

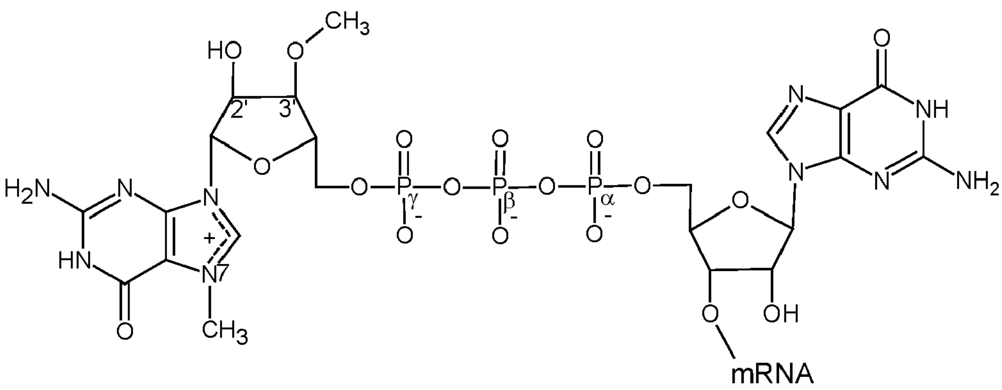

An exemplary capl mRNA comprising $m_2{}^{7,3'-O}Gppp(m_1{}^{2'-O})ApG$ and mRNA has the following structure:

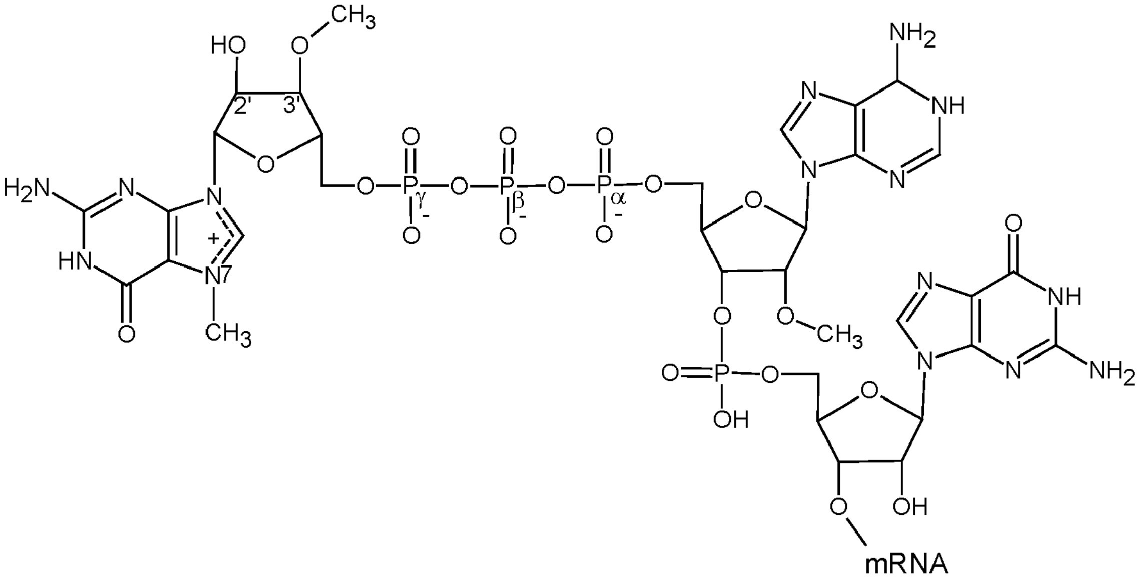

In some embodiments, an RNA comprises a poly-A tail. As used herein, the term "poly-A tail" or "poly-A sequence" refers to an uninterrupted or interrupted sequence of adenylate residues which is typically located at the 3'-end of an mRNA molecule. Poly-A tails or poly-A sequences are known to those of skill in the art and may follow the 3'-UTR in the mRNAs described herein. An uninterrupted poly-A tail is characterized by consecutive adenylate residues. In nature, an uninterrupted poly-A tail is typical. mRNAs disclosed herein can have a poly-A tail attached to the free 3'-end of the mRNA by a template-independent RNA polymerase after transcription or a poly-A tail encoded by DNA and transcribed by a template-dependent RNA polymerase.

It has been demonstrated that a poly-A tail of about 120 A nucleotides has a beneficial influence on the levels of mRNA in transfected eukaryotic cells, as well as on the levels of protein that is translated from an open reading frame that is present upstream (5') of the poly-A tail (Holtkamp et al., 2006, Blood, vol. 108, pp. 4009-4017).

The poly-A tail may be of any length. In some embodiments, a poly-A tail comprises, essentially consists of, or consists of at least 20, at least 30, at least 40, at least 80, or at least 100 and up to 500, up to 400, up to 300, up to 200, or up to 150 A nucleotides, and, in particular, about 120 A nucleotides. In this context, "essentially consists of" means that most nucleotides in the poly-A tail, typically at least 75%, at least 80%, at least 85%, at least 90%, at least 95%, at least 96%, at least 97%, at least 98%, or at least 99% by number of nucleotides in the poly-A tail are A nucleotides, but permits that remaining nucleotides are nucleotides other than A nucleotides, such as U nucleotides (uridylate), G nucleotides (guanylate), or C nucleotides (cytidylate). In this context, "consists of" means that all nucleotides in the poly-A tail, *i.e.,* 100% by number of nucleotides in the poly-A tail, are A nucleotides. The term "A nucleotide" or "A" refers to adenylate.

In some embodiments, a poly-A tail is attached during RNA transcription, *e.g.,* during preparation of *in vitro* transcribed RNA, based on a DNA template comprising repeated dT nucleotides (deoxythymidylate) in the strand complementary to the coding strand. The DNA sequence encoding a poly-A tail (coding strand) is referred to as poly(A)

cassette.

In some embodiments, the poly(A) cassette present in the coding strand of DNA essentially consists of dA nucleotides, but is interrupted by a random sequence of the four nucleotides (dA, dC, dG, and dT). Such random sequence may be 5 to 50, 10 to 30, or 10 to 20 nucleotides in length. Such a cassette is disclosed in WO 2016/005324 A1, hereby incorporated by reference. Any poly(A) cassette disclosed in WO 2016/005324 A1 may be used in the present disclosure. A poly(A) cassette that essentially consists of dA nucleotides, but is interrupted by a random sequence having an equal distribution of the four nucleotides (dA, dC, dG, dT) and having a length of *e.g.,* 5 to 50 nucleotides shows, on DNA level, constant propagation of plasmid DNA in *E. coli* and is still associated, on RNA level, with the beneficial properties with respect to supporting RNA stability and translational efficiency is encompassed. Consequently, in some embodiments, the poly-A tail contained in an mRNA molecule described herein essentially consists of A nucleotides, but is interrupted by a random sequence of the four nucleotides (A, C, G, U). Such random sequence may be 5 to 50, 10 to 30, or 10 to 20 nucleotides in length.

In some embodiments, no nucleotides other than A nucleotides flank a poly-A tail at its 3'-end, *i.e.,* the poly-A tail is not masked or followed at its 3'-end by a nucleotide other than A.

In some embodiments, a poly-A tail may comprise at least 20, at least 30, at least 40, at least 80, or at least 100 and up to 500, up to 400, up to 300, up to 200, or up to 150 nucleotides. In some embodiments, the poly-A tail may essentially consist of at least 20, at least 30, at least 40, at least 80, or at least 100 and up to 500, up to 400, up to 300, up to 200, or up to 150 nucleotides. In some embodiments, the poly-A tail may consist of at least 20, at least 30, at least 40, at least 80, or at least 100 and up to 500, up to 400, up to 300, up to 200, or up to 150 nucleotides. In some embodiments, the poly-A tail comprises the poly-A tail shown in SEQ ID NO: 8. In some embodiments, the poly-A tail comprises at least 100 nucleotides. In some embodiments, the poly-A tail comprises about 150 nucleotides. In some embodiments, the poly-A tail comprises about 120 nucleotides.

In some embodiments, mRNA used in present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5'-end, upstream of the start codon of a protein-encoding region. A 5'-UTR is downstream of the 5'-cap (if present), e.g., directly adjacent to the 5'-cap. A 3'-UTR, if present, is located at the 3'-end, downstream of the termination codon of a protein-encoding region, but the term "3'-UTR" does generally not include the poly-A sequence. Thus, the 3'-UTR is upstream of the poly-A sequence (if present), e.g., directly adjacent to the poly-A sequence. Incorporation of a 3'-UTR into the 3'-non translated region of an RNA (preferably mRNA) molecule can result in an enhancement in translation efficiency. A synergistic effect may be achieved by incorporating two or more of such 3'-UTRs (which are preferably arranged in a head-to-tail orientation; cf., *e.g.,* Holtkamp et al., Blood 108, 4009-4017 (2006)). The 3'-UTRs may be autologous or heterologous to the RNA (e.g., mRNA) into which they are introduced. In certain embodiments, the 3'-UTR is derived from a globin gene or mRNA, such as a gene or mRNA of alpha2-globin, alpha1-globin, or beta-globin, e.g., beta-globin, e.g., human beta-globin. For example, the RNA (e.g., mRNA) may be modified by the replacement of the existing 3'-UTR with or the insertion of one or more, e.g., two copies of a 3'-UTR derived from a globin gene, such as alpha2-globin, alpha1-globin, beta-globin, e.g., beta-globin, e.g., human beta-globin.

A particularly preferred 5'-UTR comprises the nucleotide sequence of SEQ ID NO: 6. A particularly preferred 3'-UTR comprises the nucleotide sequence of SEQ ID NO: 7.

In some embodiments, RNA comprises a 5'-UTR comprising the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6.

In some embodiments, RNA comprises a 3'-UTR comprising the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7.

The mRNA may have modified ribonucleotides in order to increase its stability and/or decrease immunogenicity and/or decrease cytotoxicity. For example, in some embodiments, uridine in the mRNA described herein is replaced (partially or completely, preferably completely) by a modified nucleoside. In some embodiments, the modified nucleoside is a modified uridine.

In some embodiments, the modified uridine replacing uridine is selected from the group consisting of pseudouridine ($\psi$), N1-methyl-pseudouridine (m1$\psi$), 5-methyl-uridine (m5U), and combinations thereof.

In some embodiments, the modified nucleoside replacing (partially or completely, preferably completely) uridine in the mRNA may be any one or more of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (hoSU), 5-aminoallyl-uridine, 5-halo-uridine (*e.g.,* 5-iodo-uridineor 5-bromo-uridine), uridine 5-oxyacetic acid (cmo5U), uridine 5-oxyacetic acid methyl ester (mcmoSU), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-taurinomethyl-pseudouridine, 5-taurinomethyl-2-thio-uridine(τm5s2U), 1-taurinomethyl-4-thio-pseudouridine), 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyl-uridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcmSUm), 5-carbamoylmethyl-2'-O-methyl-uridine (ncm5Um), 5-carboxymethylaminomethyl-2'-O-methyl-uridine (cmnmSUm), 3,2'-O-dimethyl-uridine (m3Um), 5-(isopentenylaminomethyl)-2'-O-methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, or any other modified uridine known in the art.

An RNA (preferably mRNA) which is modified by pseudouridine (replacing partially or completely, preferably completely, uridine) is referred to herein as "Ψ-modified", whereas the term "m1Ψ-modified" means that the RNA (preferably mRNA) contains N(1)-methylpseudouridine (replacing partially or completely, preferably completely, uridine). Furthermore, the term "m5U-modified" means that the RNA (preferably mRNA) contains 5-methyluridine (replacing partially or completely, preferably completely, uridine). Such Ψ- or m1Ψ- or m5U-modified RNAs usually exhibit decreased immunogenicity compared to their unmodified forms and, thus, are preferred in applications where the induction of an immune response is to be avoided or minimized. In some embodiments, the RNA (preferably mRNA) contains N(1)-methylpseudouridine replacing completely uridine

The codons of the mRNA used in the present disclosure may further be optimized, e.g., to increase the GC content of the RNA and/or to replace codons which are rare in the cell (or subject) in which the peptide or polypeptide of interest is to be expressed by codons which are synonymous frequent codons in said cell (or subject). In some embodiments, the amino acid sequence encoded by the mRNA used in the present disclosure is encoded by a coding sequence which is codon-optimized and/or the G/C content of which is increased compared to wild type coding sequence. This also includes embodiments, wherein one or more sequence regions of the coding sequence are codon-optimized and/or increased in the G/C content compared to the corresponding sequence regions of the wild type coding sequence. In some embodiments, the codon-optimization and/or the increase in the G/C content preferably does not change the sequence of the encoded amino acid sequence.

The term "codon-optimized" refers to the alteration of codons in the coding region of a nucleic acid molecule to reflect the typical codon usage of a host organism without preferably altering the amino acid sequence encoded by the nucleic acid molecule. Within the context of the present disclosure, coding regions may be codon-optimized for optimal expression in a subject to be treated using the mRNA described herein. Codon-optimization is based on the finding that the translation efficiency is also determined by a different frequency in the occurrence of tRNAs in cells. Thus, the sequence of mRNA may be modified such that codons for which frequently occurring tRNAs are available are inserted in place of "rare codons".

In some embodiments, the guanosine/cytosine (G/C) content of the coding region of the mRNA described herein is increased compared to the G/C content of the corresponding coding sequence of the wild type RNA, wherein the amino acid sequence encoded by the mRNA is preferably not modified compared to the amino acid sequence encoded by the wild type RNA. This modification of the mRNA sequence is based on the fact that the sequence of any RNA region to be translated is important for efficient translation of that mRNA. Sequences having an increased G (guanosine)/C (cytosine) content are more stable than sequences having an increased A (adenosine)/U (uracil) content. In respect to the fact that several codons code for one and the same amino acid (so-called degeneration of the genetic code), the most favorable codons for the stability can be determined (so-called alternative codon usage). Depending on the amino acid to be encoded by the mRNA, there are various possibilities for modification of the mRNA sequence, compared to its wild type sequence. In particular, codons which contain A and/or U nucleotides can be modified by substituting these codons by other codons, which code for the same amino acids but contain no A and/or U or contain a lower content of A and/or U nucleotides.

In various embodiments, the G/C content of the coding region of the mRNA described herein is increased by at least 10%, at least 20%, at least 30%, at least 40%, at least 50%, at least 55%, or even more compared to the G/C content of the coding region of the wild type RNA.

A combination of the above described modifications, i.e., incorporation of a 5'-cap structure, incorporation of

a poly-A sequence, unmasking of a poly-A sequence, alteration of the 5'- and/or 3'-UTR (such as incorporation of one or more 3'-UTRs), replacing one or more naturally occurring nucleotides with synthetic nucleotides (e.g., 5-methylcytidine for cytidine and/or pseudouridine (Ψ) or N(1)-methylpseudouridine (m1Ψ) or 5-methyluridine (m5U) for uridine), and codon optimization, has a synergistic influence on the stability of RNA (preferably mRNA) and increase in translation efficiency. Thus, in some embodiments, the mRNA used in the present disclosure contains a combination of at least two, at least three, at least four or all five of the above-mentioned modifications, i.e., (i) incorporation of a 5'-cap structure, (ii) incorporation of a poly-A sequence, unmasking of a poly-A sequence; (iii) alteration of the 5'- and/or 3'-UTR (such as incorporation of one or more 3'-UTRs); (iv) replacing one or more naturally occurring nucleotides with synthetic nucleotides (e.g., 5-methylcytidine for cytidine and/or pseudouridine (Ψ) or N(1)-methylpseudouridine (m1Ψ) or 5-methyluridine (m5U) for uridine), and (v) codon optimization.

Some aspects of the disclosure involve the targeted delivery of the mRNA disclosed herein to certain cells or tissues. In some embodiments, the disclosure involves targeting the lymphatic system, in particular secondary lymphoid organs, more specifically spleen. Targeting the lymphatic system, in particular secondary lymphoid organs, more specifically spleen is in particular preferred if the mRNA administered is mRNA encoding an antigen or epitope for inducing an immune response. In some embodiments, the target cell is a spleen cell. In some embodiments, the target cell is an antigen presenting cell such as a professional antigen presenting cell in the spleen. In some embodiments, the target cell is a dendritic cell in the spleen. The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naive lymphocytes and initiate an adaptive immune response.

Lipid-based mRNA delivery systems have an inherent preference to the liver. Liver accumulation is caused by the discontinuous nature of the hepatic vasculature or the lipid metabolism (liposomes and lipid or cholesterol conjugates). In some embodiments, the target organ is liver and the target tissue is liver tissue. The delivery to such target tissue is preferred, in particular, if presence of mRNA or of the encoded peptide or polypeptide in this organ or tissue is desired and/or if it is desired to express large amounts of the encoded peptide or polypeptide and/or if systemic presence of the encoded peptide or polypeptide, in particular in significant amounts, is desired or required.

In some embodiments, after administration of the mRNA particles described herein, at least a portion of the mRNA is delivered to a target cell or target organ. In some embodiments, at least a portion of the mRNA is delivered to the cytosol of the target cell. In some embodiments, the mRNA is mRNA encoding a peptide or polypeptide and the mRNA is translated by the target cell to produce the peptide or polypeptide. In some embodiments, the target cell is a cell in the liver. In some embodiments, the target cell is a muscle cell. In some embodiments, the target cell is an endothelial cell. In some embodiments the target cell is a tumor cell or a cell in the tumor microenvironment. In some embodiments, the target cell is a blood cell. In some embodiments, the target cell is a cell in the lymph nodes. In some embodiments, the target cell is a cell in the lung. In some embodiments, the target cell is a blood cell. In some embodiments, the target cell is a cell in the skin. In some embodiments, the target cell is a spleen cell. In some embodiments, the target cell is an antigen presenting cell such as a professional antigen presenting cell in the spleen. In some embodiments, the target cell is a dendritic cell in the spleen. In some embodiments, the target cell is a T cell. In some embodiments, the target cell is a B cell. In some embodiments, the target cell is a NK cell. In some embodiments, the target cell is a monocyte. Thus, RNA particles described herein may be used for delivering mRNA to such target cell.

**Pharmaceutically active peptides or polypeptides**

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

In some embodiments, RNA used in the present disclosure comprises a nucleic acid sequence encoding a peptide or polypeptide, e.g., a pharmaceutically active peptide or polypeptide.

In some embodiments, RNA used in the present disclosure comprises a nucleic acid sequence encoding a peptide or polypeptide, preferably a pharmaceutically active peptide or polypeptide, and is capable of expressing said peptide or polypeptide, in particular if transferred into a cell or subject. Thus, in some embodiments, the nucleic acid used in the present disclosure contains a coding region (open reading frame (ORF)) encoding a peptide or polypeptide,

e.g., encoding a pharmaceutically active peptide or polypeptide. In this respect, an "open reading frame" or "ORF" is a continuous stretch of codons beginning with a start codon and ending with a stop codon. Such nucleic acid encoding a pharmaceutically active peptide or polypeptide is also referred to herein as "pharmaceutically active nucleic acid". In particular, such mRNA encoding a pharmaceutically active peptide or polypeptide is also referred to herein as "pharmaceutically active mRNA". In some embodiments, RNA used in the present disclosure comprises a nucleic acid sequence encoding more than one peptide or polypeptide, e.g., two, three, four or more peptides or polypeptides.

According to the present disclosure, the term "pharmaceutically active peptide or polypeptide" means a peptide or polypeptide that can be used in the treatment of an individual where the expression of a peptide or polypeptide would be of benefit, *e.g.,* in ameliorating the symptoms of a disease. Preferably, a pharmaceutically active peptide or polypeptide has curative or palliative properties and may be administered to ameliorate, relieve, alleviate, reverse, delay onset of or lessen the severity of one or more symptoms of a disease. In some embodiments, a pharmaceutically active peptide or polypeptide has a positive or advantageous effect on the condition or disease state of an individual when administered to the individual in a therapeutically effective amount. A pharmaceutically active peptide or polypeptide may have prophylactic properties and may be used to delay the onset of a disease or to lessen the severity of such disease. The term "pharmaceutically active peptide or polypeptide" includes entire peptides or polypeptides, and can also refer to pharmaceutically active fragments thereof. It can also include pharmaceutically active variants and/or analogs of a peptide or polypeptide.

Specific examples of pharmaceutically active peptides and polypeptides include, but are not limited to, immunostimulants, e.g., cytokines, hormones, adhesion molecules, immunoglobulins, immunologically active compounds, growth factors, protease inhibitors, enzymes, receptors, apoptosis regulators, transcription factors, tumor suppressor proteins, structural proteins, reprogramming factors, genomic engineering proteins, and blood proteins.

An "immunostimulant" is any substance that stimulates the immune system by inducing activation or increasing activity of any of the immune system's components, in particular immune effector cells. The immunostimulant may be pro-inflammatory (e.g., when treating infections or cancer), or anti-inflammatory (e.g., when treating autoimmune diseases). According to one aspect, the immunostimulant is a cytokine or a variant thereof. Examples of cytokines include interferons, such as interferon-alpha (IFN-$\alpha$) or interferon-gamma (IFN-y), interleukins, such as IL2, IL7, IL12, IL15 and IL23, colony stimulating factors, such as M-CSF and GM-CSF, and tumor necrosis factor. According to another aspect, the immunostimulant includes an adjuvant-type immunostimulatory agent such as APC Toll-like Receptor agonists or costimulatory/cell adhesion membrane proteins. Examples of Toll-like Receptor agonists include costimulatory/adhesion proteins such as CD80, CD86, and ICAM-1.

The term "cytokines" relates to proteins which have a molecular weight of about 5 to 60 kDa and which participate in cell signaling (*e.g.,* paracrine, endocrine, and/or autocrine signaling). In particular, when released, cytokines exert an effect on the behavior of cells around the place of their release. Examples of cytokines include lymphokines, interleukins, chemokines, interferons, and tumor necrosis factors (TNFs). According to the present disclosure, cytokines do not include hormones or growth factors. Cytokines differ from hormones in that (i) they usually act at much more variable concentrations than hormones and (ii) generally are made by a broad range of cells (nearly all nucleated cells can produce cytokines). Interferons are usually characterized by antiviral, antiproliferative and immunomodulatory activities. Interferons are proteins that alter and regulate the transcription of genes within a cell by binding to interferon receptors on the regulated cell's surface, thereby preventing viral replication within the cells. The interferons can be grouped into two types. Particular examples of cytokines include erythropoietin (EPO), colony stimulating factor (CSF), granulocyte colony stimulating factor (G-CSF), granulocyte-macrophage colony stimulating factor (GM-CSF), tumor necrosis factor (TNF), bone morphogenetic protein (BMP), interferon alfa (IFN$\alpha$), interferon beta (IFN$\beta$), interferon gamma (INFy), interleukin 2 (IL-2), interleukin 4 (IL-4), interleukin 10 (IL-10), interleukin 11 (IL-11), interleukin 12 (IL-12), interleukin 15 (IL-15), and interleukin 21 (IL-21), as well as variants and derivatives thereof.

According to the disclosure, a cytokine may be a naturally occurring cytokine or a functional fragment or variant thereof. A cytokine may be human cytokine and may be derived from any vertebrate, especially any mammal. One particularly preferred cytokine is interferon-$\alpha$. Immunostimulants may be provided to a subject by administering to the subject RNA encoding an immunostimulant in a formulation for preferential delivery of RNA to liver or liver tissue. The delivery of RNA to such target organ or tissue is preferred, in particular, if it is desired to express large amounts of the immunostimulant and/or if systemic presence of the immunostimulant, in particular in significant amounts, is desired or required.

RNA delivery systems have an inherent preference to the liver. This pertains to lipid-based particles, cationic and neutral nanoparticles, in particular lipid nanoparticles.

Examples of suitable immunostimulants for targeting liver are cytokines involved in T cell proliferation and/or maintenance. Examples of suitable cytokines include IL2 or IL7, fragments and variants thereof, and fusion proteins of these cytokines, fragments and variants, such as extended-PK cytokines.

In another embodiment, RNA encoding an immunostimulant may be administered in a formulation for preferential delivery of RNA to the lymphatic system, in particular secondary lymphoid organs, more specifically spleen. The delivery

of an immunostimulant to such target tissue is preferred, in particular, if presence of the immunostimulant in this organ or tissue is desired (e.g., for inducing an immune response, in particular in case immunostimulants such as cytokines are required during T-cell priming or for activation of resident immune cells), while it is not desired that the immunostimulant is present systemically, in particular in significant amounts (e.g., because the immunostimulant has systemic toxicity).

Examples of suitable immunostimulants are cytokines involved in T cell priming. Examples of suitable cytokines include IL12, IL15, IFN-α, or IFN-β, fragments and variants thereof, and fusion proteins of these cytokines, fragments and variants, such as extended-PK cytokines. Interferons (IFNs) are a group of signaling proteins made and released by host cells in response to the presence of several pathogens, such as viruses, bacteria, parasites, and also tumor cells. In a typical scenario, a virus-infected cell will release interferons causing nearby cells to heighten their anti-viral defenses.

Based on the type of receptor through which they signal, interferons are typically divided among three classes: type I interferon, type II interferon, and type III interferon.

All type I interferons bind to a specific cell surface receptor complex known as the IFN-α/β receptor (IFNAR) that consists of IFNAR1 and IFNAR2 chains.

The type I interferons present in humans are IFNα, IFNβ, IFNε, IFNκ and IFNω. In general, type I interferons are produced when the body recognizes a virus that has invaded it. They are produced by fibroblasts and monocytes. Once released, type I interferons bind to specific receptors on target cells, which leads to expression of proteins that will prevent the virus from producing and replicating its RNA and DNA.

The IFNα proteins are produced mainly by plasmacytoid dendritic cells (pDCs). They are mainly involved in innate immunity against viral infection. The genes responsible for their synthesis come in 13 subtypes that are called IFNA1, IFNA2, IFNA4, IFNA5, IFNA6, IFNA7, IFNA8, IFNA10, IFNA13, IFNA14, IFNA16, IFNA17, IFNA21. These genes are found together in a cluster on chromosome 9.

The IFNβ proteins are produced in large quantities by fibroblasts. They have antiviral activity that is involved mainly in innate immune response. Two types of IFNβ have been described, IFNβ1 and IFNβ3. The natural and recombinant forms of IFNβ1 have antiviral, antibacterial, and anticancer properties.

Type II interferon (IFNy in humans) is also known as immune interferon and is activated by IL12. Furthermore, type II interferons are released by cytotoxic T cells and T helper cells.

Type III interferons signal through a receptor complex consisting of IL10R2 (also called CRF2-4) and IFNLR1 (also called CRF2-12). Although discovered more recently than type I and type II IFNs, recent information demonstrates the importance of type III IFNs in some types of virus or fungal infections.

In general, type I and II interferons are responsible for regulating and activating the immune response.

According to the disclosure, a type I interferon is preferably IFNα or IFNβ, more preferably IFNα.

According to the disclosure, an interferon may be a naturally occurring interferon or a functional fragment or variant thereof. An interferon may be human interferon and may be derived from any vertebrate, especially any mammal.

Interleukins (ILs) are a group of cytokines (secreted proteins and signal molecules) that can be divided into four major groups based on distinguishing structural features. However, their amino acid sequence similarity is rather weak (typically 15-25% identity). The human genome encodes more than 50 interleukins and related proteins.

According to the disclosure, an interleukin may be a naturally occurring interleukin or a functional fragment or variant thereof. An interleukin may be human interleukin and may be derived from any vertebrate, especially any mammal.

Immunostimulant polypeptides described herein can be prepared as fusion or chimeric polypeptides that include an immunostimulant portion and a heterologous polypeptide (i.e., a polypeptide that is not an immunostimulant). The immunostimulant may be fused to an extended-PK group, which increases circulation half-life. Non-limiting examples of extended-PK groups are described infra. It should be understood that other PK groups that increase the circulation half-life of immunostimulants such as cytokines, or variants thereof, are also applicable to the present disclosure. In certain embodiments, the extended-PK group is a serum albumin domain (e.g., mouse serum albumin, human serum albumin).

As used herein, the term "PK" is an acronym for "pharmacokinetic" and encompasses properties of a compound including, by way of example, absorption, distribution, metabolism, and elimination by a subject. As used herein, an "extended-PK group" refers to a protein, peptide, or moiety that increases the circulation half-life of a biologically active molecule when fused to or administered together with the biologically active molecule. Examples of an extended-PK group include serum albumin (e.g., HSA), Immunoglobulin Fc or Fc fragments and variants thereof, transferrin and variants thereof, and human serum albumin (HSA) binders (as disclosed in U.S. Publication Nos. 2005/0287153 and 2007/0003549). Other exemplary extended-PK groups are disclosed in Kontermann, Expert Opin Biol Ther, 2016 Jul;16(7):903-15 which is herein incorporated by reference in its entirety. As used herein, an "extended-PK" immunostimulant refers to an immunostimulant moiety in combination with an extended-PK group. In some embodiments, the extended-PK immunostimulant is a fusion protein in which an immunostimulant moiety is linked or fused to an extended-PK group.

In certain embodiments, the serum half-life of an extended-PK immunostimulant is increased relative to the

immunostimulant alone (i.e., the immunostimulant not fused to an extended-PK group). In certain embodiments, the serum half-life of the extended-PK immunostimulant is at least 20, 40, 60, 80, 100, 120, 150, 180, 200, 400, 600, 800, or 1000% longer relative to the serum half-life of the immunostimulant alone. In certain embodiments, the serum half-life of the extended-PK immunostimulant is at least 1.5-fold, 2-fold, 2.5-fold, 3-fold, 3.5 fold, 4-fold, 4.5-fold, 5-fold, 6-fold, 7-fold, 8-fold, 10-fold, 12-fold, 13-fold, 15-fold, 17-fold, 20-fold, 22- fold, 25-fold, 27-fold, 30-fold, 35-fold, 40-fold, or 50-fold greater than the serum half-life of the immunostimulant alone. In certain embodiments, the serum half-life of the extended-PK immunostimulant is at least 10 hours, 15 hours, 20 hours, 25 hours, 30 hours, 35 hours, 40 hours, 50 hours, 60 hours, 70 hours, 80 hours, 90 hours, 100 hours, 110 hours, 120 hours, 130 hours, 135 hours, 140 hours, 150 hours, 160 hours, or 200 hours.

As used herein, "half-life" refers to the time taken for the serum or plasma concentration of a compound such as a peptide or polypeptide to reduce by 50%, *in vivo,* for example due to degradation and/or clearance or sequestration by natural mechanisms. An extended-PK immunostimulant suitable for use herein is stabilized *in vivo* and its half-life increased by, e.g., fusion to serum albumin (e.g., HSA or MSA), which resist degradation and/or clearance or sequestration. The half-life can be determined in any manner known per se, such as by pharmacokinetic analysis. Suitable techniques will be clear to the person skilled in the art, and may for example generally involve the steps of suitably administering a suitable dose of the amino acid sequence or compound to a subject; collecting blood samples or other samples from said subject at regular intervals; determining the level or concentration of the amino acid sequence or compound in said blood sample; and calculating, from (a plot of) the data thus obtained, the time until the level or concentration of the amino acid sequence or compound has been reduced by 50% compared to the initial level upon dosing. Further details are provided in, e.g., standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, 2nd Rev. Edition, Marcel Dekker (1982).

In certain embodiments, the extended-PK group includes serum albumin, or fragments thereof or variants of the serum albumin or fragments thereof (all of which for the purpose of the present disclosure are comprised by the term "albumin"). Polypeptides described herein may be fused to albumin (or a fragment or variant thereof) to form albumin fusion proteins. Such albumin fusion proteins are described in U.S. Publication No. 20070048282.

As used herein, "albumin fusion protein" refers to a protein formed by the fusion of at least one molecule of albumin (or a fragment or variant thereof) to at least one molecule of a protein such as a therapeutic protein, in particular an immunostimulant. The albumin fusion protein may be generated by translation of a nucleic acid in which a polynucleotide encoding a therapeutic protein is joined in-frame with a polynucleotide encoding an albumin. The therapeutic protein and albumin, once part of the albumin fusion protein, may each be referred to as a "portion", "region" or "moiety" of the albumin fusion protein (e.g., a "therapeutic protein portion" or an "albumin protein portion"). In a highly preferred embodiment, an albumin fusion protein comprises at least one molecule of a therapeutic protein (including, but not limited to a mature form of the therapeutic protein) and at least one molecule of albumin (including but not limited to a mature form of albumin). In some embodiments, an albumin fusion protein is processed by a host cell such as a cell of the target organ for administered RNA, e.g. a liver cell, and secreted into the circulation. Processing of the nascent albumin fusion protein that occurs in the secretory pathways of the host cell used for expression of the RNA may include, but is not limited to signal peptide cleavage; formation of disulfide bonds; proper folding; addition and processing of carbohydrates (such as for example, N- and O-linked glycosylation); specific proteolytic cleavages; and/or assembly into multimeric proteins. An albumin fusion protein is preferably encoded by RNA in a non-processed form which in particular has a signal peptide at its N-terminus and following secretion by a cell is preferably present in the processed form wherein in particular the signal peptide has been cleaved off. In a most preferred embodiment, the "processed form of an albumin fusion protein" refers to an albumin fusion protein product which has undergone N-terminal signal peptide cleavage, herein also referred to as a "mature albumin fusion protein". In preferred embodiments, albumin fusion proteins comprising a therapeutic protein have a higher plasma stability compared to the plasma stability of the same therapeutic protein when not fused to albumin. Plasma stability typically refers to the time period between when the therapeutic protein is administered *in vivo* and carried into the bloodstream and when the therapeutic protein is degraded and cleared from the bloodstream, into an organ, such as the kidney or liver, that ultimately clears the therapeutic protein from the body. Plasma stability is calculated in terms of the half-life of the therapeutic protein in the bloodstream. The half-life of the therapeutic protein in the bloodstream can be readily determined by common assays known in the art.

As used herein, "albumin" refers collectively to albumin protein or amino acid sequence, or an albumin fragment or variant, having one or more functional activities (e.g., biological activities) of albumin. In particular, "albumin" refers to human albumin or fragments or variants thereof especially the mature form of human albumin, or albumin from other vertebrates or fragments thereof, or variants of these molecules. The albumin may be derived from any vertebrate, especially any mammal, for example human, cow, sheep, or pig. Non-mammalian albumins include, but are not limited to, hen and salmon. The albumin portion of the albumin fusion protein may be from a different animal than the therapeutic protein portion.

In certain embodiments, the albumin is human serum albumin (HSA), or fragments or variants thereof, such

as those disclosed in US 5,876,969, WO 2011/124718, WO 2013/075066, and WO 2011/0514789.

The terms, human serum albumin (HSA) and human albumin (HA) are used interchangeably herein. The terms, "albumin and "serum albumin" are broader, and encompass human serum albumin (and fragments and variants thereof) as well as albumin from other species (and fragments and variants thereof).

As used herein, a fragment of albumin sufficient to prolong the therapeutic activity or plasma stability of the therapeutic protein refers to a fragment of albumin sufficient in length or structure to stabilize or prolong the therapeutic activity or plasma stability of the protein so that the plasma stability of the therapeutic protein portion of the albumin fusion protein is prolonged or extended compared to the plasma stability in the non-fusion state.

The albumin portion of the albumin fusion proteins may comprise the full length of the albumin sequence, or may include one or more fragments thereof that are capable of stabilizing or prolonging the therapeutic activity or plasma stability. Such fragments may be of 10 or more amino acids in length or may include about 15, 20, 25, 30, 50, or more contiguous amino acids from the albumin sequence or may include part or all of specific domains of albumin. For instance, one or more fragments of HSA spanning the first two immunoglobulin-like domains may be used. In a preferred embodiment, the HSA fragment is the mature form of HSA.

Generally speaking, an albumin fragment or variant will be at least 100 amino acids long, preferably at least 150 amino acids long.

According to the disclosure, albumin may be naturally occurring albumin or a fragment or variant thereof. Albumin may be human albumin and may be derived from any vertebrate, especially any mammal.

Preferably, the albumin fusion protein comprises albumin as the N-terminal portion, and a therapeutic protein as the C-terminal portion. Alternatively, an albumin fusion protein comprising albumin as the C-terminal portion, and a therapeutic protein as the N-terminal portion may also be used. In other embodiments, the albumin fusion protein has a therapeutic protein fused to both the N-terminus and the C-terminus of albumin. In a preferred embodiment, the therapeutic proteins fused at the N- and C-termini are the same therapeutic proteins. In another preferred embodiment, the therapeutic proteins fused at the N- and C-termini are different therapeutic proteins. In some embodiments, the different therapeutic proteins are both cytokines.

In some embodiments, the therapeutic protein(s) is (are) joined to the albumin through (a) peptide linker(s). A linker peptide between the fused portions may provide greater physical separation between the moieties and thus maximize the accessibility of the therapeutic protein portion, for instance, for binding to its cognate receptor. The linker peptide may consist of amino acids such that it is flexible or more rigid. The linker sequence may be cleavable by a protease or chemically.

As used herein, the term "Fc region" refers to the portion of a native immunoglobulin formed by the respective Fc domains (or Fc moieties) of its two heavy chains. As used herein, the term "Fc domain" refers to a portion or fragment of a single immunoglobulin (Ig) heavy chain wherein the Fc domain does not comprise an Fv domain. In certain embodiments, an Fc domain begins in the hinge region just upstream of the papain cleavage site and ends at the C-terminus of the antibody. Accordingly, a complete Fc domain comprises at least a hinge domain, a CH2 domain, and a CH3 domain. In certain embodiments, an Fc domain comprises at least one of: a hinge (e.g., upper, middle, and/or lower hinge region) domain, a CH2 domain, a CH3 domain, a CH4 domain, or a variant, portion, or fragment thereof. In certain embodiments, an Fc domain comprises a complete Fc domain (i.e., a hinge domain, a CH2 domain, and a CH3 domain). In certain embodiments, an Fc domain comprises a hinge domain (or portion thereof) fused to a CH3 domain (or portion thereof). In certain embodiments, an Fc domain comprises a CH2 domain (or portion thereof) fused to a CH3 domain (or portion thereof). In certain embodiments, an Fc domain consists of a CH3 domain or portion thereof. In certain embodiments, an Fc domain consists of a hinge domain (or portion thereof) and a CH3 domain (or portion thereof). In certain embodiments, an Fc domain consists of a CH2 domain (or portion thereof) and a CH3 domain. In certain embodiments, an Fc domain consists of a hinge domain (or portion thereof) and a CH2 domain (or portion thereof). In certain embodiments, an Fc domain lacks at least a portion of a CH2 domain (e.g., all or part of a CH2 domain). An Fc domain herein generally refers to a polypeptide comprising all or part of the Fc domain of an immunoglobulin heavy-chain. This includes, but is not limited to, polypeptides comprising the entire CH1, hinge, CH2, and/or CH3 domains as well as fragments of such peptides comprising only, e.g., the hinge, CH2, and CH3 domain. The Fc domain may be derived from an immunoglobulin of any species and/or any subtype, including, but not limited to, a human IgG1, IgG2, IgG3, IgG4, IgD, IgA, IgE, or IgM antibody. The Fc domain encompasses native Fc and Fc variant molecules. As set forth herein, it will be understood by one of ordinary skill in the art that any Fc domain may be modified such that it varies in amino acid sequence from the native Fc domain of a naturally occurring immunoglobulin molecule. In certain embodiments, the Fc domain has reduced effector function (e.g., FcγR binding).

The Fc domains of a polypeptide described herein may be derived from different immunoglobulin molecules. For example, an Fc domain of a polypeptide may comprise a CH2 and/or CH3 domain derived from an IgG1 molecule and a hinge region derived from an IgG3 molecule. In another example, an Fc domain can comprise a chimeric hinge region derived, in part, from an IgG1 molecule and, in part, from an IgG3 molecule. In another example, an Fc domain can comprise a chimeric hinge derived, in part, from an IgG1 molecule and, in part, from an IgG4 molecule.

In certain embodiments, an extended-PK group includes an Fc domain or fragments thereof or variants of the Fc domain or fragments thereof (all of which for the purpose of the present disclosure are comprised by the term "Fc domain"). The Fc domain does not contain a variable region that binds to antigen. Fc domains suitable for use in the present disclosure may be obtained from a number of different sources. In certain embodiments, an Fc domain is derived from a human immunoglobulin. In certain embodiments, the Fc domain is from a human IgG1 constant region. It is understood, however, that the Fc domain may be derived from an immunoglobulin of another mammalian species, including for example, a rodent (e.g. a mouse, rat, rabbit, guinea pig) or non- human primate (e.g. chimpanzee, macaque) species. Moreover, the Fc domain (or a fragment or variant thereof) may be derived from any immunoglobulin class, including IgM, IgG, IgD, IgA, and IgE, and any immunoglobulin isotype, including IgG1, IgG2, IgG3, and IgG4.

A variety of Fc domain gene sequences (e.g., mouse and human constant region gene sequences) are available in the form of publicly accessible deposits. Constant region domains comprising an Fc domain sequence can be selected lacking a particular effector function and/or with a particular modification to reduce immunogenicity. Many sequences of antibodies and antibody-encoding genes have been published and suitable Fc domain sequences (e.g. hinge, CH2, and/or CH3 sequences, or fragments or variants thereof) can be derived from these sequences using art recognized techniques.

In certain embodiments, the extended-PK group is a serum albumin binding protein such as those described in US2005/0287153, US2007/0003549, US2007/0178082, US2007/0269422, US2010/0113339, WO2009/083804, and WO2009/133208, which are herein incorporated by reference in their entirety. In certain embodiments, the extended-PK group is transferrin, as disclosed in US 7,176,278 and US 8,158,579, which are herein incorporated by reference in their entirety. In certain embodiments, the extended-PK group is a serum immunoglobulin binding protein such as those disclosed in US2007/0178082, US2014/0220017, and US2017/0145062, which are herein incorporated by reference in their entirety. In certain embodiments, the extended-PK group is a fibronectin (Fn)-based scaffold domain protein that binds to serum albumin, such as those disclosed in US2012/0094909, which is herein incorporated by reference in its entirety. Methods of making fibronectin-based scaffold domain proteins are also disclosed in US2012/0094909. A non-limiting example of a Fn3-based extended-PK group is Fn3(HSA), i.e., a Fn3 protein that binds to human serum albumin.

In certain aspects, the extended-PK immunostimulant, suitable for use according to the disclosure, can employ one or more peptide linkers. As used herein, the term "peptide linker" refers to a peptide or polypeptide sequence which connects two or more domains (e.g., the extended-PK moiety and an immunostimulant moiety) in a linear amino acid sequence of a polypeptide chain. For example, peptide linkers may be used to connect an immunostimulant moiety to a HSA domain.

Linkers suitable for fusing the extended-PK group to e.g. an immunostimulant are well known in the art. Exemplary linkers include glycine-serine-polypeptide linkers, glycine-proline-polypeptide linkers, and proline-alanine polypeptide linkers. In certain embodiments, the linker is a glycine-serine-polypeptide linker, i.e., a peptide that consists of glycine and serine residues.

In some embodiments, a pharmaceutically active peptide or polypeptide comprises a replacement protein. In these embodiments, the present disclosure provides a method for treatment of a subject having a disorder requiring protein replacement (*e.g.*, protein deficiency disorders) comprising administering to the subject nucleic acid as described herein encoding a replacement protein. The term "protein replacement" refers to the introduction of a protein (including functional variants thereof) into a subject having a deficiency in such protein. The term also refers to the introduction of a protein into a subject otherwise requiring or benefiting from providing a protein, *e.g.,* suffering from protein insufficiency. The term "disorder characterized by a protein deficiency" refers to any disorder that presents with a pathology caused by absent or insufficient amounts of a protein. This term encompasses protein folding disorders, *i.e.,* conformational disorders, that result in a biologically inactive protein product. Protein insufficiency can be involved in infectious diseases, immunosuppression, organ failure, glandular problems, radiation illness, nutritional deficiency, poisoning, or other environmental or external insults.

The term "hormones" relates to a class of signaling molecules produced by glands, wherein signaling usually includes the following steps: (i) synthesis of a hormone in a particular tissue; (ii) storage and secretion; (iii) transport of the hormone to its target; (iv) binding of the hormone by a receptor; (v) relay and amplification of the signal; and (vi) breakdown of the hormone. Hormones differ from cytokines in that (1) hormones usually act in less variable concentrations and (2) generally are made by specific kinds of cells. In some embodiments, a "hormone" is a peptide or polypeptide hormone, such as insulin, vasopressin, prolactin, adrenocorticotropic hormone (ACTH), thyroid hormone, growth hormones (such as human grown hormone or bovine somatotropin), oxytocin, atrial-natriuretic peptide (ANP), glucagon, somatostatin, cholecystokinin, gastrin, and leptins.

The term "adhesion molecules" relates to proteins which are located on the surface of a cell and which are involved in binding of the cell with other cells or with the extracellular matrix (ECM). Adhesion molecules are typically transmembrane receptors and can be classified as calcium-independent (*e.g.,* integrins, immunoglobulin superfamily, lymphocyte homing receptors) and calcium-dependent (cadherins and selectins). Particular examples of adhesion molecules are integrins, lymphocyte homing receptors, selectins (*e.g.,* P-selectin), and addressins.

Integrins are also involved in signal transduction. In particular, upon ligand binding, integrins modulate cell signaling pathways, *e.g.,* pathways of transmembrane protein kinases such as receptor tyrosine kinases (RTK). Such regulation can lead to cellular growth, division, survival, or differentiation or to apoptosis. Particular examples of integrins include: $\alpha_1\beta_1$, $\alpha_2\beta_1$, $\alpha_3\beta_1$, $\alpha_4\beta_1$, $\alpha_5\beta_1$, $\alpha_6\beta_1$, $\alpha_7\beta_1$, $\alpha_L\beta_2$, $\alpha_M\beta_2$, $\alpha_{IIb}\beta_3$, $\alpha_v\beta_1$, $\alpha_v\beta_3$, $\alpha_v\beta_5$, $\alpha_v\beta_6$, $\alpha_v\beta_8$, and $\alpha_6\beta_4$.

The term "immunoglobulins" or "immunoglobulin superfamily" refers to molecules which are involved in the recognition, binding, and/or adhesion processes of cells. Molecules belonging to this superfamily share the feature that they contain a region known as immunoglobulin domain or fold. Members of the immunoglobulin superfamily include antibodies (*e.g.,* IgG), T cell receptors (TCRs), major histocompatibility complex (MHC) molecules, co-receptors (*e.g.,* CD4, CD8, CD19), antigen receptor accessory molecules (*e.g.,* CD-3$\gamma$, CD3-$\delta$, CD-3$\varepsilon$, CD79a, CD79b), co-stimulatory or inhibitory molecules (*e.g.,* CD28, CD80, CD86), and other.

The term "immunologically active compound" relates to any compound altering an immune response, e.g., by inducing and/or suppressing maturation of immune cells, inducing and/or suppressing cytokine biosynthesis, and/or altering humoral immunity by stimulating antibody production by B cells. Immunologically active compounds possess potent immunostimulating activity including, but not limited to, antiviral and antitumor activity, and can also down-regulate other aspects of the immune response, for example shifting the immune response away from a TH2 immune response, which is useful for treating a wide range of TH2 mediated diseases. Immunologically active compounds can be useful as vaccine adjuvants. Particular examples of immunologically active compounds include interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, selectins, homing receptors, and antigens, in particular tumor-associated antigens, pathogen-associated antigens (such as bacterial, parasitic, or viral antigens), allergens, and autoantigens. An immunologically active compound may be a vaccine antigen, *i.e.,* an antigen whose inoculation into a subject induces an immune response.

In some embodiments, RNA used in the present disclosure comprises a nucleic acid sequence encoding a peptide or polypeptide comprising an epitope for inducing an immune response against an antigen in a subject. The "peptide or polypeptide comprising an epitope for inducing an immune response against an antigen in a subject" is also designated herein as "vaccine antigen", "peptide and protein antigen" or simply "antigen".

In some embodiments, the RNA encoding vaccine antigen is a single-stranded, 5' capped mRNA that is translated into the respective protein upon entering cells of a subject being administered the RNA, e.g., antigen-presenting cells (APCs). Preferably, the RNA contains structural elements optimized for maximal efficacy of the RNA with respect to stability and translational efficiency (5' cap, 5' UTR, 3' UTR, poly(A) sequence).

In some embodiments, beta-S-ARCA(D1) is utilized as specific capping structure at the 5'-end of the RNA. In some embodiments, the 5'-UTR comprises the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6. In some embodiments, the 3'-UTR comprises the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7. In some embodiments, the poly(A) sequence is 110 nucleotides in length and consists of a stretch of 30 adenosine residues, followed by a 10 nucleotide linker sequence and another 70 adenosine residues. This poly(A) sequence was designed to enhance RNA stability and translational efficiency in dendritic cells. In some embodiments, the poly(A) sequence comprises the nucleotide sequence of SEQ ID NO: 8, or a nucleotide sequence having at least 99%, 98%, 97%, 96%, 95%, 90%, 85%, or 80% identity to the nucleotide sequence of SEQ ID NO: 8.

In some embodiments, the RNA encoding the vaccine antigen is expressed in cells of the subject to provide the vaccine antigen. In some embodiments, expression of the vaccine antigen is at the cell surface. In some embodiments, the vaccine antigen is presented in the context of MHC. In some embodiments, the RNA encoding the vaccine antigen is transiently expressed in cells of the subject. In some embodiments, the RNA encoding the vaccine antigen is administered systemically. In some embodiments, after systemic administration of the RNA encoding the vaccine antigen, expression of the RNA encoding the vaccine antigen in spleen occurs. In some embodiments, after systemic administration of the RNA encoding the vaccine antigen, expression of the RNA encoding the vaccine antigen in antigen presenting cells, preferably professional antigen presenting cells occurs. In some embodiments, the antigen presenting cells are selected from the group consisting of dendritic cells, macrophages and B cells. In some embodiments, after systemic administration of the RNA encoding the vaccine antigen, no or essentially no expression of the RNA encoding the vaccine antigen in lung and/or liver occurs. In some embodiments, after systemic administration of the RNA encoding the vaccine antigen, expression of the RNA encoding the vaccine antigen in spleen is at least 5-fold the amount of expression in lung.

The vaccine antigen comprises an epitope for inducing an immune response against an antigen in a subject. Accordingly, the vaccine antigen comprises an antigenic sequence for inducing an immune response against an antigen in a subject. Such antigenic sequence may correspond to a target antigen or disease-associated antigen, e.g., a protein of an infectious agent (e.g., viral or bacterial antigen) or tumor antigen, or may correspond to an immunogenic variant thereof, or an immunogenic fragment of the target antigen or disease-associated antigen or the immunogenic variant thereof. Thus, the antigenic sequence may comprise at least an epitope of a target antigen or disease-associated antigen

or an immunogenic variant thereof. The antigenic sequences, e.g., epitopes, suitable for use according to the disclosure typically may be derived from a target antigen, i.e. the antigen against which an immune response is to be elicited. For example, the antigenic sequences contained within the vaccine antigen may be a target antigen or a fragment or variant of a target antigen.

The antigenic sequence or a procession product thereof, e.g., a fragment thereof, may bind to the antigen receptor such as TCR or CAR carried by immune effector cells. In some embodiments, the antigenic sequence is selected from the group consisting of the antigen expressed by a target cell to which the immune effector cells are targeted or a fragment thereof, or a variant of the antigenic sequence or the fragment.

A vaccine antigen which may be provided to a subject according to the present disclosure by administering RNA encoding the vaccine antigen, preferably results in the induction of an immune response, e.g., in the stimulation, priming and/or expansion of immune effector cells, in the subject being provided the vaccine antigen. Said immune response, e.g., stimulated, primed and/or expanded immune effector cells, is preferably directed against a target antigen, in particular a target antigen expressed by diseased cells, tissues and/or organs, i.e., a disease-associated antigen. Thus, a vaccine antigen may comprise the disease-associated antigen, or a fragment or variant thereof. In some embodiments, such fragment or variant is immunologically equivalent to the disease-associated antigen.

In the context of the present disclosure, the term "fragment of an antigen" or "variant of an antigen" means an agent which results in the induction of an immune response, e.g., in the stimulation, priming and/or expansion of immune effector cells, which immune response, e.g., stimulated, primed and/or expanded immune effector cells, targets the antigen, i.e. a disease-associated antigen, in particular when presented by diseased cells, tissues and/or organs. Thus, the vaccine antigen may correspond to or may comprise the disease-associated antigen, may correspond to or may comprise a fragment of the disease-associated antigen or may correspond to or may comprise an antigen which is homologous to the disease-associated antigen or a fragment thereof. If the vaccine antigen comprises a fragment of the disease-associated antigen or an amino acid sequence which is homologous to a fragment of the disease-associated antigen said fragment or amino acid sequence may comprise an epitope of the disease-associated antigen to which the antigen receptor of the immune effector cells is targeted or a sequence which is homologous to an epitope of the disease-associated antigen. Thus, according to the disclosure, a vaccine antigen may comprise an immunogenic fragment of a disease-associated antigen or an amino acid sequence being homologous to an immunogenic fragment of a disease-associated antigen. An "immunogenic fragment of an antigen" according to the disclosure preferably relates to a fragment of an antigen which is capable of inducing an immune response against, e.g., stimulating, priming and/or expanding immune effector cells carrying an antigen receptor binding to, the antigen or cells expressing the antigen. It is preferred that the vaccine antigen (similar to the disease-associated antigen) provides the relevant epitope for binding by the antigen receptor present on the immune effector cells. In some embodiments, the vaccine antigen or a fragment thereof (similar to the disease-associated antigen) is expressed on the surface of a cell such as an antigen-presenting cell (optionally in the context of MHC) so as to provide the relevant epitope for binding by immune effector cells. The vaccine antigen may be a recombinant antigen.

In some embodiments of all aspects of the invention, the RNA encoding the vaccine antigen is expressed in cells of a subject to provide the antigen or a procession product thereof for binding by the antigen receptor expressed by immune effector cells, said binding resulting in stimulation, priming and/or expansion of the immune effector cells. An "antigen" according to the present disclosure covers any substance that will elicit an immune response and/or any substance against which an immune response or an immune mechanism such as a cellular response and/or humoral response is directed. This also includes situations wherein the antigen is processed into antigen peptides and an immune response or an immune mechanism is directed against one or more antigen peptides, in particular if presented in the context of MHC molecules. In particular, an "antigen" relates to any substance, such as a peptide or polypeptide, that reacts specifically with antibodies or T-lymphocytes (T-cells). The term "antigen" may comprise a molecule that comprises at least one epitope, such as a T cell epitope. In some embodiments, an antigen is a molecule which, optionally after processing, induces an immune reaction, which may be specific for the antigen (including cells expressing the antigen). In some embodiments, an antigen is a disease-associated antigen, such as a tumor antigen, a viral antigen, or a bacterial antigen, or an epitope derived from such antigen. In some embodiments, an antigen is presented or present on the surface of cells of the immune system such as antigen presenting cells like dendritic cells or macrophages. An antigen or a procession product thereof such as a T cell epitope is in some embodiments bound by an antigen receptor. Accordingly, an antigen or a procession product thereof may react specifically with immune effector cells such as T-lymphocytes (T cells).

The term "autoantigen" or "self-antigen" refers to an antigen which originates from within the body of a subject (*i.e.,* the autoantigen can also be called "autologous antigen") and which produces an abnormally vigorous immune response against this normal part of the body. Such vigorous immune reactions against autoantigens may be the cause of "autoimmune diseases". According to the present disclosure, any suitable antigen may be used, which is a candidate for an immune response, wherein the immune response may comprise a humoral or cellular immune response, or both. In the context of some embodiments of the present disclosure, the antigen is presented by a cell, such as by an antigen

presenting cell, in the context of MHC molecules, which results in an immune response against the antigen. An antigen may be a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present disclosure, an antigen may correspond to a naturally occurring product, for example, a viral protein, or a part thereof.

The term "disease-associated antigen" is used in its broadest sense to refer to any antigen associated with a disease. A disease-associated antigen is a molecule which contains epitopes that will stimulate a host's immune system to make a cellular antigen-specific immune response and/or a humoral antibody response against the disease. Disease-associated antigens include pathogen-associated antigens, *i.e.,* antigens which are associated with infection by microbes, typically microbial antigens (such as bacterial or viral antigens), or antigens associated with cancer, typically tumors, such as tumor antigens.

In some embodiments, the antigen is a tumor antigen, *i.e.,* a part of a tumor cell, in particular those which primarily occur intracellularly or as surface antigens of tumor cells. In another embodiment, the antigen is a pathogen-associated antigen, *i.e.,* an antigen derived from a pathogen, *e.g.,* from a virus, bacterium, unicellular organism, or parasite, for example a viral antigen such as viral ribonucleoprotein or coat protein. In some embodiments, the antigen should be presented by MHC molecules which results in modulation, in particular activation of cells of the immune system, such as CD4+ and CD8+ lymphocytes, in particular via the modulation of the activity of a T-cell receptor.

The term "tumor antigen" or "tumor-associated antigen" refers to a constituent of cancer cells which may be derived from the cytoplasm, the cell surface or the cell nucleus. In particular, it refers to those antigens which are produced intracellularly or as surface antigens on tumor cells. For example, tumor antigens include the carcinoembryonal antigen, $\alpha$1-fetoprotein, isoferritin, and fetal sulphoglycoprotein, $\alpha$2-H-ferroprotein and $\gamma$-fetoprotein, as well as various virus tumor antigens. According to some embodiments of the present disclosure, a tumor antigen comprises any antigen which is characteristic for tumors or cancers as well as for tumor or cancer cells with respect to type and/or expression level.

The term "viral antigen" refers to any viral component having antigenic properties, *i.e.,* being able to provoke an immune response in an individual. The viral antigen may be a viral ribonucleoprotein or an envelope protein.

The term "bacterial antigen" refers to any bacterial component having antigenic properties, *i.e.* being able to provoke an immune response in an individual. The bacterial antigen may be derived from the cell wall or cytoplasm membrane of the bacterium.

The term "epitope" refers to an antigenic determinant in a molecule such as an antigen, *i.e.,* to a part in orfragment of the molecule that is recognized by the immune system, for example, that is recognized by antibodies, T cells or B cells, in particular when presented in the context of MHC molecules. An epitope of a protein may comprises a continuous or discontinuous portion of said protein and, e.g., may be between about 5 and about 100, between about 5 and about 50, between about 8 and about 30, or about 10 and about 25 amino acids in length, for example, the epitope may be preferably 9, 10, 11, 12, 13, 14, 15, 16, 17, 18, 19, 20, 21, 22, 23, 24, or 25 amino acids in length. In some embodiments, the epitope in the context of the present disclosure is a T cell epitope.

Terms such as "epitope", "fragment of an antigen", "immunogenic peptide" and "antigen peptide" are used interchangeably herein and, e.g., may relate to an incomplete representation of an antigen which is, e.g., capable of eliciting an immune response against the antigen or a cell expressing or comprising and presenting the antigen. In some embodiments, the terms relate to an immunogenic portion of an antigen. In some embodiments, it is a portion of an antigen that is recognized (*i.e.,* specifically bound) by a T cell receptor, in particular if presented in the context of MHC molecules. Certain preferred immunogenic portions bind to an MHC class I or class II molecule. The term "epitope" refers to a part or fragment of a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, between about 8 and about 30, or between about 8 and about 25 amino acids in length, for example, the epitope may be 9, 10, 11, 12, 13, 14, 15, 16, 17, 18, 19, 20, 21, 22, 23, 24, or 25 amino acids in length. In some embodiments, an epitope is between about 10 and about 25 amino acids in length. The term "epitope" includes T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (*e.g.,* fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

The peptide and polypeptide antigen can be 2 to 100 amino acids, including for example, 5 amino acids, 10 amino acids, 15 amino acids, 20 amino acids, 25 amino acids, 30 amino acids, 35 amino acids, 40 amino acids, 45 amino acids, or 50 amino acids in length. In some embodiments, a peptide can be greater than 50 amino acids. In some embodiments, the peptide can be greater than 100 amino acids.

The peptide or polypeptide antigen can be any peptide or polypeptide that can induce or increase the ability of the immune system to develop antibodies and T cell responses to the peptide or polypeptide.

In some embodiments, vaccine antigen, *i.e.,* an antigen whose inoculation into a subject induces an immune response, is recognized by an immune effector cell. In some embodiments, the vaccine antigen if recognized by an immune effector cell is able to induce in the presence of appropriate co-stimulatory signals, stimulation, priming and/or expansion of the immune effector cell carrying an antigen receptor recognizing the vaccine antigen. In the context of the embodiments of the present disclosure, the vaccine antigen may be, e.g., presented or present on the surface of a cell, such as an antigen presenting cell.

In some embodiments, an antigen is expressed in a diseased cell (such as tumor cell or an infected cell).

In some embodiments, an antigen is presented by a diseased cell (such as tumor cell or an infected cell). In some embodiments, an antigen receptor is a TCR which binds to an epitope of an antigen presented in the context of MHC. In some embodiments, binding of a TCR when expressed by T cells and/or present on T cells to an antigen presented by cells such as antigen presenting cells results in stimulation, priming and/or expansion of said T cells. In some embodiments, binding of a TCR when expressed by T cells and/or present on T cells to an antigen presented on diseased cells results in cytolysis and/or apoptosis of the diseased cells, wherein said T cells release cytotoxic factors, *e.g.,* perforins and granzymes.

In some embodiments, an antigen is expressed on the surface of a diseased cell (such as tumor cell or an infected cell). In some embodiments, an antigen receptor is a CAR which binds to an extracellular domain or to an epitope in an extracellular domain of an antigen. In some embodiments, a CAR binds to native epitopes of an antigen present on the surface of living cells. In some embodiments, binding of a CAR when expressed by T cells and/or present on T cells to an antigen present on cells such as antigen presenting cells results in stimulation, priming and/or expansion of said T cells. In some embodiments, binding of a CAR when expressed by T cells and/or present on T cells to an antigen present on diseased cells results in cytolysis and/or apoptosis of the diseased cells, wherein said T cells preferably release cytotoxic factors, e.g., perforins and granzymes.

According to some embodiments, an amino acid sequence enhancing antigen processing and/or presentation is fused, either directly or through a linker, to an antigenic peptide or polypeptide (antigenic sequence). Accordingly, in some embodiments, the RNA described herein comprises at least one coding region encoding an antigenic peptide or polypeptide and an amino acid sequence enhancing antigen processing and/or presentation.

In some embodiments, antigen for vaccination which may be administered in the form of RNA coding therefor comprises a naturally occurring antigen or a fragment such as an epitope thereof.

Such amino acid sequences enhancing antigen processing and/or presentation are preferably located at the C-terminus of the antigenic peptide or polypeptide (and optionally at the C-terminus of an amino acid sequence which breaks immunological tolerance), without being limited thereto. Amino acid sequences enhancing antigen processing and/or presentation as defined herein preferably improve antigen processing and presentation. In some embodiments, the amino acid sequence enhancing antigen processing and/or presentation as defined herein includes, without being limited thereto, sequences derived from the human MHC class I complex (HLA-B51, haplotype A2, B27/B51, Cw2/Cw3), in particular a sequence comprising the amino acid sequence of SEQ ID NO: 2 or a functional variant thereof.

In some embodiments, an amino acid sequence enhancing antigen processing and/or presentation comprises the amino acid sequence of SEQ ID NO: 2, an amino acid sequence having at least 99%, 98%, 97%, 96%, 95%, 90%, 85%, or 80% identity to the amino acid sequence of SEQ ID NO: 2, or a functional fragment of the amino acid sequence of SEQ ID NO: 2, or the amino acid sequence having at least 99%, 98%, 97%, 96%, 95%, 90%, 85%, or 80% identity to the amino acid sequence of SEQ ID NO: 2. In some embodiments, an amino acid sequence enhancing antigen processing and/or presentation comprises the amino acid sequence of SEQ ID NO: 2.

Accordingly, in some embodiments, the RNA described herein comprises at least one coding region encoding an antigenic peptide or polypeptide and an amino acid sequence enhancing antigen processing and/or presentation, said amino acid sequence enhancing antigen processing and/or presentation preferably being fused to the antigenic peptide or polypeptide, more preferably to the C-terminus of the antigenic peptide or polypeptide as described herein.

Furthermore, a secretory sequence, e.g., a sequence comprising the amino acid sequence of SEQ ID NO: 1, may be fused to the N-terminus of the antigenic peptide or polypeptide. Amino acid sequences derived from tetanus toxoid of *Clostridium tetani* may be employed to overcome self-tolerance mechanisms in order to efficiently mount an immune response to self-antigens by providing T-cell help during priming.

It is known that tetanus toxoid heavy chain includes epitopes that can bind promiscuously to MHC class II alleles and induce $CD4^+$ memory T cells in almost all tetanus vaccinated individuals. In addition, the combination of tetanus toxoid (TT) helper epitopes with tumor-associated antigens is known to improve the immune stimulation compared

to application of tumor-associated antigen alone by providing $CD4^+$-mediated T-cell help during priming. To reduce the risk of stimulating $CD8^+$ T cells with the tetanus sequences which might compete with the intended induction of tumor antigen-specificT-cell response, not the whole fragment C of tetanus toxoid is used as it is known to contain $CD8^+$ T-cell epitopes. Two peptide sequences containing promiscuously binding helper epitopes were selected alternatively to ensure binding to as many MHC class II alleles as possible. Based on the data of the *ex vivo* studies the well-known epitopes p2 (QYIKANSKFIGITEL; $TT_{830-844}$) and p16 (MTNSVDDALINSTKIYSYFPSVISKVNQGAQG; $TT_{578-609}$) were selected. The p2 epitope was already used for peptide vaccination in clinical trials to boost anti-melanoma activity. Non-clinical data showed that RNA vaccines encoding both a tumor antigen plus promiscuously binding tetanus toxoid sequences lead to enhanced $CD8^+$ T-cell responses directed against the tumor antigen and improved break of tolerance. Immunomonitoring data from patients vaccinated with vaccines including those sequences fused in frame with the tumor antigen-specific sequences reveal that the tetanus sequences chosen are able to induce tetanus-specific T-cell responses in almost all patients.

According to some embodiments, an amino acid sequence which breaks immunological tolerance is fused, either directly or through a linker, e.g., a linker having the amino acid sequence according to SEQ ID NO: 4, to the antigenic peptide or polypeptide.

Such amino acid sequences which break immunological tolerance are preferably located at the C-terminus of the antigenic peptide or polypeptide (and optionally at the N-terminus of the amino acid sequence enhancing antigen processing and/or presentation, wherein the amino acid sequence which breaks immunological tolerance and the amino acid sequence enhancing antigen processing and/or presentation may be fused either directly or through a linker, e.g., a linker having the amino acid sequence according to SEQ ID NO: 5), without being limited thereto. Amino acid sequences which break immunological tolerance as defined herein preferably improve T cell responses. In some embodiments, the amino acid sequence which breaks immunological tolerance as defined herein includes, without being limited thereto, sequences derived from tetanus toxoid-derived helper sequences p2 and p16 (P2P16), in particular a sequence comprising the amino acid sequence of SEQ ID NO: 3 or a functional variant thereof.

In some embodiments, an amino acid sequence which breaks immunological tolerance comprises the amino acid sequence of SEQ ID NO: 3, an amino acid sequence having at least 99%, 98%, 97%, 96%, 95%, 90%, 85%, or 80% identity to the amino acid sequence of SEQ ID NO: 3, or a functional fragment of the amino acid sequence of SEQ ID NO: 3, or the amino acid sequence having at least 99%, 98%, 97%, 96%, 95%, 90%, 85%, or 80% identity to the amino acid sequence of SEQ ID NO: 3. In some embodiments, an amino acid sequence which breaks immunological tolerance comprises the amino acid sequence of SEQ ID NO: 3.

In the following, embodiments of vaccine RNAs are described, wherein certain terms used when describing elements thereof have the following meanings:

**hAg-Kozak:** 5'-UTR sequence of the human alpha-globin mRNA with an optimized 'Kozak sequence' to increase translational efficiency.

**sec/MITD:** Fusion-protein tags derived from the sequence encoding the human MHC class I complex (HLA-B51, haplotype A2, B27/B51, Cw2/Cw3), which have been shown to improve antigen processing and presentation. Sec corresponds to the 78 bp fragment coding for the secretory signal peptide, which guides translocation of the nascent polypeptide chain into the endoplasmatic reticulum. MITD corresponds to the transmembrane and cytoplasmic domain of the MHC class I molecule, also called MHC class I trafficking domain.

**Antigen:** Sequences encoding the respective vaccine antigen/epitope.

**Glycine-serine linker (GS):** Sequences coding for short linker peptides predominantly consisting of the amino acids glycine (G) and serine (S), as commonly used for fusion proteins.

**P2P16:** Sequence coding for tetanus toxoid-derived helper epitopes to break immunological tolerance.

**FI element:** The 3'-UTR is a combination of two sequence elements derived from the "amino terminal enhancer of split" (AES) mRNA (called F) and the mitochondrial encoded 12S ribosomal RNA (called I). These were identified by an *ex vivo* selection process for sequences that confer RNA stability and augment total protein expression.

**A30L70:** A poly(A)-tail measuring 110 nucleotides in length, consisting of a stretch of 30 adenosine residues, followed by a 10 nucleotide linker sequence and another 70 adenosine residues designed to enhance RNA stability and translational efficiency in dendritic cells.

In some embodiments, vaccine RNA described herein has the structure: beta-S-ARCA(D1)-hAg-Kozak-sec-GS(1)-Antigen-GS(2)-P2P16-GS(3)-MITD-FI-A30L70

In some embodiments, vaccine antigen described herein has the structure: sec-GS(1)-Antigen-GS(2)-P2P16-GS(3)-MITD

In some embodiments, hAg-Kozak comprises the nucleotide sequence of SEQ ID NO: 6. In some embodiments, sec comprises the amino acid sequence of SEQ ID NO: 1. In some embodiments, P2P16 comprises the amino acid sequence of SEQ ID NO: 3. In some embodiments, MITD comprises the amino acid sequence of SEQ ID NO: 2. In

some embodiments, GS(1) comprises the amino acid sequence of SEQ ID NO: 4. In some embodiments, GS(2) comprises the amino acid sequence of SEQ ID NO: 4. In some embodiments, GS(3) comprises the amino acid sequence of SEQ ID NO: 5. In some embodiments, FI comprises the nucleotide sequence of SEQ ID NO: 7. In some embodiments, A30L70 comprises the nucleotide sequence of SEQ ID NO: 8.

In some embodiments, an antigen receptor is an antibody or B cell receptor which binds to an epitope in an antigen. In some embodiments, an antibody or B cell receptor binds to native epitopes of an antigen.

The term "expressed on the cell surface" or "associated with the cell surface" means that a molecule such as an antigen is associated with and located at the plasma membrane of a cell, wherein at least a part of the molecule faces the extracellular space of said cell and is accessible from the outside of said cell, *e.g.,* by antibodies located outside the cell. In this context, a part may be, e.g., at least 4, at least 8, at least 12, or at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules. An antigen is expressed on the surface of cells if it is located at the surface of said cells and is accessible to binding by, *e.g.,* antigen-specific antibodies added to the cells. In some embodiments, an antigen expressed on the surface of cells is an integral membrane protein having an extracellular portion which may be recognized by a CAR.

The term "extracellular portion" or "exodomain" in the context of the present disclosure refers to a part of a molecule such as a protein that is facing the extracellular space of a cell and preferably is accessible from the outside of said cell, *e.g.,* by binding molecules such as antibodies located outside the cell. In some embodiments, the term refers to one or more extracellular loops or domains or a fragment thereof.

The terms "T cell" and "T lymphocyte" are used interchangeably herein and include T helper cells (CD4+ T cells) and cytotoxic T cells (CTLs, CD8+ T cells) which comprise cytolytic T cells. The term "antigen-specific T cell" or similar terms relate to a T cell which recognizes the antigen to which the T cell is targeted, in particular when presented on the surface of antigen presenting cells or diseased cells such as cancer cells in the context of MHC molecules and preferably exerts effector functions of T cells. T cells are considered to be specific for antigen if the cells kill target cells expressing an antigen. T cell specificity may be evaluated using any of a variety of standard techniques, for example, within a chromium release assay or proliferation assay. Alternatively, synthesis of lymphokines (such as interferon-γ) can be measured.

The term "target" shall mean an agent such as a cell or tissue which is a target for an immune response such as a cellular immune response. Targets include cells that present an antigen or an antigen epitope, *i.e.,* a peptide fragment derived from an antigen. In some embodiments, the target cell is a cell expressing an antigen and presenting said antigen with class I MHC.

"Antigen processing" refers to the degradation of an antigen into processing products which are fragments of said antigen (*e.g.,* the degradation of a polypeptide into peptides) and the association of one or more of these fragments (*e.g.,* via binding) with MHC molecules for presentation by cells, such as antigen-presenting cells to specific T-cells. Antigen-presenting cells can be distinguished in professional antigen presenting cells and non-professional antigen presenting cells.

The term "professional antigen presenting cells" relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells and macrophages.

The term "non-professional antigen presenting cells" relates to antigen presenting cells which do not constitutively express MHC class II molecules, but upon stimulation by certain cytokines such as interferon-gamma. Exemplary, non-professional antigen presenting cells include fibroblasts, thymic epithelial cells, thyroid epithelial cells, glial cells, pancreatic beta cells or vascular endothelial cells.

The term "dendritic cell" (DC) refers to a subtype of phagocytic cells belonging to the class of antigen presenting cells. In some embodiments, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors

in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In some embodiments, the dendritic cells are splenic dendritic cells.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In some embodiments, the macrophages are splenic macrophages.

By "antigen-responsive CTL" is meant a $CD8^+$ T-cell that is responsive to an antigen or a peptide derived from said antigen, which is presented with class I MHC on the surface of antigen presenting cells.

According to the disclosure, CTL responsiveness may include sustained calcium flux, cell division, production of cytokines such as IFN-$\gamma$ and TNF-$\alpha$, up-regulation of activation markers such as CD44 and CD69, and specific cytolytic killing of tumor antigen expressing target cells. CTL responsiveness may also be determined using an artificial reporter that accurately indicates CTL responsiveness.

"Activation" or "stimulation", as used herein, refers to the state of a cell that has been sufficiently stimulated to induce detectable cellular proliferation, such as an immune effector cell such as T cell. Activation can also be associated with initiation of signaling pathways, induced cytokine production, and detectable effector functions. The term "activated immune effector cells" refers to, among other things, immune effector cells that are undergoing cell division.

The term "priming" refers to a process wherein an immune effector cell such as a T cell has its first contact with its specific antigen and causes differentiation into effector cells such as effector T cells.

The term "expansion" refers to a process wherein a specific entity is multiplied. In some embodiments, the term is used in the context of an immunological response in which immune effector cells are stimulated by an antigen, proliferate, and the specific immune effector cell recognizing said antigen is amplified. In some embodiments, expansion leads to differentiation of the immune effector cells.

The terms "immune response" and "immune reaction" are used herein interchangeably in their conventional meaning and refer to an integrated bodily response to an antigen and may refer to a cellular immune response, a humoral immune response, or both. According to the disclosure, the term "immune response to" or "immune response against" with respect to an agent such as an antigen, cell or tissue, relates to an immune response such as a cellular response directed against the agent. An immune response may comprise one or more reactions selected from the group consisting of developing antibodies against one or more antigens and expansion of antigen-specific T-lymphocytes, such as $CD4^+$ and $CD8^+$ T-lymphocytes, e.g. $CD8^+$ T-lymphocytes, which may be detected in various proliferation or cytokine production tests *in vitro.*

The terms "inducing an immune response" and "eliciting an immune response" and similar terms in the context of the present disclosure refer to the induction of an immune response, such as the induction of a cellular immune response, a humoral immune response, or both. The immune response may be protective/preventive/prophylactic and/or therapeutic. The immune response may be directed against any immunogen or antigen or antigen peptide, such as against a tumor-associated antigen or a pathogen-associated antigen (*e.g.,* an antigen of a virus (such as influenza virus (A, B, or C), CMV or RSV)). "Inducing" in this context may mean that there was no immune response against a particular antigen or pathogen before induction, but it may also mean that there was a certain level of immune response against a particular antigen or pathogen before induction and after induction said immune response is enhanced. Thus, "inducing the immune response" in this context also includes "enhancing the immune response". In some embodiments, after inducing an immune response in an individual, said individual is protected from developing a disease such as an infectious disease or a cancerous disease or the disease condition is ameliorated by inducing an immune response.

The terms "cellular immune response", "cellular response", "cell-mediated immunity" or similar terms are meant to include a cellular response directed to cells characterized by expression of an antigen and/or presentation of an antigen with class I or class II MHC. The cellular response relates to cells called T cells or T lymphocytes which act as either "helpers" or "killers". The helper T cells (also termed $CD4^+$ T cells) play a central role by regulating the immune response and the killer cells (also termed cytotoxic T cells, cytolytic T cells, $CD8^+$ T cells or CTLs) kill cells such as diseased cells.

The term "humoral immune response" refers to a process in living organisms wherein antibodies are produced in response to agents and organisms, which they ultimately neutralize and/or eliminate. The specificity of the antibody response is mediated by T and/or B cells through membrane-associated receptors that bind antigen of a single specificity. Following binding of an appropriate antigen and receipt of various other activating signals, B lymphocytes divide, which

produces memory B cells as well as antibody secreting plasma cell clones, each producing antibodies that recognize the identical antigenic epitope as was recognized by its antigen receptor. Memory B lymphocytes remain dormant until they are subsequently activated by their specific antigen. These lymphocytes provide the cellular basis of memory and the resulting escalation in antibody response when re-exposed to a specific antigen.

The term "antibody" as used herein, refers to an immunoglobulin molecule, which is able to specifically bind to an epitope on an antigen. In particular, the term "antibody" refers to a glycoprotein comprising at least two heavy (H) chains and two light (L) chains inter-connected by disulfide bonds. The term "antibody" includes monoclonal antibodies, recombinant antibodies, human antibodies, humanized antibodies, chimeric antibodies and combinations of any of the foregoing. Each heavy chain is comprised of a heavy chain variable region (VH) and a heavy chain constant region (CH). Each light chain is comprised of a light chain variable region (VL) and a light chain constant region (CL). The variable regions and constant regions are also referred to herein as variable domains and constant domains, respectively. The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The CDRs of a VH are termed HCDR1, HCDR2 and HCDR3, the CDRs of a VL are termed LCDR1, LCDR2 and LCDR3. The variable regions of the heavy and light chains contain a binding domain that interacts with an antigen. The constant regions of an antibody comprise the heavy chain constant region (CH) and the light chain constant region (CL), wherein CH can be further subdivided into constant domain CH1, a hinge region, and constant domains CH2 and CH3 (arranged from amino-terminus to carboxy-terminus in the following order: CH1, CH2, CH3). The constant regions of the antibodies may mediate the binding of the immunoglobulin to host tissues or factors, including various cells of the immune system (*e.g.,* effector cells) and the first component (C1q) of the classical complement system.

Antibodies can be intact immunoglobulins derived from natural sources or from recombinant sources and can be immunoactive portions of intact immunoglobulins. Antibodies are typically tetramers of immunoglobulin molecules. Antibodies may exist in a variety of forms including, for example, polyclonal antibodies, monoclonal antibodies, Fv, Fab and $F(ab)_2$, as well as single chain antibodies and humanized antibodies.

The term "immunoglobulin" relates to proteins of the immunoglobulin superfamily, such as to antigen receptors such as antibodies or the B cell receptor (BCR). The immunoglobulins are characterized by a structural domain, *i.e.,* the immunoglobulin domain, having a characteristic immunoglobulin (Ig) fold. The term encompasses membrane bound immunoglobulins as well as soluble immunoglobulins. Membrane bound immunoglobulins are also termed surface immunoglobulins or membrane immunoglobulins, which are generally part of the BCR. Soluble immunoglobulins are generally termed antibodies. Immunoglobulins generally comprise several chains, typically two identical heavy chains and two identical light chains which are linked via disulfide bonds. These chains are primarily composed of immunoglobulin domains, such as the $V_L$ (variable light chain) domain, $C_L$ (constant light chain) domain, $V_H$ (variable heavy chain) domain, and the $C_H$ (constant heavy chain) domains $C_H1$, $C_H2$, $C_H3$, and $C_H4$. There are five types of mammalian immunoglobulin heavy chains, *i.e.,* $\alpha$, $\delta$, $\varepsilon$, $\gamma$, and $\mu$ which account for the different classes of antibodies, *i.e.,* IgA, IgD, IgE, IgG, and IgM. As opposed to the heavy chains of soluble immunoglobulins, the heavy chains of membrane or surface immunoglobulins comprise a transmembrane domain and a short cytoplasmic domain at their carboxy-terminus. In mammals there are two types of light chains, *i.e.,* lambda and kappa. The immunoglobulin chains comprise a variable region and a constant region. The constant region is essentially conserved within the different isotypes of the immunoglobulins, wherein the variable part is highly divers and accounts for antigen recognition.

The terms "vaccination" and "immunization" describe the process of treating an individual for therapeutic or prophylactic reasons and relate to the procedure of administering one or more immunogen(s) or antigen(s) or derivatives thereof, in particular in the form of RNA (especially mRNA) coding therefor, as described herein to an individual and stimulating an immune response against said one or more immunogen(s) or antigen(s) or cells characterized by presentation of said one or more immunogen(s) or antigen(s).

By "cell characterized by presentation of an antigen" or "cell presenting an antigen" or "MHC molecules which present an antigen on the surface of an antigen presenting cell" or similar expressions is meant a cell such as a diseased cell, in particular a tumor cell or an infected cell, or an antigen presenting cell presenting the antigen or an antigen peptide, either directly or following processing, in the context of MHC molecules, such as MHC class I and/or MHC class II molecules. In some embodiments, the MHC molecules are MHC class I molecules.

The term "allergen" refers to a kind of antigen which originates from outside the body of a subject (*i.e.,* the allergen can also be called "heterologous antigen") and which produces an abnormally vigorous immune response in which the immune system of the subject fights off a perceived threat that would otherwise be harmless to the subject. "Allergies" are the diseases caused by such vigorous immune reactions against allergens. An allergen usually is an antigen which is able to stimulate a type-I hypersensitivity reaction in atopic individuals through immunoglobulin E (IgE) responses. Particular examples of allergens include allergens derived from peanut proteins (*e.g.,* Ara h 2.02), ovalbumin, grass pollen proteins (*e.g.,* Phl p 5), and proteins of dust mites (*e.g.,* Der p 2).

The term "growth factors" refers to molecules which are able to stimulate cellular growth, proliferation, healing, and/or cellular differentiation. Typically, growth factors act as signaling molecules between cells. The term "growth factors" include particular cytokines and hormones which bind to specific receptors on the surface of their target cells. Examples of growth factors include bone morphogenetic proteins (BMPs), fibroblast growth factors (FGFs), vascular endothelial growth factors (VEGFs), such as VEGFA, epidermal growth factor (EGF), insulin-like growth factor, ephrins, macrophage colony-stimulating factor, granulocyte colony-stimulating factor, granulocyte macrophage colony-stimulating factor, neuregulins, neurotrophins (*e.g.,* brain-derived neurotrophic factor (BDNF), nerve growth factor (NGF)), placental growth factor (PGF), platelet-derived growth factor (PDGF), renalase (RNLS) (antiapoptotic survival factor), T-cell growth factor (TCGF), thrombopoietin (TPO), transforming growth factors (transforming growth factor alpha (TGF-$\alpha$), transforming growth factor beta (TGF-$\beta$)), and tumor necrosis factor-alpha (TNF-$\alpha$). In some embodiments, a "growth factor" is a peptide or polypeptide growth factor.

The term "protease inhibitors" refers to molecules, in particular peptides or polypeptides, which inhibit the function of proteases. Protease inhibitors can be classified by the protease which is inhibited (*e.g.,* aspartic protease inhibitors) or by their mechanism of action (*e.g.,* suicide inhibitors, such as serpins). Particular examples of protease inhibitors include serpins, such as alpha 1-antitrypsin, aprotinin, and bestatin.

The term "enzymes" refers to macromolecular biological catalysts which accelerate chemical reactions. Like any catalyst, enzymes are not consumed in the reaction they catalyze and do not alter the equilibrium of said reaction. Unlike many other catalysts, enzymes are much more specific. In some embodiments, an enzyme is essential for homeostasis of a subject, *e.g.,* any malfunction (in particular, decreased activity which may be caused by any of mutation, deletion or decreased production) of the enzyme results in a disease. Examples of enzymes include herpes simplex virus type 1 thymidine kinase (HSV1-TK), hexosaminidase, phenylalanine hydroxylase, pseudocholinesterase, and lactase.

The term "receptors" refers to protein molecules which receive signals (in particular chemical signals called ligands) from outside a cell. The binding of a signal (*e.g.,* ligand) to a receptor causes some kind of response of the cell, *e.g.*, the intracellular activation of a kinase. Receptors include transmembrane receptors (such as ion channel-linked (ionotropic) receptors, G protein-linked (metabotropic) receptors, and enzyme-linked receptors) and intracellular receptors (such as cytoplasmic receptors and nuclear receptors). Particular examples of receptors include steroid hormone receptors, growth factor receptors, and peptide receptors (*i.e.,* receptors whose ligands are peptides), such as P-selectin glycoprotein ligand-1 (PSGL-1). The term "growth factor receptors" refers to receptors which bind to growth factors.

The term "apoptosis regulators" refers to molecules, in particular peptides or polypeptides, which modulate apoptosis, *i.e.,* which either activate or inhibit apoptosis. Apoptosis regulators can be grouped into two broad classes: those which modulate mitochondrial function and those which regulate caspases. The first class includes proteins (*e.g.,* BCL-2, BCL-xL) which act to preserve mitochondrial integrity by preventing loss of mitochondrial membrane potential and/or release of pro-apoptotic proteins such as cytochrome C into the cytosol. Also to this first class belong proapoptotic proteins (*e.g.,* BAX, BAK, BIM) which promote release of cytochrome C. The second class includes proteins such as the inhibitors of apoptosis proteins (*e.g.*, XIAP) or FLIP which block the activation of caspases.

The term "transcription factors" relates to proteins which regulate the rate of transcription of genetic information from DNA to messenger RNA, in particular by binding to a specific DNA sequence. Transcription factors may regulate cell division, cell growth, and cell death throughout life; cell migration and organization during embryonic development; and/or in response to signals from outside the cell, such as a hormone. Transcription factors contain at least one DNA-binding domain which binds to a specific DNA sequence, usually adjacent to the genes which are regulated by the transcription factors. Particular examples of transcription factors include MECP2, FOXP2, FOXP3, the STAT protein family, and the HOX protein family.

The term "tumor suppressor proteins" relates to molecules, in particular peptides or polypeptides, which protect a cell from one step on the path to cancer. Tumor-suppressor proteins (usually encoded by corresponding tumor-suppressor genes) exhibit a weakening or repressive effect on the regulation of the cell cycle and/or promote apoptosis. Their functions may be one or more of the following: repression of genes essential for the continuing of the cell cycle; coupling the cell cycle to DNA damage (as long as damaged DNA is present in a cell, no cell division should take place); initiation of apoptosis, if the damaged DNA cannot be repaired; metastasis suppression (*e.g.,* preventing tumor cells from dispersing, blocking loss of contact inhibition, and inhibiting metastasis); and DNA repair. Particular examples of tumor-suppressor proteins include p53, phosphatase and tensin homolog (PTEN), SWI/SNF (SWItch/Sucrose Non-Fermentable), von Hippel-Lindau tumor suppressor (pVHL), adenomatous polyposis coli (APC), CD95, suppression of tumorigenicity 5 (ST5), suppression of tumorigenicity 5 (ST5), suppression of tumorigenicity 14 (ST14), and Yippee-like 3 (YPEL3). The term "structural proteins" refers to proteins which confer stiffness and rigidity to otherwise-fluid biological components. Structural proteins are mostly fibrous (such as collagen and elastin) but may also be globular (such as actin and tubulin). Usually, globular proteins are soluble as monomers, but polymerize to form long, fibers which, for example, may make up the cytoskeleton. Other structural proteins are motor proteins (such as myosin, kinesin, and dynein) which are capable of generating mechanical forces, and surfactant proteins. Particular examples of structural

proteins include collagen, surfactant protein A, surfactant protein B, surfactant protein C, surfactant protein D, elastin, tubulin, actin, and myosin.

The term "reprogramming factors" or "reprogramming transcription factors" relates to molecules, in particular peptides or polypeptides, which, when expressed in somatic cells optionally together with further agents such as further reprogramming factors, lead to reprogramming or de-differentiation of said somatic cells to cells having stem cell characteristics, in particular pluripotency. Particular examples of reprogramming factors include OCT4, SOX2, c-MYC, KLF4, LIN28, and NANOG.

The term "genomic engineering proteins" relates to proteins which are able to insert, delete or replace DNA in the genome of a subject. Particular examples of genomic engineering proteins include meganucleases, zinc finger nucleases (ZFNs), transcription activator-like effector nucleases (TALENs), and clustered regularly spaced short palindromic repeat-CRISPR-associated protein 9 (CR!SPR-Cas9).

The term "blood proteins" relates to peptides or polypeptides which are present in blood plasma of a subject, in particular blood plasma of a healthy subject. Blood proteins have diverse functions such as transport (*e.g.,* albumin, transferrin), enzymatic activity (*e.g.,* thrombin or ceruloplasmin), blood clotting (*e.g.,* fibrinogen), defense against pathogens (*e.g.,* complement components and immunoglobulins), protease inhibitors (*e.g.*, alpha 1-antitrypsin), etc. Particular examples of blood proteins include thrombin, serum albumin, Factor VII, Factor VIII, insulin, Factor IX, Factor X, tissue plasminogen activator, protein C, von Willebrand factor, antithrombin III, glucocerebrosidase, erythropoietin, granulocyte colony stimulating factor (G-CSF), modified Factor VIII, and anticoagulants.

Thus, in some embodiments, the pharmaceutically active peptide or polypeptide is (i) a cytokine, preferably selected from the group consisting of erythropoietin (EPO), interleukin 4 (IL-2), and interleukin 10 (IL-11), more preferably EPO; (ii) an adhesion molecule, in particular an integrin; (iii) an immunoglobulin, in particular an antibody; (iv) an immunologically active compound, in particular an antigen; (v) a hormone, in particular vasopressin, insulin or growth hormone; (vi) a growth factor, in particular VEGFA; (vii) a protease inhibitor, in particular alpha 1-antitrypsin; (viii) an enzyme, preferably selected from the group consisting of herpes simplex virus type 1 thymidine kinase (HSV1-TK), hexosaminidase, phenylalanine hydroxylase, pseudocholinesterase, pancreatic enzymes, and lactase; (ix) a receptor, in particular growth factor receptors; (x) an apoptosis regulator, in particular BAX; (xi) a transcription factor, in particular FOXP3; (xii) a tumor suppressor protein, in particular p53; (xiii) a structural protein, in particular surfactant protein B; (xiv) a reprogramming factor, *e.g.,* selected from the group consisting of OCT4, SOX2, c-MYC, KLF4, LIN28 and NANOG; (xv) a genomic engineering protein, in particular clustered regularly spaced short palindromic repeat-CRISPR-associated protein 9 (CR!SPR-Cas9); and (xvi) a blood protein, in particular fibrinogen.

In some embodiments, a pharmaceutically active peptide or polypeptide comprises one or more antigens or one or more epitopes, *i.e.,* administration of the peptide or polypeptide to a subject elicits an immune response against the one or more antigens or one or more epitopes in a subject which may be therapeutic or partially or fully protective.

In some embodiments, the RNA encodes at least one epitope, e.g., at least two epitopes, at least three epitopes, at least four epitopes, at least five epitopes, at least six epitopes, at least seven epitopes, at least eight epitopes, at least nine epitopes, or at least ten epitopes.

In some embodiments, the target antigen is a tumor antigen and the antigenic sequence (e.g., an epitope) is derived from the tumor antigen. The tumor antigen may be a "standard" antigen, which is generally known to be expressed in various cancers. The tumor antigen may also be a "neo-antigen", which is specific to an individual's tumor and has not been previously recognized by the immune system. A neo-antigen or neo-epitope may result from one or more cancer-specific mutations in the genome of cancer cells resulting in amino acid changes. If the tumor antigen is a neo-antigen, the vaccine antigen preferably comprises an epitope or a fragment of said neo-antigen comprising one or more amino acid changes.

Examples of tumor antigens include, without limitation, p53, ART-4, BAGE, beta-catenin/m, Bcr-abL CAMEL, CAP-1 , CASP-8, CDC27/m, CDK4/m, CEA, the cell surface proteins of the claudin family, such as CLAUD ΓN-6, CLAUDIN-18.2 and CLAUDIN-12, c-MYC, CT, Cyp-B, DAM, ELF2M, ETV6-AML1, G250, GAGE, GnT-V, Gap 100, HAGE, HER-2/neu, HPV-E7, HPV-E6, HAST-2, hTERT (or hTRT), LAGE, LDLR/FUT, MAGE-A, preferably MAGE-A1 , MAGE-A2, MAGE- A3, MAGE-A4, MAGE-A5, MAGE-A6, MAGE-A7, MAGE-A8, MAGE-A9, MAGE-A 10, MAGE-A 11, or MAGE- A12, MAGE-B, MAGE-C, MART- 1/Melan-A, MC1R, Myosin/m, MUC1, MUM-1, MUM-2, MUM-3, NA88-A, NF1 , NY-ESO-1 , NY-BR-1 , pl90 minor BCR-abL, Pml/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1, TRP-2, TRP-2/INT2, TPTE, WT, and WT-1. Cancer mutations vary with each individual. Thus, cancer mutations that encode novel epitopes (neo-epitopes) represent attractive targets in the development of vaccine compositions and immunotherapies. The efficacy of tumor immunotherapy relies on the selection of cancer-specific antigens and epitopes capable of inducing a potent immune response within a host. RNA can be used to deliver patient-specific tumor epitopes to a patient. Dendritic cells (DCs) residing in the spleen represent antigen-presenting cells of particular interest for RNA expression of immunogenic epitopes or antigens such as tumor epitopes. The use of multiple epitopes has been shown to promote therapeutic efficacy in tumor vaccine compositions. Rapid sequencing of the tumor mutanome may provide multiple epitopes for

individualized vaccines which can be encoded by mRNA described herein, *e.g.,* as a single polypeptide wherein the epitopes are optionally separated by linkers. In some embodiments of the present disclosure, the mRNA encodes at least one epitope, at least two epitopes, at least three epitopes, at least four epitopes, at least five epitopes, at least six epitopes, at least seven epitopes, at least eight epitopes, at least nine epitopes, or at least ten epitopes. Exemplary embodiments include mRNA that encodes at least five epitopes (termed a "pentatope") and mRNA that encodes at least ten epitopes (termed a "decatope").

In some embodiments, the antigen or epitope is derived from a pathogen-associated antigen, in particular from a viral antigen.

In some embodiments, the antigen or epitope is derived from a coronavirus protein, an immunogenic variant thereof, or an immunogenic fragment of the coronavirus protein or the immunogenic variant thereof. Thus, in some embodiments, the mRNA used in the present disclosure encodes an amino acid sequence comprising a coronavirus protein, an immunogenic variant thereof, or an immunogenic fragment of the coronavirus protein or the immunogenic variant thereof.

In some embodiments, the antigen or epitope is derived from a coronavirus S protein, an immunogenic variant thereof, or an immunogenic fragment of the coronavirus S protein or the immunogenic variant thereof. Thus, in some embodiments, the mRNA used in the present disclosure encodes an amino acid sequence comprising a coronavirus S protein, an immunogenic variant thereof, or an immunogenic fragment of the coronavirus S protein or the immunogenic variant thereof. In some embodiments, the coronavirus is MERS-CoV. In some embodiments, the coronavirus is SARS-CoV. In some embodiments, the coronavirus is SARS-CoV-2.

Coronaviruses are enveloped, positive-sense, single-stranded RNA ((+) ssRNA) viruses. They have the largest genomes (26-32 kb) among known RNA viruses and are phylogenetically divided into four genera ($\alpha$, $\beta$, $\gamma$, and $\delta$), with betacoronaviruses further subdivided into four lineages (A, B, C, and D). Coronaviruses infect a wide range of avian and mammalian species, including humans. Some human coronaviruses generally cause mild respiratory diseases, although severity can be greater in infants, the elderly, and the immunocompromised. Middle East respiratory syndrome coronavirus (MERS-CoV) and severe acute respiratory syndrome coronavirus (SARS-CoV), belonging to betacoronavirus lineages C and B, respectively, are highly pathogenic. Both viruses emerged into the human population from animal reservoirs within the last 15 years and caused outbreaks with high case-fatality rates. The outbreak of severe acute respiratory syndrome coronavirus-2 (SARS-CoV-2) that causes atypical pneumonia (coronavirus disease 2019; COVID-19) has raged in China since mid-December 2019, and has developed to be a public health emergency of international concern. SARS-CoV-2 (MN908947.3) belongs to betacoronavirus lineage B. It has at least 70% sequence similarity to SARS-CoV.

In general, coronaviruses have four structural proteins, namely, envelope (E), membrane (M), nucleocapsid (N), and spike (S). The E and M proteins have important functions in the viral assembly, and the N protein is necessary for viral RNA synthesis. The critical glycoprotein S is responsible for virus binding and entry into target cells. The S protein is synthesized as a single-chain inactive precursor that is cleaved by furin-like host proteases in the producing cell into two noncovalently associated subunits, S1 and S2. The S1 subunit contains the receptor-binding domain (RBD), which recognizes the host-cell receptor. The S2 subunit contains the fusion peptide, two heptad repeats, and a transmembrane domain, all of which are required to mediate fusion of the viral and host-cell membranes by undergoing a large conformational rearrangement. The S1 and S2 subunits trimerize to form a large prefusion spike.

The S precursor protein of SARS-CoV-2 can be proteolytically cleaved into S1 (685 aa) and S2 (588 aa) subunits. The S1 subunit comprises the receptor-binding domain (RBD), which mediates virus entry into sensitive cells through the host angiotensin-converting enzyme 2 (ACE2) receptor.

In some embodiments, the antigen or epitope is derived from a SARS-CoV-2 S protein, an immunogenic variant thereof, or an immunogenic fragment of the SARS-CoV-2 S protein or the immunogenic variant thereofIn some embodiments, the mRNA used in the present disclosure encodes an amino acid sequence comprising a SARS-CoV-2 S protein, an immunogenic variant thereof, or an immunogenic fragment of the SARS-CoV-2 S protein or the immunogenic variant thereof. Thus, in some embodiments, the encoded amino acid sequence comprises an epitope of SARS-CoV-2 S protein or an immunogenic variant thereof for inducing an immune response against coronavirus S protein, in particular SARS-CoV-2 S protein in a subject. In some embodiments, RNA is administered to provide (following expression by appropriate target cells) antigen for induction of an immune response, e.g., antibodies and/or immune effector cells, which is targeted to target antigen (coronavirus S protein, in particular SARS-CoV-2 S protein) or a procession product thereof. In some embodiments, the immune response which is to be induced according to the present disclosure is a B cell-mediated immune response, i.e., an antibody-mediated immune response. Additionally or alternatively, in some embodiments, the immune response which is to be induced according to the present disclosure is a T cell-mediated immune response. In some embodiments, the immune response is an anti-coronavirus, in particular anti-SARS-CoV-2 immune response.

SARS-CoV-2 coronavirus full length spike (S) protein consist of 1273 amino acids and has the following amino acid sequence:

MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ

SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT

(SEQ ID NO: 11)

For purposes of the present disclosure, the above sequence is considered the wildtype SARS-CoV-2 S protein amino acid sequence. Position numberings in SARS-CoV-2 S protein given herein are in relation to the amino acid sequence according to SEQ ID NO: 11 and corresponding positions in SARS-CoV-2 S protein variants.

In specific embodiments, full length spike (S) protein according to SEQ ID NO: 11 is modified in such a way that the prototypical prefusion conformation is stabilized. Certain mutations that stabilize a prefusion confirmation are known in the art, e.g., as disclosed in WO 2021243122 A2 and Hsieh, Ching-Lin, et al. ("Structure-based design of prefusion-stabilized SARS-CoV-2 20 spikes," Science 369.6510 (2020): 1501-1505), the contents of each which are incorporated by reference herein in their entirety. In some embodiments, a SARS-CoV-2 S protein may be stabilized by introducing one or more proline mutations. In some embodiments, a SARS-CoV-2 S protein comprises a proline substitution at residues 986 and/or 987 of SEQ ID NO: 1. In some embodiments, a SARS-CoV-2 S protein comprises a proline substitution at one or more of residues 817, 892, 899, and 942 of SEQ ID NO: 1. In some embodiments, a SARS-CoV-2 S protein comprises a proline substitution at each of residues 817, 892, 899, and 942 of SEQ ID NO: 1. In some embodiments, a SARS-CoV-2 S protein comprises a proline substitution at each of residues 817, 892, 899, 942, 986, and 987 of SEQ ID NO: 1. Stabilization of the prefusion conformation may be obtained by introducing two consecutive proline substitutions at AS residues 986 and 987 in the full-length spike protein. Specifically, spike (S) protein stabilized protein variants are obtained in a way that the amino acid residue at position 986 is exchanged to proline and the amino acid residue at position 987 is also exchanged to proline. In one embodiment, a SARS-CoV-2 S protein variant wherein the prototypical prefusion conformation is stabilized comprises the following amino acid sequence:

MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDPPEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT

(SEQ ID NO: 12)

In some embodiments, SARS-CoV-2 spike variants described herein comprise a mutation in a furin cleavage site (e.g., in some embodiments residues 682-685 of SEQ ID NO: 11). In some embodiments, a SARS-CoV-2 spike variant comprises a mutation in the furin cleavage site that prevents cleavage by a furin protease (e.g., a human furin protease). In some embodiments, a SARS-CoV-2 variant described herein comprises a furin mutation disclosed in WO2021163365 or WO2021243122 (e.g., a GSAS mutation), the contents of both of which are incorporated by reference herein in their entirety.

The SARs-CoV-2 spike variants described herein may or may not include a D614G mutation as compared to SEQ ID NO: 11.

Those skilled in the art are aware of various spike variants, and/or resources that document them.

In some embodiments, the encoded amino acid sequence comprises, consists essentially of or consists of a spike (S) protein of SARS-CoV-2, a variant thereof, or a fragment thereof.

In some embodiments, the encoded amino acid sequence comprises the amino acid sequence of amino acids 17 to 1273 of SEQ ID NO: 11 or 12, an amino acid sequence having at least 99%, 98%, 97%, 96%, 95%, 90%, 85%, or 80% identity to the amino acid sequence of amino acids 17 to 1273 of SEQ ID NO: 11 or 12, or an immunogenic fragment of the amino acid sequence of amino acids 17 to 1273 of SEQ ID NO: 11 or 12, or the amino acid sequence having at least 99%, 98%, 97%, 96%, 95%, 90%, 85%, or 80% identity to the amino acid sequence of amino acids 17 to 1273 of SEQ ID NO: 11 or 12. In some embodiments, the encoded amino acid sequence comprises the amino acid sequence of amino acids 17 to 1273 of SEQ ID NO: 11 or 12.

In some embodiments, the encoded amino acid sequence comprises, consists essentially of or consists of

SARS-CoV-2 spike S1 fragment (S1) (the S1 subunit of a spike protein (S) of SARS-CoV-2), a variant thereof, or a fragment thereof.

In some embodiments, the encoded amino acid sequence comprises the amino acid sequence of amino acids 17 to 683 of SEQ ID NO: 11, an amino acid sequence having at least 99%, 98%, 97%, 96%, 95%, 90%, 85%, or 80% identity to the amino acid sequence of amino acids 17 to 683 of SEQ ID NO: 11, or an immunogenic fragment of the amino acid sequence of amino acids 17 to 683 of SEQ ID NO: 11, or the amino acid sequence having at least 99%, 98%, 97%, 96%, 95%, 90%, 85%, or 80% identity to the amino acid sequence of amino acids 17 to 683 of SEQ ID NO: 11. In some embodiments, the encoded amino acid sequence comprises the amino acid sequence of amino acids 17 to 683 of SEQ ID NO: 11.

In some embodiments, the encoded amino acid sequence comprises the amino acid sequence of amino acids 17 to 685 of SEQ ID NO: 11, an amino acid sequence having at least 99%, 98%, 97%, 96%, 95%, 90%, 85%, or 80% identity to the amino acid sequence of amino acids 17 to 685 of SEQ ID NO: 11, or an immunogenic fragment of the amino acid sequence of amino acids 17 to 685 of SEQ ID NO: 11, or the amino acid sequence having at least 99%, 98%, 97%, 96%, 95%, 90%, 85%, or 80% identity to the amino acid sequence of amino acids 17 to 685 of SEQ ID NO: 11. In some embodiments, the encoded amino acid sequence comprises the amino acid sequence of amino acids 17 to 685 of SEQ ID NO: 11.

In some embodiments, the encoded amino acid sequence comprises, consists essentially of or consists of the receptor binding domain (RBD) of the S1 subunit of a spike protein (S) of SARS-CoV-2, a variant thereof, or a fragment thereof. The amino acid sequence of amino acids 327 to 528 of SEQ ID NO: 11, a variant thereof, or a fragment thereof is also referred to herein as "RBD" or "RBD domain".

In some embodiments, the encoded polypeptide comprises a sequence that corresponds to the RBD, and further comprises a trimerization domain (e.g., a trimerization domain as disclosed herein, such as a fibritin domain). In some embodiments an RBD comprises a signaling domain (e.g., a signaling domain as disclosed herein). In some embodiments an RBD comprises a transmembrane domain (e.g., a transmembrane domain as disclosed herein). In some embodiments, an RBD comprises a signaling domain and a trimerization domain. In some embodiments, an RBD comprises a signaling domain, a trimerization domain, and transmembrane domain.

In some embodiments, the encoded polypeptide comprises a sequence that corresponds to two receptor binding domains. In some embodiments, the encoded polypeptide comprises a sequence that corresponds to two receptor binding domains in tandem in an amino acid chain, e.g., as disclosed in Dai, Lianpan, et al. "A universal design of betacoronavirus vaccines against COVID-19, MERS, and SARS," Cell 182.3 (2020): 722-733, the contents of which are incorporated by reference herein in their entirety.

In some embodiments, the encoded amino acid sequence comprises the amino acid sequence of amino acids 327 to 528 of SEQ ID NO: 11, an amino acid sequence having at least 99%, 98%, 97%, 96%, 95%, 90%, 85%, or 80% identity to the amino acid sequence of amino acids 327 to 528 of SEQ ID NO: 11, or an immunogenic fragment of the amino acid sequence of amino acids 327 to 528 of SEQ ID NO: 11, or the amino acid sequence having at least 99%, 98%, 97%, 96%, 95%, 90%, 85%, or 80% identity to the amino acid sequence of amino acids 327 to 528 of SEQ ID NO: 11. In some embodiments, the encoded amino acid sequence comprises the amino acid sequence of amino acids 327 to 528 of SEQ ID NO: 11.

According to certain embodiments, a signal peptide is fused, either directly or through a linker, to a SARS-CoV-2 S protein, a variant thereof, or a fragment thereof, i.e., the antigenic peptide or protein. Accordingly, in some embodiments, a signal peptide is fused to the above described amino acid sequences derived from SARS-CoV-2 S protein or immunogenic fragments thereof (antigenic peptides or proteins) comprised by the encoded amino acid sequences described above.

In some embodiments, the encoded amino acid sequence comprises the amino acid sequence of SEQ ID NO: 11 or 12, an amino acid sequence having at least 99%, 98%, 97%, 96%, 95%, 90%, 85%, or 80% identity to the amino acid sequence of SEQ ID NO: 11 or 12, or an immunogenic fragment of the amino acid sequence of SEQ ID NO: 11 or 12, or the amino acid sequence having at least 99%, 98%, 97%, 96%, 95%, 90%, 85%, or 80% identity to the amino acid sequence of SEQ ID NO: 11 or 12. In some embodiments, the encoded amino acid sequence comprises the amino acid sequence of SEQ ID NO: 11 or 12.

In some embodiments, the encoded amino acid sequence comprises the amino acid sequence of amino acids 1 to 683 of SEQ ID NO: 11, an amino acid sequence having at least 99%, 98%, 97%, 96%, 95%, 90%, 85%, or 80% identity to the amino acid sequence of amino acids 1 to 683 of SEQ ID NO: 11, or an immunogenic fragment of the amino acid sequence of amino acids 1 to 683 of SEQ ID NO: 11, or the amino acid sequence having at least 99%, 98%, 97%, 96%, 95%, 90%, 85%, or 80% identity to the amino acid sequence of amino acids 1 to 683 of SEQ ID NO: 11. In some embodiments, the encoded amino acid sequence comprises the amino acid sequence of amino acids 1 to 683 of SEQ ID NO: 11.

In some embodiments, the encoded amino acid sequence comprises the amino acid sequence of amino acids 1 to 685 of SEQ ID NO: 11, an amino acid sequence having at least 99%, 98%, 97%, 96%, 95%, 90%, 85%, or 80%

identity to the amino acid sequence of amino acids 1 to 685 of SEQ ID NO: 11, or an immunogenic fragment of the amino acid sequence of amino acids 1 to 685 of SEQ ID NO: 11, or the amino acid sequence having at least 99%, 98%, 97%, 96%, 95%, 90%, 85%, or 80% identity to the amino acid sequence of amino acids 1 to 685 of SEQ ID NO: 11. In some embodiments, the encoded amino acid sequence comprises the amino acid sequence of amino acids 1 to 685 of SEQ ID NO: 11.

According to certain embodiments, a trimerization domain is fused, either directly or through a linker, e.g., a glycine/serine linker, to a SARS-CoV-2 S protein, a variant thereof, or a fragment thereof, i.e., the antigenic peptide or protein. Accordingly, in some embodiments, a trimerization domain is fused to the above described amino acid sequences derived from SARS-CoV-2 S protein or immunogenic fragments thereof (antigenic peptides or proteins) comprised by the encoded amino acid sequences described above (which may optionally be fused to a signal peptide as described above).

Such trimerization domains are preferably located at the C-terminus of the antigenic peptide or protein, without being limited thereto. Trimerization domains as defined herein preferably allow the trimerization of the antigenic peptide or protein as encoded by the RNA. Examples of trimerization domains as defined herein include, without being limited thereto, foldon, the natural trimerization domain of T4 fibritin. The C-terminal domain of T4 fibritin (foldon) is obligatory for the formation of the fibritin trimer structure and can be used as an artificial trimerization domain. In some embodiments, the trimerization domain as defined herein includes, without being limited thereto, a sequence comprising the amino acid sequence of SEQ ID NO: 13 or a functional variant thereof.

In some embodiments, a trimerization domain comprises the amino acid sequence of amino acids 3 to 29 of SEQ ID NO: 13, an amino acid sequence having at least 99%, 98%, 97%, 96%, 95%, 90%, 85%, or 80% identity to the amino acid sequence of amino acids 3 to 29 of SEQ ID NO: 13, or a functional fragment of the amino acid sequence of amino acids 3 to 29 of SEQ ID NO: 13, or the amino acid sequence having at least 99%, 98%, 97%, 96%, 95%, 90%, 85%, or 80% identity to the amino acid sequence of amino acids 3 to 29 of SEQ ID NO: 13. In some embodiments, a trimerization domain comprises the amino acid sequence of amino acids 3 to 29 of SEQ ID NO: 13.

In some embodiments, a trimerization domain comprises the amino acid sequence SEQ ID NO: 13, an amino acid sequence having at least 99%, 98%, 97%, 96%, 95%, 90%, 85%, or 80% identity to the amino acid sequence of SEQ ID NO: 13, or a functional fragment of the amino acid sequence of SEQ ID NO: 13, or the amino acid sequence having at least 99%, 98%, 97%, 96%, 95%, 90%, 85%, or 80% identity to the amino acid sequence of SEQ ID NO: 13. In some embodiments, a trimerization domain comprises the amino acid sequence of SEQ ID NO: 13. In some embodiments, the encoded amino acid sequence comprises the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, the encoded amino acid sequence comprises the amino acid sequence of SEQ ID NO: 14.

According to certain embodiments, a transmembrane domain is fused, either directly or through a linker, e.g., a glycine/serine linker, to a SARS-CoV-2 S protein, a variant thereof, or a fragment thereof, i.e., the antigenic peptide or protein. Accordingly, in some embodiments, a transmembrane domain is fused to the above described amino acid sequences derived from SARS-CoV-2 S protein or immunogenic fragments thereof (antigenic peptides or proteins) comprised by the encoded amino acid sequences described above (which may optionally be fused to a signal peptide and/or trimerization domain as described above). Such transmembrane domains are preferably located at the C-terminus of the antigenic peptide or protein, without being limited thereto. Preferably, such transmembrane domains are located at the C-terminus of the trimerization domain, if present, without being limited thereto. In some embodiments, a trimerization domain is present between the SARS-CoV-2 S protein, a variant thereof, or a fragment thereof, i.e., the antigenic peptide or protein, and the transmembrane domain. Transmembrane domains as defined herein preferably allow the anchoring into a cellular membrane of the antigenic peptide or protein as encoded by the RNA. In some embodiments, the transmembrane domain sequence as defined herein includes, without being limited thereto, the transmembrane domain sequence of SARS-CoV-2 S protein, in particular a sequence comprising the amino acid sequence of amino acids 1207 to 1254 of SEQ ID NO: 11 or a functional variant thereof. In some embodiments, a transmembrane domain sequence comprises the amino acid sequence of amino acids 1207 to 1254 of SEQ ID NO: 11, an amino acid sequence having at least 99%, 98%, 97%, 96%, 95%, 90%, 85%, or 80% identity to the amino acid sequence of amino acids 1207 to 1254 of SEQ ID NO: 11, or a functional fragment of the amino acid sequence of amino acids 1207 to 1254 of SEQ ID NO: 11, or the amino acid sequence having at least 99%, 98%, 97%, 96%, 95%, 90%, 85%, or 80% identity to the amino acid sequence of amino acids 1207 to 1254 of SEQ ID NO: 11. In some embodiments, a transmembrane domain sequence comprises the amino acid sequence of amino acids 1207 to 1254 of SEQ ID NO: 11.

In some embodiments, the encoded amino acid sequence comprises the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino acid sequence of SEQ ID NO: 15, or the amino acid sequence having at least 99%, 98%, 97%, 96%, 95%, 90%, 85%, or 80% identity to the amino acid sequence of

SEQ ID NO: 15. In some embodiments, the encoded amino acid sequence comprises the amino acid sequence of SEQ ID NO: 15.

In some embodiments, the encoded amino acid sequence comprises the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, the encoded amino acid sequence comprises the amino acid sequence of SEQ ID NO: 16.

In some embodiments, RNA (i) comprises the nucleotide sequence of SEQ ID NO: 17, a nucleotide sequence having at least 99%, 98%, 97%, 96%, 95%, 90%, 85%, or 80% identity to the nucleotide sequence of SEQ ID NO: 17, or a fragment of the nucleotide sequence of SEQ ID NO: 17, or the nucleotide sequence having at least 99%, 98%, 97%, 96%, 95%, 90%, 85%, or 80% identity to the nucleotide sequence of SEQ ID NO: 17; and/or (ii) encodes an amino acid sequence comprising the amino acid sequence of SEQ ID NO: 12, an amino acid sequence having at least 99%, 98%, 97%, 96%, 95%, 90%, 85%, or 80% identity to the amino acid sequence of SEQ ID NO: 12, or an immunogenic fragment of the amino acid sequence of SEQ ID NO: 12, or the amino acid sequence having at least 99%, 98%, 97%, 96%, 95%, 90%, 85%, or 80% identity to the amino acid sequence of SEQ ID NO: 12. In some embodiments, RNA (i) comprises the nucleotide sequence of SEQ ID NO: 17; and/or (ii) encodes an amino acid sequence comprising the amino acid sequence of SEQ ID NO: 12.

In some embodiments, RNA is nucleoside modified messenger RNA (modRNA) and (i) comprises the nucleotide sequence of SEQ ID NO: 17, a nucleotide sequence having at least 99%, 98%, 97%, 96%, 95%, 90%, 85%, or 80% identity to the nucleotide sequence of SEQ ID NO: 17, or a fragment of the nucleotide sequence of SEQ ID NO: 17, or the nucleotide sequence having at least 99%, 98%, 97%, 96%, 95%, 90%, 85%, or 80% identity to the nucleotide sequence of SEQ ID NO: 17; and/or (ii) encodes an amino acid sequence comprising the amino acid sequence of SEQ ID NO: 12, an amino acid sequence having at least 99%, 98%, 97%, 96%, 95%, 90%, 85%, or 80% identity to the amino acid sequence of SEQ ID NO: 12, or an immunogenic fragment of the amino acid sequence of SEQ ID NO: 12, or the amino acid sequence having at least 99%, 98%, 97%, 96%, 95%, 90%, 85%, or 80% identity to the amino acid sequence of SEQ ID NO: 12. In some embodiments, RNA is nucleoside modified messenger RNA (modRNA) and (i) comprises the nucleotide sequence of SEQ ID NO: 17; and/or (ii) encodes an amino acid sequence comprising the amino acid sequence of SEQ ID NO: 12.

In some embodiments, RNA (i) comprises the nucleotide sequence of SEQ ID NO: 18, a nucleotide sequence having at least 99%, 98%, 97%, 96%, 95%, 90%, 85%, or 80% identity to the nucleotide sequence of SEQ ID NO: 18, or a fragment of the nucleotide sequence of SEQ ID NO: 18, or the nucleotide sequence having at least 99%, 98%, 97%, 96%, 95%, 90%, 85%, or 80% identity to the nucleotide sequence of SEQ ID NO: 18; and/or (ii) encodes an amino acid sequence comprising the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, RNA (i) comprises the nucleotide sequence of SEQ ID NO: 18; and/or (ii) encodes an amino acid sequence comprising the amino acid sequence of SEQ ID NO: 14.

In some embodiments, RNA is nucleoside modified messenger RNA (modRNA) and (i) comprises the nucleotide sequence of SEQ ID NO: 18, a nucleotide sequence having at least 99%, 98%, 97%, 96%, 95%, 90%, 85%, or 80% identity to the nucleotide sequence of SEQ ID NO: 18, or a fragment of the nucleotide sequence of SEQ ID NO: 18, or the nucleotide sequence having at least 99%, 98%, 97%, 96%, 95%, 90%, 85%, or 80% identity to the nucleotide sequence of SEQ ID NO: 18; and/or (ii) encodes an amino acid sequence comprising the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, RNA is nucleoside modified messenger RNA (modRNA) and (i) comprises the nucleotide sequence of SEQ ID NO: 18; and/or (ii) encodes an amino acid sequence comprising the amino acid sequence of SEQ ID NO: 14.

In some embodiments, RNA (i) comprises the nucleotide sequence of SEQ ID NO: 19, a nucleotide sequence having at least 99%, 98%, 97%, 96%, 95%, 90%, 85%, or 80% identity to the nucleotide sequence of SEQ ID NO: 19, or a fragment of the nucleotide sequence of SEQ ID NO: 19, or the nucleotide sequence having at least 99%, 98%, 97%, 96%, 95%, 90%, 85%, or 80% identity to the nucleotide sequence of SEQ ID NO: 19; and/or (ii) encodes an amino acid sequence comprising the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino acid sequence of SEQ ID NO: 15, or the amino acid sequence having at least 99%, 98%, 97%, 96%, 95%, 90%, 85%, or 80% identity to the amino acid sequence of SEQ ID NO: 15. In some embodiments, RNA (i)

comprises the nucleotide sequence of SEQ ID NO: 19; and/or (ii) encodes an amino acid sequence comprising the amino acid sequence of SEQ ID NO: 15.

In some embodiments, RNA is nucleoside modified messenger RNA (modRNA) and (i) comprises the nucleotide sequence of SEQ ID NO: 19, a nucleotide sequence having at least 99%, 98%, 97%, 96%, 95%, 90%, 85%, or 80% identity to the nucleotide sequence of SEQ ID NO: 19, or a fragment of the nucleotide sequence of SEQ ID NO: 19, or the nucleotide sequence having at least 99%, 98%, 97%, 96%, 95%, 90%, 85%, or 80% identity to the nucleotide sequence of SEQ ID NO: 19; and/or (ii) encodes an amino acid sequence comprising the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino acid sequence of SEQ ID NO: 15, or the amino acid sequence having at least 99%, 98%, 97%, 96%, 95%, 90%, 85%, or 80% identity to the amino acid sequence of SEQ ID NO: 15. In some embodiments, RNA is nucleoside modified messenger RNA (modRNA) and (i) comprises the nucleotide sequence of SEQ ID NO: 19; and/or (ii) encodes an amino acid sequence comprising the amino acid sequence of SEQ ID NO: 15.

In some embodiments, RNA (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, or a fragment of the nucleotide sequence of SEQ ID NO: 20, or the nucleotide sequence having at least 99%, 98%, 97%, 96%, 95%, 90%, 85%, or 80% identity to the nucleotide sequence of SEQ ID NO: 20; and/or (ii) encodes an amino acid sequence comprising the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, RNA (i) comprises the nucleotide sequence of SEQ ID NO: 20; and/or (ii) encodes an amino acid sequence comprising the amino acid sequence of SEQ ID NO: 16. In some embodiments, RNA is nucleoside modified messenger RNA (modRNA) and (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, or a fragment of the nucleotide sequence of SEQ ID NO: 20, or the nucleotide sequence having at least 99%, 98%, 97%, 96%, 95%, 90%, 85%, or 80% identity to the nucleotide sequence of SEQ ID NO: 20; and/or (ii) encodes an amino acid sequence comprising the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, RNA is nucleoside modified messenger RNA (modRNA) and (i) comprises the nucleotide sequence of SEQ ID NO: 20; and/or (ii) encodes an amino acid sequence comprising the amino acid sequence of SEQ ID NO: 16.

In some embodiments, RNA described herein comprises BNT162b2. BNT162b2 is an mRNA vaccine for prevention of COVID-19 and demonstrated an efficacy of 95% or more at preventing COVID-19. In some embodiments, BNT162b2 is formulated as nanoparticles. In some embodiments, exemplary nanoparticles include lipid nanoparticles, polyplexes (PLX), lipidated polyplexes (LPLX), liposomes, or polysaccharide nanoparticles. The vaccine is made of a 5'capped mRNA encoding for the full-length SARS-CoV-2 spike glycoprotein (S) encapsulated in lipid nanoparticles (LNPs). In some embodiments, the RNA may be presented as a product containing BNT162b2 as active substance and other ingredients comprising: one or more cationically ionizable lipids; one or more neutral lipids (e.g., in some embodiments sterol such as, e.g., cholesterol; and/or phospholipids), and one or more polymer-conjugated lipids. The RNA may be presented as a product containing BNT162b2 as active substance and other ingredients comprising: ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), 1,2-Distearoyl-sn-glycero-3-phosphocholine (DSPC), and cholesterol. The sequence of the S protein was chosen based on the sequence for the "SARS-CoV-2 isolate Wuhan-Hu -1": GenBank: MN908947.3 (complete genome) and GenBank: QHD43416.1 (spike surface glycoprotein). The active substance consists of a single-stranded, 5'-capped codon-optimized mRNA that is translated into the spike antigen of SARS-CoV-2. The protein sequence contains two proline mutations, which ensure an antigenically optimal pre-fusion confirmation (P2 S). The RNA does not contain any uridines; instead of uridine the modified N1-methylpseudouridine is used in RNA synthesis. The RNA contains common structural elements optimized for mediating high RNA stability and translational efficiency. The LNPs protect the RNA from degradation by RNAses and enable transfection of host cells after intramuscular (IM) delivery. The mRNA is translated into the SARS-CoV-2 S protein in the host cell. The S protein is then expressed on the cell surface where it induces an adaptive immune response. The S protein is identified as a target for neutralising antibodies against the virus and is therefore considered a relevant vaccine component. BNT162b2 is administered to adults intramuscularly (IM) in two 30 μg doses given 21 days apart.

In some embodiments, the RNA is a modified RNA, in particular a stabilized mRNA. In some embodiments, the RNA comprises a modified nucleoside in place of at least one uridine. In some embodiments, the RNA comprises a modified nucleoside in place of each uridine. In some embodiments, the modified nucleoside is independently selected

from pseudouridine (ψ), N1-methyl-pseudouridine (m1ψ), and 5-methyl-uridine (m5U).

In some embodiments, the RNA comprises a modified nucleoside in place of uridine.

In some embodiments, the modified nucleoside is selected from pseudouridine (ψ), N1-methyl-pseudouridine (m1ψ), and 5-methyl-uridine (m5U).

In some embodiments, the RNA comprises a 5' cap. In some embodiments, $m2^{7,3'-O}Gppp(m_1^{2'-O})$ ApG is utilized as specific capping structure at the 5'-end of the mRNA.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7.

In some embodiments, the RNA encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

In some embodiments, the poly-A sequence comprises at least 100 nucleotides.

In some embodiments, the poly-A sequence comprises or consists of the nucleotide sequence of SEQ ID NO: 8.

In some embodiments, the RNA is formulated or is to be formulated as a liquid, a solid, or a combination thereof.

In some embodiments, the RNA is formulated or is to be formulated for injection.

In some embodiments, the RNA is formulated or is to be formulated for intramuscular administration.

In some embodiments, the RNA is formulated or is to be formulated as particles.

In some embodiments, the RNA is formulated as nanoparticles. In some embodiments, exemplary nanoparticles include lipid nanoparticles, polyplexes (PLX), lipidated polyplexes (LPLX), liposomes, or polysaccharide nanoparticles.

In some embodiments, the particles are lipid nanoparticles (LNP).

In some embodiments, the LNP particles comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

In some embodiments, the RNA is mRNA or saRNA.

In some embodiments, the RNA is formulated in a pharmaceutical composition.

In some embodiments, the pharmaceutical composition is a vaccine.

In some embodiments, the pharmaceutical composition further comprises one or more pharmaceutically acceptable carriers, diluents and/or excipients.

In some embodiments, the RNA is a component of a kit.

In some embodiments, the kit further comprises instructions for use of the RNA for inducing an immune response against coronavirus in a subject.

In some embodiments, the kit further comprises instructions for use of the RNA for therapeutically or prophylactically treating a coronavirus infection in a subject.

In some embodiments, the subject is a human.

In some embodiments, the coronavirus is a betacoronavirus.

In some embodiments, the coronavirus is a sarbecovirus.

In some embodiments, the coronavirus is SARS-CoV-2.

The term "immunologically equivalent" means that the immunologically equivalent molecule such as the immunologically equivalent amino acid sequence exhibits the same or essentially the same immunological properties and/or exerts the same or essentially the same immunological effects, *e.g.,* with respect to the type of the immunological effect. In the context of the present disclosure, the term "immunologically equivalent" is preferably used with respect to the immunological effects or properties of antigens or antigen variants used for immunization. For example, an amino acid sequence is immunologically equivalent to a reference amino acid sequence if said amino acid sequence when exposed to the immune system of a subject induces an immune reaction having a specificity of reacting with the reference amino acid sequence. Thus, in some embodiments, a molecule which is immunologically equivalent to an antigen exhibits the same or essentially the same properties and/or exerts the same or essentially the same effects regarding the stimulation, priming and/or expansion of T cells as the antigen to which the T cells are targeted.

In some embodiments, the RNA, e.g., RNA encoding vaccine antigen, used in the present disclosure is non-immunogenic. RNA encoding an immunostimulant may be administered according to the present disclosure to provide an adjuvant effect. The RNA encoding an immunostimulant may be standard RNA or non-immunogenic RNA.

The term "non-immunogenic RNA" (such as "non-immunogenic mRNA") as used herein refers to RNA that does not induce a response by the immune system upon administration, *e.g.,* to a mammal, or induces a weaker response

than would have been induced by the same RNA that differs only in that it has not been subjected to the modifications and treatments that render the non-immunogenic RNA non-immunogenic, *i.e.,* than would have been induced by standard RNA (stdRNA). In certain embodiments, non-immunogenic RNA, which is also termed modified RNA (modRNA) herein, is rendered non-immunogenic by incorporating modified nucleosides suppressing RNA-mediated activation of innate immune receptors into the RNA and/or limiting the amount of double-stranded RNA (dsRNA), e.g., by limiting the formation of double-stranded RNA (dsRNA), e.g., during *in vitro* transcription, and/or by removing double-stranded RNA (dsRNA), e.g., following *in vitro* transcription. In certain embodiments, non-immunogenic RNA is rendered non-immunogenic by incorporating modified nucleosides suppressing RNA-mediated activation of innate immune receptors into the RNA and/or by removing double-stranded RNA (dsRNA), e.g., following *in vitro* transcription.

For rendering the non-immunogenic RNA (especially mRNA) non-immunogenic by the incorporation of modified nucleosides, any modified nucleoside may be used as long as it lowers or suppresses immunogenicity of the RNA. Particularly preferred are modified nucleosides that suppress RNA-mediated activation of innate immune receptors. In some embodiments, the modified nucleosides comprise a replacement of one or more uridines with a nucleoside comprising a modified nucleobase. In some embodiments, the modified nucleobase is a modified uracil. In some embodiments, the nucleoside comprising a modified nucleobase is selected from the group consisting of 3-methyl-uridine ($m^3U$), 5-methoxy-uridine ($mo^5U$), 5-aza-uridine, 6-aza-uridine, 2-thio-5-aza-uridine, 2-thio-uridine ($s^2U$), 4-thio-uridine ($s^4U$), 4-thio-pseudouridine, 2-thio-pseudouridine, 5-hydroxy-uridine ($ho^5U$), 5-aminoallyl-uridine, 5-halo-uridine (*e.g.,* 5-iodo-uridine or 5-bromo-uridine), uridine 5-oxyacetic acid ($cmo^5U$), uridine 5-oxyacetic acid methyl ester ($mcmo^5U$), 5-carboxymethyl-uridine ($cm^5U$), 1-carboxymethyl-pseudouridine, 5-carboxyhydroxymethyl-uridine ($chm^5U$), 5-carboxyhydroxymethyl-uridine methyl ester ($mchm^5U$), 5-methoxycarbonylmethyl-uridine ($mcm^5U$), 5-methoxycarbonylmethyl-2-thio-uridine ($mcm^5s^2U$), 5-aminomethyl-2-thio-uridine ($nm^5s^2U$), 5-methylaminomethyl-uridine ($mnm^5U$), 1-ethyl-pseudouridine, 5-methylaminomethyl-2-thio-uridine ($mnm^5s^2U$), 5-methylaminomethyl-2-seleno-uridine ($mnm^5se^2U$), 5-carbamoylmethyl-uridine ($ncm^5U$), 5-carboxymethylaminomethyl-uridine ($cmnm^5U$), 5-carboxymethylaminomethyl-2-thio-uridine ($cmnm^5s^2U$), 5-propynyl-uridine, 1-propynyl-pseudouridine, 5-taurinomethyl-uridine ($\tau m^5U$), 1-taurinomethyl-pseudouridine, 5-taurinomethyl-2-thio-uridine($\tau$m5s2U), 1-taurinomethyl-4-thio-pseudouridine), 5-methyl-2-thio-uridine ($m^5s^2U$), 1-methyl-4-thio-pseudouridine ($m^1s^4\psi$), 4-thio-1-methyl-pseudouridine, 3-methyl-pseudouridine ($m^3\psi$), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine ($m^5D$), 2-thiodihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine ($acp^3U$), 1-methyl-3-(3-amino-3-carboxypropyl)pseudouridine ($acp^3$ $\psi$), 5-(isopentenylaminomethyl)uridine ($inm^5U$), 5-(isopentenylaminomethyl)-2-thio-uridine ($inm^5s^2U$), $\alpha$-thio-uridine, 2'-O-methyl-uridine (Um), 5,2'-O-dimethyl-uridine ($m^5Um$), 2'-O-methyl-pseudouridine ($\psi$m), 2-thio-2'-O-methyl-uridine ($s^2Um$), 5-methoxycarbonylmethyl-2'-O-methyl-uridine ($mcm^5Um$), 5-carbamoylmethyl-2'-O-methyl-uridine ($ncm^5Um$), 5-carboxymethylaminomethyl-2'-O-methyl-uridine ($cmnm^5Um$), 3,2'-O-dimethyl-uridine ($m^3Um$), 5-(isopentenylaminomethyl)-2'-O-methyl-uridine ($inm^5Um$), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)uridine. In certain embodiments, the nucleoside comprising a modified nucleobase is pseudouridine ($\psi$), N1-methyl-pseudouridine (m1$\psi$) or 5-methyl-uridine (m5U), in particular N1-methyl-pseudouridine.

In some embodiments, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of the uridines. During synthesis of mRNA by *in vitro* transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. Formation of dsRNA can be limited during synthesis of mRNA by *in vitro* transcription (IVT), for example, by limiting the amount of uridine triphosphate (UTP) during synthesis. Optionally, UTP may be added once or several times during synthesis of mRNA. Also, dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using *E. coli* RNaseIII that specifically hydrolyzes dsRNA but not ssRNA, thereby eliminating dsRNA contaminants from IVT RNA preparations can be used. Furthermore, dsRNA can be separated from ssRNA by using a cellulose material. In some embodiments, an RNA preparation is contacted with a cellulose material and the ssRNA is separated from the cellulose material under conditions which allow binding of dsRNA to the cellulose material and do not allow binding of ssRNA to the cellulose material. Suitable methods for providing ssRNA are disclosed, for example, in WO 2017/182524.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

In some embodiments, the amount of double-stranded RNA (dsRNA) is limited, e.g., dsRNA (especially mRNA) is removed from non-immunogenic RNA, such that less than 10%, less than 5%, less than 4%, less than 3%, less than 2%, less than 1%, less than 0.5%, less than 0.3%, less than 0.1%, less than 0.05%, less than 0.03%, less than 0.01%, less than 0.005%, less than 0.004%, less than 0.003%, less than 0.002%, less than 0.001%, or less than 0.0005% of the RNA in the non-immunogenic RNA composition is dsRNA. In some embodiments, the non-immunogenic RNA (especially mRNA) is free or essentially free of dsRNA. In some embodiments, the non-immunogenic RNA (especially mRNA) composition comprises a purified preparation of single-stranded nucleoside modified RNA. In some embodiments, the non-immunogenic RNA (especially mRNA) composition comprises single-stranded nucleoside modified RNA (especially mRNA) and is substantially free of double stranded RNA (dsRNA). In some embodiments, the non-immunogenic RNA (especially mRNA) composition comprises at least 90%, at least 91%, at least 92%, at least 93 %, at least 94%, at least 95%, at least 96%, at least 97%, at least 98%, at least 99%, at least 99.5%, at least 99.9%, at least 99.99%, at least 99.991%, at least 99.992%, , at least 99.993%,, at least 99.994%, , at least 99.995%, at least 99.996%, , at least 99.997%, or at least 99.998% single stranded nucleoside modified RNA, relative to all other nucleic acid molecules (DNA, dsRNA, etc.).

Various methods can be used to determine the amount of dsRNA. For example, a sample may be contacted with dsRNA-specific antibody and the amount of antibody binding to RNA may be taken as a measure for the amount of dsRNA in the sample. A sample containing a known amount of dsRNA may be used as a reference.

For example, RNA may be spotted onto a membrane, e.g., nylon blotting membrane. The membrane may be blocked, e.g., in TBS-T buffer (20 mM TRIS pH 7.4, 137 mM NaCl, 0.1% (v/v) TWEEN-20) containing 5% (w/v) skim milk powder. For detection of dsRNA, the membrane may be incubated with dsRNA-specific antibody, e.g., dsRNA-specific mouse mAb (English & Scientific Consulting, Szirák, Hungary). After washing, e.g., with TBS-T, the membrane may be incubated with a secondary antibody, e.g., HRP-conjugated donkey anti-mouse IgG (Jackson ImmunoResearch, Cat #715-035-150), and the signal provided by the secondary antibody may be detected.

In some embodiments, the non-immunogenic RNA (especially mRNA) is translated in a cell more efficiently than standard RNA with the same sequence. In some embodiments, translation is enhanced by a factor of 2-fold relative to its unmodified counterpart. In some embodiments, translation is enhanced by a 3-fold factor. In some embodiments, translation is enhanced by a 4-fold factor. In some embodiments, translation is enhanced by a 5-fold factor. In some embodiments, translation is enhanced by a 6-fold factor. In some embodiments, translation is enhanced by a 7-fold factor. In some embodiments, translation is enhanced by an 8-fold factor. In some embodiments, translation is enhanced by a 9-fold factor. In some embodiments, translation is enhanced by a 10-fold factor. In some embodiments, translation is enhanced by a 15-fold factor. In some embodiments, translation is enhanced by a 20-fold factor. In some embodiments, translation is enhanced by a 50-fold factor. In some embodiments, translation is enhanced by a 100-fold factor. In some embodiments, translation is enhanced by a 200-fold factor. In some embodiments, translation is enhanced by a 500-fold factor. In some embodiments, translation is enhanced by a 1000-fold factor. In some embodiments, translation is enhanced by a 2000-fold factor. In some embodiments, the factor is 10-1000-fold. In some embodiments, the factor is 10-100-fold. In some embodiments, the factor is 10-200-fold. In some embodiments, the factor is 10-300-fold. In some embodiments, the factor is 10-500-fold. In some embodiments, the factor is 20-1000-fold. In some embodiments, the factor is 30-1000-fold. In some embodiments, the factor is 50-1000-fold. In some embodiments, the factor is 100-1000-fold. In some embodiments, the factor is 200-1000-fold. In some embodiments, translation is enhanced by any other significant amount or range of amounts.

In some embodiments, the non-immunogenic RNA (especially mRNA) exhibits significantly less innate immunogenicity than standard RNA with the same sequence. In some embodiments, the non-immunogenic RNA (especially mRNA) exhibits an innate immune response that is 2-fold less than its unmodified counterpart. In some embodiments, innate immunogenicity is reduced by a 3-fold factor. In some embodiments, innate immunogenicity is reduced by a 4-fold factor. In some embodiments, innate immunogenicity is reduced by a 5-fold factor. In some embodiments, innate immunogenicity is reduced by a 6-fold factor. In some embodiments, innate immunogenicity is reduced by a 7-fold factor. In some embodiments, innate immunogenicity is reduced by a 8-fold factor. In some embodiments, innate immunogenicity is reduced by a 9-fold factor. In some embodiments, innate immunogenicity is reduced by a 10-fold factor. In some embodiments, innate immunogenicity is reduced by a 15-fold factor. In some embodiments, innate immunogenicity is reduced by a 20-fold factor. In some embodiments, innate immunogenicity is reduced by a 50-fold factor. In some embodiments, innate immunogenicity is reduced by a 100-fold factor. In some embodiments, innate immunogenicity is reduced by a 200-fold factor. In some embodiments, innate immunogenicity is reduced by a 500-fold factor. In some embodiments, innate immunogenicity is reduced by a 1000-fold factor. In some embodiments, innate immunogenicity is reduced by a 2000-fold factor.

The term "exhibits significantly less innate immunogenicity" refers to a detectable decrease in innate immunogenicity. In some embodiments, the term refers to a decrease such that an effective amount of the non-immunogenic

RNA (especially mRNA) can be administered without triggering a detectable innate immune response. In some embodiments, the term refers to a decrease such that the non-immunogenic RNA (especially mRNA) can be repeatedly administered without eliciting an innate immune response sufficient to detectably reduce production of the protein encoded by the non-immunogenic RNA. In some embodiments, the decrease is such that the non-immunogenic RNA (especially mRNA) can be repeatedly administered without eliciting an innate immune response sufficient to eliminate detectable production of the protein encoded by the non-immunogenic RNA.

"Immunogenicity" is the ability of a foreign substance, such as RNA, to provoke an immune response in the body of a human or other animal. The innate immune system is the component of the immune system that is relatively unspecific and immediate. It is one of two main components of the vertebrate immune system, along with the adaptive immune system.

**Particles**

Nucleic acids such as RNA, in particular mRNA, described herein may be present in particles comprising (i) the nucleic acid, and (ii) at least one cationic or cationically ionizable compound such as a polymer or lipid complexing the nucleic acid. Electrostatic interactions between positively charged molecules such as polymers and lipids and negatively charged nucleic acid are involved in particle formation. This results in complexation and spontaneous formation of nucleic acid particles.

Different types of RNA containing particles have been described previously to be suitable for delivery of RNA in particulate form (cf., *e.g.,* Kaczmarek, J. C. et al., 2017, Genome Medicine 9, 60). For non-viral RNA delivery vehicles, nanoparticle encapsulation of RNA physically protects RNA from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (*e.g.,* one or more layers or lamellas) made of one or more types of amphiphilic substances (*e.g.,* amphiphilic lipids). In this context, the expression "amphiphilic substance" means that the substance possesses both hydrophilic and lipophilic properties. The envelope may also comprise additional substances (*e.g.,* additional lipids) which do not have to be amphiphilic. Thus, the particle may be a monolamellar or multilamellar structure, wherein the substances constituting the one or more layers or lamellas comprise one or more types of amphiphilic substances (in particular selected from the group consisting of amphiphilic lipids) optionally in combination with additional substances (*e.g.,* additional lipids) which do not have to be amphiphilic. In some embodiments, the term "particle" relates to a micro- or nano-sized structure, such as a micro-or nano-sized compact structure. According to the present disclosure, the term "particle" includes nanoparticles.

An "RNA particle" can be used to deliver RNA to a target site of interest (*e.g.,* cell, tissue, organ, and the like). An RNA particle may be formed from lipids comprising at least one cationic or cationically ionizable lipid or lipid-like material. Without intending to be bound by any theory, it is believed that the cationic or cationically ionizable lipid or lipid-like material combines together with the RNA to form aggregates, and this aggregation results in colloidally stable particles.

RNA particles described herein include nanoparticles. In some embodiments, exemplary nanoparticles include lipid nanoparticles, lipoplex, polyplexes (PLX), lipidated polyplexes (LPLX), liposomes, or polysaccharide nanoparticles.

Polyplexes (PLX), polysaccharide nanoparticles, and liposomes, are all delivery technologies that are well known to a person of skill in the art. See, e.g., Lächelt, Ulrich, and Ernst Wagner. "Nucleic acid therapeutics using polyplexes: a journey of 50 years (and beyond)" Chemical reviews 115.19 (2015): 11043-11078; Plucinski, Alexander, Zan Lyu, and Bernhard VKJ Schmidt, "Polysaccharide nanoparticles: from fabrication to applications." Journal of Materials Chemistry B (2021); and Tenchov, Rumiana, et al. "Lipid Nanoparticles- From Liposomes to mRNA Vaccine Delivery, a Landscape of Research Diversity and Advancement," ACS nano 15.11 (2021): 16982-17015, respectively, the contents of each of which are hereby incorporated by reference herein in their entirety.

In general, a lipoplex (LPX) is obtainable from mixing two aqueous phases, namely a phase comprising RNA and a phase comprising a dispersion of lipids. In some embodiments, the lipid phase comprises liposomes.

In some embodiments, liposomes are self-closed unilamellar or multilamellar vesicular particles wherein the lamellae comprise lipid bilayers and the encapsulated lumen comprises an aqueous phase. A prerequisite for using liposomes for nanoparticle formation is that the lipids in the mixture as required are able to form lamellar (bilayer) phases in the applied aqueous environment.

In some embodiments, liposomes comprise unilamellar or multilamellar phospholipid bilayers enclosing an aqueous core (also referred to herein as an aqueous lumen). They may be prepared from materials possessing polar head (hydrophilic) groups and nonpolar tail (hydrophobic) groups. In some embodiments, cationic lipids employed in formulating liposomes designed for the delivery of nucleic acids are amphiphilic in nature and consist of a positively charged (cationic) amine head group linked to a hydrocarbon chain or cholesterol derivative via glycerol.

In some embodiments, lipoplexes are multilamellar liposome-based formulations that form upon electrostatic

interaction of cationic liposomes with RNAs. In some embodiments, formed lipoplexes possess distinct internal arrangements of molecules that arise due to the transformation from liposomal structure into compact RNA-lipoplexes. In some embodiments, these formulations are characterized by their poor encapsulation of the RNA and incomplete entrapment of the RNA.

In some embodiments, an LPX particle comprises an amphiphilic lipid, in particular cationic or cationically ionizable amphiphilic lipid, and RNA (especially mRNA) as described herein. In some embodiments, electrostatic interactions between positively charged liposomes (made from one or more amphiphilic lipids, in particular cationic or cationically ionizable amphiphilic lipids) and negatively charged nucleic acid (especially mRNA) results in complexation and spontaneous formation of nucleic acid lipoplex particles. Positively charged liposomes may be generally synthesized using a cationic or cationically ionizable amphiphilic lipid, such as DOTMA and/or DODMA, and additional lipids, such as DOPE. In some embodiments, an RNA (especially mRNA) lipoplex particle is a nanoparticle.

In general, a lipid nanoparticle (LNP) is obtainable from direct mixing of RNA in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/ionizable lipid and helper lipids such as phospholipids, cholesterol, and/or polyethylene glycol (PEG) lipids. In some embodiments, in the RNA LNPs described herein the mRNA is bound by ionizable lipid that occupies the central core of the LNP. In some embodiments, PEG lipid forms the surface of the LNP, along with phospholipids. In some embodiments, the surface comprises a bilayer. In some embodiments, cholesterol and ionizable lipid in charged and uncharged forms can be distributed throughout the LNP.

In some embodiments, RNA (*e.g.,* mRNA) may be noncovalently associated with a particle as described herein. In embodiments, the RNA (especially mRNA) may be adhered to the outer surface of the particle (surface RNA (especially surface mRNA)) and/or may be contained in the particle (encapsulated RNA (especially encapsulated mRNA)).

In some embodiments, the particles (e.g., LNPs and LPXs) described herein have a size (such as a diameter) in the range of about 10 to about 2000 nm, such as at least about 15 nm (e.g., at least about 20 nm, at least about 25 nm, at least about 30 nm, at least about 35 nm, at least about 40 nm, at least about 45 nm, at least about 50 nm, at least about 55 nm, at least about 60 nm, at least about 65 nm, at least about 70 nm, at least about 75 nm, at least about 80 nm, at least about 85 nm, at least about 90 nm, at least about 95 nm, or at least about 100 nm) and/or at most 1900 nm (e.g., at most about 1900 nm, at most about 1800 nm, at most about 1700 nm, at most about 1600 nm, at most about 1500 nm, at most about 1400 nm, at most about 1300 nm, at most about 1200 nm, at most about 1100 nm, at most about 1000 nm, at most about 950 nm, at most about 900 nm, at most about 850 nm, at most about 800 nm, at most about 750 nm, at most about 700 nm, at most about 650 nm, at most about 600 nm, at most about 550 nm, or at most about 500 nm), such as in the range of about 20 to about 1500 nm, such as about 30 to about 1200 nm, about 40 to about 1100 nm, about 50 to about 1000 nm, about 60 to about 900 nm, about 70 to 800 nm, about 80 to 700 nm, about 90 to 600 nm, or about 50 to 500 nm or about 100 to 500 nm, such as in the range of 10 to 1000 nm, 15 to 500 nm, 20 to 450 nm, 25 to 400 nm, 30 to 350 nm, 40 to 300 nm, 50 to 250 nm, 60 to 200 nm, or 70 to 150 nm.

In some embodiments, the particles (e.g., LNPs and LPXs) described herein have an average diameter that in some embodiments ranges from about 50 nm to about 1000 nm, from about 50 nm to about 800 nm, from about 50 nm to about 700 nm, from about 50 nm to about 600 nm, from about 50 nm to about 500 nm, from about 50 nm to about 450 nm, from about 50 nm to about 400 nm, from about 50 nm to about 350 nm, from about 50 nm to about 300 nm, from about 50 nm to about 250 nm, from about 50 nm to about 200 nm, from about 100 nm to about 1000 nm, from about 100 nm to about 800 nm, from about 100 nm to about 700 nm, from about 100 nm to about 600 nm, from about 100 nm to about 500 nm, from about 100 nm to about 450 nm, from about 100 nm to about 400 nm, from about 100 nm to about 350 nm, from about 100 nm to about 300 nm, from about 100 nm to about 250 nm, from about 100 nm to about 200 nm, from about 150 nm to about 1000 nm, from about 150 nm to about 800 nm, from about 150 nm to about 700 nm, from about 150 nm to about 600 nm, from about 150 nm to about 500 nm, from about 150 nm to about 450 nm, from about 150 nm to about 400 nm, from about 150 nm to about 350 nm, from about 150 nm to about 300 nm, from about 150 nm to about 250 nm, from about 150 nm to about 200 nm, from about 200 nm to about 1000 nm, from about 200 nm to about 800 nm, from about 200 nm to about 700 nm, from about 200 nm to about 600 nm, from about 200 nm to about 500 nm, from about 200 nm to about 450 nm, from about 200 nm to about 400 nm, from about 200 nm to about 350 nm, from about 200 nm to about 300 nm, or from about 200 nm to about 250 nm.

In some embodiments, the particles described herein are nanoparticles. The term "nanoparticle" relates to a nano-sized particle comprising nucleic acid (especially mRNA) as described herein and at least one cationic or cationically ionizable lipid, wherein all three external dimensions of the particle are in the nanoscale, *i.e.,* at least about 1 nm and below about 1000 nm. Preferably, the size of a particle is its diameter.

Nucleic acid particles described herein (especially mRNA particles) may exhibit a polydispersity index (PDI) less than about 0.5, less than about 0.4, less than about 0.3, less than about 0.2, less than about 0.1, or less than about 0.05. By way of example, the nucleic acid particles can exhibit a polydispersity index in a range of about 0.01 to about 0.4 or about 0.1 to about 0.3. The N/P ratio gives the ratio of the nitrogen groups in the lipid to the number of phosphate

groups in the nucleic acid. It is correlated to the charge ratio, as the nitrogen atoms (depending on the pH) are usually positively charged and the phosphate groups are negatively charged. The N/P ratio, where a charge equilibrium exists, depends on the pH. Lipid formulations are frequently formed at N/P ratios larger than four up to twelve, because positively charged nanoparticles are considered favorable for transfection. In that case, RNA is considered to be completely bound to nanoparticles.

Nucleic acid particles (especially RNA particles such as mRNA particles) described herein can be prepared using a wide range of methods that may involve obtaining a colloid from at least one cationic or cationically ionizable lipid and mixing the colloid with nucleic acid to obtain nucleic acid particles.

The term "colloid" as used herein relates to a type of homogeneous mixture in which dispersed particles do not settle out. The insoluble particles in the mixture are microscopic, with particle sizes between 1 and 1000 nanometers. The mixture may be termed a colloid or a colloidal suspension. Sometimes the term "colloid" only refers to the particles in the mixture and not the entire suspension.

For the preparation of colloids comprising at least one cationic or cationically ionizable lipid methods are applicable herein that are conventionally used for preparing liposomal vesicles and are appropriately adapted. The most commonly used methods for preparing liposomal vesicles share the following fundamental stages: (i) lipids dissolution in organic solvents, (ii) drying of the resultant solution, and (iii) hydration of dried lipid (using various aqueous media). In the film hydration method, lipids are firstly dissolved in a suitable organic solvent, and dried down to yield a thin film at the bottom of the flask. The obtained lipid film is hydrated using an appropriate aqueous medium to produce a liposomal dispersion. Furthermore, an additional downsizing step may be included.

Reverse phase evaporation is an alternative method to the film hydration for preparing liposomal vesicles that involves formation of a water-in-oil emulsion between an aqueous phase and an organic phase containing lipids. A brief sonication of this mixture is required for system homogenization. The removal of the organic phase under reduced pressure yields a milky gel that turns subsequently into a liposomal suspension.

The term "ethanol injection technique" refers to a process, in which an ethanol solution comprising lipids is rapidly injected into an aqueous solution through a needle. This action disperses the lipids throughout the solution and promotes lipid structure formation, for example lipid vesicle formation such as liposome formation. Generally, the RNA (especially mRNA) lipoplex particles described herein are obtainable by adding RNA (especially mRNA) to a colloidal liposome dispersion. Using the ethanol injection technique, such colloidal liposome dispersion is, in some embodiments, formed as follows: an ethanol solution comprising lipids, such as cationic or cationically ionizable lipids like DOTMA and/or DODMA and additional lipids, is injected into an aqueous solution under stirring. In some embodiments, the RNA (especially mRNA) lipoplex particles described herein are obtainable without a step of extrusion.

The term "extruding" or "extrusion" refers to the creation of particles having a fixed, cross-sectional profile. In particular, it refers to the downsizing of a particle, whereby the particle is forced through filters with defined pores.

Other methods having organic solvent free characteristics may also be used according to the present disclosure for preparing a colloid.

In some embodiments, LNPs comprise four components: ionizable cationic lipids, neutral lipids such as phospholipids, a steroid such as cholesterol, and a polymer conjugated lipid. In some embodiments, LNPs may be prepared by mixing lipids dissolved in ethanol rapidly with RNA in an aqueous buffer. While RNA particles described herein may comprise polymer conjugated lipids such as PEG lipids, provided herein are also RNA particles which do not comprise polymer conjugated lipids such as PEG lipids.

In some embodiments, the LNPs comprising RNA and at least one cationic or cationically ionizable lipid described herein are prepared by (a) preparing an RNA solution containing water and a buffering system; (b) preparing an ethanolic solution comprising the cationic or cationically ionizable lipid and, if present, one or more additional lipids; and (c) mixing the RNA solution prepared under (a) with the ethanolic solution prepared under (b), thereby preparing the formulation comprising LNPs. After step (c) one or more steps selected from diluting and filtrating, such as tangential flow filtrating, can follow.

In some embodiments, the LNPs comprising RNA and at least one cationic or cationically ionizable lipid described herein are prepared by (a’) preparing liposomes or a colloidal preparation of the cationic or cationically ionizable lipid and, if present, one or more additional lipids in an aqueous phase; and (b’) preparing an RNA solution containing water and a buffering system; and (c’) mixing the liposomes or colloidal preparation prepared under (a’) with the RNA solution prepared under (b’). After step (c’) one or more steps selected from diluting and filtrating, such as tangential flow filtrating, can follow.

The present disclosure describes particles comprising RNA (especially mRNA) and at least one cationic or cationically ionizable lipid which associates with the RNA to form RNA particles and compositions comprising such particles. The RNA particles may comprise RNA which is complexed in different forms by non-covalent interactions to the particle. The particles described herein are not viral particles, in particular infectious viral particles, *i.e.,* they are not able to virally infect cells.

Suitable cationic or cationically ionizable lipids are those that form nucleic acid particles and are included by

the term "particle forming components" or "particle forming agents". The term "particle forming components" or "particle forming agents" relates to any components which associate with nucleic acid to form nucleic acid particles. Such components include any component which can be part of nucleic acid particles.

In some embodiments, RNA particles (especially mRNA particles) comprise more than one type of RNA molecules, where the molecular parameters of the RNA molecules may be similar or different from each other, like with respect to molar mass or fundamental structural elements such as molecular architecture, capping, coding regions or other features,

In particulate formulation, it is possible that each RNA species is separately formulated as an individual particulate formulation. In that case, each individual particulate formulation will comprise one RNA species. The individual particulate formulations may be present as separate entities, e.g. in separate containers. Such formulations are obtainable by providing each RNA species separately (typically each in the form of an RNA-containing solution) together with a particle-forming agent, thereby allowing the formation of particles. Respective particles will contain exclusively the specific RNA species that is being provided when the particles are formed (individual particulate formulations). In some embodiments, a composition such as a pharmaceutical composition comprises more than one individual particle formulation. Respective pharmaceutical compositions are referred to as mixed particulate formulations. Mixed particulate formulations according to the invention are obtainable by forming, separately, individual particulate formulations, followed by a step of mixing of the individual particulate formulations. By the step of mixing, a formulation comprising a mixed population of RNA-containing particles is obtainable. Individual particulate populations may be together in one container, comprising a mixed population of individual particulate formulations. Alternatively, it is possible that all RNA species of the pharmaceutical composition are formulated together as a combined particulate formulation. Such formulations are obtainable by providing a combined formulation (typically combined solution) of all RNA species together with a particle-forming agent, thereby allowing the formation of particles. As opposed to a mixed particulate formulation, a combined particulate formulation will typically comprise particles which comprise more than one RNA species. In a combined particulate composition different RNA species are typically present together in a single particle.

In some embodiments, a particle (e.g., an RNA particle such as, for example, in some embodiments, an RNA-lipid nanoparticle) comprises two or more RNA molecules, each comprising a different nucleic acid sequence. In some embodiments, a particle comprises two or more RNA molecules, each comprising a different nucleic acid sequence. In some embodiments, a particle comprises two or more RNA molecules, each encoding a different immunogenic polypeptide or immunogenic fragment thereof. In some embodiments, two or more RNA molecules present in a particle comprise: a first RNA molecule encoding an immunogenic polypeptide or immunogenic fragment thereof from a coronavirus and a second RNA molecule encoding an immunogenic polypeptide or immunogenic fragment thereof from an infectious disease pathogen (e.g., virus, bacteria, parasite, etc.). For example, in some embodiments, two or more RNA molecules present in a particle comprise: a first RNA molecule encoding an immunogenic polypeptide or immunogenic fragment thereof from a coronavirus (e.g., in some embodiments SARS-CoV-2 Wuhan strain or a variant thereof, e.g., a SARS-CoV-2 having one or more mutations characteristic of an Omicron variant (e.g., a BA.1, BA.2, BA.4/5, BA.2.75, BA.2.75.2, BA.4.6, or XBB variant)) and a second RNA molecule encoding an immunogenic polypeptide or immunogenic fragment thereof from an influenza virus.

In some embodiments, two or more RNA molecules present in a particle each encode an immunogenic polypeptide or an immunogenic fragment thereof from the same and/or different strains and/or variants of coronavirus (e.g., in some embodiments SARS-CoV-2 strains or variants). For example, in some embodiments, two or more RNA molecules present in a particle each encode a different immunogenic polypeptide or immunogenic fragment thereof from a coronavirus membrane protein, a coronavirus nucleocapsid protein, a coronavirus spike protein, a coronavirus non-structural protein and/or a coronavirus accessory protein. In some embodiments, such immunogenic polypeptides or immunogenic fragments thereof may be from the same or a different coronavirus (e.g., in some embodiments a SARS-CoV-2 Wuhan strain or variants thereof, for example, in some embodiments a variant having one or more mutations characteristic of a prevalent variant such as an Omicron variant (e.g., a BA.1, BA.2, BA.4/5, BA.2.75, BA.2.75.2, BA.4.6, or XBB variant)). In some embodiments, a particle comprises a first RNA molecule encoding a SARS-CoV-2 S protein or an immunogenic fragment thereof from a first strain or variant, and a second RNA molecule encoding a SARS-CoV-2 S protein or an immunogenic fragment thereof from a second strain or variant, wherein the second strain or variant is different from the first strain or variant.

In some embodiments, a particle (e.g., in some embodiments an RNA-lipid nanoparticle as described herein) comprises a first RNA molecule encoding a SARS-CoV-2 S protein from a Wuhan strain and a second RNA molecule encoding a SARS-CoV-2 S protein comprising one or more mutations that are characteristic of an Omicron variant (e.g., a BA.1, BA.2, BA.4/5, BA.2.75, BA.2.75.2, BA.4.6, or XBB variant)). In some embodiments, a first RNA molecule encoding a SARS-CoV-2 S protein from a Wuhan strain comprises a nucleotide sequence that encodes the amino acid sequence of SEQ ID NO: 12. In some embodiments, a first RNA molecule encoding a SARS-CoV-2 S protein from a Wuhan strain comprises a nucleotide sequence that is at least 80% identical (e.g., at least 85%, at least 90%, at least 91%, at least 92%, at least 93%, at least 94%, at least 95%, at least 96%, at least 912%, at least 98%, or at least 99% identical) to

SEQ ID NO: 21. In some embodiments, a first RNA molecule encoding a SARS-COV-2 S protein from a Wuhan strain comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 17. In some embodiments, a first RNA molecule encoding a SARS-COV-2 S protein from a Wuhan strain comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 12. In some embodiments, a second RNA molecule encoding a SARS-CoV-2 S protein having one or more mutations that are characteristic of an Omicron BA.1 variant comprises a nucleotide sequence that encodes the amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 31. In some embodiments, a second RNA molecule encoding a SARS-CoV-2 S protein comprising one or more mutations characteristic of an Omicron BA.1 variant comprises a nucleotide sequence that is at least 80% identical (e.g., at least 85%, at least 90%, at least 91%, at least 92%, at least 93%, at least 94%, at least 95%, at least 96%, at least 97%, at least 98%, or at least 99% identical) to SEQ ID NO: 32. In some embodiments, a second RNA molecule encoding a SARS-COV-2 S protein comprising one or more mutations characteristic of an Omicron BA.1 variant comprises a nucleotide sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 33. In some embodiments, a second RNA molecule encoding a SARS-COV-2 S protein comprising one or more mutations characteristic of an Omicron BA.1 variant comprises a nucleotide sequence that encodes an amino acid sequence that is at least 80% identical to (e.g., at least 85%, at least 90%, at least 91%, at least 92%, at least 93%, at least 94%, at least 95%, at least 96%, at least 97%, at least 98%, or at least 99% identical to) SEQ ID NO: 31.

In some embodiments, a particle (e.g., in some embodiments an RNA-lipid nanoparticle as described herein) comprises: a first RNA molecule comprising a nucleotide sequence that encodes the amino acid sequence of SEQ ID NO: 12 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 12); and a second RNA molecule comprising a nucleotide sequence that encodes the amino acid sequence of SEQ ID NO: 31 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 31.

In some embodiments, a particle (e.g., in some embodiments an RNA-lipid nanoparticle as described herein) comprises: a first RNA molecule comprising a nucleotide sequence of SEQ ID NO: 21 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 21); and a second RNA molecule comprising a nucleotide sequence of SEQ ID NO: 32 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 32.

In some embodiments, a particle (e.g., in some embodiments an RNA-lipid nanoparticle as described herein) comprises: a first RNA molecule comprising a nucleotide sequence of SEQ ID NO: 17 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 17; and a second RNA molecule comprising a nucleotide sequence of SEQ ID NO: 33 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 33.

In some embodiments, a particle (e.g., in some embodiments an RNA-lipid nanoparticle as described herein) comprises: a first RNA molecule comprising a nucleotide sequence that encodes the amino acid sequence of SEQ ID NO: 12 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 12); and a second RNA molecule comprising a nucleotide sequence that encodes the amino acid sequence of SEQ ID NO: 22, 25, or 28 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 22, 25, or 28.

In some embodiments, a particle (e.g., in some embodiments an RNA-lipid nanoparticle as described herein) comprises: a first RNA molecule comprising a nucleotide sequence of SEQ ID NO: 21 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 21; and a second RNA molecule comprising a nucleotide sequence of SEQ ID NO: 23, 26, or 29 or a nucleotide sequence that is at least 80% (e.g., at least 85%, at least 90%, at least 91%, at least 92%, at least 93%, at least 94%, at least 95%, at least 96%, at least 97%,

at least 98%, at least 99% or higher) identical to SEQ ID NO: 23, 26, or 29.

In some embodiments, a particle (e.g., in some embodiments an RNA-lipid nanoparticle as described herein) comprises: a first RNA molecule comprising a nucleotide sequence of SEQ ID NO: 17 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 17; and a second RNA molecule comprising a nucleotide sequence of SEQ ID NO: 24, 27, or 30 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 24, 27, or 30.

In some embodiments, a particle (e.g., in some embodiments an RNA-lipid nanoparticle as described herein) comprises: a first RNA molecule comprising a nucleotide sequence that encodes the amino acid sequence of SEQ ID NO: 25 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 25; and a second RNA molecule comprising a nucleotide sequence that encodes an amino acid sequence of SEQ ID NO: 31, 22, or 28 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 31, 22, or 28.

In some embodiments, a particle (e.g., in some embodiments an RNA-lipid nanoparticle as described herein) comprises: a first RNA molecule comprising a nucleotide sequence of SEQ ID NO: 26 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 26; and a second RNA molecule comprising a nucleotide sequence of SEQ ID NO: 32, 23, or 29, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 32, 23, or 29.

In some embodiments, a particle (e.g., in some embodiments an RNA-lipid nanoparticle as described herein) comprises: a first RNA molecule comprising a nucleotide sequence of SEQ ID NO: 27 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 27; and a second RNA molecule comprising a nucleotide sequence of SEQ ID NO: 33, 24, or 30, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 33, 24, or 30.

In some embodiments, a particle (e.g., in some embodiments an RNA-lipid nanoparticle as described herein) comprises: a first RNA molecule comprising a nucleotide sequence that encodes the amino acid sequence of SEQ ID NO: 31 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 31; and a second RNA molecule comprising a nucleotide sequence that encodes the amino acid sequence of SEQ ID NO: 22 or 28 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 22 or 28. In some embodiments, a particle (e.g., in some embodiments an RNA-lipid nanoparticle as described herein) comprises: a first RNA molecule comprising a nucleotide sequence of SEQ ID NO: 32 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 32; and a second RNA molecule comprising a nucleotide sequence of SEQ ID NO: 23 or 29 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 23 or 29.

In some embodiments, a particle (e.g., in some embodiments an RNA-lipid nanoparticle as described herein) comprises: a first RNA molecule comprising a nucleotide sequence of SEQ ID NO: 33 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 33; and a second RNA molecule comprising a nucleotide sequence of SEQ ID NO: 24 or 30 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 24 or 30.

In some embodiments, a particle (e.g., in some embodiments an RNA-lipid nanoparticle as described herein) comprises: a first RNA molecule comprising a nucleotide sequence that encodes the amino acid sequence of SEQ ID NO: 22 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 22; and a second RNA molecule comprising a nucleotide sequence that encodes the amino acid sequence of SEQ ID NO: 28 or an amino acid sequence that is at least 80% (e.g., at least 85%, at least 90%, at least 91%, at least 92%, at least 93%, at least 94%, at least 95%, at least 96%, at least 97%, at least 98%, at least 99% or higher) identical

to SEQ ID NO: 28.

In some embodiments, a particle (e.g., in some embodiments an RNA-lipid nanoparticle as described herein) comprises: a first RNA molecule comprising a nucleotide sequence of SEQ ID NO: 23 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 23; and a second RNA molecule comprising a nucleotide sequence of SEQ ID NO: 29, or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 29.

In some embodiments, a particle (e.g., in some embodiments an RNA-lipid nanoparticle as described herein) comprises: a first RNA molecule comprising a nucleotide sequence of SEQ ID NO: 24 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 24; and a second RNA molecule comprising a nucleotide sequence of SEQ ID NO: 30 or a nucleotide sequence that is at least 80% (e.g., at least 85%, at least 90%, at least 91%, at least 92%, at least 93%, at least 94%, at least 95%, at least 96%, at least 97%, at least 98%, at least 99% or higher) identical to SEQ ID NO: 30.

In some embodiments, a particle (e.g., in some embodiments an RNA-lipid nanoparticle) containing nucleic acids (e.g., RNAs) encoding different polypeptides can be formed by mixing a plurality of (e.g., at least two, at least three, or more) RNA molecules with particle-forming agents (e.g., lipids). In some embodiments, nucleic acids (e.g., RNAs) encoding different polypeptides can be mixed (e.g., in some embodiments in substantially equal proportions, e.g., in some embodiments at a 1:1 ratio when two RNA molecules are present) prior to mixing with particle-forming agents (e.g., lipids).

While in some embodiments, two or more RNA molecules each encoding a different polypeptide (e.g., as described herein) can be mixed with particle-forming agents to form nucleic acid containing particles as described above. In alternative embodiments, two or more RNA molecules each encoding a different polypeptide (e.g., as described herein) can be formulated into separate particle compositions, which are then mixed together. For example, in some embodiments, individual populations of nucleic acid containing particles, each population comprising an RNA molecule encoding a different immunogenic polypeptide or immunogenic fragment thereof (e.g., as described herein), can be separately formed and then mixed together, for example, prior to filling into vials during a manufacturing process, or immediately prior to administration (e.g., by an administering health-care professional)). Accordingly, in some embodiments, described herein is a composition comprises two or more populations of particles (e.g., in some embodiments, lipid nanoparticles), each population comprising at least one RNA molecule encoding a different immunogenic polypeptide or immunogenic fragment thereof (e.g., a SARS-CoV-2 S protein, or fragments thereof, from a different variant). In some embodiments, each population may be provided in a composition at a desirable proportion (e.g., in some embodiments, each population may be provided in a composition in an amount that provides the same amount of RNA molecules).

## Polymers

Given their high degree of chemical flexibility, polymers are commonly used materials for nanoparticle-based delivery. Typically, cationic polymers are used to electrostatically condense the negatively charged nucleic acid into nanoparticles. These positively charged groups often consist of amines that change their state of protonation in the pH range between 5.5 and 7.5, thought to lead to an ion imbalance that results in endosomal rupture. Polymers such as poly-L-lysine, polyamidoamine, protamine and polyethyleneimine, as well as naturally occurring polymers such as chitosan have all been applied to nucleic acid delivery and are suitable as cationic polymers herein. In addition, some investigators have synthesized polymers specifically for nucleic acid delivery. Poly(β-amino esters), in particular, have gained widespread use in nucleic acid delivery owing to their ease of synthesis and biodegradability. Such synthetic polymers are also suitable as cationic polymers herein.

A "polymer," as used herein, is given its ordinary meaning, i.e., a molecular structure comprising one or more repeat units (monomers), connected by covalent bonds. The repeat units can all be identical, or in some cases, there can be more than one type of repeat unit present within the polymer. In some cases, the polymer is biologically derived, i.e., a biopolymer such as a protein. In some cases, additional moieties can also be present in the polymer, for example targeting moieties.

If more than one type of repeat unit is present within the polymer, then the polymer is said to be a "copolymer." It is to be understood that the polymer being employed herein can be a copolymer. The repeat units forming the copolymer can be arranged in any fashion. For example, the repeat units can be arranged in a random order, in an alternating order, or as a "block" copolymer, i.e., comprising one or more regions each comprising a first repeat unit (e.g., a first block), and one or more regions each comprising a second repeat unit (e.g., a second block), etc. Block copolymers can have two (a diblock copolymer), three (a triblock copolymer), or more numbers of distinct blocks.

In certain embodiments, the polymer is biocompatible. Biocompatible polymers are polymers that typically do

not result in significant cell death at moderate concentrations. In certain embodiments, the biocompatible polymer is biodegradable, i.e., the polymer is able to degrade, chemically and/or biologically, within a physiological environment, such as within the body.

In certain embodiments, polymer may be protamine or polyalkyleneimine.

The term "protamine" refers to any of various strongly basic proteins of relatively low molecular weight that are rich in arginine and are found associated especially with DNA in place of somatic histones in the sperm cells of various animals (as fish). In particular, the term "protamine" refers to proteins found in fish sperm that are strongly basic, are soluble in water, are not coagulated by heat, and yield chiefly arginine upon hydrolysis. In purified form, they are used in a long-acting formulation of insulin and to neutralize the anticoagulant effects of heparin.

According to the disclosure, the term "protamine" as used herein is meant to comprise any protamine amino acid sequence obtained or derived from natural or biological sources including fragments thereof and multimeric forms of said amino acid sequence or fragment thereof as well as (synthesized) polypeptides which are artificial and specifically designed for specific purposes and cannot be isolated from native or biological sources.

In one embodiment, the polyalkyleneimine comprises polyethylenimine and/or polypropylenimine, preferably polyethyleneimine. A preferred polyalkyleneimine is polyethyleneimine (PEI). The average molecular weight of PE! is preferably $0.75 \cdot 10^2$ to $10^7$ Da, preferably 1000 to $10^5$ Da, more preferably 10000 to 40000 Da, more preferably 15000 to 30000 Da, even more preferably 20000 to 25000 Da.

Preferred according to the disclosure is linear polyalkyleneimine such as linear polyethyleneimine (PEI).

Cationic polymers (including polycationic polymers) contemplated for use herein include any cationic polymers which are able to electrostatically bind nucleic acid. In one embodiment, cationic polymers contemplated for use herein include any cationic polymers with which nucleic acid can be associated, e.g. by forming complexes with the nucleic acid or forming vesicles in which the nucleic acid is enclosed or encapsulated.

Particles described herein may also comprise polymers other than cationic polymers, i.e., non-cationic polymers and/or anionic polymers. Collectively, anionic and neutral polymers are referred to herein as non-cationic polymers.

**Lipids**

The terms "lipid" and "lipid-like material" are broadly defined herein as molecules which comprise one or more hydrophobic moieties or groups and optionally also one or more hydrophilic moieties or groups. Molecules comprising hydrophobic moieties and hydrophilic moieties are also frequently denoted as amphiphiles. Lipids are usually insoluble or poorly soluble in water, but soluble in many organic solvents. In an aqueous environment, the amphiphilic nature allows the molecules to self-assemble into organized structures and different phases. One of those phases consists of lipid bilayers, as they are present in vesicles, multilamellar/unilamellar liposomes, or membranes in an aqueous environment. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). The hydrophilic groups may comprise polar and/or charged groups and include carbohydrates, phosphate, carboxylic, sulfate, amino, sulfhydryl, nitro, hydroxyl, and other like groups.

As used herein, the term "hydrophobic" refers to any a molecule, moiety or group which is substantially immiscible or insoluble in aqueous solution. The term hydrophobic group includes hydrocarbons having at least 6 carbon atoms. The hydrophobic group can have functional groups (e.g., ether, ester, halide, etc.) and atoms other than carbon and hydrogen as long as the group satisfies the condition of being substantially immiscible or insoluble in aqueous solution.

The term "hydrocarbon" includes alkyl, alkenyl, or alkynyl as defined herein. It should be appreciated that one or more of the hydrogen in alkyl, alkenyl, or alkynyl may be substituted with other atoms, e.g., halogen, oxygen or sulfur. Unless stated otherwise, hydrocarbon groups can also include a cyclic (alkyl, alkenyl or alkynyl) group or an aryl group, provided that the overall polarity of the hydrocarbon remains relatively nonpolar.

The term "alkyl" refers to a saturated linear or branched monovalent hydrocarbon moiety which may have six to thirty, typically six to twenty, often six to eighteen carbon atoms. Exemplary nonpolar alkyl groups include, but are not limited to, hexyl, decyl, dodecyl, tetradecyl, hexadecyl, octadecyl, and the like.

The term "alkenyl" refers to a linear or branched monovalent hydrocarbon moiety having at least one carbon double bond in which the total carbon atoms may be six to thirty, typically six to twenty often six to eighteen.

The term "alkynyl" refers to a linear or branched monovalent hydrocarbon moiety having at least one carbon carbon triple bond in which the total carbon atoms may be six to thirty, typically six to twenty, often six to eighteen. Alkynyl groups can optionally have one or more carbon carbon double bonds.

As used herein, the term "amphiphilic" refers to a molecule having both a polar portion and a non-polar portion. Often, an amphiphilic compound has a polar head attached to a long hydrophobic tail. In some embodiments, the polar portion is soluble in water, while the non-polar portion is insoluble in water. In addition, the polar portion may have either a formal positive charge, or a formal negative charge. Alternatively, the polar portion may have both a formal positive and a negative charge, and be a zwitterion or inner salt. For purposes of the disclosure, the amphiphilic compound can

be, but is not limited to, one or a plurality of natural or non-natural lipids and lipid-like compounds.

The term "lipid-like material", "lipid-like compound" or "lipid-like molecule" relates to substances, in particular amphiphilic substances, that structurally and/or functionally relate to lipids but may not be considered as lipids in a strict sense. For example, the term includes compounds that are able to form amphiphilic layers as they are present in vesicles, multilamellar/unilamellar liposomes, or membranes in an aqueous environment and includes surfactants, or synthesized compounds with both hydrophilic and hydrophobic moieties. Generally speaking, the term includes molecules, which comprise hydrophilic and hydrophobic moieties with different structural organization, which may or may not be similar to that of lipids. Examples of lipid-like compounds capable of spontaneous integration into cell membranes include functional lipid constructs such as synthetic function-spacer-lipid constructs (FSL), synthetic function-spacer-sterol constructs (FSS) as well as artificial amphipathic molecules. Lipids are generally cylindrical. The area occupied by the two alkyl chains is similar to the area occupied by the polar head group. Lipids have low solubility as monomers and tend to aggregate into planar bilayers that are water insoluble. Traditional surfactant monomers are generally cone shaped. The hydrophilic head groups tend to occupy more molecular space than the linear alkyl chains. In some embodiments, surfactants tend to aggregate into spherical or elliptoid micelles that are water soluble. While lipids also have the same general structure as surfactants - a polar hydrophilic head group and a nonpolar hydrophobic tail - lipids differ from surfactants in the shape of the monomers, in the type of aggregates formed in solution, and in the concentration range required for aggregation. As used herein, the term "lipid" is to be construed to cover both lipids and lipid-like materials unless otherwise indicated herein or clearly contradicted by context.

Generally, lipids may be divided into eight categories: fatty acids, glycerolipids, glycerophospholipids, sphingolipids, saccharolipids, polyketides (derived from condensation of ketoacyl subunits), sterol lipids and prenol lipids (derived from condensation of isoprene subunits). Although the term "lipid" is sometimes used as a synonym for fats, fats are a subgroup of lipids called triglycerides. Lipids also encompass molecules such as fatty acids and their derivatives (including tri-, di-, monoglycerides, and phospholipids), as well as steroids, i.e., sterol-containing metabolites such as cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'- hydroxybutyl ether, tocopherol and derivatives thereof, and mixtures thereof.

Fatty acids, or fatty acid residues are a diverse group of molecules made of a hydrocarbon chain that terminates with a carboxylic acid group; this arrangement confers the molecule with a polar, hydrophilic end, and a nonpolar, hydrophobic end that is insoluble in water. The carbon chain, typically between four and 24 carbons long, may be saturated or unsaturated, and may be attached to functional groups containing oxygen, halogens, nitrogen, and sulfur. If a fatty acid contains a double bond, there is the possibility of either a cis or trans geometric isomerism, which significantly affects the molecule's configuration. Cis-double bonds cause the fatty acid chain to bend, an effect that is compounded with more double bonds in the chain. Other major lipid classes in the fatty acid category are the fatty esters and fatty amides. Glycerolipids are composed of mono-, di-, and tri-substituted glycerols, the best-known being the fatty acid triesters of glycerol, called triglycerides. The word "triacylglycerol" is sometimes used synonymously with "triglyceride". In these compounds, the three hydroxyl groups of glycerol are each esterified, typically by different fatty acids. Additional subclasses of glycerolipids are represented by glycosylglycerols, which are characterized by the presence of one or more sugar residues attached to glycerol via a glycosidic linkage.

The glycerophospholipids are amphipathic molecules (containing both hydrophobic and hydrophilic regions) that contain a glycerol core linked to two fatty acid-derived "tails" by ester linkages and to one "head" group by a phosphate ester linkage. Examples of glycerophospholipids, usually referred to as phospholipids (though sphingomyelins are also classified as phospholipids) are phosphatidylcholine (also known as PC, GPCho or lecithin), phosphatidylethanolamine (PE or GPEtn) and phosphatidylserine (PS or GPSer). Sphingolipids are a complex family of compounds that share a common structural feature, a sphingoid base backbone. The major sphingoid base in mammals is commonly referred to as sphingosine. Ceramides (N-acyl-sphingoid bases) are a major subclass of sphingoid base derivatives with an amide-linked fatty acid. The fatty acids are typically saturated or mono-unsaturated with chain lengths from 16 to 26 carbon atoms. The major phosphosphingolipids of mammals are sphingomyelins (ceramide phosphocholines), whereas insects contain mainly ceramide phosphoethanolamines and fungi have phytoceramide phosphoinositols and mannose-containing headgroups. The glycosphingolipids are a diverse family of molecules composed of one or more sugar residues linked via a glycosidic bond to the sphingoid base. Examples of these are the simple and complex glycosphingolipids such as cerebrosides and gangliosides.

Sterol lipids, such as cholesterol and its derivatives, or tocopherol and its derivatives, are an important component of membrane lipids, along with the glycerophospholipids and sphingomyelins.

Saccharolipids describe compounds in which fatty acids are linked directly to a sugar backbone, forming structures that are compatible with membrane bilayers. In the saccharolipids, a monosaccharide substitutes for the glycerol backbone present in glycerolipids and glycerophospholipids. The most familiar saccharolipids are the acylated glucosamine precursors of the Lipid A component of the lipopolysaccharides in Gram-negative bacteria. Typical lipid A molecules are disaccharides of glucosamine, which are derivatized with as many as seven fatty-acyl chains. The minimal

lipopolysaccharide required for growth in E. coli is Kdo2-Lipid A, a hexa-acylated disaccharide of glucosamine that is glycosylated with two 3-deoxy-D-manno-octulosonic acid (Kdo) residues.

Polyketides are synthesized by polymerization of acetyl and propionyl subunits by classic enzymes as well as iterative and multimodular enzymes that share mechanistic features with the fatty acid synthases. They comprise a large number of secondary metabolites and natural products from animal, plant, bacterial, fungal and marine sources, and have great structural diversity. Many polyketides are cyclic molecules whose backbones are often further modified by glycosylation, methylation, hydroxylation, oxidation, or other processes.

According to the disclosure, lipids and lipid-like materials may be cationic, anionic or neutral. Neutral lipids or lipid-like materials exist in an uncharged or neutral zwitterionic form at a selected pH.

**Cationic/Cationically ionizable lipids**

The nucleic acid particles such RNA particles described herein comprise at least one cationic or cationically ionizable lipid as particle forming agent. Cationic or cationically ionizable lipids contemplated for use herein include any cationic or cationically ionizable lipids (including lipid-like materials) which are able to electrostatically bind nucleic acid. In some embodiments, cationic or cationically ionizable lipids contemplated for use herein can be associated with nucleic acid, e.g. by forming complexes with the nucleic acid or forming vesicles in which the nucleic acid is enclosed or encapsulated.

As used herein, a "cationic lipid" refers to a lipid or lipid-like material having a net positive charge. Cationic lipids bind negatively charged nucleic acid by electrostatic interaction. Generally, cationic lipids possess a lipophilic moiety, such as a sterol, an acyl chain, a diacyl or more acyl chains, and the head group of the lipid typically carries the positive charge.

In some embodiments, a cationic lipid has a net positive charge only at certain pH, in particular acidic pH, while it has preferably no net positive charge, preferably has no charge, i.e., it is neutral, at a different, preferably higher pH such as physiological pH. This ionizable behavior is thought to enhance efficacy through helping with endosomal escape and reducing toxicity as compared with particles that remain cationic at physiological pH.

As used herein, a "cationically ionizable lipid" refers to a lipid or lipid-like material which has a net positive charge or is neutral, *i.e.,* which is not permanently cationic. Thus, depending on the pH of the composition in which the cationically ionizable lipid is solved, the cationically ionizable lipid is either positively charged or neutral. For purposes of the present disclosure, cationically ionizable lipids are covered by the term "cationic lipid" unless contradicted by the circumstances.

In some embodiments, the cationic or cationically ionizable lipid comprises a head group which includes at least one nitrogen atom (N) which is positive charged or capable of being protonated, e.g., under physiological conditions.

Examples of cationic or cationically ionizable lipids include, but are not limited to N,N-dimethyl-2,3-dioleyloxypropylamine (DODMA), 1,2-dioleoyl-3-trimethylammonium propane (DOTAP); 1,2-di-O-octadecenyl-3-trimethylammonium propane (DOTMA), 3-(N-(N',N'-dimethylaminoethane)-carbamoyl)cholesterol (DC-Chol), dimethyldioctadecylammonium (DDAB); 1,2-dioleoyl-3-dimethylammonium-propane (DODAP); 1,2-diacyloxy-3-dimethylammonium propanes; 1,2-dialkyloxy-3-dimethylammonium propanes; dioctadecyldimethyl ammonium chloride (DODAC), 1,2-distearyloxy-N,N-dimethyl-3-aminopropane (DSDMA), 2,3-di(tetradecoxy)propyl-(2-hydroxyethyl)-dimethylazanium (DMRIE), 1,2-dimyristoyl-sn-glycero-3-ethylphosphocholine (DMEPC), 1,2-dimyristoyl-3-trimethylammonium propane (DMTAP), 1,2-dioleyloxypropyl-3-dimethyl-hydroxyethyl ammonium bromide (DORIE), and 2,3-dioleoyloxy- N-[2(spermine carboxamide)ethyl]-N,N-dimethyl-I-propanamium trifluoroacetate (DOSPA), 1,2-dilinoleyloxy-N,N-dimethylaminopropane (DLinDMA), 1,2-dilinolenyloxy-N,N-dimethylaminopropane (DLenDMA), dioctadecylamidoglycyl spermine (DOGS), 3-dimethylamino-2-(cholest-5-en-3-beta-oxybutan-4-oxy)-1-(cis,cis-9,12-oc-tadecadienoxy)propane (CLinDMA), 2-[5'-(cholest-5-en-3-beta-oxy)-3'-oxapentoxy)-3-dimethyl-1-(cis,cis-9',12'-octadecadienoxy)propane

(CpLinDMA), N,N-dimethyl-3,4-dioleyloxybenzylamine (DMOBA), 1,2-N,N'-dioleylcarbamyl-3-dimethylaminopropane (DOcarbDAP), 2,3-Dilinoleoyloxy-N,N-dimethylpropylamine (DLinDAP), 1,2-N,N'-Dilinoleylcarbamyl-3-dimethylaminopropane (DLincarbDAP), 1,2-Dilinoleoylcarbamyl-3-dimethylaminopropane (DLinCDAP), 2,2-dilinoleyl-4-dimethylaminomethyl-[1,3]-dioxolane (DLin-K-DMA), 2,2-dilinoleyl-4-dimethylaminoethyl-[1,3]-dioxolane (DLin-K-XTC2-DMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA), N-(2-Hydroxyethyl)-N,N-dimethyl-2,3-bis(tetradecyloxy)-1-propanaminium bromide (DMRIE), (±)-N-(3-aminopropyl)-N,N-dimethyl-2,3-bis(cis-9-tetradecenyloxy)-1-propanaminium bromide (GAP-DMORIE), (±)-N-(3-aminopropyl)-N,N-dimethyl-2,3-bis(dodecyloxy)-1-propanaminium bromide (GAP-DLRIE), (±)-N-(3-aminopropyl)-N,N-dimethyl-2,3-bis(tetradecyloxy)-1-propanaminium bromide (GAP-DMRIE), N-(2-Aminoethyl)-N,N-dimethyl-2,3-bis(tetradecyloxy)-1-propanaminium bromide (βAE-DMRIE), N-(4-carboxybenzyl)-N,N-dimethyl-2,3-bis(oleoyloxy)propan-1-aminium (DOBAQ), 2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA), 1,2-dimyristoyl-3-dimethylammonium-propane (DMDAP), 1,2-dipalmitoyl-3-dimethylammonium-propane (DPDAP), N1-[2-((1S)-1-[(3-aminopropyl)amino]-4-[di(3-amino-propyl)amino]butylcarboxamido)ethyl]-3,4-di[oleyloxy]-benzamide (MVL5), 1,2-dioleoyl-sn-glycero-3-ethylphosphocholine (DOEPC), 2,3-bis(dodecyloxy)-N-(2-hydroxyethyl)-N,N-dimethylpropan-1-amonium bromide (DLRIE), N-(2-aminoethyl)-N,N-dimethyl-2,3-bis(tetradecyloxy)propan-1-aminium bromide (DMORIE), di((Z)-non-2-en-1-yl) 8,8'-((((2(dimethylamino)ethyl)thio)carbonyl)azanediyl)dioctanoate (ATX), N,N-dimethyl-2,3-bis(dodecyloxy)propan-1-amine (DLDMA), N,N-dimethyl-2,3-bis(tetradecyloxy)propan-1-amine (DMDMA), Di((Z)-non-2-en-1-yl)-9-((4-(dimethylaminobutanoyl)oxy)heptadecanedioate (L319), N-Dodecyl-3-((2-dodecylcarbamoyl-ethyl)-{2-[(2-dodecylcarbamoyl-ethyl)-2-{(2-dodecylcarbamoyl-ethyl)-[2-(2-dodecylcarbamoyl-ethylamino)-ethyl]-amino}-ethylamino)propionamide(lipidoid $98N_{12}$-5), 1-[2-[bis(2-hydroxydodecyl)amino]ethyl-[2-[4-[2-[bis(2 hydroxydodecyl)amino]ethyl]piperazin-1-yl]ethyl]amino]dodecan-2-ol (lipidoid C12-200).

In some embodiments, the cationic or cationically ionizable lipid is DOTMA. In some embodiments, the cationic or cationically ionizable lipid is DODMA.

DOTMA is a cationic lipid with a quarternary amine headgroup. The structure of DOTMA may be represented as follows:

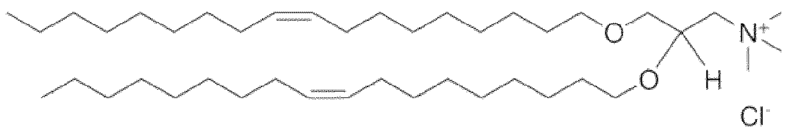

DODMA is an ionizable cationic lipid with a tertiary amine headgroup. The structure of DODMA may be represented as follows:

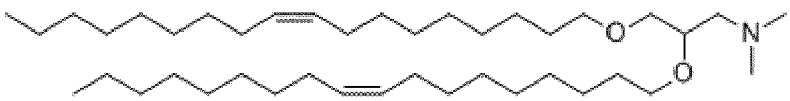

In some embodiments, the cationic or cationically ionizable lipid may comprise from about 10 mol % to about 95 mol %, from about 20 mol % to about 95 mol %, from about 20 mol % to about 90 mol %, from about 30 mol % to about 90 mol %, from about 40 mol % to about 90 mol %, or from about 40 mol % to about 80 mol % of the total lipid present in the particle.

**Additional lipids**

Particles described herein may also comprise lipids (including lipid-like materials) other than cationic or cationically ionizable lipids (also collectively referred to herein as cationic lipids), i.e., non-cationic lipids (including non-cationic or non-cationically ionizable lipids or lipid-like materials). Collectively, anionic and neutral lipids or lipid-like materials are referred to herein as non-cationic lipids. Optimizing the formulation of nucleic acid particles by addition of other hydrophobic moieties, such as cholesterol and lipids, in addition to a cationic or cationically ionizable lipid may enhance particle stability and efficacy of nucleic acid delivery.

One or more additional lipids may or may not affect the overall charge of the nucleic acid particles. In some embodiments, the one or more additional lipids are a non-cationic lipid or lipid-like material. The non-cationic lipid may comprise, *e.g.,* one or more anionic lipids and/or neutral lipids. As used herein, an "anionic lipid" refers to any lipid that is negatively charged at a selected pH. As used herein, a "neutral lipid" refers to any of a number of lipid species that exist either in an uncharged or neutral zwitterionic form at a selected pH.

In some embodiments, the nucleic acid particles (especially the particles comprising mRNA) described herein comprise a cationic or cationically ionizable lipid and one or more additional lipids.

Without wishing to be bound by theory, the amount of the cationic or cationically ionizable lipid compared to the amount of the one or more additional lipids may affect important nucleic acid particle characteristics, such as charge, particle size, stability, tissue selectivity, and bioactivity of the nucleic acid. Accordingly, in some embodiments, the molar ratio of the cationic or cationically ionizable lipid to the one or more additional lipids is from about 10:0 to about 1:9, about 4:1 to about 1:2, about 4:1 to about 1:1, about 3:1 to about 1:1, or about 3:1 to about 2:1.

In some embodiments, the one or more additional lipids comprised in the nucleic acid particles (especially in the particles comprising mRNA) described herein comprise one or more of the following: neutral lipids, steroids, and combinations thereof.

In some embodiments, the one or more additional lipids comprise a neutral lipid which is a phospholipid. In some embodiments, the phospholipid is selected from the group consisting of phosphatidylcholines, phosphatidylethanolamines, phosphatidylglycerols, phosphatidic acids, phosphatidylserines and sphingomyelins. Specific phospholipids that can be used include, but are not limited to, phosphatidylcholines, phosphatidylethanolamines, phosphatidylglycerols, phosphatidic acids, phosphatidylserines or sphingomyelin. Such phospholipids include in particular diacylphosphatidylcholines, such as distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC) and phosphatidylethanolamines, in particular diacylphosphatidylethanolamines, such as dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), 1,2-di-(9Z-octadecenoyl)-sn-glycero-3-phosphocholine (DOPG), 1,2-dipalmitoyl-sn-glycero-3-phospho-(1'-rac-glycerol) (DPPG), 1-palmitoyl-2-oleoyl-sn-glycero-3-phosphoethanolamine (POPE), N-palmitoyl-D-erythro-sphingosylphosphorylcholine (SM), and further phosphatidylethanolamine lipids with different hydrophobic chains. In some embodiments, the neutral lipid is selected from the group consisting of DSPC, DOPC, DMPC, DPPC, POPC, DOPE, DOPG, DPPG, POPE, DPPE, DMPE, DSPE, and SM. In some embodiments, the neutral lipid is selected from the group consisting of DSPC, DPPC, DMPC, DOPC, POPC, DOPE and SM. In some embodiments, the neutral lipid is DOPE.

In some embodiments, the additional lipid comprises one of the following: (1) a phospholipid, (2) cholesterol or a derivative thereof; or (3) a mixture of a phospholipid and cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'-hydroxybutyl ether, tocopherol and derivatives thereof, and mixtures thereof. Thus, in some embodiments, the nucleic acid particles (especially the particles comprising mRNA) described herein comprise (1) a cationic or cationically ionizable lipid, and a phospholipid such as DOPE or (2) a cationic or cationically ionizable lipid and a phospholipid such as DOPE and cholesterol.

In some embodiments, the nucleic acid particles (especially the particles comprising mRNA) described herein comprise (1) DOTMA and DOPE, (2) DOTMA, DOPE and cholesterol, (3) DODMA and DOPE or (4) DODMA, DOPE and cholesterol.

DOPE is a neutral phospholipid. The structure of DOPE may be represented as follows:

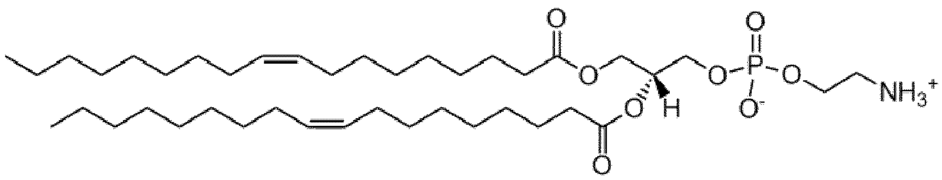

The structure of cholesterol may be represented as follows:

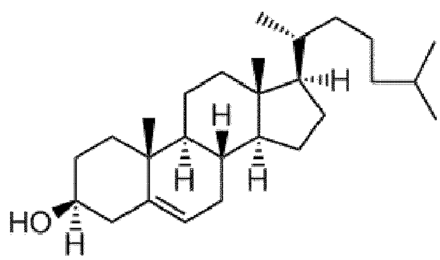

In some embodiments, particles described herein do not include a polymer conjugated lipid such as a pegylated lipid. The term "pegylated lipid" refers to a molecule comprising both a lipid portion and a polyethylene glycol portion. Pegylated lipids are known in the art.

In some embodiments, the additional lipid (e.g., one or more phospholipids and/or cholesterol) may comprise from about 0 mol % to about 90 mol %, from about 0 mol % to about 80 mol %, from about 2 mol % to about 80 mol %, from about 5 mol % to about 80 mol %, from about 5 mol % to about 60 mol %, from about 5 mol % to about 50 mol %, from about 7.5 mol % to about 50 mol %, or from about 10 mol % to about 40 mol % of the total lipid present in the particle. In some embodiments, the additional lipid (e.g., one or more phospholipids and/or cholesterol) comprises about 10 mol %, about 15 mol %, or about 20 mol % of the total lipid present in the particle.

In some embodiments, the additional lipid comprises a mixture of: (i) a phospholipid such as DOPE; and (ii) cholesterol or a derivative thereof. In some embodiments, the molar ratio of the phospholipid such as DOPE to the cholesterol or a derivative thereof is from about 9:0 to about 1:10, about 2:1 to about 1:4, about 1:1 to about 1:4, or about 1:1 to about 1:3.

### Polymer-conjugated lipids

In some embodiments, a particle may comprise at least one polymer-conjugated lipid. A polymer-conjugated lipid is typically a molecule comprising a lipid portion and a polymer portion conjugated thereto. In some embodiments, a polymer-conjugated lipid is a PEG-conjugated lipid, also referred to herein as pegylated lipid or PEG-lipid.

In some embodiments, a polymer-conjugated lipid is designed to sterically stabilize a lipid particle by forming a protective hydrophilic layer that shields the hydrophobic lipid layer. In some embodiments, a polymer-conjugated lipid can reduce its association with serum proteins and/or the resulting uptake by the reticuloendothelial system when such lipid particles are administered *in vivo.*

### Polyethyleneglycol (PEG)-conjugated lipids

Various PEG-conjugated lipids are known in the art and include, but are not limited to pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3-di(tetradecanoxy)propyl)carbamate or 2,3-di(tetradecanoxy)propyl-N-(ω methoxy(polyethoxy)ethyl)carbamate, and the like.

In some embodiments, a particle may comprise one or more PEG-conjugated lipids or pegylated lipids as described in WO 2017/075531 and WO 2018/081480, the entire contents of each of which are incorporated herein by reference for the purposes described herein.

### Lipoplex Particles

In some embodiments of the present disclosure, the RNA described herein may be present in RNA lipoplex particles.

Lipoplexes (LPX) are electrostatic complexes which are generally formed by mixing preformed cationic lipid liposomes with anionic RNA. Formed lipoplexes possess distinct internal arrangements of molecules that arise due to the transformation from liposomal structure into compact RNA-lipoplexes. These formulations are generally characterized by their poor encapsulation of the nucleic acid and incomplete entrapment of the nucleic acid.

In certain embodiments, the RNA lipoplex particles include both a cationic lipid and an additional lipid. In an exemplary embodiment, the cationic lipid is DOTMA and the additional lipid is DOPE.

In some embodiments, the molar ratio of the at least one cationic lipid to the at least one additional lipid is from about 10:0 to about 1:9, about 4:1 to about 1:2, or about 3:1 to about 1:1. In specific embodiments, the molar ratio may be about 3:1, about 2.75:1, about 2.5:1, about 2.25:1, about 2:1, about 1.75:1, about 1.5:1, about 1.25:1, or about 1:1. In an exemplary embodiment, the molar ratio of the at least one cationic lipid to the at least one additional lipid is about 2:1.

RNA lipoplex particles described herein have an average diameter that in some embodiments ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In an embodiment, the RNA lipoplex particles have an average diameter that ranges from about 250 nm to about 700 nm. In another embodiment, the RNA lipoplex particles have an average diameter that ranges from about 300 nm to about 500 nm. In an exemplary embodiment, the RNA lipoplex particles have an average diameter of about 400 nm.

The RNA lipoplex particles and compositions comprising RNA lipoplex particles described herein are useful for delivery of RNA to a target tissue after parenteral administration, in particular after intravenous administration.

Spleen targeting RNA lipoplex particles are described in WO 2013/143683, herein incorporated by reference. It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex

particles of the disclosure may be used for expressing RNA in the spleen. In an embodiment, after administration of the RNA lipoplex particles, no or essentially no RNA accumulation and/or RNA expression in the lung and/or liver occurs. In some embodiments, after administration of the RNA lipoplex particles, RNA accumulation and/or RNA expression in antigen presenting cells, such as professional antigen presenting cells in the spleen occurs. Thus, RNA lipoplex particles of the disclosure may be used for targeting RNA, e.g., RNA encoding an antigen or at least one epitope, to the lymphatic system, in particular secondary lymphoid organs, more specifically spleen. Targeting the lymphatic system, in particular secondary lymphoid organs, more specifically spleen is in particular preferred if the RNA administered is RNA encoding vaccine antigen. In some embodiments, the target cell is a spleen cell. In some embodiments, the target cell is an antigen presenting cell such as a professional antigen presenting cell in the spleen. In some embodiments, the target cell is a dendritic cell in the spleen.

The electric charge of the RNA lipoplex particles of the present disclosure is the sum of the electric charges present in the at least one cationic lipid and the electric charges present in the RNA. The charge ratio is the ratio of the positive charges present in the at least one cationic lipid to the negative charges present in the RNA. The charge ratio of the positive charges present in the at least one cationic lipid to the negative charges present in the RNA is calculated by the following equation: charge ratio=[(cationic lipid concentration (mol)) * (the total number of positive charges in the cationic lipid)] / [(RNA concentration (mol)) * (the total number of negative charges in RNA)]. The concentration of RNA and the at least one cationic lipid amount can be determined using routine methods by one skilled in the art.

In some embodiments, at physiological pH the charge ratio of positive charges to negative charges in the RNA lipoplex particles is from about 1.6:2 to about 1:2, or about 1.6:2 to about 1.1:2. In specific embodiments, the charge ratio of positive charges to negative charges in the RNA lipoplex particles at physiological pH is about 1.6:2.0, about 1.5:2.0, about 1.4:2.0, about 1.3:2.0, about 1.2:2.0, about 1.1:2.0, or about 1:2.0.

**Embodiments of Lipid nanoparticles (LNPs)**

In some embodiments, RNA described herein is present in the form of lipid nanoparticles (LNPs). The LNP may comprise any lipid capable of forming a particle to which the one or more nucleic acid molecules are attached, or in which the one or more nucleic acid molecules are encapsulated.

LNPs typically comprise four components: ionizable cationic lipids, neutral lipids such as phospholipids, a steroid such as cholesterol, and a polymer-conjugated lipid such as PEG-lipid. LNPs may be prepared by mixing lipids dissolved in ethanol with nucleic acid in an aqueous buffer.

In some embodiments, in the RNA LNPs described herein the mRNA is bound by ionizable lipid that occupies the central core of the LNP. PEG lipid forms the surface of the LNP, along with phospholipids. In some embodiments, the surface comprises a bilayer. In some embodiments, cholesterol and ionizable lipid in charged and uncharged forms can be distributed throughout the LNP.

In some embodiments, the LNP comprises one or more cationic lipids, and one or more stabilizing lipids. Stabilizing lipids include neutral lipids and pegylated lipids.

In some embodiments, the LNP comprises a cationic lipid, a neutral lipid, a steroid, a polymer-conjugated lipid; and the RNA, encapsulated within or associated with the lipid nanoparticle. In some embodiments, the LNP comprises from 40 to 55 mol percent, from 40 to 50 mol percent, from 41 to 50 mol percent, from 42 to 50 mol percent, from 43 to 50 mol percent, from 44 to 50 mol percent, from 45 to 50 mol percent, from 46 to 50 mol percent, or from 46 to 49 mol percent of the cationic lipid.

In some embodiments, the neutral lipid is present in a concentration ranging from 5 to 15 mol percent, from 7 to 13 mol percent, or from 9 to 11 mol percent.

In some embodiments, the steroid is present in a concentration ranging from 30 to 50 mol percent, from 35 to 45 mol percent or from 38 to 43 mol percent.

In some embodiments, the LNP comprises from 1 to 10 mol percent, from 1 to 5 mol percent, or from 1 to 2.5 mol percent of the polymer-conjugated lipid.

In some embodiments, the LNP comprises from 45 to 50 mol percent a cationic lipid; from 5 to 15 mol percent of a neutral lipid; from 35 to 45 mol percent of a steroid; from 1 to 5 mol percent of a polymer-conjugated lipid; and the RNA, encapsulated within or associated with the lipid nanoparticle.

In some embodiments, the mol percent is determined based on total mol of lipid present in the lipid nanoparticle. In some embodiments, the mol percent is determined based on total mol of cationic lipid, neutral lipid, steroid and polymer-conjugated lipid present in the lipid nanoparticle.

In some embodiments, the neutral lipid is selected from the group consisting of DSPC, DPPC, DMPC, DOPC, POPC, DOPE, DOPG, DPPG, POPE, DPPE, DMPE, DSPE, and SM. In some embodiments, the neutral lipid is selected from the group consisting of DSPC, DPPC, DMPC, DOPC, POPC, DOPE and SM. In some embodiments, the neutral lipid is DSPC.

In some embodiments, the steroid is cholesterol.

In some embodiments, the polymer conjugated lipid is a pegylated lipid. In some embodiments, the pegylated lipid has the following structure:

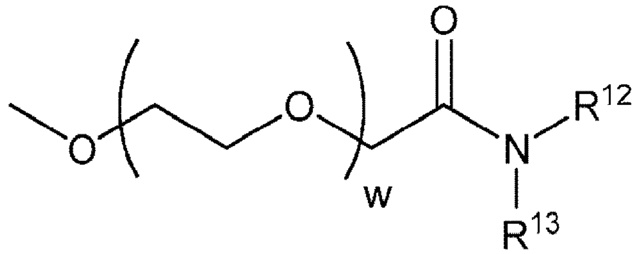

or a pharmaceutically acceptable salt, tautomer or stereoisomer thereof, wherein: $R^{12}$ and $R^{13}$ are each independently a straight or branched, saturated or unsaturated alkyl chain containing from 10 to 30 carbon atoms, wherein the alkyl chain is optionally interrupted by one or more ester bonds; and w has a mean value ranging from 30 to 60. In some embodiments, $R^{12}$ and $R^{13}$ are each independently straight, saturated alkyl chains containing from 12 to 16 carbon atoms. In some embodiments, w has a mean value ranging from 40 to 55. In some embodiments, the average w is about 45. In some embodiments, $R^{12}$ and $R^{13}$ are each independently a straight, saturated alkyl chain containing about 14 carbon atoms, and w has a mean value of about 45.

In some embodiments, a pegylated lipid is or comprises 2-[(Polyethylene glycol)-2000]-N,N-ditetradecylacetamide.

In some embodiments, the pegylated lipid is DMG-PEG 2000, e.g., having the following structure:

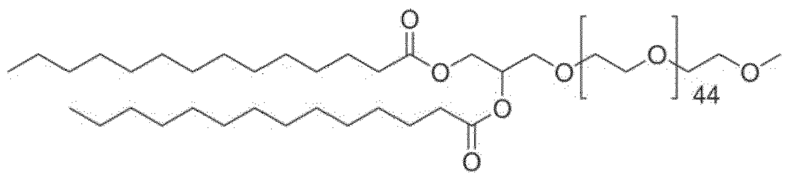

In some embodiments, the cationic lipid component of the LNPs has the structure of Formula (III):

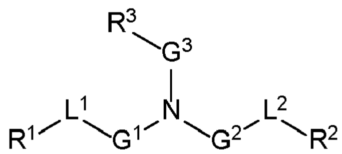

(III)

or a pharmaceutically acceptable salt, tautomer, prodrug or stereoisomer thereof, wherein:

one of $L^1$ or $L^2$ is -O(C=O)-, -(C=O)O-, -C(=O)-, -O-, $-S(O)_x-$, -S-S-, -C(=O)S-, SC(=O)-, $-NR^aC(=O)-$, $-C(=O)NR^a-$, $NR^aC(=O)NR^a-$, $-OC(=O)NR^a-$ or $-NR^aC(=O)O-$, and the other of $L^1$ or $L^2$ is -O(C=O)-, -(C=O)O-, -C(=O)-, -O-, $-S(O)_x-$, -S-S-, -C(=O)S-, SC(=O)-, $-NR^aC(=O)-$, $-C(=O)NR^a-$, $NR^aC(=O)NR^a-$, $-OC(=O)NR^a-$ or $-NR^aC(=O)O-$ or a direct bond;
$G^1$ and $G^2$ are each independently unsubstituted $C_1$-$C_{12}$ alkylene or $C_1$-$C_{12}$ alkenylene;
$G^3$ is $C_1$-$C_{24}$ alkylene, $C_1$-$C_{24}$ alkenylene, $C_3$-$C_8$ cycloalkylene, $C_3$-$C_8$ cycloalkenylene;
$R^a$ is H or $C_1$-$C_{12}$ alkyl;
$R^1$ and $R^2$ are each independently $C_6$-$C_{24}$ alkyl or $C_6$-$C_{24}$ alkenyl;
$R^3$ is H, $OR^5$, CN, $-C(=O)OR^4$, $-OC(=O)R^4$ or $-NR^5C(=O)R^4$;
$R^4$ is $C_1$-$C_{12}$ alkyl;
$R^5$ is H or $C_1$-$C_6$ alkyl; and
x is 0, 1 or 2.

In some of the foregoing embodiments of Formula (III), the lipid has one of the following structures (IIIA) or (IIIB):

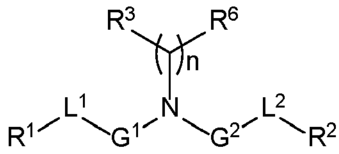

or

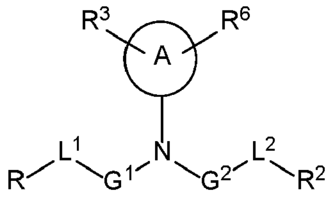

(IIIA) (IIIB)

wherein:

A is a 3 to 8-membered cycloalkyl or cycloalkylene ring;
$R^6$ is, at each occurrence, independently H, OH or $C_1$-$C_{24}$ alkyl;
n is an integer ranging from 1 to 15.

In some of the foregoing embodiments of Formula (III), the lipid has structure (IIIA), and in other embodiments, the lipid has structure (IIIB).

In other embodiments of Formula (III), the lipid has one of the following structures (IIIC) or (IIID):

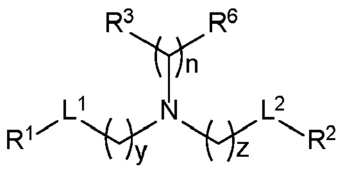

or

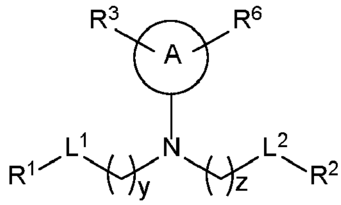

(IIIC) (IIID)

wherein y and z are each independently integers ranging from 1 to 12.

In any of the foregoing embodiments of Formula (III), one of $L^1$ or $L^2$ is -O(C=O)-. For example, in some embodiments each of $L^1$ and $L^2$ are -O(C=O)-. In some different embodiments of any of the foregoing, $L^1$ and $L^2$ are each independently -(C=O)O- or -O(C=O)-. For example, in some embodiments each of $L^1$ and $L^2$ is -(C=O)O-.

In some different embodiments of Formula (III), the lipid has one of the following structures (IIIE) or (IIIF):

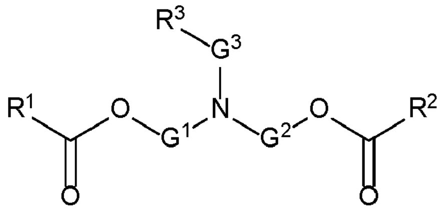

or

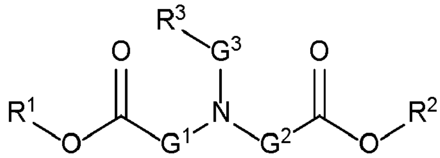

.

(IIIE) (IIIF)

In some of the foregoing embodiments of Formula (III), the lipid has one of the following structures (IIIG), (IIIH), (IIII), or (IIIJ):

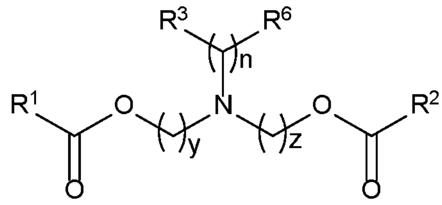 ; 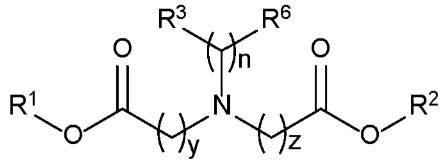 ;

(IIIG) (IIIH)

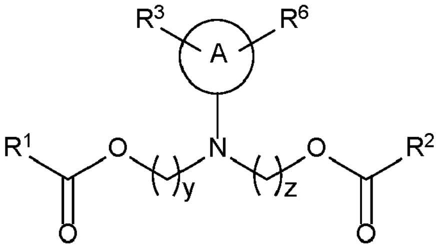 or 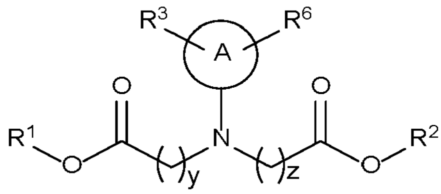 .

(IIII) (IIIJ)

In some of the foregoing embodiments of Formula (III), n is an integer ranging from 2 to 12, for example from 2 to 8 or from 2 to 4. For example, in some embodiments, n is 3, 4, 5 or 6. In some embodiments, n is 3. In some embodiments, n is 4. In some embodiments, n is 5. In some embodiments, n is 6.

In some other of the foregoing embodiments of Formula (III), y and z are each independently an integer ranging from 2 to 10. For example, in some embodiments, y and z are each independently an integer ranging from 4 to 9 or from 4 to 6.

In some of the foregoing embodiments of Formula (III), $R^6$ is H. In other of the foregoing embodiments, $R^6$ is $C_1$-$C_{24}$ alkyl. In other embodiments, $R^6$ is OH.

In some embodiments of Formula (III), $G^3$ is unsubstituted. In other embodiments, G3 is substituted. In various different embodiments, $G^3$ is linear $C_1$-$C_{24}$ alkylene or linear $C_1$-$C_{24}$ alkenylene.

In some other foregoing embodiments of Formula (III), $R^1$ or $R^2$, or both, is $C_6$-$C_{24}$ alkenyl. For example, in some embodiments, $R^1$ and $R^2$ each, independently have the following structure:

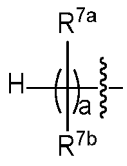 .

wherein:

$R^{7a}$ and $R^{7b}$ are, at each occurrence, independently H or $C_1$-$C_{12}$ alkyl; and
a is an integer from 2 to 12,
wherein $R^{7a}$, $R^{7b}$ and a are each selected such that $R^1$ and $R^2$ each independently comprise from 6 to 20 carbon atoms. For example, in some embodiments a is an integer ranging from 5 to 9 or from 8 to 12.

In some of the foregoing embodiments of Formula (III), at least one occurrence of $R^{7a}$ is H. For example, in some embodiments, $R^{7a}$ is H at each occurrence. In other different embodiments of the foregoing, at least one occurrence of $R^{7b}$ is $C_1$-$C_8$ alkyl. For example, in some embodiments, $C_1$-$C_8$ alkyl is methyl, ethyl, n-propyl, iso-propyl, n-butyl, iso-butyl, tert-butyl, n-hexyl or n-octyl.

In different embodiments of Formula (III), $R^1$ or $R^2$, or both, has one of the following structures:

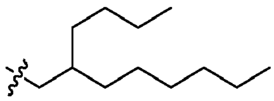 ; 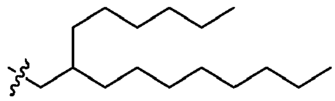 ; 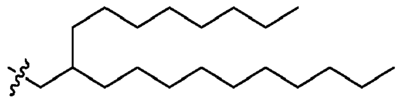 ;

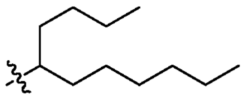 ; 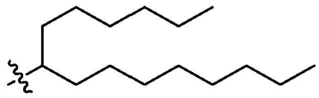 ; 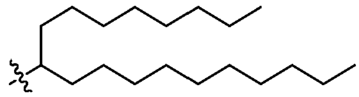 ; 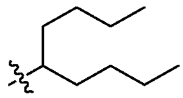 ;

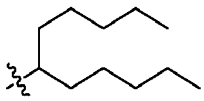 ; 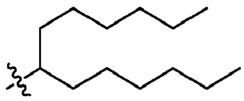 ; 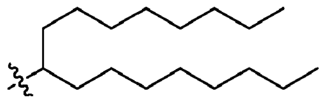 .

In some of the foregoing embodiments of Formula (III), $R^3$ is OH, CN, $-C(=O)OR^4$, $-OC(=O)R^4$ or $-NHC(=O)R^4$. In some embodiments, $R^4$ is methyl or ethyl.

In various different embodiments, the cationic lipid of Formula (III) has one of the structures set forth in the table below.

Representative Compounds of Formula (III).

| No. | Structure |
|---|---|
| III-1 | 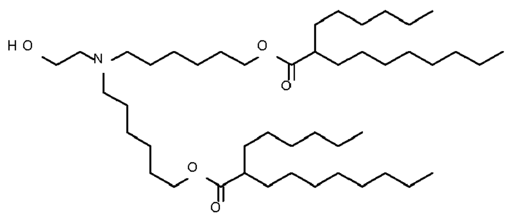 |
| III-2 | 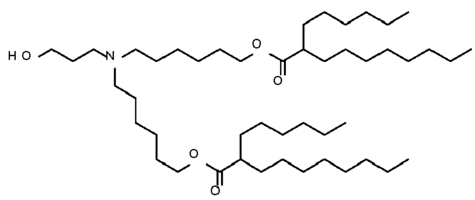 |
| III-3 | 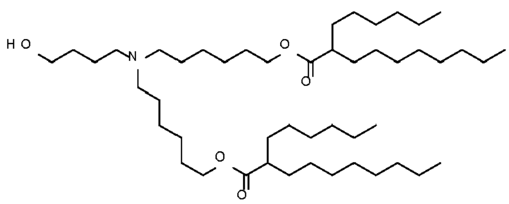 |
| III-4 | 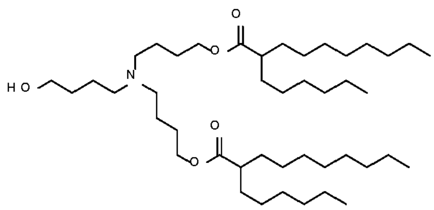 |

(continued)

| No. | Structure |
|---|---|
| III-5 | 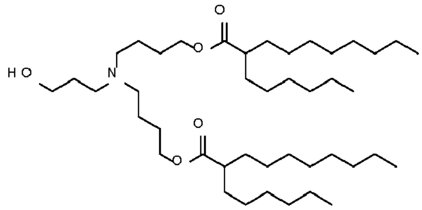 |
| III-6 | 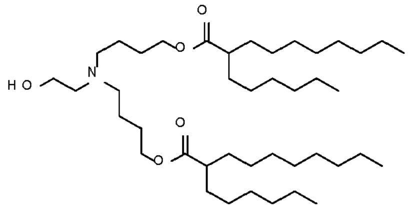 |
| III-7 | 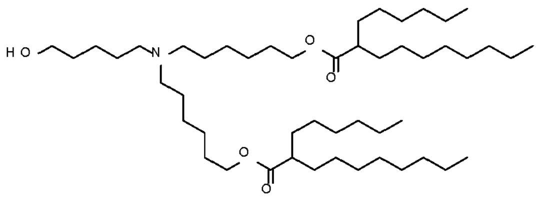 |
| III-8 | 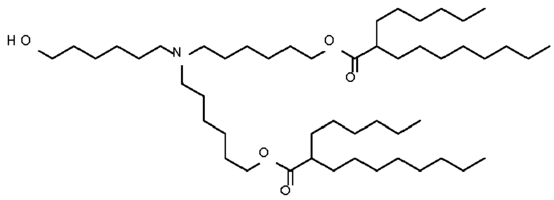 |
| III-9 | 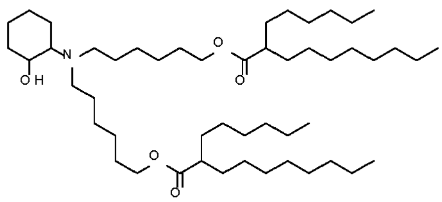 |
| III-10 | 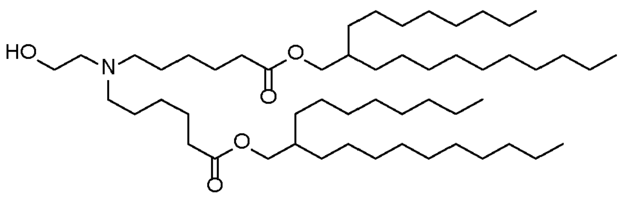 |

(continued)

| No. | Structure |
|---|---|
| III-11 | 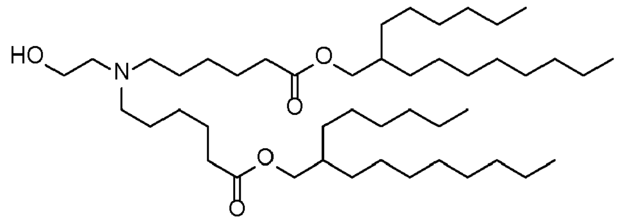 |
| III-12 | 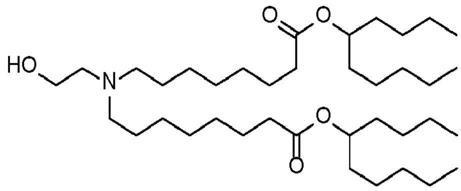 |
| III-13 | 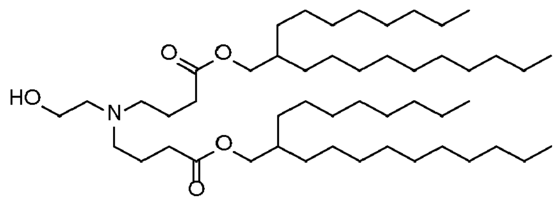 |
| III-14 | 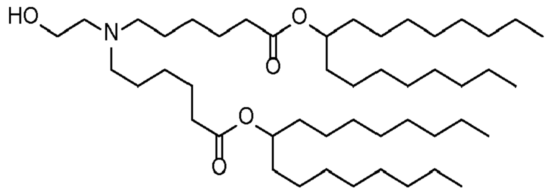 |
| III-15 | 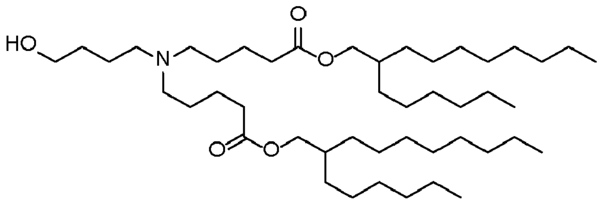 |
| III-16 | 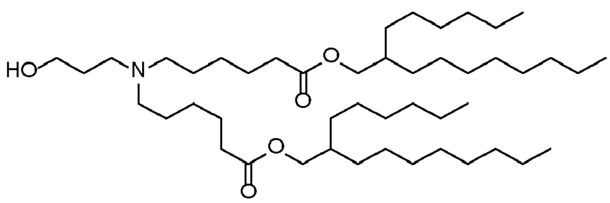 |
| III-17 | 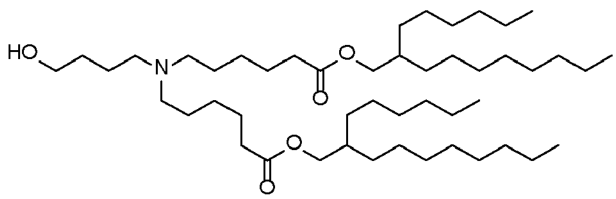 |

(continued)

| No. | Structure |
|---|---|
| III-18 | 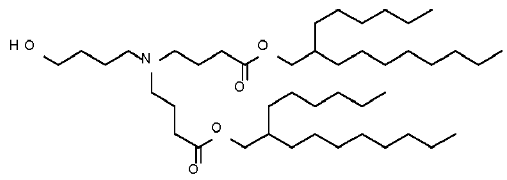 |
| III-19 | 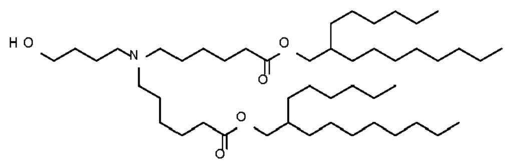 |
| III-20 | 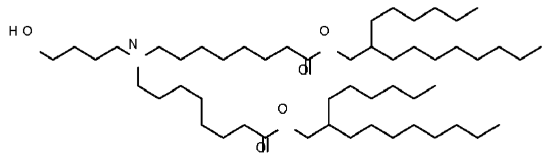 |
| III-21 | 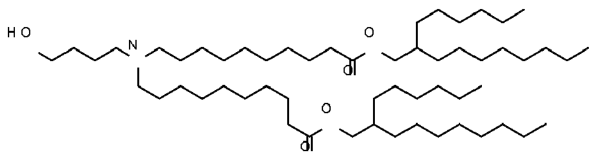 |
| III-22 | 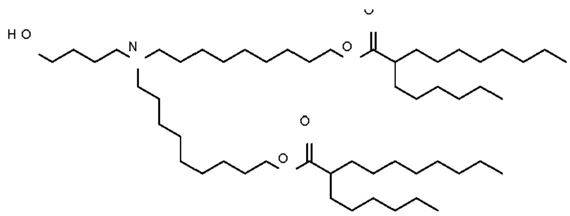 |
| III-23 | 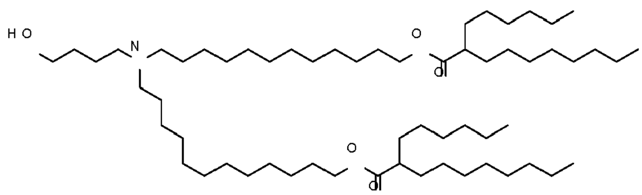 |
| III-24 | 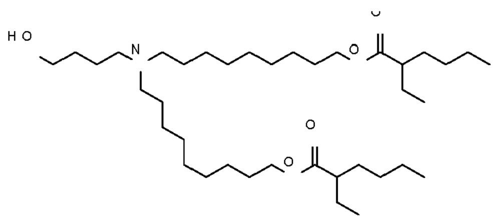 |

(continued)

| No. | Structure |
|---|---|
| III-25 | 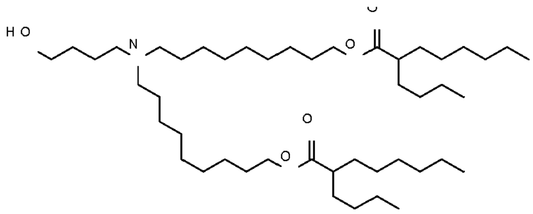 |
| III-26 | 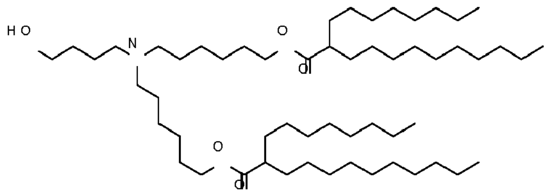 |
| III-27 | 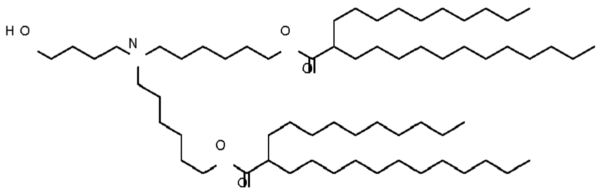 |
| III-28 | 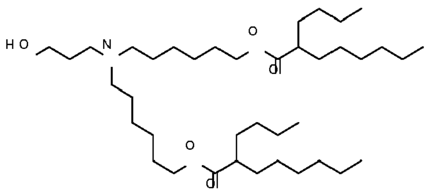 |
| III-29 | 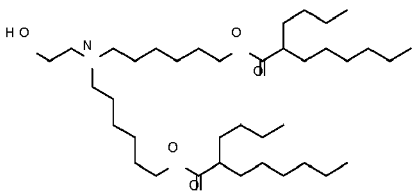 |
| III-30 | 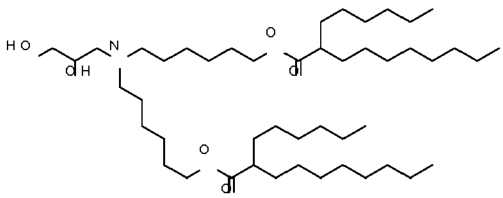 |
| III-31 | 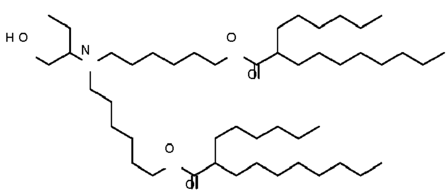 |

(continued)

| No. | Structure |
|---|---|
| III-32 | 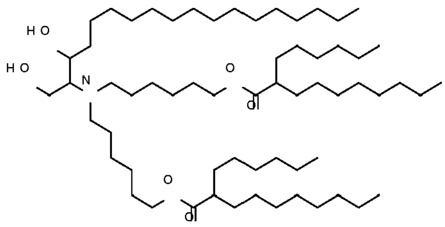 |
| III-33 | 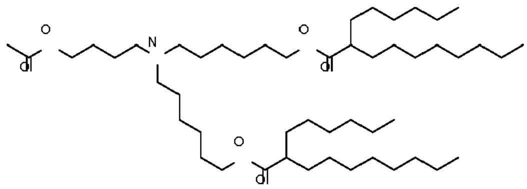 |
| III-34 | 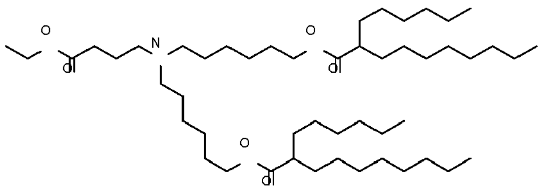 |
| III-35 | 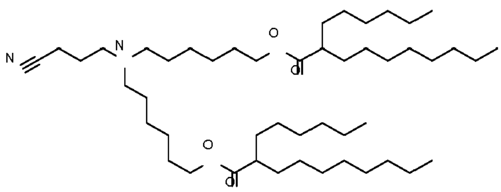 |
| III-36 | 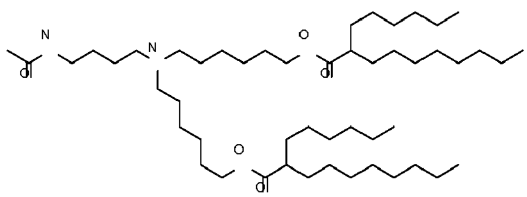 |
| III-37 | 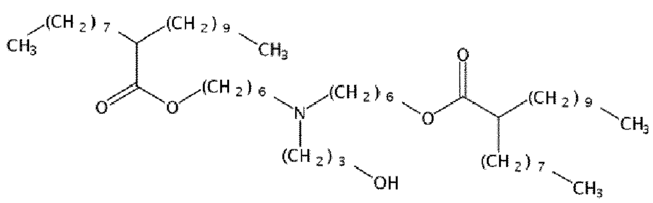 |
| III-38 | 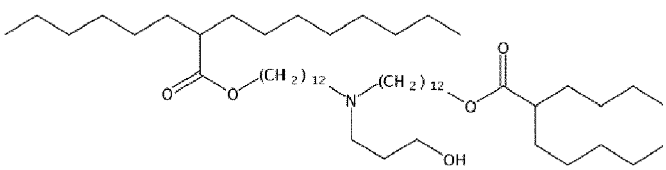 |

(continued)

| No. | Structure |
|---|---|
| III-39 | 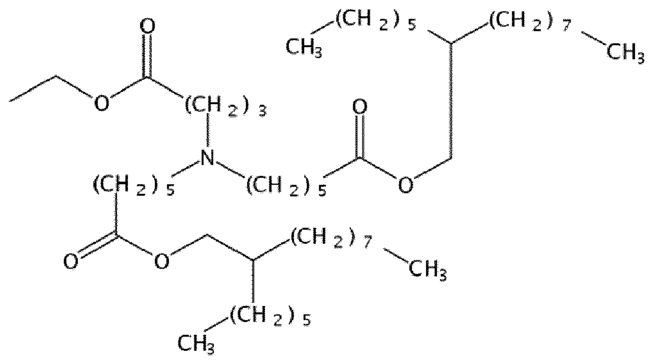 |
| III-40 | 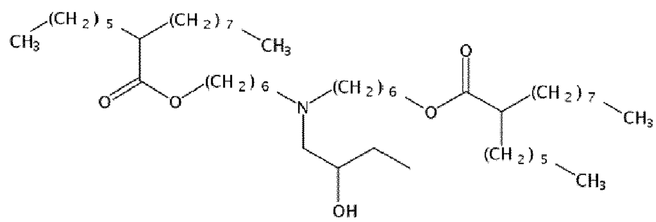 |
| III-41 | 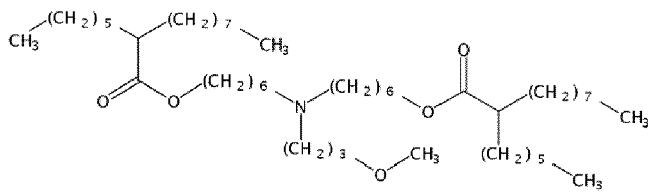 |
| III-42 | 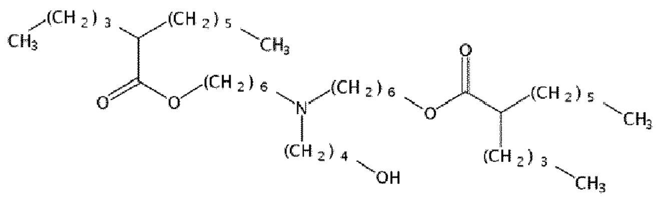 |
| III-43 | 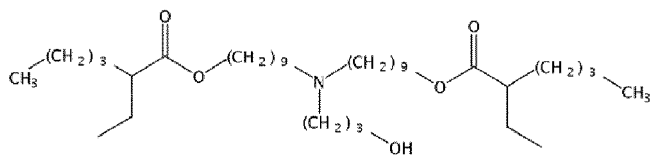 |
| III-44 | 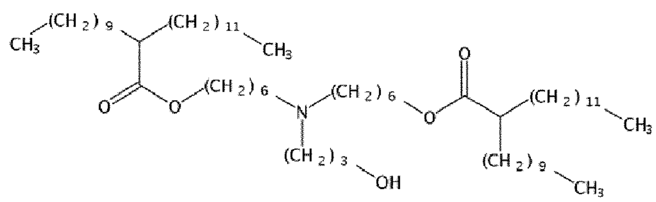 |

(continued)

| No. | Structure |
|---|---|
| III-45 | 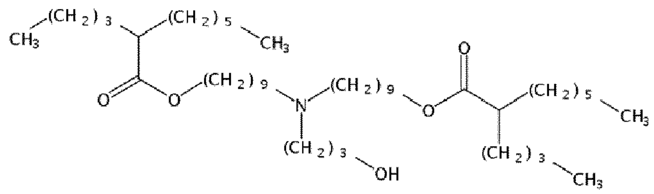 |
| III-46 | 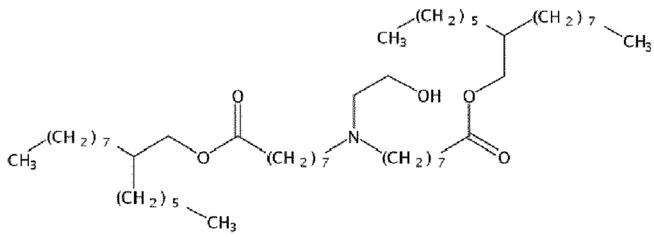 |
| III-47 | 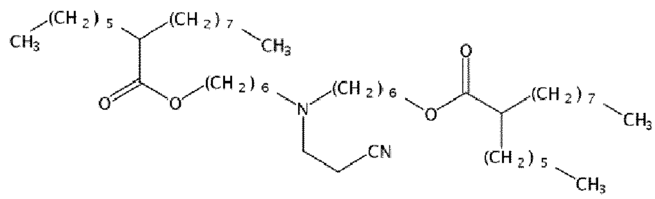 |
| III-48 | 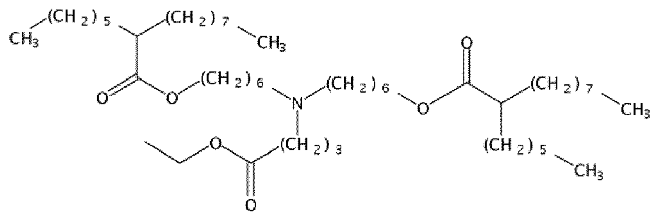 |
| III-49 | 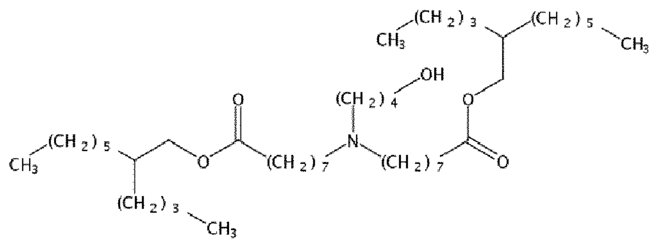 |

Further representative cationic lipids are as follows:

| No. | Structure |
|---|---|
| A | 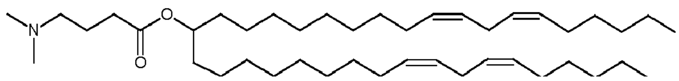 |

(continued)

| No. | Structure |
|---|---|
| B | 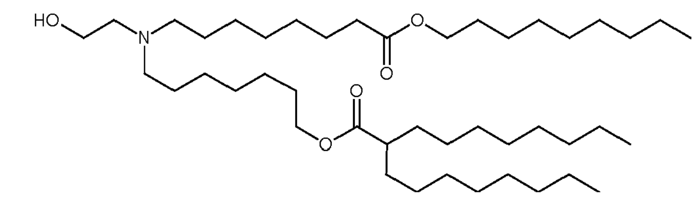 |
| C | 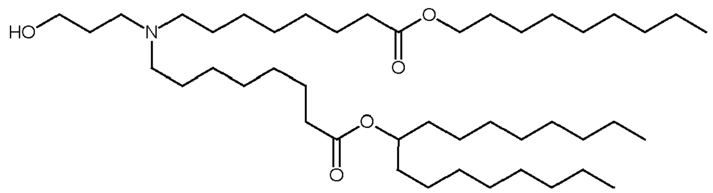 |
| D | 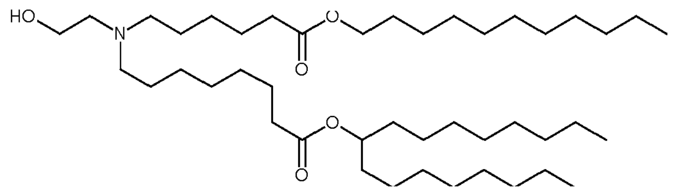 |
| E | 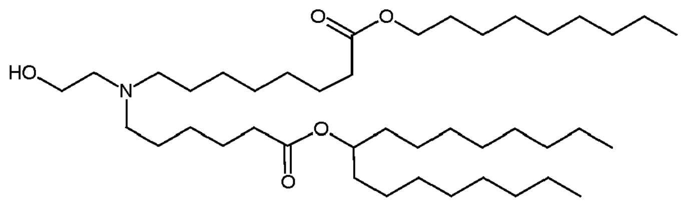 |
| F | 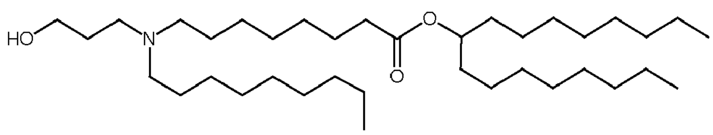 |

In some embodiments, the LNP comprises a cationic lipid shown in the above table, e.g., a cationic lipid of Formula (B) or Formula (D), in particular a cationic lipid of Formula (D), RNA, a neutral lipid, a steroid and a pegylated lipid. In some embodiments, the neutral lipid is DSPC. In some embodiments, the steroid is cholesterol. In some embodiments, the pegylated lipid is DMG-PEG 2000.

Various lipids (including, e.g., cationic lipids, neutral lipids, and polymer-conjugated lipids) are known in the art and can be used herein to form lipid nanoparticles, e.g., lipid nanoparticles targeting a specific cell type (e.g., liver cells). In some embodiments, a neutral lipid may be or comprise a phospholipid or derivative thereof (e.g., 1,2-Distearoyl-sn-glycero-3-phosphocholine (DPSC)) and/or cholesterol. In some embodiments, a polymer-conjugated lipid may be a PEG-conjugated lipid (e.g., 2-[(polyethylene glycol)-2000]-N,N-ditetradecylacetamide or a derivative thereof).

In some embodiments, the LNP comprises a lipid of Formula (III), RNA, a neutral lipid, a steroid and a pegylated lipid. In some embodiments, the neutral lipid is DSPC. In some embodiments, the steroid is cholesterol. In some embodiments, the pegylated lipid is ALC-0159.

**ALC-0159:**

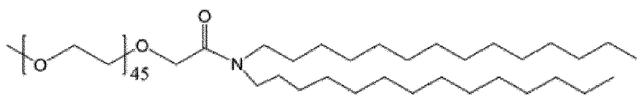

In some embodiments, RNA described herein is formulated in lipid nanoparticles (LNP). In one embodiment, the LNP comprise a cationic lipid, a neutral lipid, a steroid, a polymer conjugated lipid; and the RNA. In one embodiment, the cationic lipid is ALC-0315, the neutral lipid is DSPC, the steroid is cholesterol, and the polymer conjugated lipid is ALC-0159. The preferred mode of administration is intramuscular administration, more preferably in aqueous cryoprotectant buffer for intramuscular administration. The drug product is a preferably a preservative-free, sterile dispersion of RNA formulated in lipid nanoparticles (LNP) in aqueous cryoprotectant buffer for intramuscular administration.

ALC-0315 = ((4-hydroxybutyl)azanediyl)bis(hexane-6,1-diyl)bis(2-hexyldecanoate) / 6-[N-6-(2-hexyldecanoyloxy)hexyl-N-(4-hydroxybutyl)amino]hexyl 2-hexyldecanoate

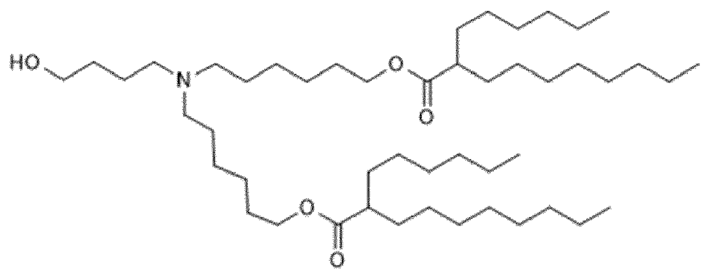

ALC-0159 = 2-[(polyethylene glycol)-2000]-*N*,*N*-ditetradecylacetamide / 2-[2-(ω-methoxy (polyethyleneglycol2000) ethoxy]-N,N-ditetradecylacetamide

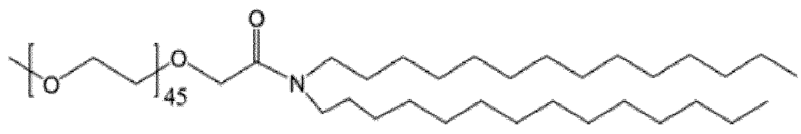

DSPC = 1,2-Distearoyl-sn-glycero-3-phosphocholine

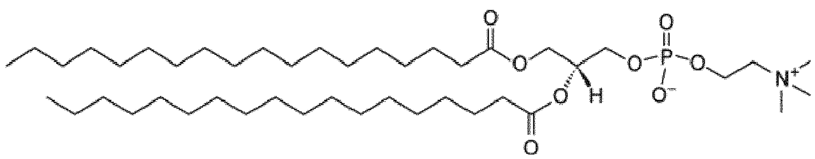

**Cholesterol:**

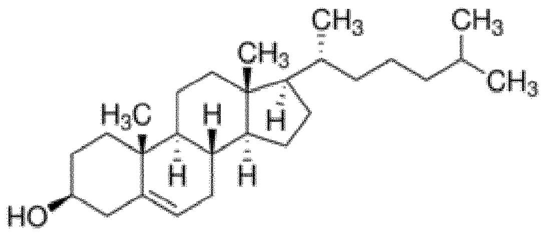

In one embodiment, the ratio of mRNA to total lipid (N/P) is between 6.0 and 6.5 such as about 6.0 or about 6.3.
In some embodiments, the cationic lipid is present in the LNP in an amount from about 45 to about 50 mole percent. In some embodiments, the neutral lipid is present in the LNP in an amount from about 5 to about 15 mole percent. In some embodiments, the steroid is present in the LNP in an amount from about 35 to about 45 mole percent. In some embodiments, the pegylated lipid is present in the LNP in an amount from about 1 to about 5 mole percent.
In some embodiments, the LNP comprises a cationic lipid in an amount from about 45 to about 50 mole percent, DSPC in an amount from about 5 to about 15 mole percent, cholesterol in an amount from about 35 to about 45 mole percent, and ALC-0159 in an amount from about 1 to about 5 mole percent.
The N/P value is preferably at least about 4. In some embodiments, the N/P value ranges from 4 to 20, 4 to 12, 4 to 10, 4 to 8, or 5 to 7. In some embodiments, the N/P value is about 6.

**Doses**

The term "dose" as used herein refers in general to a "dose amount" which relates to the amount of RNA administered per administration, i.e., per dosing.
In some embodiments, administration of RNA of the present invention may be performed by single administration or boosted by multiple administrations.
In some embodiments, an amount the RNA described herein from 0.1 μg to 300 μg, 0.5 μg to 200 μg, or 1 μg to 100 μg, such as about 1 μg, about 3 μg, about 10 μg, about 30 μg, about 50 μg, or about 100 μg may be administered per dose.
In some embodiments, a regimen described herein includes at least one dose. In some embodiments, a regimen includes a first dose and at least one subsequent dose. In some embodiments, the first dose is the same amount as at least one subsequent dose. In some embodiments, the first dose is the same amount as all subsequent doses. In some embodiments, the first dose is a different amount as at least one subsequent dose. In some embodiments, the first dose is a different amount than all subsequent doses. In some embodiments, a regimen comprises two doses. In some embodiments, a provided regimen consists of two doses. In some embodiments, a regimen comprises three doses.
In one embodiment, the invention envisions administration of a single dose. In one embodiment, the invention envisions administration of a priming dose followed by one or more booster doses. The booster dose or the first booster dose may be administered 7 to 28 days or 14 to 24 days following administration of the priming dose. In some embodiments, a first booster dose may be administered 1 week to 3 months (e.g., 1 week, 2 weeks, 3 weeks, 4 weeks, 5 weeks, 6 weeks, 7 weeks, 8 weeks, 9 weeks, 10 weeks, 11 weeks, 12 weeks) following administration of a priming dose. In some embodiments, a subsequent booster dose may be administered at least 1 week or longer, including, e.g., at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, or longer, following a preceding booster dose. In some embodiments, subsequent booster doses may be administered about 5-9 weeks or 6-8 weeks apart. In some embodiments, at least one subsequent booster dose (e.g., after a first booster dose) may be administered at least 3 months or longer, including, e.g., at least 4 months, at least 5 months, at least 6 months, at least 7 months, at least 8 months, at least 9 months, at least 10 months, or longer, following a preceding dose.
In some embodiments, a subsequent dose given to an individual (e.g., as part of a primary regimen or booster regimen) can have the same amount of RNA as previously given to the individual. In some embodiments, a subsequent dose given to an individual (e.g., as part of a primary regimen or booster regimen) can differ in the amount of RNA, as compared to the amount previously given to the individual. For example, in some embodiments, a subsequent dose can be higher or lower than the prior dose, for example, based on consideration of various factors, including, e.g., immunogenicity and/or reactogenicity induced by the prior dose, prevalence of the disease, etc. In some embodiments, a subsequent dose can be higher than a prior dose by at least 30%, at least 40%, at least 50%, at least 60%, at least 70%, at least 80%, at least 90%, or higher. In some embodiments, a subsequent dose can be higher than a prior dose by at least 1.5-fold, at least 2-fold, at least 2.5 fold, at least 3-fold, or higher. In some embodiments, a subsequent dose can be higher than a prior dose by at least 30%, at least 40%, at least 50%, at least 60%, at least 70%, at least 80%, at least 90%, or higher. In some embodiments, a subsequent dose can be lower than a prior dose by at least 10%, at least 20%,

at least 30%, at least 40%, at least 50%, at least 60%, at least 70% or lower.

In some embodiments, an amount the RNA described herein from 0.1 μg to 300 μg, 0.5 μg to 200 μg, or 1 μg to 100 μg, such as about 1 μg, about 2 μg, about 3 μg, about 10 μg, about 15 μg, about 20 μg, about 25 μg, about 30 μg, about 35 μg, about 40 μg, about 45 μg, about 50 μg, about 55 μg, about 60 μg, about 70 μg, about 80 μg, about 90 μg, or about 100 μg may be administered per dose (e.g., in a given dose).

In some embodiments, an amount of the RNA described herein of 60 μg or lower, 55 μg or lower, 50 μg or lower, 45 μg or lower, 40 μg or lower, 35 μg or lower, 30 μg or lower, 25 μg or lower, 20 μg or lower, 15 μg or lower, 10 μg or lower, 5 μg or lower, 3 ng or lower, 2.5 μg or lower, or 1 μg or lower may be administered per dose (e.g., in a given dose).

In some embodiments, an amount of the RNA described herein of at least 0.25 μg, at least 0.5 μg, at least 1 μg, at least 2 μg, at least 3 μg, at least 4 μg, at least 5 μg, at least 10 μg, at least 15 μg, at least 20 μg, at least 25 μg, at least 30 μg, at least 40 μg, at least 50 μg, or at least 60 μg may be administered per dose (e.g., in a given dose). In some embodiments, an amount of the RNA described herein of at least 3 ug may be administered in at least one of given doses. In some embodiments, an amount of the RNA described herein of at least 10 ug may be administered in at least one of given doses. In some embodiments, an amount of the RNA described herein of at least 15 ug may be administered in at least one of given doses. In some embodiments, an amount of the RNA described herein of at least 20 ug may be administered in at least one of given doses. In some embodiments, an amount of the RNA described herein of at least 25 ug may be administered in at least one of given doses. In some embodiments, an amount of the RNA described herein of at least 30 ug may be administered in at least one of given doses. In some embodiments, an amount of the RNA described herein of at least 50 ug may be administered in at least one of given doses. In some embodiments, an amount of the RNA described herein of at least 60 ug may be administered in at least one of given doses. In some embodiments, combinations of aforementioned amounts may be administered in a regimen comprising two or more doses (e.g., a prior dose and a subsequent dose can be of different amounts as described herein). In some embodiments, combinations of aforementioned amounts may be administered in a primary regimen and a booster regimen (e.g., different doses can be given in a primary regimen and a booster regimen).

In some embodiments, an amount of the RNA described herein of 0.25 μg to 60 μg, 0.5 μg to 55 μg, 1 μg to 50 μg, 5 μg to 40 μg, or 10 μg to 30 μg may be administered per dose. In some embodiments, an amount of the RNA described herein of 3 μg to 30 μg may be administered in at least one of given doses. In some embodiments, an amount of the RNA described herein of 3 μg to 20 μg may be administered in at least one of given doses. In some embodiments, an amount of the RNA described herein of 3 μg to 15 μg may be administered in at least one of given doses. In some embodiments, an amount of the RNA described herein of 3 μg to 10 μg may be administered in at least one of given doses. In some embodiments, an amount of the RNA described herein of 10 μg to 30 μg may be administered in at least one of given doses. In some embodiments, a regimen administered to a subject may comprise a plurality of doses (e.g., at least two doses, at least three doses, or more). In some embodiments, a regimen administered to a subject may comprise a first dose and a second dose, which are given at least 2 weeks apart, at least 3 weeks apart, at least 4 weeks apart, or more. In some embodiments, such doses may be at least 1 month, at least 2 months, at least 3 months, at least 4 months, at least 5 months, at least 6 months, at least 7 months, at least 8 months, at least 9 months, at least 10 months, at least 11 months, at least 12 months, or more apart. In some embodiments, doses may be administered days apart, such as 1, 2, 3, 4, 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more days apart. In some embodiments, doses may be administered about 1 to about 3 weeks apart, or about 1 to about 4 weeks apart, or about 1 to about 5 weeks apart, or about 1 to about 6 weeks apart, or about 1 to more than 6 weeks apart. In some embodiments, doses may be separated by a period of about 7 to about 60 days, such as for example about 14 to about 48 days, etc. In some embodiments, a minimum number of days between doses may be about 1, 2, 3, 4, 5, 6, 7, 8, 9, 10, 11, 12, 13, 14, 15, 16, 17, 18, 19, 20, 21 or more. In some embodiments, a maximum number of days between doses may be about 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, or fewer. In some embodiments, doses may be about 21 to about 28 days apart. In some embodiments, doses may be about 19 to about 42 days apart. In some embodiments, doses may be about 7 to about 28 days apart. In some embodiments, doses may be about 14 to about 24 days. In some embodiments, doses may be about 21 to about 42 days.

In some embodiments, a vaccination regimen comprises a first dose and a second dose. In some embodiments, a first dose and a second dose are administered by at least 21 days apart. In some embodiments, a first dose and a second dose are administered by at least 28 days apart.

In some embodiments, a vaccination regimen comprises at least two doses, including, e.g., at least three doses, at least four doses or more. In some embodiments, a vaccination regimen comprises three doses. In some embodiments, the time interval between the first dose and the second dose can be the same as the time interval between the second dose and the third dose. In some embodiments, the time interval between the first dose and the second dose can be longer than the time interval between the second dose and the third dose, e.g., by days or weeks (including, e.g., at

least 3 days, at least 4 days, at least 5 days, at least 6 days, at least 1 week, at least 2 weeks, at least 3 weeks, at least 4 weeks, at least 5 weeks, at least 6 weeks, or longer). In some embodiments, the time interval between the first dose and the second dose can be shorter than the time interval between the second dose and the third dose, e.g., by days or weeks (including, e.g., at least 3 days, at least 4 days, at least 5 days, at least 6 days, at least 1 week, at least 2 weeks, at least 3 weeks, at least 4 weeks, at least 5 weeks, at least 6 weeks, or longer). In some embodiments, the time interval between the first dose and the second dose can be shorter than the time interval between the second dose and the third dose, e.g., by at least 1 month (including, e.g., at least 2 months, at least 3 months, at least 4 months, at least 5 months, at least 6 months, at least 7 months, at least 8 months, at least 9 months, at least 10 months, at least 11 months, at least 12 months, or longer).

In some embodiments, a last dose of a primary regimen and a first dose of a booster regimen are given at least 2 months, at least 3 months, at least 4 months, at least 5 months, at least 6 months, at least 7 months, at least 8 months, at least 9 months, at least 10 months, at least 11 months, at least 12 months, or more apart. In some embodiments, a primary regimen may comprises two doses. In some embodiments, a primary regimen may comprises three doses.

In some embodiments, a first dose and a second dose (and/or other subsequent dose) may be administered by intramuscular injection. In some embodiments, a first dose and a second dose (and/or other subsequent dose) may be administered in the deltoid muscle. In some embodiments, a first dose and a second dose (and/or other subsequent dose) may be administered in the same arm.

In some embodiments, an mRNA composition described herein is administered (e.g., by intramuscular injection) as a series of two doses (e.g., 0.3 mL each) 21 days apart. In some embodiments, an mRNA composition described herein is administered (e.g., by intramuscular injection) as a series of two doses (e.g., 0.2 mL each) 21 days apart. In some embodiments, an mRNA composition described herein is administered (e.g., by intramuscular injection) as a series of three doses (e.g., 0.3 mL or lower including, e.g., 0.2 mL), wherein doses are given at least 3 weeks apart. In some embodiments, the first and second doses may be administered 3 weeks apart, while the second and third doses may be administered at a longer time interval than that between the first and the second doses, e.g., at least 4 weeks apart or longer (including, at least 5 weeks, at least 6 weeks, at least 7 weeks, at least 8 weeks, at least 9 weeks, or longer). In some embodiments, each dose is about 60 ug. In some embodiments, each dose is about 50 ug. In some embodiments, each dose is about 30 ug. In some embodiments, each dose is about 25 ug. In some embodiments, each dose is about 20 ug. In some embodiments, each dose is about 15 ug. In some embodiments, each dose is about 10 ug. In some embodiments, each dose is about 3 ug.

In some embodiments, each dose is about 30 ug. In some embodiments, each dose is about 20 ug. In some embodiments, each dose is about 15 ug. In some embodiments, each dose is about 10 ug. In some embodiments, each dose is about 3 ug.

In some embodiments, at least one dose given in a vaccination regimen (e.g., a primary vaccination regimen and/or a booster vaccination regimen) is about 60 ug. In some embodiments, at least one dose given in a vaccination regimen (e.g., a primary vaccination regimen and/or a booster vaccination regimen) is about 50 ug. In some embodiments, at least one dose given in a vaccination regimen (e.g., a primary vaccination regimen and/or a booster vaccination regimen) is about 30 ug. In some embodiments, at least one dose given in a vaccination regimen (e.g., a primary vaccination regimen and/or a booster vaccination regimen) is about 25 ug. In some embodiments, at least one dose given in a vaccination regimen (e.g., a primary vaccination regimen and/or a booster vaccination regimen) is about 20 ug. In some embodiments, at least one dose given in a vaccination regimen (e.g., a primary vaccination regimen and/or a booster vaccination regimen) is about 15 ug. In some embodiments, at least one dose given in a vaccination regimen (e.g., a primary vaccination regimen and/or a booster vaccination regimen) is about 10 ug. In some embodiments, at least one dose given in a vaccination regimen (e.g., a primary vaccination regimen and/or a booster vaccination regimen) is about 3 ug.

In some such embodiments, an mRNA composition described herein is administered to subjects of age 12 or older. In some such embodiments, an mRNA composition described herein is administered to subjects of age 12 to 18. In some such embodiments, an mRNA composition described herein is administered to subjects of age 5 to 11. In some such embodiments, an mRNA composition described herein is administered to subjects of age 2 to less than 5. In some such embodiments, an mRNA composition described herein is administered to subjects of less than 2 years old, for example, 6 months to less than 2 years old. In some such embodiments, an mRNA composition described herein is administered to subjects of less than 6 months old, for example, 1 month to less than 4 months old.

In some such embodiments, an mRNA composition described herein is administered to subjects of age 12 or older and each dose is about 30 ug. In some embodiments, an mRNA composition described herein is administered to subjects of age 12 or older (including, e.g., age 18 or older) and each dose is higher than 30 ug, including, e.g., 35 ug, 40 ug, 45 ug, 50 ug, 55 ug, 60 ug, 65 ug , 70 ug, or higher. In some such embodiments, an mRNA composition described herein is administered to subjects of age 12 or older and each dose is about 60 ug. In some such embodiments, an mRNA composition described herein is administered to subjects of age 12 or older and each dose is about 50 ug. In some such embodiments, an mRNA composition described herein is administered to subjects of age 5 to 11 and each

dose is about 10 ug. In some such embodiments, an mRNA composition described herein is administered to subjects of age 2 to less than 5 and each dose is about 3 ug.

In one embodiment, an amount of the RNA described herein of about 60 μg is administered per dose. In one embodiment, an amount of the RNA described herein of about 50 μg is administered per dose. In one embodiment, an amount of the RNA described herein of about 30 μg is administered per dose. In one embodiment, an amount of the RNA described herein of about 25 μg is administered per dose. In one embodiment, an amount of the RNA described herein of about 20 μg is administered per dose. In one embodiment, an amount of the RNA described herein of about 15 μg is administered per dose. In one embodiment, an amount of the RNA described herein of about 10 μg is administered per dose. In one embodiment, an amount of the RNA described herein of about 5 μg is administered per dose. In one embodiment, an amount of the RNA described herein of about 3 μg is administered per dose. In one embodiment, at least two of such doses are administered. For example, a second dose may be administered about 21 days following administration of the first dose.

In some embodiments, an mRNA composition described herein is administered to subjects of age 12 or older and at least one dose given in a vaccination regimen (e.g., a primary vaccination regimen and/or a booster vaccination regimen) is about 60 ug. In some embodiments, an mRNA composition described herein is administered to subjects of age 12 or older and at least one dose given in a vaccination regimen (e.g., a primary vaccination regimen and/or a booster vaccination regimen) is about 30 ug. In some embodiments, an mRNA composition described herein is administered to subjects of age 12 or older and at least one dose given in a vaccination regimen (e.g., a primary vaccination regimen and/or a booster vaccination regimen) is about 15 ug. In some embodiments, an mRNA composition described herein is administered to subjects of age 5 to less than 12 years of age and at least one dose given in a vaccination regimen (e.g., a primary vaccination regimen and/or a booster vaccination regimen) is about 10 ug. In some embodiments, an mRNA composition described herein is administered to subjects of age 2 to less than 5 and at least one dose given in a vaccination regimen (e.g., a primary vaccination regimen and/or a booster vaccination regimen) is about 3 ug. In some embodiments, an mRNA composition described herein is administered to subjects of 6 months to less than age 2 and at least one dose given in a vaccination regimen (e.g., a primary vaccination regimen and/or a booster vaccination regimen) is about 3 ug or lower, including, e.g., 2 ug, 1 ug, or lower). In some embodiments, an mRNA composition described herein is administered to infants of less than 6 months and at least one dose given in a vaccination regimen (e.g., a primary vaccination regimen and/or a booster vaccination regimen) is about 3 ug or lower, including, e.g., 2 ug, 1 ug, 0.5 ug, or lower).

In some embodiments, an mRNA composition described herein is administered (e.g., by intramuscular injection) as a single dose. In some embodiments, a single dose comprise a single RNA encoding a SARS-CoV-2 S protein or an immunogenic fragment thereof (e.g., an RBD domain). In some embodiments, a single dose comprise at least two RNAs described herein, for example, each RNA encoding a SARS-CoV-2 S protein or an immunogenic fragment thereof (e.g., an RBD domain) from different strains. In some embodiments, such at least two RNAs described herein can be administered as a single mixture. For example, in some such embodiments, two separate RNA compositions described herein can be mixed to generate a single mixture prior to injection. In some embodiments, such at least two RNAs described herein can be administered as two separate compositions, which, for example, can be administered at different injection sites (e.g., on different arms, or different sites on the same arm).

In some embodiments, a dose administered to subjects in need thereof may comprise administration of a single mRNA composition described herein.

In some embodiments, a dose administered to subjects in need thereof may comprise administration of at least two or more (including, e.g., at least three or more) different drug products/formulations. For example, in some embodiments, at least two or more different drug products/formulations may comprise at least two different mRNA compositions described herein (e.g., in some embodiments each comprising a different RNA construct).

In some embodiments, an mRNA composition disclosed herein may be administered in conjunction with a vaccine targeting a different infectious agent. In some embodiments, the different infectious agent is one that increases the likelihood of a subject experiencing deleterious symptoms when coinfected with SARS-CoV-2 and the infectious agent. In some embodiments, the infectious agent is one that increases the infectivity of SARS-CoV-2 when a subject is coinfected with SARS-CoV-2 and the infectious agent.

In some embodiments, an RNA composition described herein is co-administered with one or more vaccines against a non-SARS-CoV-2 disease. In some embodiments, an RNA composition described herein is co-administered with one or more vaccines against a non-SARS-COV-2 viral disease. In some embodiments, an RNA composition described herein is co-administered with one or more vaccines against a non-SARS-CoV-2 respiratory disease. In some embodiments, the non-SARS-CoV-2 respiratory disease is a non-SARS-CoV-2 Coronavirus, an Influenza virus, a Pneumoviridae virus, or a Paramyxoviridae virus. In some embodiments, the Pneumoviridae virus is a Respiratory syncytial virus or a Metapneumovirus. In some embodiments, the Metapneumovirus is a human metapneumovirus (hMPV). In some embodiments, the Paramyxoviridae virus is a Parainfluenza virus or a Henipavirus. In some embodiments, the parainfluenzavirus is PIV3. In some embodiments, the non-SAR-CoV-2 coronavirus is a betacoronavirus (e.g., SARS-CoV-1). In come embodiments the non-SARS-CoV-2 coronavirus is a Merbecovirus (e.g., a MERS-CoV virus).

In some embodiments, an RNA composition described herein is co-administered with an RSV vaccine (e.g., an RSV A or RSV B vaccine). In some embodiments, the RSV vaccine comprises an RSV fusion protein (F), an RSV attachment protein (G), an RSV small hydrophobic protein (SH), an RSV matrix protein (M), an RSV nucleoprotein (N), an RSV M2-1 protein, an RSV Large polymerase (L), and/or an RSV phosphoprotein (P), or an immunogenic fragment of immunogenic variant thereof, or a nucleic acid (e.g., RNA), encoding any one of the same.

In some embodiments, an RNA composition described herein is co-administered with an influenza vaccine. In some embodiments, the influenza vaccine is an alphainfluenza virus, a betainfluenza virus, a gammainfluenza virus or a deltainfluenza virus vaccine. In some embodiments the vaccine is an Influenza A virus, an Influenza B virus, an Influenza C virus, or an Influenza D virus vaccine. In some embodiments, the influenza A virus vaccine comprises a hemagglutinin selected from H1, H2, H3, H4, H5, H6, H7, H8, H9, H10, H11, H12, H13, H14, H15, H16, H17, and H18, or an immunogenic fragment or variant of the same, or a nucleic acid (e.g., RNA) encoding any one of the same. In some embodiments the influenza A vaccine comprises or encodes a neuraminidase (NA) selected from N1, N2, N3, N4, N5, N6, N7, N8, N9, N10, and N11, or an immunogenic fragment or variant of the same, or a nucleic acid (e.g., RNA) encoding any one of the same. In some embodiments, the influenza vaccine comprises at least one Influenza virus hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, and/or polymerase basic protein 2 (PB2), or an immunogenic fragment or variant thereof, or a nucleic acid (e.g., RNA) encoding any of one of the same.

In some embodiments, an RNA composition provided herein and other injectable vaccine(s) 15 are administered at different times. In some embodiments, an RNA composition provided herein is administered at the same time as other injectable vaccine(s). In some such embodiments, an RNA composition provided herein and at least one another injectable vaccine(s) are administered at different injection sites. In some embodiments, an RNA composition provided herein is not mixed with any other vaccine in the same syringe. In some embodiments, an RNA composition provided herein is not combined with other coronavirus vaccines as part of vaccination against coronavirus, e.g., SARS-CoV-2.

In some embodiments, different drug products/ formulations are separately administered. In some embodiments, such different drug product/formulations are separately administered at the same time (e.g., at the same vaccination session) at different sites of a subject (e.g., at different arms of the subject).

In one embodiment, at least two doses are administered. For example, a second dose may be administered about 21 days following administration of the first dose.

In some embodiments, at least one single dose is administered. In some embodiments, such single dose is administered to subjects, for example, who may have previously received one or more doses of, or a complete regimen of, a SARS-CoV-2 vaccine (e.g., of a BNT162b2 vaccine [including, e.g., as described herein], an mRNA-1273 vaccine, an Ad26.CoV2.S vaccine, a ChAxOx1 vaccine, an NVX-CoV2373 vaccine, a CvnCoV vaccine, a GAM-COVID0Vac vaccine, a CoronaVac vaccine, a BBIBP-CorV vaccine, etc. Alternatively or additionally, in some embodiments, a single dose is administered to subjects who have been exposed to and/or infected by SARS-CoV-2. In some embodiments, at least one single dose is administered to subjects who both have received one or more doses of, or a complete regimen of, a SARS-CoV-2 vaccine and have been exposed to and/or infected with SARS-CoV-2.

In some embodiments, the formulations described herein comprise one or more T-cell epitopes of SARS-CoV-2 or RNA encoding the same. In some embodiments, a formulation comprises (i) one or more T-cell epitopes of SARS-CoV-2 or RNA comprising a nucleotide sequence encoding the same, and (ii) a SARS-CoV-2 S protein, an immunogenic variant thereof, or a fragment thereof, or RNA encoding the same (e.g., RNA described herein). In some embodiments, a formulation comprises one or more T-cell epitopes, or RNA encoding the same, derived from an M protein, an N protein, and/or an ORFlab protein of SARS-CoV-2, e.g., a composition disclosed in WO2021188969, the contents of which is incorporated by reference herein in its entirety. In some embodiments, a formulation comprising RNA described herein (e.g., RNA encoding a SARS-CoV-2 S protein comprising mutations characteristic of a BA.1, BA.2, or BA.4/5 Omicron variant, and optionally RNA encoding a SARS-CoV-2 S protein of a Wuhan variant) also comprises a T-string construct described in WO2021188969 (e.g., an RNA encoding SEQ ID NO: RS C7p2full of WO2021/188969). In some embodiments, a formulation comprises RNA described herein and a T-string construct described in WO2021188969 in a combination of up to about 100 ug RNA total. In some embodiments, subjects are administered with at least 2 doses of RNA described herein (e.g., in some embodiments at 30 ug each) in combination with a T-string construct (e.g., an RNA encoding SEQ ID NO: RS C7p2full of WO2021/188969), e.g., each dose of a combination of RNA described herein and an RNA encoding SEQ ID NO: RS C7p2full of up to about 100 ug RNA total, wherein the two doses are administered, for example, at least 4 weeks or longer (including, e.g., at least 5 weeks, at least 6 weeks, at least 7 weeks, at least 8 weeks, at least 9 weeks, at least 10 weeks, at least 11 weeks, or at least 12 weeks, or longer) apart from one another. In some embodiments, subjects are administered at least 3 doses of RNA described herein (e.g., in some embodiments at 30 ug each) in combination with a T-string construct (e.g., an RNA encoding SEQ ID NO: RS C7p2full of WO2021/188969), e.g., each dose of a combination of RNA described herein and an RNA encoding SEQ ID NO: RS

C7p2full of up to about 100 ug RNA total, wherein the first and the second doses and the second and third doses are each independently administered at least 4 weeks or longer (including, e.g., at least 5 weeks, at least 6 weeks, at least 7 weeks, at least 8 weeks, at least 9 weeks, at least 10 weeks, at least 11 weeks, or at least 12 weeks, or longer) apart from one another. In some embodiments, RNA comprising a nucleotide sequence encoding a SARS-CoV-2 S protein, a variant thereof, or a fragment thereof (e.g., as described herein) and a T-string construct may be co-administered as separate formulations (e.g., formulations administered on the same day to separate injection sites). In some embodiments, RNA comprising a nucleotide sequence encoding a SARS-CoV-2 S protein, a variant thereof, or a fragment thereof (e.g., as described herein) and a T-string construct may be coadministered as a co-formulation (e.g., a formulation comprising RNA comprising a nucleotide sequence encoding a SARS-CoV-2 S protein, a variant thereof, or a fragment thereof (e.g., as described herein) and a T-string construct as separate LNP formulations or as LNP formulations comprising both a T-string construct and RNA comprising a nucleotide sequence encoding a SARS-CoV-2 S protein, a variant thereof, or a fragment thereof).

In some particular embodiments where at least one single dose is administered to subjects who have received one or more doses of a prior SARS-CoV-2 vaccine, such prior SARS-CoV-2 vaccine is a different vaccine, or a different form (e.g., formulation) and/or dose of a vaccine with the same active (e.g., BNT162b2); in some such embodiments, such subjects have not received a complete regimen of such prior vaccine and/or have experienced one or more undesirable reactions to or effects of one or more received doses of such prior vaccine. In some particular embodiments, such prior vaccine is or comprises higher dose(s) of the same active (e.g., BNT162b2). Alternatively or additionally, in some such embodiments, such subjects were exposed to and/or infected by SARS-CoV-2 prior to completion (but, in some embodiments, after initiation) of a full regimen of such prior vaccine..

In one embodiment, at least two doses are administered. For example, a second dose may be administered about 21 days following administration of the first dose.

In one embodiment, at least three doses are administered. In some embodiments, such third dose is administered a period of time after the second dose that is comparable to (e.g., the same as) the period of time between the first and second doses. For example, in some embodiments, a third dose may be administered about 21 days following administration of the second dose. In some embodiments, a third dose is administered after a longer period of time relative to the second dose than the second dose was relative to the first dose. In some embodiments, a three-dose regimen is administered to an immunocomprised patient, e.g., a cancer patient, an HIV patient, a patient who has received and/or is receiving immunosuppressant therapy (e.g., an organ transplant patient).

In some embodiments, a vaccination regimen comprises administering the same amount of RNA in different doses (e.g., in first and/or second and/or third and/or subsequent doses). In some embodiments, a vaccination regimen comprises administering different amounts of RNA in different doses. In some embodiments, one or more later doses is larger than one or more earlier doses. (e.g., in situations where waning of vaccine efficacy from one or more earlier doses is observed and/or immune escape by a variant (e.g., one described herein) that is prevalent or rapidly spreading is observed in a relevant jurisdiction at the time of administration is observed). In some embodiments, one or more later doses may be larger than one or more earlier doses by at least 30%, at least 40%, at least 50%, at least 60%, at least 70%, at least 80%, at least 90%, at least 95%, or higher, provided that safety and/or tolerability of such a dose is clinically acceptable. In some embodiments, one or more later doses may be larger than one or more earlier doses by at least 1.1-fold, at least 1.5-fold, at least 2-fold, at least 3-fold, at least 4-fold, or higher provided that safety and/or tolerability of such a dose is clinically acceptable. In some embodiments, one or more later doses is smaller than one or more earlier doses (e.g., in a negative reaction was experienced after one or more earlier doses and/or if exposure to and/or infection by SARS-CoV-2 between an earlier dose and a subsequent dose). In some embodiments, one or more later doses may be smaller than one or more earlier doses by at least 10%, at least 20%, at least 30%, at least 40%, at least 50%, at least 60%, or higher. In some embodiments, where different doses are utilized, they are related to one another by identity with and/or dilution of a common stock as described herein.

In some embodiments, a vaccination regimen comprises a first dose and a second dose, wherein the amount of RNA administered in the first dose is the same as the amount of RNA administered in the second dose. In some embodiments, a vaccination regimen comprises a first dose and a second dose wherein the amount of RNA administered in the first dose differs from that administered in the second dose.

In some embodiments, a vaccination regimen comprises a first dose and a second dose, wherein the amount of RNA administered in the first dose is less than that administered in the second dose. In some embodiments, the amount of RNA administered in the first dose is 10%-90% of the second dose. In some embodiments, the amount of RNA administered in the first dose is 10%-50% of the second dose. In some embodiments, the amount of RNA administered in the first dose is 10%-20% of the second dose. In some embodiments, the first dose and the second dose are administered at least 2 weeks apart, including, at least 3 weeks apart, at least 4 weeks apart, at least 5 weeks apart, at least 6 weeks apart or longer. In some embodiments, the first dose and the second dose are administered at least 3 weeks apart.

In some embodiments, a first dose comprises less than about 30 ug of RNA and a second dose comprises at

least about 30 ug of RNA. In some embodiments, a first dose comprises about 1 to less than about 30 ug of RNA (e.g., about 0.1, about 1, about 3, about 5, about 10, about 15, about 20, about 25, or less than about 30 ug of RNA) and a second dose comprises about 30 to about 100 ug of RNA (e.g., about 30, about 40, about 50, or about 60 ug of RNA). In some embodiments, a first dose comprises about 1 to about 20 ug of RNA, about 1 to about 10 ug of RNA, or about 1 to about 5 ug of RNA and a second dose comprises about 30 to about 60 ug of RNA.

In some embodiments, a first dose comprises about 1 to about 10 ug of RNA (e.g., about 1, about 2, about 3, about 4, about 5, about 6, about 7, about 8, about 9, or about 10 ug of RNA) and a second dose comprises about 30 to about 60 ug of RNA (e.g., about 30, about 35, about 40, about 45, about 50, about 55, or about 60 ug of RNA).

In some embodiments, a first dose comprises about 1 ug of RNA and a second dose comprises about 30 ug of RNA. In some embodiments, a first dose comprises about 3 ug of RNA and a second dose comprises about 30 ug of RNA. In some embodiments, a first dose comprises about 5 ug of RNA and a second dose comprises about 30 ug of RNA. In some embodiments, a first dose comprises about 10 ug of RNA and a second dose comprises about 30 ug of RNA. In some embodiments, a first dose comprises about 15 ug of RNA and a second dose comprises about 30 ug of RNA.

In some embodiments, a first dose comprises about 1 ug of RNA and a second dose comprises about 60 ug of RNA. In some embodiments, a first dose comprises about 3 ug of RNA and a second dose comprises about 60 ug of RNA. In some embodiments, a first dose comprises about 5 ug of RNA and a second dose comprises about 60 ug of RNA. In some embodiments, a first dose comprises about 6 ug of RNA and a second dose comprises about 60 ug of RNA. In some embodiments, a first dose comprises about 10 ug of RNA and a second dose comprises about 60 ug of RNA. In some embodiments, a first dose comprises about 15 ug of RNA and a second dose comprises about 60 ug of RNA. In some embodiments, a first dose comprises about 20 ug of RNA and a second dose comprises about 60 ug of RNA. In some embodiments, a first dose comprises about 25 ug of RNA and a second dose comprises about 60 ug of RNA. In some embodiments, a first dose comprises about 30 ug of RNA and a second dose comprises about 60 ug of RNA.

In some embodiments, a first dose comprises less than about 10 ug of RNA and a second dose comprises at least about 10 ug of RNA. In some embodiments, a first dose comprises about 0.1 to less than about 10 ug of RNA (e.g., about 0.1, about 0.5, about 1, about 2, about 3, about 4, about 5, about 6, about 7, about 8, or less than about 10 ug of RNA) and a second dose comprises about 10 to about 30 ug of RNA (e.g., about 10, about 15, about 20, about 25, or about 30 ug of RNA). In some embodiments, a first dose comprises about 0.1 to about 10 ug of RNA, about 1 to about 5 ug of RNA, or about 0.1 to about 3 ug of RNA and a second dose comprises about 10 to about 30 ug of RNA.

In some embodiments, a first dose comprises about 0.1 to about 5 ug of RNA (e.g., about 0.1, about 0.5, about 1, about 2, about 3, about 4, about 5 ug of RNA) and a second dose comprises about 10 to about 20 ug of RNA (e.g., about 10, about 12, about 14, about 16, about 18, about 20 ug of RNA).

In some embodiments, a first dose comprises about 0.1 ug of RNA and a second dose comprises about 10 ug of RNA. In some embodiments, a first dose comprises about 0.3 ug of RNA and a second dose comprises about 10 ug of RNA. In some embodiments, a first dose comprises about 1 ug of RNA and a second dose comprises about 10 ug of RNA. In some embodiments, a first dose comprises about 3 ug of RNA and a second dose comprises about 10 ug of RNA.

In some embodiments, a first dose comprises less than about 3 ug of RNA and a second dose comprises at least about 3 ug of RNA. In some embodiments, a first dose comprises about 0.1 to less than about 3 ug of RNA (e.g., about 0.1, about 0.2, about 0.3, about 0.5, about 0.6, about 0.7, about 0.8, about 0.9, about 1.0, about 1.5, about 2.0, or about 2.5 ug of RNA) and a second dose comprises about 3 to about 10 ug of RNA (e.g., about 3, about 4, about 5, about 6, or about 7, about 8, about 9, or about 10 ug of RNA). In some embodiments, a first dose comprises about 0.1 to about 3 ug of RNA, about 0.1 to about 1 ug of RNA, or about 0.1 to about 0.5 ug of RNA and a second dose comprises about 3 to about 10 ug of RNA.

In some embodiments, a first dose comprises about 0.1 to about 1.0 ug of RNA (e.g., about 0.1, about 0.2, about 0.3, about 0.4, about 0.5, about 0.6, about 0.7, about 0.8, about 0.9, or about 1.0 ug of RNA) and a second dose comprises about 1 to about 3 ug of RNA (e.g., about 1.0, about 1.5, about 2.0, about 2.5, or about 3.0 ug of RNA).

In some embodiments, a first dose comprises about 0.1 ug of RNA and a second dose comprises about 3 ug of RNA. In some embodiments, a first dose comprises about 0.3 ug of RNA and a second dose comprises about 3 ug of RNA. In some embodiments, a first dose comprises about 0.5 ug of RNA and a second dose comprises about 3 ug of RNA. In some embodiments, a first dose comprises about 1 ug of RNA and a second dose comprises about 3 ug of RNA.

In some embodiments, a vaccination regimen comprises a first dose and a second dose, wherein the amount of RNA administered in the first dose is greater than that administered in the second dose. In some embodiments, the amount of RNA administered in the second dose is 10%-90% of the first dose. In some embodiments, the amount of RNA administered in the second dose is 10%-50% of the first dose. In some embodiments, the amount of RNA administered in the second dose is 10%-20% of the first dose. In some embodiments, the first dose and the second dose are administered at least 2 weeks apart, including, at least 3 weeks apart, at least 4 weeks apart, at least 5 weeks apart, at

least 6 weeks apart or longer. In some embodiments, the first dose and the second dose are administered at least 3 weeks apart

In some embodiments, a first dose comprises at least about 30 ug of RNA and a second dose comprises less than about 30 ug of RNA. In some embodiments, a first dose comprises about 30 to about 100 ug of RNA (e.g., about 30, about 40, about 50, or about 60 ug of RNA) and a second dose comprises about 1 to about 30 ug of RNA (e.g., about 0.1, about 1, about 3, about 5, about 10, about 15, about 20, about 25, or about 30 ug of RNA). In some embodiments, a second dose comprises about 1 to about 20 ug of RNA, about 1 to about 10 ug of RNA, or about 1 to 5 ug of RNA. In some embodiments, a first dose comprises about 30 to about 60 ug of RNA and a second dose comprises about 1 to about 20 ug of RNA, about 1 to about 10 ug of RNA, or about 0.1 to about 3 ug of RNA.

In some embodiments, a first dose comprises about 30 to about 60 ug of RNA (e.g., about 30, about 35, about 40, about 45, about 50, about 55, or about 60 ug of RNA) and a second dose comprises about 1 to about 10 ug of RNA (e.g., about 1, about 2, about 3, about 4, about 5, about 6, about 7, about 8, about 9, or about 10 ug of RNA).

In some embodiments, a first dose comprises about 30 ug of RNA and a second dose comprises about 1 ug of RNA. In some embodiments, a first dose comprises about 30 ug of RNA and a second dose comprises about 3 ug of RNA. In some embodiments, a first dose comprises about 30 ug of RNA and a second dose comprises about 5 ug of RNA. In some embodiments, a first dose comprises about 30 ug of RNA and a second dose comprises about 10 ug of RNA. In some embodiments, a first dose comprises about 30 ug of RNA and a second dose comprises about 15 ug of RNA.

In some embodiments, a first dose comprises about 60 ug of RNA and a second dose comprises about 1 ug of RNA. In some embodiments, a first dose comprises about 60 ug of RNA and a second dose comprises about 3 ug of RNA. In some embodiments, a first dose comprises about 60 ug of RNA and a second dose comprises about 5 ug of RNA. In some embodiments, a first dose comprises about 60 ug of RNA and a second dose comprises about 6 ug of RNA. In some embodiments, a first dose comprises about 60 ug of RNA and a second dose comprises about 10 ug of RNA. In some embodiments, a first dose comprises about 60 ug of RNA and a second dose comprises about 15 ug of RNA. In some embodiments, a first dose comprises about 60 ug of RNA and a second dose comprises about 20 ug of RNA. In some embodiments, a first dose comprises about 60 ug of RNA and a second dose comprises about 25 ug of RNA. In some embodiments, a first dose comprises about 60 ug of RNA and a second dose comprises about 30 ug of RNA.

In some embodiments, a first dose comprises at least about 10 ug of RNA and a second dose comprises less than about 10 ug of RNA. In some embodiments, a first dose comprises about 10 to about 30 ug of RNA (e.g., about 10, about 15, about 20, about 25, or about 30 ug of RNA) and a second dose comprises about 0.1 to less than about 10 ug of RNA (e.g., about 0.1, about 0.5, about 1, about 2, about 3, about 4, about 5, about 6, about 7, about 8, or less than about 10 ug of RNA). In some embodiments, a first dose comprises about 10 to about 30 ug of RNA, or about 0.1 to about 3 ug of RNA and a second dose comprises about 1 to about 10 ug of RNA, or about 1 to about 5 ug of RNA.

In some embodiments, a first dose comprises about 10 to about 20 ug of RNA (e.g., about 10, about 12, about 14, about 16, about 18, about 20 ug of RNA) and a second dose comprises about 0.1 to about 5 ug of RNA (e.g., about 0.1, about 0.5, about 1, about 2, about 3, about 4, or about 5 ug of RNA).

In some embodiments, a first dose comprises about 10 ug of RNA and a second dose comprises about 0.1 ug of RNA. In some embodiments, a first dose comprises about 10 ug of RNA and a second dose comprises about 0.3 ug of RNA. In some embodiments, a first dose comprises about 10 ug of RNA and a second dose comprises about 1 ug of RNA. In some embodiments, a first dose comprises about 10 ug of RNA and a second dose comprises about 3 ug of RNA.

In some embodiments, a first dose comprises at least about 3 ug of RNA and a second dose comprises less than about 3 ug of RNA. In some embodiments, a first dose comprises about 3 to about 10 ug of RNA (e.g., about 3, about 4, about 5, about 6, or about 7, about 8, about 9, or about 10 ug of RNA) and a second dose comprises 0.1 to less than about 3 ug of RNA (e.g., about 0.1, about 0.2, about 0.3, about 0.5, about 0.6, about 0.7, about 0.8, about 0.9, about 1.0, about 1.5 about 2.0, or about 2.5 ug of RNA). In some embodiments, a first dose comprises about 3 to about 10 ug of RNA and a second dose comprises about 0.1 to about 3 ug of RNA, about 0.1 to about 1 ug of RNA, or about 0.1 to about 0.5 ug of RNA.

In some embodiments, a first dose comprises about 1 to about 3 ug of RNA (e.g., about 1, about 1.5, about 2.0, about 2.5, or about 3.0 ug of RNA) and a second dose comprises about 0.1 to 0.3 ug of RNA (e.g., about 0.1, about 0.2, about 0.3, about 0.4, about 0.5, about 0.6, about 0.7, about 0.8, about 0.9, or about 1.0 ug of RNA).

In some embodiments, a first dose comprises about 3 ug of RNA and a second dose comprises about 0.1 ug of RNA. In some embodiments, a first dose comprises about 3 ug of RNA and a second dose comprises about 0.3 ug of RNA. In some embodiments, a first dose comprises about 3 ug of RNA and a second dose comprises about 0.6 ug of RNA. In some embodiments, a first dose comprises about 3 ug of RNA and a second dose comprises about 1 ug of RNA.

In some embodiments, where at least two or more doses are administered (e.g., at least two doses administered in a primary regimen, at least two doses administered in a booster regimen, or at least one dose administered in a

primary regimen and at least one dose in a booster regimen), the same RNA compositions described herein may be administered in such doses and each of such doses can be the same or different (as described herein). In some embodiments, where at least two or more doses are administered (e.g., at least two doses administered in a primary regimen, at least two doses administered in a booster regimen, or at least one dose administered in a primary regimen and at least one dose in a booster regimen), different RNA compositions described herein (e.g., different encoded viral polypeptides, e.g., from different coronavirus clades, or from different strains of the same coronavirus clade; different construct elements such as 5' cap, 3' UTR, 5' UTR, etc.; different formulations, e.g., different excipients and/or buffers (e.g., PBS vs. Tris); different LNP compositions; or combinations thereof) may be administered in such doses and each of such doses can be the same or different (e.g., as described herein).

In some embodiments, a subject is administered two or more RNAs (e.g., as part of either a primary regimen or a booster regimen), wherein the two or more RNAs are administered on the same day or same visit. In some embodiments, the two or more RNAs are administered in separate compositions, e.g., by administering each RNA to a separate part of the subject (e.g., by intramuscular administration to different arms of the subject or to different sites of the same arm of the subject). In some embodiments, the two or more RNAs are mixed prior to administration (e.g., mixed immediately prior to administration, e.g., by the administering practitioner). In some embodiments, the two or more RNAs comprise an RNA that encode a coronavirus S protein or immunogenic fragment thereof (e.g., RBD or other relevant domains) from one strain (e.g., Wuhan strain) and a variant that is prevalent or rapidly spreading in a relevant jurisdiction at the time of administration (e.g., a variant described herein). In some embodiments, such a variant is an Omicron variant (e.g., a BA.1, BA.2, BA.4/5, BA.4.6, BA.2.75, BA.2.75.1, or XBB variant).

In some embodiments, a vaccination regimen comprises a first vaccination regimen (e.g., a primary regimen) that includes at least two doses of an RNA composition as described herein, e.g., wherein the second dose may be administered about 21 days following administration of the first dose, and a second vaccination (e.g., a booster regimen) that comprises a single dose or multiple doses, e.g., two doses, of an RNA composition as described herein. In some embodiments, doses of a booster regimen are related to those of a primary regimen by identity with or dilution from a common stock as described herein. In various embodiments, a booster regimen is administered (e.g., is initiated) at least 1 week, at least 2 weeks, at least 3 weeks, at least 1 month, at least 2 months, at least 3 months, at least 4 months, at least 5 months, at least 6 months, at least 7 months, at least 8 months, at least 9 months, at least 10 months, at least 11 months, at least 12 months, or longer, after administration of a primary regimen, e.g., after completion of a primary regimen comprising at least two doses. In various embodiments, a booster regimen is administered (e.g., is initiated) 1-12 months, 2-12 months, 3-12 months, 4-12 months, 6-12 months, 1-6 months, 1-5 months, 1-4 months, 1-3 months, or 2-3 months after administration of a primary regimen, e.g., after completion of a primary regimen comprising at least two doses. In various embodiments, a booster regimen is administered (e.g., is initiated) 1 to 60 months, 2 to 48 months, 2 to 24 months, 3 to 24 months, 6 to 18 months, 6 to 12 months, or 5 to 7 months after administration of a primary regimen, e.g., after completion of a two-dose primary regimen.. In some embodiments, each dose of a primary regimen is about 60 μg per dose. In some embodiments, each dose of a primary regimen is about 50 μg per dose. In some embodiments, each dose of a primary regimen is about 30 μg per dose. In some embodiments, each dose of a primary regimen is about 25 μg per dose. In some embodiments, each dose of a primary regimen is about 20 μg per dose. In some embodiments, each dose of a primary regimen is about 15 μg per dose. In some embodiments, each dose of a primary regimen is about 10 μg per dose. In some embodiments, each dose of a primary regimen is about 3 μg per dose. In some embodiments, each dose of a booster regimen is the same as that of the primary regimen. In some embodiments, each dose of a booster regimen comprises the same amount of RNA as a dose administered in a primary regimen. In some embodiments, at least one dose of a booster regimen is the same as that of the primary regimen. In some embodiments, at least one dose of a booster regimen comprises the same amount of RNA as at least one dose of a primary regimen. In some embodiments, at least one dose of a booster regimen is lower than that of the primary regimen. In some embodiments, at least one dose of a booster regimen comprises an amount of RNA that is lower than that of a primary regimen. In some embodiments, at least one dose of a booster regimen is higher than that of the primary regimen. In some embodiments, at least one dose of a booster regimen comprises an amount of RNA that is higher than that of a primary regimen.

In some embodiments, a booster regimen (e.g., as described herein) is administered to a pediatric patient (e.g., a patient aged 2 through 5 years old, a patient aged 5 through 11 years old, or a patient aged 12 through 15 years old). In some embodiments, a booster regimen is administered to a pediatric patient who is 6 months old to less than 2 years old. In some embodiments, a booster regimen is administered to a pediatric patient who is less than 6 months old. In some embodiments, a booster regimen is administered to a pediatric patient who is 6 months old to less than 5 years old. In some embodiments, a booster regimen is administered to a pediatric patient who is 2 years old to less than 5 years old. In some embodiments, a booster regimen is administered to a pediatric patient who is 5 years old to less than 12 years old. In some embodiments, a booster regimen is administered to a pediatric patient who is 12 years old to less than 16 years old. In some embodiments, each dose of a pediatric booster regimen comprises about 3 μg of RNA. In some embodiments, each dose of a pediatric booster regimen comprises about 10 μg of RNA. In some embodiments,

each dose of a pediatric booster regimen comprises about 15 μg of RNA. In some embodiments, each dose of a pediatric booster regimen comprises about 20 μg of RNA. In some embodiments, each dose of a pediatric booster regimen comprises about 25 μg of RNA. In some embodiments, each dose of a pediatric booster regimen comprises about 30 μg of RNA. In some embodiments, a booster regimen is administered to a non-pediatric patient (e.g., a patient 16 years or older, a patient aged 18 through 64 years old, and/or a patient 65 years and older). In some embodiments, each dose of a non-pediatric booster regimen comprises about 3 ug of RNA, about 10 ug of RNA, about 25 μg or RNA, about 30 μg of RNA, about 40 μg of RNA, about 45 μg of RNA, about 50 μg of RNA, about 55 μg of RNA, or about 60 μg of RNA. In some embodiments, the same booster regimen may be administered to both pediatric and non-pediatric patients (e.g., to a patient 12 years or older). In some embodiments, a booster regimen that is administered to a non-pediatric patient is administered in a formulation and dose that is related to that of a primary regimen previously received by the patient by identity with or by dilution as described herein. In some embodiments, a non-pediatric patient who receives a booster regimen at a lower dose than a primary regimen may have experienced an adverse reaction to one or more doses of such primary regimen and/or may have been exposed to and/or infected by SARS-CoV-2 between such primary regimen and such booster regimen, or between doses of such primary regimen and/or of such booster regimen. In some embodiments, pediatric and non-pediatric patients may receive a booster regimen at a higher dose than a primary regimen when waning of vaccine efficacy at lower doses is observed, and/or when immune escape of a variant that is prevalent and/or spreading rapidly at a relevant jurisdiction at the time of administration is observed.

In some embodiments one or more doses of a booster regimen differs from that of a primary regimen. For example, in some embodiments, an administered dose may correspond to a subject's age and a patient may age out of one treatment age group and into a next. Alternatively or additionally, in some embodiments, an administered dose may correspond to a patient's condition (e.g., immunocompromised state) and a different dose may be selected for one or more doses of a booster regimen than for a primary regimen (e.g., due to intervening cancer treatment, infection with HIV, receipt of immunosuppressive therapy, for example associated with an organ transplant. In some embodiments, at least one dose of a booster regimen may comprise an amount of RNA that is higher than at least one dose administered in a primary regimen (e.g., in situations where waning of vaccine efficacy from one or more earlier doses is observed and/or immune escape by a variant (e.g., one described herein) that is prevalent or rapidly spreading is observed in a relevant jurisdiction at the time of administration).

In some embodiments, a primary regimen may involve one or more 3 ug doses and a booster regimen may involve one or more 10 ug doses, and/or one or more 20 ug doses, or one or more 30 ug doses. In some embodiments, a primary regimen may involve one or more 3 ug doses and a booster regimen may involve one or more 3 ug doses. In some embodiments, a primary regimen may involve two or more 3 ug doses (e.g., at least two doses, each comprising 3 ug of RNA, and administered about 21 days after one another) and a booster regimen may involve one or more 3 ug doses. In some embodiments, a primary regimen may involve one or more 10 ug doses and a booster regimen may involve one or more 20 ug doses, and/or one or more 30 ug doses. In some embodiments, a primary regimen may involve one or more 10 ug doses and a booster regimen may involve one or more 10 ug doses. In some embodiments, a primary regimen may involve two or more 10 ug doses (e.g., two doses, each comprising 10 ug of RNA, administered about 21 days apart) and a booster regimen may involve one or more 10 ug doses. In some embodiments, a primary regimen may involve one or more 20 ug doses and a booster regimen may involve one or more 30 ug doses. In some embodiments, a primary regimen may involve one or more 20 ug doses and a booster regimen may involve one or more 20 ug doses. In some embodiments, a primary regimen may involve one or more 30 ug doses, and a booster regimen may also involve one or more 30 ug doses. In some embodiments, a primary regimen may involve two or more 30 ug doses (e.g., two doses, each comprising 30 ug of RNA, administered about 21 days apart), and a booster regimen may also involve one or more 30 ug doses. In some embodiments, a primary regimen may involve two or more 30 ug doses (e.g., two doses, each comprising 30 ug of RNA, administered about 21 days apart), and a booster regimen may involve one or more 50 ug doses. In some embodiments, a primary regimen may involve two or more 30 ug doses (e.g., two doses, each comprising 30 ug of RNA, administered about 21 days apart), and a booster regimen may involve one or more 60 ug doses.

In some embodiments, a patient is administered a primary regimen comprising two 30 ug doses, administered approximately 21 days apart, and a booster regimen comprising at least one 60 ug dose of RNA. In some embodiments, a patient is administered a primary regimen comprising two 30 ug doses, administered approximately 21 days apart, and a booster regimen comprising at least one 50 ug dose of RNA.

In some embodiments, a primary regimen may involve one or more 30 ug doses and a booster regimen may involve one or more 20 ug doses, one or more 10 ug doses, and/or one or more 3 ug doses. In some embodiments, a primary regimen may involve one or more 20 ug doses and a booster regimen may involve one or more 10 ug doses, and/or one or more 3 ug doses. In some embodiments, a primary regimen may involve one or more 10 ug doses and a booster regimen may involve one or more 3ug doses. In some embodiments, a primary regimen may involve one or more 3 ug doses, and a booster regimen may also involve one or more 3 ug doses.

In some embodiments, a booster regimen comprises a single dose, e.g., for patients who experienced an

adverse reaction while receiving the primary regimen.

In some embodiments, the same RNA as used in a primary regimen is used in a booster regimen. In some embodiment, an RNA used in primary and booster regimens is BNT162b2. In some embodiments, a different RNA is used in a booster regimen relative to that used in a primary regimen administered to the same subject. In some embodiments, BNT162b2 is used in a primary regimen but not in a booster regimen. In some embodiments, BNT162b2 is used in a booster regimen but not in a primary regimen. In some embodiments, a similar BNT162b2 construct can be used in a primary regimen and in a booster regimen, except that the RNA constructs used in the primary and booster regimens encode a SARS-CoV-2 S protein (or an immunogenic portion thereof) of different SARS-CoV-2 strains (e.g., as described herein).

In some embodiments, where BNT162b2 is used for a primary regimen or a booster regimen but not both, and a different RNA is used in the other, such different RNA may be an RNA encoding the same SARS-CoV-2 S protein but with different codon optimization or other different RNA sequence. In some embodiments, such different RNA may encode a SARS-CoV-2 S protein (or an immunogenic portion thereof) of a different SARS-CoV-2 strain, e.g., of a variant strain discussed herein. In some such embodiments, such variant strain that is prevalent or rapidly spreading in a relevant jurisdiction. In some embodiments, such different RNA may be an RNA encoding a SARS-CoV-2 S protein or variant thereof (or immunogenic portion of either) comprising one or more mutations described herein for S protein variants such as SARS-CoV-2 S protein variants, in particular naturally occurring S protein variants; in some such embodiments, a SARS-CoV-2 variant may be selected from the group consisting of VOC-202012/01, 501.V2, Cluster 5 and B.1.1.248. In some embodiments, a SARS-CoV-2 variant may be selected from the group consisting of VOC-202012/01, 501.V2, Cluster 5 and B.1.1.248, B.1.1.7, B.1.617.2, and B.1.1.529. In some embodiments, a booster regimen comprises at least one dose of RNA that encodes a SARS-CoV-2 S protein (or an immunogenic fragment thereof) of a variant that is spreading rapidly in a relevant jurisdiction at the time of administration. In some such embodiments, a variant that is encoded by RNA administered in a booster regimen may be different from that encoded by RNA administered in a primary regimen. In some embodiments, a booster regimen comprises (i) a dose of RNA encoding the same SARS-CoV-2 S protein (or an immunogenic fragment thereof) as the RNA administered in the primary regimen (e.g., an RNA encoding a SARS-CoV-2 S protein (or an immunogenic fragment thereof) from the SARS-CoV-2 Wuhan strain) and (ii) a dose of RNA encoding a SARS-CoV-2 S protein (or an immunogenic fragment thereof) of a variant that is spreading rapidly in a relevant jurisdiction at the time of administration (e.g., a SARS-CoV-2 S protein (or an immunogenic fragment thereof) from one of SARS-CoV-2 variants discussed herein).

In some embodiments, a booster regimen comprises multiple doses (e.g., at least two doses, at least three doses, or more). For example, in some embodiments, a first dose of a booster regimen may comprise RNA encoding the same SARS-CoV-2 S protein (or an immunogenic fragment thereof) administered in the primary regimen and a second dose of a booster regimen may comprise RNA encoding a SARS-CoV-2 S protein of a variant that is spreading rapidly in a relevant jurisdiction at the time of administration. In some embodiments, a first dose of a booster regimen may comprise RNA encoding a SARS-CoV-2 S protein (or an immunogenic fragment thereof) of a variant that is spreading rapidly in a relevant jurisdiction at the time of administration and a second dose of a booster regimen may comprise RNA encoding the same SARS-CoV-2 S protein (or an immunogenic fragment thereof) administered in the primary regimen.

In some embodiments, doses (e.g., a first and a second dose or any two consecutive doses) in a booster regimen are administered at least 2 weeks apart, including, e.g., at least 3 weeks, at least 4 weeks, at least 5 weeks, at least 6 weeks, at least 7 week, at least 8 weeks, at least 9 weeks, at least 10 weeks, at least 11 weeks, at least 12 weeks, at least 13 weeks, at least 14 weeks, at least 15 weeks, at least 16 weeks, or longer, apart. In some embodiments, doses (e.g., a first and a second dose or any two consecutive doses) in a booster regimen are administered approximately 2 to 16 weeks apart. In some embodiments, doses (e.g., a first and a second dose or any two consecutive doses) in a booster regimen are administered approximately 3 to 12 weeks apart. In some embodiments, doses (e.g., a first and a second dose or any two consecutive doses) in a booster regimen are administered approximately 4 to 10 weeks apart. In some embodiments, doses (e.g., a first and a second dose or any two consecutive doses) in a booster regimen are administered approximately 6 to 8 weeks apart. In some embodiments, multiple booster regimens may be administered. In some embodiments, a booster regimen is administered to a patient who has previously been administered a booster regimen.

In some embodiments, a second booster regimen is administered to a patient who has previously received a first booster regimen, and the amount of RNA administered in at least one dose of a second booster regimen is higher than the amount of RNA administered in at least one dose of a first booster regimen.

In some embodiments, a second booster regimen comprises administering at least one dose of 3 ug of RNA. In some embodiments, a second booster regimen comprises administering at least one dose of 5 ug of RNA. In some embodiments, a second booster regimen comprises administering at least one dose of 10 ug of RNA. In some embodiments, a second booster regimen comprises administering at least one dose of 15 ug of RNA. In some embodiments, a second booster regimen comprises administering at least one dose of 20 ug of RNA. In some embodiments, a second

booster regimen comprises administering at least one dose of 25 ug of RNA. In some embodiments, a second booster regimen comprises administering at least one dose of 50 ug of RNA. In some embodiments, a second booster regimen comprises administering at least one dose of 60 ug of RNA.

In some embodiments, a subject is administered a primary regimen that comprises two doses of 30 ug of RNA, administered approximately 21 days apart, and a booster regimen comprising at least one dose of approximately 30 ug of RNA. In some embodiments, a subject is administered a primary regimen that comprises two doses of 30 ug of RNA, administered approximately 21 days apart, and a booster regimen comprising at least one dose of approximately 50 ug of RNA. In some embodiments, a subject is administered a primary regimen that comprises two doses of 30 ug of RNA, administered approximately 21 days apart, and a booster regimen comprising at least one dose of approximately 60 ug of RNA.

In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 30 ug of RNA. In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 50 ug of RNA. In some embodiments, a subject is administered a primary regimen that comprises two doses of 30 ug of RNA, administered approximately 21 days apart, a first booster regimen comprising at least one dose of approximately 30 ug of RNA, and a second booster regimen comprising at least one dose of approximately 60 ug of RNA. In some embodiments, a first booster regimen comprises two doses of RNA, wherein each dose comprises an RNA encoding a Spike protein from a different SARS-CoV-2 variant. In some embodiments, a first booster regimen comprises two doses of RNA, wherein each dose comprises an RNA encoding a Spike protein from a different SARS-CoV-2 variant, and wherein the two doses of RNA are administered on the same day. In some embodiments, the two doses of RNA are administered in a single composition (e.g., by mixing a first composition comprising an RNA encoding a Spike protein from a first SARS-CoV-2 variant with a second composition comprising an RNA encoding a Spike protein from a second SARS-CoV-2 variant).

In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a variant that is prevalent and/or rapidly spreading in a relevant jurisdiction at the time of administering the booster regimen, wherein the first dose and the second dose of RNA may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from an alpha variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a beta variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from a delta variant of SARS-CoV-2, wherein the first dose and the second dose may be administered on the same day. In some embodiments, a subject is administered a booster regimen comprising a first dose comprising an RNA that encodes a Spike protein from a Wuhan strain of SARS-CoV-2 and a second dose comprising an RNA that encodes a Spike protein comprising mutations from an omicron variant of SARS-CoV-2 (e.g., a BA.1, BA.2, BA.4/5, BA.2.75, BA.2.75.1, and/or BA.4.6 variant), wherein the first dose and the second dose may be administered on the same day. Such booster regimens may be administered, e.g., to a subject previously administered a primary dosing regimen and/or to a subject previously administered a primary dosing regimen and a booster regimen.

In some embodiments, a subject is administered a first booster regimen comprising a first dose of 15 ug of RNA encoding a Spike protein from a Wuhan variant and a second dose of 15 ug of RNA encoding a Spike protein from an Omicron variant of SARS-CoV-2 (e.g., a BA.1, BA.2, BA.4/5, BA.2.75, BA.2.75.2, BA.4.6, or XBB variant), where the first and the second dose are administered on the same day (e.g., wherein compositions comprising the RNA are mixed prior to administration, and the mixture is then administered to a patient). In some embodiments, a subject is administered a first booster regimen comprising a first dose of 25 ug of RNA encoding a Spike protein from a Wuhan variant and a second dose of 25 ug of RNA encoding a Spike protein from an Omicron variant of SARS-CoV-2 (e.g., a BA.1, BA.2, BA.4/5, BA.2.75, BA.2.75.2, BA.4.6, or XBB variant). In some embodiments, a subject is administered a first booster regimen comprising a first dose of 25 ug of RNA encoding a Spike protein from a Wuhan variant and a second dose of 25 ug of RNA encoding a Spike protein from an Omicron variant of SARS-CoV-2 (e.g., a BA.1, BA.2, BA.4/5, BA.2.75, BA.4.6, BA.2.75.2, or XBB variant). In some embodiments, such a first booster regimen is administered to a subject previously administered a primary regimen comprising two doses of 30 ug of RNA, administered about 21 days apart wherein the first booster regimen is administered at least 3 months (e.g., at least 4, at least 5, or at least 6 months) after

administration of a primary regimen.

In some embodiments, a subject is administered a second booster regimen comprising a first dose of 15 ug of RNA encoding a Spike protein from a Wuhan variant and a second dose of 15 ug of RNA encoding a Spike protein from an Omicron variant of SARS-CoV-2 (e.g., a BA.1, BA.2, BA.4/5, BA.2.75, BA.2.75.2, BA.4.6, or XBB variant), where the first and the second dose are administered on the same day (e.g., wherein compositions comprising the RNA are mixed prior to administration, and the mixture is then administered to a patient). In some embodiments, a subject is administered a second booster regimen comprising a first dose of 25 ug of RNA encoding a Spike protein from a Wuhan variant and a second dose of 25 ug of RNA encoding a Spike protein from an Omicron variant of SARS-CoV-2 (e.g., a BA.1, BA.2, BA.4/5, BA.2.75, BA.2.75.2, BA.4.6, or XBB variant). In some embodiments, a subject is administered a second booster regimen comprising a first dose of 25 ug of RNA encoding a Spike protein from a Wuhan variant and a second dose of 25 ug of RNA encoding a Spike protein from an Omicron variant of SARS-CoV-2 (e.g., a BA.1, BA.2, BA.4/5, BA.2.75, BA.2.75.2, BA.4.6, or XBB variant). In some embodiments, such a second booster regimen is administered to a subject previously administered a primary regimen comprising two doses of 30 ug of RNA, administered about 21 days apart. In some embodiments, such a second booster regimen is administered to a subject previously administered a primary regimen comprising two doses of 30 ug of RNA, administered about 21 days apart, and a first booster regimen comprising a dose of 30 ug of RNA, wherein the second booster regimen is administered at least 3 months (e.g., at least 4, at least 5, or at least 6 months) after administration of a first booster regimen.

In some embodiments, an RNA used for a primary regimen or for a booster regimen (but not for both)encodes a polypeptide comprising an amino acid sequence with proline residue substitutions at positions 986 and 987 of SEQ ID NO:1 and an RNA used for the other regimen encodes a polypeptide comprising an amino acid sequence with alanine substitution at position 80, glycine substitution at position 215, lysine substitution at position 484, tyrosine substitution at position 501, valine substitution at position 701, phenylalanine substitution at position 18, isoleucine substitution at position 246, asparagine substitution at position 417, glycine substitution at position 614, deletions at positions 242 to 244, and proline substitutions at positions 986 and 987 of SEQ ID NO:1.

In some embodiments, patients receiving dose(s) of RNA compositions as described herein are monitored for one or more particular conditions, e.g., following administration of one or more doses. In some embodiments, such condition(s) may be or comprise allergic reaction(s) (particularly in subject(s) with a history of relevant allergies or allergic reactions), myocarditis (inflammation of the heart muscle, particularly where the subject is a young male and/or may have experienced prior such inflammation), pericarditis (inflammation of the lining outside the heart, particularly where the subject is a young males and/or may have experienced prior such inflammation), fever, bleeding (particularly where the subject is known to have a bleeding disorder or to be receiving therapy with a blood thinner). Alternatively or additionally, patients who may receive closer monitoring may be or include patients who are immunocompromised or are receiving therapy with a medicine that affects the immune system, are pregnant or planning to become pregnant, are breastfeeding, have received another COVID-19 vaccine, and/or have ever fainted in association with an injection. In some embodiments, patients are monitored for myocarditis following administration of one of the compositions disclosed herein. In some embodiments, patients are monitored for pericarditis following administration of one of the compositions disclosed herein. Patients may be monitored and/or treated for the condition using current standards of care.

In some embodiments, a vaccination regimen (e.g., a primary vaccination regimen and/or a booster dose regimen) comprises a dose (e.g., a first dose or a subsequent dose) of an RNA construct (e.g., as described herein) formulated in a Tris buffered solution and a dose (e.g., a first dose or a subsequent dose) of an RNA construct (e.g., as described herein) formulated in a PBS buffered solution. In some embodiments, a PBS buffered formulation comprises potassium chloride, potassium dihydrogen phosphate, sodium chloride, disodium phosphate dihydrate, sucrose and water for injection. In some embodiments, a PBS buffered formulation further comprises ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), 1,2-Distearoyl-sn-glycero-3-phosphocholine (DSPC), and cholesterol. In some embodiments, the PBS-buffered solution is one of the formulations described in WO2021213924A1, the contents of which are incorporated by reference in their entirety.

## Additional treatments

In certain embodiments, additional treatments may be administered to a patient in combination with the treatments described herein. Such additional treatments include classical cancer therapy, e.g., radiation therapy, surgery, hyperthermia therapy and/or chemotherapy. Furthermore, such additional treatments include treatments involving immune checkpoint modulators.

Chemotherapy is a type of cancer treatment that uses one or more anti-cancer drugs (chemotherapeutic agents), usually as part of a standardized chemotherapy regimen. The term chemotherapy has come to connote non-specific usage of intracellular poisons to inhibit mitosis. The connotation excludes more selective agents that block extracellular signals (signal transduction). The development of therapies with specific molecular or genetic targets, which inhibit

growth-promoting signals from classic endocrine hormones (primarily estrogens for breast cancer and androgens for prostate cancer) are now called hormonal therapies. By contrast, other inhibitions of growth-signals like those associated with receptor tyrosine kinases are referred to as targeted therapy.

Importantly, the use of drugs (whether chemotherapy, hormonal therapy or targeted therapy) constitutes systemic therapy for cancer in that they are introduced into the blood stream and are therefore in principle able to address cancer at any anatomic location in the body. Systemic therapy is often used in conjunction with other modalities that constitute local therapy (i.e. treatments whose efficacy is confined to the anatomic area where they are applied) for cancer such as radiation therapy, surgery or hyperthermia therapy.

Traditional chemotherapeutic agents are cytotoxic by means of interfering with cell division (mitosis) but cancer cells vary widely in their susceptibility to these agents. To a large extent, chemotherapy can be thought of as a way to damage or stress cells, which may then lead to cell death if apoptosis is initiated.

Chemotherapeutic agents include alkylating agents, antimetabolites, anti-microtubule agents, topoisomerase inhibitors, and cytotoxic antibiotics.

Alkylating agents have the ability to alkylate many molecules, including proteins, RNA and DNA. The subtypes of alkylating agents are the nitrogen mustards, nitrosoureas, tetrazines, aziridines, cisplatins and derivatives, and non-classical alkylating agents. Nitrogen mustards include mechlorethamine, cyclophosphamide, melphalan, chlorambucil, ifosfamide and busulfan. Nitrosoureas include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide. Aziridines include thiotepa, mytomycin and diaziquone (AZQ). Cisplatin and derivatives include cisplatin, carboplatin and oxaliplatin. They impair cell function by forming covalent bonds with the amino, carboxyl, sulfhydryl, and phosphate groups in biologically important molecules. Non-classical alkylating agents include procarbazine and hexamethylmelamine. In one particularly preferred embodiment, the alkylating agent is cyclophosphamide.

Anti-metabolites are a group of molecules that impede DNA and RNA synthesis. Many of them have a similar structure to the building blocks of DNA and RNA. Anti-metabolites resemble either nucleobases or nucleosides, but have altered chemical groups. These drugs exert their effect by either blocking the enzymes required for DNA synthesis or becoming incorporated into DNA or RNA. Subtypes of the anti-metabolites are the anti-folates, fluoropyrimidines, deoxynucleoside analogues and thiopurines. The anti-folates include methotrexate and pemetrexed. The fluoropyrimidines include fluorouracil and capecitabine. The deoxynucleoside analogues include cytarabine, gemcitabine, decitabine, azacitidine, fludarabine, nelarabine, cladribine, clofarabine, and pentostatin. The thiopurines include thioguanine and mercaptopurine.

Anti-microtubule agents block cell division by preventing microtubule function. The vinca alkaloids prevent the formation of the microtubules, whereas the taxanes prevent the microtubule disassembly. Vinca alkaloids include vinorelbine, vindesine, and vinflunine. Taxanes include docetaxel (Taxotere) and paclitaxel (Taxol).

Topoisomerase inhibitors are drugs that affect the activity of two enzymes: topoisomerase I and topoisomerase II and include irinotecan, topotecan, camptothecin, etoposide, doxorubicin, mitoxantrone, teniposide, novobiocin, merbarone, and aclarubicin.

The cytotoxic antibiotics are a varied group of drugs that have various mechanisms of action. The common theme that they share in their chemotherapy indication is that they interrupt cell division. The most important subgroup is the anthracyclines (e.g., doxorubicin, daunorubicin, epirubicin, idarubicin pirarubicin, and aclarubicin) and the bleomycins; other prominent examples include mitomycin C, mitoxantrone, and actinomycin.

In some embodiments, prior to administration of immune effector cells, a lymphodepleting treatment may be applied, e.g., by administering cyclophosphamide and fludarabine. Such treatment may increase cell persistence and the incidence and duration of clinical responses. "Immune checkpoint" refers to regulators of the immune system, and, in particular, co-stimulatory and inhibitory signals that regulate the amplitude and quality of T cell activity. In certain embodiments, the immune checkpoint is an inhibitory signal. In certain embodiments, the inhibitory signal is the interaction between PD-1 and PD-L1 and/or PD-L2.

The "Programmed Death-1 (PD-1)" receptor refers to an immuno-inhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. "Programmed Death Ligand-1 (PD-L1)" is one of two cell surface glycoprotein ligands for PD-1 (the other being PD-L2) that downregulates T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The term "PD-L2" as used herein includes human PD-L2 (hPD-L2), variants, isoforms, and species homologs of hPD-L2, and analogs having at least one common epitope with hPD-L2. The ligands of PD-1 (PD-L1 and PD-L2) are expressed on the surface of antigen-presenting cells, such as dendritic cells or macrophages, and other immune cells. Binding of PD-1 to PD-L1 or PD-L2 results in downregulation of T cell activation. Cancer cells expressing PD-L1 and/or PD-L2 are able to switch off T cells expressing PD-1 which results in suppression of the anticancer immune response. The interaction between PD-1 and its ligands results in a decrease in tumor infiltrating lymphocytes, a decrease in T cell receptor mediated proliferation, and immune evasion by the cancerous cells. Immune suppression can be reversed by inhibiting the local interaction of PD-1 with PD-L1, and the effect is additive when the interaction of PD-1 with PD-L2 is blocked as well.

Many of the immune checkpoints are regulated by interactions between specific receptor and ligand pairs, such as those described above. Thus, immune checkpoint proteins mediate immune checkpoint signaling. For example, checkpoint proteins directly or indirectly regulate T cell activation, T cell proliferation and/or T cell function. Cancer cells often exploit these checkpoint pathways to protect themselves from being attacked by the immune system. Hence, the function of checkpoint proteins, which is modulated according to the present disclosure is typically the regulation of T cell activation, T cell proliferation and/or T cell function. Immune checkpoint proteins thus regulate and maintain self-tolerance and the duration and amplitude of physiological immune responses.

As used herein, the term "immune checkpoint modulator" or "checkpoint modulator" refers to a molecule or to a compound that modulates the function of one or more checkpoint proteins. Immune checkpoint modulators are typically able to modulate self-tolerance and/or the amplitude and/or the duration of the immune response. Preferably, the immune checkpoint modulator modulates the function of one or more human checkpoint proteins and is, thus, a "human checkpoint modulator". Specifically, the human checkpoint modulator is an immune checkpoint inhibitor.

As used herein, "immune checkpoint inhibitor" or "checkpoint inhibitor" refers to a molecule that totally or partially reduces, inhibits, interferes with or negatively modulates one or more checkpoint proteins or that totally or partially reduces, inhibits, interferes with or negatively modulates expression of one or more checkpoint proteins. In certain embodiments, the immune checkpoint inhibitor binds to one or more checkpoint proteins. In certain embodiments, the immune checkpoint inhibitor binds to one or more molecules regulating checkpoint proteins.

In certain embodiments, the immune checkpoint inhibitor prevents inhibitory signals associated with the immune checkpoint. In certain embodiments, the immune checkpoint inhibitor is an antibody, or fragment thereof that disrupts inhibitory signaling associated with the immune checkpoint. In certain embodiments, the immune checkpoint inhibitor is a small molecule inhibitor that disrupts inhibitory signaling. In certain embodiments, the immune checkpoint inhibitor is a peptide-based inhibitor that disrupts inhibitory signaling.

In certain embodiments, the immune checkpoint inhibitor is an antibody, fragment thereof, or antibody mimic, that prevents the interaction between checkpoint blocker proteins.

In some embodiments, inhibiting or blocking of inhibitory immune checkpoint signaling, as described herein, results in preventing or reversing immune-suppression and establishment or enhancement of T cell immunity. In some embodiments, inhibition of immune checkpoint signaling, as described herein, reduces or inhibits dysfunction of the immune system. In some embodiments, inhibition of immune checkpoint signaling, as described herein, renders dysfunctional immune cells less dysfunctional. In some embodiments, inhibition of immune checkpoint signaling, as described herein, renders a dysfunctional T cell less dysfunctional.

In certain embodiments, the inhibitory immunoregulator (immune checkpoint blocker) is a component of the PD-1/PD-L1 or PD-1/PD-L2 signaling pathway.

In certain embodiments, the inhibitory immunoregulator (immune checkpoint blocker) is a PD-1 axis binding antagonist.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonist" refers to a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In some embodiments, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody. Specific examples of PD-1 binding antagonists are provided infra.

The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof,

immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In some embodiments, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. Specific examples of PD-L1 binding antagonists are provided infra.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In some embodiments, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

In some embodiments, a PD-1 axis binding antagonist includes a PD-1 binding antagonist, a PD-L1 binding antagonist and a PD-L2 binding antagonist. Alternative names for "PD-1" include CD279 and SLEB2. Alternative names for "PD-L1" include B7-H1, B7-4, CD274, and B7-H. Alternative names for "PD-L2" include B7-DC, Btdc, and CD273. In some embodiments, PD-1, PD-L1, and PD-L2 are human PD-1, PD-L1 and PD-L2.

In some embodiments, the PD-1 binding antagonist is a molecule that inhibits the binding of PD-1 to its ligand binding partner(s). In a specific aspect the PD-1 ligand binding partners are PD-L1 and/or PD-L2.

In some embodiments, the PD-L1 binding antagonist is a molecule that inhibits the binding of PD-L1 to its binding partner(s). In a specific aspect, PD-L1 binding partner(s) are PD-1 and/or B7-1.

In some embodiments, the PD-L2 binding antagonist is a molecule that inhibits the binding of PD-L2 to its binding partner(s). In a specific aspect, a PD-L2 binding partner is PD-1.

The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody).

In some embodiments, the PD-L1 binding antagonist is an anti-PD-L1 antibody.

PD-1 axis binding antagonists such as anti-PD-1 antibodies and anti-PD-L1 antibodies may be administered in any manner and by any route known in the art. The mode and route of administration will depend on the type of PD-1 axis binding antagonist to be used.

PD-1 axis binding antagonists may be administered in the form of any suitable pharmaceutical composition as described herein.

PD-1 axis binding antagonists such as anti-PD-1 antibodies and anti-PD-L1 antibodies may be administered in the form of nucleic acid, such DNA or RNA, encoding a PD-1 axis binding antagonist such as anti-PD-1 antibody or anti-PD-L1 antibody. For example, antibodies can be delivered encoded in expressing nucleic acids, as described herein. Nucleic acid molecules can be delivered as such, e.g., in the form of a plasmid or mRNA molecule, or complexed with a delivery vehicle, e.g., a liposome, lipoplex or any other nucleic-acid particle such as nucleic-acid lipid particle. PD-1 axis binding antagonists such as anti-PD-1 antibodies and anti-PD-L1 antibodies may also be administered via an oncolytic virus comprising an expression cassette encoding the PD-1 axis binding antagonist.

### Compositions comprising nucleic acid

A composition comprising one or more nucleic acids described herein, e.g., in the form of nucleic acid particles, may comprise salts, buffers, or other components as further described below.

In some embodiments, a salt for use in the compositions described herein comprises sodium chloride. Without wishing to be bound by theory, sodium chloride functions as an ionic osmolality agent for preconditioning RNA prior to mixing with lipids. In some embodiments, the compositions described herein may comprise alternative organic or inorganic salts. Alternative salts include, without limitation, potassium chloride, dipotassium phosphate, monopotassium phosphate, potassium acetate, potassium bicarbonate, potassium sulfate, disodium phosphate, monosodium phosphate, sodium acetate, sodium bicarbonate, sodium sulfate, lithium chloride, magnesium chloride, magnesium phosphate, calcium chloride, and sodium salts of ethylenediaminetetraacetic acid (EDTA).

Generally, compositions for storing RNA particles such as for freezing RNA particles comprise low sodium chloride concentrations, or comprises a low ionic strength. In some embodiments, the sodium chloride is at a concentration

from 0 mM to about 50 mM, from 0 mM to about 40 mM, or from about 10 mM to about 50 mM.

According to the present disclosure, the RNA particle compositions described herein have a pH suitable for the stability of the RNA particles and, in particular, for the stability of the RNA. Without wishing to be bound by theory, the use of a buffer system maintains the pH of the particle compositions described herein during manufacturing, storage and use of the compositions. In some embodiments of the present disclosure, the buffer system may comprise a solvent (in particular, water, such as deionized water, in particular water for injection) and a buffering substance. The buffering substance may be selected from 2-[4-(2-hydroxyethyl)piperazin-1-yl]ethanesulfonic acid (HEPES), 2-amino-2-(hydroxymethyl)propane-1,3-diol (Tris), acetate, and histidine. A preferred buffering substance is HEPES. In some embodiments, a pH may be above about 7.0; for example, in some embodiments, a pH may be about 7.4 or about 7.5.

Compositions described herein may also comprise a cyroprotectant and/or a surfactant as stabilizer to avoid substantial loss of the product quality and, in particular, substantial loss of mRNA activity during storage, freezing, and/or lyophilization, for example to reduce or prevent aggregation, particle collapse, mRNA degradation and/or other types of damage.

In an embodiment, the cryoprotectant is a carbohydrate. The term "carbohydrate", as used herein, refers to and encompasses monosaccharides, disaccharides, trisaccharides, oligosaccharides and polysaccharides.

In an embodiment, the cryoprotectant is a monosaccharide. The term "monosaccharide", as used herein refers to a single carbohydrate unit (e.g., a simple sugar) that cannot be hydrolyzed to simpler carbohydrate units. Exemplary monosaccharide cryoprotectants include glucose, fructose, galactose, xylose, ribose and the like.

In an embodiment, the cryoprotectant is a disaccharide. The term "disaccharide", as used herein refers to a compound or a chemical moiety formed by 2 monosaccharide units that are bonded together through a glycosidic linkage, for example through 1-4 linkages or 1-6 linkages. A disaccharide may be hydrolyzed into two monosaccharides. Exemplary disaccharide cryoprotectants include sucrose, trehalose, lactose, maltose and the like.

The term "trisaccharide" means three sugars linked together to form one molecule. Examples of a trisaccharides include raffinose and melezitose.

In an embodiment, the cryoprotectant is an oligosaccharide. The term "oligosaccharide", as used herein refers to a compound or a chemical moiety formed by 3 to about 15, such as 3 to about 10 monosaccharide units that are bonded together through glycosidic linkages, for example through 1-4 linkages or 1-6 linkages, to form a linear, branched or cyclic structure. Exemplary oligosaccharide cryoprotectants include cyclodextrins, raffinose, melezitose, maltotriose, stachyose, acarbose, and the like. An oligosaccharide can be oxidized or reduced. In an embodiment, the cryoprotectant is a cyclic oligosaccharide. The term "cyclic oligosaccharide", as used herein refers to a compound or a chemical moiety formed by 3 to about 15, such as 6, 7, 8, 9, or 10 monosaccharide units that are bonded together through glycosidic linkages, for example through 1-4 linkages or 1-6 linkages, to form a cyclic structure. Exemplary cyclic oligosaccharide cryoprotectants include cyclic oligosaccharides that are discrete compounds, such as $\alpha$ cyclodextrin, $\beta$ cyclodextrin, or $\gamma$ cyclodextrin.

Other exemplary cyclic oligosaccharide cryoprotectants include compounds which include a cyclodextrin moiety in a larger molecular structure, such as a polymer that contains a cyclic oligosaccharide moiety. A cyclic oligosaccharide can be oxidized or reduced, for example, oxidized to dicarbonyl forms. The term "cyclodextrin moiety", as used herein refers to cyclodextrin (e.g., an $\alpha$, $\beta$, or $\gamma$ cyclodextrin) radical that is incorporated into, or a part of, a larger molecular structure, such as a polymer. A cyclodextrin moiety can be bonded to one or more other moieties directly, or through an optional linker. A cyclodextrin moiety can be oxidized or reduced, for example, oxidized to dicarbonyl forms.

Carbohydrate cryoprotectants, e.g., cyclic oligosaccharide cryoprotectants, can be derivatized carbohydrates. For example, in an embodiment, the cryoprotectant is a derivatized cyclic oligosaccharide, *e.g.,* a derivatized cyclodextrin, *e.g.,* 2-hydroxypropyl-$\beta$-cyclodextrin, *e.g.,* partially etherified cyclodextrins (e.g., partially etherified $\beta$ cyclodextrins).

An exemplary cryoprotectant is a polysaccharide. The term "polysaccharide", as used herein refers to a compound or a chemical moiety formed by at least 16 monosaccharide units that are bonded together through glycosidic linkages, for example through 1-4 linkages or 1-6 linkages, to form a linear, branched or cyclic structure, and includes polymers that comprise polysaccharides as part of their backbone structure. In backbones, the polysaccharide can be linear or cyclic. Exemplary polysaccharide cryoprotectants include glycogen, amylase, cellulose, dextran, maltodextrin and the like.

In some embodiments, RNA particle compositions may include sucrose. Without wishing to be bound by theory, sucrose functions to promote cryoprotection of the compositions, thereby preventing RNA (especially mRNA) particle aggregation and maintaining chemical and physical stability of the composition. In some embodiments, RNA particle compositions may include alternative cryoprotectants to sucrose. Alternative stabilizers include, without limitation, trehalose and glucose. In a specific embodiment, an alternative stabilizerto sucrose is trehalose or a mixture of sucrose and trehalose.

A preferred cryoprotectant is selected from the group consisting of sucrose, trehalose, glucose, and a combination thereof, such as a combination of sucrose and trehalose. In a preferred embodiment, the cryoprotectant is sucrose.

Some embodiments of the present disclosure contemplate the use of a chelating agent in an RNA composition

described herein. Chelating agents refer to chemical compounds that are capable of forming at least two coordinate covalent bonds with a metal ion, thereby generating a stable, water-soluble complex. Without wishing to be bound by theory, chelating agents reduce the concentration of free divalent ions, which may otherwise induce accelerated RNA degradation in the present disclosure. Examples of suitable chelating agents include, without limitation, ethylenediaminetetraacetic acid (EDTA), a salt of EDTA, desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), diethylenetriaminepentaacetic acid (DTPA), and bis(aminoethyl)glycolether-N,N,N',N'-tetraacetic acid. In some embodiments, the chelating agent is EDTA or a salt of EDTA. In an exemplary embodiment, the chelating agent is EDTA disodium dihydrate. In some embodiments, the EDTA is at a concentration from about 0.05 mM to about 5 mM, from about 0.1 mM to about 2.5 mM or from about 0.25 mM to about 1 mM.

In an alternative embodiment, the RNA particle compositions described herein do not comprise a chelating agent.

Terms such as "stability" or "desired storage stability" as used herein may refer to physicochemical stability of the product, e.g., Tris/sucrose finished product, in unopened thawed vials for up to 24 hours at 30 °C, and in syringes for up to 24 hours at 2-8 °C and 12 hours at 30 °C. Such terms may refer to shelf-life for the product of 6 months or more when stored at -90 to -60 °C.

**Pharmaceutical compositions**

The agents described herein may be administered in pharmaceutical compositions or medicaments and may be administered in the form of any suitable pharmaceutical composition. In some embodiments, the pharmaceutical composition is for therapeutic or prophylactic treatments, e.g., for use in treating or preventing a disease involving an antigen such as a cancer disease or an infectious disease.

The term "pharmaceutical composition" relates to a composition comprising a therapeutically effective agent, preferably together with pharmaceutically acceptable carriers, diluents and/or excipients. Said pharmaceutical composition is useful for treating, preventing, or reducing the severity of a disease by administration of said pharmaceutical composition to a subject.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cytokines, such as monokines, lymphokines, interleukins, chemokines. The chemokines may be IL-1, IL-2, IL-3, IL-4, IL-5, IL-6, IL-7, IL-8, IL-9, IL-10, IL-12, INFa, INF-y, GM-CSF, LT-a. Further known adjuvants are aluminum hydroxide, Freund's adjuvant or oil such as Montanide® ISA51. Other suitable adjuvants for use in the present disclosure include lipopeptides, such as Pam3Cys, as well as lipophilic components, such as saponins, trehalose-6,6-dibehenate (TDB), monophosphoryl lipid-A (MPL), monomycoloyl glycerol (MMG), or glucopyranosyl lipid adjuvant (GLA).

The pharmaceutical compositions of the present disclosure may be in a storable form (e.g., in a frozen or lyophilized/freeze-dried form) or in a "ready-to-use form" (i.e., in a form which can be immediately administered to a subject, e.g., without any processing such as diluting). Thus, prior to administration of a storable form of a pharmaceutical composition, this storable form has to be processed or transferred into a ready-to-use or administrable form. *E.g.,* a frozen pharmaceutical composition has to be thawed, or a freeze-dried pharmaceutical composition has to be reconstituted, e.g. by using a suitable solvent (e.g., deionized water, such as water for injection) or liquid (e.g., an aqueous solution).

The pharmaceutical compositions according to the present disclosure are generally applied in a "pharmaceutically effective amount" and in "a pharmaceutically acceptable preparation". The term "pharmaceutically acceptable" refers to the non-toxicity of a material which does not interact with the action of the active component of the pharmaceutical composition. The term "pharmaceutically effective amount" refers to the amount which achieves a desired reaction or a desired effect alone or together with further doses. In some embodiments relating to the treatment of a particular disease, the desired reaction may relate to inhibition of the course of the disease. This comprises slowing down the progress of the disease and, in some embodiments, interrupting or reversing the progress of the disease. The desired reaction in a treatment of a disease may also be delay of the onset or a prevention of the onset of said disease or said condition. An effective amount of the pharmaceutical compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the pharmaceutical compositions described herein may depend on various of such parameters. In the case that a reaction in a patient is insufficient with an initial dose, higher doses (or effectively higher doses achieved by a different, more localized route of administration) may be

used.

The pharmaceutical compositions of the present disclosure may contain buffers, preservatives, and optionally other therapeutic agents. In some embodiments, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In some embodiments, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

**Routes of administration of pharmaceutical compositions**

In some embodiments, the pharmaceutical compositions described herein may be administered intravenously, intraarterially, subcutaneously, intradermally, dermally, intranodally, intramuscularly, intratumorally, or peritumorally. In some embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In some embodiments, the pharmaceutical compositions are formulated for systemic administration. In some embodiments, the systemic administration is by intravenous administration.

**Use of compositions**

Compositions described herein may be used in the therapeutic or prophylactic treatment of various diseases, in particular diseases in which provision of a peptide or polypeptide, e.g., vaccine antigen, to a subject results in a therapeutic or prophylactic effect, e.g., a disease characterized by the presence of diseased cells expressing an antigen such as cancer diseases or infectious diseases. For example, provision of an antigen or epitope which is derived from a virus may be useful in the treatment of a viral disease caused by said virus. Provision of a tumor antigen or epitope may be useful in the treatment of a cancer disease wherein cancer cells express said tumor antigen. Provision of a functional protein or enzyme may be useful in the treatment of genetic disorder characterized by a dysfunctional protein, for example in lysosomal storage diseases (e.g. Mucopolysaccharidoses) or factor deficiencies. Provision of a cytokine or a cytokine-fusion may be useful to modulate tumor microenvironment.

The term "disease" (also referred to as "disorder" herein) refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

The term "disease involving an antigen" refers to any disease which implicates an antigen, e.g. a disease which is characterized by the presence of an antigen. The disease involving an antigen can be an infectious disease, or a cancer disease or simply cancer. The antigen may be a disease-associated antigen, such as a tumor-associated antigen, a viral antigen, or a bacterial antigen. In some embodiments, a disease involving an antigen is a disease involving cells expressing an antigen, and preferably presenting the antigen on the cell surface, e.g., in the context of MHC.

The term "infectious disease" refers to any disease which can be transmitted from individual to individual or from organism to organism, and is caused by a microbial agent (e.g. common cold). Infectious diseases are known in the art and include, for example, a viral disease, a bacterial disease, or a parasitic disease, which diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza.

The terms "cancer disease" or "cancer" refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the disclosure also comprises cancer metastases.

In the present context, the term "treatment", "treating" or "therapeutic intervention" relates to the management and care of a subject for the purpose of combating a condition such as a disease. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g., mouse, rat, rabbit, dog, cat, cattle, swine, sheep, horse or primate), or any other non-mammal-animal, including birds (chicken), fish or any other animal species that can be afflicted with or is susceptible to a disease (e.g., cancer, infectious diseases) but may or may not have the disease, or may have a need for prophylactic intervention such as vaccination, or may have a need for interventions such as by protein replacement. In many embodiments, the individual is a human being. Unless otherwise stated, the terms "individual" and "subject" do not denote a particular age, and thus encompass adults, elderlies, children, and newborns. In some embodiments of the present disclosure, the "individual" or "subject" is a "patient".

The term "patient" means an individual or subject for treatment, in particular a diseased individual or subject.

Nucleic acid, in particular RNA may be administered to a subject for delivering the nucleic acid to cells of the subject.

Nucleic acid, in particular RNA may be administered to a subject for delivering a therapeutic or prophylactic peptide or polypeptide (e.g., a pharmaceutically active peptide or polypeptide) to the subject, wherein the nucleic acid encodes a therapeutic or prophylactic peptide or polypeptide.

Nucleic acid, in particular RNA may be administered to a subject for treating or preventing a disease in a subject, wherein delivering the nucleic acid to cells of the subject is beneficial in treating or preventing the disease.

Nucleic acid, in particular RNA may be administered to a subject for treating or preventing a disease in a subject, wherein the nucleic acid encodes a therapeutic or prophylactic peptide or polypeptide and wherein delivering the therapeutic or prophylactic peptide or polypeptide to the subject is beneficial in treating or preventing the disease.

In some embodiments, the nucleic acid is present in a composition as described herein.

In some embodiments, the nucleic acid is administered in a pharmaceutically effective amount.

In some embodiments, the subject is a mammal. In some embodiments, the mammal is a human.

In some embodiments of the disclosure, the aim is to induce an immune response by providing a vaccine.

A person skilled in the art will know that one of the principles of immunotherapy and vaccination is based on the fact that an immunoprotective reaction to a disease is produced by immunizing a subject with an antigen or an epitope, which is immunologically relevant with respect to the disease to be treated. Accordingly, nucleic acids described herein are applicable for inducing or enhancing an immune response. Nucleic acids described herein are thus useful in a prophylactic and/or therapeutic treatment of a disease involving an antigen or epitope. In some embodiments of the disclosure, the aim is to provide an immune response against diseased cells expressing an antigen such as cancer cells expressing a tumor antigen, and to treat a disease such as a cancer disease involving cells expressing an antigen such as a tumor antigen.

In some embodiments of the disclosure, the aim is to treat cancer by vaccination.

In some embodiments of the disclosure, the aim is to provide an immune response against cancer cells expressing a tumor antigen and to treat a cancer disease involving cells expressing a tumor antigen.

In some embodiments of the disclosure, the aim is to provide protection against an infectious disease by vaccination.

In some embodiments of the disclosure, the aim is to provide secreted therapeutic proteins, such as antibodies, bispecific antibodies, cytokines, cytokine fusion proteins, enzymes, to a subject, in particular a subject in need thereof.

In some embodiments of the disclosure, the aim is to provide a protein replacement therapy, such as production of erythropoietin, Factor VII, Von Willebrand factor, β-galactosidase, Alpha-N-acetylglucosaminidase, to a subject, in particular a subject in need thereof.

In some embodiments of the disclosure, the aim is to modulate/reprogram immune cells in the blood.

In some embodiments of the disclosure, the aim is to provide one or more cytokines or cytokine fusions which modulate tumor microenvironment to a subject, in particular a subject in need thereof.

In some embodiments of the disclosure, the aim is to provide one or more cytokines or cytokine fusions which have antitumoral activity to a subject, in particular a subject in need thereof.

Certain Exemplary Embodiments:

1. An immunogenic composition comprising: a lipid nanoparticle (LNP) comprising an RNA, wherein the RNA encodes the polypeptide of SEQ ID NO: 31 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 31, and comprises the nucleotide sequence of SEQ ID NO: 32 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 32, and wherein the RNA comprises:

   (a) modified uridines;
   (b) a 5' cap; and

   wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

2. The immunogenic composition of embodiment 1, wherein the nucleotide sequence includes modified uridines in place of all uridines.

3. The immunogenic of embodiment 1 or 2, wherein the modified uridines are each N1-methyl-pseudouridine.

4. The immunogenic composition of any one of embodiments 1 to 3, wherein the RNA further comprises at least one, at least two, or all of the following features:

   a 5' untranslated region (UTR) comprising SEQ ID NO: 6;
   a 3' untranslated region (UTR) comprising SEQ ID NO: 7; and
   a poly-A sequence of at least 100 A nucleotides.

5. The immunogenic composition of embodiment 4, wherein the poly-A sequence comprises 30 adenine nucleotides followed by 70 adenine nucleotides, wherein the 30 adenine nucleotides and 70 adenine nucleotides are separated

by a linker sequence.

6. The immunogenic composition of embodiment 4, wherein the poly-A sequence comprises SEQ ID NO: 8.

7. The immunogenic composition of any one of embodiments 1 to 6, wherein the RNA comprises SEQ ID NO: 33.

8. An immunogenic composition comprising a lipid nanoparticle (LNP) comprising an RNA, wherein the RNA encodes the polypeptide of SEQ ID NO: 22 and comprises the nucleotide sequence of SEQ ID NO: 23 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 23, and wherein the RNA comprises:

(a) modified uridines;
(b) a 5' cap; and

wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

9. The immunogenic composition of embodiment 8, wherein the nucleotide sequence includes modified uridines in place of all uridines.

10. The immunogenic of embodiment 8 or 9, wherein the modified uridines are each N1-methyl-pseudouridine.

11. The immunogenic composition of any one of embodiments 8 to 10, wherein the RNA further comprises at least one, at least two, or all of the following features:

a 5' untranslated region (UTR) comprising SEQ ID NO: 6;
a 3' untranslated region (UTR) comprising SEQ ID NO: 7; and

a poly-A sequence of at least 100 A nucleotides.

12. The immunogenic composition of embodiment 11, wherein the poly-A sequence comprises 30 adenine nucleotides followed by 70 adenine nucleotides, wherein the 30 adenine nucleotides and 70 adenine nucleotides are separated by a linker sequence.

13. The immunogenic composition of embodiment 11, wherein the poly-A sequence comprises SEQ ID NO: 8.

14. The immunogenic composition of any one of embodiments 8 to 13, wherein the RNA comprises SEQ ID NO: 24.

15. An immunogenic composition comprising a a lipid nanoparticle (LNP) comprising an RNA, wherein the RNA encodes the polypeptide of SEQ ID NO: 25 and comprises the nucleotide sequence of SEQ ID NO: 26 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 26, and wherein the RNA comprises:

(a) modified uridines;
(b) a 5' cap; and

wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

16. The immunogenic composition of embodiment 15, wherein the nucleotide sequence includes modified uridines in place of all uridines.

17. The immunogenic of embodiment 15 or 16, wherein the modified uridines are each N1-methyl-pseudouridine.

18. The immunogenic composition of any one of embodiments 15 to 17, wherein the RNA further comprises at least one, at least two, or all of the following features:

a 5' untranslated region (UTR) comprising SEQ ID NO: 6;

a 3' untranslated region (UTR) comprising SEQ ID NO: 7; and
a poly-A sequence of at least 100 A nucleotides.

19. The immunogenic composition of embodiment 18, wherein the poly-A sequence comprises 30 adenine nucleotides followed by 70 adenine nucleotides, wherein the 30 adenine nucleotides and 70 adenine nucleotides are separated by a linker sequence.

20. The immunogenic composition of embodiment 18, wherein the poly-A sequence comprises SEQ ID NO: 8.

21. The immunogenic composition of any one of embodiments 15 to 20, wherein the RNA comprises SEQ ID NO: 27.

22. An immunogenic composition comprising a lipid nanoparticle (LNP) comprising an RNA, wherein the RNA encodes the polypeptide of SEQ ID NO: 28 and comprises the nucleotide sequence of SEQ ID NO: 29 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 29, and wherein the RNA comprises:

(a) modified uridines;
(b) a 5' cap; and

wherein the LNP comprises ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

23. The immunogenic composition of embodiment 22, wherein the nucleotide sequence includes modified uridines in place of all uridines.

24. The immunogenic of embodiment 22 or 23, wherein the modified uridines are each N1-methyl-pseudouridine.

25. The immunogenic composition of any one of embodiments 22 to 24, wherein the RNA further comprises at least one, at least two, or all of the following features:

a 5' untranslated region (UTR) comprising SEQ ID NO: 6;
a 3' untranslated region (UTR) comprising SEQ ID NO: 7; and

a poly-A sequence of at least 100 A nucleotides.

26. The immunogenic composition of embodiment 25, wherein the poly-A sequence comprises 30 adenine nucleotides followed by 70 adenine nucleotides, wherein the 30 adenine nucleotides and 70 adenine nucleotides are separated by a linker sequence.

27. The immunogenic composition of embodiment 25, wherein the poly-A sequence comprises SEQ ID NO: 8.

28. The immunogenic composition of any one of embodiments 22 to 27, wherein the RNA comprises SEQ ID NO: 30.

29. An immunogenic composition comprising a first RNA and a second RNA, wherein:

the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
the second RNA encodes the polypeptide of SEQ ID NO: 31 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 31, and comprises the nucleotide sequence of SEQ ID NO: 32 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 32, and
wherein each of the first RNA and the second RNA comprise:

(a) modified uridines; and
(b) a 5' cap, and

wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

30. The immunogenic composition of embodiment 29, wherein the first RNA and the second RNA are formulated in the same lipid nanoparticles.

31. The immunogenic composition of embodiment 29, wherein the first RNA and the second RNA are formulated in seperate lipid nanoparticles.

32. The immunogenic composition of any one of embodiments 29 to 31, wherein each of the first RNA and the second RNA include modified uridines in place of all uridines.

33. The immunogenic of any one of embodiments 29 to 32, wherein the modified uridines are each N1-methyl-pseudouridine.

34. The immunogenic composition of any one of embodiments 29 to 33, wherein the first RNA and the second RNA each indepedently comprise at least one, at least two, or all of the following features:

a 5' untranslated region (UTR) comprising SEQ ID NO: 6;
a 3' untranslated region (UTR) comprising SEQ ID NO: 7; and

a poly-A sequence of at least 100 A nucleotides.

35. The immunogenic composition of embodiment 34, wherein the poly-A sequence comprises 30 adenine nucleotides followed by 70 adenine nucleotides, wherein the 30 adenine nucleotides and 70 adenine nucleotides are separated by a linker sequence.

36. The immunogenic composition of embodiment 34, wherein the poly-A sequence comprises SEQ ID NO: 8.

37. The immunogenic composition of any one of embodiments 29 to 36, wherein the first RNA comprises SEQ ID NO: 20 and the second RNA comprises SEQ ID NO: 33.

38. An immunogenic composition comprising a first RNA and a second RNA, wherein:

the first RNA encodes the polypeptide of SEQ ID NO: 7 and comprises the nucleotide sequence of SEQ ID NO: 9 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 9, and
the second RNA encodes the polypeptide of SEQ ID NO: 22, 25, or 28 and comprises the nucleotide sequence of SEQ ID NO: 23, 26, or 29, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 23, 27, or 29, and
wherein each of the first RNA and the second RNA comprise:

(a) modified uridines; and
(b) a 5' cap, and

wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoyl-sn-glycero-3-phosphocholine, and cholesterol.

39. The immunogenic composition of embodiment 38, wherein the first RNA and the second RNA are formulated in separate lipid nanoparticles.

40. The immunogenic composition of embodiment 38, wherein the first RNA and the second RNA are formulated in the same lipid nanoparticles.

41. The immunogenic composition of any one of embodiments 38 to 40, wherein the first RNA and the second RNA

each include modified uridines in place of all uridines.

42. The immunogenic composition of any one of embodiments 38 to 41, wherein the modified uridines are each N1-methyl-pseudouridine.

43. The immunogenic composition of any one of embodiments 38 to 42, wherein the first RNA and the second RNA each independently further comprise at least one, at least two, or all of the following features:

a 5' untranslated region (UTR) comprising SEQ ID NO: 6;
a 3' untranslated region (UTR) comprising SEQ ID NO: 7; and
a poly-A sequence of at least 100 A nucleotides.

44. The immunogenic composition of embodiment 43, wherein the poly-A sequence comprises 30 adenine nucleotides followed by 70 adenine nucleotides, wherein the 30 adenine nucleotides and 70 adenine nucleotides are separated by a linker sequence.

45. The immunogenic composition of embodiment 43, wherein the poly-A sequence comprises SEQ ID NO: 8.

46. The immunogenic composition of any one of embodiments 38 to 45, wherein the first RNA comprises SEQ ID NO: 9 and the second RNA comprises SEQ ID NO: 23.

47. The immunogenic composition of any one of embodiments 38 to 45, wherein the first RNA comprises SEQ ID NO: 9 and the second RNA comprises SEQ ID NO: 26.

48. The immunogenic composition of any one of embodiments 38 to 47, wherein the first RNA comprises SEQ ID NO: 9 and the second RNA comprises SEQ ID NO: 29.

49. The immunogenic composition of any one of embodiments 38 to 48, wherein the first RNA comprises SEQ ID NO: 20 and the second RNA comprises SEQ ID NO: 24.

50. The immunogenic composition of any one of embodiments 38 to 48, wherein the first RNA comprises SEQ ID NO: 20 and the second RNA comprises SEQ ID NO: 27.

51. The immunogenic composition of any one of embodiments 38 to 48, wherein the first RNA comprises SEQ ID NO: 20 and the second RNA comprises SEQ ID NO: 30.

52. An immunogenic composition comprising a first RNA and a second RNA, wherein:

the first RNA encodes the polypeptide of SEQ ID NO: 25 and comprises the nucleotide sequence of SEQ ID NO: 26 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 26, and
the second RNA encodes the polypeptide of SEQ ID NO: 31, 22, or 28 or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 31, 22, or 28, and comprises the nucleotide sequence of SEQ ID NO: 32, 23, or 29, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 32, 23, or 29, and
wherein each of the first RNA and the second RNA comprise:

(a) modified uridines; and
(b) a 5' cap, and

wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoylsn-glycero-3-phosphocholine, and cholesterol.

53. The immunogenic composition of embodiment 52, wherein the first RNA and the second RNA are formulated

in separate lipid nanoparticles.

54. The immunogenic composition of embodiment 52, wherein the first RNA and the second RNA are formulated in the same lipid nanoparticles.

55. The immunogenic composition of any one of embodiments 52 to 54, wherein the first RNA and the second RNA each include modified uridines in place of all uridines.

56. The immunogenic of any one of embodiments 52 to 55, wherein the modified uridines are each N1-methyl-pseudouridine.

57. The immunogenic composition of any one of embodiments 52 to 56, wherein the first RNA and the second RNA each independently further comprise at least one, at least two, or all of the following features:

a 5' untranslated region (UTR) comprising SEQ ID NO: 6;
a 3' untranslated region (UTR) comprising SEQ ID NO: 7; and

a poly-A sequence of at least 100 A nucleotides.

58. The immunogenic composition of embodiment 57, wherein the poly-A sequence comprises 30 adenine nucleotides followed by 70 adenine nucleotides, wherein the 30 adenine nucleotides and 70 adenine nucleotides are separated by a linker sequence.

59. The immunogenic composition of embodiment 57, wherein the poly-A sequence comprises SEQ ID NO: 8.

60. The immunogenic composition of any one of embodiments 52 to 59, wherein the first RNA comprises SEQ ID NO: 26 and the second RNA comprises SEQ ID NO: 32.

61. The immunogenic composition of any one of embodiments 52 to 59, wherein the first RNA comprises SEQ ID NO: 26 and the second RNA comprises SEQ ID NO: 23.

62. The immunogenic composition of any one of embodiments 52 to 59, wherein the first RNA comprises SEQ ID NO: 26 and the second RNA comprises SEQ ID NO: 29.

63. The immunogenic composition of any one of embodiments 52 to 59, wherein the first RNA comprises SEQ ID NO: 27 and the second RNA comprises SEQ ID NO: 33.

64. The immunogenic composition of any one of embodiments 52 to 59, wherein the first RNA comprises SEQ ID NO: 27 and the second RNA comprises SEQ ID NO: 24.

65. The immunogenic composition of any one of embodiments 52 to 59, wherein the first RNA comprises SEQ ID NO: 27 and the second RNA comprises SEQ ID NO: 30.

66. An immunogenic composition comprising a first RNA and a second RNA, wherein:

the first RNA encodes the polypeptide of SEQ ID NO: 31, or an amino acid sequence that is at least 80% (e.g., at 85%, at least 90%, at least 91%, at least 92%, at least 93%, at least 94%, at least 95%, at least 97%, at least 98%, or 99% or higher) identical to SEQ ID NO: 31, and comprises the nucleotide sequence of SEQ ID NO: 32 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 32, and the second RNA encodes the polypeptide of SEQ ID NO: 22 or 28 and comprises the nucleotide sequence of SEQ ID NO: 23 or 29, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 23 or 29, and
wherein each of the first RNA and the second RNA comprise:

(a) modified uridines; and
(b) a 5' cap, and

wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoylsn-glycero-3-phosphocholine, and cholesterol.

67. The immunogenic composition of embodiment 66, wherein the first RNA and the second RNA are formulated in separate lipid nanoparticles.

68. The immunogenic composition of embodiment 66, wherein the first RNA and the second RNA are formulated in the same lipid nanoparticles.

69. The immunogenic composition of any one of embodiments 66 to 68, wherein the first RNA and the second RNA each include modified uridines in place of all uridines.

70. The immunogenic of any one of embodiments 66 to 69, wherein the modified uridines are each N1-methyl-pseudouridine.

71. The immunogenic composition of any one of embodiments 66 to 70, wherein the first RNA and the second RNA further each independently further comprise at least one, at least two, or all of the following features:

a 5' untranslated region (UTR) comprising SEQ ID NO: 6;
a 3' untranslated region (UTR) comprising SEQ ID NO: 7; and
a poly-A sequence of at least 100 A nucleotides.

72. The immunogenic composition of embodiment 71, wherein the poly-A sequence comprises 30 adenine nucleotides followed by 70 adenine nucleotides, wherein the 30 adenine nucleotides and 70 adenine nucleotides are separated by a linker sequence.

73. The immunogenic composition of embodiment 71, wherein the poly-A sequence comprises SEQ ID NO: 8.

74. The immunogenic composition of any one of embodiments 66 to 73, wherein the first RNA comprises SEQ ID NO: 32 and the second RNA comprises SEQ ID NO: 23.

75. The immunogenic composition of any one of embodiments 66 to 74, wherein the first RNA comprises SEQ ID NO: 32 and the second RNA comprises SEQ ID NO: 29.

76. The immunogenic composition of any one of embodiments 66 to 74, wherein the first RNA comprises SEQ ID NO: 33 and the second RNA comprises SEQ ID NO: 24.

77. The immunogenic composition of any one of embodiments 66 to 74, wherein the first RNA comprises SEQ ID NO: 33 and the second RNA comprises SEQ ID NO: 30.

78. An immunogenic composition comprising a first RNA and a second RNA, wherein:

the first RNA encodes the polypeptide of SEQ ID NO: 22 and comprises the nucleotide sequence of SEQ ID NO: 23 or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 23, and
the second RNA encodes the polypeptide of SEQ ID NO: 28 and comprises the nucleotide sequence of SEQ ID NO: 29, or a nucleotide sequence that is at least 80% (e.g., at 85%, at least 90%, at least 91%, at least 92%, at least 93%, at least 94%, at least 95%, at least 97%, at least 98%, or 99% or higher) identical to SEQ ID NO: 29, and
wherein each of the first RNA and the second RNA comprise:

(a) modified uridines; and
(b) a 5' cap, and

wherein the first RNA and the second RNA are formulated in lipid nanoparticles (LNPs), wherein the LNPs comprise ((4-hydroxybutyl)azanediyl)bis(hexane-6,1-diyl)bis(2-hexyldecanoate), 2-[(polyethylene glycol)-2000]-N,N-ditetradecylacetamide, 1,2-Distearoylsn-glycero-3-phosphocholine, and cholesterol.

79. The immunogenic composition of embodiment 78, wherein the first RNA and the second RNA are formulated in separate lipid nanoparticles.

80. The immunogenic composition of embodiment 78, wherein the first RNA and the second RNA are formulated in the same lipid nanoparticles.

81. The immunogenic composition of any one of embodiments 78 to 80, wherein the first RNA and the second RNA each include modified uridines in place of all uridines.

82. The immunogenic of any one of embodiments 78 to 81, wherein the modified uridines are each N1-methyl-pseudouridine.

83. The immunogenic composition of any one of embodiments 78 to 82, wherein the first RNA and the second RNA each independently further comprise at least one, at least two, or all of the following features:

a 5' untranslated region (UTR) comprising SEQ ID NO: 6;
a 3' untranslated region (UTR) comprising SEQ ID NO: 7; and

a poly-A sequence of at least 100 A nucleotides.

84. The immunogenic composition of embodiment 83, wherein the poly-A sequence comprises 30 adenine nucleotides followed by 70 adenine nucleotides, wherein the 30 adenine nucleotides and 70 adenine nucleotides are separated by a linker sequence.

85. The immunogenic composition of embodiment 83, wherein the poly-A sequence comprises SEQ ID NO: 8.

86. The immunogenic composition of any one of embodiments 78 to 85, wherein the first RNA comprises SEQ ID NO: 24 and the second RNA comprises SEQ ID NO: 30.

87. The immunogenic composition of any one of embodiments 1 to 85, wherein the 5'-cap is or comprises $m_2^{7,3'-O}$G-ppp($m_1^{2'-O}$)ApG.

88. The immunogenic composition of any one of embodiments 1 to 86, wherein the LNP comprises about 40 to about 50 mole percent ((4-hydroxybutyl)azanediyl)bis(hexane-6,1-diyl)bis(2-hexyldecanoate), about 35 to about 45 mole percent cholesterol, about 5 to about 15 mole percent 1,2-Distearoyl-sn-glycero-3-phosphocholine, and about 1 to about 10 mole percent 2-[(polyethylene glycol)-2000]-N,N-ditetradecylacetamide.

89. The immunogenic composition of any one of embodiments 1 to 88, wherein the composition comprises a plurality of LNPs, wherein the average diameter of the plurality of LNPs is about 30 nm to about 200 nm or about 60 nm to about 120 nm (e.g., as determined by dynamic light scattering measurements).

90. A method of eliciting an immune response against SARS-CoV-2 comprising administering the immunogenic composition of any one of embodiments 74 to 89.

91. The method of embodiment 90, wherein the immune response is elicited against an Omicron variant of SARS-CoV-2.

92. The method of embodiment 90, wherein the immune response is elicited against a Beta variant of SARS-CoV-2.

93. The method of embodiment 90, wherein the immune response is elicited against an Alpha variant of SARS-CoV-2.

94. The method of embodiment 90, wherein the immune response is elicited against a Delta variant of SARS-CoV-2.

95. The method of embodiment 90, wherein the immune response is elicited against a Wuhan strain, an Omicron variant, a Beta variant, an Alpha variant, and a Delta variant of SARS-CoV-2.

## EXEMPLIFICATION

### Example 1: Exemplary compositions for administration

In some embodiments, a dose of an RNA composition described herein for administration includes 30 ug of RNA (e.g., mRNA) encoding a SARS-CoV-2 protein or an immunogenic fragment thereof. In some embodiments, such RNA (e.g., mRNA) encodes a viral spike (S) glycoprotein of SARS-CoV-2 as described herein. In some embodiments, such an RNA (e.g., mRNA) is a nucleoside-modified mRNA. In some such embodiments, a dose of an RNA composition containing 30 ug of RNA encoding a SARS-CoV-2 protein or an immunogenic fragment thereof can further comprise the following ingredients: lipids (0.43 mg (4-hydroxybutyl)azanediyl)bis(hexane-6,1-diyl)bis(2-hexyldecanoate), 0.05 mg 2[(polyethylene glycol)-2000]-N,N-ditetradecylacetamide, 0.09 mg 1,2-distearoyl-sn-glycero-3-phosphocholine, and 0.19 mg cholesterol), 0.06 mg tromethamine, 0.4 mg tromethamine hydrochloride, and 31 mg sucrose. In some embodiments, such an RNA composition is useful for administration to subjects of 12 years of age or older. In some embodiments, such a dose can be achieved by injecting 300 uL of a stock RNA composition as described herein, e.g., as shown in Figure 2. In some embodiments, an RNA composition described herein does not comprise preservative.

In some embodiments, a dose of an RNA composition described herein for administration includes 10 ug of RNA (e.g., mRNA) encoding a SARS-CoV-2 protein or an immunogenic fragment thereof. In some embodiments, such RNA (e.g., mRNA) encodes a viral spike (S) glycoprotein of SARS-CoV-2 as described herein. In some embodiments, such an RNA (e.g., mRNA) is a nucleoside-modified mRNA. In some such embodiments, a dose of an RNA composition containing 10 ug of RNA encoding a SARS-CoV-2 protein or an immunogenic fragment thereof can further comprise the following ingredients: lipids (0.14 mg (4-hydroxybutyl)azanediyl)bis(hexane-6,1-diyl)bis(2-hexyldecanoate), 0.02 mg 2[(polyethylene glycol)-2000]-N,N-ditetradecylacetamide, 0.03 mg 1,2-distearoyl-sn-glycero-3-phosphocholine, and 0.06 mg cholesterol), 10.3 mg sucrose, 0.02 mg tromethamine, and 0.13 mg tromethamine hydrochloride. In some embodiments, such a dose of an RNA composition described herein can further comprise 0.9 mg sodium chloride (which in some embodiments can be contributed by a diluent used as described below).

In some embodiments, an RNA composition for 10 ug-dose administration can be prepared by diluting a stock RNA composition as described herein, e.g., as shown in Figure 2, with an appropriate diluent, which in some embodiments may be 0.9% Sodium Chloride Injection, USP. In some embodiments, a 0.9% Sodium Chloride Injection, USP is not bacteriostatic 0.9% Sodium Chloride Injection. For example, in some embodiments, 1.3 mL of a stock RNA composition as shown in Figure 2 can be diluted with 1.3 mL diluent (e.g., 0.9% Sodium Chloride Injection, USP) to produce a diluted drug product for administration. In some embodiments, such an RNA composition is useful for administration to subjects who may benefit from a low-dose regimen, including, e.g., pediatric subjects, immunocompromised subjects, subjects who may have shown adverse reaction to a prior COVID-19 vaccine injection, or subjects who got COVID in between vaccinations, etc. In some embodiments, such an RNA composition can be particularly useful for administration to subjects of 5-11 years of age. In some embodiments, a dose as described herein can be achieved by administering 200 uL of a diluted drug product as described herein.

In some embodiments, a dose of an RNA composition described herein for administration includes 3 ug of RNA (e.g., mRNA) encoding a SARS-CoV-2 protein or an immunogenic fragment thereof. In some embodiments, such RNA (e.g., mRNA) encodes a viral spike (S) glycoprotein of SARS-CoV-2 as described herein. In some embodiments, such an RNA (e.g., mRNA) is a nucleoside-modified mRNA. In some such embodiments, a dose of an RNA composition containing 3 ug of RNA encoding a SARS-CoV-2 protein or an immunogenic fragment thereof can further comprise the following ingredients: lipids (0.043 mg (4-hydroxybutyl)azanediyl)bis(hexane-6,1-diyl)bis(2-hexyldecanoate), 0.005 mg 2[(polyethylene glycol)-2000]-N,N-ditetradecylacetamide, 0.009 mg 1,2-distearoyl-sn-glycero-3-phosphocholine, and 0.019 mg cholesterol), 0.006 mg tromethamine, 0.04 mg tromethamine hydrochloride, and 3.1 mg sucrose. In some embodiments, such a dose of an RNA composition described herein can further comprise approximately 1.54 mg sodium chloride (which in some embodiments can be contributed by a diluent used as described below).

In some embodiments, an RNA composition for 3 ug-dose administration can be prepared by diluting a stock RNA composition as described herein, e.g., as shown in Figure 2, with an appropriate diluent, which in some embodiments may be 0.9% Sodium Chloride Injection, USP. In some embodiments, a 0.9% Sodium Chloride Injection, USP is not bacteriostatic 0.9% Sodium Chloride Injection. For example, in some embodiments, 0.4 mL of a stock RNA composition as shown in Figure 2 can be diluted with 2.3 mL diluent (e.g., 0.9% Sodium Chloride Injection, USP) to produce a diluted drug product for administration. In some embodiments, such an RNA composition is useful for administration to subjects who may benefit from a low-dose regimen, including, e.g., pediatric subjects, immunocompromised subjects, subjects who may have shown adverse reaction to a prior COVID-19 vaccine injection, or subjects who got COVID in between vaccinations, etc. In some embodiments, such an RNA composition can be particularly useful for administration to subjects of two years of age to less than 5 years of age. In some embodiments, such an RNA composition can be particularly useful for administration to subjects of 6 months to less than 5 years of age. In some embodiments, such an RNA composition can be particularly useful for administration to subjects of less than 6 months old. In some embodiments, a dose as described herein can be achieved by administering 200 uL of a diluted drug product as described herein.

**Example 2: Exemplary dosing regimens**

*30 μg (e.g., in 300 uL) Tris doses*

Compositions disclosed herein can be used as part of a 2-dose vaccination regimen, where each dose comprises 30 μg of RNA, and the first dose and the second dose are administered about 21 days apart. Such dosages may be suitable for any subject, e.g., subjects 12 years and older, subjects 12 through 15 years, subjects 12 years of age and older who have been determined to have certain kinds of immunocompromise; patients 16 years and older, patients 18 to 64 years of age, and patients 65 years of age and older.

Compositions disclosed herein that have been formulated to provide 30 μg doses can be used as part of a single dose, two dose, three dose, or four or more dose vaccination regimen, where each dose comprises 30 μg of RNA. A single dose regimen may be appropriate, e.g., when a subject has received a dose of another vaccine, when a subject has previously been infected with SARS-CoV-2, when a subject is receiving the dose as a booster dose and the subject has previously been administered a dose of another vaccine composition, or as a booster dose for a subject who has previously experienced one or more adverse effects after receiving a first dose. A three dose regimen may be appropriate, for example, for immunocompromised patients, e.g., patients who have undergone a solid organ transplant.

In some embodiments, 30 μg dose composition(s) disclosed herein may be used in a vaccination regimen that comprise a first vaccination (comprising one, two, or three or more doses) and a second vaccination (comprising one, two, or three or more doses), where the first and second vaccination are about 6 months apart from one another. Such vaccination regimens comprising a first and second vaccination may be appropriate for, e.g., patients 12 years and older.

*10 μg (e.g., in 200 uL) Tris doses*

Compositions disclosed herein can be used as part of a 2-dose vaccination regimen, where each dose comprises 10 μg of RNA and is administered about 21 days apart. Such dosages may be suitable for administration to any subject, e.g., subjects 5 years to 11 years old.

Compositions disclosed herein that have been formulated to provide 10 μg doses can be used as part of a single dose, two dose, three dose, or four or more dose vaccination regimen, where each dose comprises 10 μg of RNA. A single dose regimen may be appropriate, e.g., when a subject has received a dose of another vaccine, when a subject has already been infected with SARS-CoV-2, the dose is being administered as a booster dose, when a subject has received a dose of another vaccine, or when a subject experiences one or more adverse effects after receiving a first vaccine dose. A three dose regimen may be appropriate for immunocompromised patients, e.g., patients who have undergone a solid organ transplant.

Compositions disclosed herein that have been formulated to provide 10 μg doses can also be used as part of a two dose, three dose, or four or more dose vaccination regimen, where one dose comprises 10 μg of RNA and one or more doses comprise an amount of RNA other than 10 μg. A two-dose regimen in which a patient is administered 30 μg of RNA as a first dose and 10 μg of a RNA as a second dose may be appropriate, e.g., when a subject has experienced an adverse reaction to the first 30 μg dose. A two-dose regimen in which a patient is administered 10 μg of RNA as a first dose and 3 μg of a RNA as a second dose may be appropriate, e.g., when a subject has experienced an adverse reaction to the first 10 μg dose.

10 μg dose compositions disclosed herein may also be used in vaccination regimens that comprise a first vaccination (comprising one, two, or three or more doses) and a second vaccination (comprising one, two, or three or more doses), where the first and second vaccination are typically about 6 months apart from one another. Such vaccination regimens comprising a first and second vaccination may be appropriate for patients 5 years old through 11 years old.

*3 μg (e.g., in 200 uL) Tris doses*

Compositions disclosed herein can be used as part of a 2-dose regimen, where each dose comprises 3 μg of RNA. Such dosages may be suitable for any subject, e.g., subjects 2 through 5 years old or subjects 6 months through 2 years old. In some embodiments, such dosages may be suitable for infant subjects, e.g., less than 12 months old, less than 11 months old, less than 10 months old, less than 9 months old, less than 8 months old, less than 7 months old, or less than 6 months old, or smaller.

3 μg dose compositions disclosed herein can be used as part of a single dose, two dose, three dose, or four or more dose vaccination regimen. A single dose regimen may be appropriate, e.g., when a subject has received a dose of another vaccine, when a subject has already been infected with SARS-CoV-2, or as part of a booster regimen, when the subject has received a dose of another vaccine, or when a subject experienced one or more adverse effects in a

first vaccination. A three dose regimen may be appropriate for immunocompromised patients, e.g., patients who have undergone a solid organ transplant.

3 μg dose compositions disclosed herein may also be used in vaccination regimens that comprise a first vaccination (comprising one, two, or three or more doses) and a second vaccination (comprising one, two, or three or more doses), where the first and second vaccination are typically about 6 months apart. Such vaccination regimens comprising a first and second vaccination may be appropriate for patients 2 years old through 5 years old or for patients 6 months through 2 years old. In some embodiments, such vaccination regimens comprising a first and second vaccination may be appropriate for infant subjects, e.g., less than 12 months old, less than 11 months old, less than 10 months old, less than 9 months old, less than 8 months old, less than 7 months old, or less than 6 months old, or smaller.

## Example 3: Exemplary Dosing Regimens

In some embodiments, compositions and methods disclosed herein can be used in accordance with an exemplary vaccination regimen as illustrated in Figure 3.

## Primary Dosing Regimens

In some embodiments, subjects are administered a primary dosing regimen. A primary dosing regimen can comprise one or more doses. For example, in some embodiments, a primary dosing regimen comprises a single dose ($PD_1$). In some embodiments a primary dosing regimen comprises a first dose ($PD_1$) and a second dose ($PD_2$). In some embodiments, a primary dosing regimen comprises a first dose, a second dose, and a third dose ($PD_3$). In some embodiments, a primary dosing regimen comprises a first dose, a second dose, a third dose, and one or more additional doses ($PD_n$) of any one of the pharmaceutical compositions described herein.

In some embodiments, $PD_1$ comprises administering 1 to 100 ug of RNA. In some embodiments, $PD_1$ comprises administering 1 to 60 ug of RNA In some embodiments, $PD_1$ comprises administering 1 to 50 ug of RNA. In some embodiments, $PD_1$ comprises administering 1 to 30 ug of RNA. In some embodiments, $PD_1$ comprises administering about 3 ug of RNA. In some embodiments, $PD_1$ comprises administering about 5 ug of RNA. In some embodiments, $PD_1$ comprises administering about 10 ug of RNA. In some embodiments, $PD_1$ comprises administering about 15 ug of RNA. In some embodiments, $PD_1$ comprises administering about 20 ug of RNA. In some embodiments, PDicomprises administering about 30 ug of RNA. In some embodiments, $PD_1$ comprises administering about 50 ug of RNA. In some embodiments, $PD_1$ comprises administering about 60 ug of RNA.

In some embodiments, $PD_2$ comprises administering 1 to 100 ug of RNA. In some embodiments, $PD_2$ comprises administering 1 to 60 ug of RNA. In some embodiments, $PD_2$ comprises administering 1 to 50 ug of RNA. In some embodiments, $PD_2$ comprises administering 1 to 30 ug of RNA. In some embodiments, $PD_2$ comprises administering about 3 ug. In some embodiments, PDzcomprises administering about 5 ug of RNA. In some embodiments, $PD_2$ comprises administering about 10 ug of RNA. In some embodiments, $PD_2$ comprises administering about 15 ug of RNA. In some embodiments, $PD_2$ comprises administering about 20 ug RNA. In some embodiments, $PD_2$ comprises administering about 30 ug of RNA. In some embodiments, PDzcomprises administering about 50 ug of RNA. In some embodiments, $PD_2$ comprises administering about 60 ug of RNA.

In some embodiments, $PD_3$ comprises administering 1 to 100 ug of RNA. In some embodiments, $PD_3$ comprises administering 1 to 60 ug of RNA. In some embodiments, $PD_3$ comprises administering 1 to 50 ug of RNA. In some embodiments, $PD_3$ comprises administering 1 to 30 ug of RNA. In some embodiments, $PD_3$ comprises administering about 3 ug of RNA. In some embodiments, $PD_3$ comprises administering about 5 ug of RNA. In some embodiments, $PD_3$ comprises administering about 10 ug of RNA. In some embodiments, $PD_3$ comprises administering about 15 ug of RNA. In some embodiments, $PD_3$ comprises administering about 20 ug of RNA. In some embodiments, $PD_3$ comprises administering about 30 ug of RNA. In some embodiments, $PD_3$ comprises administering about 50 ug of RNA. In some embodiments, $PD_3$ comprises administering about 60 ug of RNA.

In some embodiments, $PD_n$ comprises administering 1 to 100 ug of RNA. In some embodiments, $PD_n$ comprises administering 1 to 60 ug of RNA. In some embodiments, $PD_n$ comprises administering 1 to 50 ug of RNA. In some embodiments, $PD_n$ comprises administering 1 to 30 ug of RNA. In some embodiments, $PD_n$ comprises administering about 3 ug of RNA. In some embodiments, $PD_n$ comprises administering about 5 ug of RNA. In some embodiments, $PD_n$ comprises administering about 10 ug of RNA. In some embodiments, $PD_n$ comprises administering about 15 ug of RNA. In some embodiments, $PD_n$ comprises administering about 20 ug of RNA. In some embodiments, $PD_n$ comprises administering about 30 ug of RNA. In some embodiments, $PD_n$ comprises administering about 50 ug of RNA. In some embodiments, $PD_n$ comprises administering about 60 ug of RNA.

In some embodiments, $PD_1$ comprises an RNA encoding a SARS-CoV-2 Spike protein or an immunogenic fragment thereof from the Wuhan strain. In some embodiments, $PD_1$ comprises an RNA encoding a Spike protein or an immunogenic fragment thereof from a SARS-CoV-2 strain that is prevalent and/or spreading rapidly in a relevant juris-

diction. In some embodiments, $PD_1$ comprises an RNA encoding a SARS-CoV-2 Spike protein or an immunogenic fragment thereof comprising one or more mutations from an alpha variant. In some embodiments, $PD_1$ comprises an RNA encoding a SARS-CoV-2 Spike protein or an immunogenic fragment thereof comprising one or more mutations from a delta variant. In some embodiments, $PD_1$ comprises an RNA encoding a SARS-CoV-2 Spike protein or an immunogenic fragment thereof comprising one or more mutations from a beta variant. In some embodiments, $PD_1$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, $PD_1$ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, $PD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, $PD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $PD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $PD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $PD_2$ comprises an RNA encoding a SARS-CoV-2 Spike protein or an immunogenic fragment thereof from the Wuhan strain. In some embodiments, $PD_2$ comprises an RNA encoding a Spike protein or an immunogenic fragment thereof from a SARS-CoV-2 strain that is prevalent and/or spreading rapidly in a relevant jurisdiction. In some embodiments, $PD_2$ comprises an RNA encoding a SARS-CoV-2 Spike protein or an immunogenic fragment thereof comprising one or more mutations from an alpha variant. In some embodiments, $PD_2$ comprises an RNA encoding a SARS-CoV-2 Spike protein or an immunogenic fragment thereof comprising one or more mutations from a delta variant. In some embodiments, $PD_2$ comprises an RNA encoding a SARS-CoV-2 Spike protein or an immunogenic fragment thereof comprising one or more mutations from a beta variant. In some embodiments, $PD_2$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, $PD_2$ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, $PD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, $PD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $PD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $PD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $PD_3$ comprises an RNA encoding a SARS-CoV-2 Spike protein or an immunogenic fragment thereof from the Wuhan strain. In some embodiments, $PD_3$ comprises an RNA encoding a Spike protein or an immunogenic fragment thereof from a SARS-CoV-2 strain that is prevalent and/or spreading rapidly in a relevant jurisdiction. In some embodiments, $PD_3$ comprises an RNA encoding a SARS-CoV-2 Spike protein or an immunogenic fragment thereof comprising one or more mutations from an alpha variant. In some embodiments, $PD_3$ comprises an RNA encoding a SARS-CoV-2 Spike protein or an immunogenic fragment thereof comprising one or more mutations from a delta variant. In some embodiments, $PD_3$ comprises an RNA encoding a SARS-CoV-2 Spike protein or an immunogenic fragment thereof comprising one or more mutations from a beta variant. In some embodiments, $PD_3$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, $PD_3$ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, $PD_3$

comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, $PD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $PD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $PD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $PD_n$ comprises an RNA encoding a SARS-CoV-2 Spike protein or an immunogenic fragment thereof from the Wuhan strain. In some embodiments, $PD_n$ comprises an RNA encoding a Spike protein or an immunogenic fragment thereof from a SARS-CoV-2 strain that is prevalent and/or spreading rapidly in a relevant jurisdiction. In some embodiments, $PD_n$ comprises an RNA encoding a SARS-CoV-2 Spike protein or an immunogenic fragment thereof comprising one or more mutations from an alpha variant. In some embodiments, $PD_n$ comprises an RNA encoding a SARS-CoV-2 Spike protein or an immunogenic fragment thereof comprising one or more mutations from a delta variant. In some embodiments, $PD_n$ comprises an RNA encoding a SARS-CoV-2 Spike protein or an immunogenic fragment thereof comprising one or more mutations from a beta variant. In some embodiments, $PD_n$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, $PD_n$ comprises an RNA encoding a SARS-CoV-2 Spike protein or an immunogenic fragment thereof from the Wuhan strain and one or more additional RNAs encoding a Spike protein or an immunogenic fragment thereof from a SARS-CoV-2 strain that is prevalent and/or spreading rapidly in a relevant jurisdiction. In some embodiments, $PD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an alpha variant. In some embodiments, $PD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $PD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $PD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $PD_1$, $PD_2$, $PD_3$, and $PD_n$ can each independently comprise a plurality of (e.g., at least two) mRNA compositions described herein. In some embodiments $PD_1$, $PD_2$, $PD_3$, and $PD_n$ can each independently comprise a first and a second mRNA composition. In some embodiments, at least one of a plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant. In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurarity of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, a plurality of mRNA compositions given in $PD_1$, $PD_2$, $PD_3$, and/or $PD_n$ can each independently comprise at least two different mRNA constructs (e.g., differing in at protein-encoding sequences). For example, in some embodiments a plurality of mRNA compositions given in $PD_1$, $PD_2$, $PD_3$, and/or $PD_n$ can each independently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in $PD_1$, $PD_2$, $PD_3$, and/or $PD_n$ can each independently comprise an mRNA

encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an omicron variant (e.g., a BA.1, BA.2, BA.4/5, BA.2.75, BA.2.75.2, BA.4.6, or XBB variant).

In some embodiments, each of a plurality of mRNA compositions given in $PD_1$, $PD_2$, $PD_3$, and/or $PD_n$ can independently comprise at least two mRNAs, each encoding a SARS-CoV-2 S protein or an immunogenic fragment thereof comprising one or more mutations from a distinct variant that is prevalent and/or spreading rapidly in a relevant jurisdiction. In some embodiments, each of a plurality of mRNA compositions given in $PD_1$, $PD_2$, $PD_3$, and/or $PD_n$ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments, each of a plurality of mRNA compositions given in $PD_1$, $PD_2$, $PD_3$, and/or $PD_n$ can independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, each of a plurality of mRNA compositions given in $PD_1$, $PD_2$, $PD_3$, and/or $PD_n$ can independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $PD_1$, $PD_2$, $PD_3$, and/or $PD_n$ each comprise a plurality of mRNA compositions, wherein each mRNA composition is separately administered to a subject. For example, in some embodiments each mRNA composition is administered via intramuscular injection at different injection sites. For example, in some embodiments, a first and second mRNA composition given in $PD_1$, $PD_2$, $PD_3$, and/or $PD_n$ are separately administered to different arms of a subject via intramuscular injection.

In some embodiments, $PD_1$, $PD_2$, $PD_3$, and/or $PD_n$ comprise administering a plurality of RNA molecules, wherein each RNA molecule encodes a Spike protein comprising mutations from a different SARS-CoV-2 variant, and wherein the plurality of RNA molecules are administered to the subject in a single formulation. In some embodiments, the single formulation comprises an RNA encoding a Spike protein or an immunogenic variant thereof from the Wuhan strain and an RNA encoding a SARS-CoV-2 Spike protein or an immunogenic fragment thereof comprising one or more mutations from an alpha variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, the single formulation comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, the length of time between $PD_1$ and $PD_2$ ($PI_1$) is at least about 1 week, at least about 2 weeks, at least about 3 weeks, or at least about 4 weeks. In some embodiments, $PI_1$ is about 1 week to about 12 weeks. In some embodiments, $PI_1$ is about 1 week to about 10 weeks. In some embodiments, $PI_1$ is about 2 weeks to about 10 weeks. In some embodiments, $PI_1$ is about 2 weeks to about 8 weeks. In some embodiments, $PI_1$ is about 3 weeks to about 8 weeks. In some embodiments, $PI_1$ is about 4 weeks to about 8 weeks. In some embodiments, $PI_1$ is about 6 weeks to about 8 weeks. In some embodiments $PI_1$ is about 3 to about 4 weeks. In some embodiments, $PI_1$ is about 1 week. In some embodiments, $PI_1$ is about 2 weeks. In some embodiments, $PI_1$ is about 3 weeks. In some embodiments, $PI_1$ is about 4 weeks. In some embodiments, $PI_1$ is about 5 weeks. In some embodiments, $PI_1$ is about 6 weeks. In some embodiments, $PI_1$ is about 7 weeks. In some embodiments, $PI_1$ is about 8 weeks. In some embodiments, $PI_1$ is about 9 weeks. In some embodiments, $PI_1$ is about 10 weeks. In some embodiments, $PI_1$ is about 11 weeks. In some embodiments, $PI_1$ is about 12 weeks.

In some embodiments, the length of time between $PD_2$ and $PD_3$ ($PI_2$) is at least about 1 week, at least about 2 weeks, or at least about 3 weeks. In some embodiments, $PI_2$ is about 1 week to about 12 weeks. In some embodiments, $PI_2$ is about 1 week to about 10 weeks. In some embodiments, $PI_2$ is about 2 weeks to about 10 weeks. In some embodiments, $PI_2$ is about 2 weeks to about 8 weeks. In some embodiments, $PI_2$ is about 3 weeks to about 8 weeks. In some embodiments, $PI_2$ is about 4 weeks to about 8 weeks. In some embodiments, $PI_2$ is about 6 weeks to about 8 weeks. In some embodiments $PI_2$ is about 3 to about 4 weeks. In some embodiments, $PI_2$ is about 1 week. In some embodiments, $PI_2$ is about 2 weeks. In some embodiments, $PI_2$ is about 3 weeks. In some embodiments, $PI_2$ is about 4 weeks. In some embodiments, $PI_2$ is about 5 weeks. In some embodiments, $PI_2$ is about 6 weeks. In some embodiments, $PI_2$ is about 7 weeks. In some embodiments, $PI_2$ is about 8 weeks. In some embodiments, $PI_2$ is about 9 weeks. In some

embodiments, $PI_2$ is about 10 weeks. In some embodiments, $PI_2$ is about 11 weeks. In some embodiments, $PI_2$ is about 12 weeks.

In some embodiments, the length of time between $PD_3$ and a subsequent dose that is part of the Primary Dosing Regimen, or between doses for any dose beyond $PD_3$ ($PI_n$) is each separately and independently selected from: about 1 week or more, about 2 weeks or more, or about 3 weeks or more. In some embodiments, $PI_n$ is about 1 week to about 12 weeks. In some embodiments, $PI_n$ is about 1 week to about 10 weeks. In some embodiments, $PI_n$ is about 2 weeks to about 10 weeks. In some embodiments, $PI_n$ is about 2 weeks to about 8 weeks. In some embodiments, $PI_n$ is about 3 weeks to about 8 weeks. In some embodiments, $PI_n$ is about 4 weeks to about 8 weeks. In some embodiments, $PI_n$ is about 6 weeks to about 8 weeks. In some embodiments $PI_n$ is about 3 to about 4 weeks. In some embodiments, $PI_2$ is about 1 week. In some embodiments, $PI_n$ is about 2 weeks. In some embodiments, $PI_n$ is about 3 weeks. In some embodiments, $PI_n$ is about 4 weeks. In some embodiments, $PI_n$ is about 5 weeks. In some embodiments, $PI_n$is about 6 weeks. In some embodiments, $PI_n$ is about 7 weeks. In some embodiments, $PI_n$is about 8 weeks. In some embodiments, $PI_n$ is about 9 weeks. In some embodiments, $PI_n$ is about 10 weeks. In some embodiments, $PI_n$ is about 11 weeks. In some embodiments, $PI_n$ is about 12 weeks.

In some embodiments, one or more compositions adminstered in $PD_1$ are formulated in a Tris buffer. In some embodiments, one or more compositions administered in $PD_2$ are formulated in a Tris buffer. In some embodiments, one or more compositions administering in $PD_3$ are formulated in a Tris buffer. In some embodiments, one or more compositions adminsitered in $PD_n$ are formulated in a Tris buffer.

In some embodiments, the primary dosing regimen comprises administering two or more mRNA compositions described herein, and at least two of the mRNA compositions have different formulations. In some embodiments, the primary dosing regimen comprises $PD_1$ and $PD_2$, where $PD_1$ comprises administering an mRNA formulated in a Tris buffer and $PD_2$ comprises administering an mRNA formulated in a PBS buffer. In some embodiments, the primary dosing regimen comprises $PD_1$ and $PD_2$, where $PD_1$ comprises administering an mRNA formulated in a PBS buffer and $PD_2$ comprises administering an mRNA formulated in a Tris buffer.

In some embodiments, one or more mRNA compositions given in $PD_1$, $PD_2$, $PD_3$, and/or $PD_n$ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in $PD_1$, $PD_2$, $PD_3$, and/or $PD_n$ are separately administered, for example, in some embodiments via intramuscular injection, at different injection sites. For example, in some embodiments, an influenza vaccine and one or more SARS-CoV-2 mRNA compositions described herein given in $PD_1$, $PD_2$, $PD_3$, and/or $PD_n$ are separately administered to different arms of a subject via intramuscular injection.

**Booster Dosing Regimens**

In some embodiments, methods of vaccination disclosed herein comprise one or more Booster Dosing Regimens. The Booster Dosing Regimens disclosed herein comprise one or more doses. In some embodiments, a Booster Dosing Regimen is administered to patients who have been administered a Primary Dosing Regimen (e.g., as described herein). In some embodiments a Booster Dosing Regimen is administered to patients who have not received a pharmaceutical composition disclosed herein. In some embodiments a Booster Dosing Regimen is administered to patients who have been previously vaccinated with a COVID-19 vaccine that is different from the vaccine administered in a Primary Dosing Regimen.

In some embodiments, the length of time between the Primary Dosing Regimen and the Booster Dosing Regimen is at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 2 months, at least 3 months, at least 4 months, at least 5 months, at least 6 months, at least 7 months, at least 8 months, at least 9 months, at least 10 months, at least 11 months, or at least 12 months or longer. In some embodiments, the length of time between the Primary Dosing Regimen and the Booster Dosing Regimen is about 1 month. In some embodiments, the length of time between the Primary Dosing Regimen and the Booster Dosing Regimen is at least about 2 months. In some embodiments, the length of time between the Primary Dosing Regimen and the Booster Dosing Regimen is at least about 3 months. In some embodiments, the length of time between the Primary Dosing Regimen and the Booster Dosing Regimen is at least about 4 months. In some embodiments, the length of time between the Primary Dosing Regimen and the Booster Dosing Regimen is at least about 5 months. In some embodiments, the length of time between the Primary Dosing Regimen and the Booster Dosing Regimen is at least about 6 months. In some embodiments, the

length of time between the Primary Dosing Regimen and the Booster Dosing Regimen is from about 1 month to about 48 months. In some embodiments, the length of time between the Primary Dosing Regimen and the Booster Dosing Regimen is from about 1 month to about 36 months. In some embodiments, the length of time between the primary dosing regimen and the Booster Dosing Regimen is from about 1 month to about 24 months. In some embodiments, the length of time between the Primary Dosing Regimen and the Booster Dosing Regimen is from about 2 months to about 24 months. In some embodiments, the length of time between the Primary Dosing Regimen and the Booster Dosing Regimen is from about 3 months to about 24 months. In some embodiments, the length of time between the primary dosing regimen and the Booster Dosing Regimen is from about 3 months to about 18 months. In some embodiments, the length of time between the primary dosing regimen and the Booster Dosing Regimen is from about 3 months to about 12 months. In some embodiments, the length of time between the primary dosing regimen and the Booster Dosing Regimen is from about 6 months to about 12 months. In some embodiments, the length of time between the Primary Dosing Regimen and the Booster Dosing Regimen is from about 3 months to about 9 months. In some embodiments, the length of time between the Primary Dosing Regimen and the Booster Dosing Regimen is from about 5 months to about 7 months. In some embodiments, the length of time between the Primary Dosing Regimen and the Booster Dosing Regimen is about 6 months.

In some embodiments, subjects are administered a Booster Dosing Regimen. A Booster dosing regimen can comprise one or more doses. For example, in some embodiments, a Booster Dosing Regimen comprises a single dose ($BD_1$). In some embodiments a Booster Dosing Regimen comprises a first dose ($BD_1$) and a second dose ($BD_2$). In some embodiments, a Booster Dosing Regimen comprises a first dose, a second dose, and a third dose ($BD_3$). In some embodiments, a Booster Dosing Regimen comprises a first dose, a second dose, a third dose, and one or more additional doses ($BD_n$) of any one of the pharmaceutical compositions described herein.

In some embodiments, $BD_1$ comprises administering 1 to 100 ug of RNA. In some embodiments, $BD_1$ comprises administering 1 to 60 ug of RNA. In some embodiments, $BD_1$ comprises administering 1 to 50 ug of RNA. In some embodiments, $BD_1$ comprises administering 1 to 30 ug of RNA. In some embodiments, $BD_1$ comprises administering about 3 ug of RNA. In some embodiments, $BD_1$ comprises administering about 5 ug of RNA. In some embodiments, $BD_1$ comprises administering about 10 ug of RNA. In some embodiments, $BD_1$ comprises administering about 15 ug of RNA. In some embodiments, $BD_1$ comprises administering about 20 ug of RNA. In some embodiments, $BD_1$ comprises administering about 30 ug of RNA. In some embodiments, $BD_1$ comprises administering about 50 ug of RNA. In some embodiments, $BD_1$ comprises administering about 60 ug of RNA.

In some embodiments, $BD_2$ comprises administering 1 to 100 ug of RNA. In some embodiments, $BD_2$ comprises administering 1 to 60 ug of RNA. In some embodiments, $BD_2$ comprises administering 1 to 50 ug of RNA. In some embodiments, $BD_2$ comprises administering 1 to 30 ug of RNA. In some embodiments, $BD_2$ comprises administering about 3 ug. In some embodiments, $BD_2$ comprises administering about 5 ug of RNA. In some embodiments, $BD_2$ comprises administering about 10 ug of RNA. In some embodiments, $BD_2$ comprises administering about 15 ug of RNA. In some embodiments, $BD_2$ comprises administering about 20 ug RNA. In some embodiments, $BD_2$ comprises administering about 30 ug of RNA. In some embodiments, $BD_2$ comprises administering about 50 ug of RNA. In some embodiments, BDzcomprises administering about 60 ug of RNA.

In some embodiments, $BD_3$ comprises administering 1 to 100 ug of RNA. In some embodiments, $BD_3$ comprises administering 1 to 60 ug of RNA. In some embodiments, $BD_3$ comprises administering 1 to 50 ug of RNA. In some embodiments, $BD_3$ comprises administering 1 to 30 ug of RNA. In some embodiments, $BD_3$ comprises administering about 3 ug of RNA. In some embodiments, $BD_3$ comprises administering about 5 ug of RNA. In some embodiments, $BD_3$ comprises administering about 10 ug of RNA. In some embodiments, $BD_3$ comprises administering about 15 ug of RNA. In some embodiments, $BD_3$ comprises administering about 20 ug of RNA. In some embodiments, $BD_3$ comprises administering about 30 ug of RNA. In some embodiments, $BD_3$ comprises administering about 50 ug of RNA. In some embodiments, $BD_3$ comprises administering about 60 ug of RNA.

In some embodiments, $BD_n$ comprises administering 1 to 100 ug of RNA. In some embodiments, $BD_n$ comprises administering 1 to 60 ug of RNA. In some embodiments, $BD_n$ comprises administering 1 to 50 ug of RNA. In some embodiments, $BD_n$ comprises administering 1 to 30 ug of RNA. In some embodiments, $BD_n$ comprises administering about 3 ug of RNA. In some embodiments, $BD_n$ comprises administering about 5 ug of RNA. In some embodiments, $BD_n$ comprises administering about 10 ug of RNA. In some embodiments, $BD_n$ comprises administering about 15 ug of RNA. In some embodiments, $BD_n$ comprises administering about 20 ug of RNA. In some embodiments, $BD_n$ comprises administering about 30 ug of RNA. In some embodiments, $BD_n$ comprises administering about 60 ug of RNA. In some embodiments, $BD_n$ comprises administering about 50 ug of RNA.

In some embodiments, $BD_1$ comprises an RNA encoding a SARS-CoV-2 Spike protein or an immunogenic fragment thereof from the Wuhan strain. In some embodiments, $BD_1$ comprises an RNA encoding a Spike protein or an immunogenic fragment thereof from a SARS-CoV-2 strain that is prevalent and/or spreading rapidly in a relevant jurisdiction. In some embodiments, $BD_1$ comprises an RNA encoding a SARS-CoV-2 Spike protein or an immunogenic fragment thereof comprising one or more mutations from an alpha variant. In some embodiments, $BD_1$ comprises an

RNA encoding a SARS-CoV-2 Spike protein or an immunogenic fragment thereof comprising one or more mutations from a delta variant. In some embodiments, $BD_1$ comprises an RNA encoding a SARS-CoV-2 Spike protein or an immunogenic fragment thereof comprising one or more mutations from a beta variant. In some embodiments, $BD_1$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $BD_1$ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, $BD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, $BD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $BD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $BD_1$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $BD_2$ comprises an RNA encoding a SARS-CoV-2 Spike protein or an immunogenic fragment thereof from the Wuhan strain. In some embodiments, $BD_2$ comprises an RNA encoding a Spike protein or an immunogenic fragment thereof from a SARS-CoV-2 strain that is prevalent and/or spreading rapidly in a relevant jurisdiction. In some embodiments, $BD_2$ comprises an RNA encoding a SARS-CoV-2 Spike protein or an immunogenic fragment thereof comprising one or more mutations from an alpha variant. In some embodiments, $BD_2$ comprises an RNA encoding a SARS-CoV-2 Spike protein or an immunogenic fragment thereof comprising one or more mutations from a delta variant. In some embodiments, $BD_2$ comprises an RNA encoding a SARS-CoV-2 Spike protein or an immunogenic fragment thereof comprising one or more mutations from a beta variant. In some embodiments, $BD_2$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments, $BD_2$ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, $BD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, $BD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $BD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $BD_2$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.4.6, BA.2.75, BA.2.75.2, or XBB variant).

In some embodiments, $BD_3$ comprises an RNA encoding a SARS-CoV-2 Spike protein or an immunogenic fragment thereof from the Wuhan strain. In some embodiments, $BD_3$ comprises an RNA encoding a Spike protein or an immunogenic fragment thereof from a SARS-CoV-2 strain that is prevalent and/or spreading rapidly in a relevant jurisdiction. In some embodiments, $BD_3$ comprises an RNA encoding a SARS-CoV-2 Spike protein or an immunogenic fragment thereof comprising one or more mutations from an alpha variant. In some embodiments, $BD_3$ comprises an RNA encoding a SARS-CoV-2 Spike protein or an immunogenic fragment thereof comprising one or more mutations from a delta variant. In some embodiments, $BD_3$ comprises an RNA encoding a SARS-CoV-2 Spike protein or an immunogenic fragment thereof comprising one or more mutations from a beta variant. In some embodiments, $BD_3$ comprises an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $BD_3$ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, $BD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one

or more mutations from a alpha variant. In some embodiments, $BD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $BD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $BD_3$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $BD_n$ comprises an RNA encoding a SARS-CoV-2 Spike protein or an immunogenic fragment thereof from the Wuhan strain. In some embodiments, $BD_n$ comprises an RNA encoding a Spike protein or an immunogenic fragment thereof from a SARS-CoV-2 strain that is prevalent and/or spreading rapidly in a relevant jurisdiction. In some embodiments, $BD_n$ comprises an RNA encoding a SARS-CoV-2 Spike protein or an immunogenic fragment thereof comprising one or more mutations from an alpha variant. In some embodiments, $BD_n$ comprises an RNA encoding a SARS-CoV-2 Spike protein or an immunogenic fragment thereof comprising one or more mutations from a delta variant. In some embodiments, $BD_n$ comprises an RNA encoding a SARS-CoV-2 Spike protein or an immunogenic fragment thereof comprising one or more mutations from a beta variant. In some embodiments, $BD_n$ comprises an RNA encoding a SARS-CoV-2 Spike protein or an immunogenic fragment thereof comprising one or more mutations from an omicron variant(e.g., a BA.1, BA.2, BA.4/5, BA.2.75, BA.2.75.2, BA.4.6, or XBB variant).

In some embodiments, $BD_n$ comprises an RNA encoding a SARS-CoV-2 Spike protein or an immunogenic fragment thereof from the Wuhan strain and one or more RNA encoding a Spike protein or an immunogenic fragment thereof from a SARS-CoV-2 strain that is prevalent and/or spreading rapidly in a relevant jurisdiction. In some embodiments, $BD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a alpha variant. In some embodiments, $BD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a delta variant. In some embodiments, $BD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from a beta variant. In some embodiments, $BD_n$ comprises an RNA encoding a SARS-CoV-2 Spike protein or an immunogenic fragment thereof from the Wuhan strain and an RNA encoding a SARS-CoV-2 Spike protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, $BD_1$, $BD_2$, $BD_3$, and $BD_n$ can each independently comprise a plurality of (e.g., at least two) mRNA compositions described herein. In some embodiments $BD_1$, $BD_2$, $BD_3$, and $BD_n$ can each independently comprise a first and a second mRNA composition. In some embodiments, $BD_1$, $BD_2$, $BD_3$, and $BD_n$ can each independently comprise a plurality of (e.g., at least two) mRNA compositions, wherein , at least one of the plurality of mRNA compositions comprises BNT162b2 (e.g., as described herein). In some embodiments, at least one of a plurality of mRNA compositions comprises an mRNA encoding a SARS-CoV-2 S protein or an immunogenic fragment thereof from a different SARS-CoV-2 variant (e.g., a variant that is prevalent or rapidly spreading in a relevant jurisdiction, e.g., a variant disclosed herein). In some embodiments, at least one of a plurality of mRNA compositions comprises an mRNA encoding a SARS-CoV-2 S protein or an immunogenic fragment thereof from a Wuhan strain of SARS-CoV-2. In some embodiments, at least one of a plurality of mRNA compositions comprises an RNA encoding a SARS-CoV-2 S protein or an immunogenic fragment thereof comprising one or more mutations from a variant that is prevalent and/or spreading rapidly in a relevant jurisdiction. In some embodiments, at least one of a plurality of mRNA compositions comprises an mRNA encoding a SARS-CoV-2 S protein or an immunogenic fragment thereof comprising one or more mutations from an alpha variant. In some embodiments, at least one of a plurality of mRNA compositions comprises an mRNA encoding a SARS-CoV-2 S protein or an immunogenic fragment thereof comprising one or more mutations from a delta variant. In some embodiments, at least one of a plurality of mRNA compositions comprises an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, a plurality of mRNA compositions given in $BD_1$, $BD_2$, $BD_3$, and/or $BD_n$ can each indendently comprise at least two different mRNA constructs (e.g., mRNA constructs having differing protein-encoding sequences). For example, in some embodiments a plurality of mRNA compositions given in $BD_1$, $BD_2$, $BD_3$, and/or $BD_n$ can each indendently comprise an mRNA encoding a SARS-CoV-2 S protein or an immunogenic fragment thereof from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some embodiments a plurality of mRNA compositions given in $BD_1$, $BD_2$, $BD_3$, and/or $BD_n$ can each independently

comprise an mRNA encoding a SARS-CoV-2 S protein or an immunogenic fragment thereof derived from a Wuhan strain of SARS-CoV-2 and an mRNA encoding a SARS-CoV-2 S protein or an immunogenic fragment thereof comprising one or more mutations from a variant that is prevalent and/or spreading rapidly in a relevant jurisdiction. In some such embodiments, a variant can be an alpha variant. In some such embodiments, a variant can be a delta variant. In some such embodiments a variant can be an omicron variant (e.g., a BA.1, BA.2, BA.4/5, BA.2.75, BA.2.75.2, BA.4.6, or XBB variant).

In some embodiments, a plurality of mRNA compositions given in $BD_1$, $BD_2$, $BD_3$, and/or $BD_n$ can each independently comprise at least two mRNAs each encoding a SARS-CoV-2 S protein or an immunogenic fragment thereof comprising one or more mutations from a distinct variant that is prevalent and/or spreading rapidly in a relevant jurisdiction. In some embodiments a plurality of mRNA compositions given in $BD_1$, $BD_2$, $BD_3$, and/or $BD_n$ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from a delta variant. In some embodiments a plurality of mRNA compositions given in $BD_1$, $BD_2$, $BD_3$, and/or $BD_n$ can each independently comprise an mRNA encoding a SARS-CoV-2 S protein or an immunogenic fragment thereof from an alpha variant and an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant). In some embodiments a plurality of mRNA compositions given in $BD_1$, $BD_2$, $BD_3$, and/or $BD_n$ can each independently comprise an mRNA encoding a SARS-CoV-2 S protein or an immunogenic fragment thereof from a delta variant and an mRNA encoding a SARS-CoV-2 S protein or an immunogenic fragment thereof comprising one or more mutations from an omicron variant (e.g., a BA.1, BA.2, BA.4/5, BA.2.75, BA.2.75.2, BA.4.6, or XBB variant).

In some embodiments, a plurality of mRNA compositions given in $BD_1$, $BD_2$, $BD_3$, and/or $BD_n$ are separately administered to a subject, for example, in some embodiments via intramuscular injection, at different injection sites. For example, in some embodiments, a first and second mRNA composition given in $BD_1$, $BD_2$, $BD_3$, and/or $BD_n$ are separately administered to different arms of a subject via intramuscular injection.

In some embodiments, the length of time between $BD_1$ and $BD_2$ ($BI_1$) is at least about 1 week, at least about 2 weeks, at least about 3 weeks, or at least about 4 weeks. In some embodiments, $BI_1$ is about 1 week to about 12 weeks. In some embodiments, $BI_1$ is about 1 week to about 10 weeks. In some embodiments, $BI_1$ is about 2 weeks to about 10 weeks. In some embodiments, $BI_1$ is about 2 weeks to about 8 weeks. In some embodiments, $BI_1$ is about 3 weeks to about 8 weeks. In some embodiments, $BI_1$ is about 4 weeks to about 8 weeks. In some embodiments, $BI_1$ is about 6 weeks to about 8 weeks. In some embodiments $BI_1$ is about 3 to about 4 weeks. In some embodiments, $BI_1$ is about 1 week. In some embodiments, $BI_1$ is about 2 weeks. In some embodiments, $BI_1$ is about 3 weeks. In some embodiments, $BI_1$ is about 4 weeks. In some embodiments, $BI_1$ is about 5 weeks. In some embodiments, $BI_1$ is about 6 weeks. In some embodiments, $BI_1$ is about 7 weeks. In some embodiments, $BI_1$ is about 8 weeks. In some embodiments, $BI_1$ is about 9 weeks. In some embodiments, $BI_1$ is about 10 weeks.

In some embodiments, the length of time between $BD_2$ and $BD_3$ ($BI_2$) is at least about 1 week, at least about 2 weeks, or at least about 3 weeks. In some embodiments, $BI_2$ is about 1 week to about 12 weeks. In some embodiments, $BI_2$ is about 1 week to about 10 weeks. In some embodiments, $BI_2$ is about 2 weeks to about 10 weeks. In some embodiments, $BI_2$ is about 2 weeks to about 8 weeks. In some embodiments, $BI_2$ is about 3 weeks to about 8 weeks. In some embodiments, $BI_2$ is about 4 weeks to about 8 weeks. In some embodiments, $BI_2$ is about 6 weeks to about 8 weeks. In some embodiments $BI_2$ is about 3 to about 4 weeks. In some embodiments, $BI_2$ is about 1 week. In some embodiments, $BI_2$ is about 2 weeks. In some embodiments, $BI_2$ is about 3 weeks. In some embodiments, $BI_2$ is about 4 weeks. In some embodiments, $BI_2$ is about 5 weeks. In some embodiments, $BI_2$ is about 6 weeks. In some embodiments, $BI_2$ is about 7 weeks. In some embodiments, $BI_2$ is about 8 weeks. In some embodiments, $BI_2$ is about 9 weeks. In some embodiments, $BI_2$ is about 10 weeks.

In some embodiments, the length of time between $BD_3$ and a subsequent dose that is part of the Booster Dosing Regimen, or between doses for any dose beyond $BD_3$ ($BI_n$) is each separately and independently selected from: about 1 week or more, about 2 weeks or more, or about 3 weeks or more. In some embodiments, $BI_n$ is about 1 week to about 12 weeks. In some embodiments, $BI_n$ is about 1 week to about 10 weeks. In some embodiments, $BI_n$ is about 2 weeks to about 10 weeks. In some embodiments, $BI_n$ is about 2 weeks to about 8 weeks. In some embodiments, $BI_n$ is about 3 weeks to about 8 weeks. In some embodiments, $BI_n$ is about 4 weeks to about 8 weeks. In some embodiments, $BI_n$ is about 6 weeks to about 8 weeks. In some embodiments $BI_n$ is about 3 to about 4 weeks. In some embodiments, $BI_n$ is about 1 week. In some embodiments, $BI_n$ is about 2 weeks. In some embodiments, $BI_n$ is about 3 weeks. In some embodiments, $BI_n$ is about 4 weeks. In some embodiments, $BI_n$ is about 5 weeks. In some embodiments, $BI_n$ is about 6 weeks. In some embodiments, $BI_n$ is about 7 weeks. In some embodiments, $BI_n$ is about 8 weeks. In some embodiments, $BI_n$ is about 9 weeks. In some embodiments, $BI_n$ is about 10 weeks.

In some embodiments, one or more compositions adminstered in $BD_1$ are formulated in a Tris buffer. In some embodiments, one or more compositions administered in $BD_2$ are formulated in a Tris buffer. In some embodiments, one or more compositions administering in $BD_3$ are formulated in a Tris buffer. In some embodiments, one or more

compositions adminsitered in $BD_3$ are formulated in a Tris buffer.

In some embodiments, the Booster dosing regimen comprises administering two or more mRNA compositions described herein, and at least two of the mRNA compositions have differnent formulations. In some embodiments, the Booster dosing regimen comprises $BD_1$ and $BD_2$, where $BD_1$ comprises administering an mRNA formulated in a Tris buffer and $BD_2$ comprises administering an mRNA formulated in a PBS buffer. In some embodiments, the Booster dosing regimen comprises $BD_1$ and $BD_2$, where $BD_1$ comprises administering an mRNA formulated in a PBS buffer and $BD_2$ comprises administering an mRNA formulated in a Tris buffer.

In some embodiments, one or more mRNA compositions given in $BD_1$, $BD_2$, $BD_3$, and/or $BD_n$ can be administered in combination with another vaccine. In some embodiments, another vaccine is for a disease that is not COVID-19. In some embodiments, the disease is one that increases deleterious effects of SARS-CoV-2 when a subject is coinfected with the disease and SARS-CoV-2. In some embodiments, the disease is one that increases the transmission rate of SARS-CoV-2 when a subject is coinfected with the disease and SARS-CoV-2. In some embodiments, another vaccine is a different commerically available vaccine. In some embodiments, the different commercially available vaccine is an RNA vaccine. In some embodiments, the different commercially available vaccine is a polypeptide-based vaccine. In some embodiments, another vaccine (e.g., as described herein) and one or more mRNA compositions given in $BD_1$, $BD_2$, $BD_3$, and/or $BD_n$ are separately administered, for example, in some embodiments via intramuscular injection, at different injection sites. For example, in some embodiments, an influenza vaccine and one or more SARS-CoV-2 mRNA compositions described herein given in $BD_1$, $BD_2$, $BD_3$, and/or $BD_n$ are separately administered to different arms of a subject via intramuscular injection.

**Additional Booster Regimens**

In some embodiments, methods of vaccination disclosed herein comprise administering more than one Booster Dosing Regimen. In some embodiments, more than one Booster Dosing Regimen may need to be administered to increase neutralizing antibody response. In some embodiments, more than one booster dosing regimen may be needed to counteract a SARS-CoV-2 strain that has been shown to have a high likelihood of evading immune response elicited by vaccines that a patient has previously received. In some embodiments, an additional Booster Dosing Regimen is administered to a patient who has been determined to produce low concentrations of neutralizing antibodies. In some embodiments, an additional booster dosing regimen is administered to a patient who has been determined to have a high likelihood of being susceptible to SARS-CoV-2 infection, despite previous vaccination (e.g., an immunocompromised patient, a cancer patient, and/or an organ transplant patient).

The description provided above for the first Booster Dosing Regimen also describes the one or more additional Booster Dosing Regimens. The interval of time between the first Booster Dosing Regimen and a second Booster Dosing Regimen, or between subsequent Booster Dosing Regimens can be any of the acceptable intervals of time described above between the Primary Dosing Regimen and the First Booster Dosing Regimen.

In some embodiments, a dosing regimen comprises a primary regimen and a booster regimen, wherein at least one dose given in the primary regimen and/or the booster regimen comprises a composition comprising an RNA that encodes a S protein or immungenic fragment thereof from a variant that is prevalent or is spreading rapidly in a relevant jurisdiction (e.g., Omicron variant as described herein). For example, in some embodiments, a primary regimen comprises at least 2 doses of BNT162b2 (e.g., encoding a Wuhan strain), for example, given at least 3 weeks apart, and a booster regimen comprises at least 1 dose of a composition comprising RNA that encodes a S protein or immungenic fragment thereof from a variant that is prevalent or is spreading rapidly in a relevant jurisdiction (e.g., Omicron variant as described herein). In some such embodiments, such a dose of a booster regimen may further comprise an RNA that encodes a S protein or immungenic fragment thereof from a Wuhan strain, which can be administered with an RNA that encodes a S protein or immungenic fragment thereof from a variant that is prevalent or is spreading rapidly in a relevant jurisdiction (e.g., Omicron variant as described herein), as a single mixture, or as two separate compositions, for example, at 1:1 weight ratio. In some embodiments, a booster regimen can also comprise at least 1 dose of BNT162b2, which can be administered as a first booster dose or a subsequent booster dose.

In some embodiments, an RNA composition described herein is given as a booster at a dose that is higher than the doses given during a primary regimen (primary doses) and/or the dose given for a first booster, if any. For example, in some embodiments, such a dose may be 60 ug; or in some embodiments such a dose may be higher than 30 ug and lower than 60 ug (e.g., 55 ug, 50 ug, or lower). In some embodiments, an RNA composition described herein is given as a booster at least 3-12 months or 4-12 months, or 5-12 months, or 6-12 months after the last dose (e.g., the last dose of a primary regimen or a first dose of a booster regimen). In some embodiments, the primary doses and/or the first booster dose (if any) may comprise BNT162b2, for example at 30 ug per dose.

In some embodiments, the formulations disclosed herein can be used to carry out any of the dosing regimens described in Table 7 (below).

Table 7: Exemplary Dosing Regimens:

| | Primary Regimen | | | | Time between the last dose of a Primary regimen and a first dose of Booster Regimen | Booster Regimen | | | |
|---|---|---|---|---|---|---|---|---|---|
| # | Dose 1 (Pg RNA) | Dose 2 (Pg RNA) | Time Between Doses 1 and 2 | Dose 1 and Dose 2 Formulation | | Dose 1 (μg RNA) | Dose 2 (μg RNA) | Time Between Doses 1 and 2 | Dose 1 and Dose 2 Formulation |
| 1 | 30 | 30 | 2 to 8 weeks | PBS | At least 2 months | 30 | N/A[1] | N/A | PBS |
| 2 | 30 | 30 | 2 to 8 weeks | PBS | At least 3 months | 30 | N/A[1] | N/A | PBS |
| 3 | 30 | 30 | 2 to 8 weeks | PBS | 6 to 12 months | 30 | N/A[1] | N/A | PBS |
| 4 | 30 | 30 | 2 to 8 weeks | PBS or Tris | 4 to 12 months | 15 | N/A[1] | N/A | PBS or Tris |
| 5 | 30 | 30 | 2 to 8 weeks | PBS or Tris | 4 to 12 months | 10 | N/A[1] | N/A | PBS or Tris |
| 6 | 30 | 30 | 2 to 8 weeks | PBS or Tris | 4 to 12 months | 30 | 30 | 4 to 12 months | PBS or Tris |
| 7 | 30 | 30 | 2 to 8 weeks | PBS or Tris | 4 to 12 months | 30 | 15 | 4 to 12 months | PBS or Tris |
| 8 | 30 | 30 | 2 to 8 weeks | PBS or Tris | 4 to 12 months | 30 | 10 | 4 to 12 months | PBS or Tris |
| 9 | 30 | 30 | 2 to 8 weeks | PBS or Tris | 4 to 12 months | 30 | 60 | 4 to 12 months | PBS or Tris |
| 10 | 30 | 30 | 2 to 8 weeks | PBS or Tris | 4 to 12 months | 30 | >30 to < 60 | 4 to 12 months | PBS or Tris |
| 11 | 30 | 30 | 2 to 8 weeks | PBS or Tris | 4 to 12 months | 30 | 50 | 4 to 12 months | PBS or Tris |
| 12 | 30 | 30 | 2 to 8 weeks | PBS | At least 6 months | 30 | N/A[1] | N/A | PBS |
| 13 | 30 | 30 | ~21 days | PBS | At least 2 months | 30 | N/A[1] | N/A | PBS |
| 14 | 30 | 30 | ~21 days | PBS | At least 3 months | 30 | N/A[1] | N/A | PBS |
| 15 | 30 | 30 | ~21 days | PBS | 6 to 12 months | 30 | N/A[1] | N/A | PBS |
| 16 | 30 | 30 | ~21 days | PBS | At least 6 months | 30 | N/A[1] | N/A | PBS |
| 17 | 30 | 30 | 21 days | PBS | At least 6 months | 15 | 15 | ~21 days | PBS |
| 18 | 30 | 30 | 21 days | PBS | At least 6 months | 15 | 15 | ~21 days | PBS |
| | | | | | | | | | |
| 19 | 30 | 30 | 2 to 8 weeks | PBS | At least 2 months | 30 | N/A[1] | N/A | Tris |

(continued)

| | Primary Regimen | | | | Time between the last dose of a Primary regimen and a first dose of Booster Regimen | Booster Regimen | | | |
|---|---|---|---|---|---|---|---|---|---|
| # | Dose 1 (Pg RNA) | Dose 2 (Pg RNA) | Time Between Doses 1 and 2 | Dose 1 and Dose 2 Formulation | | Dose 1 (μg RNA) | Dose 2 (μg RNA) | Time Between Doses 1 and 2 | Dose 1 and Dose 2 Formulation |
| 20 | 30 | 30 | 2 to 8 weeks | PBS | At least 3 months | 30 | N/A[1] | N/A | Tris |
| 21 | 30 | 30 | 2 to 8 weeks | PBS | 6 to 12 months | 30 | N/A[1] | N/A | Tris |
| 22 | 30 | 30 | 2 to 8 weeks | PBS | At least 6 months | 30 | N/A[1] | N/A | Tris |
| 23 | 30 | 30 | ~21 days | PBS | At least 2 months | 30 | N/A[1] | N/A | Tris |
| 24 | 30 | 30 | ~21 days | PBS | At least 3 months | 30 | N/A[1] | N/A | Tris |
| 25 | 30 | 30 | ~21 days | PBS | 6 to 12 months | 30 | N/A[1] | N/A | Tris |
| 26 | 30 | 30 | ~21 days | PBS | At least 6 months | 30 | N/A[1] | N/A | Tris |
| 27 | 30 | 30 | 21 days | PBS | At least 6 months | 15 | 15 | ~21 days | Tris |
| 28 | 30 | 30 | 21 days | PBS | At least 6 months | 15 | 15 | ~21 days | Tris |
| | | | | | | | | | |
| 29 | 30 | 30 | 2 to 8 weeks | Tris | At least 2 months | 30 | N/A[1] | N/A | Tris |
| 30 | 30 | 30 | 2 to 8 weeks | Tris | At least 3 months | 30 | N/A[1] | N/A | Tris |
| 31 | 30 | 30 | 2 to 8 weeks | Tris | 6 to 12 months | 30 | N/A[1] | N/A | Tris |
| 32 | 30 | 30 | 2 to 8 weeks | Tris | At least 6 months | 30 | N/A[1] | N/A | Tris |
| 33 | 30 | 30 | ~21 days | Tris | At least 2 months | 30 | N/A[1] | N/A | Tris |
| 34 | 30 | 30 | ~21 days | Tris | At least 3 months | 30 | N/A[1] | N/A | Tris |
| 35 | 30 | 30 | ~21 days | Tris | 6 to 12 months | 30 | N/A[1] | N/A | Tris |
| 36 | 30 | 30 | ~21 days | Tris | At least 6 months | 30 | N/A[1] | N/A | Tris |
| 37 | 30 | 30 | 21 days | Tris | At least 6 months | 15 | 15 | ~21 days | Tris |
| 38 | 30 | 30 | 21 days | Tris | At least 6 months | 15 | 15 | ~21 days | Tris |
| | | | | | | | | | |

(continued)

| # | Primary Regimen | | | | Time between the last dose of a Primary regimen and a first dose of Booster Regimen | Booster Regimen | | | |
|---|---|---|---|---|---|---|---|---|---|
| | Dose 1 (Pg RNA) | Dose 2 (Pg RNA) | Time Between Doses 1 and 2 | Dose 1 and Dose 2 Formulation | | Dose 1 (μg RNA) | Dose 2 (μg RNA) | Time Between Doses 1 and 2 | Dose 1 and Dose 2 Formulation |
| 39 | 10 | 10 | 2 to 8 weeks | Tris | At least 2 months | 10 | N/A[1] | N/A | Tris |
| 40 | 10 | 10 | 2 to 8 weeks | Tris | At least 3 months | 10 | N/A[1] | N/A | Tris |
| 41 | 10 | 10 | 2 to 8 weeks | Tris | 6 to 12 months | 10 | N/A[1] | N/A | Tris |
| 42 | 10 | 10 | 2 to 8 weeks | Tris | At least 6 months | 10 | N/A[1] | N/A | Tris |
| 43 | 10 | 10 | ~21 days | Tris | At least 2 months | 10 | N/A[1] | N/A | Tris |
| 44 | 10 | 10 | ~21 days | Tris | At least 3 months | 10 | N/A[1] | N/A | Tris |
| 45 | 10 | 10 | ~21 days | Tris | 6 to 12 months | 10 | N/A[1] | N/A | Tris |
| 46 | 10 | 10 | ~21 days | Tris | At least 6 months | 10 | N/A[1] | N/A | Tris |
| | | | | | | | | | |
| 47 | 3 | 3 | 2 to 8 weeks | Tris | At least 2 months | 3 | N/A[1] | N/A | Tris |
| 48 | 3 | 3 | 2 to 8 weeks | Tris | At least 3 months | 3 | N/A[1] | N/A | Tris |
| 49 | 3 | 3 | 2 to 8 weeks | Tris | 6 to 12 months | 3 | N/A[1] | N/A | Tris |
| 50 | 3 | 3 | 2 to 8 weeks | Tris | At least 6 months | 3 | N/A[1] | N/A | Tris |
| 51 | 3 | 3 | ~21 days | Tris | At least 2 months | 3 | N/A[1] | N/A | Tris |
| 52 | 3 | 3 | ~21 days | Tris | At least 3 months | 3 | N/A[1] | N/A | Tris |
| 53 | 3 | 3 | ~21 days | Tris | 6 to 12 months | 3 | N/A[1] | N/A | Tris |
| 54 | 3 | 3 | ~21 days | Tris | At least 6 months | 3 | N/A[1] | N/A | Tris |
| [1]N/A refers to no dose necessary. | | | | | | | | | |

In some embodiments of certain exemplary dosing regimens as described in Table 7 above, an RNA composition (e.g., as described herein) given in a first dose of a primary regimen can be the same or different from an RNA composition (e.g., as described herein) given in a second dose of a primary regimen. In some embodiments of certain exemplary dosing regimens as described in Table 7 above, an RNA composition (e.g., as described herein) given in a first dose of a booster regimen can be the same or different from an RNA composition (e.g., as described herein) given in a second dose of a booster regimen. In some embodiments of certain exemplary dosing regimens as described in Table 7 above, an RNA composition (e.g., as described herein) given in any dose of a booster regimen can be the same or different

from an RNA composition given in any dose of a primary regimen. For example, in some embodiments of certain exemplary dosing regimens as described in Table 7 above, all doses comprise a composition comprising RNA encoding the same S protein or immunogenic fragment thereof. In some embodiments of certain exemplary dosing regimens as described in Table 7 above, all doses of a primary regimen comprise a composition comprising RNA that encodes the same S protein or immunogenic fragment thereof from a certain coronavirus (e.g., a Wuhan strain of SARS-CoV-2 such as, e.g., BNT162b2) and at least one dose of a booster regimen comprise a composition comprising RNA that encodes a S protein or immunogenic fragment thereof from a different coronavirus (e.g., a variant of SARS-CoV-2 such as in some embodiments an Omicron variant of SARS-CoV-2 (e.g., a BA.1, BA.2, BA.4/5, BA.2.75, BA.2.75.2, BA.4.6, or XBB variant)).

In some embodiments of certain exemplary dosing regimens as described in Table 7 above, at least one dose can comprise at least two RNAs each encoding a S protein or an immunogenic fragment thereof from different strains. For example, in some embodiments, at least one dose can comprise an RNA encoding a S protein or an immunogenic fragment thereof from a Wuhan strain and an RNA encoding a S protein or an immunogenic fragment thereof from a variant that is prevalent or rapidly spreading in a relevant geographical location at the time of adminstration. In some such embodiments, such at least two RNAs can be administered as a single mixture, or as separate compositions. In some embodiments, such at least two RNAs can be present in a given dose at about 1:1 weight ratio. By way of example, in some embodiments of Regimens #9-11 as described in Table 7 above, a first dose or a second dose of a booster regimen can comprise a BNT162b2 RNA construct and RNA encoding a S protein or immungenic fragment thereof from a variant described herein such as Omicron (e.g., a BA.1, BA.2, BA.4/5, BA.2.75, BA.2.75.2, BA.4.6, or XBB variant)), for example, at about 1: 1 weight ratio, while doses of a corresponding primary regimen can comprise a BNT162b2 RNA construct.

In some embodiments of Regimen #6 as described in Table 7 above, a first dose and a second dose of a primary regimen and a first dose and a second dose of a booster regimen each comprise an RNA composition described herein. In some such embodiments, a second dose of a booster regimen may not be necessary.

In some embodiments of Regimen #6 as described in Table 7 above, a first dose and a second dose of a primary regimen each comprise a BNT162b2 RNA construct, and a first dose and a second dose of a booster regimen each comprise an RNA encoding a S protein or an immunogenic fragment thereof from a variant that is prevalent or rapidly spreading in a relevant geographical location at the time of adminstration. In some such embodiments, a second dose of a booster regimen may not be necessary.

In some embodiments of Regimen #6 as descried in Table 7 above, a first dose and a second dose of a primary regimen and a first dose of a booster regimen each comprise a BNT162b2 RNA construct, and a second dose of a booster regimen comprises an RNA encoding a S protein or an immunogenic fragment thereof from a variant that is prevalent or rapidly spreading in a relevant geographical location at the time of adminstration.

For any of the dosing regimens subjects should inform their vaccination provider if they have experienced one or more of the following medical conditions or indications:

- allergies
- myocarditis (inflammation of the heart muscle) or pericarditis (inflammation of the lining outside the heart)
- fever
- a bleeding disorder or are currently taking a blood thinner
- being immunocompromised or being on a medicine that affects the immune system
- pregnancy or plans to become pregnant
- breastfeeding
- have received another COVID-19 vaccine
- have ever fainted in association with an injection

The above conditions or indications should be monitored and/or monitored using the current standard of care as appropriate.

Management of Acute Allergic Reactions: Appropriate medical treatment used to manage immediate allergic reactions must be immediately available in the event an acute anaphylactic reaction occurs following administration of Pfizer-BioNTech COVID-19 Vaccine.

Myocarditis and Pericarditis

Postmarketing data demonstrate increased risks of myocarditis and pericarditis, particularly within 7 days following the second dose. The observed risk is higher among males under 40 years of age than among females and older males. The observed risk is highest in males 12 through 17 years of age. Although some cases required intensive care support, available data from short-term follow up suggest that most individuals have had resolution of symptoms with

conservative management. Information is not yet available about potential long term sequelae. The CDC has published considerations related to myocarditis and pericarditis after vaccination, including for vaccination of individuals with a history of myocarditis or pericarditis (https://www.cdc.gov/vaccines/covid-19/clinical-considerations/myocarditis.html).

Syncope

Syncope (fainting) may occur in association with administration of injectable vaccines, in particular in adolescents. Procedures should be in place to avoid injury from fainting.

Altered Immunocompetence

Immunocompromised persons, including individuals receiving immunosuppressant therapy, may have a diminished immune response to the Pfizer-BioNTech COVID 19 Vaccine.
Citation of documents and studies referenced herein is not intended as an admission that any of the foregoing is pertinent prior art. All statements as to the contents of these documents are based on the information available to the applicants and do not constitute any admission as to the correctness of the contents of these documents.
The description (including the following examples) is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

**Example 4: Induced antibody response and reactogenecity of BNT162b2 or Omicron-specific vaccine as monovalent, bivalent and high dose in participants 55+ years of age**

To test the efficacy and safety of (i) higher doses of RNA vaccines (e.g., as described herein), (ii) RNA vaccines encoding a SARS-CoV-2 S protein having one or more mutations characteristic of an Omicron variant (in the present example, an Omicron BA.1-specific vaccine), and (iii) a bivalent vaccine comprising an RNA encoding a SARS-CoV-2 S protein from a Wuhan variant and RNA encoding a SARS-CoV-2 S protein having one or more mutations characteristic of an Omicron variant (in the present example, an Omicron BA.1-variant), subjects previously administered at least one dose of an RNA vaccine encoding a SARS-CoV-2 S protein of a Wuhan strain were administered one of several booster doses (e.g., as described herein). Specifically, subjects who had previously been administered two doses of 30 ug of an RNA vaccine encoding a SARS-CoV-2 S protein from a Wuhan strain (in the present example, BNT162b2), and a third dose of 30 ug of RNA encoding a SARS-CoV-2 S protein of a Wuhan strain (also BNT162b2 in the present example), were administered a fourth dose comprising:

(a) 30 ug of RNA encoding a SARS-CoV-2 S protein from a Wuhan strain,
(b) 60 ug of RNA encoding a SARS-CoV-2 S protein from a Wuhan strain,
(c) 30 ug of an Omicron-specific vaccine,
(d) 60 ug of an Omicron-specific vaccine,
(e) 30 ug of a bivalent RNA vaccine, comprising 15 ug of RNA encoding a SARS-CoV-2 S protein from a Wuhan strain and 15 ug of RNA encoding a SARS-CoV-2 S protein comprising mutations characteristic of an Omicron variant, or
(f) 60 ug of a bivalent RNA vaccine, comprising 30 ug of RNA encoding a SARS-CoV-2 S protein from a Wuhan strain and 30 ug of RNA encoding a SARS-CoV-2 S protein comprising mutations characteristic of an Omicron variant.

In the present example, for the fourth dose, the RNA encoding a SARS-CoV-2 S protein from a Wuhan variant was BNT162b2, and the RNA encoding a SARS-CoV-2 S protein having mutations characteristic of an Omicron variant comprised the nucleotide sequence of SEQ ID NO: 33.
Sera samples were collected at the time of administering the 4th dose and 7 days afterward, and tested for neutralization antibody titers against a viral particle comprising a SARS-CoV-2 S protein from a Wuhan strain, or a SARS-CoV-2 S protein comprising mutations characteristic of a Delta variant or an Omicron variant.
Neutralization antibody titers were determined using a Fluorescent Focus Reduction Neutralization Test ("FFRNT"). Suitable FFRNT assays are known in the art. The neutralization responses are shown in Fig. 4.
As shown in Fig. 4 (A) subjects administered a fourth dose of 30 ug of an Omciron-specific vaccine exhibited an increase in neutralization antibodies against an Omicron variant as compared to subjects administered a fourth dose of 30 ug of BNT162b2. Administering 60 ug of RNA increased neutralization responses both for BNT162b2 and an

Omicron-specific vaccine, with 60 ug of an Omicron-specific vaccine showing a stronger immune response against an Omicron variant. As shown in Fig. 4 (B), similar effects were observed in a population that included subjects previously or currently infected with SARS-CoV-2 (e.g., as determined by an antibody test and a PCR test, respectively).

Fig. 4 (C-D) provides data for neutalization responses against a Wuhan strain of SARS-CoV-2 in a population of subjects excluding subjects previously or currently infected with SARS-CoV-2 (Fig. 4(C)) and a population of subjects including these subjects (Fig. 4(D)).

Fig. 4 (E-F) provides data for neutralization responses against a Delta variant in a population of subjects excluding subjects previously or currently infected with SARS-CoV-2 (Fig. 4(E)) and a population of subjects including these subjects (Fig. 4(F)).

Fig. 4 (G) shows neutralization responses as compared to subjects administered a 4th dose of 30 ug of BNT162b2. As can be seen in the table, an Omicron-specific vaccine induced a strong response against an Omicron variant, and responses that were at least comparable to that of BNT162b2 for other variants. A bivalent vaccine produced a strong immune response against each SARS-CoV-2 variant tested, both at 30 ug and 60 ug doses.

Reactogenicity of the tested 4th doses was also monitored in patients for 7 days following administration of the 4th dose. Fig. 5 (A) shows local immune responses observed in subjects of different groups as indicated. As can be seen in the figure, 60 ug doses of an Omicron specific vaccine and a bivalent vaccine were found to be more likely to produce pain at the injection site, as compared to that observed with other tested booster doses; however, the pain was rated as mild or moderate for both doses. Redness and swelling responses were low and comparable at each dose tested.

Fig. 5 (B) shows systemic immune responses observed in subjects of different groups as indicated. Systemic responses (as characterized by fever, fatigue, headache, chills, vomiting, diarrhea, muscle pain, or joint pain) were similar for each dose, while fatigue trended higher with the 60 ug doses.

## Claims

1. A method for providing a pharmaceutical RNA preparation for the administration of different doses of the RNA comprising the steps:

   (i) determining different doses in which the RNA is to be administered,
   (ii) determining a concentration of the RNA in the pharmaceutical RNA preparation which allows the administration of the different doses of the RNA with suitable administration volumes and, optionally, suitable dilution of the pharmaceutical RNA preparation, and
   (iii) determining a suitable formulation for the pharmaceutical RNA preparation to ensure a desired storage stability of the RNA in the pharmaceutical RNA preparation at the determined concentration.

2. The method according to claim 1, wherein the lowest dose of the RNA and the largest dose of the RNA differ by a factor of 2 or more and the smallest administration volume for one dose and the largest administration volume for another dose differ by a factor of 2 or less.

3. The method according to claim 1 or 2, wherein the lowest dose of the RNA and the largest dose of the RNA differ by a factor of 2 or more and suitable administration volumes are 100 to 400 μl.

4. The method according to any one of claims 1 to 3, wherein the different doses comprise at least two doses.

5. The method according to any one of claims 1 to 4, wherein the different doses comprise at least three doses.

6. The method according to any one of claims 1 to 5, wherein the different doses of the RNA are to be administered to different age groups and/or different conditions.

7. The method according to any one of claims 1 to 6, wherein the lowest dose of the RNA and the largest dose of the RNA differ by a factor of 2 or more.

8. The method according to any one of claims 1 to 7, wherein the lowest dose of the RNA and the largest dose of the RNA differ by a factor of 3 or more.

9. The method according to any one of claims 1 to 8, wherein the lowest dose of the RNA and the largest dose of the RNA differ by a factor of 5 or more.

10. The method according to any one of claims 1 to 9, wherein the lowest dose of the RNA and the largest dose of the RNA differ by a factor of 10 or more.

11. The method according to any one of claims 1 to 10, wherein the smallest administration volume for one dose and the largest administration volume for another dose differ by a factor of 2 or less.

12. The method according to any one of claims 1 to 11, wherein the smallest administration volume for one dose and the largest administration volume for another dose differ by a factor of 1.5 or less.

13. The method according to any one of claims 1 to 12, wherein suitable administration volumes are 100 to 400 μl.

14. The method according to any one of claims 1 to 13, wherein suitable administration volumes are 150 to 350 μl.

15. The method according to any one of claims 1 to 14, wherein suitable administration volumes are 200 to 300 μl.

16. The method according to any one of claims 1 to 15, wherein optionally a dilution of the pharmaceutical RNA preparation by a factor of 1:10 or less takes place for at least one dose.

17. The method according to any one of claims 1 to 16, wherein optionally a dilution of the pharmaceutical RNA preparation by a factor of 1:8 or less takes place for at least one dose.

18. The method according to any one of claims 1 to 17, wherein optionally a dilution of the pharmaceutical RNA preparation by a factor of 1:6 or less takes place for at least one dose.

19. The method according to any one of claims 1 to 18, wherein at least one dose of the RNA is to be administered without diluting the RNA.

20. The method according to claim 19, wherein the at least one dose of the RNA which is to be administered without diluting the RNA comprises the largest dose of the RNA.

21. The method according to any one of claims 1 to 20, wherein at least one dose of the RNA is to be administered with diluting the RNA.

22. The method according to claim 21, wherein the at least one dose of the RNA which is to be administered with diluting the RNA comprises the lowest dose of the RNA.

23. The method according to any one of claims 1 to 22, wherein the pharmaceutical RNA preparation is provided as multi-dose preparations, wherein each multi-dose preparation allows multiple administrations of a dose of the RNA.

24. The method according to claim 23, wherein the multi-dose preparations have filling volumes that are suitable for pharmaceutical manufacture.

25. The method according to claim 23 or 24, wherein a multi-dose preparation allows the administration of a desired number of doses of the RNA.

26. The method according to claim 25, wherein the desired number of doses of the RNA is 5 or more, e.g., 5-20, 5-15, or 5-10.

27. The method according to any one of claims 23 to 26, wherein a multi-dose preparation allows an optional dilution of the pharmaceutical RNA preparation.

28. The method according to any one of claims 1 to 27, wherein the pharmaceutical RNA preparation is provided in vials.

29. The method according to claim 28, wherein vials which are used to administer different doses of the RNA are labelled differently.

30. The method according to claim 29, wherein the different labels comprises a different color of the lid.

**31.** The method according to any one of claims 1 to 30, wherein the pharmaceutical RNA preparation for administration of different doses of the RNA has a uniform RNA concentration.

**32.** The method according to any one of claims 1 to 31, wherein the pharmaceutical RNA preparation is a vaccine.

**33.** The method according to any one of claims 1 to 32, wherein the RNA encodes an amino acid sequence comprising an antigen, an immunogenic variant thereof, or an immunogenic fragment of the antigen or the immunogenic variant thereof.

**34.** The method according to any one of claims 1 to 33, wherein the RNA encodes an amino acid sequence comprising a SARS-CoV-2 S protein, an immunogenic variant thereof, or an immunogenic fragment of the SARS-CoV-2 S protein or the immunogenic variant thereof.

**35.** The method according to any one of claims 1 to 34, wherein the pharmaceutical RNA preparation is for inducing an immune response against SARS-CoV-2.

**36.** The method according to any one of claims 1 to 35, wherein the pharmaceutical RNA preparation is for vaccination against SARS-CoV-2.

**37.** Use of a pharmaceutical RNA preparation for the administration of different doses of the RNA, comprising the steps:

(i) providing the pharmaceutical RNA preparation,
(ii) administering different doses of the RNA, wherein said different doses of the RNA are administered by administering the same and/or different volumes of the optionally diluted pharmaceutical RNA preparation, wherein
the concentration of the RNA in the pharmaceutical RNA preparation is selected such that it allows the administration of the different doses of the RNA with suitable administration volumes and, optionally, suitable dilution of the pharmaceutical RNA preparation, and
the pharmaceutical RNA preparation is formulated to ensure a desired stability of the RNA in the pharmaceutical RNA preparation.

**38.** The use according to claim 37, wherein the lowest dose of the RNA and the largest dose of the RNA differ by a factor of 2 or more and the smallest administration volume for one dose and the largest administration volume for another dose differ by a factor of 2 or less.

**39.** The use according to claim 37 or 38, wherein the lowest dose of the RNA and the largest dose of the RNA differ by a factor of 2 or more and suitable administration volumes are 100 to 400 $\mu$l.

**40.** The use according to any one of claims 37 to 39, wherein the different doses comprise at least two doses.

**41.** The use according to any one of claims 37 to 40, wherein the different doses comprise at least three doses.

**42.** The use according to any one of claims 37 to 41, wherein the different doses of the RNA are administered to different age groups.

**43.** The use according to any one of claims 37 to 42, wherein the lowest dose of the RNA and the largest dose of the RNA differ by a factor of 2 or more.

**44.** The use according to any one of claims 37 to 43, wherein the lowest dose of the RNA and the largest dose of the RNA differ by a factor of 3 or more.

**45.** The use according to any one of claims 37 to 44, wherein the lowest dose of the RNA and the largest dose of the RNA differ by a factor of 5 or more.

**46.** The use according to any one of claims 37 to 45, wherein the lowest dose of the RNA and the largest dose of the RNA differ by a factor of 10 or more.

**47.** The use according to any one of claims 37 to 46, wherein the smallest administration volume for one dose and the

largest administration volume for another dose differ by a factor of 2 or less.

**48.** The use according to any one of claims 37 to 47, wherein the smallest administration volume for one dose and the largest administration volume for another dose differ by a factor of 1.5 or less.

**49.** The use according to any one of claims 37 to 48, wherein suitable administration volumes are 100 to 400 μl.

**50.** The use according to any one of claims 37 to 49, wherein suitable administration volumes are 150 to 350 μl.

**51.** The use according to any one of claims 37 to 50, wherein suitable administration volumes are 200 to 300 μl.

**52.** The use according to any one of claims 37 to 51, wherein optionally a dilution of the pharmaceutical RNA preparation by a factor of 1:10 or less takes place for at least one dose.

**53.** The use according to any one of claims 37 to 52, wherein optionally a dilution of the pharmaceutical RNA preparation by a factor of 1:8 or less takes place for at least one dose.

**54.** The use according to any one of claims 37 to 53, wherein optionally a dilution of the pharmaceutical RNA preparation by a factor of 1:6 or less takes place for at least one dose.

**55.** The use according to any one of claims 37 to 54, wherein at least one dose of the RNA is administered without diluting the RNA.

**56.** The use according to claim 55, wherein the at least one dose of the RNA which is administered without diluting the RNA comprises the largest dose of the RNA.

**57.** The use according to any one of claims 37 to 56, wherein at least one dose of the RNA is administered with diluting the RNA.

**58.** The use according to claim 57, wherein the at least one dose of the RNA which is administered with diluting the RNA comprises the lowest dose of the RNA.

**59.** The use according to any one of claims 37 to 58, wherein the pharmaceutical RNA preparation is provided as multi-dose preparations, wherein each multi-dose preparation allows multiple administrations of a dose of the RNA.

**60.** The use according to claim 59, wherein the multi-dose preparations have filling volumes that are suitable for pharmaceutical manufacture.

**61.** The use according to claim 59 or 60, wherein a multi-dose preparation allows the administration of a desired number of doses of the RNA.

**62.** The use according to claim 61, wherein the desired number of doses of the RNA is 5 or more, e.g., 5-20, 5-15, or 5-10.

**63.** The use according to any one of claims 59 to 62, wherein a multi-dose preparation allows an optional dilution of the pharmaceutical RNA preparation.

**64.** The use according to any one of claims 37 to 63, wherein the pharmaceutical RNA preparation is provided in vials.

**65.** The use according to claim 64, wherein vials which are used to administer different doses of the RNA are labelled differently.

**66.** The use according to claim 65, wherein the different labels comprises a different color of the lid.

**67.** The use according to any one of claims 37 to 66, wherein the pharmaceutical RNA preparation for administration of different doses of the RNA has a uniform RNA concentration.

**68.** The use according to any one of claims 37 to 67, wherein the pharmaceutical RNA preparation is a vaccine.

**69.** The use according to any one of claims 37 to 68, wherein the RNA encodes an amino acid sequence comprising an antigen, an immunogenic variant thereof, or an immunogenic fragment of the antigen or the immunogenic variant thereof.

**70.** The use according to any one of claims 37 to 69, wherein the RNA encodes an amino acid sequence comprising a SARS-CoV-2 S protein, an immunogenic variant thereof, or an immunogenic fragment of the SARS-CoV-2 S protein or the immunogenic variant thereof.

**71.** The use according to any one of claims 37 to 70, wherein the pharmaceutical RNA preparation is for inducing an immune response against SARS-CoV-2.

**72.** The use according to any one of claims 37 to 71, wherein the pharmaceutical RNA preparation is for vaccination against SARS-CoV-2.

**73.** The use according to any one of claims 37 to 72, wherein the different doses comprise doses of about 10 μg and about 30 μg.

**74.** The use according to any one of claims 37 to 73, wherein the different doses comprise doses of about 3μg, about 10 μg and about 30 μg.

**75.** The use according to any one of claims 37 to 74, wherein the concentration of the RNA in the pharmaceutical RNA preparation is about 0.1 mg/ml.

**76.** The use according to any one of claims 37 to 75, wherein the administration volumes are between about 200 μl and about 300 μl.

**77.** The use according to any one of claims 37 to 76, wherein the RNA in pharmaceutical RNA preparation is formulated in about 10 mM Tris buffer, about 10% sucrose.

**78.** The use according to any one of claims 37 to 77, wherein the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose and wherein

(i) a first dose of the RNA of about 30 μg is administered by administering about 300 μl of undiluted pharmaceutical RNA preparation, and
(ii) a second dose of the RNA of about 10 μg is administered by diluting the pharmaceutical RNA preparation about 1:1 and administering about 200 μl of diluted pharmaceutical RNA preparation.

**79.** The use according to claim 78, wherein a third dose of the RNA of about 3 μg is administered by diluting the pharmaceutical RNA preparation about 1:5,75 and administering about 200 μl of diluted pharmaceutical RNA preparation.

**80.** A pharmaceutical RNA preparation for the administration of different doses of the RNA, wherein the concentration of the RNA in the pharmaceutical RNA preparation is selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation.

**81.** A pharmaceutical RNA preparation for the administration of different doses of the RNA, wherein the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose.

**82.** A system for the administration of different doses of RNA, wherein the system comprises a plurality of vials for administering a plurality of different doses of the RNA, wherein each of the plurality of vials comprises optionally

different volumes of a pharmaceutical RNA preparation, wherein the concentration of the RNA in the pharmaceutical RNA preparation and the volumes of the pharmaceutical RNA preparation in the vials are selected such that it allows the administration of the different doses of the RNA with suitable administration volumes and, optionally, suitable dilution of the pharmaceutical RNA preparation, and the pharmaceutical RNA preparation is formulated to ensure a desired stability of the RNA in the pharmaceutical RNA preparation.

**83.** A system for the administration of different doses of RNA, wherein the system comprises a plurality of vials for administering a plurality of different doses of the RNA, wherein each of the plurality of vials comprises a pharmaceutical RNA preparation, wherein

the RNA encodes an amino acid sequence comprising a SARS-CoV-2 S protein, an immunogenic variant thereof, or an immunogenic fragment of the SARS-CoV-2 S protein or the immunogenic variant thereof, the concentration of the RNA in the pharmaceutical RNA preparation is about 0.1 mg/ml, and the RNA in the pharmaceutical RNA preparation is formulated in about 10 mM Tris buffer, about 10% sucrose, and wherein
a first of the plurality of vials is for administering a first dose of the RNA of about 30 μg by administering about 300 μl of undiluted pharmaceutical RNA preparation, and
a second of the plurality of vials is for administering a second dose of the RNA of about 10 μg by diluting the pharmaceutical RNA preparation about 1:1 and administering about 200 μl of diluted pharmaceutical RNA preparation.

**84.** The system according to claim 83, wherein

the volume of the pharmaceutical RNA preparation in the first of the plurality of vials is about 2.25 ml for administering a total of at least about 6 doses, and
the volume of the pharmaceutical RNA preparation in the second of the plurality of vials is about 1.3 ml for administering a total of at least about 10 doses.

**85.** The system according to claim 83 or 84, wherein a third of the plurality of vials is for administering a third dose of the RNA of about 3 μg by diluting the pharmaceutical RNA preparation about 1:5,75 and administering about 200 μl of diluted pharmaceutical RNA preparation.

**86.** The system according to claim 85, wherein the volume of the pharmaceutical RNA preparation in the third of the plurality of vials is about 0.4 ml for administering a total of at least about 10 doses.

**87.** The system according to any one of claims 82 to 86, wherein vials for administering different doses of the RNA are labelled differently.

**88.** The system according to claim 87, wherein the different labels comprises a different color of the lid.

**89.** A sealed multi-dose vial comprising a stock composition comprising:

(a) 0.1 mg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 10 mM Tris buffer; and
(c) 300 mM sucrose.

**90.** The vial of claim 89, wherein the pH of the stock composition is pH 7.4.

**91.** The vial of claim 89 or 90, which is **characterized in that** the RNA and the lipid nanoparticles remain stable at a refrigerated temperature for at least 10 weeks.

**92.** The vial of any one of claims 89-91, comprising 2.25 mL of the stock composition.

**93.** The vial of claim 92, comprising 6 doses.

**94.** A tray comprising 10 sealed multi-dose vials of claim 92 or 93.

**95.** A package comprising 20 trays of claim 94.

**96.** The vial of any one of claims 89-91, comprising 1.3 mL of the stock composition.

**97.** The vial of claim 96, comprising 10 doses.

**98.** A tray comprising 10 sealed multi-dose vials of claim 96 or 97.

**99.** A package comprising 20 trays of claim 98.

**100.** The vial of any one of claims 89-91, comprising 0.4 mL of the stock composition.

**101.** The vial of claim 100, comprising 10 doses.

**102.** A tray comprising 10 sealed multi-dose vials of claim 100 or 101.

**103.** A package comprising 20 trays of claim 102.

**104.** A multi-dose vial comprising 2.6 mL of diluted drug product, wherein the diluted drug product comprises:

(a) 50 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 5 mM Tris buffer;
(c) 150 mM sucrose; and
(d) 0.45% sodium chloride.

**105.** A multi-dose vial comprising 2.7 mL of diluted drug product, wherein the diluted drug product comprises:

(a) 14.8 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 1.5 mM Tris buffer;
(c) 44.4 mM sucrose; and
(d) 0.77% sodium chloride.

**106.** A syringe comprising an injection volume of 300 uL of a drug product, wherein the drug product comprises:

(a) 0.1 mg/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 10 mM Tris buffer; and
(c) 300 mM sucrose.

**107.** The syringe of claim 106, wherein the syringe is a low dead-volume syringe.

**108.** A syringe comprising an injection volume of 200 uL of the diluted drug product, wherein the diluted drug product comprises:

(a) 50 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 5 mM Tris buffer;
(c) 150 mM sucrose; and
(d) 0.45% sodium chloride.

**109.** The syringe of claim 108, wherein the syringe is a low dead-volume syringe.

**110.** A syringe comprising an injection volume of 200 uL of the diluted drug product, wherein the diluted drug product comprises:

(a) 14.8 ug/mL RNA comprising an open reading frame encoding a polypeptide that comprises a SARS-CoV-2 protein or an immunogenic fragment or variant thereof, wherein the RNA is formulated in lipid nanoparticles comprising a cationically ionizable lipid, a neutral lipid, a steroid, and a polyethylene glycol (PEG)-lipid.
(b) 1.5 mM Tris buffer;
(c) 44.4 mM sucrose; and
(d) 0.77% sodium chloride.

**111.** The syringe of claim 110, wherein the syringe is a low dead-volume syringe.

# Figure 1A

| *Age Cohort* | 12+ | 12+ | 5-11 | 2-<5y |
|---|---|---|---|---|
| Formulation | PBS | TS | TS | TS |
| Vial Cap Color | PURPLE 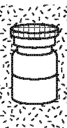 | GRAY 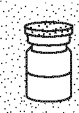 | ORANGE 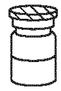 | MAROON 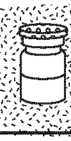 |
| Refrigerated Storage Time | 1 month | 10 Weeks | 10 Weeks | 10 Weeks |
| Fill Volume | .45mL | 2.25mL | 1.3mL | 0.4mL |
| Dose/Injectable Volume | 30 mcg | 30 mcg | 10 mcg | 3 mcg |
| Dilution Status | Dilution Required | No Dilution | Dilution Required | Dilution Required |
| Doses per Vial | 6; requires LDV | 6; requires LDV | 10; requires LDV | 10; requires LDV |

# Figure 1B

| Dose | 30 mcg | 30 mcg | 10 mcg | 3 mcg |
|---|---|---|---|---|
| Stock Buffer | PBS<br>Sucrose<br>pH 7.4 | 10 mM Tris<br>10% sucrose*<br>pH 7.4 | 10 mM Tris<br>10% sucrose*<br>pH 7.4 | 10 mM Tris<br>10% sucrose*<br>pH 7.4 |
| **Dilution**<br>(e.g., using 0.9% sodium chloride) | 0.45:1.8 | NA | 1:1 | 0.4:2.3 |
| Administered Buffer | PBS<br>Sucrose | 10 mM Tris<br>10% sucrose | 5 mM Tris<br>5% sucrose | 1.5 mM Tris<br>1.5% sucrose |
| Administered Volume | 300 μL | 300 μL | 200 μL | 200 μL |

*10% sucrose corresponds to 300 mM sucrose

| Name of Ingredients | | Function | Concentration (mg/mL) | Amount per 0.3 mL dose |
|---|---|---|---|---|
| BNT162b2 drug substance | | Active ingredient | 0.1 | 30 µg |
| ALC-0315 | | Functional lipid | 1.43 | 0.43 mg |
| ALC-0159 | | Functional lipid | 0.18 | 0.05 mg |
| DSPC | | Structural lipid | 0.31 | 0.09 mg |
| Cholesterol | | Structural lipid | 0.62 | 0.19 mg |
| Sucrose | | Cryoprotectant | 103 | 31 mg[b] |
| Tromethamine (Tris base) | | Buffer component | 0.20 | 0.06 |
| Tris (hydroxymethyl) aminomethane hydrochloride (Tris HCl) | | Buffer component | 1.32 | 0.4 |
| Water for Injection | | Solvent/vehicle | q.s. | q.s. |
| **Processing Aids/Residues**[a] | | | | |
| Ethanol | | Processing aid | N/A | |
| Citric acid monohydrate | | Processing aid | | |
| Sodium citrate | | Processing aid | | |
| Sodium hydroxide | | Processing aid | | |
| HEPES | | Drug substance buffer component | | |
| EDTA | | Drug substance buffer component | | |

a. The processing aids and drug substance formulation buffer components are residues that are essentially removed through the manufacturing process and are not considered ingredients (excipients).

b. Values are rounded to maintain the same level of precision as the label claim, with trailing decimals not shown, where applicable. For example, 31 mg sucrose is rounded from 30.81.

Abbreviations:

ALC-0315 = ((4-hydroxybutyl)azanediyl)bis(hexane-6,1-diyl)bis(2-hexyldecanoate)
ALC-0159 = 2-[(polyethylene glycol)-2000]-N,N-ditetradecylacetamide
DSPC = 1,2-distearoyl-sn-glycero-3-phosphocholine
q.s. = quantum satis (as much as may suffice)
HEPES = 4-(2-hydroxyethyl)-1-piperazineethanesulfonic acid
EDTA = edetate disodium dihydrate

**Figure 2**

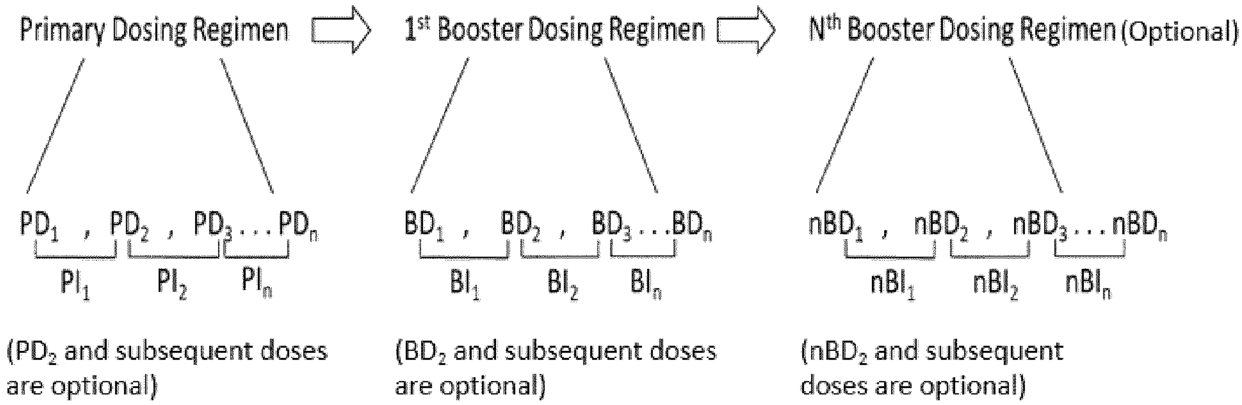

Primary Dosing Regimen
1st Booster Dosing Regimen
Nth Booster Dosing Regimen (Optional)
PD1 , PD2 , PD3 . . . PDn
PI1 PI2 PIn
BD1 , BD2 , BD3 . . . BDn
BI1 BI2 BIn
nBD1 , nBD2 , nBD3 . . . nBDn
nBI1 nBI2 nBIn
(PD2 and subsequent doses are optional)
(BD2 and subsequent doses are optional)
(nBD2 and subsequent doses are optional)


**Figure 3**

# Figure 4

A

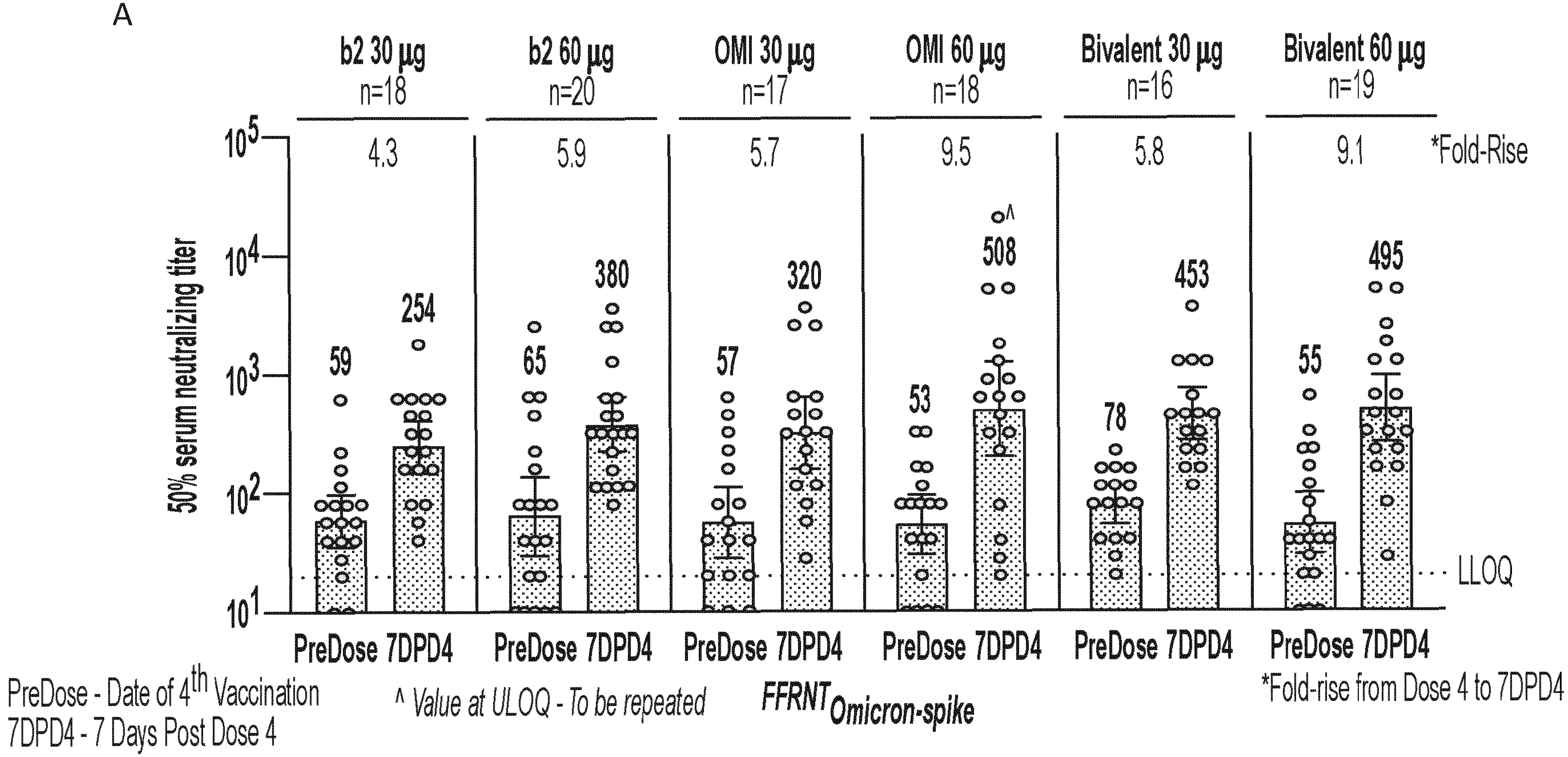
b2 30 µg n=18
b2 60 µg n=20
OMI 30 µg n=17
OMI 60 µg n=18
Bivalent 30 µg n=16
Bivalent 60 µg n=19
4.3
5.9
5.7
9.5
5.8
9.1
*Fold-Rise
50% serum neutralizing titer
59
254
65
380
57
320
53
508
78
453
55
495
LLOQ
PreDose 7DPD4
PreDose 7DPD4
PreDose 7DPD4
PreDose 7DPD4
PreDose 7DPD4
PreDose 7DPD4
PreDose - Date of 4th Vaccination
7DPD4 - 7 Days Post Dose 4
^ Value at ULOQ - To be repeated
FFRNT Omicron-spike
*Fold-rise from Dose 4 to 7DPD4

# Figure 4 (Continued)

B

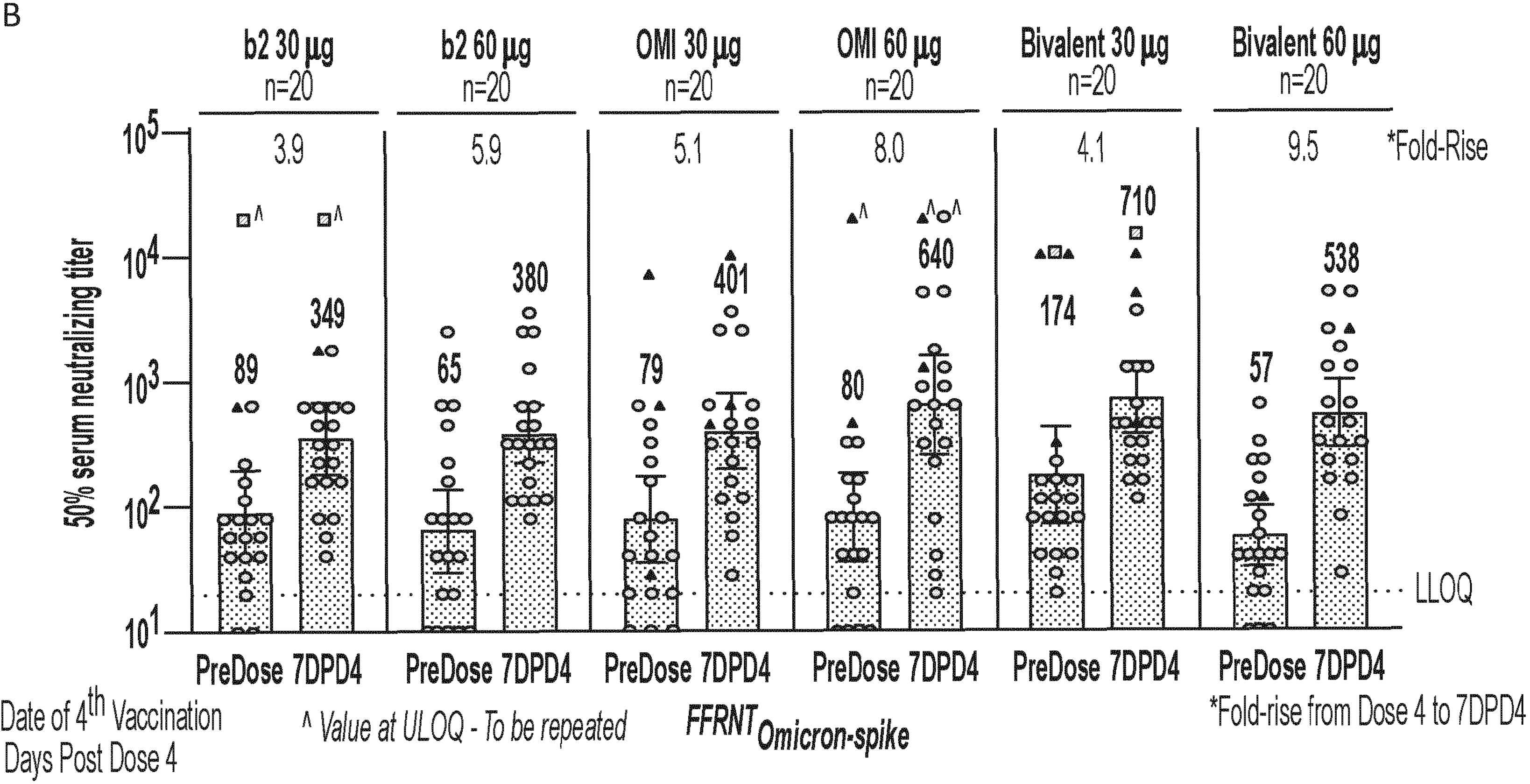


PreDose - Date of 4th Vaccination
7DPD4 - 7 Days Post Dose 4

^ Value at ULOQ - To be repeated

$FFRNT_{Omicron\text{-}spike}$

*Fold-rise from Dose 4 to 7DPD4

# Figure 4 (Continued)

C

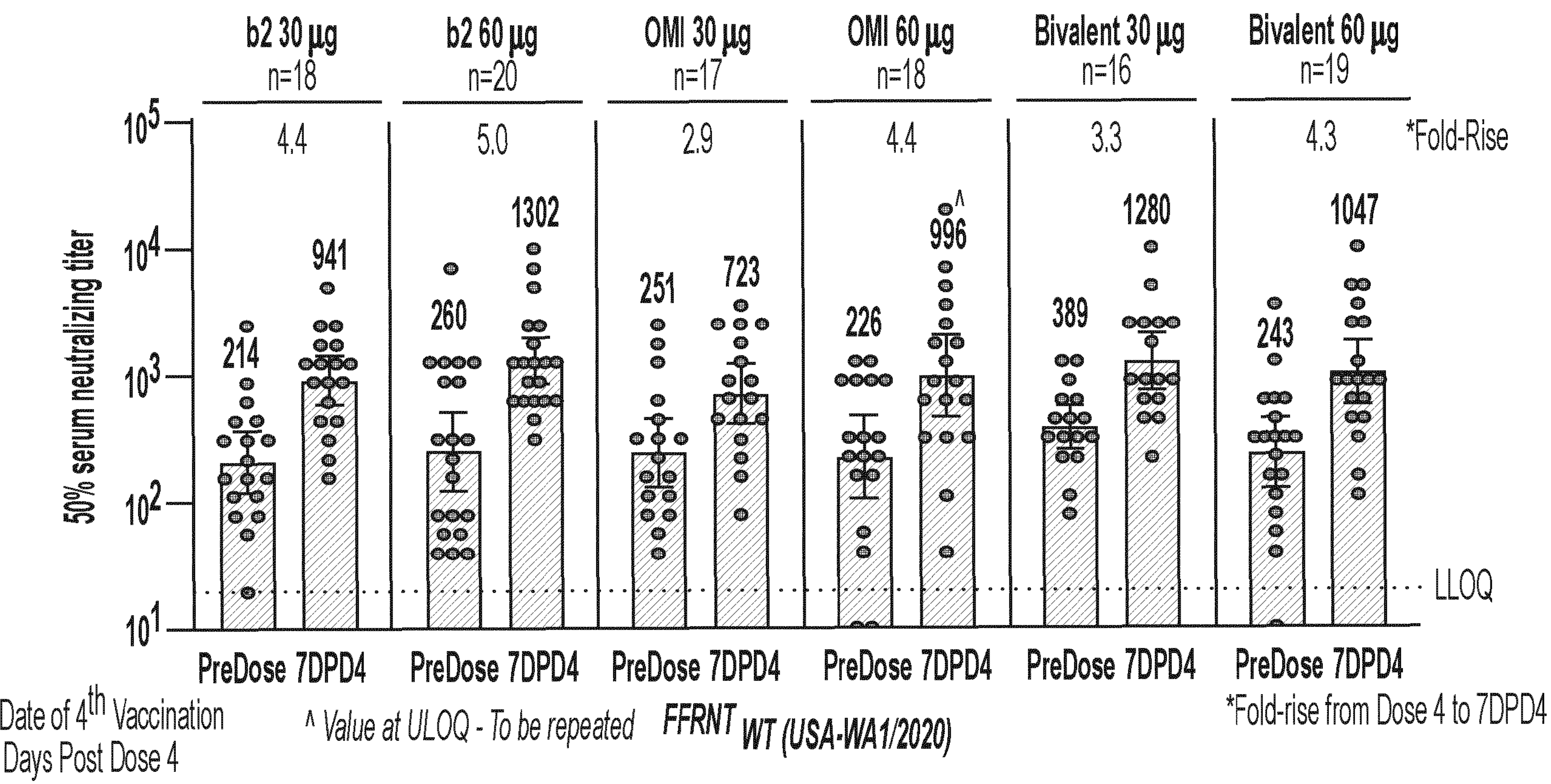


PreDose - Date of 4th Vaccination
7DPD4 - 7 Days Post Dose 4

^ *Value at ULOQ - To be repeated* ***FFRNT WT (USA-WA1/2020)***

*Fold-rise from Dose 4 to 7DPD4

# Figure 4 (Continued)

D

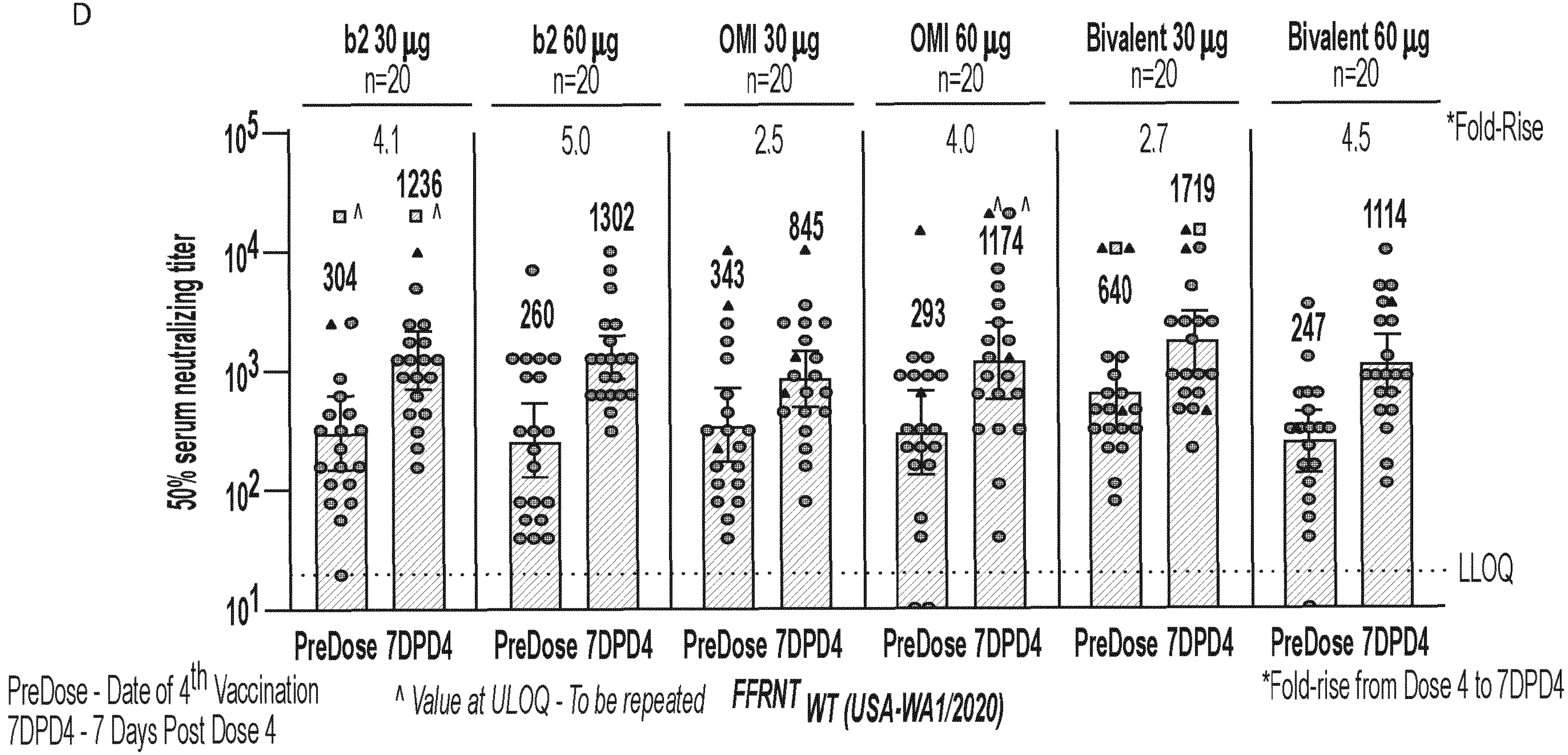

# Figure 4 (Continued)

E

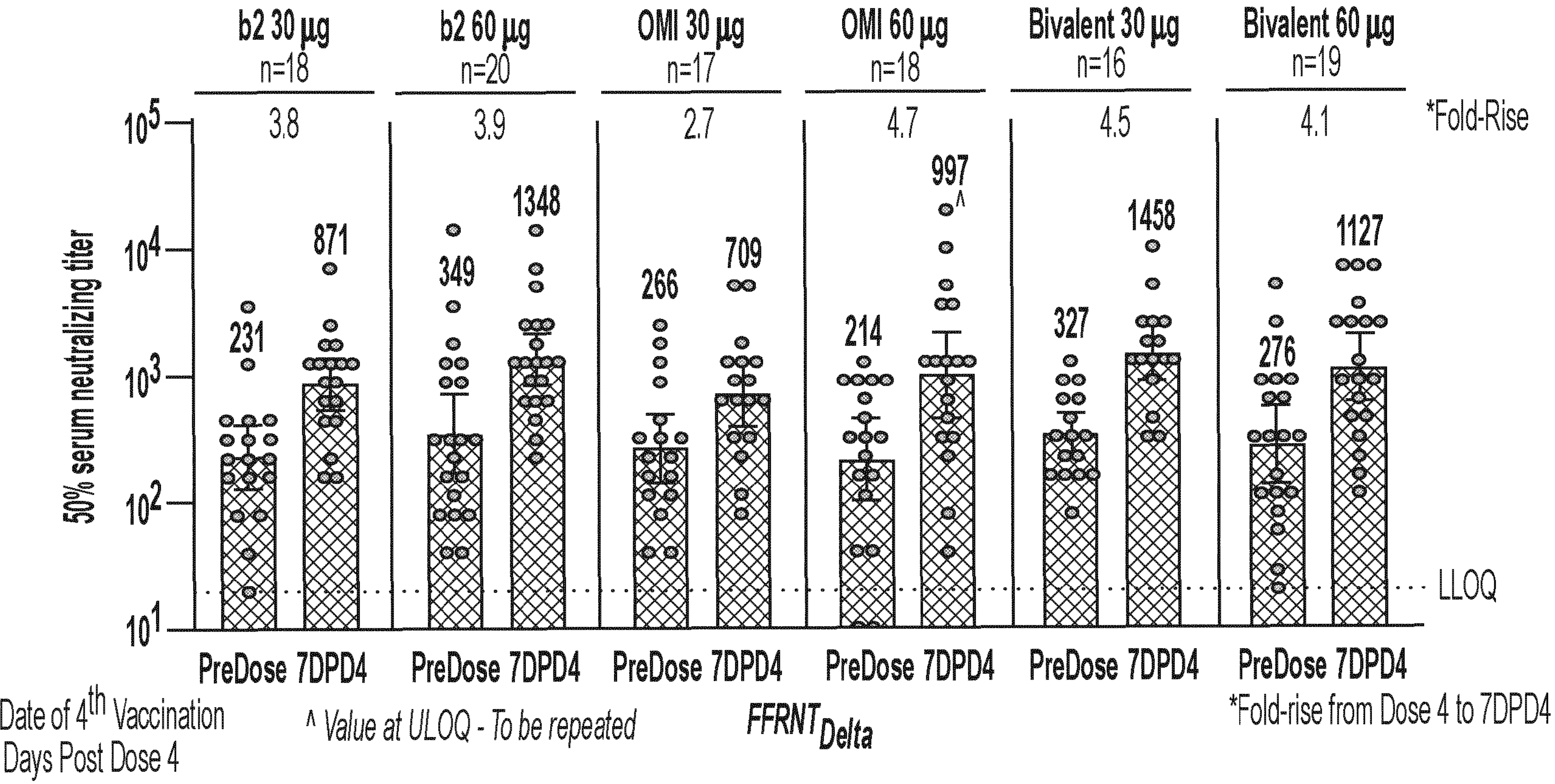


PreDose - Date of 4th Vaccination
7DPD4 - 7 Days Post Dose 4

^ *Value at ULOQ - To be repeated*

***FFRNT$_{Delta}$***

*Fold-rise from Dose 4 to 7DPD4

# Figure 4 (Continued)

F

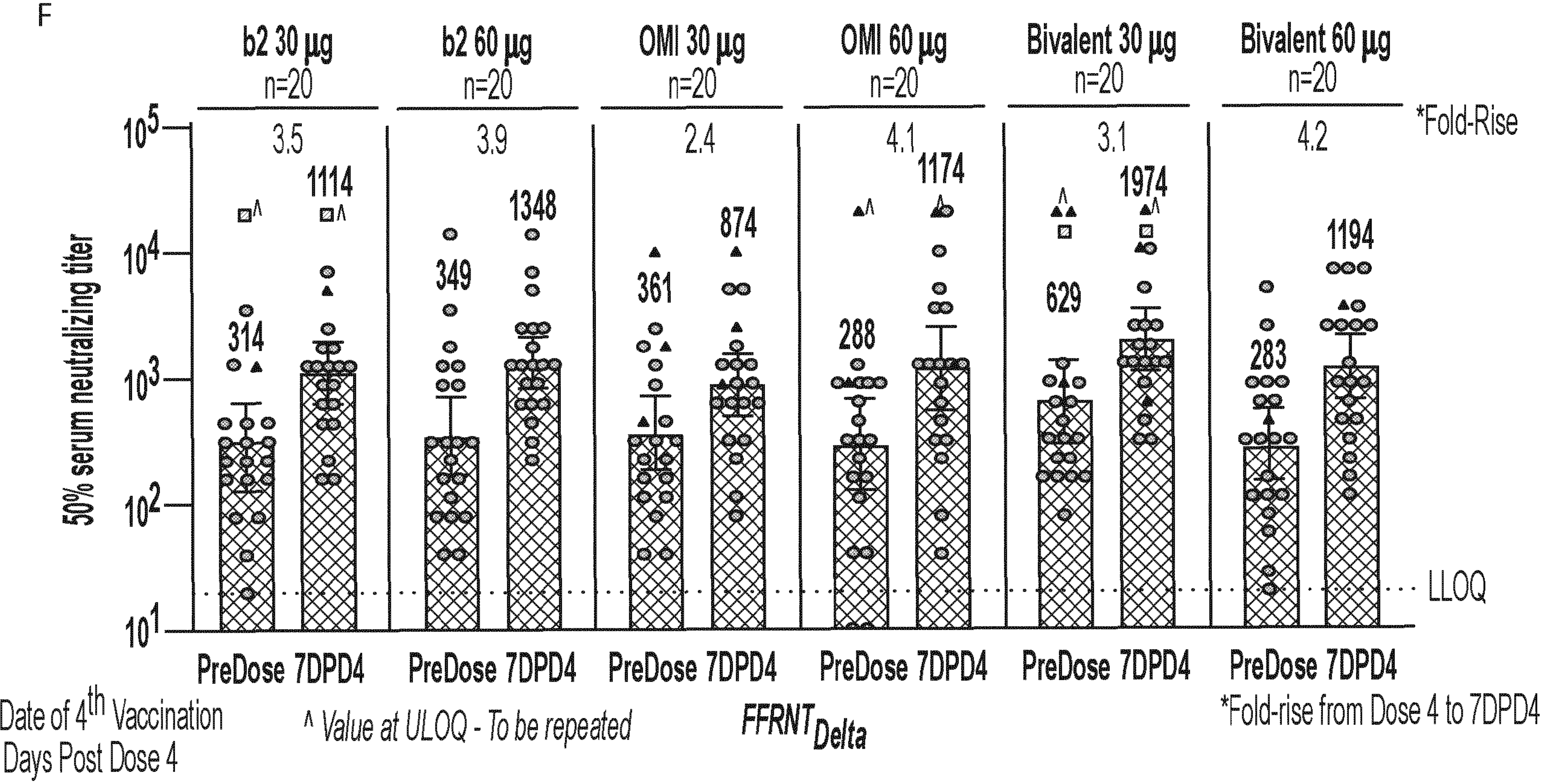


PreDose - Date of 4th Vaccination
7DPD4 - 7 Days Post Dose 4

^ *Value at ULOQ - To be repeated*

***FFRNT$_{Delta}$***

*Fold-rise from Dose 4 to 7DPD4

# Figure 4 (Continued)

G

**D7PD4 GMR vs. b2-30 µg**

*Subjects with evidence of existing (POS PCR) or preexisting (POS N-Ig-Binding) SARS-CoV-2 infection excluded*

| | b2-60 µg | OMI 30 µg | OMI 60 µg | Bivalent 30 µg | Bivalent 60 µg |
|---|---|---|---|---|---|
| WT | 1.38 | 0.77 | 1.06 | 1.36 | 1.11 |
| Delta | 1.55 | 0.81 | 1.14 | 1.67 | 1.29 |
| Omicron | 1.50 | 1.26 | 2.00 | 1.78 | 1.95 |

*All subjects included*

| | b2-60 µg | OMI 30 µg | OMI 60 µg | Bivalent 30 µg | Bivalent 60 µg |
|---|---|---|---|---|---|
| WT | 1.05 | 0.68 | 0.95 | 1.39 | 0.90 |
| Delta | 1.21 | 0.78 | 1.05 | 1.77 | 1.07 |
| Omicron | 1.09 | 1.15 | 1.83 | 2.03 | 1.54 |

# Figure 5

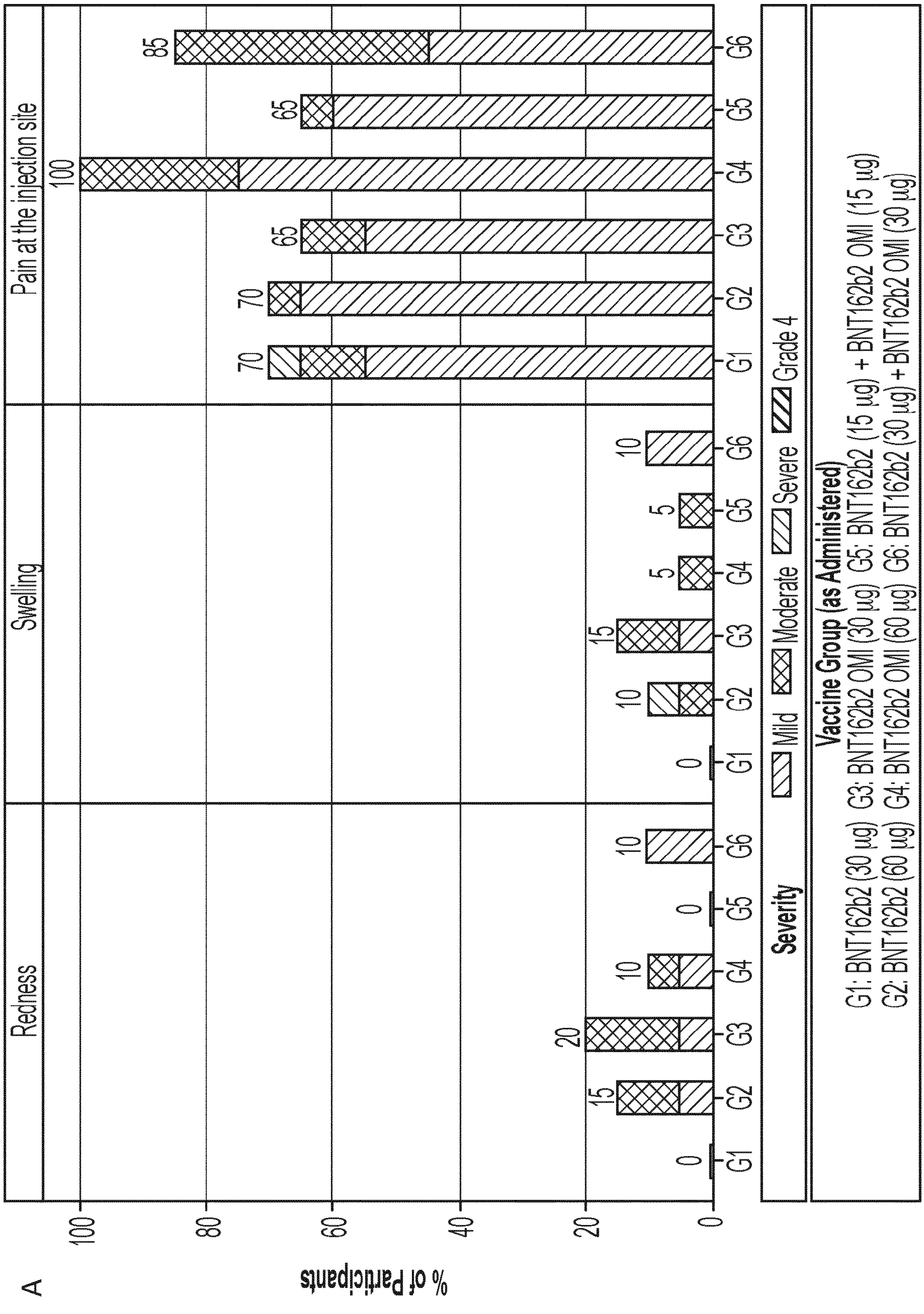
A
% of Participants
0
20
40
60
80
100
Redness
Swelling
Pain at the injection site
G1
G2
G3
G4
G5
G6
0
15
20
10
0
10
0
10
15
5
5
10
70
70
65
100
65
85
Severity
Mild
Moderate
Severe
Grade 4
Vaccine Group (as Administered)
G1: BNT162b2 (30 µg) G3: BNT162b2 OMI (30 µg) G5: BNT162b2 (15 µg) + BNT162b2 OMI (15 µg)
G2: BNT162b2 (60 µg) G4: BNT162b2 OMI (60 µg) G6: BNT162b2 (30 µg) + BNT162b2 OMI (30 µg)

# Figure 5 (Continued)

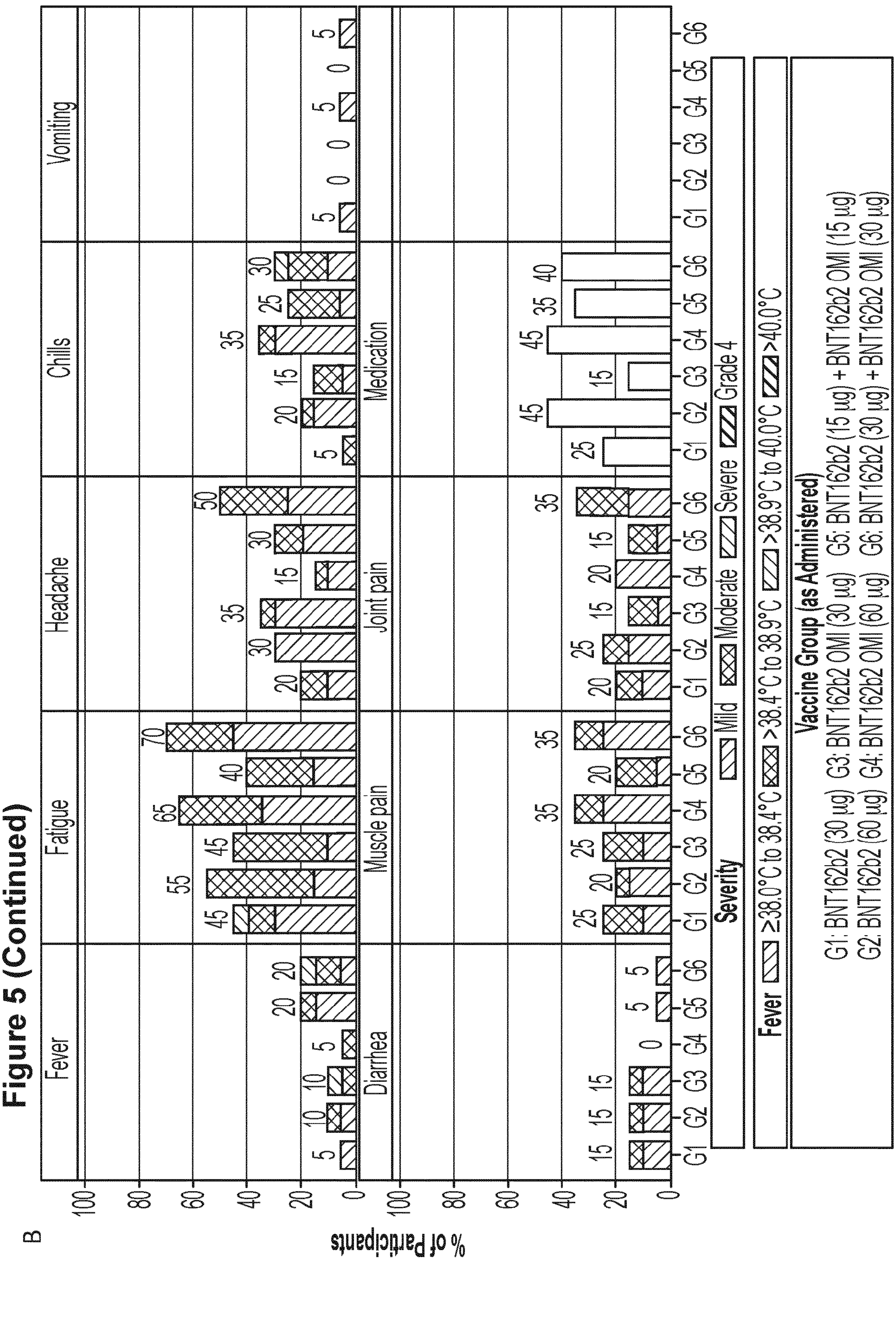

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017060314 A
- WO 2007036366 A
- EP 2019056502 W
- WO 2016005324 A1
- US 20050287153 A
- US 20070003549 A
- US 20070048282 A
- US 5876969 A
- WO 2011124718 A
- WO 2013075066 A
- WO 20110514789 A
- US 20070178082 A
- US 20070269422 A
- US 20100113339 A
- WO 2009083804 A
- WO 2009133208 A
- US 7176278 B
- US 8158579 B
- US 20140220017 A
- US 20170145062 A
- US 20120094909 A
- WO 2021243122 A2
- WO 2021163365 A
- WO 2021243122 A
- WO 2017182524 A
- WO 2017075531 A
- WO 2018081480 A
- WO 2013143683 A
- WO 2021188969 A
- WO 2021213924 A1


### Non-patent literature cited in the description


- **VOLZ et al.** *Nature,* 2021, https://doi.org/10.1038/s41586-021-03470-x
- **WASHINGTON et al.** *Cell,* 2021, https://doi.org/10.1016/j.cell.2021.03.052
- **SMITH ; WATERMAN.** *Ads App. Math.,* 1981, vol. 2, 482
- **NEDDLEMAN ; WUNSCH.** *J. Mol. Biol.,* 1970, vol. 48, 443
- Molecular Cloning: A Laboratory Manual. Cold Spring Harbor Laboratory Press, 2012
- **KOPPEL, D.** *J. Chem. Phys.,* 1972, vol. 57, 4814-4820
- **BUCHHOLZ et al.** *Electrophoresis,* 2001, vol. 22, 4118-4128
- **B.H. ZIMM.** *J. Chem. Phys.,* 1945, vol. 13, 141
- **P. DEBYE.** *J. Appl. Phys.,* 1944, vol. 15, 338
- **W. BURCHARD.** *Anal. Chem.,* 2003, vol. 75, 4279-4291
- **JOSÉ et al.** *Future Microbiol.,* 2009, vol. 4, 837-856
- **GOULD et al.** *Antiviral Res.,* 2010, vol. 87, 111-124
- **HOLTKAMP et al.** *Blood,* 2006, vol. 108, 4009-4017
- **KONTERMANN.** *Expert Opin Biol Ther,* July 2016, vol. 16 (7), 903-15
- **KENNETH, A. et al.** *Chemical Stability of Pharmaceuticals: A Handbook for Pharmacists*
- **PETERS et al.** *Pharmacokinetic Analysis: A Practical Approach,* 1996
- **GIBALDI, M. et al.** Pharmacokinetics. Marcel Dekker, 1982
- **HSIEH, CHING-LIN et al.** Structure-based design of prefusion-stabilized SARS-CoV-2 20 spikes. *Science,* 2020, vol. 369.6510, 1501-1505
- **DAI, LIANPAN et al.** A universal design of betacoronavirus vaccines against COVID-19, MERS, and SARS. *Cell,* 2020, vol. 182.3, 722-733
- **KACZMAREK, J. C. et al.** *Genome Medicine,* 2017, vol. 9, 60
- **LÄCHELT, ULRICH ; ERNST WAGNER.** Nucleic acid therapeutics using polyplexes: a journey of 50 years (and beyond). *Chemical reviews,* 2015, vol. 115.19, 11043-11078
- **PLUCINSKI, ALEXANDER ; ZAN LYU ; BERNHARD VKJ SCHMIDT.** Polysaccharide nanoparticles: from fabrication to applications. *Journal of Materials Chemistry B,* 2021
- **TENCHOV, RUMIANA et al.** Lipid Nanoparticles-From Liposomes to mRNA Vaccine Delivery, a Landscape of Research Diversity and Advancement. *ACS nano,* 2021, vol. 15.11, 16982-17015
- Remington's Pharmaceutical Sciences. Mack Publishing Co, 1985